# EUROPEAN PATENT APPLICATION

(11) **EP 1 889 835 A1**
(43) Date of publication of application: **20.02.2008**
(21) Application number: 06746885.0
(22) Date of filing: 26.05.2006
(51) Int. Cl.: C07D 211/72, A61K 31/445, A61K 31/454, A61K 31/4545, A61K 31/496, A61K 31/5377, A61K 31/541, A61P 7/02, C07D 401/06

(54) **CYCLIC AMINE DERIVATIVE HAVING SUBSTITUTED ALKYL GROUP**

(30) Priority: 27.05.2005 JP 2005155009
(71) Applicant: Daiichi Sankyo Company, Limited, Chuo-ku Tokyo 103-8426 (JP)
(72) Inventor: KIMURA, Tomio c/o Daiichi Sankyo Company Ltd.,, Shinagawa-ku Tokyo 140-8710 (JP); TANAKA, Naoki c/o Daiichi Sankyo Company Ltd.,, Shinagawa-ku Tokyo 140-8710 (JP); KOBAYASHI, Hiroyuki c/o Daiichi Sankyo Company Ltd.,, Shinagawa-ku Tokyo 140-8710 (JP); SUGIDACHI, Atsuhiro c/o Daiichi Sankyo Company Ltd.,, Shinagawa-ku Tokyo 140-8710 (JP)
(74) Representative: Gibson, Christian John Robert
(86) International application number: PCT/JP2006/310556
(87) International publication number: WO 2006/126676

(57) **Abstract**

A compound having the general formula (I), pharmacologically acceptable salts thereof or prodrugs thereof: 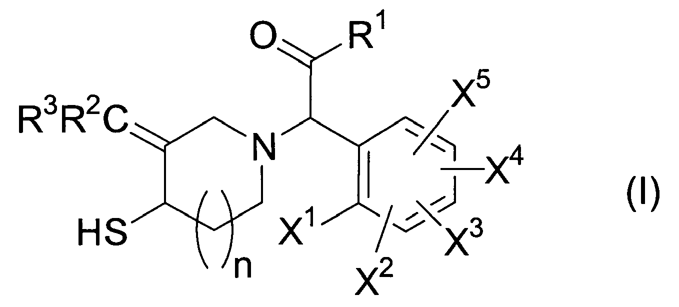 [wherein R¹ represents a hydrogen atom, a C1-C6 alkyl group which may be substituted, a C3-C6 cycloalkyl group which may be substituted, a C1-C6 alkoxy group which may be substituted or a C6-C10 aryl group which may be substituted;
R² represents a hydrogen atom, a halogen atom, a carboxy group, a C2-C7 alkoxycarbonyl group, a carbamoyl group, a cyano group, a C1-C6 alkyl group, a halogeno C1-C6 alkyl group, a C1-C6 alkyl group substituted by a heteroaryl group, a C1-C6 alkoxy group, a halogeno C1-C6 alkoxy group, a hydroxy C1-C6 alkyl group, a C2-C12 alkoxyalkyl group, a formyl group, a C2-C7 alkanoyl group, a C4-C7 cycloalkylcarbonyl group, a C2-C7 alkylcarbamoyl group, a di(C1-C6 alkyl)carbamoyl group, a group of formula R⁴-CO-CR⁵R⁶-(CH₂)ₘ-, a group of formula R⁷-CO-(CH₂)₁-N(R⁸)- or a sulfamoyl C1-C6 alkyl group;
R³ represents a substituted C1-C6 alkyl group, a heterocyclyl group or a heterocyclyl group substituted with 1 to 5 substituents;
X¹, X², X³, X⁴ and X⁵ each independently represents a hydrogen atom, a halogen atom, an amino group, a carboxy group, a carbamoyl group, a cyano group, a nitro group, a C1-C6 alkyl group, a halogeno C1-C6 alkyl group, a C1-C6 alkoxy group, or a halogeno C1-C6 alkoxy group; and
n represents an integer of 0 to 2].

## Description

### [Technical Field]

The present invention relates to compounds which exhibit activity in the inhibition of platelet aggregation, pharmacologically acceptable salts thereof, and prodrugs thereof.

### [Background of the Invention]

Recently, the number of patients with cardiovascular diseases associated with aging of the population and changes of eating habits and lifestyle has risen markedly. Since thrombotic diseases such as cerebral infarction, myocardial infarction and peripheral circulatory disorders have not only high morbidity but also result in poor prognosis and limitation on activities of daily living, patient with these disorders have an undue burden of personal and social disability. It is well known that the direct causes of these diseases are angiostenosis caused by thrombus induced by platelet activation (adhesion to damaged areas of blood vessels, release of physiologically active substances, clot formation, and so on) and ischemia associated with angiostenosis. Thus, antithrombotic agents that inhibit platelet activation play important roles in preventing the occurrence and recurrence of these diseases as well as in their treatment. Furthermore, these agents are considered to become more and more important in the future as the number of patients with thrombotic diseases increases.

Several biological substances related to platelet aggregation, such as adenosine 5'-diphosphate (ADP), thromboxane A₂ (TXA₂), collagen, serotonin (5-hydroxytryptamine, 5-HT) and the like, are known. Moreover, P2Y₁ and P2Y₁₂ receptors are known as ADP receptors. Some existing antithrombotic agents act by exerting antagonistic action against these receptors. Examples of such antithrombotic agents are ticlopidine and clopidogrel, which have thienopyridine structures.

In addition, compounds as described in Patent Documents 1 and 2 are known as compounds having non-thienopyridine structures and antagonistic action against ADP receptors. However, there are certain problems in that these compounds are chemically unstable or only weakly active.
Patent Document 1: WO98/08811
Patent Document 2: WO99/43648

### [Disclosure of the Invention]

### [Problems to be Solved by the Invention]

The present inventors, in order to create novel antithrombotic agents, have diligently explored chemically stable compounds having non-thienopyridine structures and activity in the inhibition of platelet aggregation, and found that compounds having the general formula (I) of the present invention, pharmacologically acceptable salts thereof and prodrugs thereof have desirable characteristics, and thus completed the present invention.

The present invention provides compounds having the general formula (I), pharmacologically acceptable salts thereof or prodrugs thereof; pharmaceutical compositions comprising compounds having the general formula (I), pharmacologically acceptable salts thereof or prodrugs thereof, as active ingredients (particularly pharmaceutical compositions for prophylactic or therapeutic agents for diseases related to thrombus or embolus formation); use of the compound having the general formula (I), pharmacologically acceptable salts thereof or prodrugs thereof to manufacture pharmaceutical compositions (particularly pharmaceutical compositions for prophylactic or therapeutic agents for diseases related to thrombus or embolus formation); use of compounds having the general formula (I), pharmacologically acceptable salts thereof or prodrugs thereof for prophylactic or therapeutic agents for diseases related to thrombus or embolus formation; and prophylactic or therapeutic methods for diseases (particularly diseases related to thrombus or embolus formation) by administration of pharmacologically effective dose of compounds having the general formula (I), pharmacologically acceptable salts thereof or prodrugs thereof to warm-blooded animals (especially humans).

### [Means for Solving the Problems]

The present invention relates the compound having the general formula (I) shown below, 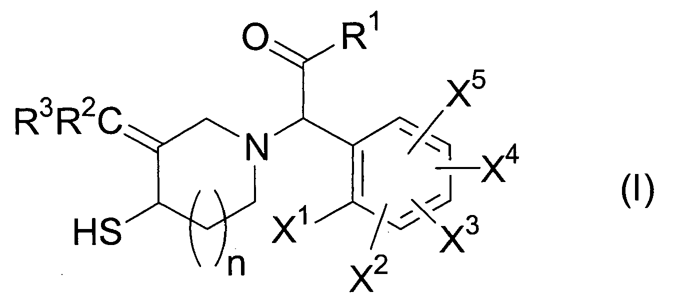
[wherein R¹ represents a hydrogen atom, a C1-C6 alkyl group which may be substituted (said substituent group represents a halogen atom or a C1-C6 alkoxy group), a C3-C6 cycloalkyl group which may be substituted (said substituent group represents a halogen atom or a C1-C6 alkoxy group), a C1-C6 alkoxy group which may be substituted (said substituent group represents a halogen atom or a C1-C6 alkoxy group), or a C6-C10 aryl group which may be substituted (said substituent group represents a halogen atom, a C1-C6 alkyl group, a C1-C6 alkoxy group, a cyano group or a nitro group);
R² represents a hydrogen atom, a halogen atom, a carboxy group, a C2-C7 alkoxycarbonyl group, a carbamoyl group, a cyano group, a C1-C6 alkyl group, a halogeno C1-C6 alkyl group, a C1-C6 alkyl group substituted by a heteroaryl group, a C1-C6 alkoxy group, a halogeno C1-C6 alkoxy group, a hydroxy C1-C6 alkyl group, a C2-C12 alkoxyalkyl group, a formyl group, a C2-C7 alkanoyl group, a C4-C7 cycloalkylcarbonyl group, a C2-C7 alkylcarbamoyl group, a di(C1-C6 alkyl)carbamoyl group, a group of formula R⁴-CO-CR⁵R⁶-(CH₂)ₘ- {wherein R⁴ represents a hydroxyl group, an amino group, a C1-C6 alkyl group, a C1-C6 alkylamino group, a di(C1-C6 alkyl)amino group, a hydroxyamino group, a C1-C6 alkoxyamino group or a C1-C6 alkoxy group; R⁵ and R⁶ are the same or different and each represents a hydrogen atom or a C1-C6 alkyl group; and m represents an integer of from 0 to 5}, a group of formula R⁷-CO-(CH₂)₁-N(R⁸)- {wherein R⁷ represents a hydroxyl group, an amino group, a C1-C6 alkyl group, a C1-C6 alkylamino group, a di(C1-C6 alkyl)amino group, a hydroxyamino group, a C1-C6 alkoxyamino group or a C1-C6 alkoxy group; R⁸ represents a hydrogen atom or a C1-C6 alkyl group; and 1 represents an integer of from 0 to 5} or a sulfamoyl C1-C6 alkyl group;
R³ represents a substituted C1-C6 alkyl group {said substituent group represents a C6-C10 aryl group or a C6-C10 aryl group substituted with from 1 to 5 substituents selected from <Substituent group α>; a heterocyclyl group or a heterocyclyl group substituted with from 1 to 5 substituents selected from <Substituent group α> (said heterocyclyl groups may be substituted by from 1 to 4 oxo groups); a heteroaryl group or a heteroaryl group substituted with from 1 to 5 substituents selected from <Substituent group α>; or a substituent selected from <Substituent group β>}, or a heterocyclyl group or a heterocyclyl group substituted with from 1 to 5 substituents selected from <Substituent group α> (said heterocyclyl groups may be substituted by from 1 to 4 oxo groups);
X¹, X², X³, X⁴ and X⁵ each independently represent a hydrogen atom, a halogen atom, an amino group, a carboxy group, a carbamoyl group, a cyano group, a nitro group, a C1-C6 alkyl group, a halogeno C1-C6 alkyl group, a C1-C6 alkoxy group or a halogeno C1-C6 alkoxy group;
n represents an integer of from 0 to 2,
<Substituent group α> is defined by a halogen atom, an amino group, a carboxy group, a C2-C7 alkoxycarbonyl group, a carbamoyl group, a cyano group, a hydroxyl group, a nitro group, a C1-C6 alkyl group, a halogeno C1-C6 alkyl group, a C1-C6 alkyl group substituted with heteroaryl group(s), a C1-C6 alkoxy group, a halogeno C1-C6 alkoxy group, a hydroxy C1-C6 alkyl group, a C2-C12 alkoxyalkyl group, a formyl group, a C2-C7 alkanoyl group, a C4-C7 cycloalkylcarbonyl group, a C1-C6 alkylamino group, a di(C1-C6 alkyl)amino group, a C2-C7 alkylcarbamoyl group, a di(C1-C6 alkyl)carbamoyl group, a group of formula R⁴-CO-CR⁵R⁶-(CH₂)ₘ- {wherein R⁴ represents a hydroxyl group, an amino group, a C1-C6 alkyl group, a C1-C6 alkylamino group, a di(C1-C6 alkyl)amino group, a hydroxyamino group, a C1-C6 alkoxyamino group or a C1-C6 alkoxy group; R⁵ and R⁶ are the same or different and each represents a hydrogen atom or a C1-C6 alkyl group; and m represents an integer of from 0 to 5} and a sulfamoyl C1-C6 alkyl group; and
<Substituent group β> is defined by a halogen atom, an amino group, a carboxy group, a C2-C7 alkoxycarbonyl group, a carbamoyl group, a cyano group, a hydroxyl group, a nitro group, a C1-C6 alkoxy group, a halogeno C1-C6 alkoxy group, a formyl group, a C2-C7 alkanoyl group, a C4-C7 cycloalkylcarbonyl group, a C1-C6 alkylamino group, a di(C1-C6 alkyl)amino group, a C2-C7 alkylcarbamoyl group, a di(C1-C6 alkyl)carbamoyl group, a hydroxyaminocarbonyl group, a (C1-C6 alkoxy)aminocarbonyl group, a group of formula R⁹-CO-(CH₂)ₖ-N(R¹⁰)- {wherein R⁹ represents a hydroxyl group, an amino group, a C1-C6 alkyl group, a C1-C6 alkylamino group, a di(C1-C6 alkyl)amino group, a hydroxyamino group, a C1-C6 alkoxyamino group or a C1-C6 alkoxy group; R¹⁰ represents a hydrogen atom or a C1-C6 alkyl group; and k represents an integer of from 0 to 5} and a sulfamoyl C1-C6 alkyl group], pharmacologically acceptable salts thereof or prodrugs thereof.

A compound having the general formula (I) shown above, a pharmacologically acceptable salt thereof or a prodrug thereof is preferably
(1) a compound wherein R¹ represents a C1-C6 alkyl group, a halogeno C1-C6 alkyl group, a C3-C6 cycloalkyl group, a halogeno C3-C6 cycloalkyl group or a C1-C6 alkoxy group, pharmacologically acceptable salts thereof or prodrugs thereof;
(2) a compound wherein R¹ represents a C3-C6 cycloalkyl group, a halogeno C3-C6 cycloalkyl group or a C1-C6 alkoxy group, pharmacologically acceptable salts thereof or prodrugs thereof;
(3) a compound wherein R¹ represents a C3-C6 cycloalkyl group or a C1-C6 alkoxy group, pharmacologically acceptable salts thereof or prodrugs thereof;
(4) a compound wherein R¹ represents a cyclopropyl group or a methoxy group, pharmacologically acceptable salts thereof or prodrugs thereof;
(5) a compound wherein R¹ represents a cyclopropyl group, pharmacologically acceptable salts thereof or prodrugs thereof;
(6) a compound wherein R² represents a hydrogen atom or a C1-C6 alkyl group, pharmacologically acceptable salts thereof or prodrugs thereof;
(7) a compound wherein R² represents a hydrogen atom or a methyl group, pharmacologically acceptable salts thereof or prodrugs thereof;
(8) a compound wherein R² represents a hydrogen atom, pharmacologically acceptable salts thereof or prodrugs thereof;
(9) a compound wherein R³ represents a substituted C1-C6 alkyl group {said substituent group represents a heterocyclyl group or a heterocyclyl group substituted with 1 or 2 substituents selected from <Substituent group α> (said heterocyclyl groups may be substituted by 1 or 2 oxo groups), a heteroaryl group or a heteroaryl group substituted with 1 or 2 substituents selected from <Substituent group α> or a substituent selected from <Substituent group β>}, or a heterocyclyl group or a heterocyclyl group substituted with 1 or 2 substituents selected from <Substituent group α> (said heterocyclyl groups may be substituted by 1 or 2 oxo groups), pharmacologically acceptable salts thereof or prodrugs thereof;
(10) a compound wherein R³ represents a substituted C1-C6 alkyl group {said substituent group represents a 4- to 7-membered heterocyclyl group containing at least one nitrogen atom which may be substituted with 1 or 2 substituents selected from <Substituent group α1> (said heterocyclyl group may be substituted by one oxo group), a heteroaryl group containing at least one nitrogen atom which may be substituted with 1 or 2 substituents selected from <Substituent group α1>, a carboxy group, a C2-C7 alkoxycarbonyl group, a cyano group, a hydroxyl group, a C1-C6 alkoxy group or a group of formula R⁹-CO-(CH₂)ₖ-N(R¹⁰)- (wherein R⁹ represents a hydroxyl group or a C1-C6 alkoxy group; R¹⁰ represents a C1-C6 alkyl group; and k represents an integer of from 1 to 5)}, or a 4- to 7-membered heterocyclyl group containing at least one nitrogen atom which may be substituted with one substituent selected from <Substituent group α1> (said heterocyclyl group may be substituted by one oxo group), and
   <Substituent group α1> is a group consisting of a carboxy group, a C2-C7 alkoxycarbonyl group and a group of formula R⁴-CO-CR⁵R⁶-(CH₂)ₘ- (wherein R⁴ represents a hydroxyl group, an amino group, a C1-C6 alkylamino group, a di(C1-C6 alkyl)amino group, a hydroxyamino group, a C1-C6 alkoxyamino group or a C1-C6 alkoxy group; R⁵ and R⁶ each represents a hydrogen atom; and m represents an integer of from 0 to 5), pharmacologically acceptable salts thereof or prodrugs thereof;
(11) a compound wherein R³ represents a substituted C1-C3 alkyl group {said substituent group represents a pyrrolidinyl, piperidinyl, morpholinyl, thiomorpholinyl, piperazinyl, pyrazolyl, triazolyl or tetrazolyl group which may be substituted with 1 or 2 substituents selected from <Substituent group α2> (said pyrrolidinyl, piperidinyl, morpholinyl, thiomorpholinyl or piperazinyl group may be substituted by one oxo group), a carboxy group, a C2-C4 alkoxycarbonyl group, a hydroxyl group or a group of formula R⁹-CO-(CH₂)ₖ-N(R¹⁰)- (wherein R⁹ represents a hydroxyl group or a C1-C3 alkoxy group; R¹⁰ represents a C1-C3 alkyl group; and k represents an integer of from 1 to 3)}, or a pyrrolidinyl, piperidinyl, morpholinyl, thiomorpholinyl or piperazinyl group which may be substituted with one substituent selected from <Substituent group α2> (said pyrrolidinyl, piperidinyl, morpholinyl, thiomorpholinyl or piperazinyl group may be substituted by one oxo group), and
   <Substituent group α2> is a group consisting of a carboxy group, a C2-C4 alkoxycarbonyl group and a group of formula R⁴-CO-CR⁵R⁶-(CH₂)ₘ- (wherein R⁴ represents a hydroxyl group or a C1-C3 alkoxy group, R⁵ and R⁶ each represents a hydrogen atom, and m represents an integer of from 0 to 2), pharmacologically acceptable salts thereof,or prodrugs thereof;
(12) a compound wherein R³ represents a substituted methyl or ethyl group {said substituent group represents a pyrrolidinyl, piperidinyl, piperazinyl or pyrazolyl group which may be substituted with 1 or 2 substituents selected from <Substituent group α3> (said pyrrolidinyl, piperidinyl or piperazinyl group may be substituted by one oxo group), a carboxy group or a group of formula R⁹-CO-(CH₂)ₖ-N(R¹⁰)- (wherein R⁹ represents a hydroxyl group, a methoxy group or an ethoxy group; R¹⁰ represents a methyl group, an ethyl group or an isopropyl group; and k represents an integer of from 1 to 3), and
   <Substituent group α3> is a group consisting of a carboxy group, a methoxycarbonyl group, an ethoxycarbonyl group, a carboxymethyl group, a methoxycarbonylmethyl group, an ethoxycarbonylmethyl group, a 2-(carboxy)ethyl group, a 2-(methoxycarbonyl)ethyl group and a 2-(ethoxycarbonyl)ethyl group, pharmacologically acceptable salts thereof or prodrugs thereof;
(13) a compound wherein X¹, X², X³, X⁴ and X⁵ represent independently a hydrogen atom or a halogen atom, pharmacologically acceptable salts thereof or prodrugs thereof;
(14) a compound wherein X¹ and X² represent independently a hydrogen atom or a halogen atom; and X³, X⁴ and X⁵ represent a hydrogen atom, pharmacologically acceptable salts thereof or prodrugs thereof;
(15) a compound wherein X¹ represents a halogen atom; and X², X³, X⁴ and X⁵ represent a hydrogen atom, pharmacologically acceptable salts thereof or prodrugs thereof;
(16) a compound wherein X¹ represents a fluorine atom; and X², X³, X⁴ and X⁵ represent a hydrogen atom, pharmacologically acceptable salts thereof or prodrugs thereof;
(17) a compound wherein n represents 0 or 1, pharmacologically acceptable salts thereof or prodrugs thereof; and
(18) a compound wherein n represents 1, pharmacologically acceptable salts thereof or prodrugs thereof.
   Further, in each group of (1) - (5), (6) - (8), (9) - (12), (13) - (16) and (17) - (18) described above, a more preferable compound is shown as the number increases [the same concept is applied to each group of (19) - (22) described below]. A compound obtained by selecting R¹ from each group of (1) - (5); R² from each group of (6) - (8); R³ from each group of (9) - (12); X¹, X², X³, X⁴ and X⁵ from each group of (13) - (16); and n from each group of (17) - (18), respectively, followed by arbitrarily combining these selected groups is also preferable, and can be, for example, the following:
(19) a compound wherein R¹ represents a C3-C6 cycloalkyl group or a C1-C6 alkoxy group;
   R² represents a hydrogen atom or a C1-C6 alkyl group;
   R³ represents a substituted C1-C6 alkyl group {said substituent group represents a heterocyclyl group or a heterocyclyl group substituted with 1 or 2 substituents selected from <Substituent group α> (said heterocyclyl groups may be substituted by 1 or 2 oxo groups), a heteroaryl group or a heteroaryl group substituted with 1 or 2 substituents selected from <Substituent group α>, or a substituent selected from <Substituent group β>}, or a heterocyclyl group or a heterocyclyl group substituted with 1 or 2 substituents selected from <Substituent group α> (said heterocyclyl groups may be substituted by 1 or 2 oxo groups);
   X¹ and X² represent independently a hydrogen atom or a halogen atom;
   X³, X⁴, and X⁵ represent a hydrogen atom; and
   n is 0 or 1, a pharmacologically acceptable salt thereof, or a prodrug thereof;
(20) a compound wherein R¹ represents a cyclopropyl group or a methoxy group;
   R² represents a hydrogen atom or a methyl group;
   R³ represents a substituted C1-C6 alkyl group {said substituent group represents a 4- to 7-membered heterocyclyl group containing at least one nitrogen atom which may be substituted with 1 or 2 substituents selected from <Substituent group α1> (said heterocyclyl group may be substituted by one oxo group), a heteroaryl group containing at least one nitrogen atom which may be substituted with 1 or 2 substituents selected from <Substituent group α1>, a carboxy group, a C2-C7 alkoxycarbonyl group, a cyano group, a hydroxyl group, a C1-C6 alkoxy group or a group of formula R⁹-CO-(CH₂)ₖ-N(R¹⁰)- (wherein R⁹ represents a hydroxyl group or a C1-C6 alkoxy group; R¹⁰ represents a C1-C6 alkyl group; and k represents an integer of from 1 to 5)}, or a 4- to 7-membered heterocyclyl group containing at least one nitrogen atom which may be substituted with one substituent selected from <Substituent group α1> (said heterocyclyl group may be substituted by one oxo group), and
   <Substituent group α1> is a group consisting of a carboxy group, a C2-C7 alkoxycarbonyl group, and a group of formula R⁴-CO-CR⁵R⁶-(CH₂)ₘ- (wherein R⁴ represents a hydroxyl group, an amino group, a C1-C6 alkylamino group, a di(C1-C6 alkyl)amino group, a hydroxyamino group, a C1-C6 alkoxyamino group or a C1-C6 alkoxy group; R⁵ and R⁶ each represents a hydrogen atom; and m represents an integer of from 0 to 5);
   X¹ represents a halogen atom;
   X², X³, X⁴ and X⁵ represent a hydrogen atom; and
   n represents 1, pharmacologically acceptable salts thereof or prodrugs thereof;
(21) a compound wherein R¹ represents a cyclopropyl group;
   R² represents a hydrogen atom;
   R³ represents a substituted C1-C3 alkyl group {said substituent group represents a pyrrolidinyl, piperidinyl, morpholinyl, thiomorpholinyl, piperazinyl, pyrazolyl, triazolyl or tetrazolyl group which may be substituted with 1 or 2 substituents selected from <Substituent group α2> (said pyrrolidinyl, piperidinyl, morpholinyl, thiomorpholinyl or piperazinyl group may be substituted by one oxo group), a carboxy group, a C2-C4 alkoxycarbonyl group, a hydroxyl group or a group of formula R⁹-CO-(CH₂)ₖ-N(R¹⁰)- (wherein R⁹ represents a hydroxyl group or a C1-C3 alkoxy group; R¹⁰ represents a C1-C3 alkyl group; and k represents an integer of from 1 to 3)}, or a pyrrolidinyl, piperidinyl, morpholinyl, thiomorpholinyl or piperazinyl group which may be substituted with one substituent selected from <Substituent group α2> (said pyrrolidinyl, piperidinyl, morpholinyl, thiomorpholinyl, or piperazinyl group may be substituted by one oxo group), and
   <Substituent group α2> is a group consisting of a carboxy group, a C2-C4 alkoxycarbonyl group, and a group of formula R⁴-CO-CR⁵R⁶-(CH₂)ₘ- (wherein R⁴ represents a hydroxyl group or a C1-C3 alkoxy group, R⁵ and R⁶ each represents a hydrogen atom, and m represents an integer of from 0 to 2);
   X¹ represents a fluorine atom;
   X², X³, X⁴ and X⁵ represent a hydrogen atom; and
   n represents 1, pharmacologically acceptable salts thereof or prodrugs thereof, and
(22) a compound wherein R¹ represents a cyclopropyl group;
   R² represents a hydrogen atom;
   R³ represents a substituted methyl or ethyl group {said substituent group represents a pyrrolidinyl, piperidinyl, piperazinyl or pyrazolyl group which may be substituted with 1 or 2 substituents selected from <Substituent group α3> (said pyrrolidinyl, piperidinyl or piperazinyl group may be substituted by one oxo group), a carboxy group or a group of formula R⁹-CO-(CH₂)ₖ-N(R¹⁰)- (wherein R⁹ represents a hydroxyl group, a methoxy group or an ethoxy group; R¹⁰ represents a methyl group, an ethyl group or an isopropyl group; and k represents an integer of from 1 to 3), and
   <Substituent group α3> is a group consisting of a carboxy group, a methoxycarbonyl group, an ethoxycarbonyl group, a carboxymethyl group, a methoxycarbonylmethyl group, an ethoxycarbonylmethyl group, a 2-(carboxy)ethyl group, a 2-(methoxycarbonyl)ethyl group and a 2-(ethoxycarbonyl)ethyl group;
   X¹ represents a fluorine atom;
   X², X³, X⁴ and X⁵ represent a hydrogen atom; and
   n represents 1, pharmacologically acceptable salts thereof or prodrugs thereof.

Furthermore, another aspect of the present invention relates to a medicament containing the compound, pharmacologically acceptable salts thereof or prodrugs thereof described in (1) - (22) above (preferably an antithrombotic agent); use of the compound, pharmacologically acceptable salts thereof or prodrugs thereof to manufacture pharmaceutical compositions; use of the compound, pharmacologically acceptable salts thereof or prodrugs thereof for prophylactic or therapeutic agents for diseases related to thrombus or embolus formation; and prophylactic or therapeutic methods for diseases (particularly diseases related to thrombus or embolus formation) by administration of pharmacologically effective dose of the compound, pharmacologically acceptable salts thereof or prodrugs thereof to a warm-blooded animals (especially humans).

The "C1-C6 alkyl group" in the general formula (I) shown above can be, for example, a straight or branched chain alkyl group having from 1 to 6 carbon atoms such as a methyl, ethyl, propyl, isopropyl, butyl, isobutyl, s-butyl, t-butyl, pentyl, isopentyl, 2-methylbutyl, neopentyl, 1-ethylpropyl, hexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl or 2-ethylbutyl group, and is preferably a straight or branched chain alkyl group having from 1 to 4 carbon atoms, more preferably a straight or branched chain alkyl group having from 1 to 3 carbon atoms, more preferably a methyl or ethyl group, and most preferably a methyl group.

The "halogen atom" in the general formula (I) shown above can be, for example, a fluorine atom, a chlorine atom, a bromine atom or an iodine atom, and is preferably a fluorine atom or a chlorine atom, and more preferably a fluorine atom.

The "C1-C6 alkoxy group" in the general formula (I) shown above indicates a group wherein said "C1-C6 alkyl group" is bonded to an oxygen atom, and can be, for example, a straight or branched chain alkoxy group having from 1 to 6 carbon atoms such as a methoxy, ethoxy, n-propoxy, isopropoxy, n-butoxy, isobutoxy, s-butoxy, t-butoxy, n-pentyloxy, isopentyloxy, 2-methylbutoxy, neopentyloxy, n-hexyloxy, 4-methylpentyloxy, 3-methylpentyloxy, 2-methylpentyloxy, 3,3-dimethylbutoxy, 2,2-dimethylbutoxy, 1,1-dimethylbutoxy, 1,2-dimethylbutoxy, 1,3-dimethylbutoxy or 2,3-dimethylbutoxy group, and is preferably a straight or branched chain alkoxy group having from 1 to 4 carbon atoms, more preferably a straight or branched chain alkoxy group having from 1 to 3 carbon atoms, and most preferably a methoxy or ethoxy group.

The "C3-C6 cycloalkyl group" in general formula (I) shown above can be, for example, a 3- to 6-membered saturated cyclic hydrocarbon group such as a cyclopropyl, cyclobutyl, cyclopentyl or cyclohexyl group, and is preferably a cyclopropyl group.

The "C6-C10 aryl group" in general formula (I) shown above can be, for example, an aromatic hydrocarbon group having from 6 to 10 carbon atoms such as phenyl or naphthyl group, and is preferably a phenyl group.

The "C2-C7 alkoxycarbonyl group" in general formula (I) shown above can be, for example, a straight or branched chain alkoxycarbonyl group having from 2 to 7 carbon atoms such as a methoxycarbonyl, ethoxycarbonyl, n-propoxycarbonyl, isopropoxycarbonyl, n-butoxycarbonyl, isobutoxycarbonyl, s-butoxycarbonyl, t-butoxycarbonyl, n-pentyloxycarbonyl, isopentyloxycarbonyl, 2-methylbutoxycarbonyl, neopentyloxycarbonyl, n-hexyloxycarbonyl, 4-methylpentyloxycarbonyl, 3-methylpentyloxycarbonyl, 2-methylpentyloxycarbonyl, 3,3-dimethylbutoxycarbonyl, 2,2-dimethylbutoxycarbonyl, 1,1-dimethylbutoxycarbonyl, 1,2-dimethylbutoxycarbonyl, 1,3-dimethylbutoxycarbonyl or 2,3-dimethylbutoxycarbonyl group, and is preferably a straight or branched chain alkoxycarbonyl group having from 2 to 5 carbon atoms, more preferably a straight or branched chain alkoxycarbonyl group having from 2 to 4 carbon atoms, and further more preferably a methoxycarbonyl or ethoxycarbonyl group.

The "halogeno C1-C6 alkyl group" in general formula (I) shown above indicates a group wherein said "C1-C6 alkyl group" is substituted with halogen atom(s), and can be, for example, a trifluoromethyl, trichloromethyl, difluoromethyl, dichloromethyl, dibromomethyl, fluoromethyl, 2,2,2-trifluoroethyl, 2,2,2-trichloroethyl, 2-bromoethyl, 2-chloroethyl, 2-fluoroethyl, 2-iodoethyl, 3-chloropropyl, 4-fluorobutyl, 6-iodohexyl or 2,2-dibromoethyl group, and is preferably a trifluoromethyl group.

The "heteroaryl group" in general formula (I) shown above can be, for example, a 5- to 7-membered aromatic heterocyclic group containing from 1 to 4 sulfur atom(s), oxygen atom(s) and/or nitrogen atom(s) such as a furyl, thienyl, pyrrolyl, pyrazolyl, imidazolyl, oxazolyl, isoxazolyl, thiazolyl, isothiazolyl, triazolyl, tetrazolyl, thiadiazolyl, oxadiazolyl, pyranyl, pyridyl, pyridazinyl, pyrimidinyl or pyrazinyl group, or an aromatic heterocyclic group fused with other cyclic groups such as an isoindolyl, indolyl, isoquinolyl and quinolyl group, and is preferably a 5-to 7-membered aromatic heterocyclic group containing at least one nitrogen atom, and more preferably a pyrazolyl, triazolyl or tetrazolyl group.

The "halogeno C1-C6 alkoxy group" in general formula (I) shown above indicates a group wherein said "C1-C6 alkoxy group" is substituted with halogen atom(s), and can be, for example, a trifluoromethoxy, trichloromethoxy, difluoromethoxy, dichloromethoxy, dibromomethoxy, fluoromethoxy, 2,2,2-trichloroethoxy, 2,2,2-trifluoroethoxy, 2-bromoethoxy, 2-chloroethoxy, 2-fluoroethoxy or 2,2-dibromoethoxy group, and is preferably a 2-bromoethoxy, 2-chloroethoxy or 2-fluoroethoxy group.

The "hydroxy C1-C6 alkyl group" in general formula (I) shown above indicates a group wherein said "C1-C6 alkyl group" is substituted with hydroxyl group(s), and can be, for example, a hydroxymethyl, 1-hydroxyethyl, 2-hydroxyethyl, 1-hydroxy-2-methylethyl, 2-hydroxy-2-methylethyl, 1-hydroxypropyl, 2-hydroxypropyl, 3-hydroxypropyl, 1-hydroxybutyl, 2-hydroxybutyl, 3-hydroxybutyl, 4-hydroxybutyl, 5-hydroxypentyl or 6-hydroxyhexyl group, and is preferably a hydroxymethyl, 2-hydroxyethyl or 3-hydroxypropyl group.

The "C2-C12 alkoxyalkyl group" in general formula (I) shown above indicates a group wherein said "C1-C6 alkoxy group" is bonded to said "C1-C6 alkyl group", and can be, for example, a methoxymethyl, ethoxymethyl, n-propoxymethyl, isopropoxymethyl, n-butoxymethyl, isobutoxymethyl, s-butoxymethyl, t-butoxymethyl, n-pentyloxymethyl, isopentyloxymethyl, 2-methylbutoxymethyl, neopentyloxymethyl, n-hexyloxymethyl, 4-methylpentyloxymethyl, 3-methylpentyloxymethyl, 2-methylpentyloxymethyl, 3,3-dimethylbutoxymethyl, 2,2-dimethylbutoxymethyl, 1,1-dimethylbutoxymethyl, 1,2-dimethylbutoxymethyl, 1,3-dimethylbutoxymethyl, 2,3-dimethylbutoxymethyl, 2-methoxyethyl, 2-ethoxyethyl, 2-(n-propoxy)ethyl, 2-(isopropoxy)ethyl, 2-(n-butoxy)ethyl, 2-(isobutoxy)ethyl, 2-(s-butoxy)ethyl, 2-(t-butoxy)ethyl, 2-(n-pentyloxy)ethyl, 2-(isopentyloxy)ethyl, 2-(2-methylbutoxy)ethyl, 2-(neopentyloxy)ethyl, 2-(n-hexyloxy)ethyl, 2-(4-methylpentyloxy)ethyl, 2-(3-methylpentyloxy)ethyl, 2-(2-methylpentyloxy)ethyl, 2-(3,3-dimethylbutoxy)ethyl, 2,2-dimethylbutoxyethyl, 1,1-dimethylbutoxyethyl, 1,2-dimethylbutoxyethyl, 1,3-dimethylbutoxyethyl or 2,3-dimethylbutoxyethyl group, and is preferably a straight or branched chain alkoxyalkyl group having from 2 to 4 carbon atoms, and more preferably a methoxymethyl or methoxyethyl group.

The "C2-C7 alkanoyl group" in the general formula (I) shown above can be, for example, a straight or branched chain alkanoyl group having from 2 to 7 carbon atoms such as an acetyl, propionyl, butyryl, isobutyryl, pentanoyl, pivaloyl, valeryl, isovaleryl, hexanoyl or heptanoyl group, and is preferably a straight or branched chain alkanoyl group having from 2 to 5 carbon atoms, more preferably a straight or branched chain alkanoyl group having from 2 to 4 carbon atoms, and further more preferably an acetyl group.

The "C4-C7 cycloalkylcarbonyl group" in general formula (I) shown above indicates a group wherein said "C3-C6 cycloalkyl group" is bonded to a carbonyl group, and can be, for example, a cyclopropylcarbonyl, cyclobutylcarbonyl, cyclopentylcarbonyl or cyclohexylcarbonyl group, and is preferably a cyclopropylcarbonyl group.

The "C2-C7 alkylcarbamoyl group" in general formula (I) shown above indicates a group wherein one "C1-C6 alkyl group" is bonded to a carbamoyl group, and can be, for example, a methylcarbamoyl, ethylcarbamoyl, propylcarbamoyl, isopropylcarbamoyl, butylcarbamoyl, isobutylcarbamoyl, s-butylcarbamoyl, t-butylcarbamoyl, pentylcarbamoyl, isopentylcarbamoyl, 2-methylbutylcarbamoyl, neopentylcarbamoyl, 1-ethylpropylcarbamoyl, hexylcarbamoyl, 4-methylpentylcarbamoyl, 3-methylpentylcarbamoyl, 2-methylpentylcarbamoyl, 1-methylpentylcarbamoyl, 3,3-dimethylbutylcarbamoyl, 2,2-dimethylbutylcarbamoyl, 1,1-dimethylbutylcarbamoyl, 1,2-dimethylbutylcarbamoyl, 1,3-dimethylbutylcarbamoyl, 2,3-dimethylbutylcarbamoyl or 2-ethylbutylcarbamoyl group, and is preferably an alkylcarbamoyl group having from 2 to 5 carbon atoms, and more preferably a methylcarbamoyl or ethylcarbamoyl group.

The "di(C1-C6 alkyl)carbamoyl group" in general formula (I) shown above indicates a group wherein a carbamoyl group is disubstituted with two "C1-C6 alkyl groups", and can be, for example, a dimethylcarbamoyl, methylethylcarbamoyl, diethylcarbamoyl, di-n-propylcarbamoyl, diisopropylcarbamoyl, N-(n-propyl)-N-ethylcarbamoyl, di-n-butylcarbamoyl, diisobutylcarbamoyl, di-s-butylcarbamoyl, dit-butylcarbamoyl, di-n-pentylcarbamoyl, diisopentylcarbamoyl, di-2-methylbutylcarbamoyl, dineopentylcarbamoyl, di-1-ethylpropylcarbamoyl, di-n-hexylcarbamoyl, di-4-methylpentylcarbamoyl, di-3-methylpentylcarbamoyl, di-2-methylpentylcarbamoyl, di-1-methylpentylcarbamoyl, di-3,3-dimethylbutylcarbamoyl, di-2,2-dimethylbutylcarbamoyl, di-1,1-dimethylbutylcarbamoyl, di-1,2-dimethylbutylcarbamoyl, di-1,3-dimethylbutylcarbamoyl, di-2,3-dimethylbutylcarbamoyl or di-2-ethylbutylcarbamoyl group, and is preferably a dimethylcarbamoyl, methylethylcarbamoyl or diethylcarbamoyl group.

The "C1-C6 alkylamino group" in general formula (I) shown above indicates a group wherein one "C1-C6 alkyl group" is bonded to an amino group, and can be, for example, a straight or branched chain alkylamino group having from 1 to 6 carbon atoms such as methylamino, ethylamino, propylamino, isopropylamino, butylamino, isobutylamino, s-butylamino, t-butylamino, pentylamino, isopentylamino, 2-methylbutylamino, neopentylamino, 1-ethylpropylamino, hexylamino, 4-methylpentylamino, 3-methylpentylamino, 2-methylperitylamino, 1-methylpentylamino, 3,3-dimethylbutylamino, 2,2-dimethylbutylamino, 1,1-dimethylbutylamino, 1,2-dimethylbutylamino, 1,3-dimethylbutylamino, 2,3-dimethylbutylamino or 2-ethylbutylamino group, and is preferably a straight or branched chain alkylamino group having from 1 to 4 carbon atoms, more preferably a straight or branched chain alkylamino group having from 1 to 3 carbon atoms, and further more preferably a methylamino group.

The "di(C1-C6 alkyl)amino group" in general formula (I) shown above indicates a group wherein an amino group is substituted with two "C1-C6 alkyl groups", and can be, for example, a dimethylamino, methylethylamino, diethylamino, di-n-propylamino, diisopropylamino, N-(n-propyl)-N-ethylamino, di-n-butylamino, diisobutylamino, di-s-butylamino, di-t-butylamino, di-n-pentylamino, diisopentylamino, di-2-methylbutylamino, dineopentylamino, di-1-ethylpropylamino, di-n-hexylamino, di-4-methylpentylamino, di-3-methylpentylamino, di-2-methylpentylamino, di-1-methylpentylamino, di-3,3-dimethylbutylamino, di-2,2-dimethylbutylamino, di-1,1-dimethylbutylamino, di-1,2-dimethylbutylamino, di-1,3-dimethylbutylamino, di-2,3-dimethylbutylamino or di-2-ethylbutylamino group, and is preferably a dimethylamino, methylethylamino or diethylamino group.

The "C1-C6 alkoxyamino group" in general formula (I) shown above indicates a group wherein the oxygen atom of a hydroxyamino group is substituted with said "C1-C6 alkyl group", and can be, for example, a methoxyamino, ethoxyamino, n-propoxyamino, isopropoxyamino, n-butoxyamino, isobutoxyamino, s-butoxyamino, t-butoxyamino, n-pentyloxyamino, isopentyloxyamino, 2-methylbutoxyamino, neopentyloxyamino, n-hexyloxyamino, 4-methylpentyloxyamino, 3-methylpentyloxyamino, 2-methylpentyloxyamino, 3,3-dimethylbutoxyamino, 2,2-dimethylbutoxyamino, 1,1-dimethylbutoxyamino, 1,2-dimethylbutoxyamino, 1,3-dimethylbutoxyamino or 2,3-dimethylbutoxyamino group, and is preferably a straight or branched chain alkoxyamino group having from 1 to 4 carbon atoms, more preferably a straight or branched chain alkoxyamino group having from 1 to 3 carbon atoms, and further more preferably a methoxyamino group.

The "sulfamoyl C1-C6 alkyl group" in general formula (I) shown above indicates a group wherein said "C1-C6 alkyl group" is substituted with sulfamoyl group(s), and can be, for example, a sulfamoylmethyl, 1-sulfamoylethyl, 2-sulfamoylethyl, 1-sulfamoyl-2-methylethyl, 2-sulfamoyl-2-methylethyl, 1-sulfamoylpropyl, 2-sulfamoylpropyl, 3-sulfamoylpropyl, 1-sulfamoylbutyl, 2-sulfamoylbutyl, 3-sulfamoylbutyl, 4-sulfamoylbutyl, 5-sulfamoylpentyl or 6-sulfamoylhexyl group, and is preferably a sulfamoylalkyl group having from 1 to 4 carbon atoms, more preferably a sulfamoylalkyl group having from 1 to 3 carbon atoms, and further more preferably a sulfamoylmethyl, 2-sulfamoylethyl or 3-sulfamoylpropyl group.

The "heterocyclyl group" in general formula (I) shown above can be, for example, a partially or completely reduced 4- to 7-membered heterocyclic group containing from 1 to 3 sulfur atom(s), oxygen atom(s) and/or nitrogen atom(s) such as a morpholinyl, thiomorpholinyl, azetidinyl, pyrrolidinyl, pyrrolinyl, imidazolidinyl, imidazolinyl, pyrrazolidinyl, pyrrazolinyl, piperidyl, piperazinyl, homopiperazinyl or homopiperidinyl group, and is preferably a 4- to 7-membered heterocyclyl group containing at least one nitrogen atom, more preferably a morpholinyl, thiomorpholinyl, pyrrolidinyl, pyrrolinyl, imidazolidinyl, imidazolinyl, pyrrazolidinyl, pyrrazolinyl, piperidyl or piperazinyl group, and more preferably a pyrrolidinyl, piperidinyl, morpholinyl, thiomorpholinyl or piperazinyl group.

The "heterocyclyl group (said heterocyclyl group may be substituted by from 1 to 4 oxo groups)" in general formula (I) shown above can be oxopiperazinyl or dioxopiperazinyl group, in addition to said "heterocyclyl group"; and is preferably a 2-oxo-1-piperazino, 3-oxo-1-piperazino or 2,5-dioxo-1-piperazino group; and more preferably a 2-oxo-1-piperazino or 3-oxo-1-piperazino group.

The Substituent group α in general formula (I) shown above, is preferably a group consisting of a carboxy group, a C2-C7 alkoxycarbonyl group, and a group of formula R⁴-CO-CR⁵R⁶-(CH₂)ₘ- (wherein R⁴ represents a hydroxyl group, an amino group, a C1-C6 alkylamino group, a di(C1-C6 alkyl)amino group, a hydroxyamino group, a C1-C6 alkoxyamino group or a C1-C6 alkoxy group; R⁵ and R⁶ each represents a hydrogen atom; and m represents an integer of from 0 to 5), more preferably a group consisting of a carboxy group, a C2-C4 alkoxycarbonyl group and a group of formula R⁴-CO-CR⁵R⁶-(CH₂)ₘ- (wherein R⁴ represents a hydroxyl group or a C1-C3 alkoxy group; R⁵ and R⁶ each represents a hydrogen atom; and m represents an integer of from 0 to 2), and further more preferably a group consisting of a carboxy group, a methoxycarbonyl group, an ethoxycarbonyl group, a carboxymethyl group, a methoxycarbonylmethyl group, an ethoxycarbonylmethyl group, a 2-(carboxy)ethyl group, a 2-(methoxycarbonyl)ethyl group and a 2-(ethoxycarbonyl)ethyl group.

The Substituent group β in general formula (I) shown above is preferably a group consisting of a carboxy group, a C2-C7 alkoxycarbonyl group, a cyano group, a hydroxyl group, a C1-C6 alkoxy group and a group of formula R⁹-CO-(CH₂)ₖ-N(R¹⁰)- (wherein R⁹ represents a hydroxyl group or a C1-C6 alkoxy group; R¹⁰ represents a C1-C6 alkyl group; and k represents an integer of from 1 to 5), more preferably a group consisting of a carboxy group, a C2-C4 alkoxycarbonyl group, a hydroxyl group and a group of formula R⁹-CO-(CH₂)ₖ-N(R¹⁰)- (wherein R⁹ represents a hydroxyl group or a C1-C3 alkoxy group; R¹⁰ represents a C1-C3 alkyl group; and k represents an integer of from 1 to 3), and more preferably a group consisting of a carboxy group and a group of formula R⁹-CO-(CH₂)ₖ-N(R¹⁰)-(wherein R⁹ represents a hydroxyl group, a methoxy group or an ethoxy group; R¹⁰ represents a methyl group, an ethyl group or an isopropyl group; and k represents an integer of from 1 to 3).

In the compounds (I) of the present invention, optical isomers due to asymmetric carbon atoms in their structures (including diastereomers) may be present, and additionally, geometrical isomers due to the carbon-carbon double bond may be also present in the same compound. The present invention encompasses all of these isomers.

As some compounds (I) of the present invention have various groups such as a sulfanyl group, a carboxy group, a hydroxyl group or an amino group in their structure, the "prodrug thereof' means a derivative in which any of such groups is modified by an appropriate functional group that can be cleaved by a biological process such as hydrolysis in vivo. In these cases, it can be determined whether the derivative is "an appropriate functional group that can be cleaved by a biological process such as hydrolysis in vivo" or not according to whether the original compound or a pharmacologically acceptable salt thereof can be detected, by administering the derivative to an experimental animal such as a rat or a mouse by an intravenous injection, subcutaneous injection or oral administration, and measuring a body fluid of the animal thereafter.

When the compounds (I) of the present invention contain a sulfanyl group in their structures, the functional group employed for forming a prodrug thereof is not particularly restricted, and can be, for example, an "aliphatic acyl group", including an alkanoyl group such as a formyl, acetyl, propionyl, butyryl, isobutyryl, pentanoyl, pivaloyl, valeryl, isovaleryl, octanoyl, nonanoyl, decanoyl, 3-methylnonanoyl, 8-methylnonanoyl, 3-ethyloctanoyl, 3,7-dimethyloctanoyl, undecanoyl, dodecanoyl, tridecanoyl, tetradecanoyl, pentadecanoyl, hexadecanoyl, 1-methylpentadecanoyl, 14-methylpentadecanoyl, 13,13-dimethyltetradecanoyl, heptadecanoyl, 15-methylhexadecanoyl, octadecanoyl, 1-methylheptadecanoyl, nonadecanoyl, icosanoyl or henicosanoyl group; an alkylcarbonyl group substituted with a carboxy group such as a succinoyl, glutaroyl or adipoyl group; a carbonyl group substituted with a halogeno lower alkyl group such as a chloroacetyl, dichloroacetyl, trichloroacetyl or trifluoroacetyl group; a saturated cyclic hydrocarbon-carbonyl group such as a cyclopropylcarbonyl, cyclobutylcarbonyl, cyclopentylcarbonyl, cyclohexylcarbonyl, cycloheptylcarbonyl or cyclooctylcarbonyl group; an alkylcarbonyl group substituted with lower alkoxy group(s) such as a methoxyacetyl group; and an unsaturated alkylcarbonyl group such as a (E)-2-methyl-2-butenoyl group (preferably C1-C6 alkanoyl group); a "carbonyloxyalkyl group", including an oxodioxolenylmethyl group such as a (5-methyl-2-oxo-1,3-dioxolen-4-yl)methyl or a (5-phenyl-2-oxo-1,3-dioxolen-4-yl)methyl group; an "aromatic acyl group", including an arylcarbonyl group such as a benzoyl, α-naphthoyl, β-naphthoyl, pyridoyl, thienoyl or furoyl group; a halogeno arylcarbonyl group such as a 2-bromobenzoyl or 4-chlorobenzoyl group; an arylcarbonyl group substituted with lower alkyl group(s) such as a 2,4,6-trimethylbenzoyl or 4-toluoyl group; a lower alkoxylated arylcarbonyl group such as a 4-anisoyl group; an arylcarbonyl group substituted with carboxy group(s) such as a 2-carboxybenzoyl, 3-carboxybenzoyl or 4-carboxybenzoyl group; a nitrated arylcarbonyl group such as a 4-nitrobenzoyl or 2-nitrobenzoyl group; an arylcarbonyl group substituted with lower alkoxycarbonyl group(s) such as a 2-(methoxycarbonyl)benzoyl group; and an arylcarbonyl group substituted with aryl group(s) such as a 4-phenylbenzoyl group (preferably an arylcarbonyl group); an "aralkylcarbonyl group", including a carbonyl group substituted with a lower alkyl group which is substituted with from 1 to 3 aryl groups such as a phenylacetyl, α-naphthylpropionyl, β-naphthylbutyryl, diphenylisobutyryl, triphenylacetyl, α-naphthyldiphenylisobutyryl or 9-anthrylpentanoyl group; and a lower alkylcarbonyl group substituted with from 1 to 3 aryl groups, of which an aryl ring is substituted with lower alkyl group(s), lower alkoxy group(s), nitro group(s), halogen atom(s) or cyano group(s), such as a 4-methylphenylacetyl, 2,4,6-trimethylphenylformyl, 3,4,5-trimethylphenylbutyryl, 4-methoxyphenylisobutyryl, 4-methoxyphenyldiphenylpivaloyl, 2-nitrophenylacetyl, 4-nitrophenylpropionyl, 4-chlorophenylbutyryl, 4-bromophenylacetyl or 4-cyanophenylpentanoyl group; a "tetrahydropyranyl or tetrahydrothiopyranyl group" such as a tetrahydropyran-2-yl, 3-bromotetrahydropyran-2-yl, 4-methoxytetrahydropyran-4-yl, tetrahydrothiopyran-2-yl or 4-methoxytetrahydrothiopyran-4-yl group; a "tetrahydrofuranyl or tetrahydrothiofuranyl group" such as a tetrahydrofuran-2-yl or tetrahydrothiofuran-2-yl group; an "alkoxymethyl group", including a lower alkoxymethyl group such as a methoxymethyl, 1,1-dimethyl-1-methoxymethyl, ethoxymethyl, propoxymethyl, isopropoxymethyl, butoxymethyl or t-butoxymethyl group; a lower alkoxymethyl group substituted with lower alkoxy group(s) such as a 2-methoxyethoxymethyl group; and a halogeno lower alkoxymethyl group such as a 2,2,2-trichloroethoxymethyl or bis(2-chloroethoxy)methyl group; a "substituted ethyl group", including a lower alkoxylated ethyl group such as a 1-ethoxyethyl or 1-(isopropoxy)ethyl group; and a halogenated ethyl group such as a 2,2,2-trichloroethyl group; an "aralkyl group", including a lower alkyl group substituted with from 1 to 3 aryl groups such as a benzyl, α-naphthylmethyl, β-naphthylmethyl, diphenylmethyl, triphenylmethyl, α-naphthyldiphenylmethyl or 9-anthrylmethyl group; and a lower alkyl group substituted with from 1 to 3 aryl groups, of which an aryl ring is substituted with lower alkyl group(s), lower alkoxy group(s), nitro group(s), halogen atom(s) or cyano group(s), such as a 4-methylbenzyl, 2,4,6-trimethylbenzyl, 3,4,5-trimethylbenzyl, 4-methoxybenzyl, 4-methoxyphenyldiphenylmethyl, 2-nitrobenzyl, 4-nitrobenzyl, 4-chlorobenzyl, 4-bromobenzyl or 4-cyanobenzyl group; an "alkoxycarbonyl group", including a lower alkoxycarbonyl group such as a methoxycarbonyl, ethoxycarbonyl, t-butoxycarbonyl or isobutoxycarbonyl group; and a lower alkoxycarbonyl group substituted with halogen atom(s) or tri-lower alkylsilyl group(s) such as a 2,2,2-trichloroethoxycarbonyl or 2-trimethylsilylethoxycarbonyl group; an "alkenyloxycarbonyl group" such as a vinyloxycarbonyl and allyloxycarbonyl group; an "aralkyloxycarbonyl group", of which an aryl ring may be substituted with 1 or 2 substituents selected from lower alkoxy group(s) or nitro group(s), such as a benzyloxycarbonyl, 4-methoxybenzyloxycarbonyl, 3,4-dimethoxybenzyloxycarbonyl, 2-nitrobenzyloxycarbonyl or 4-nitrobenzyloxycarbonyl group; a straight or branched chain alkylsulfanyl group having from 1 to 6 carbon atoms such as a methylsulfanyl, ethylsulfanyl, n-propylsulfanyl, isopropylsulfanyl, n-butylsulfanyl, isobutylsulfanyl, s-butylsulfanyl, t-butylsulfanyl, n-pentylsulfanyl, isopentylsulfanyl, 2-methylbutylsulfanyl, neopentylsulfanyl, 1-ethylpropylsulfanyl, n-hexylsulfanyl, 4-methylpentylsulfanyl, 3-methylpentylsulfanyl, 2-methylpentylsulfanyl, 1-methylpentylsulfanyl, 3,3-dimethylbutylsulfanyl, 2,2-dimethylbutylsulfanyl, 1,1-dimethylbutylsulfanyl, 1,2-dimethylbutylsulfanyl, 1,3-dimethylbutylsulfanyl, 2,3-dimethylbutylsulfanyl or 2-ethylbutylsulfanyl group; or an "aminoacyl group of an α-amino acid" such as a phenylalanine, and is preferably a group which forms a pharmacologically acceptable ester such as an "aliphatic acyl group" or an "aromatic acyl group", or "C1-C6 alkylsulfanyl group" such as a methylsulfanyl, ethylsulfanyl, n-propylsulfanyl, isopropylsulfanyl, n-butylsulfanyl, isobutylsulfanyl, s-butylsulfanyl or t-butylsulfanyl group; more preferably a group which forms a pharmacologically acceptable ester; further more preferably a "C1-C6 alkanoyl group" such as a formyl, acetyl, propionyl, butyryl, isobutyryl, pentanoyl or pivaloyl group or an "arylcarbonyl group" such as a benzoyl group; particularly preferably a "C1-C3 alkanoyl group" or a benzoyl group; and most preferably an acetyl group.

When the compounds (I) of the present invention contain a carboxy group in their structures, the functional group employed for forming a prodrug thereof can be, for example, a "lower alkyl groups" such as a methyl, ethyl, n-propyl, isopropyl, n-butyl, isobutyl, s-butyl, tert-butyl, n-pentyl, isopentyl, 2-methylbutyl, neopentyl, 1-ethylpropyl, n-hexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl or 2-ethylbutyl group (preferably a C1-C6 alkyl group); an "alkoxy lower alkyl group", including a lower alkoxy lower alkyl group such as a methoxymethyl, 1-ethoxyethyl, 1-methyl-1-methoxyethyl, 1-(isopropoxy)ethyl, 2-methoxyethyl, 2-ethoxyethyl, 1,1-dimethyl-1-methoxymethyl, ethoxymethyl, n-propoxymethyl, isopropoxymethyl, n-butoxymethyl or tert-butoxymethyl group; a lower alkoxylated lower alkoxy lower alkyl group such as a 2-methoxyethoxymethyl group; an "aryl"oxy "lower alkyl group" such as a phenoxymethyl group; and a halogenated lower alkoxy lower alkyl group such as a 2,2,2-trichloroethoxymethyl or bis(2-chloroethoxy)methyl group; a "lower alkoxy" carbonyl "lower alkyl group" such as a methoxycarbonylmethyl group; a cyano "lower alkyl group" such as a cyanomethyl or 2-cyanoethyl group; a "lower alkyl" thiomethyl group such as a methylthiomethyl or ethylthiomethyl group; an "aryl" thiomethyl group such as a phenylthiomethyl or naphthylthiomethyl group; a "lower alkyl" sulfonyl "lower alkyl group", which may be substituted with halogen atom(s), such as a 2-methanesulfonylethyl or 2-trifluoromethanesulfonylethyl group; an "aryl" sulfonyl "lower alkyl group" such as a 2-benzenesulfonylethyl or 2-toluenesulfonylethyl group; an acyloxy "lower alkyl group", including an "aliphatic acyl"oxy "lower alkyl group" such as a formyloxymethyl, acetoxymethyl, propionyloxymethyl, butyryloxymethyl, pivaloyloxymethyl, valeryloxymethyl, isovaleryloxymethyl, hexanoyloxymethyl, 1-formyloxyethyl, 1-acetoxyethyl, 1-propionyloxyethyl, 1-butyryloxyethyl, 1-pivaloyloxyethyl, 1-valeryloxyethyl, 1-isovaleryloxyethyl, 1-hexanoyloxyethyl, 2-formyloxyethyl, 2-acetoxyethyl, 2-propionyloxyethyl, 2-butyryloxyethyl, 2-pivaloyloxyethyl, 2-valeryloxyethyl, 2-isovaleryloxyethyl, 2-hexanoyloxyethyl, 1-formyloxypropyl, 1-acetoxypropyl, 1-propionyloxypropyl, 1-butyryloxypropyl, 1-pivaloyloxypropyl, 1-valeryloxypropyl, 1-isovaleryloxypropyl, 1-hexanoyloxypropyl, 1-acetoxybutyl, 1-propionyloxybutyl, 1-butyryloxybutyl, 1-pivaloyloxybutyl, 1-acetoxypentyl, 1-propionyloxypentyl, 1-butyryloxypentyl, 1-pivaloyloxypentyl or 1-pivaloyloxyhexyl group; a "cycloalkyl" carbonyloxy "lower alkyl group" such as a cyclopentanoyloxymethyl, cyclohexanoyloxymethyl, 1-cyclopentanoyloxyethyl, 1-cyclohexanoyloxyethyl, 1-cyclopentanoyloxypropyl, 1-cyclohexanoyloxypropyl, 1-cyclopentanoyloxybutyl or 1-cyclohexanoyloxybutyl group; and an "aromatic acyl"oxy "lower alkyl group" such as a benzoyloxymethyl group; an "(alkoxycarbonyloxy)alkyl group" such as a methoxycarbonyloxymethyl, ethoxycarbonyloxymethyl, propoxycarbonyloxymethyl, isopropoxycarbonyloxymethyl, butoxycarbonyloxymethyl, isobutoxycarbonyloxymethyl, pentyloxycarbonyloxymethyl, hexyloxycarbonyloxymethyl, cyclohexyloxycarbonyloxymethyl, cyclohexyloxycarbonyloxy(cyclohexyl)methyl, 1-(methoxycarbonyloxy)ethyl, 1-(ethoxycarbonyloxy)ethyl, 1-propoxycarbonyloxyethyl, 1-(isopropoxycarbonyloxy)ethyl, 1-butoxycarbonyloxyethyl, 1-isobutoxycarbonyloxyethyl, 1-(tert-butoxycarbonyloxy)ethyl, 1-pentyloxycarbonyloxyethyl, 1-hexyloxycarbonyloxyethyl, 1-cyclopentyloxycarbonyloxyethyl, 1-cyclopentyloxycarbonyloxypropyl, 1-cyclohexyloxycarbonyloxypropyl, 1-cyclopentyloxycarbonyloxybutyl, 1-cyclohexyloxycarbonyloxybutyl, 1-(cyclohexyloxycarbonyloxy)ethyl, 1-(ethoxycarbonyloxy)propyl, 2-methoxycarbonyloxyethyl, 2-ethoxycarbonyloxyethyl, 2-propoxycarbonyloxyethyl, 2-isopropoxycarbonyloxyethyl, 2-butoxycarbonyloxyethyl, 2-isobutoxycarbonyloxyethyl, 2-pentyloxycarbonyloxyethyl, 2-hexyloxycarbonyloxyethyl, 1-methoxycarbonyloxypropyl, 1-ethoxycarbonyloxypropyl, 1-propoxycarbonyloxypropyl, 1-isopropoxycarbonyloxypropyl, 1-butoxycarbonyloxypropyl, 1-isobutoxycarbonyloxypropyl, 1-pentyloxycarbonyloxypropyl, 1-hexyloxycarbonyloxypropyl, 1-methoxycarbonyloxybutyl, 1-ethoxycarbonyloxybutyl, 1-propoxycarbonyloxybutyl, 1-isopropoxycarbonyloxybutyl, 1-butoxycarbonyloxybutyl, 1-isobutoxycarbonyloxybutyl, 1-methoxycarbonyloxypentyl, 1-ethoxycarbonyloxypentyl, 1-methoxycarbonyloxyhexyl or 1-ethoxycarbonyloxyhexyl group; a "carbonyloxyalkyl group", including an oxodioxolenylmethyl group such as a (5-phenyl-2-oxo-1,3-dioxolen-4-yl)methyl, [5-(4-methylphenyl)-2-oxo-1,3-dioxolen-4-yl]methyl, [5-(4-methoxyphenyl)-2-oxo-1,3-dioxolen-4-yl]methyl, [5-(4-fluorophenyl)-2-oxo-1,3-dioxolen-4-yl]methyl, [5-(4-chlorophenyl)-2-oxo-1,3-dioxolen-4-yl]methyl, (2-oxo-1,3-dioxolen-4-yl)methyl, (5-methyl-2-oxo-1,3-dioxolen-4-yl)methyl, (5-ethyl-2-oxo-1,3-dioxolen-4-yl)methyl, (5-propyl-2-oxo-1,3-dioxolen-4-yl)methyl, (5-isopropyl-2-oxo-1,3-dioxolen-4-yl)methyl or (5-butyl-2-oxo-1,3-dioxolen-4-yl)methyl group; a "phthalidyl group" such as a phthalidyl, dimethylphthalidyl or dimethoxyphthalidyl group; an "aryl group" such as a phenyl and indanyl group; a "carboxyalkyl group" such as a carboxymethyl group; or a "residual group forming an amino acid amide" such as a phenylalanine, and is preferably a group which forms pharmacologically acceptable esters such as an "alkyl group", "alkoxyalkyl group", "carbonyloxyalkyl group" or (alkoxycarbonyloxy)alkyl group; more preferably a "C1-C6 alkyl group" such as a methyl, ethyl, n-propyl, isopropyl, n-butyl, isobutyl, s-butyl, tert-butyl, n-pentyl, isopentyl, 2-methylbutyl, neopentyl, 1-ethylpropyl, n-hexyl, 4-methylpentyl, 3-methylpentyl, 2-methylpentyl or 1-methylpentyl; and particularly preferably methyl or ethyl group.

When the compounds (I) of the present invention contain a hydroxyl group in their structures, the functional group employed for forming a prodrug thereof can be, for example, an "aliphatic acyl group", including an alkylcarbonyl group such as a formyl, acetyl, propionyl, butyryl, isobutyryl, pentanoyl, pivaloyl, valeryl, isovaleryl, octanoyl, nonanoyl, decanoyl, 3-methylnonanoyl, 8-methylnonanoyl, 3-ethyloctanoyl, 3,7-dimethyloctanoyl, undecanoyl, dodecanoyl, tridecanoyl, tetradecanoyl, pentadecanoyl, hexadecanoyl, 1-methylpentadecanoyl, 14-methylpentadecanoyl, 13,13-dimethyltetradecanoyl, heptadecanoyl, 15-methylhexadecanoyl, octadecanoyl, 1-methylheptadecanoyl, nonadecanoyl, icosanoyl or henicosanoyl group; a carboxylated alkylcarbonyl group such as a succinoyl, glutaroyl or adipoyl group; a halogeno lower alkylcarbonyl group such as a chloroacetyl, dichloroacetyl, trichloroacetyl or trifluoroacetyl group; a lower alkoxy lower alkylcarbonyl group such as a methoxyacetyl group; and an unsaturated alkylcarbonyl group such as a (E)-2-methyl-2-butenoyl group (preferably a C1-C6 alkanoyl group); an "aromatic acyl group", including an arylcarbonyl group such as a benzoyl, α-naphthoyl or β-naphthoyl group; a halogenoarylcarbonyl group such as a 2-bromobenzoyl or 4-chlorobenzoyl group; a lower alkylated arylcarbonyl group such as a 2,4,6-trimethylbenzoyl or 4-toluoyl group; a lower alkoxylated arylcarbonyl group such as a 4-anisoyl group; a carboxylated arylcarbonyl group such as a 2-carboxybenzoyl, 3-carboxybenzoyl or 4-carboxybenzoyl group; a nitrated arylcarbonyl group such as a 4-nitrobenzoyl or 2-nitrobenzoyl group; a lower alkoxycarbonylated arylcarbonyl group such as a 2-(methoxycarbonyl)benzoyl group; and an arylated arylcarbonyl group such as a 4-phenylbenzoyl group; a "carbonyloxyalkyl group", including an acyloxyalkyl group such as an ethylcarbonyloxymethyl, pivaloyloxymethyl, dimethylaminoacetyloxymethyl or 1-acetoxyethyl group; a 1-(alkoxycarbonyloxy)alkyl group such as a 1-(methoxycarbonyloxy)ethyl, 1-(ethoxycarbonyloxy)ethyl, ethoxycarbonyloxymethyl, 1-(isopropoxycarbonyloxy)ethyl, 1-(t-butoxycarbonyloxy)ethyl, 1-(ethoxycarbonyloxy)propyl or 1-(cyclohexyloxycarbonyloxy)ethyl group; a phthalidyl group; and an oxodioxolenylmethyl group such as a 4-methyl-oxodioxolenylmethyl, 4-phenyl-oxodioxolenylmethyl or oxodioxolenylmethyl group; a "residual group of a salt of a succinic acid half-ester"; a "residual group of a salt of a phosphoric acid ester"; a "residual group forming an amino acid ester"; a carbamoyl group; a carbamoyl group substituted with 1 or 2 lower alkyl groups; or a "carbonyloxyalkyloxycarbonyl group" such as a pivaloyloxymethyloxycarbonyl group, and is preferably a group which forms a pharmacologically acceptable ester such as an "aliphatic acyl group" or an "aromatic acyl group", more preferably a "C1-C6 alkanoyl group" such as an acetyl, propionyl, butyryl, isobutyryl, pentanoyl or pivaloyl group; and particularly preferably an acetyl group.

When the compounds (I) of the present invention contain an amino group in their structures, the functional group employed for forming a prodrug thereof can be, for example, an aliphatic acyl group, including an alkanoyl group such as a formyl, acetyl, propionyl, butyryl, isobutyryl, pentanoyl, pivaloyl, valeryl, isovaleryl, octanoyl, lauroyl, palmitoyl or stearoyl group; a halogeno lower alkylcarbonyl group such as a chloroacetyl, dichloroacetyl, trichloroacetyl or trifluoroacetyl group; a lower alkoxy lower alkylcarbonyl group such as a methoxyacetyl group; and an unsaturated alkylcarbonyl group such as a (E)-2-methyl-2-butenoyl group (preferably a C1-C6 alkanoyl group); an aromatic acyl group, including an arylcarbonyl group such as a benzoyl, α-naphthoyl or β-naphthoyl group, a halogenoarylcarbonyl group such as a 2-bromobenzoyl or 4-chlorobenzoyl group, a lower alkylated arylcarbonyl group such as a 2,4,6-trimethylbenzoyl or 4-toluoyl group, a lower alkoxylated arylcarbonyl group such as a 4-anisoyl group, a nitrated arylcarbonyl group such as a 4-nitrobenzoyl or 2-nitrobenzoyl group, a lower alkoxycarbonylated arylcarbonyl group such as a 2-(methoxycarbonyl)benzoyl group, and an arylated arylcarbonyl group such as a 4-phenylbenzoyl group; an alkoxycarbonyl group, including a lower alkoxycarbonyl group such as a methoxycarbonyl, ethoxycarbonyl, t-butoxycarbonyl or isobutoxycarbonyl group, and a lower alkoxycarbonyl group substituted with halogen atom(s) or tri-lower alkylsilyl group(s), such as a 2,2,2-trichloroethoxycarbonyl or 2-trimethylsilylethoxycarbonyl group; an alkenyloxycarbonyl group such as a vinyloxycarbonyl or allyloxycarbonyl group; or an aryloxycarbonyl group, of which an aryl ring may be substituted with 1 or 2 lower alkoxy group(s), nitro group(s) or halogen atom(s), such as a phenoxycarbonyl, 4-methoxyphenoxycarbonyl, 3,4-dimethoxyphenoxycarbonyl, 2-nitrophenoxycarbonyl, 4-nitrophenoxycarbonyl or 4-fluorophenoxycarbonyl group, and is preferably a C1-C6 alkanoyl group.

The "prodrug" of the compounds having the formula (I) is preferably a pharmacologically acceptable ester thereof that are prepared by converting the sulfanyl group, carboxy group or hydroxyl group contained in said compounds, respectively.

The "pharmacologically acceptable salts thereof" mean a salt that is prepared from the compounds (I) of the present invention. Such salt is preferably a metal salt, including an alkali metal salt such as sodium salt, potassium salt or lithium salt, an alkaline earth metal salt such as calcium salt or magnesium salt, an aluminium salt, an iron salt, a zinc salt, a copper salt, a nickel salt and a cobalt salt; an amine salt, including an inorganic salt such as ammonium salt, and an organic salt such as t-octylamine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzyl-phenethylamine salt, piperazine salt, tetramethylammonium salt or tris(hydroxymethyl)aminomethane salt; an inorganic acid salt, including a hydrohalogenic acid salt such as hydrofluoride, hydrochloride, hydrobromide or hydroiodide, a nitrate, a perchlorate, a sulfate and a phosphate; an organic acid salt, including a lower alkanesulfonate such as methanesulfonate, trifluoromethanesulfonate or ethanesulfonate, an arylsulfonate such as a benzenesulfonate or p-toluenesulfonate, an acetate, a malate, a fumarate, a succinate, a citrate, a tartrate, an oxalate, a maleate and a trifluoroacetate; or an amino acid salt such as glycine salt, lysine salt, arginine salt, ornithine salt, glutamate or aspartate, and more preferably an inorganic acid salt or an organic acid salt.

Furthermore, the compounds (I) of the present invention can exist as a hydrate or solvate thereof.

The preferred examples of the compounds of general formula (I) can be specifically shown in Tables 1-6, but the scope of the present invention should not be limited to these compounds.

The meaning of the abbreviations in the following Tables is shown below.
Ac: acetyl group,
Me: methyl group,
Et: ethyl group,
iPr: isopropyl group,
1-Pyza: pyrazol-1-yl group,
3-Pyza: pyrazol-3-yl group,
4-Pyza: pyrazol-4-yl group,
5-Pyza: pyrazol-5-yl group,
1-Triz: 1,2,3-triazol-1-yl group,
2-Triz: 1,2,3-triazol-2-yl group,
4-Triz: 1,2,3-triazol-4-yl group,
5-Triz: 1,2,3-triazol-5-yl group,
1-Tez: tetrazol-1-yl group,
2-Tez: tetrazol-2-yl group,
5-Tez: tetrazol-5-yl group,
1-Pyrd: pyrrolidino group,
1-Pip: piperidino group,
4-Pip: piperidin-4-yl group,
Mor: morpholino group,
Thim: thiomorpholino group,
1-Piz: piperazino group, and
2,5-dioxo-1-Pip: 2,5-dioxo-1-piperidino group.

**(Table 1)**

| | | | | | | |
|---|---|---|---|---|---|---|
| 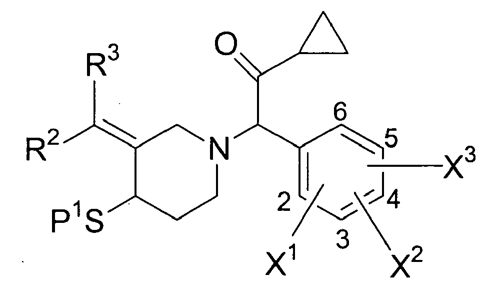 | | | | | | |

| Compound No. | P¹ | R² | R³ | X¹ | X² | X³ |
|---|---|---|---|---|---|---|
| 1-1 | H | H | CH₂-1-Pyza | 2-F | H | H |
| 1-2 | Ac | H | CH₂-1-Pyza | 2-F | H | H |
| 1-3 | H | H | CH₂-1-Pyza | 2-F | 4-F | H |
| 1-4 | Ac | H | CH₂-1-Pyza | 2-F | 4-F | H |
| 1-5 | H | H | CH₂-(3-CO₂H-1-Pyza) | 2-F | H | H |
| 1-6 | Ac | H | CH₂-(3-CO₂H-1-Pyza) | 2-F | H | H |
| 1-7 | H | H | CH₂-(3-CO₂H-1-Pyza) | 2-F | 4-F | H |
| 1-8 | Ac | H | CH₂-(3-CO₂H-1-Pyza) | 2-F | 4-F | H |
| 1-9 | H | H | CH₂-(3-CO₂Me-1-Pyza) | 2-F | H | H |
| 1-10 | Ac | H | CH₂-(3-CO₂Me-1-Pyza) | 2-F | H | H |
| 1-11 | H | H | CH₂-(3-CO₂Me-1-Pyza) | 2-F | 4-F | H |
| 1-12 | Ac | H | CH₂-(3-CO₂Me-1-Pyza) | 2-F | 4-F | H |
| 1-13 | H | H | CH₂-(3-CO₂Et-1-Pyza) | 2-F | H | H |
| 1-14 | Ac | H | CH₂-(3-CO₂Et-1-Pyza) | 2-F | H | H |
| 1-15 | H | H | CH₂-(3-CO₂Et-1-Pyza) | 2-F | 4-F | H |
| 1-16 | Ac | H | CH₂-(3-CO₂Et-1-Pyza) | 2-F | 4-F | H |
| 1-17 | H | H | CH₂-(4-CO₂H-1-Pyza) | 2-F | H | H |
| 1-18 | Ac | H | CH₂-(4-CO₂H-1-Pyza) | 2-F | H | H |
| 1-19 | H | H | CH₂-(4-CO₂H-1-Pyza) | 2-F | 4-F | H |
| 1-20 | Ac | H | CH₂-(4-CO₂H-1-Pyza) | 2-F | 4-F | H |
| 1-21 | H | H | CH₂-(4-CO₂Me-1-Pyza) | 2-F | H | H |
| 1-22 | Ac | H | CH₂-(4-CO₂Me-1-Pyza) | 2-F | H | H |
| 1-23 | H | H | CH₂-(4-CO₂Me-1-Pyza) | 2-F | 4-F | H |
| 1-24 | Ac | H | CH₂-(4-CO₂Me-1-Pyza) | 2-F | 4-F | H |
| 1-25 | H | H | CH₂-(4-CO₂Et-1-Pyza) | 2-F | H | H |
| 1-26 | Ac | H | CH₂-(4-CO₂Et-1-Pyza) | 2-F | H | H |
| 1-27 | H | H | CH₂-(4-CO₂Et-1-Pyza) | 2-F | 4-F | H |
| 1-28 | Ac | H | CH₂-(4-CO₂Et-1-Pyza) | 2-F | 4-F | H |
| 1-29 | H | H | CH₂-(5-CO₂H-1-Pyza) | 2-F | H | H |
| 1-30 | Ac | H | CH₂-(5-CO₂H-1-Pyza) | 2-F | H | H |
| 1-31 | H | H | CH₂-(5-CO₂H-1-Pyza) | 2-F | 4-F | H |
| 1-32 | Ac | H | CH₂-(5-CO₂H-1-Pyza) | 2-F | 4-F | H |
| 1-33 | H | H | CH₂-(5-CO₂Me-1-Pyza) | 2-F | H | H |
| 1-34 | Ac | H | CH₂-(5-CO₂Me-1-Pyza) | 2-F | H | H |
| 1-35 | H | H | CH₂-(5-CO₂Me-1-Pyza) | 2-F | 4-F | H |
| 1-36 | Ac | H | CH₂-(5-CO₂Me-1-Pyza) | 2-F | 4-F | H |
| 1-37 | H | H | CH₂-(5-CO₂Et-1-Pyza) | 2-F | H | H |
| 1-38 | Ac | H | CH₂-(5-CO₂Et-1-Pyza) | 2-F | H | H |
| 1-39 | H | H | CH₂-(5-CO₂Et-1-Pyza) | 2-F | 4-F | H |
| 1-40 | Ac | H | CH₂-(5-CO₂Et-1-Pyza) | 2-F | 4-F | H |
| 1-41 | H | H | CH₂-(3-CH₂CO₂H-1-Pyza) | 2-F | H | H |
| 1-42 | Ac | H | CH₂-(3-CH₂CO₂H-1-Pyza) | 2-F | H | H |
| 1-43 | H | H | CH₂-(3-CH₂CO₂H-1-Pyza) | 2-F | 4-F | H |
| 1-44 | Ac | H | CH₂-(3-CH₂CO₂H-1-Pyza) | 2-F | 4-F | H |
| 1-45 | H | H | CH₂-(3-CH₂CO₂Me-1-Pyza) | 2-F | H | H |
| 1-46 | Ac | H | CH₂-(3-CH₂CO₂Me-1-Pyza) | 2-F | H | H |
| 1-47 | H | H | CH₂-(3-CH₂CO₂Me-1-Pyza) | 2-F | 4-F | H |
| 1-48 | Ac | H | CH₂-(3-CH₂CO₂Me-1-Pyza) | 2-F | 4-F | H |
| 1-49 | H | H | CH₂-(3-CH₂CO₂Et-1-Pyza) | 2-F | H | H |
| 1-50 | Ac | H | CH₂-(3-CH₂CO₂Et-1-Pyza) | 2-F | H | H |
| 1-51 | H | H | CH₂-(3-CH₂CO₂Et-1-Pyza) | 2-F | 4-F | H |
| 1-52 | Ac | H | CH₂-(3-CH₂CO₂Et-1-Pyza) | 2-F | 4-F | H |
| 1-53 | H | H | CH₂-(4-CH₂CO₂H-1-Pyza) | 2-F | H | H |
| 1-54 | Ac | H | CH₂-(4-CH₂CO₂H-1-Pyza) | 2-F | H | H |
| 1-55 | H | H | CH₂-(4-CH₂CO₂H-1-Pyza) | 2-F | 4-F | H |
| 1-56 | Ac | H | CH₂-(4-CH₂CO₂H-1-Pyza) | 2-F | 4-F | H |
| 1-57 | H | H | CH₂-(4-CH₂CO₂Me-1-Pyza) | 2-F | H | H |
| 1-58 | Ac | H | CH₂-(4-CH₂CO₂Me-1-Pyza) | 2-F | H | H |
| 1-59 | H | H | CH₂-(4-CH₂CO₂Me-1-Pyza) | 2-F | 4-F | H |
| 1-60 | Ac | H | CH₂-(4-CH₂CO₂Me-1-Pyza) | 2-F | 4-F | H |
| 1-61 | H | H | CH₂-(4-CH₂CO₂Et-1-Pyza) | 2-F | H | H |
| 1-62 | Ac | H | CH₂-(4-CH₂CO₂Et-1-Pyza) | 2-F | H | H |
| 1-63 | H | H | CH₂-(4-CH₂CO₂Et-1-Pyza) | 2-F | 4-F | H |
| 1-64 | Ac | H | CH₂-(4-CH₂CO₂Et-1-Pyza) | 2-F | 4-F | H |
| 1-65 | H | H | CH₂-(5-CH₂CO₂H-1-Pyza) | 2-F | H | H |
| 1-66 | Ac | H | CH₂-(5-CH₂CO₂H-1-Pyza) | 2-F | H | H |
| 1-67 | H | H | CH₂-(5-CH₂CO₂H-1-Pyza) | 2-F | 4-F | H |
| 1-68 | Ac | H | CH₂-(5-CH₂CO₂H-1-Pyza) | 2-F | 4-F | H |
| 1-69 | H | H | CH₂-(5-CH₂CO₂Me-1-Pyza) | 2-F | H | H |
| 1-70 | Ac | H | CH₂-(5-CH₂CO₂Me-1-Pyza) | 2-F | H | H |
| 1-71 | H | H | CH₂-(5-CH₂CO₂Me-1-Pyza) | 2-F | 4-F | H |
| 1-72 | Ac | H | CH₂-(5-CH₂CO₂Me-1-Pyza) | 2-F | 4-F | H |
| 1-73 | H | H | CH₂-(5-CH₂CO₂Et-1-Pyza) | 2-F | H | H |
| 1-74 | Ac | H | CH₂-(5-CH₂CO₂Et-1-Pyza) | 2-F | H | H |
| 1-75 | H | H | CH₂-(5-CH₂CO₂Et-1-Pyza) | 2-F | 4-F | H |
| 1-76 | Ac | H | CH₂-(5-CH₂CO₂Et-1-Pyza) | 2-F | 4-F | H |
| 1-77 | H | H | CH₂-(3-CH₂CH₂CO₂H-1-Pyza) | 2-F | H | H |
| 1-78 | Ac | H | CH₂-(3-CH₂CH₂CO₂H-1-Pyza) | 2-F | H | H |
| 1-79 | H | H | CH₂-(3-CH₂CH₂CO₂Me-1-Pyza) | 2-F | H | H |
| 1-80 | Ac | H | CH₂-(3-CH₂CH₂CO₂Me-1-Pyza) | 2-F | H | H |
| 1-81 | H | H | CH₂-(3-CH₂CH₂CO₂Et-1-Pyza) | 2-F | H | H |
| 1-82 | Ac | H | CH₂-(3-CH₂CH₂CO₂Et-1-Pyza) | 2-F | H | H |
| 1-83 | H | H | CH₂-(4-CH₂CH₂CO₂H-1-Pyza) | 2-F | H | H |
| 1-84 | Ac | H | CH₂-(4-CH₂CH₂CO₂H-1-Pyza) | 2-F | H | H |
| 1-85 | H | H | CH₂-(4-CH₂CH₂CO₂Me-1-Pyza) | 2-F | H | H |
| 1-86 | Ac | H | CH₂-(4-CH₂CH₂CO₂Me-1-Pyza) | 2-F | H | H |
| 1-87 | H | H | CH₂-(4-CH₂CH₂CO₂Et-1-Pyza) | 2-F | H | H |
| 1-88 | Ac | H | CH₂-(4-CH₂CH₂CO₂Et-1-Pyza) | 2-F | H | H |
| 1-89 | H | H | CH₂-(5-CH₂CH₂CO₂H-1-Pyza) | 2-F | H | H |
| 1-90 | Ac | H | CH₂-(5-CH₂CH₂CO₂H-1-Pyza) | 2-F | H | H |
| 1-91 | H | H | CH₂-(5-CH₂CH₂CO₂Me-1-Pyza) | 2-F | H | H |
| 1-92 | Ac | H | CH₂-(5-CH₂CH₂CO₂Me-1-Pyza) | 2-F | H | H |
| 1-93 | H | H | CH₂-(5-CH₂CH₂CO₂Et-1-Pyza) | 2-F | H | H |
| 1-94 | Ac | H | CH₂-(5-CH₂CH₂CO₂Et-1-Pyza) | 2-F | H | H |
| 1-95 | H | H | CH₂-(3-CH₂CH₂CH₂CO₂H-1-Pyza) | 2-F | H | H |
| 1-96 | Ac | H | CH₂-(3-CH₂CH₂CH₂CO₂H-1-Pyza) | 2-F | H | H |
| 1-97 | H | H | CH₂-(3-CH₂CH₂CH₂CO₂Me-1-Pyza) | 2-F | H | H |
| 1-98 | Ac | H | CH₂-(3-CH₂CH₂CH₂CO₂Me-1-Pyza) | 2-F | H | H |
| 1-99 | H | H | CH₂-(3-CH₂CH₂CH₂CO₂Et-1-Pyza) | 2-F | H | H |
| 1-100 | Ac | H | CH₂-(3-CH₂CH₂CH₂CO₂Et-1-Pyza) | 2-F | H | H |
| 1-101 | H | H | CH₂-(4-CH₂CH₂CH₂CO₂H-1-Pyza) | 2-F | H | H |
| 1-102 | Ac | H | CH₂-(4-CH₂CH₂CH₂CO₂H-1-Pyza) | 2-F | H | H |
| 1-103 | H | H | CH₂-(4-CH₂CH₂CH₂CO₂Me-1-Pyza) | 2-F | H | H |
| 1-104 | Ac | H | CH₂-(4-CH₂CH₂CH₂CO₂Me-1-Pyza) | 2-F | H | H |
| 1-105 | H | H | CH₂-(4-CH₂CH₂CH₂CO₂Et-1-Pyza) | 2-F | H | H |
| 1-106 | Ac | H | CH₂-(4-CH₂CH₂CH₂CO₂Et-1-Pyza) | 2-F | H | H |
| 1-107 | H | H | CH₂-(5-CH₂CH₂CH₂CO₂H-1-Pyza) | 2-F | H | H |
| 1-108 | Ac | H | CH₂-(5-CH₂CH₂CH₂CO₂H-1-Pyza) | 2-F | H | H |
| 1-109 | H | H | CH₂-(5-CH₂CH₂CH₂CO₂Me-1-Pyza) | 2-F | H | H |
| 1-110 | Ac | H | CH₂-(5-CH₂CH₂CH₂CO₂Me-1-Pyza) | 2-F | H | H |
| 1-111 | H | H | CH₂-(5-CH₂CH₂CH₂CO₂Et-1-Pyza) | 2-F | H | H |
| 1-112 | Ac | H | CH₂-(5-CH₂CH₂CH₂CO₂Et-1-Pyza) | 2-F | H | H |
| 1-113 | H | H | CH₂CH₂-1-Pyza | 2-F | H | H |
| 1-114 | Ac | H | CH₂CH₂-1-Pyza | 2-F | H | H |
| 1-115 | H | H | CH₂CH₂-(3-CO₂H-1-Pyza) | 2-F | H | H |
| 1-116 | Ac | H | CH₂CH₂-(3-CO₂H-1-Pyza) | 2-F | H | H |
| 1-117 | H | H | CH₂CH₂-(3-CO₂Me-1-Pyza) | 2-F | H | H |
| 1-118 | Ac | H | CH₂CH₂-(3-CO₂Me-1-Pyza) | 2-F | H | H |
| 1-119 | H | H | CH₂CH₂-(3-CO₂Et-1-Pyza) | 2-F | H | H |
| 1-120 | Ac | H | CH₂CH₂-(3-CO₂Et-1-Pyza) | 2-F | H | H |
| 1-121 | H | H | CH₂CH₂-(4-CO₂H-1-Pyza) | 2-F | H | H |
| 1-122 | Ac | H | CH₂CH₂-(4-CO₂H-1-Pyza) | 2-F | H | H |
| 1-123 | H | H | CH₂CH₂-(4-CO₂Me-1-Pyza) | 2-F | H | H |
| 1-124 | Ac | H | CH₂CH₂-(4-CO₂Me-1-Pyza) | 2-F | H | H |
| 1-125 | H | H | CH₂CH₂-(4-CO₂Et-1-Pyza) | 2-F | H | H |
| 1-126 | Ac | H | CH₂CH₂-(4-CO₂Et-1-Pyza) | 2-F | H | H |
| 1-127 | H | H | CH₂CH₂-(5-CO₂H-1-Pyza) | 2-F | H | H |
| 1-128 | Ac | H | CH₂CH₂-(5-CO₂H-1-Pyza) | 2-F | H | H |
| 1-129 | H | H | CH₂CH₂-(5-CO₂Me-1-Pyza) | 2-F | H | H |
| 1-130 | Ac | H | CH₂CH₂-(5-CO₂Me-1-Pyza) | 2-F | H | H |
| 1-131 | H | H | CH₂CH₂-(5-CO₂Et-1-Pyza) | 2-F | H | H |
| 1-132 | Ac | H | CH₂CH₂-(5-CO₂Et-1-Pyza) | 2-F | H | H |
| 1-133 | H | H | CH₂CH₂-(3-CH₂CO₂H-1-Pyza) | 2-F | H | H |
| 1-134 | Ac | H | CH₂CH₂-(3-CH₂CO₂H-1-Pyza) | 2-F | H | H |
| 1-135 | H | H | CH₂CH₂-(3-CH₂CO₂Me-1-Pyza) | 2-F | H | H |
| 1-136 | Ac | H | CH₂CH₂-(3-CH₂CO₂Me-1-Pyza) | 2-F | H | H |
| 1-137 | H | H | CH₂CH₂-(3-CH₂CO₂Et-1-Pyza) | 2-F | H | H |
| 1-138 | Ac | H | CH₂CH₂-(3-CH₂CO₂Et-1-Pyza) | 2-F | H | H |
| 1-139 | H | H | CH₂CH₂-(4-CH₂CO₂H-1-Pyza) | 2-F | H | H |
| 1-140 | Ac | H | CH₂CH₂-(4-CH₂CO₂H-1-Pyza) | 2-F | H | H |
| 1-141 | H | H | CH₂CH₂-(4-CH₂CO₂Me-1-Pyza) | 2-F | H | H |
| 1-142 | Ac | H | CH₂CH₂-(4-CH₂CO₂Me-1-Pyza) | 2-F | H | H |
| 1-143 | H | H | CH₂CH₂-(4-CH₂CO₂Et-1-Pyza) | 2-F | H | H |
| 1-144 | Ac | H | CH₂CH₂-(4-CH₂CO₂Et-1-Pyza) | 2-F | H | H |
| 1-145 | H | H | CH₂CH₂-(5-CH₂CO₂H-1-Pyza) | 2-F | H | H |
| 1-146 | Ac | H | CH₂CH₂-(5-CH₂CO₂H-1-Pyza) | 2-F | H | H |
| 1-147 | H | H | CH₂CH₂-(5-CH₂CO₂Me-1-Pyza) | 2-F | H | H |
| 1-148 | Ac | H | CH₂CH₂-(5-CH₂CO₂Me-1-Pyza) | 2-F | H | H |
| 1-149 | H | H | CH₂CH₂-(5-CH₂CO₂Et-1-Pyza) | 2-F | H | H |
| 1-150 | Ac | H | CH₂CH₂-(5-CH₂CO₂Et-1-Pyza) | 2-F | H | H |
| 1-151 | H | H | CH₂CH₂-(3-CH₂CH₂CO₂H-1-Pyza) | 2-F | H | H |
| 1-152 | Ac | H | CH₂CH₂-(3-CH₂CH₂CO₂H-1-Pyza) | 2-F | H | H |
| 1-153 | H | H | CH₂CH₂-(3-CH₂CH₂CO₂Me-1-Pyza) | 2-F | H | H |
| 1-154 | Ac | H | CH₂CH₂-(3-CH₂CH₂CO₂Me-1-Pyza) | 2-F | H | H |
| 1-155 | H | H | CH₂CH₂-(3-CH₂CH₂CO₂Et-1-Pyza) | 2-F | H | H |
| 1-156 | Ac | H | CH₂CH₂-(3-CH₂CH₂CO₂Et-1-Pyza) | 2-F | H | H |
| 1-157 | H | H | CH₂CH₂-(4-CH₂CH₂CO₂H-1-Pyza) | 2-F | H | H |
| 1-158 | Ac | H | CH₂CH₂-(4-CH₂CH₂CO₂H-1-Pyza) | 2-F | H | H |
| 1-159 | H | H | CH₂CH₂-(4-CH₂CH₂CO₂Me-1-Pyza) | 2-F | H | H |
| 1-160 | Ac | H | CH₂CH₂-(4-CH₂CH₂CO₂Me-1-Pyza) | 2-F | H | H |
| 1-161 | H | H | CH₂CH₂-(4-CH₂CH₂CO₂Et-1-Pyza) | 2-F | H | H |
| 1-162 | Ac | H | CH₂CH₂-(4-CH₂CH₂CO₂Et-1-Pyza) | 2-F | H | H |
| 1-163 | H | H | CH₂CH₂-(5-CH₂CH₂CO₂H-1-Pyza) | 2-F | H | H |
| 1-164 | Ac | H | CH₂CH₂-(5-CH₂CH₂CO₂H-1-Pyza) | 2-F | H | H |
| 1-165 | H | H | CH₂CH₂-(5-CH₂CH₂CO₂Me-1-Pyza) | 2-F | H | H |
| 1-166 | Ac | H | CH₂CH₂-(5-CH₂CH₂CO₂Me-1-Pyza) | 2-F | H | H |
| 1-167 | H | H | CH₂CH₂-(5-CH₂CH₂CO₂Et-1-Pyza | 2-F | H | H |
| 1-168 | Ac | H | CH₂CH₂-(5-CH₂CH₂CO₂Et-1-Pyza) | 2-F | H | H |
| 1-169 | H | H | CH₂CH₂-(3-CH₂CH₂CH₂CO₂H-1-Pyza) | 2-F | H | H |
| 1-170 | Ac | H | CH₂CH₂-(3-CH₂CH₂CH₂CO₂H-1-Pyza) | 2-F | H | H |
| 1-171 | H | H | CH₂CH₂-(3-CH₂CH₂CH₂CO₂Me-1-Pyza) | 2-F | H | H |
| 1-172 | Ac | H | CH₂CH₂-(3-CH₂CH₂CH₂CO₂Me-1-Pyza) | 2-F | H | H |
| 1-173 | H | H | CH₂CH₂-(3-CH₂CH₂CH₂CO₂Et-1-Pyza) | 2-F | H | H |
| 1-174 | Ac | H | CH₂CH₂(3-CH₂CH₂CH₂CO₂Et-1-Pyza) | 2-F | H | H |
| 1-175 | H | H | CH₂CH₂-(4-CH₂CH₂CH₂CO₂H-1-Pyza) | 2-F | H | H |
| 1-176 | Ac | H | CH₂CH₂-(4-CH₂CH₂CH₂CO₂H-1-Pyza) | 2-F | H | H |
| 1-177 | H | H | CH₂CH₂-(4-CH₂CH₂CH₂CO₂Me-1-Pyza) | 2-F | H | H |
| 1-178 | Ac | H | CH₂CH₂-(4-CH₂CH₂CH₂CO₂Me-1-Pyza) | 2-F | H | H |
| 1-179 | H | H | CH₂CH₂-(4-CH₂CH₂CH₂CO₂Et-1-Pyza) | 2-F | H | H |
| 1-180 | Ac | H | CH₂CH₂-(4-CH₂CH₂CH₂CO₂Et-1-Pyza) | 2-F | H | H |
| 1-181 | H | H | CH₂CH₂-(5-CH₂CH₂CH₂CO₂H-1-Pyza) | 2-F | H | H |
| 1-182 | Ac | H | CH₂CH₂-(5-CH₂CH₂CH₂CO₂H-1-Pyza) | 2-F | H | H |
| 1-183 | H | H | CH₂CH₂-(5-CH₂CH₂CH₂CO₂Me-1-Pyza) | 2-F | H | H |
| 1-184 | Ac | H | CH₂CH₂-(5-CH₂CH₂CH₂CO₂Me-1-Pyza) | 2-F | H | H |
| 1-185 | H | H | CH₂CH₂-(5-CH₂CH₂CH₂CO₂Et-1-Pyza) | 2-F | H | H |
| 1-186 | Ac | H | CH₂CH₂-(5-CH₂CH₂CH₂CO₂Et-1-Pyza) | 2-F | H | H |
| 1-187 | H | H | CH₂-3-Pyza | 2-F | H | H |
| 1-188 | Ac | H | CH₂-3-Pyza | 2-F | H | H |
| 1-189 | H | H | CH₂-3-Pyza | 2-F | 4-F | H |
| 1-190 | Ac | H | CH₂-3-Pyza | 2-F | 4-F | H |
| 1-191 | H | H | CH₂-(1-CO₂Me-3-Pyza) | 2-F | H | H |
| 1-192 | Ac | H | CH₂-(1-CO₂Me-3-Pyza) | 2-F | H | H |
| 1-193 | H | H | CH₂-(1-CO₂Et-3-Pyza) | 2-F | H | H |
| 1-194 | Ac | H | CH₂-(1-CO₂Et-3-Pyza) | 2-F | H | H |
| 1-195 | H | H | CH₂-(4-CO₂H-3-Pyza) | 2-F | H | H |
| 1-196 | Ac | H | CH₂-(4-CO₂H-3-Pyza) | 2-F | H | H |
| 1-197 | H | H | CH₂-(4-CO₂Me-3-Pyza) | 2-F | H | H |
| 1-198 | Ac | H | CH₂-(4-CO₂Me-3-Pyza) | 2-F | H | H |
| 1-199 | H | H | CH₂-(4-CO₂Et-3-Pyza) | 2-F | H | H |
| 1-200 | Ac | H | CH₂-(4-CO₂Et-3-Pyza) | 2-F | H | H |
| 1-201 | H | H | CH₂-(5-CO₂H-3-Pyza) | 2-F | H | H |
| 1-202 | Ac | H | CH₂-(5-CO₂H-3-Pyza) | 2-F | H | H |
| 1-203 | H | H | CH₂-(5-CO₂Me-3-Pyza) | 2-F | H | H |
| 1-204 | Ac | H | CH₂-(5-CO₂Me-3-Pyza) | 2-F | H | H |
| 1-205 | H | H | CH₂-(5-CO₂Et-3-Pyza) | 2-F | H | H |
| 1-206 | Ac | H | CH₂-(5-CO₂Et-3-Pyza) | 2-F | H | H |
| 1-207 | H | H | CH₂-(1-CH₂CO₂H-3-Pyza) | 2-F | H | H |
| 1-208 | Ac | H | CH₂-(1-CH₂CO₂H-3-Pyza) | 2-F | H | H |
| 1-209 | H | H | CH₂-(1-CH₂CO₂Me-3-Pyza) | 2-F | H | H |
| 1-210 | Ac | H | CH₂-(1-CH₂CO₂Me-3-Pyza) | 2-F | H | H |
| 1-211 | H | H | CH₂-(1-CH₂CO₂Et-3-Pyza) | 2-F | H | H |
| 1-212 | Ac | H | CH₂-(1-CH₂CO₂Et-3-Pyza) | 2-F | H | H |
| 1-213 | H | H | CH₂-(4-CH₂CO₂H-3-Pyza) | 2-F | H | H |
| 1-214 | Ac | H | CH₂-(4-CH₂CO₂H-3-Pyza) | 2-F | H | H |
| 1-215 | H | H | CH₂-(4-CH₂CO₂Me-3-Pyza) | 2-F | H | H |
| 1-216 | Ac | H | CH₂-(4-CH₂CO₂Me-3-Pyza) | 2-F | H | H |
| 1-217 | H | H | CH₂-(4-CH₂CO₂Et-3-Pyza) | 2-F | H | H |
| 1-218 | Ac | H | CH₂-(4-CH₂CO₂Et-3-Pyza) | 2-F | H | H |
| 1-219 | H | H | CH₂-(5-CH₂CO₂H-3-Pyza) | 2-F | H | H |
| 1-220 | Ac | H | CH₂-(5-CH₂CO₂H-3-Pyza) | 2-F | H | H |
| 1-221 | H | H | CH₂-(5-CH₂CO₂Me-3-Pyza) | 2-F | H | H |
| 1-222 | Ac | H | CH₂-(5-CH₂CO₂Me-3-Pyza) | 2-F | H | H |
| 1-223 | H | H | CH₂-(5-CH₂CO₂Et-3-Pyza) | 2-F | H | H |
| 1-224 | Ac | H | CH₂-(5-CH₂CO₂Et-3-Pyza) | 2-F | H | H |
| 1-225 | H | H | CH₂-(1-CH₂CH₂CO₂H-3-Pyza) | 2-F | H | H |
| 1-226 | Ac | H | CH₂-(1-CH₂CH₂CO₂H-3-Pyza) | 2-F | H | H |
| 1-227 | H | H | CH₂-(1-CH₂CH₂CO₂Me-3-Pyza) | 2-F | H | H |
| 1-228 | Ac | H | CH₂-(1-CH₂CH₂CO₂Me-3-Pyza) | 2-F | H | H |
| 1-229 | H | H | CH₂-(1-CH₂CH₂CO₂Et-3-Pyza) | 2-F | H | H |
| 1-230 | Ac | H | CH₂-(1-CH₂CH₂CO₂Et-3-Pyza) | 2-F | H | H |
| 1-231 | H | H | CH₂-(4-CH₂CH₂CO₂H-3-Pya) | 2-F | H | H |
| 1-232 | Ac | H | CH₂-(4-CH₂CH₂CO₂H-3-Pyza) | 2-F | H | H |
| 1-233 | H | H | CH₂-(4-CH₂CH₂CO₂Me-3-Pyza) | 2-F | H | H |
| 1-234 | Ac | H | CH₂-(4-CH₂CH₂CO₂Me-3-Pyza) | 2-F | H | H |
| 1-235 | H | H | CH₂-(4-CH₂CH₂CO₂Et-3-Pyza) | 2-F | H | H |
| 1-236 | Ac | H | CH₂-(4-CH₂CH₂CO₂Et-3-Pyza) | 2-F | H | H |
| 1-237 | H | H | CH₂-(5-CH₂CH₂CO₂H-3-Pyza) | 2-F | H | H |
| 1-238 | Ac | H | CH₂-(5-CH₂CH₂CO₂H-3-Pyza) | 2-F | H | H |
| 1-239 | H | H | CH₂-(5-CH₂CH₂CO₂Me-3-Pyza) | 2-F | H | H |
| 1-240 | Ac | H | CH₂-(5-CH₂CH₂CO₂Me-3-Pyza) | 2-F | H | H |
| 1-241 | H | H | CH₂-(5-CH₂CH₂CO₂Et-3-Pyza) | 2-F | H | H |
| 1-242 | Ac | H | CH₂-(5-CH₂CH₂CO₂Et-3-Pyza) | 2-F | H | H |
| 1-245 | H | H | CH₂-(1-CH₂CH₂CH₂CO₂H-3-Pyza) | 2-F | H | H |
| 1-246 | Ac | H | CH₂-(1-CH₂CH₂CH₂CO₂H-3-Pyza) | 2-F | H | H |
| 1-247 | H | H | CH₂-(1-CH₂CH₂CH₂CO₂Me-3-Pyza) | 2-F | H | H |
| 1-248 | Ac | H | CH₂-(1-CH₂CH₂CH₂CO₂Me-3-Pyza) | 2-F | H | H |
| 1-249 | H | H | CH₂-(1-CH₂CH₂CH₂CO₂Et-3-Pyza) | 2-F | H | H |
| 1-250 | Ac | H | CH₂-(1-CH₂CH₂CH₂CO₂Et-3-Pyza) | 2-F | H | H |
| 1-251 | H | H | CH₂-(4-CH₂CH₂CH₂CO₂H-3-Pyza) | 2-F | H | H |
| 1-252 | Ac | H | CH₂-(4-CH₂CH₂CH₂CO₂H-3-Pyza) | 2-F | H | H |
| 1-253 | H | H | CH₂-(4-CH₂CH₂CH₂CO₂Me-3-Pyza) | 2-F | H | H |
| 1-254 | Ac | H | CH₂-(4-CH₂CH₂CH₂CO₂Me-3-Pyza) | 2-F | H | H |
| 1-255 | H | H | CH₂-(4-CH₂CH₂CH₂CO₂Et-3-Pyza) | 2-F | H | H |
| 1-256 | Ac | H | CH₂-(4-CH₂CH₂CH₂CO₂Et-3-Pyza) | 2-F | H | H |
| 1-257 | H | H | CH₂-(5-CH₂CH₂CH₂CO₂H-3-Pyza) | 2-F | H | H |
| 1-258 | Ac | H | CH₂-(5-CH₂CH₂CH₂CO₂H-3-Pyza) | 2-F | H | H |
| 1-259 | H | H | CH₂-(5-CH₂CH₂CH₂CO₂Me-3-Pyza) | 2-F | H | H |
| 1-260 | Ac | H | CH₂-(5-CH₂CH₂CH₂CO₂Me-3-Pyza) | 2-F | H | H |
| 1-261 | H | H | CH₂-(5-CH₂CH₂CH₂CO₂Et-3-Pyza) | 2-F | H | H |
| 1-262 | Ac | H | CH₂-(5-CH₂CH₂CH₂CO₂Et-3-Pyza) | 2-F | H | H |
| 1-263 | H | H | CH₂CH₂-3-Pyza | 2-F | H | H |
| 1-264 | Ac | H | CH₂CH₂-3-Pyza | 2-F | H | H |
| 1-265 | H | H | CH₂CH₂-3-Pyza | 2-F | 4-F | H |
| 1-266 | Ac | H | CH₂CH₂-3-Pyza | 2-F | 4-F | H |
| 1-267 | H | H | CH₂CH₂-(1-CO₂Me-3-Pyza) | 2-F | H | H |
| 1-268 | Ac | H | CH₂CH₂-(1-CO₂Me-3-Pyza) | 2-F | H | H |
| 1-269 | H | H | CH₂CH₂-(1-CO₂Et-3-Pyza) | 2-F | H | H |
| 1-270 | Ac | H | CH₂CH₂-(1-CO₂Et-3-Pyza) | 2-F | H | H |
| 1-271 | H | H | CH₂CH₂-(4-CO₂H-3-Pyza) | 2-F | H | H |
| 1-272 | Ac | H | CH₂CH₂-(4-CO₂H-3-Pyza) | 2-F | H | H |
| 1-273 | H | H | CH₂CH₂-(4-CO₂Me-3-Pyza) | 2-F | H | H |
| 1-274 | Ac | H | CH₂CH₂-(4-CO₂Me-3-Pyza) | 2-F | H | H |
| 1-275 | H | H | CH₂CH₂-(4-CO₂Et-3-Pyza) | 2-F | H | H |
| 1-276 | Ac | H | CH₂CH₂-(4-CO₂Et-3-Pyza) | 2-F | H | H |
| 1-277 | H | H | CH₂CH₂-(5-CO₂H-3-Pyza) | 2-F | H | H |
| 1-278 | Ac | H | CH₂CH₂-(5-CO₂H-3-Pyza) | 2-F | H | H |
| 1-279 | H | H | CH₂CH₂-(5-CO₂Me-3-Pyza) | 2-F | H | H |
| 1-280 | Ac | H | CH₂CH₂-(5-CO₂Me-3-Pyza) | 2-F | H | H |
| 1-281 | H | H | CH₂CH₂-(5-CO₂Et-3-Pyza) | 2-F | H | H |
| 1-282 | Ac | H | CH₂CH₂-(5-CO₂Et-3-Pyza) | 2-F | H | H |
| 1-283 | H | H | CH₂CH₂-(1-CH₂CO₂H-3-Pyza) | 2-F | H | H |
| 1-284 | Ac | H | CH₂CH₂-(1-CH₂CO₂H-3-Pyza) | 2-F | H | H |
| 1-285 | H | H | CH₂CH₂-(1-CH₂CO₂Me-3-Pyza) | 2-F | H | H |
| 1-286 | Ac | H | CH₂CH₂-(1-CH₂CO₂Me-3-Pyza) | 2-F | H | H |
| 1-287 | H | H | CH₂CH₂-(1-CH₂CO₂Et-3-Pyza) | 2-F | H | H |
| 1-288 | Ac | H | CH₂CH₂-(1-CH₂CO₂Et-3-Pyza) | 2-F | H | H |
| 1-289 | H | H | CH₂CH₂-(4-CH₂CO₂H-3-Pyza) | 2-F | H | H |
| 1-290 | Ac | H | CH₂CH₂-(4-CH₂CO₂H-3-Pyza) | 2-F | H | H |
| 1-291 | H | H | CH₂CH₂-(4-CH₂CO₂Me-3-Pyza) | 2-F | H | H |
| 1-292 | Ac | H | CH₂CH₂-(4-CH₂CO₂Me-3-Pyza) | 2-F | H | H |
| 1-293 | H | H | CH₂CH₂-(4-CH₂CO₂Et-3-Pyza) | 2-F | H | H |
| 1-294 | Ac | H | CH₂CH₂-(4-CH₂CO₂Et-3-Pyza) | 2-F | H | H |
| 1-295 | H | H | CH₂CH₂-(5-CH₂CO₂H-3-Pyza) | 2-F | H | H |
| 1-296 | Ac | H | CH₂CH₂-(5-CH₂CO₂H-3-Pyza) | 2-F | H | H |
| 1-297 | H | H | CH₂CH₂-(5-CH₂CO₂Me-3-Pyza) | 2-F | H | H |
| 1-298 | Ac | H | CH₂CH₂-(5-CH₂CO₂Me-3-Pyza) | 2-F | H | H |
| 1-299 | H | H | CH₂CH₂-(5-CH₂CO₂Et-3-Pyza) | 2-F | H | H |
| 1-300 | Ac | H | CH₂CH₂-(5-CH₂CO₂Et-3-Pyza) | 2-F | H | H |
| 1-301 | H | H | CH₂CH₂-(1-CH₂CH₂CO₂H-3-Pyza) | 2-F | H | H |
| 1-302 | Ac | H | CH₂CH₂-(1-CH₂CH₂CO₂H-3-Pyza) | 2-F | H | H |
| 1-303 | H | H | CH₂CH₂-(1-CH₂CH₂CO₂Me-3-Pyza) | 2-F | H | H |
| 1-304 | Ac | H | CH₂CH₂-(1-CH₂CH₂CO₂Me-3-Pyza | 2-F | H | H |
| 1-305 | H | H | CH₂CH₂-(1-CH₂CH₂CO₂Et-3-Pyza) | 2-F | H | H |
| 1-306 | Ac | H | CH₂CH₂-(1-CH₂CH₂CO₂Et-3-Pyza) | 2-F | H | H |
| 1-307 | H | H | CH₂CH₂(4-CH₂CH₂CO₂H-3-Pyza) | 2-F | H | H |
| 1-308 | Ac | H | CH₂CH₂-(4-CH₂CH₂CO₂H-3-Pyza) | 2-F | H | H |
| 1-309 | H | H | CH₂CH₂-(4-CH₂CH₂CO₂Me-3-Pyza) | 2-F | H | H |
| 1-310 | Ac | H | CH₂CH₂-(4-CH₂CH₂CO₂Me-3-Pyza | 2-F | H | H |
| 1-311 | H | H | CH₂CH₂-(4-CH₂CH₂CO₂Et-3-Pyza) | 2-F | H | H |
| 1-312 | Ac | H | CH₂CH₂-(4-CH₂CH₂CO₂Et-3-Pyza) | 2-F | H | H |
| 1-313 | H | H | CH₂CH₂-(5-CH₂CH₂CO₂H-3-Pyza) | 2-F | H | H |
| 1-314 | Ac | H | CH₂CH₂-(5-CH₂CH₂CO₂H-3-Pyza) | 2-F | H | H |
| 1-315 | H | H | CH₂CH₂-(5-CH₂CH₂CO₂Me-3-Pyza) | 2-F | H | H |
| 1-316 | Ac | H | CH₂CH₂-(5-CH₂CH₂CO₂Me-3-Pyza) | 2-F | H | H |
| 1-317 | H | H | CH₂CH₂-(5-CH₂CH₂CO₂Et-3-Pyza) | 2-F | H | H |
| 1-318 | Ac | H | CH₂CH₂-(5-CH₂CH₂CO₂Et-3-Pyza) | 2-F | H | H |
| 1-319 | H | H | CH₂CH₂(-CH₂CH₂CH₂CO₂H-3-Pyza) | 2-F | H | H |
| 1-320 | Ac | H | CH₂CH₂-(1-CH₂CH₂CH₂CO₂H-3-Pyza) | 2-F | H | H |
| 1-321 | H | H | CH₂CH₂-(1-CH₂CH₂CH₂CO₂Me-3-Pyza) | 2-F | H | H |
| 1-322 | Ac | H | CH₂CH₂-(1-CH₂CH₂CH₂CO₂Me-3-Pyza) | 2-F | H | H |
| 1-323 | H | H | CH₂CH₂-(-CH₂CH₂CH₂CO₂Et-3-Pyza) | 2-F | H | H |
| 1-324 | Ac | H | CH₂CH₂-(1-CH₂CH₂CH₂CO₂Et-3-Pyza) | 2-F | H | H |
| 1-325 | H | H | CH₂CH₂-(4-CH₂CH₂CH₂CO₂H-3-Pyza) | 2-F | H | H |
| 1-326 | Ac | H | CH₂CH₂-(4-CH₂CH₂CH₂CO₂H-3-Pyza) | 2-F | H | H |
| 1-327 | H | H | CH₂CH₂-(4-CH₂CH₂CH₂CO₂Me-3-Pyza) | 2-F | H | H |
| 1-328 | Ac | H | CH₂CH₂-(4-CH₂CH₂CH₂CO₂Me-3-Pyza) | 2-F | H | H |
| 1-329 | H | H | CH₂CH₂-(4-CH₂CH₂CH₂CO₂Et-3-Pyza) | 2-F | H | H |
| 1-330 | Ac | H | CH₂CH₂-(4-CH₂CH₂CH₂CO₂Et-3-Pyza) | 2-F | H | H |
| 1-331 | H | H | CH₂CH₂-(5-CH₂CH₂CH₂CO₂H-3-Pyza) | 2-F | H | H |
| 1-332 | Ac | H | CH₂CH₂-(5-CH₂CH₂CH₂CO₂H-3-Pyza) | 2-F | H | H |
| 1-333 | H | H | CH₂CH₂-(5-CH₂CH₂CH₂CO₂Me-3-Pyza) | 2-F | H | H |
| 1-334 | Ac | H | CH₂CH₂-(5-CH₂CH₂CH₂CO₂Me-3-Pyza) | 2-F | H | H |
| 1-335 | H | H | CH₂CH₂-(5-CH₂CH₂CH₂CO₂Et-3-Pyza) | 2-F | H | H |
| 1-336 | Ac | H | CH₂CH₂-(5-CH₂CH₂CH₂CO₂Et-3-Pyza) | 2-F | H | H |
| 1-337 | H | H | CH₂-4-Pyza | 2-F | H | H |
| 1-338 | Ac | H | CH₂-4-Pyza | 2-F | H | H |
| 1-339 | H | H | CH₂-4-Pyza | 2-F | 4-F | H |
| 1-340 | Ac | H | CH₂-4-Pyza | 2-F | 4-F | H |
| 1-341 | H | H | CH₂-(1-CO₂Me-4-Pyza) | 2-F | H | H |
| 1-342 | Ac | H | CH₂-(1-CO₂Me-4-Pyza) | 2-F | H | H |
| 1-343 | H | H | CH₂-(1-CO₂Et-4-Pyza) | 2-F | H | H |
| 1-344 | Ac | H | CH₂-(1-CO₂Et-4-Pyza) | 2-F | H | H |
| 1-345 | H | H | CH₂-(3-CO₂H-4-Pyza) | 2-F | H | H |
| 1-346 | Ac | H | CH₂-(3-CO₂H-4-Pyza) | 2-F | H | H |
| 1-347 | H | H | CH₂-(3-CO₂Me-4-Pyza) | 2-F | H | H |
| 1-348 | Ac | H | CH₂-(3-CO₂Me-4-Pyza) | 2-F | H | H |
| 1-349 | H | H | CH₂-(3-CO₂Et-4-Pyza) | 2-F | H | H |
| 1-350 | Ac | H | CH₂-(3-CO₂Et-4-Pyza) | 2-F | H | H |
| 1-351 | H | H | CH₂-(1-CH₂CO₂H-4-Pyza) | 2-F | H | H |
| 1-352 | Ac | H | CH₂-(1-CH₂CO₂H-4-Pyza) | 2-F | H | H |
| 1-353 | H | H | CH₂-(1-CH₂CO₂Me-4-Pyza) | 2-F | H | H |
| 1-354 | Ac | H | CH₂-(1-CH₂CO₂Me-4-Pyza) | 2-F | H | H |
| 1-355 | H | H | CH₂-(1-CH₂CO₂Et-4-Pyza) | 2-F | H | H |
| 1-356 | Ac | H | CH₂-(1-CH₂CO₂Et-4-Pyza) | 2-F | H | H |
| 1-357 | H | H | CH₂-(3-CH₂CO₂H-4-Pyza) | 2-F | H | H |
| 1-358 | Ac | H | CH₂-(3-CH₂CO₂H-4-Pyza) | 2-F | H | H |
| 1-359 | H | H | CH₂-(3-CH₂CO₂Me-4-Pyza) | 2-F | H | H |
| 1-360 | Ac | H | CH₂-(3-CH₂CO₂Me-4-Py | 2-F | H | H |
| 1-361 | H | H | CH₂-(3-CH₂CO₂Et-4-Pyza) | 2-F | H | H |
| 1-362 | Ac | H | CH₂-(3-CH₂CO₂Et-4-Pyza) | 2-F | H | H |
| 1-363 | H | H | CH₂-(1-CH₂CH₂CO₂H-4-Pyza) | 2-F | H | H |
| 1-364 | Ac | H | CH₂-(1-CH₂CH₂CO₂H-4-Pyza) | 2-F | H | H |
| 1-365 | H | H | CH₂-(1-CH₂CH₂CO₂Me-4-Pyza) | 2-F | H | H |
| 1-366 | Ac | H | CH₂-(1-CH₂CH₂CO₂Me-4-Pyza) | 2-F | H | H |
| 1-367 | H | H | CH₂-(1-CH₂CH₂CO₂Et-4-Pyza) | 2-F | H | H |
| 1-368 | Ac | H | CH₂-(1-CH₂CH₂CO₂Et-4-Pyza) | 2-F | H | H |
| 1-369 | H | H | CH₂-(3-CH₂CH₂CO₂H-4-Pyza) | 2-F | H | H |
| 1-370 | Ac | H | CH₂-(3-CH₂CH₂CO₂H-4-Pyza) | 2-F | H | H |
| 1-371 | H | H | CH₂-(3-CH₂CH₂CO₂Me-4-Pyza) | 2-F | H | H |
| 1-372 | Ac | H | CH₂-(3-CH₂CH₂CO₂Me-4-Pyza) | 2-F | H | H |
| 1-373 | H | H | CH₂-(3-CH₂CH₂CO₂Et-4-Pyza) | 2-F | H | H |
| 1-374 | Ac | H | CH₂-(3-CH₂CH₂CO₂Et-4-Pyza) | 2-F | H | H |
| 1-375 | H | H | CH₂-(1-CH₂CH₂CH₂CO₂H-4-Pyza) | 2-F | H | H |
| 1-376 | Ac | H | CH₂-(1-CH₂CH₂CH₂CO₂H-4-Pyza) | 2-F | H | H |
| 1-377 | H | H | CH₂-(1-CH₂CH₂CH₂CO₂Me-4-Pyza) | 2-F | H | H |
| 1-378 | Ac | H | CH₂-(1-CH₂CH₂CH₂CO₂Me-4-Pyza) | 2-F | H | H |
| 1-379 | H | H | CH₂-(1-CH₂CH₂CH₂CO₂Et-4-Pyza) | 2-F | H | H |
| 1-380 | Ac | H | CH₂-( -CH₂CH₂CH₂CO₂Et-4-Pyza) | 2-F | H | H |
| 1-381 | H | H | CH₂-(3-CH₂CH₂CH₂CO₂H-4-Pyza) | 2-F | H | H |
| 1-382 | Ac | H | CH₂-(3-CH₂CH₂CH₂CO₂H-4-Pyza) | 2-F | H | H |
| 1-383 | H | H | CH₂-(3-CH₂CH₂CH₂CO₂Me-4-Pyza) | 2-F | H | H |
| 1-384 | Ac | H | CH₂-(3-CH₂CH₂CH₂CO₂Me-4-Pyza) | 2-F | H | H |
| 1-385 | H | H | CH₂-(3-CH₂CH₂CH₂CO₂Et-4-Pyza) | 2-F | H | H |
| 1-386 | Ac | H | CH₂-(3-CH₂CH₂CH₂CO₂Et-4-Pyza) | 2-F | H | H |
| 1-387 | H | H | CH₂CH₂-4-Pyza | 2-F | H | H |
| 1-388 | Ac | H | CH₂CH₂-4-Pyza | 2-F | H | H |
| 1-389 | H | H | CH₂CH₂-4-Pyza | 2-F | 4-F | H |
| 1-390 | Ac | H | CH₂CH₂-4-Pyza | 2-F | 4-F | H |
| 1-391 | H | H | CH₂CH₂(1-CO₂Me-4-Pyza) | 2-F | H | H |
| 1-392 | Ac | H | CH₂CH₂-(1-CO₂Me-4-Pyza) | 2-F | H | H |
| 1-393 | H | H | CH₂CH₂-(1-CO₂Et-4-Pyza) | 2-F | H | H |
| 1-394 | Ac | H | CH₂CH₂-(1-CO₂Et-4-Pyza) | 2-F | H | H |
| 1-395 | H | H | CH₂CH₂-(3-CO₂H-4-Pyza) | 2-F | H | H |
| 1-396 | Ac | H | CH₂CH₂-(3-CO₂H-4-Pyza) | 2-F | H | H |
| 1-397 | H | H | CH₂CH₂-(3-CO₂Me-4-Pyza) | 2-F | H | H |
| 1-398 | Ac | H | CH₂CH₂-(3-CO₂Me-4-Pyza | 2-F | H | H |
| 1-399 | H | H | CH₂CH₂-(3-CO₂Et-4-Pyza) | 2-F | H | H |
| 1-400 | Ac | H | CH₂CH₂-(3-CO₂Et-4-Pyza) | 2-F | H | H |
| 1-401 | H | H | CH₂CH₂-(1-CH₂CO₂H-4-Pyza) | 2-F | H | H |
| 1-402 | Ac | H | CH₂CH₂-(1-CH₂CO₂H-4-Pyza) | 2-F | H | H |
| 1-403 | H | H | CH₂CH₂-(1-CH₂CO₂Me-4-Pyza) | 2-F | H | H |
| 1-404 | Ac | H | CH₂CH₂-(1-CH₂CO₂Me-4-Pyza) | 2-F | H | H |
| 1-405 | H | H | CH₂CH₂-(1-CH₂CO₂Et-4-Pyza) | 2-F | H | H |
| 1-406 | Ac | H | CH₂CH₂-(1-CH₂CO₂Et-4-Pyza) | 2-F | H | H |
| 1-407 | H | H | CH₂CH₂-(3-CH₂CO₂H-4-Pyza) | 2-F | H | H |
| 1-408 | Ac | H | CH₂CH₂-(3-CH₂CO₂H-4-Pyza) | 2-F | H | H |
| 1-409 | H | H | CH₂CH₂-(3-CH₂CO₂Me-4-Pyza) | 2-F | H | H |
| 1-410 | Ac | H | CH₂CH₂-(3-CH₂CO₂Me-4-Pyza) | 2-F | H | H |
| 1-411 | H | H | CH₂CH₂-(3-CH₂CO₂Et-4-Pyza) | 2-F | H | H |
| 1-412 | Ac | H | CH₂CH₂-(3-CH₂CO₂Et-4-Pyza) | 2-F | H | H |
| 1-413 | H | H | CH₂CH₂-(1-CH₂CH₂CO₂H-4-Pyza) | 2-F | H | H |
| 1-414 | Ac | H | CH₂CH₂-(1-CH₂CH₂CO₂H-4-Pyza) | 2-F | H | H |
| 1-415 | H | H | CH₂CH₂-(1-CH₂CH₂CO₂Me-4-Pyza) | 2-F | H | H |
| 1-416 | Ac | H | CH₂CH₂-(1-CH₂CH₂CO₂Me-4-Pyza) | 2-F | H | H |
| 1-417 | H | H | CH₂CH₂-(1-CH₂CH₂CO₂Et-4-Pyza) | 2-F | H | H |
| 1-418 | Ac | H | CH₂CH₂-(1-CH₂CH₂CO₂Et-4-Pyza) | 2-F | H | H |
| 1-419 | H | H | CH₂CH₂-(3-CH₂CH₂CO₂H-4-Pyza) | 2-F | H | H |
| 1-420 | Ac | H | CH₂CH₂-(3-CH₂CH₂CO₂H-4-Pyza) | 2-F | H | H |
| 1-421 | H | H | CH₂CH₂-(3-CH₂CH₂CO₂Me-4-Pyza) | 2-F | H | H |
| 1-422 | Ac | H | CH₂CH₂-(3-CH₂CH₂CO₂Me-4-Pyza) | 2-F | H | H |
| 1-423 | H | H | CH₂CH₂-(3-CH₂CH₂CO₂Et-4-Pyza) | 2-F | H | H |
| 1-424 | Ac | H | CH₂CH₂-(3-CH₂CH₂CO₂Et-4-Pyza) | 2-F | H | H |
| 1-425 | H | H | CH₂CH₂-(1-CH₂CH₂CH₂CO₂H-4-Pyza) | 2-F | H | H |
| 1-426 | Ac | H | CH₂CH₂-(1-CH₂CH₂CH₂CO₂H-4-Pyza) | 2-F | H | H |
| 1-427 | H | H | CH₂CH₂-(1-CH₂CH₂CH₂CO₂Me-4-Pyza) | 2-F | H | H |
| 1-428 | Ac | H | CH₂CH₂-(1-CH₂CH₂CH₂CO₂Me-4-Pyza) | 2-F | H | H |
| 1-429 | H | H | CH₂CH₂-(1-CH₂CH₂CH₂CO₂Et-4-Pyza) | 2-F | H | H |
| 1-430 | Ac | H | CH₂CH₂-( 1-CH₂CH₂CH₂CO₂Et-4-Pyza) | 2-F | H | H |
| 1-431 | H | H | CH₂CH₂-(3-CH₂CH₂CH₂CO₂H-4-Pyza) | 2-F | H | H |
| 1-432 | Ac | H | CH₂CH₂-(3-CH₂CH₂CH₂CO₂H-4-Pyza) | 2-F | H | H |
| 1-433 | H | H | CH₂CH₂-(3-CH₂CH₂CH₂CO₂Me-4-Pyza) | 2-F | H | H |
| 1-434 | Ac | H | CH₂CH₂-(3-CH₂CH₂CH₂CO₂Me-4-Pyza) | 2-F | H | H |
| 1-435 | H | H | CH₂CH₂-(3-CH₂CH₂CH₂CO₂Et-4-Pyza) | 2-F | H | H |
| 1-436 | Ac | H | CH₂CH₂-(3-CH₂CH₂CH₂CO₂Et-4-Pyza) | 2-F | H | H |
| 1-437 | H | H | CH₂-(1-CO₂Me-5-Pyza) | 2-F | 4-F | H |
| 1-438 | Ac | H | CH₂-(1-CO₂Me-5-Pyza) | 2-F | 4-F | H |
| 1-439 | H | H | CH₂-(1-CO₂Me-5-Pyza) | 2-F | H | H |
| 1-440 | Ac | H | CH₂-(1-CO₂Me-5-Pyza) | 2-F | H | H |
| 1-441 | H | H | CH₂-(1-CO₂Et-5-Pyza) | 2-F | H | H |
| 1-442 | Ac | H | CH₂-(1-CO₂Et-5-Pyza) | 2-F | H | H |
| 1-443 | H | H | CH₂-(1-CH₂CO₂H-5-Pyza) | 2-F | H | H |
| 1-444 | Ac | H | CH₂-(1-CH₂CO₂H-5-Pyza) | 2-F | H | H |
| 1-445 | H | H | CH₂-(1-CH₂CO₂Me-5-Pyza) | 2-F | H | H |
| 1-446 | Ac | H | CH₂-(1-CH₂CO₂Me-5-Pyza) | 2-F | H | H |
| 1-447 | H | H | CH₂-(1-CH₂CO₂Et-5-Pyza) | 2-F | H | H |
| 1-448 | Ac | H | CH₂-(1-CH₂CO₂Et-5-Pyza) | 2-F | H | H |
| 1-449 | H | H | CH₂-(1-CH₂CH₂CO₂H-5-Pyza) | 2-F | H | H |
| 1-450 | Ac | H | CH₂-(1-CH₂CH₂CO₂H-5-Pyza) | 2-F | H | H |
| 1-451 | H | H | CH₂-(1-CH₂CH₂CO₂Me-5-Pyza) | 2-F | H | H |
| 1-452 | Ac | H | CH₂-(1-CH₂CH₂CO₂Me-5-Pyza) | 2-F | H | H |
| 1-453 | H | H | CH₂-(1-CH₂CH₂CO₂Et-5-Pyza) | 2-F | H | H |
| 1-454 | Ac | H | CH₂-(1-CH₂CH₂CO₂Et-5-Pyza) | 2-F | H | H |
| 1-455 | H | H | CH₂-(1-CH₂CH₂CH₂CO₂H-5-Pyza) | 2-F | H | H |
| 1-456 | Ac | H | CH₂-(1-CH₂CH₂CH₂CO₂H-5-Pyza) | 2-F | H | H |
| 1-457 | H | H | CH₂-(1-CH₂CH₂CH₂CO₂Me-5-Pyza) | 2-F | H | H |
| 1-458 | Ac | H | CH₂-(1-CH₂CH₂CH₂CO₂Me-5-Pyza) | 2-F | H | H |
| 1-459 | H | H | CH₂-(1-CH₂CH₂CH₂CO₂Et-5-Pyza) | 2-F | H | H |
| 1-460 | Ac | H | CH₂-(1-CH₂CH₂CH₂CO₂Et-5-Pyza) | 2-F | H | H |
| 1-461 | H | H | CH₂CH₂-(1-CO₂Me-5-Pyza) | 2-F | 4-F | H |
| 1-462 | Ac | H | CH₂CH₂-(1-CO₂Me-5-Pyza) | 2-F | 4-F | H |
| 1-463 | H | H | CH₂CH₂-(1-CO₂Me-5-Pyza) | 2-F | H | H |
| 1-464 | Ac | H | CH₂CH₂-(1-CO₂Me-5-Pyza) | 2-F | H | H |
| 1-465 | H | H | CH₂CH₂-(1-CO₂Et-5-Pyza) | 2-F | H | H |
| 1-466 | Ac | H | CH₂CH₂-(1-CO₂Et-5-Pyza) | 2-F | H | H |
| 1-467 | H | H | CH₂CH₂-(-CH₂CO₂H-5-Pyza) | 2-F | H | H |
| 1-468 | Ac | H | CH₂CH₂-(1-CH₂CO₂H-5-Pyza) | 2-F | H | H |
| 1-469 | H | H | CH₂CH₂-(1-CH₂CO₂Me-5-Pyza) | 2-F | H | H |
| 1-470 | Ac | H | CH₂CH₂-(1-CH₂CO₂Me-5-Pyza) | 2-F | H | H |
| 1-471 | H | H | CH₂CH₂-(1-CH₂CO₂Et-5-Pyza) | 2-F | H | H |
| 1-472 | Ac | H | CH₂CH₂-(1-CH₂CO₂Et-5-Pyza) | 2-F | H | H |
| 1-473 | H | H | CH₂CH₂-(1-CH₂CH₂CO₂H-5-Pyza) | 2-F | H | H |
| 1-474 | Ac | H | CH₂CH₂-(1-CH₂CH₂CO₂H-5-Pyza) | 2-F | H | H |
| 1-475 | H | H | CH₂CH₂-(1-CH₂CH₂CO₂Me-5-Pyza) | 2-F | H | H |
| 1-476 | Ac | H | CH₂CH₂-(1-CH₂CH₂CO₂Me-5-Pyza) | 2-F | H | H |
| 1-477 | H | H | CH₂CH₂-(1-CH₂CH₂CO₂Et-5-Pyza) | 2-F | H | H |
| 1-478 | Ac | H | CH₂CH₂-(-CH₂CH₂CO₂Et-5-Pyza) | 2-F | H | H |
| 1-479 | H | H | CH₂CH₂-(-CH₂CH₂CH₂CO₂H-5-Pyza) | 2-F | H | H |
| 1-480 | Ac | H | CH₂CH₂-(1-CH₂CH₂CH₂CO₂H-5-Pyza) | 2-F | H | H |
| 1-481 | H | H | CH₂CH₂-(1-CH₂CH₂CH₂CO₂Me-5-Pyza) | 2-F | H | H |
| 1-482 | Ac | H | CH₂CH₂-(1-CH₂CH₂CH₂CO₂Me-5-Pyza) | 2-F | H | H |
| 1-483 | H | H | CH₂CH₂-(1-CH₂CH₂CH₂CO₂Et-5-Pyza) | 2-F | H | H |
| 1-484 | Ac | H | CH₂CH2-(1-CH₂CH₂CH₂CO₂Et-5-Pyza) | 2-F | H | H |
| 1-485 | H | H | CH₂-1-Triz | 2-F | H | H |
| 1-486 | Ac | H | CH₂-1-Triz | 2-F | H | H |
| 1-487 | H | H | CH₂-(4-CO₂H-1-Triz) | 2-F | H | H |
| 1-488 | Ac | H | CH₂-(4-CO₂H-1-Triz) | 2-F | H | H |
| 1-489 | H | H | CH₂-(4-CO₂Me-1-Triz) | 2-F | H | H |
| 1-490 | Ac | H | CH₂-(4-CO₂Me-1-Triz) | 2-F | H | H |
| 1-491 | H | H | CH₂-(4-CO₂Et-1-Triz) | 2-F | H | H |
| 1-492 | Ac | H | CH₂-(4-CO₂Et-1-Triz) | 2-F | H | H |
| 1-493 | H | H | CH₂-(5-CO₂H-1-Triz) | 2-F | H | H |
| 1-494 | Ac | H | CH₂-(5-CO₂H-1-Triz) | 2-F | H | H |
| 1-495 | H | H | CH₂-(5-CO₂Me-1-Triz) | 2-F | H | H |
| 1-496 | Ac | H | CH₂-(5-CO₂Me-1-Triz) | 2-F | H | H |
| 1-497 | H | H | CH₂-(5-CO₂Et-1-Triz) | 2-F | H | H |
| 1-498 | Ac | H | CH₂-(5-CO₂Et-1-Triz) | 2-F | H | H |
| 1-499 | H | H | CH₂-(4-CH₂CO₂H-1-Triz) | 2-F | H | H |
| 1-500 | Ac | H | CH₂-(4-CH₂CO₂H-1-Triz) | 2-F | H | H |
| 1-501 | H | H | CH₂-(4-CH₂CO₂Me-1-Triz) | 2-F | H | H |
| 1-502 | Ac | H | CH₂-(4-CH₂CO₂Me-1-Triz) | 2-F | H | H |
| 1-503 | H | H | CH₂-(4-CH₂CO₂Et-1-Triz) | 2-F | H | H |
| 1-504 | Ac | H | CH₂-(4-CH₂CO₂Et-1-Triz) | 2-F | H | H |
| 1-505 | H | H | CH₂-(5-CH₂CO₂H-1-Triz) | 2-F | H | H |
| 1-506 | Ac | H | CH₂-(5-CH₂CO₂H-1-Triz) | 2-F | H | H |
| 1-507 | H | H | CH₂-(5-CH₂CO₂Me-1-Triz) | 2-F | H | H |
| 1-508 | Ac | H | CH₂-(5-CH₂CO₂Me-1-Triz) | 2-F | H | H |
| 1-509 | H | H | CH₂-(5-CH₂CO₂Et-1-Triz) | 2-F | H | H |
| 1-510 | Ac | H | CH₂-(5-CH₂CO₂Et-1-Triz) | 2-F | H | H |
| 1-511 | H | H | CH₂-(4-CH₂CH₂CO₂H-1-Triz) | 2-F | H | H |
| 1-512 | Ac | H | CH₂-(4-CH₂CH₂CO₂H-1-Triz) | 2-F | H | H |
| 1-513 | H | H | CH₂-(4-CH₂CH₂CO₂Me-1-Triz) | 2-F | H | H |
| 1-514 | Ac | H | CH₂-(4-CH₂CH₂CO₂Me-1-Triz) | 2-F | H | H |
| 1-515 | H | H | CH₂-(4-CH₂CH₂CO₂Et-1-Triz) | 2-F | H | H |
| 1-516 | Ac | H | CH₂-(4-CH₂CH₂CO₂Et-1-Triz) | 2-F | H | H |
| 1-517 | H | H | CH₂-(5-CH₂CH₂CO₂H-1-Triz) | 2-F | H | H |
| 1-518 | Ac | H | CH₂-(5-CH₂CH₂CO₂H-1-Triz) | 2-F | H | H |
| 1-519 | H | H | CH₂-(5-CH₂CH₂CO₂Me-1-Triz) | 2-F | H | H |
| 1-520 | Ac | H | CH₂(5-CH₂CH₂CO₂Me-1-Triz) | 2-F | H | H |
| 1-521 | H | H | CH₂-(5-CH₂CH₂CO₂Et-1-Triz) | 2-F | H | H |
| 1-522 | Ac | H | CH₂-(5-CH₂CH₂CO₂Et-1-Triz) | 2-F | H | H |
| 1-523 | H | H | CH₂-(4-CH₂CH₂CH₂CO₂H-1-Triz) | 2-F | H | H |
| 1-524 | Ac | H | CH₂-(4-CH₂CH₂CH₂CO₂H-1-Triz) | 2-F | H | H |
| 1-525 | H | H | CH₂-(4-CH₂CH₂CH₂CO₂Me-1-Triz) | 2-F | H | H |
| 1-526 | Ac | H | CH₂-(4-CH₂CH₂CH₂CO₂Me-1-Triz) | 2-F | H | H |
| 1-527 | H | H | CH₂-(4-CH₂CH₂CH₂CO₂Et-1-Triz) | 2-F | H | H |
| 1-528 | Ac | H | CH₂-(4-CH₂CH₂CH₂CO₂Et-1-Triz) | 2-F | H | H |
| 1-529 | H | H | CH₂-(5-CH₂CH₂CH₂CO₂H-1-Triz) | 2-F | H | H |
| 1-530 | Ac | H | CH₂-(5-CH₂CH₂CH₂CO₂H-1-Triz) | 2-F | H | H |
| 1-531 | H | H | CH₂-(5-CH₂CH₂CH₂CO₂Me-1-Triz) | 2-F | H | H |
| 1-532 | Ac | H | CH₂-(5-CH₂CH₂CH₂CO₂Me-1-Triz) | 2-F | H | H |
| 1-533 | H | H | CH₂-(5-CH₂CH₂CH₂CO₂Et-1-Triz) | 2-F | H | H |
| 1-534 | Ac | H | CH₂-(5-CH₂CH₂CH₂CO₂Et-1-Triz) | 2-F | H | H |
| 1-535 | H | H | CH₂CH₂-1-Triz | 2-F | H | H |
| 1-356[TY1] | Ac | H | CH₂CH₂-1-Triz | 2-F | H | H |
| 1-537 | H | H | CH₂CH₂-(4-CO₂H-1-Triz) | 2-F | H | H |
| 1-538 | Ac | H | CH₂CH₂-(4-CO₂H-1-Triz) | 2-F | H | H |
| 1-539 | H | H | CH₂CH₂-(4-CO₂Me-1-Triz) | 2-F | H | H |
| 1-540 | Ac | H | CH₂CH₂-(4-CO₂Me-1-Triz) | 2-F | H | H |
| 1-541 | H | H | CH₂CH₂-(4-CO₂Et-1-Triz) | 2-F | H | H |
| 1-542 | Ac | H | CH₂CH₂-(4-CO₂Et-1-Triz) | 2-F | H | H |
| 1-543 | H | H | CH₂CH₂-(5-CO₂H-1-Triz) | 2-F | H | H |
| 1-544 | Ac | H | CH₂CH₂-(5-CO₂H-1-Triz) | 2-F | H | H |
| 1-545 | H | H | CH₂CH₂-(5-CO₂Me-1-Triz) | 2-F | H | H |
| 1-546 | Ac | H | CH₂CH₂-(5-CO₂Me-1-Triz) | 2-F | H | H |
| 1-547 | H | H | CH₂CH₂-(5-CO₂Et-1-Triz) | 2-F | H | H |
| 1-548 | Ac | H | CH₂CH₂-(5-CO₂Et-1-Triz) | 2-F | H | H |
| 1-549 | H | H | CH₂CH₂-(4-CH₂CO₂H-1-Triz) | 2-F | H | H |
| 1-550 | Ac | H | CH₂CH₂-(4-CH₂CO₂H-1-Triz) | 2-F | H | H |
| 1-551 | H | H | CH₂CH₂-(4-CH₂CO₂Me-1-Triz) | 2-F | H | H |
| 1-552 | Ac | H | CH₂CH₂-(4-CH₂CO₂Me-1-Triz) | 2-F | H | H |
| 1-553 | H | H | CH₂CH₂-(4-CH₂CO₂Et-1-Triz) | 2-F | H | H |
| 1-554 | Ac | H | CH₂CH₂-(4-CH₂CO₂Et-1-Triz) | 2-F | H | H |
| 1-555 | H | H | CH₂CH₂-(5-CH₂CO₂H-1-Triz) | 2-F | H | H |
| 1-556 | Ac | H | CH₂CH₂-(5-CH₂CO₂H-1-Triz) | 2-F | H | H |
| 1-557 | H | H | CH₂CH₂-(5-CH₂CO₂Me-1-Triz) | 2-F | H | H |
| 1-558 | Ac | H | CH₂CH₂-(5-CH₂CO₂Me-1-Triz) | 2-F | H | H |
| 1-559 | H | H | CH₂CH₂-(5-CH₂CO₂Et-1-Triz) | 2-F | H | H |
| 1-560 | Ac | H | CH₂CH₂-(5-CH₂CO₂Et-1-Triz) | 2-F | H | H |
| 1-561 | H | H | CH₂CH₂-(4-CH₂CH₂CO₂H-1-Triz) | 2-F | H | H |
| 1-562 | Ac | H | CH₂CH₂-(4-CH₂CH₂CO₂H-1-Triz) | 2-F | H | H |
| 1-563 | H | H | CH₂CH₂-(4-CH₂CH₂CO₂Me-1-Triz) | 2-F | H | H |
| 1-564 | Ac | H | CH₂CH₂-(4-CH₂CH₂CO₂Me-1-Triz) | 2-F | H | H |
| 1-565 | H | H | CH₂CH₂-(4-CH₂CH₂CO₂Et-1-Triz) | 2-F | H | H |
| 1-566 | Ac | H | CH₂CH₂-(4-CH₂CH₂CO₂Et-1-Triz) | 2-F | H | H |
| 1-567 | H | H | CH₂CH₂-(5-CH₂CH₂CO₂H-1-Triz) | 2-F | H | H |
| 1-568 | Ac | H | CH₂CH₂-(5-CH₂CH₂CO₂H-1-Triz) | 2-F | H | H |
| 1-569 | H | H | CH₂CH₂-(5-CH₂CH₂CO₂Me-1-Triz) | 2-F | H | H |
| 1-570 | Ac | H | CH₂CH₂-(5-CH₂CH₂CO₂Me-1-Triz) | 2-F | H | H |
| 1-571 | H | H | CH₂CH₂-(5-CH₂CH₂CO₂Et-1-Triz) | 2-F | H | H |
| 1-572 | Ac | H | CH₂CH₂-(5-CH₂CH_{z}CO₂Et-1-Triz) | 2-F | H | H |
| 1-573 | H | H | CH₂CH₂-(4-CH₂CH₂CH₂CO₂H-1-Triz) | 2-F | H | H |
| 1-574 | Ac | H | CH₂CH₂-(4-CH₂CH₂CH₂CO₂H-1-Triz) | 2-F | H | H |
| 1-575 | H | H | CH₂CH₂-(4-CH₂CH₂CH₂CO₂Me-1-Triz) | 2-F | H | H |
| 1-576 | Ac | H | CH₂CH₂-(4-CH₂CH₂CH₂CO₂Me-1-Triz) | 2-F | H | H |
| 1-577 | H | H | CH₂CH₂-(4-CH₂CH₂CH₂CO₂Et-1-Triz) | 2-F | H | H |
| 1-578 | Ac | H | CH₂CH₂-(4-CH₂CH₂CH₂CO₂Et-1-Triz) | 2-F | H | H |
| 1-579 | H | H | CH₂CH₂-(5-CH₂CH₂CH₂CO₂H-1-Triz) | 2-F | H | H |
| 1-580 | Ac | H | CH₂CH₂-(5 -CH₂CH₂CH₂CO₂H-1-Triz) | 2-F | H | H |
| 1-581 | H | H | CH₂CH₂-(5-CH₂CH₂CH₂CO₂Me-1-Triz) | 2-F | H | H |
| 1-582 | Ac | H | CH₂CH₂-(5-CH₂CH₂CH₂CO₂Me-1-Triz) | 2-F | H | H |
| 1-583 | H | H | CH₂CH₂(5-CH₂CH₂CH₂CO₂Et-1-Triz) | 2-F | H | H |
| 1-584 | Ac | H | CH₂CH₂-(5-CH₂CH₂CH₂CO₂Et-1-Triz) | 2-F | H | H |
| 1-585 | H | H | CH₂-2-Triz | 2-F | H | H |
| 1-586 | Ac | H | CH₂-2-Triz | 2-F | H | H |
| 1-587 | H | H | CH₂-(4-CO₂H-2-Triz) | 2-F | H | H |
| 1-588 | Ac | H | CH₂-(4-CO₂H-2-Triz) | 2-F | H | H |
| 1-589 | H | H | CH₂-(4-CO₂Me-2-Triz) | 2-F | H | H |
| 1-590 | Ac | H | CH₂-(4-CO₂Me-2-Triz) | 2-F | H | H |
| 1-591 | H | H | CH₂-(4-CO₂Et-2-Triz) | 2-F | H | H |
| 1-592 | Ac | H | CH₂-(4-CO₂Et-2-Triz) | 2-F | H | H |
| 1-593 | H | H | CH₂-(4-CH₂CO₂H-2-Triz) | 2-F | H | H |
| 1-594 | Ac | H | CH₂-(4-CH₂CO₂H-2-Triz) | 2-F | H | H |
| 1-595 | H | H | CH₂-(4-CH₂CO₂Me-2-Triz) | 2-F | H | H |
| 1-596 | Ac | H | CH₂-(4-CH₂CO₂Me-2-Triz) | 2-F | H | H |
| 1-597 | H | H | CH₂-(4-CH₂CO₂Et-2-Triz) | 2-F | H | H |
| 1-598 | Ac | H | CH₂-(4-CH₂CO₂Et-2-Triz) | 2-F | H | H |
| 1-599 | H | H | CH₂-(4-CH₂CH₂CO₂H-2-Triz) | 2-F | H | H |
| 1-600 | Ac | H | CH₂-(4-CH₂CH₂CO₂H-2-Triz) | 2-F | H | H |
| 1-601 | H | H | CH₂-(4-CH₂CH₂CO₂Me-2-Triz) | 2-F | H | H |
| 1-602 | Ac | H | CH₂-(4-CH₂CH₂CO₂Me-2-Triz) | 2-F | H | H |
| 1-603 | H | H | CH₂-(4-CH₂CH₂CO₂Et-2-Triz) | 2-F | H | H |
| 1-604 | Ac | H | CH₂-(4-CH₂CH₂CO₂Et-2-Triz) | 2-F | H | H |
| 1-605 | H | H | CH₂-(4-CH₂CH₂CH₂CO₂H-2-Triz) | 2-F | H | H |
| 1-606 | Ac | H | CH₂-(4-CH₂CH₂CH₂CO₂H-2-Triz) | 2-F | H | H |
| 1-607 | H | H | CH₂-(4-CH₂CH₂CH₂CO₂Me-2-Triz) | 2-F | H | H |
| 1-608 | Ac | H | CH₂-(4-CH₂CH₂CH₂CO₂Me-2-Triz) | 2-F | H | H |
| 1-609 | H | H | CH₂-(4-CH₂CH₂CH₂CO₂Et-2-Triz) | 2-F | H | H |
| 1-610 | Ac | H | CH₂-(4-CH₂CH₂CH₂CO₂Et-2-Triz) | 2-F | H | H |
| 1-611 | H | H | CH₂CH₂-2-Triz | 2-F | H | H |
| 1-612 | Ac | H | CH₂CH₂-2-Triz | 2-F | H | H |
| 1-613 | H | H | CH₂CH₂-(4-CO₂H-2-Triz) | 2-F | H | H |
| 1-614 | Ac | H | CH₂CH₂-(4-CO₂H-2-Triz) | 2-F | H | H |
| 1-615 | H | H | CH₂CH₂-(4-CO₂Me-2-Triz) | 2-F | H | H |
| 1-616 | Ac | H | CH₂CH₂-(4-CO₂Me-2-Triz) | 2-F | H | H |
| 1-617 | H | H | CH₂CH₂-(4-CO₂Et-2-Triz) | 2-F | H | H |
| 1-618 | Ac | H | CH₂CH₂-(4-CO₂Et-2-Triz) | 2-F | H | H |
| 1-619 | H | H | CH₂CH₂-(4-CH₂CO₂H-2-Triz) | 2-F | H | H |
| 1-620 | Ac | H | CH₂CH₂-(4-CH₂CO₂H-2-Triz) | 2-F | H | H |
| 1-621 | H | H | CH₂CH₂-(4-CH₂CO₂Me-2-Triz) | 2-F | H | H |
| 1-622 | Ac | H | CH₂CH₂-(4-CH₂CO₂Me-2-Triz) | 2-F | H | H |
| 1-623 | H | H | CH₂CH₂-(4-CH₂CO₂Et-2-Triz) | 2-F | H | H |
| 1-624 | Ac | H | CH₂CH₂-(4-CH₂CO₂Et-2-Triz) | 2-F | H | H |
| 1-625 | H | H | CH₂CH₂-(4-CH₂CH₂CO₂H-2-Triz) | 2-F | H | H |
| 1-626 | Ac | H | CH₂CH₂-(4-CH₂CH₂CO₂H-2-Triz) | 2-F | H | H |
| 1-627 | H | H | CH₂CH₂-(4-CH₂CH₂CO₂Me-2-Triz) | 2-F | H | H |
| 1-628 | Ac | H | CH₂CH₂-(4-CH₂CH₂CO₂Me-2-Triz) | 2-F | H | H |
| 1-629 | H | H | CH₂CH₂-(4-CH₂CH₂CO₂Et-2-Triz) | 2-F | H | H |
| 1-630 | Ac | H | CH₂CH₂-(4-CH₂CH₂CO₂Et-2-Triz) | 2-F | H | H |
| 1-631 | H | H | CH₂CH₂-(4-CH₂CH₂CH₂CO₂H-2-Triz) | 2-F | H | H |
| 1-632 | Ac | H | CH₂CH₂-(4-CH₂CH₂CH₂CO₂H-2-Triz) | 2-F | H | H |
| 1-633 | H | H | CH₂CH₂-(4-CH₂CH₂CH₂CO₂Me-2-Triz) | 2-F | H | H |
| 1-634 | Ac | H | CH₂CH₂-(4-CH₂CH₂CH₂CO₂Me-2-Triz) | 2-F | H | H |
| 1-635 | H | H | CH₂CH₂-(4-CH₂CH₂CH₂CO₂Et-2-Triz) | 2-F | H | H |
| 1-636 | Ac | H | CH₂CH₂-(4-CH₂CH₂CH₂CO₂Et-2-Triz) | 2-F | H | H |
| 1-637 | H | H | CH₂-4-Triz | 2-F | H | H |
| 1-638 | Ac | H | CH₂-4-Triz | 2-F | H | H |
| 1-639 | H | H | CH₂-4-Triz | 2-F | 4-F | H |
| 1-640 | Ac | H | CH₂-4-Triz | 2-F | 4-F | H |
| 1-641 | H | H | CH₂-(1-CO₂Me-4-Triz) | 2-F | H | H |
| 1-642 | Ac | H | CH₂-(1-CO₂Me-4-Triz) | 2-F | H | H |
| 1-643 | H | H | CH₂-(1-CO₂Et-4-Triz) | 2-F | H | H |
| 1-644 | Ac | H | CH₂-(1-CO₂Et-4-Triz) | 2-F | H | H |
| 1-645 | H | H | CH₂-(5-CO₂H-4-Triz) | 2-F | H | H |
| 1-646 | Ac | H | CH₂-(5-CO₂H-4-Triz) | 2-F | H | H |
| 1-647 | H | H | CH₂-(5-CO₂Me-4-Triz) | 2-F | H | H |
| 1-648 | Ac | H | CH₂-(5-CO₂Me-4-Triz) | 2-F | H | H |
| 1-649 | H | H | CH₂-(5-CO₂Et-4-Triz) | 2-F | H | H |
| 1-650 | Ac | H | CH₂-(5-CO₂Et-4-Triz) | 2-F | H | H |
| 1-651 | H | H | CH₂-(1-CH₂CO₂H-4-Triz) | 2-F | H | H |
| 1-652 | Ac | H | CH₂-(1-CH₂CO₂H-4-Triz) | 2-F | H | H |
| 1-653 | H | H | CH₂-(1-CH₂CO₂Me-4-Triz) | 2-F | H | H |
| 1-654 | Ac | H | CH₂-(1-CH₂CO₂Me-4-Triz) | 2-F | H | H |
| 1-655 | H | H | CH₂-(1-CH₂CO₂Et-4-Triz) | 2-F | H | H |
| 1-656 | Ac | H | CH₂-(1-CH₂CO₂Et-4-Triz) | 2-F | H | H |
| 1-657 | H | H | CH₂-(5-CH₂CO₂H-4-Triz) | 2-F | H | H |
| 1-658 | Ac | H | CH₂-(5-CH₂CO₂H-4-Triz) | 2-F | H | H |
| 1-659 | H | H | CH₂-(5-CH₂CO₂Me-4-Triz) | 2-F | H | H |
| 1-660 | Ac | H | CH₂-(5-CH₂CO₂Me-4-Triz) | 2-F | H | H |
| 1-661 | H | H | CH₂-(5-CH₂CO₂Et-4-Triz) | 2-F | H | H |
| 1-662 | Ac | H | CH₂-(5-CH₂CO₂Et-4-Triz) | 2-F | H | H |
| 1-663 | H | H | CH₂-(1-CH₂CH₂CO₂H-4-Triz) | 2-F | H | H |
| 1-664 | Ac | H | CH₂-(1-CH₂CH₂CO₂H-4-Triz) | 2-F | H | H |
| 1-665 | H | H | CH₂-(1-CH₂CH₂CO₂Me-4-Triz) | 2-F | H | H |
| 1-666 | Ac | H | CH₂-(1-CH₂CH₂CO₂Me-4-Triz) | 2-F | H | H |
| 1-667 | H | H | CH₂-(1-CH₂CH₂CO₂Et-4-Triz) | 2-F | H | H |
| 1-668 | Ac | H | CH₂-(1-CH₂CH₂CO₂Et-4-Triz) | 2-F | H | H |
| 1-669 | H | H | CH₂-(5-CH₂CH₂CO₂H-4-Triz) | 2-F | H | H |
| 1-670 | Ac | H | CH₂-(5-CH₂CH₂CO₂H-4-Triz) | 2-F | H | H |
| 1-671 | H | H | CH₂-(5-CH₂CH₂CO₂Me-4-Triz) | 2-F | H | H |
| 1-672 | Ac | H | CH₂-(5-CH₂CH₂CO₂Me-4-Triz) | 2-F | H | H |
| 1-673 | H | H | CH₂-(5-CH₂CH₂CO₂Et-4-Triz) | 2-F | H | H |
| 1-674 | Ac | H | CH₂-(5-CH₂CH₂CO₂Et-4-Triz) | 2-F | H | H |
| 1-675 | H | H | CH₂-(-CH₂CH₂CH₂CO₂H-4-Triz) | 2-F | H | H |
| 1-676 | Ac | H | CH₂-(-CH₂CH₂CH₂CO2H-4-Triz) | 2-F | H | H |
| 1-677 | H | H | CH₂-(1-CH₂CH₂CH₂CO₂Me-4-Triz) | 2-F | H | H |
| 1-678 | Ac | H | CH₂-(1-CH₂CH₂CH₂CO₂Me-4-Triz) | 2-F | H | H |
| 1-679 | H | H | CH₂-(1-CH₂CH₂CH₂CO₂Et-4-Triz) | 2-F | H | H |
| 1-680 | Ac | H | CH₂-(1-CH₂CH₂CH₂CO₂Et-4-Triz) | 2-F | H | H |
| 1-681 | H | H | CH₂-(5-CH₂CH₂CH₂CO₂H-4-Triz) | 2-F | H | H |
| 1-682 | Ac | H | CH₂-(5-CH₂CH₂CH₂CO₂H-4-Triz) | 2-F | H | H |
| 1-683 | H | H | CH₂-(5-CH₂CH₂CH₂CO₂Me-4-Triz) | 2-F | H | H |
| 1-684 | Ac | H | CH₂-(5-CH₂CH₂CH₂CO₂Me-4-Triz) | 2-F | H | H |
| 1-685 | H | H | CH₂-(5-CH₂CH₂CH₂CO₂Et-4-Triz) | 2-F | H | H |
| 1-686 | Ac | H | CH₂-(5-CH₂CH₂CH₂CO₂Et-4-Triz) | 2-F | H | H |
| 1-687 | H | H | CH₂CH₂-4-Triz | 2-F | H | H |
| 1-688 | Ac | H | CH₂CH₂-4-Triz | 2-F | H | H |
| 1-689 | H | H | CH₂CH₂-4-Triz | 2-F | 4-F | H |
| 1-690 | Ac | H | CH₂CH₂-4-Triz | 2-F | 4-F | H |
| 1-691 | H | H | CH₂CH₂-(1-CO₂Me-4-Triz) | 2-F | H | H |
| 1-692 | Ac | H | CH₂CH₂-(1-CO₂Me-4-Triz) | 2-F | H | H |
| 1-693 | H | H | CH₂CH₂-(1-CO₂Et-4-Triz) | 2-F | H | H |
| 1-694 | Ac | H | CH₂CH₂-(1-CO₂Et-4-Triz) | 2-F | H | H |
| 1-695 | H | H | CH₂CH₂-(5-CO₂H-4-Triz) | 2-F | H | H |
| 1-696 | Ac | H | CH₂CH₂-(5-CO₂H-4-Triz) | 2-F | H | H |
| 1-697 | H | H | CH₂CH₂-(5-CO₂Me-4-Triz) | 2-F | H | H |
| 1-698 | Ac | H | CH₂CH₂-(5-CO₂Me-4-Triz) | 2-F | H | H |
| 1-699 | H | H | CH₂CH₂-(5-CO₂Et-4-Triz) | 2-F | H | H |
| 1-700 | Ac | H | CH₂CH₂-(5-CO₂Et-4-Triz) | 2-F | H | H |
| 1-701 | H | H | CH₂CH₂-(1-CH₂CO₂H-4-Triz) | 2-F | H | H |
| 1-702 | Ac | H | CH₂CH₂-(1-CH₂CO₂H-4-Triz) | 2-F | H | H |
| 1-703 | H | H | CH₂CH₂-(1-CH₂CO₂Me-4-Triz) | 2-F | H | H |
| 1-704 | Ac | H | CH₂CH₂-(1-CH₂CO₂Me-4-Triz) | 2-F | H | H |
| 1-705 | H | H | CH₂CH₂-(1-CH₂CO₂Et-4-Triz) | 2-F | H | H |
| 1-706 | Ac | H | CH₂CH₂-(1-CH₂CO₂Et-4-Triz) | 2-F | H | H |
| 1-707 | H | H | CH₂CH₂-(5-CH₂CO₂H-4-Triz) | 2-F | H | H |
| 1-708 | Ac | H | CH₂CH₂-(5-CH₂CO₂H-4-Triz) | 2-F | H | H |
| 1-709 | H | H | CH₂CH₂-(5-CH₂CO₂Me-4-Triz) | 2-F | H | H |
| 1-710 | Ac | H | CH₂CH₂-(5-CH₂CO₂Me-4-Triz) | 2-F | H | H |
| 1-711 | H | H | CH₂CH₂-(5-CH₂CO₂Et-4-Triz) | 2-F | H | H |
| 1-712 | Ac | H | CH₂CH₂-(5-CH₂CO₂Et-4-Triz) | 2-F | H | H |
| 1-713 | H | H | CH₂CH₂-(1-CH₂CH₂CO₂H-4-Triz) | 2-F | H | H |
| 1-714 | Ac | H | CH₂CH₂-(1-CH₂CH₂CO₂H-4-Triz) | 2-F | H | H |
| 1-715 | H | H | CH₂CH₂-(1-CH₂CH₂CO₂Me-4-Triz) | 2-F | H | H |
| 1-716 | Ac | H | CH₂CH₂-(1-CH₂CH₂CO₂Me-4-Triz) | 2-F | H | H |
| 1-717 | H | H | CH₂CH₂-(1-CH₂CH₂CO₂Et-4-Triz) | 2-F | H | H |
| 1-718 | Ac | H | CH₂CH₂-(1-CH₂CH₂CO₂Et-4-Triz) | 2-F | H | H |
| 1-719 | H | H | CH₂CH₂-(5-CH₂CH₂CO₂H-4-Triz) | 2-F | H | H |
| 1-720 | Ac | H | CH₂CH₂-(5-CH₂CH₂CO₂H-4-Triz) | 2-F | H | H |
| 1-721 | H | H | CH₂CH₂-(5-CH₂CH₂CO₂Me-4-Triz) | 2-F | H | H |
| 1-722 | Ac | H | CH₂CH₂-(5-CH₂CH₂CO₂Me-4-Triz) | 2-F | H | H |
| 1-723 | H | H | CH₂CH₂-(5-CH₂CH₂CO₂Et-4-Triz) | 2-F | H | H |
| 1-724 | Ac | H | CH₂CH₂-(5-CH₂CH₂CO₂Et-4-Triz) | 2-F | H | H |
| 1-725 | H | H | CH₂CH₂-(1-CH₂CH₂CH₂CO₂H-4-Triz) | 2-F | H | H |
| 1-726 | Ac | H | CH₂CH₂-(1-CH₂CH₂CH₂CO₂H-4-Triz) | 2-F | H | H |
| 1-727 | H | H | CH₂CH₂-(1-CH₂CH₂CH₂CO₂Me-4-Triz) | 2-F | H | H |
| 1-728 | Ac | H | CH₂CH₂-(1-CH₂CH₂CH₂CO₂Me-4-Triz) | 2-F | H | H |
| 1-729 | H | H | CH₂CH₂-(1-CH₂CH₂CH₂CO₂Et-4-Triz) | 2-F | H | H |
| 1-730 | Ac | H | CH₂CH₂-(1-CH₂CH₂CH₂CO₂Et-4-Triz) | 2-F | H | H |
| 1-731 | H | H | CH₂CH₂-(5-CH₂CH₂CH₂CO₂H-4-Triz) | 2-F | H | H |
| 1-732 | Ac | H | CH₂CH₂-(5-CH₂CH₂CH₂CO₂H-4-Triz) | 2-F | H | H |
| 1-733 | H | H | CH₂CH₂-(5-CH₂CH₂CH₂CO₂Me-4-Triz) | 2-F | H | H |
| 1-734 | Ac | H | CH₂CH₂-(S-CH₂CH₂CH₂CO₂Me-4-Triz) | 2-F | H | H |
| 1-735 | H | H | CH₂CH₂-(5-CH₂CH₂CH₂CO₂Et-4-Triz) | 2-F | H | H |
| 1-736 | Ac | H | CH₂CH₂-(5-CH₂CH₂CH₂CO₂Et-4-Triz) | 2-F | H | H |
| 1-737 | H | H | CH₂-(1-CO₂Me-5-Triz) | 2-F | 4-F | H |
| 1-738 | Ac | H | CH₂-(1-CO₂Me-5-Triz) | 2-F | 4-F | H |
| 1-739 | H | H | CH₂-(1-CO₂Me-5-Triz) | 2-F | H | H |
| 1-740 | Ac | H | CH₂-(1-CO₂Me-5-Triz) | 2-F | H | H |
| 1-741 | H | H | CH₂-(1-CO₂Et-5-Triz) | 2-F | H | H |
| 1-742 | Ac | H | CH₂-(1-CO₂Et-5-Triz) | 2-F | H | H |
| 1-743 | H | H | CH₂-(1-CH₂CO₂H-5-Triz) | 2-F | H | H |
| 1-744 | Ac | H | CH₂-(1-CH₂CO₂H-5-Triz) | 2-F | H | H |
| 1-745 | H | H | CH₂-(1-CH₂CO₂Me-5-Triz) | 2-F | H | H |
| 1-746 | Ac | H | CH₂-(1-CH₂CO₂Me-5-Triz) | 2-F | H | H |
| 1-747 | H | H | CH₂-(1-CH₂CO₂Et-5-Triz) | 2-F | H | H |
| 1-748 | Ac | H | CH₂-(1-CH₂CO₂Et-5-Triz) | 2-F | H | H |
| 1-749 | H | H | CH₂-(1-CH₂CH₂CO₂H-5-Triz) | 2-F | H | H |
| 1-750 | Ac | H | CH₂-(1-CH₂CH₂CO₂H-5-Triz) | 2-F | H | H |
| 1-751 | H | H | CH₂-(1-CH₂CH₂CO₂Me-5-Triz) | 2-F | H | H |
| 1-752 | Ac | H | CH₂-(1-CH₂CH₂CO₂Me-5-Triz) | 2-F | H | H |
| 1-753 | H | H | CH₂-(1-CH₂CH₂CO₂Et-5-Triz) | 2-F | H | H |
| 1-754 | Ac | H | CH₂-(1-CH₂CH₂CO₂Et-5-Triz) | 2-F | H | H |
| 1-755 | H | H | CH₂-(1-CH₂CH₂CH₂CO₂H-5-Triz) | 2-F | H | H |
| 1-756 | Ac | H | CH₂-(1-CH₂CH₂CH₂CO₂H-5-Triz) | 2-F | H | H |
| 1-757 | H | H | CH₂-(1-CH₂CH₂CH₂CO₂Me-5-Triz) | 2-F | H | H |
| 1-758 | Ac | H | CH₂-(1-CH₂CH₂CH₂CO₂Me-5-Triz) | 2-F | H | H |
| 1-759 | H | H | CH₂-(1-CH₂CH₂CH₂CO₂Et-5-Triz) | 2-F | H | H |
| 1-760 | Ac | H | CH₂-(1-CH₂CH₂CH₂CO₂Et-5-Triz) | 2-F | H | H |
| 1-761 | H | H | CH₂-1-Tez | 2-F | H | H |
| 1-762 | Ac | H | CH₂-1-Tez | 2-F | H | H |
| 1-763 | H | H | CH₂-(5-CO₂H-1-Tez) | 2-F | H | H |
| 1-764 | Ac | H | CH₂-(5-CO₂H-1-Tez) | 2-F | H | H |
| 1-765 | H | H | CH₂-(5-CO₂Me-1-Tez) | 2-F | H | H |
| 1-766 | Ac | H | CH₂-(5-CO₂Me-1-Tez) | 2-F | H | H |
| 1-767 | H | H | CH₂-(5-CO₂Et-1-Tez) | 2-F | H | H |
| 1-768 | Ac | H | CH₂-(5-CO₂Et-1-Tez) | 2-F | H | H |
| 1-769 | H | H | CH₂-(5-CH₂CO₂H-1-Tez) | 2-F | H | H |
| 1-770 | Ac | H | CH₂-(5-CH₂CO₂H-1-Tez) | 2-F | H | H |
| 1-771 | H | H | CH₂-(5-CH₂CO₂Me-1-Tez) | 2-F | H | H |
| 1-772 | Ac | H | CH₂-(5-CH₂CO₂Me-1-Tez) | 2-F | H | H |
| 1-773 | H | H | CH₂-(5-CH₂CO₂Et-1-Tez) | 2-F | H | H |
| 1-774 | Ac | H | CH₂-(5-CH₂CO₂Et-1-Tez) | 2-F | H | H |
| 1-775 | H | H | CH₂-(5-CH₂CH₂CO₂H-1-Tez) | 2-F | H | H |
| 1-776 | Ac | H | CH₂-(5-CH₂CH₂CO₂H-1-Tez) | 2-F | H | H |
| 1-777 | H | H | CH₂-(5-CH₂CH₂CO₂Me-1-Tez) | 2-F | H | H |
| 1-778 | Ac | H | CH₂-(5-CH₂CH₂CO₂Me-1-Tez) | 2-F | H | H |
| 1-779 | H | H | CH₂-(5-CH₂CH₂CO₂Et-1-Tez) | 2-F | H | H |
| 1-780 | Ac | H | CH₂-(5-CH₂CH₂CO₂Et-1-Tez) | 2-F | H | H |
| 1-781 | H | H | CH₂-(5-CH₂CH₂CH₂CO₂H-1-Tez) | 2-F | H | H |
| 1-782 | Ac | H | CH₂-(5-CH₂CH₂CH₂CO₂H-1-Tez) | 2-F | H | H |
| 1-783 | H | H | CH₂-(5-CH₂CH₂CH₂CO₂Me-1-Tez) | 2-F | H | H |
| 1-784 | Ac | H | CH₂-(5-CH₂CH₂CH₂CO₂Me-1-Tez) | 2-F | H | H |
| 1-785 | H | H | CH₂-(5-CH₂CH₂CH₂CO₂Et-1-Tez) | 2-F | H | H |
| 1-786 | Ac | H | CH₂-(5-CH₂CH₂CH₂CO₂Et-1-Tez) | 2-F | H | H |
| 1-787 | H | H | CH₂CH₂-1-Tez | 2-F | H | H |
| 1-788 | Ac | H | CH₂CH₂-1-Tez | 2-F | H | H |
| 1-789 | H | H | CH₂CH₂-(5 -CO₂H-1-Tez) | 2-F | H | H |
| 1-790 | Ac | H | CH₂CH₂-(5-CO₂H-1-Tez) | 2-F | H | H |
| 1-791 | H | H | CH₂CH₂-(5-CO₂Me-1-Tez) | 2-F | H | H |
| 1-792 | Ac | H | CH₂CH₂-(5-CO₂Me-1-Tez) | 2-F | H | H |
| 1-793 | H | H | CH₂CH₂-(5-CO₂Et-1-Tez) | 2-F | H | H |
| 1-794 | Ac | H | CH₂CH₂-(5-CO₂Et-1-Tez) | 2-F | H | H |
| 1-795 | H | H | CH₂CH₂-(5-CH₂CO₂H-1-Tez) | 2-F | H | H |
| 1-796 | Ac | H | CH₂CH₂-(5-CH₂CO₂H-1-Tez) | 2-F | H | H |
| 1-797 | H | H | CH₂CH₂-(5-CH₂CO₂Me-1-Tez) | 2-F | H | H |
| 1-798 | Ac | H | CH₂CH₂-(5-CH₂CO₂Me-1-Tez) | 2-F | H | H |
| 1-799 | H | H | CH₂CH₂-(5-CH₂CO₂Et-1-Tez) | 2-F | H | H |
| 1-800 | Ac | H | CH₂CH₂-(5-CH₂CO₂Et-1-Tez) | 2-F | H | H |
| 1-801 | H | H | CH₂CH₂-(5-CH₂CH₂CO₂H-1-Tez) | 2-F | H | H |
| 1-802 | Ac | H | CH₂CH₂-(5-CH₂CH₂CO₂H-1-Tez) | 2-F | H | H |
| 1-803 | H | H | CH₂CH₂-(5-CH₂CH₂CO₂Me-1-Tez) | 2-F | H | H |
| 1-804 | Ac | H | CH₂CH₂(5-CH₂CH₂CO₂Me-1-Tez) | 2-F | H | H |
| 1-805 | H | H | CH₂CH₂-(5-CH₂CH₂CO₂Et-1-Tez) | 2-F | H | H |
| 1-806 | Ac | H | CH₂CH₂-(5-CH₂CH₂CO₂Et-1-Tez) | 2-F | H | H |
| 1-807 | H | H | CH₂CH₂-(5-CH₂CH₂CH₂CO₂H-1-Tez) | 2-F | H | H |
| 1-808 | Ac | H | CH₂CH₂-(5-CH₂CH₂CH₂CO₂H-1-Tez) | 2-F | H | H |
| 1-809 | H | H | CH₂CH₂-(5-CH₂CH₂CH₂CO₂Me-1-Tez) | 2-F | H | H |
| 1-810 | Ac | H | CH₂CH₂-(5-CH₂CH₂CH₂CO₂Me-1-Tez) | 2-F | H | H |
| 1-811 | H | H | CH₂CH₂-(5-CH₂CH₂CH₂CO₂Et-1-Tez) | 2-F | H | H |
| 1-812 | Ac | H | CH₂CH₂-(5-CH₂CH₂CH₂CO₂Et-1-Tez) | 2-F | H | H |
| 1-813 | H | H | CH₂-2-Tez | 2-F | H | H |
| 1-814 | Ac | H | CH₂-2-Tez | 2-F | H | H |
| 1-815 | H | H | CH₂-(5-CO₂H-2-Tez) | 2-F | H | H |
| 1-816 | Ac | H | CH₂-(5-CO₂H-2-Tez) | 2-F | H | H |
| 1-817 | H | H | CH₂-(5-CO₂Me-2-Tez) | 2-F | H | H |
| 1-818 | Ac | H | CH₂-(5-CO₂Me-2-Tez) | 2-F | H | H |
| 1-819 | H | H | CH₂-(5-CO₂Et-2-Tez) | 2-F | H | H |
| 1-820 | Ac | H | CH₂-(5-CO₂Et-2-Tez) | 2-F | H | H |
| 1-821 | H | H | CH₂-(5-CH₂CO₂H-2-Tez) | 2-F | H | H |
| 1-822 | Ac | H | CH₂-(5-CH₂CO₂H-2-Tez) | 2-F | H | H |
| 1-823 | H | H | CH₂-(5-CH₂CO₂Me-2-Tez) | 2-F | H | H |
| 1-824 | Ac | H | CH₂-(5-CH₂CO₂Me-2-Tez) | 2-F | H | H |
| 1-825 | H | H | CH₂-(5-CH₂CO₂Et-2-Tez) | 2-F | H | H |
| 1-826 | Ac | H | CH₂-(5-CH₂CO₂Et-2-Tez) | 2-F | H | H |
| 1-827 | H | H | CH₂-(5-CH₂CH₂CO₂H-2-Tez) | 2-F | H | H |
| 1-828 | Ac | H | CH₂-(5-CH₂CH₂CO₂H-2-Tez) | 2-F | H | H |
| 1-829 | H | H | CH₂-(5-CH₂CH₂CO₂Me-2-Tez) | 2-F | H | H |
| 1-830 | Ac | H | CH₂-(5-CH₂CH₂CO₂Me-2-Tez) | 2-F | H | H |
| 1-831 | H | H | CH₂-(5-CH₂CH₂CO₂Et-2-Tez) | 2-F | H | H |
| 1-832 | Ac | H | CH₂-(5-CH₂CH₂CO₂Et-2-Tez) | 2-F | H | H |
| 1-833 | H | H | CH₂-(5-CH₂CH₂CH₂CO₂H-2-Tez) | 2-F | H | H |
| 1-834 | Ac | H | CH₂-(5-CH₂CH₂CH₂CO₂H-2-Tez) | 2-F | H | H |
| 1-835 | H | H | CH₂-(5-CH₂CH₂CH₂CO₂Me-2-Tez) | 2-F | H | H |
| 1-836 | Ac | H | CH₂-(5-CH₂CH₂CH₂CO₂Me-2-Tez) | 2-F | H | H |
| 1-837 | H | H | CH₂-(5-CH₂CH₂CH₂CO₂Et-2-Tez) | 2-F | H | H |
| 1-838 | Ac | H | CH₂-(5-CH₂CH₂CH₂CO₂Et-2-Tez) | 2-F | H | H |
| 1-839 | H | H | CH₂CH₂-2-Tez | 2-F | H | H |
| 1-840 | Ac | H | CH₂CH₂-2-Tez | 2-F | H | H |
| 1-841 | H | H | CH₂CH₂-(5-CO₂H-2-Tez) | 2-F | H | H |
| 1-842 | Ac | H | CH₂CH₂-(5-CO₂H-2-Tez) | 2-F | H | H |
| 1-843 | H | H | CH₂CH₂-(5-CO₂Me-2-Tez) | 2-F | H | H |
| 1-844 | Ac | H | CH₂CH₂-(5-CO₂Me-2-Tez) | 2-F | H | H |
| 1-845 | H | H | CH₂CH₂-(5-CO₂Et-2-Tez) | 2-F | H | H |
| 1-846 | Ac | H | CH₂CH2-(5-CO₂Et-2-Tez) | 2-F | H | H |
| 1-847 | H | H | CH₂CH₂-(5-CH₂CO₂H-2-Tez) | 2-F | H | H |
| 1-848 | Ac | H | CH₂CH₂-(5-CH₂CO₂H-2-Tez) | 2-F | H | H |
| 1-849 | H | H | CH₂CH₂-(5-CH₂CO₂Me-2-Tez) | 2-F | H | H |
| 1-850 | Ac | H | CH₂CH₂-(5-CH₂CO₂Me-2-Tez) | 2-F | H | H |
| 1-851 | H | H | CH₂CH₂-(5-CH₂CO₂Et-2-Tez) | 2-F | H | H |
| 1-852 | Ac | H | CH₂CH₂-(5-CH₂CO₂Et-2-Tez) | 2-F | H | H |
| 1-853 | H | H | CH₂CH₂-(5-CH₂CH₂CO₂H-2-Tez) | 2-F | H | H |
| 1-854 | Ac | H | CH₂CH₂-(5-CH₂CH₂CO₂H-2-Tez) | 2-F | H | H |
| 1-855 | H | H | CH₂CH₂-(5-CH₂CH₂CO₂Me-2-Tez) | 2-F | H | H |
| 1-856 | Ac | H | CH₂CH₂-(5-CH₂CH₂CO₂Me-2-Tez) | 2-F | H | H |
| 1-857 | H | H | CH₂CH₂-(5-CH₂CH₂CO₂Et-2-Tez) | 2-F | H | H |
| 1-858 | Ac | H | CH₂CH₂-(5-CH₂CH₂CO₂Et-2-Tez) | 2-F | H | H |
| 1-859 | H | H | CH₂CH₂-(5-CH₂CH₂CH₂CO₂H-2-Tez) | 2-F | H | H |
| 1-860 | Ac | H | CH₂CH₂-(5-CH₂CH₂CH₂CO₂H-2-Tez) | 2-F | H | H |
| 1-861 | H | H | CH₂CH₂-(5-CH₂CH₂CH₂CO₂Me-2-Tez) | 2-F | H | H |
| 1-862 | Ac | H | CH₂CH₂-(5-CH₂CH₂CH₂CO₂Me-2-Tez) | 2-F | H | H |
| 1-863 | H | H | CH₂CH₂-(5-CH₂CH₂CH₂CO₂Et-2-Tez) | 2-F | H | H |
| 1-864 | Ac | H | CH₂CH₂-(5-CH₂CH₂CH₂CO₂Et-2-Tez) | 2-F | H | H |
| 1-865 | H | H | CH₂-5-Tez | 2-F | H | H |
| 1-866 | Ac | H | CH₂-5-Tez | 2-F | H | H |
| 1-867 | H | H | CH₂-5-Tez | 2-F | 4-F | H |
| 1-868 | Ac | H | CH₂-5-Tez | 2-F | 4-F | H |
| 1-869 | H | H | CH₂-(1-CO₂Me-5-Tez) | 2-F | H | H |
| 1-870 | Ac | H | CH₂-(1-CO₂Me-5-Tez) | 2-F | H | H |
| 1-871 | H | H | CH₂-(1-CO₂Et-5-Tez) | 2-F | H | H |
| 1-872 | Ac | H | CH₂-(1-CO₂Et-5-Tez) | 2-F | H | H |
| 1-873 | H | H | CH₂-(2-CO₂Me-5-Tez) | 2-F | 4-F | H |
| 1-874 | Ac | H | CH₂-(2-CO₂Me-5-Tez) | 2-F | 4-F | H |
| 1-875 | H | H | CH₂-(2-CO₂Me-5-Tez) | 2-F | H | H |
| 1-876 | Ac | H | CH₂-(2-CO₂Me-5-Tez) | 2-F | H | H |
| 1-877 | H | H | CH₂-(2-CO₂Et-5-Tez) | 2-F | H | H |
| 1-878 | Ac | H | CH₂-(2-CO₂Et-5-Tez) | 2-F | H | H |
| 1-879 | H | H | CH₂-(1-CH₂CO₂H-5-Tez) | 2-F | H | H |
| 1-880 | Ac | H | CH₂-(1-CH₂CO₂H-5-Tez) | 2-F | H | H |
| 1-881 | H | H | CH₂-(1-CH₂CO₂Me-5-Tez) | 2-F | H | H |
| 1-882 | Ac | H | CH₂-(1-CH₂CO₂Me-5-Tez) | 2-F | H | H |
| 1-883 | H | H | CH₂-(1-CH₂CO₂Et-5-Tez) | 2-F | H | H |
| 1-884 | Ac | H | CH₂-(1-CH₂CO₂Et-5-Tez) | 2-F | H | H |
| 1-885 | H | H | CH₂-(2-CH₂CO₂H-5-Tez) | 2-F | H | H |
| 1-886 | Ac | H | CH₂-(2-CH₂CO₂H-5-Tez) | 2-F | H | H |
| 1-887 | H | H | CH₂-(2-CH₂CO₂Me-5-Tez) | 2-F | H | H |
| 1-888 | Ac | H | CH₂-(2-CH₂CO₂Me-5-Tez) | 2-F | H | H |
| 1-889 | H | H | CH₂-(2-CH₂CO₂Et-5-Tez) | 2-F | H | H |
| 1-890 | Ac | H | CH₂-(2-CH₂CO₂Et-5-Tez) | 2-F | H | H |
| 1-891 | H | H | CH₂-(1-CH₂CH₂CO₂H-5-Tez) | 2-F | H | H |
| 1-892 | Ac | H | CH₂-(1-CH₂CH₂CO₂H-5-Tez) | 2-F | H | H |
| 1-893 | H | H | CH₂-(1-CH₂CH₂CO₂Me-5-Tez) | 2-F | H | H |
| 1-894 | Ac | H | CH₂-(1-CH₂CH₂CO₂Me-5-Tez) | 2-F | H | H |
| 1-895 | H | H | CH₂-(1-CH₂CH₂CO₂Et-5-Tez) | 2-F | H | H |
| 1-896 | Ac | H | CH₂-(1-CH₂CH₂CO₂Et-5-Tez) | 2-F | H | H |
| 1-897 | H | H | CH₂-(2-CH₂CH₂CO₂H-5-Tez) | 2-F | H | H |
| 1-898 | Ac | H | CH₂-(2-CH₂CH₂CO₂H-5-Tez) | 2-F | H | H |
| 1-899 | H | H | CH₂-(2-CH₂CH₂CO₂Me-5-Tez) | 2-F | H | H |
| 1-900 | Ac | H | CH₂-(2-CH₂CH₂CO₂Me-5-Tez) | 2-F | H | H |
| 1-901 | H | H | CH₂-(2-CH₂CH₂CO₂Et-5-Tez) | 2-F | H | H |
| 1-902 | Ac | H | CH₂-(2-CH₂CH₂CO₂Et-5-Tez) | 2-F | H | H |
| 1-903 | H | H | CH₂-(1-CH₂CH₂CH₂CO₂H-5-Tez) | 2-F | H | H |
| 1-904 | Ac | H | CH₂-(1-CH₂CH₂CH₂CO₂H-5-Tez) | 2-F | H | H |
| 1-905 | H | H | CH₂-(1-CH₂CH₂CH₂CO₂Me-5-Tez) | 2-F | H | H |
| 1-906 | Ac | H | CH₂-(1-CH₂CH₂CH₂CO₂Me-5-Tez) | 2-F | H | H |
| 1-907 | H | H | CH₂-(1-CH₂CH₂CH₂CO₂Et-5-Tez) | 2-F | H | H |
| 1-908 | Ac | H | CH₂-(1-CH₂CH₂CH₂CO₂Et-5-Tez) | 2-F | H | H |
| 1-909 | H | H | CH₂-(2-CH₂CH₂CH₂CO₂H-5-Tez) | 2-F | H | H |
| 1-910 | Ac | H | CH₂-(2-CH₂CH₂CH₂CO₂H-5-Tez) | 2-F | H | H |
| 1-911 | H | H | CH₂-(2-CH₂CH₂CH₂CO₂Me-5-Tez) | 2-F | H | H |
| 1-912 | Ac | H | CH₂-(2-CH₂CH₂CH₂CO₂Me-5-Tez) | 2-F | H | H |
| 1-913 | H | H | CH₂-(2-CH₂CH₂CH₂CO₂Et-5-Tez) | 2-F | H | H |
| 1-914 | Ac | H | CH₂-(2-CH₂CH₂CH₂CO₂Et-5-Tez) | 2-F | H | H |
| 1-915 | H | H | CH₂CH₂-5-Tez | 2-F | H | H |
| 1-916 | Ac | H | CH₂CH₂-5-Tez | 2-F | H | H |
| 1-917 | H | H | CH₂CH₂-5-Tez | 2-F | 4-F | H |
| 1-918 | Ac | H | CH₂CH₂-5-Tez | 2-F | 4-F | H |
| 1-919 | H | H | CH₂CH₂-(1-CO₂Me-5-Tez) | 2-F | H | H |
| 1-920 | Ac | H | CH₂CH₂-(1-CO₂Me-5-Tez) | 2-F | H | H |
| 1-921 | H | H | CH₂CH₂-(1-CO₂Et-5-Tez) | 2-F | H | H |
| 1-922 | Ac | H | CH₂CH₂-(1-CO₂Et-5-Tez) | 2-F | H | H |
| 1-923 | H | H | CH₂CH₂-(2-CO₂Me-5-Tez) | 2-F | 4-F | H |
| 1-924 | Ac | H | CH₂CH₂-(2-CO₂Me-5-Tez) | 2-F | 4-F | H |
| 1-925 | H | H | CH₂CH₂-(2-CO₂Me-5-Tez) | 2-F | H | H |
| 1-926 | Ac | H | CH₂CH₂-(2-CO₂Me-5-Tez) | 2-F | H | H |
| 1-927 | H | H | CH₂CH₂-(2-CO₂Et-5-Tez) | 2-F | H | H |
| 1-928 | Ac | H | CH₂CH₂-(2-CO₂Et-5-Tez) | 2-F | H | H |
| 1-929 | H | H | CH₂CH₂-(1-CH₂CO₂H-5-Tez) | 2-F | H | H |
| 1-930 | Ac | H | CH₂CH₂-(1-CH₂CO₂H-5-Tez) | 2-F | H | H |
| 1-931 | H | H | CH₂CH₂-(1-CH₂CO₂Me-5-Tez) | 2-F | H | H |
| 1-932 | Ac | H | CH₂CH₂-(1-CH₂CO₂Me-5-Tez) | 2-F | H | H |
| 1-933 | H | H | CH₂CH₂-(1-CH₂CO₂Et-5-Tez) | 2-F | H | H |
| 1-934 | Ac | H | CH₂CH₂(1-CH₂CO₂Et-5-Tez) | 2-F | H | H |
| 1-935 | H | H | CH₂CH₂-(2-CH₂CO₂H-5-Tez) | 2-F | H | H |
| 1-936 | Ac | H | CH₂CH₂-(2-CH₂CO₂H-5-Tez) | 2-F | H | H |
| 1-937 | H | H | CH₂CH₂-(2-CH₂CO₂Me-5-Tez) | 2-F | H | H |
| 1-938 | Ac | H | CH₂CH₂-(2-CH₂CO₂Me-5-Tez) | 2-F | H | H |
| 1-939 | H | H | CH₂CH₂-(2-CH₂CO₂Et-5-Tez) | 2-F | H | H |
| 1-940 | Ac | H | CH₂CH₂-(2-CH₂CO₂Et-5-Tez) | 2-F | H | H |
| 1-941 | H | H | CH₂CH₂-(1-CH₂CH₂CO₂H-5-Tez) | 2-F | H | H |
| 1-942 | Ac | H | CH₂CH₂-(1-CH₂CH₂CO₂H-5-Tez) | 2-F | H | H |
| 1-943 | H | H | CH₂CH₂-(1-CH₂CH₂CO₂Me-5-Tez) | 2-F | H | H |
| 1-944 | Ac | H | CH₂CH₂-(1-CH₂CH₂CO₂Me-5-Tez) | 2-F | H | H |
| 1-945 | H | H | CH₂CH₂-(1-CH₂CH₂CO₂Et-5-Tez) | 2-F | H | H |
| 1-946 | Ac | H | CH₂CH₂-(-CH₂CH₂CO₂Et-5-Tez) | 2-F | H | H |
| 1-947 | H | H | CH₂CH₂-(2-CH₂CH₂CO₂H-5-Tez) | 2-F | H | H |
| 1-948 | Ac | H | CH₂CH₂-(2-CH₂CH₂CO₂H-5-Tez) | 2-F | H | H |
| 1-949 | H | H | CH₂CH₂-(2-CH₂CH₂CO₂Me-5-Tez) | 2-F | H | H |
| 1-950 | Ac | H | CH₂CH₂-(2-CH₂CH₂CO₂Me-5-Tez) | 2-F | H | H |
| 1-951 | H | H | CH₂CH₂-(2-CH₂CH₂CO₂Et-5-Tez) | 2-F | H | H |
| 1-952 | Ac | H | CH₂CH₂-(2-CH₂CH₂CO₂Et-5-Tez) | 2-F | H | H |
| 1-953 | H | H | CH₂CH₂-(1-CH₂CH₂CH₂CO₂H-5-Tez) | 2-F | H | H |
| 1-954 | Ac | H | CH₂CH₂-(1-CH₂CH₂CH₂CO₂H-5-Tez) | 2-F | H | H |
| 1-955 | H | H | CH₂CH₂-(1-CH₂CH₂CH₂CO₂Me-5-Tez) | 2-F | H | H |
| 1-956 | Ac | H | CH₂CH₂-(1-CH₂CH₂CH₂CO₂Me-5-Tez) | 2-F | H | H |
| 1-957 | H | H | CH₂CH₂-(1-CH₂CH₂CH₂CO₂Et-5-Tez) | 2-F | H | H |
| 1-958 | Ac | H | CH₂CH₂-(1-CH₂CH₂CH₂CO₂Et-5-Tez) | 2-F | H | H |
| 1-959 | H | H | CH₂CH₂-(2-CH₂CH₂CH₂CO₂H-5-Tez) | 2-F | H | H |
| 1-960 | Ac | H | CH₂CH₂-(2-CH₂CH₂CH₂CO₂H-5-Tez) | 2-F | H | H |
| 1-961 | H | H | CH₂CH₂-(2-CH₂CH₂CH₂CO₂Me-5-Tez) | 2-F | H | H |
| 1-962 | Ac | H | CH₂CH₂-(2-CH₂CH₂CH₂CO₂Me-5-Tez) | 2-F | H | H |
| 1-963 | H | H | CH₂CH₂-(2-CH₂CH₂CH₂CO₂Et-5-Tez) | 2-F | H | H |
| 1-964 | Ac | H | CH₂CH₂-(2-CH₂CH₂CH₂CO₂Et-5-Tez) | 2-F | H | H |
| 1-965 | H | Me | CH₂-1-Pyza | 2-F | H | H |
| 1-966 | Ac | Me | CH₂-1-Pyza | 2-F | H | H |
| 1-967 | H | Me | CH₂-(3-CO₂H-1-Pyza) | 2-F | H | H |
| 1-968 | Ac | Me | CH₂-(3-CO₂H-1-Pyza) | 2-F | H | H |
| 1-969 | H | Me | CH₂-(3-CO₂Me-1-Pyza) | 2-F | H | H |
| 1-970 | Ac | Me | CH₂-(3-CO₂Me-1-Pyza) | 2-F | H | H |
| 1-971 | H | Me | CH₂-(3-CO₂Et-1-Pyza) | 2-F | H | H |
| 1-972 | Ac | Me | CH₂-(3-CO₂Et-1-Pyza) | 2-F | H | H |
| 1-973 | H | Me | CH₂-(4-CO₂H-1-Pyza) | 2-F | H | H |
| 1-974 | Ac | Me | CH₂-(4-CO₂H-1-Pyza) | 2-F | H | H |
| 1-975 | H | Me | CH₂-(4-CO₂Me-1-Pyza) | 2-F | H | H |
| 1-976 | Ac | Me | CH₂-(4-CO₂Me-1-Pyza) | 2-F | H | H |
| 1-977 | H | Me | CH₂-(4-CO₂Et-1-Pyza) | 2-F | H | H |
| 1-978 | Ac | Me | CH₂-(4-CO₂Et-1-Pyza) | 2-F | H | H |
| 1-979 | H | Me | CH₂-(3-CH₂CO₂H-1-Pyza) | 2-F | H | H |
| 1-980 | Ac | Me | CH₂-(3-CH₂CO₂H-1-Pyza) | 2-F | H | H |
| 1-981 | H | Me | CH₂-(3-CH₂CO₂Me-1-Pyza) | 2-F | H | H |
| 1-982 | Ac | Me | CH₂-(3-CH₂CO₂Me-1-Pyza) | 2-F | H | H |
| 1-983 | H | Me | CH₂-(3-CH₂CO₂Et-1-Pyza) | 2-F | H | H |
| 1-984 | Ac | Me | CH₂-(3-CH₂CO₂Et-1-Pyza) | 2-F | H | H |
| 1-985 | H | Me | CH₂-(4-CH₂CO₂H-1-Pyza) | 2-F | H | H |
| 1-986 | Ac | Me | CH₂-(4-CH₂CO₂H-1-Pyza) | 2-F | H | H |
| 1-987 | H | Me | CH₂-(4-CH₂CO₂Me-1-Pyza) | 2-F | H | H |
| 1-988 | Ac | Me | CH₂-(4-CH₂CO₂Me-1-Pyza) | 2-F | H | H |
| 1-989 | H | Me | CH₂-(4-CH₂CO₂Et-1-Pyza) | 2-F | H | H |
| 1-990 | Ac | Me | CH₂-(4-CH₂CO₂Et-1-Pyza) | 2-F | H | H |
| 1-991 | H | Me | CH₂-(3-CH₂CH₂CO₂H-1-Pyza) | 2-F | H | H |
| 1-992 | Ac | Me | CH₂-(3-CH₂CH₂CO₂H-1-Pyza) | 2-F | H | H |
| 1-993 | H | Me | CH₂-(3-CH₂CH₂CO₂Me-1-Pyza) | 2-F | H | H |
| 1-994 | Ac | Me | CH₂-(3-CH₂CH₂CO₂Me-1-Pyza) | 2-F | H | H |
| 1-995 | H | Me | CH₂-(3-CH₂CH₂CO₂Et-1-Pyza) | 2-F | H | H |
| 1-996 | Ac | Me | CH₂-(3-CH₂CH₂CO₂Et-1-Pyza) | 2-F | H | H |
| 1-997 | H | Me | CH₂-(4-CH₂CH₂CO₂H-1-Pyza) | 2-F | H | H |
| 1-998 | Ac | Me | CH₂-(4-CH₂CH₂CO₂H-1-Pyza) | 2-F | H | H |
| 1-999 | H | Me | CH₂-(4-CH₂CH₂CO₂Me-1-Pyza) | 2-F | H | H |
| 1-1000 | Ac | Me | CH₂-(4-CH₂CH₂CO₂Me-1-Pyza) | 2-F | H | H |
| 1-1001 | H | Me | CH₂-(4-CH₂CH₂CO₂Et-1-Pyza) | 2-F | H | H |
| 1-1002 | Ac | Me | CH₂-(4-CH₂CH₂CO₂Et-1-Pyza) | 2-F | H | H |
| 1-1003 | H | Me | CH₂CH₂-1-Pyza | 2-F | H | H |
| 1-1004 | Ac | Me | CH₂CH₂-1-Pyza | 2-F | H | H |
| 1-1005 | H | Me | CH₂CH₂-(3-CO₂H-1-Pyza) | 2-F | H | H |
| 1-1006 | Ac | Me | CH₂CH₂-(3-CO₂H-1-Pyza) | 2-F | H | H |
| 1-1007 | H | Me | CH₂CH₂-(3-CO₂Me-1-Pyza) | 2-F | H | H |
| 1-1008 | Ac | Me | CH₂CH₂-(3-CO₂Me-1-Pyza) | 2-F | H | H |
| 1-1009 | H | Me | CH₂CH₂-(3-CO₂Et-1-Pyza) | 2-F | H | H |
| 1-1010 | Ac | Me | CH₂CH₂-(3-CO₂Et-1-Pyza) | 2-F | H | H |
| 1-1011 | H | Me | CH₂CH₂-(4-CO₂H-1-Pyza) | 2-F | H | H |
| 1-1012 | Ac | Me | CH₂CH₂-(4-CO₂H-1-Pyza) | 2-F | H | H |
| 1-1013 | H | Me | CH₂CH₂-(4-CO₂Me-1-Pyza) | 2-F | H | H |
| 1-1014 | Ac | Me | CH₂CH₂-(4-CO₂Me-1-Pyza) | 2-F | H | H |
| 1-1015 | H | Me | CH₂CH₂-(4-CO₂Et-1-Pyza) | 2-F | H | H |
| 1-1016 | Ac | Me | CH₂CH₂-(4-CO₂Et-1-Pyza) | 2-F | H | H |
| 1-1017 | H | Me | CH₂CH₂-(3-CH₂CO₂H-1-Pyza) | 2-F | H | H |
| 1-1018 | Ac | Me | CH₂CH₂-(3-CH₂CO₂H-1-Pyza) | 2-F | H | H |
| 1-1019 | H | Me | CH₂CH₂-(3-CH₂CO₂Me-1-Pyza) | 2-F | H | H |
| 1-1020 | Ac | Me | CH₂CH₂-(3-CH₂CO₂Me-1-Pyza) | 2-F | H | H |
| 1-1021 | H | Me | CH₂CH₂-(3-CH₂CO₂Et-1-Pyza) | 2-F | H | H |
| 1-1022 | Ac | Me | CH₂CH₂-(3-CH₂CO₂Et-1-Pyza) | 2-F | H | H |
| 1-1023 | H | Me | CH₂CH₂-(4-CH₂CO₂H-1-Pyza) | 2-F | H | H |
| 1-1024 | Ac | Me | CH₂CH₂-(4-CH₂CO₂H-1-Pyza) | 2-F | H | H |
| 1-1025 | H | Me | CH₂CH₂-(4-CH₂CO₂Me-1-Pyza) | 2-F | H | H |
| 1-1026 | Ac | Me | CH₂CH₂-(4-CH₂CO₂Me-1-Pyza) | 2-F | H | H |
| 1-1027 | H | Me | CH₂CH₂-(4-CH₂CO₂Et-1-Pyza) | 2-F | H | H |
| 1-1028 | Ac | Me | CH₂CH₂-(4-CH₂CO₂Et-1-Pyza) | 2-F | H | H |
| 1-1029 | H | Me | CH₂CH₂-(3-CH₂CH₂CO₂H-1-Pyza) | 2-F | H | H |
| 1-1030 | Ac | Me | CH₂CH₂-(3-CH₂CH₂CO₂H-1-Pyza) | 2-F | H | H |
| 1-1031 | H | Me | CH₂CH₂-(3-CH₂CH₂CO₂Me-1-Pyza) | 2-F | H | H |
| 1-1032 | Ac | Me | CH₂CH₂-(3-CH₂CH₂CO₂Me-l-Pyza) | 2-F | H | H |
| 1-1033 | H | Me | CH₂CH₂-(3-CH₂CH₂CO₂Et-1-Pyza) | 2-F | H | H |
| 1-1034 | Ac | Me | CH₂CH₂-(3-CH₂CH₂CO₂Et-1-Pyza) | 2-F | H | H |
| 1-1035 | H | Me | CH₂CH₂-(4-CH₂CH₂CO₂H-1-Pyza) | 2-F | H | H |
| 1-1036 | Ac | Me | CH₂CH₂-(4-CH₂CH₂CO₂H-1-Pyza) | 2-F | H | H |
| 1-1037 | H | Me | CH₂CH₂-(4-CH₂CH₂CO₂Me-1-Pyza) | 2-F | H | H |
| 1-1038 | Ac | Me | CH₂CH₂-(4-CH₂CH₂CO₂Me-1-Pyza) | 2-F | H | H |
| 1-1039 | H | Me | CH₂CH₂-(4-CH₂CH₂CO₂Et-1-Pyza) | 2-F | H | H |
| 1-1040 | Ac | Me | CH₂CH₂-(4-CH₂CH₂CO₂Et-1-Pyza) | 2-F | H | H |
| 1-1041 | H | H | CH₂-(2-CO₂Me-4-Triz) | 2-F | H | H |
| 1-1042 | Ac | H | CH₂-(2-CO₂Me-4-Triz) | 2-F | H | H |
| 1-1043 | H | H | CH₂-(2-CO₂Et-4-Triz) | 2-F | H | H |
| 1-1044 | Ac | H | CH₂-(2-CO₂Et-4-Triz) | 2-F | H | H |
| 1-1045 | H | H | CH₂-(2-CH₂CO₂H-4-Triz) | 2-F | H | H |
| 1-1046 | Ac | H | CH₂-(2-CH₂CO₂H-4-Triz) | 2-F | H | H |
| 1-1047 | H | H | CH₂-(2-CH₂CO₂Me-4-Triz) | 2-F | H | H |
| 1-1048 | Ac | H | CH₂-(2-CH₂CO₂Me-4-Triz) | 2-F | H | H |
| 1-1049 | H | H | CH₂-(2-CH₂CO₂Et-4-Triz) | 2-F | H | H |
| 1-1050 | Ac | H | CH₂-(2-CH₂CO₂Et-4-Triz) | 2-F | H | H |
| 1-1051 | H | H | CH₂-(2-CH₂CH₂CO₂H-4-Triz) | 2-F | H | H |
| 1-1052 | Ac | H | CH₂-(2-CH₂CH₂CO₂H-4-Triz) | 2-F | H | H |
| 1-1053 | H | H | CH₂-(2-CH₂CH₂CO₂Me-4-Triz) | 2-F | H | H |
| 1-1054 | Ac | H | CH₂-(2-CH₂CH₂CO₂Me-4-Triz) | 2-F | H | H |
| 1-1055 | H | H | CH₂-(2-CH₂CH₂CO₂Et4-Triz) | 2-F | H | H |
| 1-1056 | Ac | H | CH₂-(2-CH₂CH₂CO₂Et-4-Triz) | 2-F | H | H |
| 1-1057 | H | H | CH₂-(2-CH₂CH₂CH₂CO₂H-4-Triz) | 2-F | H | H |
| 1-1058 | Ac | H | CH₂-(2-CH₂CH₂CH₂CO₂H-4-Triz) | 2-F | H | H |
| 1-1059 | H | H | CH₂-(2-CH₂CH₂CH₂CO₂Me-4-Triz) | 2-F | H | H |
| 1-1060 | Ac | H | CH₂-(2-CH₂CH₂CH₂CO₂Me-4-Triz) | 2-F | H | H |
| 1-1061 | H | H | CH₂-(2-CH₂CH₂CH₂CO₂Et-4-Triz) | 2-F | H | H |
| 1-1062 | Ac | H | CH₂-(2-CH₂CH₂CH₂CO₂Et-4-Triz) | 2-F | H | H |
| 1-1063 | H | H | CH₂CH₂-(2-CO₂Me-4-Triz) | 2-F | H | H |
| 1-1064 | Ac | H | CH₂CH₂-(2-CO₂Me-4-Triz) | 2-F | H | H |
| 1-1065 | H | H | CH₂CH₂-(2-CO₂Et-4-Triz) | 2-F | H | H |
| 1-1066 | Ac | H | CH₂CH₂-(2-CO₂Et-4-Triz) | 2-F | H | H |
| 1-1067 | H | H | CH₂CH₂-(2-CH₂CO₂H-4-Triz) | 2-F | H | H |
| 1-1068 | Ac | H | CH₂CH₂-(2-CH₂CO₂H-4-Triz) | 2-F | H | H |
| 1-1069 | H | H | CH₂CH₂-(2-CH₂CO₂Me-4-Triz) | 2-F | H | H |
| 1-1070 | Ac | H | CH₂CH₂-(2-CH₂CO₂Me-4-Triz) | 2-F | H | H |
| 1-1071 | H | H | CH₂CH₂-(2-CH₂CO₂Et-4-Triz) | 2-F | H | H |
| 1-1072 | Ac | H | CH₂CH₂-(2-CH₂CO₂Et-4-Triz) | 2-F | H | H |
| 1-1073 | H | H | CH₂CH₂-(2-CH₂CH₂CO₂H-4-Triz) | 2-F | H | H |
| 1-1074 | Ac | H | CH₂CH₂-(2-CH₂CH₂CO₂H-4-Triz) | 2-F | H | H |
| 1-1075 | H | H | CH₂CH₂-(2-CH₂CH₂CO₂Me-4-Triz) | 2-F | H | H |
| 1-1076 | Ac | H | CH₂CH₂-(2-CH₂CH₂CO₂Me-4-Triz) | 2-F | H | H |
| 1-1077 | H | H | CH₂CH₂-(2-CH₂CH₂CO₂Et-4-Triz) | 2-F | H | H |
| 1-1078 | Ac | H | CH₂CH₂-(2-CH₂CH₂CO₂Et-4-Triz) | 2-F | H | H |
| 1-1079 | H | H | CH₂CH₂-(2-CH₂CH₂CH₂CO₂H-4-Triz) | 2-F | H | H |
| 1-1080 | Ac | H | CH₂CH₂-(2-CH₂CH₂CH₂CO₂H-4-Triz) | 2-F | H | H |
| 1-1081 | H | H | CH₂CH₂-(2-CH₂CH₂CH₂CO₂Me-4-Triz) | 2-F | H | H |
| 1-1082 | Ac | H | CH₂CH₂-(2-CH₂CH₂CH₂CO₂Me-4-Triz) | 2-F | H | H |
| 1-1083 | H | H | CH₂CH₂-(2-CH₂CH₂CH₂CO₂Et-4-Triz) | 2-F | H | H |
| 1-1084 | Ac | H | CH₂CH₂-(2-CH₂CH₂CH₂CO₂Et-4-Triz) | 2-F | H | H |

**(Table 2)**

| | | | | | | |
|---|---|---|---|---|---|---|
| 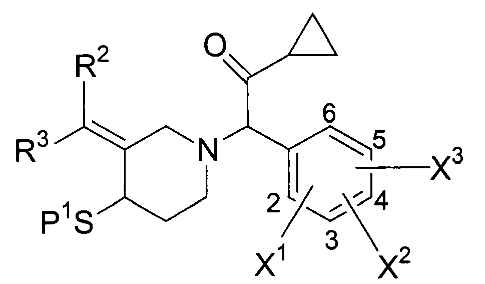 | | | | | | |

| Compound No. | P¹ | R² | R³ | X¹ | X² | X³ |
|---|---|---|---|---|---|---|
| 2-1 | H | H | CH₂-1-Pyza | 2-F | H | H |
| 2-2 | Ac | H | CH₂-1-Pyza | 2-F | H | H |
| 2-3 | H | H | CH₂-1-Pyza | 2-F | 4-F | H |
| 2-4 | Ac | H | CH₂-1-Pyza | 2-F | 4-F | H |
| 2-5 | H | H | CH₂-(3-CO₂H-1-Pyza) | 2-F | H | H |
| 2-6 | Ac | H | CH₂-(3-CO₂H-1-Pyza) | 2-F | H | H |
| 2-7 | H | H | CH₂-(3-CO₂H-1-Pyza) | 2-F | 4-F | H |
| 2-8 | Ac | H | CH₂-(3-CO₂H-1-Pyza) | 2-F | 4-F | H |
| 2-9 | H | H | CH₂-(3-CO₂Me-1-Pyza) | 2-F | H | H |
| 2-10 | Ac | H | CH₂-(3-CO₂Me-1-Pyza) | 2-F | H | H |
| 2-11 | H | H | CH₂-(3-CO₂Me-1-Pyza) | 2-F | 4-F | H |
| 2-12 | Ac | H | CH₂-(3-CO₂Me-1-Pyza) | 2-F | 4-F | H |
| 2-13 | H | H | CH₂-(3-CO₂Et-1-Pyza) | 2-F | H | H |
| 2-14 | Ac | H | CH₂-(3-CO₂Et-1-Pyza) | 2-F | H | H |
| 2-15 | H | H | CH₂-(3-CO₂Et-1-Pyza) | 2-F | 4-F | H |
| 2-16 | Ac | H | CH₂-(3-CO₂Et-1-Pyza) | 2-F | 4-F | H |
| 2-17 | H | H | CH₂-(4-CO₂H-1-Pyza) | 2-F | H | H |
| 2-18 | Ac | H | CH₂-(4-CO₂H-1-Pyza) | 2-F | H | H |
| 2-19 | H | H | CH₂-(4-CO₂H-1-Pyza) | 2-F | 4-F | H |
| 2-20 | Ac | H | CH₂-(4-CO₂H-1-Pyza) | 2-F | 4-F | H |
| 2-21 | H | H | CH₂-(4-CO₂Me-1-Pyza) | 2-F | H | H |
| 2-22 | Ac | H | CH₂-(4-CO₂Me-1-Pyza) | 2-F | H | H |
| 2-23 | H | H | CH₂-(4-CO₂Me-1-Pyza) | 2-F | 4-F | H |
| 2-24 | Ac | H | CH₂-(4-CO₂Me-1-Pyza) | 2-F | 4-F | H |
| 2-25 | H | H | CH₂-(4-CO₂Et-1-Pyza) | 2-F | H | H |
| 2-26 | Ac | H | CH₂-(4-CO₂Et-1-Pyza) | 2-F | H | H |
| 2-27 | H | H | CH₂-(4-CO₂Et-1-Pyza) | 2-F | 4-F | H |
| 2-28 | Ac | H | CH₂-(4-CO₂Et-1-Pyza) | 2-F | 4-F | H |
| 2-29 | H | H | CH₂-(5-CO₂H-1-Pyza) | 2-F | H | H |
| 2-30 | Ac | H | CH₂-(5-CO₂H-1-Pyza) | 2-F | H | H |
| 2-31 | H | H | CH₂-(5-CO₂H-1-Pyza) | 2-F | 4-F | H |
| 2-32 | Ac | H | CH₂-(5-CO₂H-1-Pyza) | 2-F | 4-F | H |
| 2-33 | H | H | CH₂-(5-CO₂Me-1-Pyza) | 2-F | H | H |
| 2-34 | Ac | H | CH₂-(5-CO₂Me-1-Pyza) | 2-F | H | H |
| 2-35 | H | H | CH₂-(5-CO₂Me-1-Pyza) | 2-F | 4-F | H |
| 2-36 | Ac | H | CH₂-(5-CO₂Me-1-Pyza) | 2-F | 4-F | H |
| 2-37 | H | H | CH₂-(5-CO₂Et-1-Pyza) | 2-F | H | H |
| 2-38 | Ac | H | CH₂-(5-CO₂Et-1-Pyza) | 2-F | H | H |
| 2-39 | H | H | CH₂-(5-CO₂Et-1-Pyza) | 2-F | 4-F | H |
| 2-40 | Ac | H | CH₂-(5-CO₂Et-1-Pyza) | 2-F | 4-F | H |
| 2-41 | H | H | CH₂-(3-CH₂CO₂H-1-Pyza) | 2-F | H | H |
| 2-42 | Ac | H | CH₂-(3-CH₂CO₂H-1-Pyza) | 2-F | H | H |
| 2-43 | H | H | CH₂-(3-CH₂CO₂H-1-Pyza) | 2-F | 4-F | H |
| 2-44 | Ac | H | CH₂-(3-CH₂CO₂H-1-Pyza) | 2-F | 4-F | H |
| 2-45 | H | H | CH₂-(3-CH₂CO₂Me-1-Pyza) | 2-F | H | H |
| 2-46 | Ac | H | CH₂-(3-CH₂CO₂Me-1-Pyza) | 2-F | H | H |
| 2-47 | H | H | CH₂-(3-CH₂CO₂Me-1-Pyza) | 2-F | 4-F | H |
| 2-48 | Ac | H | CH₂-(3-CH₂CO₂Me-1-Pyza) | 2-F | 4-F | H |
| 2-49 | H | H | CH₂-(3-CH₂CO₂Et-1-Pyza) | 2-F | H | H |
| 2-50 | Ac | H | CH₂-(3-CH₂CO₂Et-1-Pyza) | 2-F | H | H |
| 2-51 | H | H | CH₂-(3-CH₂CO₂Et-1-Pyza) | 2-F | 4-F | H |
| 2-52 | Ac | H | CH₂-(3-CH₂CO₂Et-1-Pyza) | 2-F | 4-F | H |
| 2-53 | H | H | CH₂-(4-CH₂CO₂H-1-Pyza) | 2-F | H | H |
| 2-54 | Ac | H | CH₂-(4-CH₂CO₂H-1-Pyza) | 2-F | H | H |
| 2-55 | H | H | CH₂-(4-CH₂CO₂H-1-Pyza) | 2-F | 4-F | H |
| 2-56 | Ac | H | CH₂-(4-CH₂CO₂H-1-Pyza) | 2-F | 4-F | H |
| 2-57 | H | H | CH₂-(4-CH₂CO₂Me-1-Pyza) | 2-F | H | H |
| 2-58 | Ac | H | CH₂-(4-CH₂CO₂Me-1-Pyza) | 2-F | H | H |
| 2-59 | H | H | CH₂-(4-CH₂CO₂Me-1-Pyza) | 2-F | 4-F | H |
| 2-60 | Ac | H | CH₂-(4-CH₂CO₂Me-1-Pyza) | 2-F | 4-F | H |
| 2-61 | H | H | CH₂-(4-CH₂CO₂Et-1-Pyza) | 2-F | H | H |
| 2-62 | Ac | H | CH₂-(4-CH₂CO₂Et-1-Pyza) | 2-F | H | H |
| 2-63 | H | H | CH₂-(4-CH₂CO₂Et-1-Pyza) | 2-F | 4-F | H |
| 2-64 | Ac | H | CH₂-(4-CH₂CO₂Et-1-Pyza) | 2-F | 4-F | H |
| 2-65 | H | H | CH₂-(5-CH₂CO₂H-1-Pyza) | 2-F | H | H |
| 2-66 | Ac | H | CH₂-(5-CH₂CO₂H-1-Pyza) | 2-F | H | H |
| 2-67 | H | H | CH₂-(5-CH₂CO₂H-1-Pyza) | 2-F | 4-F | H |
| 2-68 | Ac | H | CH₂-(5-CH₂CO₂H-1-Pyza) | 2-F | 4-F | H |
| 2-69 | H | H | CH₂-(5-CH₂CO₂Me-1-Pyza) | 2-F | H | H |
| 2-70 | Ac | H | CH₂-(5-CH₂CO₂Me-1-Pyza) | 2-F | H | H |
| 2-71 | H | H | CH₂-(5-CH₂CO₂Me-1-Pyza) | 2-F | 4-F | H |
| 2-72 | Ac | H | CH₂-(5-CH₂CO₂Me-1-Pyza) | 2-F | 4-F | H |
| 2-73 | H | H | CH₂-(5-CH₂CO₂Et-1-Pyza) | 2-F | H | H |
| 2-74 | Ac | H | CH₂-(5-CH₂CO₂Et-1-Pyza) | 2-F | H | H |
| 2-75 | H | H | CH₂-(5-CH₂CO₂Et-1-Pyza) | 2-F | 4-F | H |
| 2-76 | Ac | H | CH₂-(5-CH₂CO₂Et-1-Pyza) | 2-F | 4-F | H |
| 2-77 | H | H | CH₂-(3-CH₂CH₂CO₂H-1-Pyza) | 2-F | H | H |
| 2-78 | Ac | H | CH₂-(3-CH₂CH₂CO₂H-1-Pyza) | 2-F | H | H |
| 2-79 | H | H | CH₂-(3-CH₂CH₂CO₂Me-1-Pyza) | 2-F | H | H |
| 2-80 | Ac | H | CH₂-(3-CH₂CH₂CO₂Me-1-Pyza) | 2-F | H | H |
| 2-81 | H | H | CH₂-(3-CH₂CH₂CO₂Et-1-Pyza) | 2-F | H | H |
| 2-82 | Ac | H | CH₂-(3-CH₂CH₂CO₂Et-1-Pyza) | 2-F | H | H |
| 2-83 | H | H | CH₂-(4-CH₂CH₂CO₂H-1-Pyza) | 2-F | H | H |
| 2-84 | Ac | H | CH₂-(4-CH₂CH₂CO₂H-1-Pyza) | 2-F | H | H |
| 2-85 | H | H | CH₂-(4-CH₂CH₂CO₂Me-1-Pyza) | 2-F | H | H |
| 2-86 | Ac | H | CH₂-(4-CH₂CH₂CO₂Me-1-Pyza) | 2-F | H | H |
| 2-87 | H | H | CH₂-(4-CH₂CH₂CO₂Et-1-Pyza) | 2-F | H | H |
| 2-88 | Ac | H | CH₂-(4-CH₂CH₂CO₂Et-1-Pyza) | 2-F | H | H |
| 2-89 | H | H | CH₂-(5-CH₂CH₂CO₂H-1-Pyza) | 2-F | H | H |
| 2-90 | Ac | H | CH₂-(5-CH₂CH₂CO₂H-1-Pyza) | 2-F | H | H |
| 2-91 | H | H | CH₂-(5-CH₂CH₂CO₂Me-1-Pyza) | 2-F | H | H |
| 2-92 | Ac | H | CH₂-(5-CH₂CH₂CO₂Me-1-Pyza) | 2-F | H | H |
| 2-93 | H | H | CH₂-(5-CH₂CH₂CO₂Et-1-Pyza) | 2-F | H | H |
| 2-94 | Ac | H | CH₂-(5-CH₂CH₂CO₂Et-1-Pyza) | 2-F | H | H |
| 2-95 | H | H | CH₂-(3-CH₂CH₂CH₂CO₂H-1-Pyza) | 2-F | H | H |
| 2-96 | Ac | H | CH₂-(3-CH₂CH₂CH₂CO₂H-1-Pyza) | 2-F | H | H |
| 2-97 | H | H | CH₂-(3-CH₂CH₂CH₂CO₂Me-1-Pyza) | 2-F | H | H |
| 2-98 | Ac | H | CH₂-(3-CH₂CH₂CH₂CO₂Me-1-Pyza) | 2-F | H | H |
| 2-99 | H | H | CH₂-(3-CH₂CH₂CH₂CO₂Et-1-Pyza) | 2-F | H | H |
| 2-100 | Ac | H | CH₂-(3-CH₂CH₂CH₂CO₂Et-1-Pyza) | 2-F | H | H |
| 2-101 | H | H | CH₂-(4-CH₂CH₂CH₂CO₂H-1-Pyza) | 2-F | H | H |
| 2-102 | Ac | H | CH₂-(4-CH₂CH₂CH₂CO₂H-1-Pyza) | 2-F | H | H |
| 2-103 | H | H | CH₂-(4-CH₂CH₂CH₂CO₂Me-1-Pyza) | 2-F | H | H |
| 2-104 | Ac | H | CH₂-(4-CH₂CH₂CH₂CO₂Me-1-Pyza) | 2-F | H | H |
| 2-105 | H | H | CH₂-(4-CH₂CH₂CH₂CO₂Et-1-Pyza) | 2-F | H | H |
| 2-106 | Ac | H | CH₂-(4-CH₂CH₂CH₂CO₂Et-1-Pyza) | 2-F | H | H |
| 2-107 | H | H | CH₂-(5-CH₂CH₂CH₂CO₂H-1-Pyza) | 2-F | H | H |
| 2-108 | Ac | H | CH₂-(5-CH₂CH₂CH₂CO₂H-1-Pyza) | 2-F | H | H |
| 2-109 | H | H | CH₂-(5-CH₂CH₂CH₂CO₂Me-1-Pyza) | 2-F | H | H |
| 2-110 | Ac | H | CH₂-(5-CH₂CH₂CH₂CO₂Me-1-Pyza) | 2-F | H | H |
| 2-111 | H | H | CH₂-(5-CH₂CH₂CH₂CO₂Et-1-Pyza) | 2-F | H | H |
| 2-112 | Ac | H | CH₂-(5-CH₂CH₂CH₂CO₂Et-1-Pyza) | 2-F | H | H |
| 2-113 | H | H | CH₂CH₂-1-Pyza | 2-F | H | H |
| 2-114 | Ac | H | CH₂CH₂-1-Pyza | 2-F | H | H |
| 2-115 | H | H | CH₂CH₂-(3-CO₂H-1-Pyza) | 2-F | H | H |
| 2-116 | Ac | H | CH₂CH₂-(3-CO₂H-1-Pyza) | 2-F | H | H |
| 2-117 | H | H | CH₂CH₂-(3-CO₂Me-1-Pyza) | 2-F | H | H |
| 2-118 | Ac | H | CH₂CH₂-(3-CO₂Me-1-Pyza) | 2-F | H | H |
| 2-119 | H | H | CH₂CH₂-(3-CO₂Et-1-Pyza) | 2-F | H | H |
| 2-120 | Ac | H | CH₂CH₂-(3-CO₂Et-1-Pyza) | 2-F | H | H |
| 2-121 | H | H | CH₂CH₂-(4-CO₂H-1-Pyza) | 2-F | H | H |
| 2-122 | Ac | H | CH₂CH₂-(4-CO₂H-1-Pyza) | 2-F | H | H |
| 2-123 | H | H | CH₂CH₂-(4-CO₂Me-1-Pyza) | 2-F | H | H |
| 2-124 | Ac | H | CH₂CH₂-(4-CO₂Me-1-Pyza) | 2-F | H | H |
| 2-125 | H | H | CH₂CH₂-(4-CO₂Et-1-Pyza) | 2-F | H | H |
| 2-126 | Ac | H | CH₂CH₂-(4-CO₂Et-1-Pyza) | 2-F | H | H |
| 2-127 | H | H | CH₂CH₂-(5-CO₂H-1-Pyza) | 2-F | H | H |
| 2-128 | Ac | H | CH₂CH₂-(5-CO₂H-1-Pyza) | 2-F | H | H |
| 2-129 | H | H | CH₂CH₂-(5-CO₂Me-1-Pyza) | 2-F | H | H |
| 2-130 | Ac | H | CH₂CH₂-(5-CO₂Me-1-Pyza) | 2-F | H | H |
| 2-131 | H | H | CH₂CH₂-(5-CO₂Et-1-Pyza) | 2-F | H | H |
| 2-132 | Ac | H | CH₂CH₂-(5-CO₂Et-1-Pyza) | 2-F | H | H |
| 2-133 | H | H | CH₂CH₂-(3-CH₂CO₂H-1-Pyza) | 2-F | H | H |
| 2-134 | Ac | H | CH₂CH₂-(3-CH₂CO₂H-1-Pyza) | 2-F | H | H |
| 2-135 | H | H | CH₂CH₂-(3-CH₂CO₂Me-1-Pyza) | 2-F | H | H |
| 2-136 | Ac | H | CH₂CH₂-(3-CH₂CO₂Me-1-Pyza) | 2-F | H | H |
| 2-137 | H | H | CH₂CH₂-(3-CH₂CO₂Et-1-Pyza) | 2-F | H | H |
| 2-138 | Ac | H | CH₂CH₂-(3-CH₂CO₂Et-1-Pyza) | 2-F | H | H |
| 2-139 | H. | H | CH₂CH₂-(4-CH₂CO₂H-1-Pyza) | 2-F | H | H |
| 2-140 | Ac | H | CH₂CH₂-(4-CH₂CO₂H-1-Pyza) | 2-F | H | H |
| 2-141 | H | H | CH₂CH₂-(4-CH₂CO₂Me-1-Pyza) | 2-F | H | H |
| 2-142 | Ac | H | CH₂CH₂-(4-CH₂CO₂Me-1-Pyza) | 2-F | H | H |
| 2-143 | H | H | CH₂CH₂-(4-CH₂CO₂Et-1-Pyza) | 2-F | H | H |
| 2-144 | Ac | H | CH₂CH₂-(4-CH₂CO₂Et-1-Pyza) | 2-F | H | H |
| 2-145 | H | H | CH₂CH₂-(5-CH₂CO₂H-1-Pyza) | 2-F | H | H |
| 2-146 | Ac | H | CH₂CH₂-(5-CH₂CO₂H-1-Pyza) | 2-F | H | H |
| 2-147 | H | H | CH₂CH₂-(5-CH₂CO₂Me-1-Pyza) | 2-F | H | H |
| 2-148 | Ac | H | CH₂CH₂-(5-CH₂CO₂Me-1-Pyza) | 2-F | H | H |
| 2-149 | H | H | CH₂CH₂-(5-CH₂CO₂Et-1-Pyza) | 2-F | H | H |
| 2-150 | Ac | H | CH₂CH₂-(5-CH₂CO₂Et-1-Pyza) | 2-F | H | H |
| 2-151 | H | H | CH₂CH₂-(3-CH₂CH₂CO₂H-1-Pyza) | 2-F | H | H |
| 2-152 | Ac | H | CH₂CH₂-(3-CH₂CH₂CO₂H-1-Pyza) | 2-F | H | H |
| 2-153 | H | H | CH₂CH₂-(3-CH₂CH₂CO₂Me-1-Pyza) | 2-F | H | H |
| 2-154 | Ac | H | CH₂CH₂-(3-CH₂CH₂CO₂Me-1-Pyza) | 2-F | H | H |
| 2-155 | H | H | CH₂CH₂-(3-CH₂CH₂CO₂Et-1-Pyza) | 2-F | H | H |
| 2-156 | Ac | H | CH₂CH₂-(3-CH₂CH₂CO₂Et-1-Pyza) | 2-F | H | H |
| 2-157 | H | H | CH₂CH₂-(4-CH₂CH₂CO₂H-1-Pyza) | 2-F | H | H |
| 2-158 | Ac | H | CH₂CH₂-(4-CH₂CH₂CO₂H-1-Pyza) | 2-F | H | H |
| 2-159 | H | H | CH₂CH₂-(4-CH₂CH₂CO₂Me-1-Pyza) | 2-F | H | H |
| 2-160 | Ac | H | CH₂CH₂-(4-CH₂CH₂CO₂Me-1-Pyza) | 2-F | H | H |
| 2-161 | H | H | CH₂CH₂-(4-CH₂CH₂CO₂Et-1-Pyza) | 2-F | H | H |
| 2-162 | Ac | H | CH₂CH₂-(4-CH₂CH₂CO₂Et-1-Pyza) | 2-F | H | H |
| 2-163 | H | H | CH₂CH₂-(5-CH₂CH₂CO₂H-1-Pyza) | 2-F | H | H |
| 2-164 | Ac | H | CH₂CH₂-(5-CH₂CH₂CO₂H-1-Pyza) | 2-F | H | H |
| 2-165 | H | H | CH₂CH₂-(5-CH₂CH₂CO₂Me-1-Pyza) | 2-F | H | H |
| 2-166 | Ac | H | CH₂CH₂-(5-CH₂CH₂CO₂Me-1-Pyza) | 2-F | H | H |
| 2-167 | H | H | CH₂CH₂-(5-CH₂CH₂CO₂Et-1-Pyza) | 2-F | H | H |
| 2-168 | Ac | H | CH₂CH₂-(5-CH₂CH₂CO₂Et-1-Pyza) | 2-F | H | H |
| 2-169 | H | H | CH₂CH₂-(3-CH₂CH₂CH₂CO₂H-1-Pyza) | 2-F | H | H |
| 2-170 | Ac | H | CH₂CH₂-(3-CH₂CH₂CH₂CO₂H-1-Pyza) | 2-F | H | H |
| 2-171 | H | H | CH₂CH₂-(3-CH₂CH₂CH₂CO₂Me-1-Pyza) | 2-F | H | H |
| 2-172 | Ac | H | CH₂CH₂-(3-CH₂CH₂CH₂CO₂Me-1-Pyza) | 2-F | H | H |
| 2-173 | H | H | CH₂CH₂-(3-CH₂CH₂CH₂CO₂Et-1-Pyza) | 2-F | H | H |
| 2-174 | Ac | H | CH₂CH₂-(3-CH₂CH₂CH₂CO₂Et-1-Pyza) | 2-F | H | H |
| 2-175 | H | H | CH₂CH₂-(4-CH₂CH₂CH₂CO₂H-1-Pyza) | 2-F | H | H |
| 2-176 | Ac | H | CH₂CH₂-(4-CH₂CH₂CH₂CO₂H-1-Pyza) | 2-F | H | H |
| 2-177 | H | H | CH₂CH₂-(4-CH₂CH₂CH₂CO₂Me-1-Pyza) | 2-F | H | H |
| 2-178 | Ac | H | CH₂CH₂-(4-CH₂CH₂CH₂CO₂Me-1-Pyza) | 2-F | H | H |
| 2-179 | H | H | CH₂CH₂-(4-CH₂CH₂CH₂CO₂Et-1-Pyza) | 2-F | H | H |
| 2-180 | Ac | H | CH₂CH₂-(4-CH₂CH₂CH₂CO₂Et-1-Pyza) | 2-F | H | H |
| 2-181 | H | H | CH₂CH₂-(5-CH₂CH₂CH₂CO₂H-1-Pyza) | 2-F | H | H |
| 2-182 | Ac | H | CH₂CH₂-(5-CH₂CH₂CH₂CO₂H-1-Pyza) | 2-F | H | H |
| 2-183 | H | H | CH₂CH₂-(5-CH₂CH₂CH₂CO₂Me-1-Pyza) | 2-F | H | H |
| 2-184 | Ac | H | CH₂CH₂-(5-CH₂CH₂CH₂CO₂Me-1-Pyza) | 2-F | H | H |
| 2-185 | H | H | CH₂CH₂-(5-CH₂CH₂CH₂CO₂Et-1-Pyza) | 2-F | H | H |
| 2-186 | Ac | H | CH₂CH₂-(5-CH₂CH₂CH₂CO₂Et-1-Pyza) | 2-F | H | H |
| 2-187 | H | H | CH₂-3-Pyza | 2-F | H | H |
| 2-188 | Ac | H | CH₂-3-Pyza | 2-F | H | H |
| 2-189 | H | H | CH₂-3-Pyza | 2-F | 4-F | H |
| 2-190 | Ac | H | CH₂-3-Pyza | 2-F | 4-F | H |
| 2-191 | H | H | CH₂-(1-CO₂Me-3-Pyza) | 2-F | H | H |
| 2-192 | Ac | H | CH₂-(1-CO₂Me-3-Pyza) | 2-F | H | H |
| 2-193 | H | H | CH₂-(1-CO₂Et-3-Pyza) | 2-F | H | H |
| 2-194 | Ac | H | CH₂-(1-CO₂Et-3-Pyza) | 2-F | H | H |
| 2-195 | H | H | CH₂-(4-CO₂H-3-Pyza) | 2-F | H | H |
| 2-196 | Ac | H | CH₂-(4-CO₂H-3-Pyza) | 2-F | H | H |
| 2-197 | H | H | CH₂-(4-CO₂Me-3-Pyza) | 2-F | H | H |
| 2-198 | Ac | H | CH₂-(4-CO₂Me-3-Pyza) | 2-F | H | H |
| 2-199 | H | H | CH₂-(4-CO₂Et-3-Pyza) | 2-F | H | H |
| 2-200 | Ac | H | CH₂-(4-CO₂Et-3-Pyza) | 2-F | H | H |
| 2-201 | H | H | CH₂-(5-CO₂H-3-Pyza) | 2-F | H | H |
| 2-202 | Ac | H | CH₂-(5-CO₂H-3-Pyza) | 2-F | H | H |
| 2-203 | H | H | CH₂-(5-CO₂Me-3-Pyza) | 2-F | H | H |
| 2-204 | Ac | H | CH₂-(5-CO₂Me-3-Pyza) | 2-F | H | H |
| 2-205 | H | H | CH₂-(5-CO₂Et-3-Pyza) | 2-F | H | H |
| 2-206 | Ac | H | CH₂-(5-CO₂Et-3-Pyza) | 2-F | H | H |
| 2-207 | H | H | CH₂-(1-CH₂CO₂H-3-Pyza) | 2-F | H | H |
| 2-208 | Ac | H | CH₂-(1-CH₂CO₂H-3-Pyza) | 2-F | H | H |
| 2-209 | H | H | CH₂-(1-CH₂CO₂Me-3-Pyza) | 2-F | H | H |
| 2-210 | Ac | H | CH₂-(1-CH₂CO₂Me-3-Pyza) | 2-F | H | H |
| 2-211 | H | H | CH₂-(1-CH₂CO₂Et-3-Pyza) | 2-F | H | H |
| 2-212 | Ac | H | CH₂-(1-CH₂CO₂Et-3-Pyza) | 2-F | H | H |
| 2-213 | H | H | CH₂-(4-CH₂CO₂H-3-Pyza) | 2-F | H | H |
| 2-214 | Ac | H | CH₂-(4-CH₂CO₂H-3-Pyza) | 2-F | H | H |
| 2-215 | H | H | CH₂-(4-CH₂CO₂Me-3-Pyza) | 2-F | H | H |
| 2-216 | Ac | H | CH₂-(4-CH₂CO₂Me-3-Pyza) | 2-F | H | H |
| 2-217 | H | H | CH₂-(4-CH₂CO₂Et-3-Pyza) | 2-F | H | H |
| 2-218 | Ac | H | CH₂-(4-CH₂CO₂Et-3-Pyza) | 2-F | H | H |
| 2-219 | H | H | CH₂-(5-CH₂CO₂H-3-Pyza) | 2-F | H | H |
| 2-220 | Ac | H | CH₂-(5-CH₂CO₂H-3-Pyza) | 2-F | H | H |
| 2-221 | H | H | CH₂-(5-CH₂CO₂Me-3-Pyza) | 2-F | H | H |
| 2-222 | Ac | H | CH₂-(5-CH₂CO₂Me-3-Pyza) | 2-F | H | H |
| 2-223 | H | H | CH₂-(5-CH₂CO₂Et-3-Pyza) | 2-F | H | H |
| 2-224 | Ac | H | CH₂-(5-CH₂CO₂Et-3-Pyza) | 2-F | H | H |
| 2-225 | H | H | CH₂-(1-CH₂CH₂CO₂H-3-Pyza) | 2-F | H | H |
| 2-226 | Ac | H | CH₂-(1-CH₂CH₂O₂H-3-Pyza) | 2-F | H | H |
| 2-227 | H | H | CH₂-(1-CH₂CH₂CO₂Me-3-Pyza) | 2-F | H | H |
| 2-228 | Ac | H | CH₂-(-CH₂CH₂CO₂Me-3-Pyza) | 2-F | H | H |
| 2-229 | H | H | CH₂-(1-CH₂CH₂CO₂Et-3-Pyza) | 2-F | H | H |
| 2-230 | Ac | H | CH₂(1-CH₂CH₂CO₂Et-3-Pyza) | 2-F | H | H |
| 2-231 | H | H | CH₂-(4-CH₂CH₂CO₂H-3-Pyza) | 2-F | H | H |
| 2-232 | Ac | H | CH₂-(4-CH₂CH₂CO₂H-3-Pyza) | 2-F | H | H |
| 2-233 | H | H | CH₂-(4-CH₂CH₂CO₂Me-3-Pyza) | 2-F | H | H |
| 2-234 | Ac | H | CH₂-(4-CH₂CH₂CO₂Me-3-Pyza) | 2-F | H | H |
| 2-235 | H | H | CH₂-(4-CH₂CH₂CO₂Et-3-Pyza) | 2-F | H | H |
| 2-236 | Ac | H | CH₂-(4-CH₂CH₂CO₂Et-3-Pyza) | 2-F | H | H |
| 2-237 | H | H | CH₂-(5-CH₂CH₂CO₂H-3-Pyza) | 2-F | H | H |
| 2-238 | Ac | H | CH₂-(5-CH₂CH₂CO₂H-3-Pyza) | 2-F | H | H |
| 2-239 | H | H | CH₂-(5-CH₂CH₂CO₂Me-3-Pyza) | 2-F | H | H |
| 2-240 | Ac | H | CH₂-(5-CH₂CH₂CO₂Me-3-Pyza) | 2-F | H | H |
| 2-241 | H | H | CH₂-(5-CH₂CH₂CO₂Et-3-Pyza) | 2-F | H | H |
| 2-242 | Ac | H | CH₂-(5-CH₂CH₂CO₂Et-3-Pyza) | 2-F | H | H |
| 2-245 | H | H | CH₂-(1-CH₂CH₂CH₂CO₂H-3-Pyza) | 2-F | H | H |
| 2-246 | Ac | H | CH₂-(1-CH₂CH₂CH₂CO₂H-3-Pyza) | 2-F | H | H |
| 2-247 | H | H | CH₂-(1-CH₂CH₂CH₂CO₂Me-3-Pyza) | 2-F | H | H |
| 2-248 | Ac | H | CH₂-(1-CH₂CH₂CH₂CO₂Me-3-Pyza) | 2-F | H | H |
| 2-249 | H | H | CH₂-(1-CH₂CH₂CH₂CO₂Et-3-Pyza) | 2-F | H | H |
| 2-250 | Ac | H | CH₂-(1-CH₂CH₂CH₂CO₂Et-3-Pyza) | 2-F | H | H |
| 2-251 | H | H | CH₂-(4-CH₂CH₂CH₂CO₂H-3-Pyza) | 2-F | H | H |
| 2-252 | Ac | H | CH₂-(4-CH₂CH₂CH₂CO₂H-3-Pyza) | 2-F | H | H |
| 2-253 | H | H | CH₂-(4-CH₂CH₂CH₂CO₂Me-3-Pyza) | 2-F | H | H |
| 2-254 | Ac | H | CH₂-(4-CH₂CH₂CH₂CO₂Me-3-Pyza) | 2-F | H | H |
| 2-255 | H | H | CH₂-(4-CH₂CH₂CH₂CO₂Et-3-Pyza) | 2-F | H | H |
| 2-256 | Ac | H | CH₂-(4-CH₂CH₂CH₂CO₂Et-3-Pyza) | 2-F | H | H |
| 2-257 | H | H | CH₂-(5-CH₂CH₂CH₂CO₂H-3-Pyza) | 2-F | H | H |
| 2-258 | Ac | H | CH₂-(5-CH₂CH₂CH₂CO₂H-3-Pyza) | 2-F | H | H |
| 2-259 | H | H | CH₂-(5-CH₂CH₂CH₂CO₂Me-3-Pyza) | 2-F | H | H |
| 2-260 | Ac | H | CH₂-(5-CH₂CH₂CH₂CO₂Me-3-Pyza) | 2-F | H | H |
| 2-261 | H | H | CH₂-(5-CH₂CH₂CH₂CO₂Et-3-Pyza) | 2-F | H | H |
| 2-262 | Ac | H | CH₂-(5-CH₂CH₂CH₂CO₂Et-3-Pyza) | 2-F | H | H |
| 2-263 | H | H | CH₂CH₂-3-Pyza | 2-F | H | H |
| 2-264 | Ac | H | CH₂CH₂-3-Pyza | 2-F | H | H |
| 2-265 | H | H | CH₂CH₂-3-Pyza | 2-F | 4-F | H |
| 2-266 | Ac | H | CH₂CH₂-3-Pyza | 2-F | 4-F | H |
| 2-267 | H | H | CH₂CH₂-(1-CO₂Me-3-Pyza) | 2-F | H | H |
| 2-268 | Ac | H | CH₂CH₂-(1-CO₂Me-3-Pyza) | 2-F | H | H |
| 2-269 | H | H | CH₂CH₂-(1-CO₂Et-3-Pyza) | 2-F | H | H |
| 2-270 | Ac | H | CH₂CH₂-(1-CO₂Et-3-Pyza) | 2-F | H | H |
| 2-271 | H | H | CH₂CH₂-(4-CO₂H-3-Pyza) | 2-F | H | H |
| 2-272 | Ac | H | CH₂CH₂-(4-CO₂H-3-Pyza) | 2-F | H | H |
| 2-273 | H | H | CH₂CH₂-(4-CO₂Me-3-Pyza) | 2-F | H | H |
| 2-274 | Ac | H | CH₂CH₂-(4-CO₂Me-3-Pyza) | 2-F | H | H |
| 2-275 | H | H | CH₂CH₂-(4-CO₂Et-3-Pyza) | 2-F | H | H |
| 2-276 | Ac | H | CH₂CH₂-(4-CO₂Et-3-Pyza) | 2-F | H | H |
| 2-277 | H | H | CH₂CH₂-(5-CO₂H-3-Pyza) | 2-F | H | H |
| 2-278 | Ac | H | CH₂CH₂-(5-CO₂H-3-Pyza) | 2-F | H | H |
| 2-279 | H | H | CH₂CH₂-(5-CO₂Me-3-Pyza) | 2-F | H | H |
| 2-280 | Ac | H | CH₂CH₂-(5-CO₂Me-3-Pyza) | 2-F | H | H |
| 2-281 | H | H | CH₂CH₂-(5-CO₂Et-3-Pyza) | 2-F | H | H |
| 2-282 | Ac | H | CH₂CH₂-(5-CO₂Et-3-Pyza) | 2-F | H | H |
| 2-283 | H | H | CH₂CH₂-(-CH₂CO₂H-3-Pyza) | 2-F | H | H |
| 2-284 | Ac | H | CH₂CH₂-(1-CH₂CO₂H-3-Pyza) | 2-F | H | H |
| 2-285 | H | H | CH₂CH₂-(1-CH₂CO₂Me-3-Pyza) | 2-F | H | H |
| 2-286 | Ac | H | CH₂CH₂-(1-CH₂CO₂Me-3-Pyza) | 2-F | H | H |
| 2-287 | H | H | CH₂CH₂-(1-CH₂CO₂Et-3-Pyza) | 2-F | H | H |
| 2-288 | Ac | H | CH₂CH₂-(1-CH₂CO₂Et-3-Pyza) | 2-F | H | H |
| 2-289 | H | H | CH₂CH₂-(4-CH₂CO₂H-3-Pyza) | 2-F | H | H |
| 2-290 | Ac | H | CH₂CH₂-(4-CH₂CO₂H-3-Pyza) | 2-F | H | H |
| 2-291 | H | H | CH₂CH₂-(4-CH₂CO₂Me-3-Pyza) | 2-F | H | H |
| 2-292 | Ac | H | CH₂CH₂-(4-CH₂CO₂Me-3-Pyza) | 2-F | H | H |
| 2-293 | H | H | CH₂CH₂-(4-CH₂CO₂Et-3-Pyza) | 2-F | H | H |
| 2-294 | Ac | H | CH₂CH₂-(4-CH₂CO₂Et-3-Pyza) | 2-F | H | H |
| 2-295 | H | H | CH₂CH₂-(5-CH₂CO₂H-3-Pyza) | 2-F | H | H |
| 2-296 | Ac | H | CH₂CH₂-(5-CH₂CO₂H-3-Pyza) | 2-F | H | H |
| 2-297 | H | H | CH₂CH₂-(5-CH₂CO₂Me-3-Pyza) | 2-F | H | H |
| 2-298 | Ac | H | CH₂CH₂-(5-CH₂CO₂Me-3-Pyza) | 2-F | H | H |
| 2-299 | H | H | CH₂CH₂-(5-CH₂CO₂Et-3-Pyza) | 2-F | H | H |
| 2-300 | Ac | H | CH₂CH₂-(5-CH₂CO₂Et-3-Pyza) | 2-F | H | H |
| 2-301 | H | H | CH₂CH₂-(1-CH₂CH₂CO₂H-3-Pyza) | 2-F | H | H |
| 2-302 | Ac | H | CH₂CH₂-(1-CH₂CH₂CO₂H-3-Pyza) | 2-F | H | H |
| 2-303 | H | H | CH₂CH₂-(1-CH₂CH₂CO₂Me-3-Pyza) | 2-F | H | H |
| 2-304 | Ac | H | CH₂CH₂-(1-CH₂CH₂CO₂Me-3-Pyza) | 2-F | H | H |
| 2-305 | H | H | CH₂CH₂-(1-CH₂CH₂CO₂Et-3-Pyza) | 2-F | H | H |
| 2-306 | Ac | H | CH₂CH₂-(1-CH₂CH₂CO₂Et-3-Pyza) | 2-F | H | H |
| 2-307 | H | H | CH₂CH₂-(4-CH₂CH₂CO₂H-3-Pyza) | 2-F | H | H |
| 2-308 | Ac | H | CH₂CH₂-(4-CH₂CH₂CO₂H-3-Pyza) | 2-F | H | H |
| 2-309 | H | H | CH₂CH₂-(4-CH₂CH₂CO₂Me-3-Pyza) | 2-F | H | H |
| 2-310 | Ac | H | CH₂CH₂-(4-CH₂CH₂CO₂Me-3-Pyza) | 2-F | H | H |
| 2-311 | H | H | CH₂CH₂-(4-CH₂CH₂CO₂Et-3-Pyza) | 2-F | H | H |
| 2-312 | Ac | H | CH₂CH₂-(4-CH₂CH₂CO₂Et-3-Pyza) | 2-F | H | H |
| 2-313 | H | H | CH₂CH₂-(5-CH₂CH₂CO₂H-3-Pyza) | 2-F | H | H |
| 2-314 | Ac | H | CH₂CH₂-(5-CH₂CH₂CO₂H-3-Pyza) | 2-F | H | H |
| 2-315 | H | H | CH₂CH₂-(5-CH₂CH₂CO₂Me-3-Pyza) | 2-F | H | H |
| 2-316 | Ac | H | CH₂CH₂-(5-CH₂CH₂CO₂Me-3-Pyza) | 2-F | H | H |
| 2-317 | H | H | CH₂CH₂-(5-CH₂CH₂CO₂Et-3-Pyza) | 2-F | H | H |
| 2-318 | Ac | H | CH₂CH₂-(5-CH₂CH₂CO₂Et-3-Pyza) | 2-F | H | H |
| 2-319 | H | H | CH₂CH₂-(1-CH₂CH₂CH₂CO₂H-3-Pyza) | 2-F | H | H |
| 2-320 | Ac | H | CH₂CH₂-(1-CH₂CH₂CH₂CO₂H-3-Pyza) | 2-F | H | H |
| 2-321 | H | H | CH₂CH₂-(1-CH₂CH₂CH₂CO₂Me-3-Pyza) | 2-F | H | H |
| 2-322 | Ac | H | CH₂CH₂-(1-CH₂CH₂CH₂CO₂Me-3-Pyza) | 2-F | H | H |
| 2-323 | H | H | CH₂CH₂-(1-CH₂CH₂CH₂CO₂Et-3-Pyza) | 2-F | H | H |
| 2-324 | Ac | H | CH₂CH₂-(1-CH₂CH₂CH₂CO₂Et-3-Pyza) | 2-F | H | H |
| 2-325 | H | H | CH₂CH₂-(4-CH₂CH₂CH₂CO₂H-3-Pyza) | 2-F | H | H |
| 2-326 | Ac | H | CH₂CH₂-(4-CH₂CH₂CH₂CO₂H-3-Pyza) | 2-F | H | H |
| 2-327 | H | H | CH₂CH₂-(4-CH₂CH₂CH₂CO₂Me-3-Pyza) | 2-F | H | H |
| 2-328 | Ac | H | CH₂CH₂-(4-CH₂CH₂CH₂CO₂Me-3-Pyza) | 2-F | H | H |
| 2-329 | H | H | CH₂CH₂-(4-CH₂CH₂CH₂CO₂Et-3-Pyza) | 2-F | H | H |
| 2-330 | Ac | H | CH₂CH₂-(4-CH₂CH₂CH₂CO₂Et-3-Pyza) | 2-F | H | H |
| 2-331 | H | H | CH₂CH₂-(5-CH₂CH₂CH₂CO₂H-3-Pyza) | 2-F | H | H |
| 2-332 | Ac | H | CH₂CH₂-(5-CH₂CH₂CH₂CO₂H-3-Pyza) | 2-F | H | H |
| 2-333 | H | H | CH₂CH₂-(5-CH₂CH₂CH₂CO₂Me-3-Pyza) | 2-F | H | H |
| 2-334 | Ac | H | CH₂CH₂-(5-CH₂CH₂CH₂CO₂Me-3-Pyza) | 2-F | H | H |
| 2-335 | H | H | CH₂CH₂-(5-CH₂CH₂CH₂CO₂Et-3-Pyza) | 2-F | H | H |
| 2-336 | Ac | H | CH₂CH₂-(5-CH₂CH₂CH₂CO₂Et-3-Pyza) | 2-F | H | H |
| 2-337 | H | H | CH₂-4-Pyza | 2-F | H | H |
| 2-338 | Ac | H | CH₂-4-Pyza | 2-F | H | H |
| 2-339 | H | H | CH₂-4-Pyza | 2-F | 4-F | H |
| 2-340 | Ac | H | CH₂-4-Pyza | 2-F | 4-F | H |
| 2-341 | H | H | CH₂-(1-CO₂Me-4-Pyza) | 2-F | H | H |
| 2-342 | Ac | H | CH₂-(1-CO₂Me-4-Pyza) | 2-F | H | H |
| 2-343 | H | H | CH₂-(1-CO₂Et-4-Pyza) | 2-F | H | H |
| 2-344 | Ac | H | CH₂-(1-CO₂Et-4-Pyza) | 2-F | H | H |
| 2-345 | H | H | CH₂-(3-CO₂H-4-Pyza) | 2-F | H | H |
| 2-346 | Ac | H | CH₂-(3-CO₂H-4-Pyza) | 2-F | H | H |
| 2-347 | H | H | CH₂-(3-CO₂Me-4-Pyza) | 2-F | H | H |
| 2-348 | Ac | H | CH₂-(3-CO₂Me-4-Pyza) | 2-F | H | H |
| 2-349 | H | H | CH₂-(3-CO₂Et-4-Pyza) | 2-F | H | H |
| 2-350 | Ac | H | CH₂-(3-CO₂Et-4-Pyza) | 2-F | H | H |
| 2-351 | H | H | CH₂-(1-CH₂CO₂H-4-Pyza) | 2-F | H | H |
| 2-352 | Ac | H | CH₂-(1-CH₂CO₂H-4-Pyza) | 2-F | H | H |
| 2-353 | H | H | CH₂-(1-CH₂CO₂Me-4-Pyza) | 2-F | H | H |
| 2-354 | Ac | H | CH₂-(1-CH₂CO₂Me-4-Pyza) | 2-F | H | H |
| 2-355 | H | H | CH₂-(1-CH₂CO₂Et-4-Pyza) | 2-F | H | H |
| 2-356 | Ac | H | CH₂-(-CH₂CO₂Et-4-Pyza) | 2-F | H | H |
| 2-357 | H | H | CH₂-(3-CH₂CO₂H-4-Pyza) | 2-F | H | H |
| 2-358 | Ac | H | CH₂-(3-CH₂CO₂H-4-Pyza) | 2-F | H | H |
| 2-359 | H | H | CH₂-(3-CH₂CO₂Me-4-Pyza) | 2-F | H | H |
| 2-360 | Ac | H | CH₂-(3-CH₂CO₂Me-4-Pyza) | 2-F | H | H |
| 2-361 | H | H | CH₂-(3-CH₂CO₂Et-4-Pyza) | 2-F | H | H |
| 2-362 | Ac | H | CH₂-(3-CH₂CO₂Et-4-Pyza) | 2-F | H | H |
| 2-363 | H | H | CH₂-(1-CH₂CH₂CO₂H-4-Pyza) | 2-F | H | H |
| 2-364 | Ac | H | CH₂-(1-CH₂CH₂CO₂H-4-Pyza) | 2-F | H | H |
| 2-365 | H | H | CH₂-(1-CH₂CH₂CO₂Me-4-Pyza) | 2-F | H | H |
| 2-366 | Ac | H | CH₂-(1-CH₂CH₂CO₂Me-4-Pyza) | 2-F | H | H |
| 2-367 | H | H | CH₂-(1-CH₂CH₂CO₂Et-4-Pyza) | 2-F | H | H |
| 2-368 | Ac | H | CH₂-(1-CH₂CH₂CO₂Et-4-Pyza) | 2-F | H | H |
| 2-369 | H | H | CH₂-(3-CH₂CH₂CO₂H-4-Pyza) | 2-F | H | H |
| 2-370 | Ac | H | CH₂-(3-CH₂CH₂CO₂H-4-Pyza) | 2-F | H | H |
| 2-371 | H | H | CH₂-(3-CH₂CH₂CO₂Me-4-Pyza) | 2-F | H | H |
| 2-372 | Ac | H | CH₂-(3-CH₂CH₂CO₂Me-4-Pyza) | 2-F | H | H |
| 2-373 | H | H | CH₂-(3-CH₂CH₂CO₂Et-4-Pyza) | 2-F | H | H |
| 2-374 | Ac | H | CH₂-(3-CH₂CH₂CO₂Et-4-Pyza) | 2-F | H | H |
| 2-375 | H | H | CH₂-(1-CH₂CH₂CH₂CO₂H-4-Pyza) | 2-F | H | H |
| 2-376 | Ac | H | CH₂-(1-CH₂CH₂CH₂CO₂H-4-Pyza) | 2-F | H | H |
| 2-377 | H | H | CH₂-(1-CH₂CH₂CH₂CO₂Me-4-Pyza) | 2-F | H | H |
| 2-378 | Ac | H | CH₂-(1-CH₂CH₂CH₂CO₂Me-4-Pyza) | 2-F | H | H |
| 2-379 | H | H | CH₂-(1-CH₂CH₂CH₂CO₂Et-4-Pyza) | 2-F | H | H |
| 2-380 | Ac | H | CH₂-(1-CH₂CH₂CH₂CO₂Et-4-Pyza) | 2-F | H | H |
| 2-381 | H | H | CH₂-(3-CH₂CH₂CH₂CO₂H-4-Pyza) | 2-F | H | H |
| 2-382 | Ac | H | CH₂-(3-CH₂CH₂CH₂CO₂H-4-Pyza) | 2-F | H | H |
| 2-383 | H | H | CH₂-(3-CH₂CH₂CH₂CO₂Me-4-Pyza) | 2-F | H | H |
| 2-384 | Ac | H | CH₂-(3-CH₂CH₂CH₂CO₂Me-4-Pyza) | 2-F | H | H |
| 2-385 | H | H | CH₂-(3-CH₂CH₂CH₂CO₂Et-4-Pyza) | 2-F | H | H |
| 2-386 | Ac | H | CH₂-(3-CH₂CH₂CH₂CO₂Et-4-Pyza) | 2-F | H | H |
| 2-387 | H | H | CH₂CH₂-4-Pyza | 2-F | H | H |
| 2-388 | Ac | H | CH₂CH₂-4-Pyza | 2-F | H | H |
| 2-389 | H | H | CH₂CH₂-4-Pyza | 2-F | 4-F | H |
| 2-390 | Ac | H | CH₂CH₂-4-Pyza | 2-F | 4-F | H |
| 2-391 | H | H | CH₂CH₂-(1-CO₂Me-4-Pyza) | 2-F | H | H |
| 2-392 | Ac | H | CH₂CH₂(1-CO₂Me-4-Pyza) | 2-F | H | H |
| 2-393 | H | H | CH₂CH₂-(1-CO₂Et-4-Pyza) | 2-F | H | H |
| 2-394 | Ac | H | CH₂CH₂-(1-CO₂Et-4-Pyza) | 2-F | H | H |
| 2-395 | H | H | CH₂CH₂-(3-CO₂H-4-Pyza) | 2-F | H | H |
| 2-396 | Ac | H | CH₂CH₂-(3-CO₂H-4-Pyza) | 2-F | H | H |
| 2-397 | H | H | CH₂CH₂-(3-CO₂Me-4-Pyza) | 2-F | H | H |
| 2-398 | Ac | H | CH₂CH₂-(3-CO₂Me-4-Pyza) | 2-F | H | H |
| 2-399 | H | H | CH₂CH₂-(3-CO₂Et-4-Pyza) | 2-F | H | H |
| 2-400 | Ac | H | CH₂CH₂-(3-CO₂Et-4-Pyza) | 2-F | H | H |
| 2-401 | H | H | CH₂CH₂-(1-CH₂CO₂H-4-Pyza) | 2-F | H | H |
| 2-402 | Ac | H | CH₂CH₂-(1-CH₂CO₂H-4-Pyza) | 2-F | H | H |
| 2-403 | H | H | CH₂CH₂-(1-CH₂CO₂Me-4-Pyza) | 2-F | H | H |
| 2-404 | Ac | H | CH₂CH₂-(1-CH₂CO₂Me-4-Pyza) | 2-F | H | H |
| 2-405 | H | H | CH₂CH₂-(1-CH₂CO₂Et-4-Pyza) | 2-F | H | H |
| 2-406 | Ac | H | CH₂CH₂-(1-CH₂CO₂Et-4-Pyza) | 2-F | H | H |
| 2-407 | H | H | CH₂CH₂-(3-CH₂CO₂H-4-Pyza) | 2-F | H | H |
| 2-408 | Ac | H | CH₂CH₂-(3-CH₂CO₂H-4-Pyza) | 2-F | H | H |
| 2-409 | H | H | CH₂CH₂-(3-CH₂CO₂Me-4-Pyza) | 2-F | H | H |
| 2-410 | Ac | H | CH₂CH₂-(3-CH₂CO₂Me-4-Pyza) | 2-F | H | H |
| 2-411 | H | H | CH₂CH₂-(3-CH₂CO₂Et-4-Pyza) | 2-F | H | H |
| 2-412 | Ac | H | CH₂CH₂-(3-CH₂CO₂Et-4-Pyza) | 2-F | H | H |
| 2-413 | H | H | CH₂CH₂-(1-CH₂CH₂CO₂H-4-Pyza) | 2-F | H | H |
| 2-414 | Ac | H | CH₂CH₂-(1-CH₂CH₂CO₂H-4-Pyza) | 2-F | H | H |
| 2-415 | H | H | CH₂CH₂-(1-CH₂CH₂CO₂Me-4-Pyza) | 2-F | H | H |
| 2-416 | Ac | H | CH₂CH₂-(1-CH₂CH₂CO₂Me-4-Pyza) | 2-F | H | H |
| 2-417 | H | H | CH₂CH₂-(1-CH₂CH₂CO₂Et-4-Pyza) | 2-F | H | H |
| 2-418 | Ac | H | CH₂CH₂-(1-CH₂CH₂CO₂Et-4-Pyza) | 2-F | H | H |
| 2-419 | H | H | CH₂CH₂-(3-CH₂CH₂CO₂H-4-Pyza) | 2-F | H | H |
| 2-420 | Ac | H | CH₂CH₂-(3-CH₂CH₂CO₂H-4-Pyza) | 2-F | H | H |
| 2-421 | H | H | CH₂CH₂-(3-CH₂CH₂CO₂Me-4-Pyza) | 2-F | H | H |
| 2-422 | Ac | H | CH₂CH₂-(3-CH₂CH₂CO₂Me-4-Pyza) | 2-F | H | H |
| 2-423 | H | H | CH₂CH₂-(3-CH₂CH₂CO₂Et-4-Pyza) | 2-F | H | H |
| 2-424 | Ac | H | CH₂CH₂-(3-CH₂CH₂CO_{z}Et-4-Pyza) | 2-F | H | H |
| 2-425 | H | H | CH₂CH₂-(1-CH₂CH₂CH₂CO₂H-4-Pyza) | 2-F | H | H |
| 2-426 | Ac | H | CH₂CH₂-(1-CH₂CH₂CH₂CO₂H-4-Pyza) | 2-F | H | H |
| 2-427 | H | H | CH₂CH₂-(1-CH₂CH₂CH₂CO₂Me-4-Pyza) | 2-F | H | H |
| 2-428 | Ac | H | CH₂CH₂-(1-CH₂CH₂CH₂CO₂Me-4-Pyza) | 2-F | H | H |
| 2-429 | H | H | CH₂CH₂-(1-CH₂CH₂CH₂CO₂Et-4-Pyza) | 2-F | H | H |
| 2-430 | Ac | H | CH₂CH₂-(1-CH₂CH₂CH₂CO₂Et-4-Pyza) | 2-F | H | H |
| 2-431 | H | H | CH₂CH₂-(3-CH₂CH₂CH₂CO₂H-4-Pyza) | 2-F | H | H |
| 2-432 | Ac | H | CH₂CH₂-(3-CH₂CH₂CH₂CO₂H-4-Pyza) | 2-F | H | H |
| 2-433 | H | H | CH₂CH₂-(3-CH₂CH₂CH₂CO₂Me-4-Pyza) | 2-F | H | H |
| 2-434 | Ac | H | CH₂CH₂-(3-CH₂CH₂CH₂CO₂Me-4-Pyza) | 2-F | H | H |
| 2-435 | H | H | CH₂CH₂-(3-CH₂CH₂CH₂CO₂Et-4-Pyza) | 2-F | H | H |
| 2-436 | Ac | H | CH₂CH₂-(3-CH₂CH₂CH₂CO₂Et-4-Pyza) | 2-F | H | H |
| 2-437 | H | H | CH₂-(1-CO₂Me-5-Pyza) | 2-F | 4-F | H |
| 2-438 | Ac | H | CH₂-(1-CO₂Me-5-Pyza) | 2-F | 4-F | H |
| 2-439 | H | H | CH₂-(1-CO₂Me-5-Pyza) | 2-F | H | H |
| 2-440 | Ac | H | CH₂-(1-CO₂Me-5-Pyza) | 2-F | H | H |
| 2-441 | H | H | CH₂-(1-CO₂Et-5-Pyza) | 2-F | H | H |
| 2-442 | Ac | H | CH₂-(1-CO₂Et-5-Pyza) | 2-F | H | H |
| 2-443 | H | H | CH₂-(1-CH₂CO₂H-5-Pyza) | 2-F | H | H |
| 2-444 | Ac | H | CH₂-(1-CH₂CO₂H-5-Pyza) | 2-F | H | H |
| 2-445 | H | H | CH₂-(1-CH₂CO₂Me-5-Pyza) | 2-F | H | H |
| 2-446 | Ac | H | CH₂-(1-CH₂CO₂Me-5-Pyza) | 2-F | H | H |
| 2-447 | H | H | CH₂-(1-CH₂CO₂Et-5-Pyza) | 2-F | H | H |
| 2-448 | Ac | H | CH₂-(1-CH₂CO₂Et-5-Pyza) | 2-F | H | H |
| 2-449 | H | H | CH₂-(1-CH₂CH₂CO₂H-5-Pyza) | 2-F | H | H |
| 2-450 | Ac | H | CH₂-(1-CH₂CH₂CO₂H-5-Pyza) | 2-F | H | H |
| 2-451 | H | H | CH₂-(1-CH₂CH₂CO₂Me-5-Pyza) | 2-F | H | H |
| 2-452 | Ac | H | CH₂-(1-CH₂CH₂CO₂Me-5-Pyza) | 2-F | H | H |
| 2-453 | H | H | CH₂-(1-CH₂CH₂CO₂Et-5-Pyza) | 2-F | H | H |
| 2-454 | Ac | H | CH₂-(1-CH₂CH₂CO₂Et-5-Pyza) | 2-F | H | H |
| 2-455 | H | H | CH₂-(1-CH₂CH₂CH₂CO₂H-5-Pyza) | 2-F | H | H |
| 2-456 | Ac | H | CH₂-(1-CH₂CH₂CH₂CO₂H-5-Pyza) | 2-F | H | H |
| 2-457 | H | H | CH₂-(1-CH₂CH₂CH₂CO₂Me-5-Pyza) | 2-F | H | H |
| 2-458 | Ac | H | CH₂-(1-CH₂CH₂CH₂CO₂Me-5-Pyza) | 2-F | H | H |
| 2-459 | H | H | CH₂-(1-CH₂CH₂CH₂CO₂Et-5-Pyza) | 2-F | H | H |
| 2-460 | Ac | H | CH₂-(1-CH₂CH₂CH₂CO₂Et-5-Pyza) | 2-F | H | H |
| 2-461 | H | H | CH₂CH₂-(1-CO₂Me-5-Pyza) | 2-F | 4-F | H |
| 2-462 | Ac | H | CH₂CH₂-(1-CO₂Me-5-Pyza) | 2-F | 4-F | H |
| 2-463 | H | H | CH₂CH₂-(1-CO₂Me-5-Pyza) | 2-F | H | H |
| 2-464 | Ac | H | CH₂CH₂-(1-CO₂Me-5-Pyza) | 2-F | H | H |
| 2-465 | H | H | CH₂CH₂-(1-CO₂Et-5-Pyza) | 2-F | H | H |
| 2-466 | Ac | H | CH₂CH₂-(1-CO₂Et-5-Pyza) | 2-F | H | H |
| 2-467 | H | H | CH₂CH₂-(1-CH₂CO₂H-5-Pyza) | 2-F | H | H |
| 2-468 | Ac | H | CH₂CH₂-(1-CH₂CO₂H-5-Pyza) | 2-F | H | H |
| 2-469 | H | H | CH₂CH₂-(1-CH₂CO₂Me-5-Pyza) | 2-F | H | H |
| 2-470 | Ac | H | CH₂CH₂-(1-CH₂CO₂Me-5-Pyza) | 2-F | H | H |
| 2-471 | H | H | CH₂CH₂-(1-CH₂CO₂Et-5-Pyza) | 2-F | H | H |
| 2-472 | Ac | H | CH₂CH₂-(1-CH₂CO₂Et-5-Pyza) | 2-F | H | H |
| 2-473 | H | H | CH₂CH₂-(1-CH₂CH₂CO₂H-5-Pyza) | 2-F | H | H |
| 2-474 | Ac | H | CH₂CH₂-(1-CH₂CH₂CO₂H-5-Pyza) | 2-F | H | H |
| 2-475 | H | H | CH₂CH₂-(1-CH₂CH₂CO₂Me-5-Pyza) | 2-F | H | H |
| 2-476 | Ac | H | CH₂CH₂-(1-CH₂CH₂CO₂Me-5-Pyza) | 2-F | H | H |
| 2-477 | H | H | CH₂CH₂-(1-CH₂CH₂CO₂Et-5-Pyza) | 2-F | H | H |
| 2-478 | Ac | H | CH₂CH₂-(1-CH₂CH₂CO₂Et-5-Pyza) | 2-F | H | H |
| 2-479 | H | H | CH₂CH₂-(1-CH₂CH₂CH₂CO₂H-5-Pyza) | 2-F | H | H |
| 2-480 | Ac | H | CH₂CH₂-(1-CH₂CH₂CH₂CO₂H-5-Pyza) | 2-F | H | H |
| 2-481 | H | H | CH₂CH₂-(1-CH₂CH₂CH₂CO₂Me-5-Pyza) | 2-F | H | H |
| 2-482 | Ac | H | CH₂CH₂-(1-CH₂CH₂CH₂CO₂Me-5-Pyza) | 2-F | H | H |
| 2-483 | H | H | CH₂CH₂-(1-CH₂CH₂CH₂CO₂Et-5-Pyza) | 2-F | H | H |
| 2-484 | Ac | H | CH₂CH₂-(1-CH₂CH₂CH₂CO₂Et-5-Pyza) | 2-F | H | H |
| 2-485 | H | H | CH₂-1-Triz | 2-F | H | H |
| 2-486 | Ac | H | CH₂-1-Triz | 2-F | H | H |
| 2-487 | H | H | CH₂-(4-CO₂H-1-Triz) | 2-F | H | H |
| 2-488 | Ac | H | CH₂-(4-CO₂H-1-Triz) | 2-F | H | H |
| 2-489 | H | H | CH₂-(4-CO₂Me-1-Triz) | 2-F | H | H |
| 2-490 | Ac | H | CH₂-(4-CO₂Me-1-Triz) | 2-F | H | H |
| 2-491 | H | H | CH₂-(4-CO₂Et-1-Triz) | 2-F | H | H |
| 2-492 | Ac | H | CH₂-(4-CO₂Et-1-Triz) | 2-F | H | H |
| 2-493 | H | H | CH₂-(5-CO₂H-1-Triz) | 2-F | H | H |
| 2-494 | Ac | H | CH₂-(5-CO₂H-1-Triz) | 2-F | H | H |
| 2-495 | H | H | CH₂-(5-CO₂Me-1-Triz) | 2-F | H | H |
| 2-496 | Ac | H | CH₂-(5-CO₂Me-1-Triz) | 2-F | H | H |
| 2-497 | H | H | CH₂-(5-CO₂Et-1-Triz) | 2-F | H | H |
| 2-498 | Ac | H | CH₂-(5-CO₂Et-1-Triz) | 2-F | H | H |
| 2-499 | H | H | CH₂-(4-CH₂CO₂H-1-Triz) | 2-F | H | H |
| 2-500 | Ac | H | CH₂-(4-CH₂CO₂H-1-Triz) | 2-F | H | H |
| 2-501 | H | H | CH₂-(4-CH₂CO₂Me-1-Triz) | 2-F | H | H |
| 2-502 | Ac | H | CH₂-(4-CH₂CO₂Me-1-Triz) | 2-F | H | H |
| 2-503 | H | H | CH₂-(4-CH₂CO₂Et-1-Triz) | 2-F | H | H |
| 2-504 | Ac | H | CH₂-(4-CH₂CO₂Et-1-Triz) | 2-F | H | H |
| 2-505 | H | H | CH₂-(5-CH₂CO₂H-1-Triz) | 2-F | H | H |
| 2-506 | Ac | H | CH₂-(5-CH₂CO₂H-1-Triz) | 2-F | H | H |
| 2-507 | H | H | CH₂-(5-CH₂CO₂Me-1-Triz) | 2-F | H | H |
| 2-508 | Ac | H | CH₂-(5-CH₂CO₂Me-1-Triz) | 2-F | H | H |
| 2-509 | H | H | CH₂-(5-CH₂CO₂Et-1-Triz) | 2-F | H | H |
| 2-510 | Ac | H | CH₂-(5-CH₂CO₂Et-1-Triz) | 2-F | H | H |
| 2-511 | H | H | CH₂-(4-CH₂CH₂CO₂H-1-Triz) | 2-F | H | H |
| 2-512 | Ac | H | CH₂-(4-CH₂CH₂CO₂H-1-Triz) | 2-F | H | H |
| 2-513 | H | H | CH₂-(4-CH₂CH₂CO₂Me-1-Triz) | 2-F | H | H |
| 2-514 | Ac | H | CH₂-(4-CH₂CH₂CO₂Me-1-Triz) | 2-F | H | H |
| 2-515 | H | H | CH₂-(4-CH₂CH₂CO₂Et-1-Triz) | 2-F | H | H |
| 2-516 | Ac | H | CH₂-(4-CH₂CH₂CO₂Et-1-Triz) | 2-F | H | H |
| 2-517 | H | H | CH₂-(5-CH₂CH₂CO₂H-1-Triz) | 2-F | H | H |
| 2-518 | Ac | H | CH₂-(5-CH₂CH₂CO₂H-1-Triz) | 2-F | H | H |
| 2-519 | H | H | CH₂-(5-CH₂CH₂CO₂Me-1-Triz) | 2-F | H | H |
| 2-520 | Ac | H | CH₂-(5-CH₂CH₂CO₂Me-1-Triz) | 2-F | H | H |
| 2-521 | H | H | CH₂-(5-CH₂CH₂CO₂Et-1-Triz) | 2-F | H | H |
| 2-522 | Ac | H | CH₂-(5-CH₂CH₂CO₂Et-1-Triz) | 2-F | H | H |
| 2-523 | H | H | CH₂-(4-CH₂CH₂CH₂CO₂H-1-Triz) | 2-F | H | H |
| 2-524 | Ac | H | CH₂-(4-CH₂CH₂CH₂CO₂H-1-Triz) | 2-F | H | H |
| 2-525 | H | H | CH₂-(4-CH₂CH₂CH₂CO₂Me-1-Triz) | 2-F | H | H |
| 2-526 | Ac | H | CH₂-(4-CH₂CH₂CH₂CO₂Me-1-Triz) | 2-F | H | H |
| 2-527 | H | H | CH₂-(4-CH₂CH₂CH₂CO₂Et-1-Triz) | 2-F | H | H |
| 2-528 | Ac | H | CH₂-(4-CH₂CH₂CH₂CO₂Et-1-Triz) | 2-F | H | H |
| 2-529 | H | H | CH₂-(5-CH₂CH₂CH₂CO₂H-1-Triz) | 2-F | H | H |
| 2-530 | Ac | H | CH₂-(5-CH₂CH₂CH₂CO₂H-1-Triz) | 2-F | H | H |
| 2-531 | H | H | CH₂(5-CH₂CH₂CH₂CO₂Me-1-Triz) | 2-F | H | H |
| 2-532 | Ac | H | CH₂-(5-CH₂CH₂CH₂CO₂Me-1-Triz) | 2-F | H | H |
| 2-533 | H | H | CH₂-(5-CH₂CH₂CH₂CO₂Et-1-Triz) | 2-F | H | H |
| 2-534 | Ac | H | CH₂-(5-CH₂CH₂CH₂CO₂Et-1-Triz) | 2-F | H | H |
| 2-535 | H | H | CH₂CH₂-1-Triz | 2-F | H | H |
| 2-356[TY2] | Ac | H | CH₂CH₂-1-Triz | 2-F | H | H |
| 2-537 | H | H | CH₂CH₂-(4-CO₂H-1-Triz) | 2-F | H | H |
| 2-538 | Ac | H | CH₂CH₂-(4-CO₂H-1-Triz) | 2-F | H | H |
| 2-539 | H | H | CH₂CH₂-(4-CO₂Me-1-Triz) | 2-F | H | H |
| 2-540 | Ac | H | CH₂CH₂-(4-CO₂Me-1-Triz) | 2-F | H | H |
| 2-541 | H | H | CH₂CH₂-(4-CO₂Et-1-Triz) | 2-F | H | H |
| 2-542 | Ac | H | CH₂CH₂-(4-CO₂Et-1-Triz) | 2-F | H | H |
| 2-543 | H | H | CH₂CH₂-(5-CO₂H-1-Triz) | 2-F | H | H |
| 2-544 | Ac | H | CH₂CH₂-(5-CO₂H-1-Triz) | 2-F | H | H |
| 2-545 | H | H | CH₂CH₂-(5-CO₂Me-1-Triz) | 2-F | H | H |
| 2-546 | Ac | H | CH₂CH₂-(5-CO₂Me-1-Triz) | 2-F | H | H |
| 2-547 | H | H | CH₂CH₂-(5-CO₂Et-1-Triz) | 2-F | H | H |
| 2-548 | Ac | H | CH₂CH₂-(5-CO₂Et-1-Triz) | 2-F | H | H |
| 2-549 | H | H | CH₂CH₂-(4-CH₂CO₂H-1-Triz) | 2-F | H | H |
| 2-550 | Ac | H | CH₂CH₂-(4-CH₂CO₂H-1-Triz) | 2-F | H | H |
| 2-551 | H | H | CH₂CH₂-(4-CH₂CO₂Me-1-Triz) | 2-F | H | H |
| 2-552 | Ac | H | CH₂CH₂-(4-CH₂CO₂Me-1-Triz) | 2-F | H | H |
| 2-553 | H | H | CH₂CH₂-(4-CH₂CO₂Et-1-Triz) | 2-F | H | H |
| 2-554 | Ac | H | CH₂CH₂-(4-CH₂CO₂Et-1-Triz) | 2-F | H | H |
| 2-555 | H | H | CH₂CH₂-(5-CH₂CO₂H-1-Triz) | 2-F | H | H |
| 2-556 | Ac | H | CH₂CH₂-(5-CH₂CO₂H-1-Triz) | 2-F | H | H |
| 2-557 | H | H | CH₂CH₂-(5-CH₂CO₂Me-1-Triz) | 2-F | H | H |
| 2-558 | Ac | H | CH₂CH₂-(5-CH₂CO₂Me-1-Triz) | 2-F | H | H |
| 2-559 | H | H | CH₂CH₂-(5-CH₂CO₂Et-1-Triz) | 2-F | H | H |
| 2-560 | Ac | H | CH₂CH₂-(5-CH₂CO₂Et-1-Triz) | 2-F | H | H |
| 2-561 | H | H | CH₂CH₂-(4-CH₂CH₂CO₂H-1-Triz) | 2-F | H | H |
| 2-562 | Ac | H | CH₂CH₂-(4-CH₂CH₂CO₂H-1-Triz) | 2-F | H | H |
| 2-563 | H | H | CH₂CH₂-(4-CH₂CH₂CO₂Me-1-Triz) | 2-F | H | H |
| 2-564 | Ac | H | CH₂CH₂-(4-CH₂CH₂CO₂Me-1-Triz) | 2-F | H | H |
| 2-565 | H | H | CH₂CH₂-(4-CH₂CH₂CO₂Et-1-Triz) | 2-F | H | H |
| 2-566 | Ac | H | CH₂CH₂-(4-CH₂CH₂CO₂Et-1-Triz) | 2-F | H | H |
| 2-567 | H | H | CH₂CH₂-(5-CH₂CH₂CO₂H-1-Triz) | 2-F | H | H |
| 2-568 | Ac | H | CH₂CH₂-(5-CH₂CH₂CO₂H-1-Triz) | 2-F | H | H |
| 2-569 | H | H | CH₂CH₂-(5-CH₂CH₂CO₂Me-1-Triz) | 2-F | H | H |
| 2-570 | Ac | H | CH₂CH₂-(5-CH₂CH₂CO₂Me-1-Triz) | 2-F | H | H |
| 2-571 | H | H | CH₂CH₂-(5-CH₂CH₂CO₂Et-1-Triz) | 2-F | H | H |
| 2-572 | Ac | H | CH₂CH₂-(5-CH₂CH₂CO₂Et-1-Triz) | 2-F | H | H |
| 2-573 | H | H | CH₂CH₂-(4-CH₂CH₂CH₂CO₂H-1-Triz) | 2-F | H | H |
| 2-574 | Ac | H | CH₂CH₂-(4-CH₂CH₂CH₂CO₂H-1-Triz) | 2-F | H | H |
| 2-575 | H | H | CH₂CH₂-(4-CH₂CH₂CH₂CO₂Me-1-Triz) | 2-F | H | H |
| 2-576 | Ac | H | CH₂CH₂-(4-CH₂CH₂CH₂CO₂Me-1-Triz) | 2-F | H | H |
| 2-577 | H | H | CH₂CH₂-(4-CH₂CH₂CH₂CO₂Et-1-Triz) | 2-F | H | H |
| 2-578 | Ac | H | CH₂CH₂-(4-CH₂CH₂CH₂CO₂Et-1-Triz) | 2-F | H | H |
| 2-579 | H | H | CH₂CH₂-(5-CH₂CH₂CH₂CO₂H-1-Triz) | 2-F | H | H |
| 2-580 | Ac | H | CH₂CH₂-(5-CH₂CH₂CH₂CO₂H-1-Triz) | 2-F | H | H |
| 2-581 | H | H | CH₂CH₂-(5-CH₂CH₂CH₂CO₂Me-1-Triz) | 2-F | H | H |
| 2-582 | Ac | H | CH₂CH₂-(5-CH₂CH₂CH₂CO₂Me-1-Triz) | 2-F | H | H |
| 2-583 | H | H | CH₂CH₂-(5-CH₂CH₂CH₂CO₂Et-1-Triz) | 2-F | H | H |
| 2-584 | Ac | H | CH₂CH₂-(5-CH₂CH₂CH₂CO₂Et-1-Triz) | 2-F | H | H |
| 2-585 | H | H | CH₂-2-Triz | 2-F | H | H |
| 2-586 | Ac | H | CH₂-2-Triz | 2-F | H | H |
| 2-587 | H | H | CH₂-(4-CO₂H-2-Triz) | 2-F | H | H |
| 2-588 | Ac | H | CH₂-(4-CO₂H-2-Triz) | 2-F | H | H |
| 2-589 | H | H | CH₂-(4-CO₂Me-2-Triz) | 2-F | H | H |
| 2-590 | Ac | H | CH₂-(4-CO₂Me-2-Triz) | 2-F | H | H |
| 2-591 | H | H | CH₂-(4-CO₂Et-2-Triz) | 2-F | H | H |
| 2-592 | Ac | H | CH₂-(4-CO₂Et-2-Triz) | 2-F | H | H |
| 2-593 | H | H | CH₂-(4-CH₂CO₂H-2-Triz) | 2-F | H | H |
| 2-594 | Ac | H | CH₂-(4-CH₂CO₂H-2-Triz) | 2-F | H | H |
| 2-595 | H | H | CH₂-(4-CH₂CO₂Me-2-Triz) | 2-F | H | H |
| 2-596 | Ac | H | CH₂-(4-CH₂CO₂Me-2-Triz) | 2-F | H | H |
| 2-597 | H | H | CH₂-(4-CH₂CO₂Et-2-Triz) | 2-F | H | H |
| 2-598 | Ac | H | CH₂-(4-CH₂CO₂Et-2-Triz) | 2-F | H | H |
| 2-599 | H | H | CH₂-(4-CH₂CH₂CO₂H-2-Triz) | 2-F | H | H |
| 2-600 | Ac | H | CH₂-(4-CH₂CH₂CO₂H-2-Triz) | 2-F | H | H |
| 2-601 | H | H | CH₂-(4-CH₂CH₂CO₂Me-2-Triz) | 2-F | H | H |
| 2-602 | Ac | H | CH₂-(4-CH₂CH₂CO₂Me-2-Triz) | 2-F | H | H |
| 2-603 | H | H | CH₂-(4-CH₂CH₂CO₂Et-2-Triz) | 2-F | H | H |
| 2-604 | Ac | H | CH₂-(4-CH₂CH₂CO₂Et-2-Triz) | 2-F | H | H |
| 2-605 | H | H | CH₂-(4-CH₂CH₂CH₂CO₂H-2-Triz) | 2-F | H | H |
| 2-606 | Ac | H | CH₂-(4-CH₂CH₂CH₂CO₂H-2-Triz) | 2-F | H | H |
| 2-607 | H | H | CH₂-(4-CH₂CH₂CH₂CO₂Me-2-Triz) | 2-F | H | H |
| 2-608 | Ac | H | CH₂-(4-CH₂CH₂CH₂CO₂Me-2-Triz) | 2-F | H | H |
| 2-609 | H | H | CH₂-(4-CH₂CH₂CH₂CO₂Et-2-Triz) | 2-F | H | H |
| 2-610 | Ac | H | CH₂-(4-CH₂CH₂CH₂CO₂Et-2-Triz) | 2-F | H | H |
| 2-611 | H | H | CH₂CH₂-2-Triz | 2-F | H | H |
| 2-612 | Ac | H | CH₂CH₂-2-Triz | 2-F | H | H |
| 2-613 | H | H | CH₂CH₂-(4-CO₂H-2-Triz) | 2-F | H | H |
| 2-614 | Ac | H | CH₂CH₂-(4-CO₂H-2-Triz) | 2-F | H | H |
| 2-615 | H | H | CH₂CH₂-(4-CO₂Me-2-Triz) | 2-F | H | H |
| 2-616 | Ac | H | CH₂CH₂-(4-CO₂Me-2-Triz) | 2-F | H | H |
| 2-617 | H | H | CH₂CH₂-(4-CO₂Et-2-Triz) | 2-F | H | H |
| 2-618 | Ac | H | CH₂CH₂-(4-CO₂Et-2-Triz) | 2-F | H | H |
| 2-619 | H | H | CH₂CH₂-(4-CH₂CO₂H-2-Triz) | 2-F | H | H |
| 2-620 | Ac | H | CH₂CH₂-(4-CH₂CO₂H-2-Triz) | 2-F | H | H |
| 2-621 | H | H | CH₂CH₂-(4-CH₂CO₂Me-2-Triz) | 2-F | H | H |
| 2-622 | Ac | H | CH₂CH₂-(4-CH₂CO₂Me-2-Triz) | 2-F | H | H |
| 2-623 | H | H | CH₂CH₂-(4-CH₂CO₂Et-2-Triz) | 2-F | H | H |
| 2-624 | Ac | H | CH₂CH₂-(4-CH₂CO₂Et-2-Triz) | 2-F | H | H |
| 2-625 | H | H | CH₂CH₂-(4-CH₂CH₂CO₂H-2-Triz) | 2-F | H | H |
| 2-626 | Ac | H | CH₂CH₂-(4-CH₂CH₂CO₂H-2-Triz) | 2-F | H | H |
| 2-627 | H | H | CH₂CH₂-(4-CH₂CH₂CO₂Me-2-Triz) | 2-F | H | H |
| 2-628 | Ac | H | CH₂CH₂-(4-CH₂CH₂CO₂Me-2-Triz) | 2-F | H | H |
| 2-629 | H | H | CH₂CH₂-(4-CH₂CH₂CO₂Et-2-Triz) | 2-F | H | H |
| 2-630 | Ac | H | CH₂CH₂-(4-CH₂CH₂CO₂Et-2-Triz) | 2-F | H | H |
| 2-631 | H | H | CH₂CH₂-(4-CH₂CH₂CH₂CO₂H-2-Triz) | 2-F | H | H |
| 2-632 | Ac | H | CH₂CH₂-(4-CH₂CH₂CH₂CO₂H-2-Triz) | 2-F | H | H |
| 2-633 | H | H | CH₂CH₂-(4-CH₂CH₂CH₂CO₂Me-2-Triz) | 2-F | H | H |
| 2-634 | Ac | H | CH₂CH₂-(4-CH₂CH₂CH₂CO₂Me-2-Triz) | 2-F | H | H |
| 2-635 | H | H | CH₂CH₂-(4-CH₂CH₂CH₂CO₂Et-2-Triz) | 2-F | H | H |
| 2-636 | Ac | H | CH₂CH₂-(4-CH₂CH₂CH₂CO₂Et-2-Triz) | 2-F | H | H |
| 2-637 | H | H | CH₂-4-Triz | 2-F | H | H |
| 2-638 | Ac | H | CH₂-4-Triz | 2-F | H | H |
| 2-639 | H | H | CH₂-4-Triz | 2-F | 4-F | H |
| 2-640 | Ac | H | CH₂-4-Triz | 2-F | 4-F | H |
| 2-641 | H | H | CH₂-(1-CO₂Me-4-Triz) | 2-F | H | H |
| 2-642 | Ac | H | CH₂-(1-CO₂Me-4-Triz) | 2-F | H | H |
| 2-643 | H | H | CH₂-(1-CO₂Et-4-Triz) | 2-F | H | H |
| 2-644 | Ac | H | CH₂-(1-CO₂Et-4-Triz) | 2-F | H | H |
| 2-645 | H | H | CH₂-(5-CO₂H-4-Triz) | 2-F | H | H |
| 2-646 | Ac | H | CH₂-(5-CO₂H-4-Triz) | 2-F | H | H |
| 2-647 | H | H | CH₂-(5-CO₂Me-4-Triz) | 2-F | H | H |
| 2-648 | Ac | H | CH₂-(5-CO₂Me-4-Triz) | 2-F | H | H |
| 2-649 | H | H | CH₂-(5-CO₂Et-4-Triz) | 2-F | H | H |
| 2-650 | Ac | H | CH₂-(5-CO₂Et-4-Triz) | 2-F | H | H |
| 2-651 | H | H | CH₂-(1-CH₂CO₂H-4-Triz) | 2-F | H | H |
| 2-652 | Ac | H | CH₂-(1-CH₂CO₂H-4-Triz) | 2-F | H | H |
| 2-653 | H | H | CH₂-(1-CH₂CO₂Me-4-Triz) | 2-F | H | H |
| 2-654 | Ac | H | CH₂-(1-CH₂CO₂Me-4-Triz) | 2-F | H | H |
| 2-655 | H | H | CH₂-(1-CH₂CO₂Et-4-Triz) | 2-F | H | H |
| 2-656 | Ac | H | CH₂-(1-CH₂CO₂Et-4-Triz) | 2-F | H | H |
| 2-657 | H | H | CH₂-(5-CH₂CO₂H-4-Triz) | 2-F | H | H |
| 2-658 | Ac | H | CH₂-(5-CH₂CO₂H-4-Triz) | 2-F | H | H |
| 2-659 | H | H | CH₂-(5-CH₂CO₂Me-4-Triz) | 2-F | H | H |
| 2-660 | Ac | H | CH₂-(5-CH₂CO₂Me-4-Triz) | 2-F | H | H |
| 2-661 | H | H | CH₂-(5-CH₂CO₂Et-4-Triz) | 2-F | H | H |
| 2-662 | Ac | H | CH₂-(5-CH₂CO₂Et-4-Triz) | 2-F | H | H |
| 2-663 | H | H | CH₂-(1-CH₂CH₂CO₂H-4-Triz) | 2-F | H | H |
| 2-664 | Ac | H | CH₂-(1-CH₂CH₂CO₂H-4-Triz) | 2-F | H | H |
| 2-665 | H | H | CH₂-(1-CH₂CH₂CO₂Me-4-Triz) | 2-F | H | H |
| 2-666 | Ac | H | CH₂-(1-CH₂CH₂CO₂Me-4-Triz) | 2-F | H | H |
| 2-667 | H | H | CH₂-(1-CH₂CH₂CO₂Et-4-Triz) | 2-F | H | H |
| 2-668 | Ac | H | CH₂-(1-CH₂CH₂CO₂Et-4-Triz) | 2-F | H | H |
| 2-669 | H | H | CH₂-(5-CH₂CH₂CO₂H-4-Triz) | 2-F | H | H |
| 2-670 | Ac | H | CH₂-(5-CH₂CH₂CO₂H-4-Triz) | 2-F | H | H |
| 2-671 | H | H | CH₂-(5-CH₂CH₂CO₂Me-4-Triz) | 2-F | H | H |
| 2-672 | Ac | H | CH₂-(5-CH₂CH₂CO₂Me-4-Triz) | 2-F | H | H |
| 2-673 | H | H | CH₂-(5-CH₂CH₂CO₂Et4-Triz) | 2-F | H | H |
| 2-674 | Ac | H | CH₂-(5-CH₂CH₂CO₂Et-4-Triz) | 2-F | H | H |
| 2-675 | H | H | CH₂-(1-CH₂CH₂CH₂CO₂H-4-Triz) | 2-F | H | H |
| 2-676 | Ac | H | CH₂-(1-CH₂CH₂CH₂CO₂H-4-Triz) | 2-F | H | H |
| 2-677 | H | H | CH₂-(1-CH₂CH₂CH₂CO₂Me-4-Triz) | 2-F | H | H |
| 2-678 | Ac | H | CH₂-(1-CH₂CH₂CH₂CO₂Me-4-Triz) | 2-F | H | H |
| 2-679 | H | H | CH₂-(1-CH₂CH₂CH₂CO₂Et-4-Triz) | 2-F | H | H |
| 2-680 | Ac | H | CH₂-(1-CH₂CH₂CH₂CO₂Et-4-Triz) | 2-F | H | H |
| 2-681 | H | H | CH₂-(5-CH₂CH₂CH₂CO₂H-4-Triz) | 2-F | H | H |
| 2-682 | Ac | H | CH₂-(5-CH₂CH₂CH₂CO₂H-4-Triz) | 2-F | H | H |
| 2-683 | H | H | CH₂-(5-CH₂CH₂CH₂CO₂Me-4-Triz) | 2-F | H | H |
| 2-684 | Ac | H | CH₂-(5-CH₂CH₂CH₂CO₂Me-4-Triz) | 2-F | H | H |
| 2-685 | H | H | CH₂-(5-CH₂CH₂CH₂CO₂Et-4-Triz) | 2-F | H | H |
| 2-686 | Ac | H | CH₂-(5-CH₂CH₂CH₂CO₂Et-4-Triz) | 2-F | H | H |
| 2-687 | H | H | CH₂CH₂-4-Triz | 2-F | H | H |
| 2-688 | Ac | H | CH₂CH₂-4-Triz | 2-F | H | H |
| 2-689 | H | H | CH₂CH₂-4-Triz | 2-F | 4-F | H |
| 2-690 | Ac | H | CH₂CH₂-4-Triz | 2-F | 4-F | H |
| 2-691 | H | H | CH₂CH₂-(1-CO₂Me-4-Triz) | 2-F | H | H |
| 2-692 | Ac | H | CH₂CH₂-(1-CO₂Me-4-Triz) | 2-F | H | H |
| 2-693 | H | H | CH₂CH₂-(1-CO₂Et-4-Triz) | 2-F | H | H |
| 2-694 | Ac | H | CH₂CH₂-(1-CO₂Et-4-Triz) | 2-F | H | H |
| 2-695 | H | H | CH₂CH₂-(5-CO₂H-4-Triz) | 2-F | H | H |
| 2-696 | Ac | H | CH₂CH₂-(5-CO₂H-4-Triz) | 2-F | H | H |
| 2-697 | H | H | CH₂CH₂-(5-CO₂Me-4-Triz) | 2-F | H | H |
| 2-698 | Ac | H | CH₂CH₂-(5-CO₂Me-4-Triz) | 2-F | H | H |
| 2-699 | H | H | CH₂CH₂-(5-CO₂Et-4-Triz) | 2-F | H | H |
| 2-700 | Ac | H | CH₂CH₂-(5-CO₂Et-4-Triz) | 2-F | H | H |
| 2-701 | H | H | CH₂CH₂-(1-CH₂CO₂H-4-Triz) | 2-F | H | H |
| 2-702 | Ac | H | CH₂CH₂-(1-CH₂CO₂H-4-Triz) | 2-F | H | H |
| 2-703 | H | H | CH₂CH₂-(1-CH₂CO₂Me-4-Triz) | 2-F | H | H |
| 2-704 | Ac | H | CH₂CH₂-(1-CH₂CO₂Me-4-Triz) | 2-F | H | H |
| 2-705 | H | H | CH₂CH₂-(1-CH₂CO₂Et-4-Triz) | 2-F | H | H |
| 2-706 | Ac | H | CH₂CH₂-(1-CH₂CO₂Et-4-Triz) | 2-F | H | H |
| 2-707 | H | H | CH₂CH₂-(5-CH₂CO₂H-4-Triz) | 2-F | H | H |
| 2-708 | Ac | H | CH₂CH₂-(5-CH₂CO₂H-4-Triz) | 2-F | H | H |
| 2-709 | H | H | CH₂CH₂-(5-CH₂CO₂Me-4-Triz) | 2-F | H | H |
| 2-710 | Ac | H | CH₂CH₂-(5-CH₂CO₂Me-4-Triz) | 2-F | H | H |
| 2-711 | H | H | CH₂CH₂-(5-CH₂CO₂Et4-Triz) | 2-F | H | H |
| 2-712 | Ac | H | CH₂CH₂-(5-CH₂CO₂Et-4-Triz) | 2-F | H | H |
| 2-713 | H | H | CH₂CH₂-(1-CH₂CH₂CO₂H-4-Triz) | 2-F | H | H |
| 2-714 | Ac | H | CH₂CH₂-(1-CH₂CH₂CO₂H-4-Triz) | 2-F | H | H |
| 2-715 | H | H | CH₂CH₂-(1-CH₂CH₂CO₂Me-4-Triz) | 2-F | H | H |
| 2-716 | Ac | H | CH₂CH₂-(1-CH₂CH₂CO₂Me-4-Triz) | 2-F | H | H |
| 2-717 | H | H | CH₂CH₂-(1-CH₂CH₂CO₂Et-4-Triz) | 2-F | H | H |
| 2-718 | Ac | H | CH₂CH₂-(1-CH₂CH₂CO₂Et-4-Triz) | 2-F | H | H |
| 2-719 | H | H | CH₂CH₂-(5-CH₂CH₂CO₂H-4-Triz) | 2-F | H | H |
| 2-720 | Ac | H | CH₂CH₂-(5-CH₂CH₂CO₂H-4-Triz) | 2-F | H | H |
| 2-721 | H | H | CH₂CH₂-(5-CH₂CH₂CO₂Me-4-Triz) | 2-F | H | H |
| 2-722 | Ac | H | CH₂CH₂-(5-CH₂CH₂CO₂Me-4-Triz) | 2-F | H | H |
| 2-723 | H | H | CH₂CH₂-(5-CH₂CH₂CO₂Et-4-Triz) | 2-F | H | H |
| 2-724 | Ac | H | CH₂CH₂-(5-CH₂CH₂CO₂Et-4-Triz) | 2-F | H | H |
| 2-725 | H | H | CH₂CH₂-(1-CH₂CH₂CH₂CO₂H-4-Triz) | 2-F | H | H |
| 2-726 | Ac | H | CH₂CH₂-(1-CH₂CH₂CH₂CO₂H-4-Triz) | 2-F | H | H |
| 2-727 | H | H | CH₂CH₂-(1-CH₂CH₂CH₂CO₂Me-4-Triz) | 2-F | H | H |
| 2-728 | Ac | H | CH₂CH₂-(1-CH₂CH₂CH₂CO₂Me-4-Triz) | 2-F | H | H |
| 2-729 | H | H | CH₂CHᵣ(1-CH₂CH₂CH₂CO₂Et-4-Triz) | 2-F | H | H |
| 2-730 | Ac | H | CH₂CH₂-(1-CH₂CH₂CH₂CO₂Et-4-Triz) | 2-F | H | H |
| 2-731 | H | H | CH₂CH₂-(5-CH₂CH₂CH₂CO₂H-4-Triz) | 2-F | H | H |
| 2-732 | Ac | H | CH₂CH₂-(5-CH₂CH₂CH₂CO₂H-4-Triz) | 2-F | H | H |
| 2-733 | H | H | CH₂CH₂-(5-CH₂CH₂CH₂CO₂Me-4-Triz) | 2-F | H | H |
| 2-734 | Ac | H | CH₂CH₂-(5-CH₂CH₂CH₂CO₂Me-4-Triz) | 2-F | H | H |
| 2-735 | H | H | CH₂CH₂-(5-CH₂CH₂CH₂CO₂Et-4-Triz) | 2-F | H | H |
| 2-736 | Ac | H | CH₂CH₂-(5-CH₂CH₂CH₂CO₂Et-4-Triz) | 2-F | H | H |
| 2-737 | H | H | CH₂-(1-CO₂Me-5-Triz) | 2-F | 4-F | H |
| 2-738 | Ac | H | CH₂-(1-CO₂Me-5-Triz) | 2-F | 4-F | H |
| 2-739 | H | H | CH₂-(1-CO₂Me-5-Triz) | 2-F | H | H |
| 2-740 | Ac | H | CH₂-(1-CO₂Me-5-Triz) | 2-F | H | H |
| 2-741 | H | H | CH₂-(1-CO₂Et-5-Triz) | 2-F | H | H |
| 2-742 | Ac | H | CH₂-(1-CO₂Et-5-Triz) | 2-F | H | H |
| 2-743 | H | H | CH₂-(1-CH₂CO₂H-5-Triz) | 2-F | H | H |
| 2-744 | Ac | H | CH₂-(1-CH₂CO₂H-5-Triz) | 2-F | H | H |
| 2-745 | H | H | CH₂-(1-CH₂CO₂Me-5-Triz) | 2-F | H | H |
| 2-746 | Ac | H | CH₂-(1-CH₂CO₂Me-5-Triz) | 2-F | H | H |
| 2-747 | H | H | CH₂-(1-CH₂CO₂Et-5-Triz) | 2-F | H | H |
| 2-748 | Ac | H | CH₂-(1-CH₂CO₂Et-5-Triz) | 2-F | H | H |
| 2-749 | H | H | CH₂-(1-CH₂CH₂CO₂H-5-Triz) | 2-F | H | H |
| 2-750 | Ac | H | CH₂-(1-CH₂CH₂CO₂H-5-Triz) | 2-F | H | H |
| 2-751 | H | H | CH₂-(1-CH₂CH₂CO₂Me-5-Triz) | 2-F | H | H |
| 2-752 | Ac | H | CH₂-(1-CH₂CH₂CO₂Me-5-Triz) | 2-F | H | H |
| 2-753 | H | H | CH₂-(1-CH₂CH₂CO₂Et-5-Triz) | 2-F | H | H |
| 2-754 | Ac | H | CH₂-(1-CH₂CH₂CO₂Et-5-Triz) | 2-F | H | H |
| 2-755 | H | H | CH₂-(1-CH₂CH₂CH₂CO₂H-5-Triz) | 2-F | H | H |
| 2-756 | Ac | H | CH₂-(1-CH₂CH₂CH₂CO₂H-5-Triz) | 2-F | H | H |
| 2-757 | H | H | CH₂-(1-CH₂CH₂CH₂CO₂Me-5-Triz) | 2-F | H | H |
| 2-758 | Ac | H | CH₂-(1-CH₂CH₂CH₂CO₂Me-5-Triz) | 2-F | H | H |
| 2-759 | H | H | CH₂-(1-CH₂CH₂CH₂CO₂Et-5-Triz) | 2-F | H | H |
| 2-760 | Ac | H | CH₂-(1-CH₂CH₂CH₂CO₂Et-5-Triz) | 2-F | H | H |
| 2-761 | H | H | CH₂-1-Tez | 2-F | H | H |
| 2-762 | Ac | H | CH₂-1-Tez | 2-F | H | H |
| 2-763 | H | H | CH₂-(5-CO₂H-1-Tez) | 2-F | H | H |
| 2-764 | Ac | H | CH₂-(5-CO₂H-1-Tez) | 2-F | H | H |
| 2-765 | H | H | CH₂-(5-CO₂Me-1-Tez) | 2-F | H | H |
| 2-766 | Ac | H | CH₂-(5-CO₂Me-1-Tez) | 2-F | H | H |
| 2-767 | H | H | CH₂-(5-CO₂Et-1-Tez) | 2-F | H | H |
| 2-768 | Ac | H | CH₂-(5-CO₂Et-1-Tez) | 2-F | H | H |
| 2-769 | H | H | CH₂-(5-CH₂CO₂H-1-Tez) | 2-F | H | H |
| 2-770 | Ac | H | CH₂-(5-CH₂CO₂H-1-Tez) | 2-F | H | H |
| 2-771 | H | H | CH₂-(5-CH₂CO₂Me-1-Tez) | 2-F | H | H |
| 2-772 | Ac | H | CH₂-(5-CH₂CO₂Me-1-Tez) | 2-F | H | H |
| 2-773 | H | H | CH₂-(5-CH₂CO₂Et-1-Tez) | 2-F | H | H |
| 2-774 | Ac | H | CH₂-(5-CH₂CO₂Et-1-Tez) | 2-F | H | H |
| 2-775 | H | H | CH₂-(5-CH₂CH₂CO₂H-1-Tez) | 2-F | H | H |
| 2-776 | Ac | H | CH₂-(5-CH₂CH₂CO₂H-1-Tez) | 2-F | H | H |
| 2-777 | H | H | CH₂-(5-CH₂CH₂CO₂Me-1-Tez) | 2-F | H | H |
| 2-778 | Ac | H | CH₂-(5-CH₂CH₂CO₂Me-1-Tez) | 2-F | H | H |
| 2-779 | H | H | CH₂-(5-CH₂CH₂CO₂Et-1-Tez) | 2-F | H | H |
| 2-780 | Ac | H | CH₂-(5-CH₂CH₂CO₂Et-1-Tez) | 2-F | H | H |
| 2-781 | H | H | CH₂-(5-CH₂CH₂CH₂CO₂H-1-Tez) | 2-F | H | H |
| 2-782 | Ac | H | CH₂-(5-CH₂CH₂CH₂CO₂H-1-Tez) | 2-F | H | H |
| 2-783 | H | H | CH₂-(5-CH₂CH₂CH₂CO₂Me-1-Tez) | 2-F | H | H |
| 2-784 | Ac | H | CH₂-(5-CH₂CH₂CH₂CO₂Me-1-Tez) | 2-F | H | H |
| 2-785 | H | H | CH₂-(5-CH₂CH₂CH₂CO₂Et-1-Tez) | 2-F | H | H |
| 2-786 | Ac | H | CH₂-(5-CH₂CH₂CH₂CO₂Et-1-Tez) | 2-F | H | H |
| 2-787 | H | H | CH₂CH₂-1-Tez | 2-F | H | H |
| 2-788 | Ac | H | CH₂CH₂-1-Tez | 2-F | H | H |
| 2-789 | H | H | CH₂CH₂-(5-CO₂H-1-Tez) | 2-F | H | H |
| 2-790 | Ac | H | CH₂CH₂-(5-CO₂H-1-Tez) | 2-F | H | H |
| 2-791 | H | H | CH₂CH₂-(5-CO₂Me-1-Tez) | 2-F | H | H |
| 2-792 | Ac | H | CH₂CH₂-(5-CO₂Me-1-Tez) | 2-F | H | H |
| 2-793 | H | H | CH₂CH₂(5-CO₂Et-1- -Tez) | 2-F | H | H |
| 2-794 | Ac | H | CH₂CH₂-(5-CO₂Et-1-Tez) | 2-F | H | H |
| 2-795 | H | H | CH₂CH₂-(5-CH₂CO₂H-1-Tez) | 2-F | H | H |
| 2-796 | Ac | H | CH₂CH₂-(5-CH₂CO₂H-1-Tez) | 2-F | H | H |
| 2-797 | H | H | CH₂CH₂-(5-CH₂CO₂Me-1- -Tez) | 2-F | H | H |
| 2-798 | Ac | H | CH₂CH₂-(5-CH₂CO₂Me-1-Tez) | 2-F | H | H |
| 2-799 | H | H | CH₂CH₂-(5-CH₂CO₂Et-1 -Tez) | 2-F | H | H |
| 2-800 | Ac | H | CH₂CH₂-(5-CH₂CO₂Et-1-Tez) | 2-F | H | H |
| 2-801 | H | H | CH₂CH₂(5-CH₂CH₂CO₂H-1-Tez) | 2-F | H | H |
| 2-802 | Ac | H | CH₂CH₂-(5-CH₂CH₂CO₂H-1-Tez) | 2-F | H | H |
| 2-803 | H | H | CH₂CH₂-(5-CH₂CH₂CO₂Me-1-Tez) | 2-F | H | H |
| 2-804 | Ac | H | CH₂CH₂-(5-CH₂CH₂CO₂Me-1 -Tez) | 2-F | H | H |
| 2-805 | H | H | CH₂CH₂-(5-CH₂CH₂CO₂Et-1-Tez) | 2-F | H | H |
| 2-806 | Ac | H | CH₂CH₂-(5-CH₂CH₂CO₂Et-1-Tez) | 2-F | H | H |
| 2-807 | H | H | CH₂CH₂-(5-CH₂CH₂CH₂CO₂H-1-Tez) | 2-F | H | H |
| 2-808 | Ac | H | CH₂CH₂-(5-CH₂CH₂CH₂CO₂H-1-Tez) | 2-F | H | H |
| 2-809 | H | H | CH₂CH₂-(5-CH₂CH₂CH₂CO₂Me-1-Tez) | 2-F | H | H |
| 2-810 | Ac | H | CH₂CH₂-(5-CH₂CH₂CH₂CO₂Me-1-Tez) | 2-F | H | H |
| 2-811 | H | H | CH₂CH₂-(5-CH₂CH₂CH₂CO₂Et-1-Tez) | 2-F | H | H |
| 2-812 | Ac | H | CH₂CH₂-(5-CH₂CH₂CH₂CO₂Et-1-Tez) | 2-F | H | H |
| 2-813 | H | H | CH₂-2-Tez | 2-F | H | H |
| 2-814 | Ac | H | CH₂-2-Tez | 2-F | H | H |
| 2-815 | H | H | CH₂-(5-CO₂H-2-Tez) | 2-F | H | H |
| 2-816 | Ac | H | CH₂-(5-CO₂H-2-Tez) | 2-F | H | H |
| 2-817 | H | H | CH₂-(5-CO₂Me-2-Tez) | 2-F | H | H |
| 2-818 | Ac | H | CH₂-(5-CO₂Me-2-Tez) | 2-F | H | H |
| 2-819 | H | H | CH₂-(5-CO₂Et-2-Tez) | 2-F | H | H |
| 2-820 | Ac | H | CH₂-(5-CO₂Et-2-Tez) | 2-F | H | H |
| 2-821 | H | H | CH₂-(5-CH₂CO₂H-2-Tez) | 2-F | H | H |
| 2-822 | Ac | H | CH₂-(5-CH₂CO₂H-2-Tez) | 2-F | H | H |
| 2-823 | H | H | CH₂-(5-CH₂CO₂Me-2-Tez) | 2-F | H | H |
| 2-824 | Ac | H | CH₂-(5-CH₂CO₂Me-2-Tez) | 2-F | H | H |
| 2-825 | H | H | CH₂-(5-CH₂CO₂Et-2-Tez) | 2-F | H | H |
| 2-826 | Ac | H | CH₂-(5-CH₂CO₂Et-2-Tez) | 2-F | H | H |
| 2-827 | H | H | CH₂-(5-CH₂CH₂CO₂H-2-Tez) | 2-F | H | H |
| 2-828 | Ac | H | CH₂-(5-CH₂CH₂CO₂H-2-Tez) | 2-F | H | H |
| 2-829 | H | H | CH₂-(5-CH₂CH₂CO₂Me-2-Tez) | 2-F | H | H |
| 2-830 | Ac | H | CH₂-(5-CH₂CH₂CO₂Me-2-Tez) | 2-F | H | H |
| 2-831 | H | H | CH₂-(5-CH₂CH₂CO₂Et-2-Tez) | 2-F | H | H |
| 2-832 | Ac | H | CH₂-(5-CH₂CH₂CO₂Et-2-Tez) | 2-F | H | H |
| 2-833 | H | H | CH₂-(5-CH₂CH₂CH₂CO₂H-2-Tez) | 2-F | H | H |
| 2-834 | Ac | H | CH₂-(5-CH₂CH₂CH₂CO₂H-2-Tez) | 2-F | H | H |
| 2-835 | H | H | CH₂-(5-CH₂CH₂CH₂CO₂Me-2-Tez) | 2-F | H | H |
| 2-836 | Ac | H | CH₂-(5-CH₂CH₂CH₂CO₂Me-2-Tez) | 2-F | H | H |
| 2-837 | H | H | CH₂-(5-CH₂CH₂CH₂CO₂Et-2-Tez) | 2-F | H | H |
| 2-838 | Ac | H | CH₂-(5-CH₂CH₂CH₂CO₂Et-2-Tez) | 2-F | H | H |
| 2-839 | H | H | CH₂CH₂-2-Tez | 2-F | H | H |
| 2-840 | Ac | H | CH₂CH₂-2-Tez | 2-F | H | H |
| 2-841 | H | H | CH₂CH₂-(5-CO₂H-2-Tez) | 2-F | H | H |
| 2-842 | Ac | H | CH₂CH₂-(5-CO₂H-2-Tez) | 2-F | H | H |
| 2-843 | H | H | CH₂CH₂-(5-CO₂Me-2-Tez) | 2-F | H | H |
| 2-844 | Ac | H | CH₂CH₂-(5-CO₂Me-2-Tez) | 2-F | H | H |
| 2-845 | H | H | CH₂CH₂-(5-CO₂Et-2-Tez) | 2-F | H | H |
| 2-846 | Ac | H | CH₂CH₂-(5-CO₂Et-2-Tez) | 2-F | H | H |
| 2-847 | H | H | CH₂CH₂-(5-CH₂CO₂H-2-Tez) | 2-F | H | H |
| 2-848 | Ac | H | CH₂CH₂-(5-CH₂CO₂H-2-Tez) | 2-F | H | H |
| 2-849 | H | H | CH₂CH₂-(5-CH₂CO₂Me-2-Tez) | 2-F | H | H |
| 2-850 | Ac | H | CH₂CH₂-(5-CH₂CO₂Me-2-Tez) | 2-F | H | H |
| 2-851 | H | H | CH₂CH₂-(5-CH₂CO₂Et-2-Tez) | 2-F | H | H |
| 2-852 | Ac | H | CH₂CH₂-(5-CH₂CO₂Et-2-Tez) | 2-F | H | H |
| 2-853 | H | H | CH₂CH₂-(5-CH₂CH₂CO₂H-2-Tez) | 2-F | H | H |
| 2-854 | Ac | H | CH₂CH₂-(5-CH₂CH₂CO₂H-2-Tez) | 2-F | H | H |
| 2-855 | H | H | CH₂CH₂-(5-CH₂CH₂CO₂Me-2-Tez) | 2-F | H | H |
| 2-856 | Ac | H | CH₂CH₂-(5-CH₂CH₂CO₂Me-2-Tez) | 2-F | H | H |
| 2-857 | H | H | CH₂CH₂-(5-CH₂CH₂CO₂Et-2-Tez) | 2-F | H | H |
| 2-858 | Ac | H | CH₂CH₂-(5-CH₂CH₂CO₂Et-2-Tez) | 2-F | H | H |
| 2-859 | H | H | CH₂CH₂-(5-CH₂CH₂CH₂CO₂H-2-Tez) | 2-F | H | H |
| 2-860 | Ac | H | CH₂CH₂-(5-CH₂CH₂CH₂CO₂H-2-Tez) | 2-F | H | H |
| 2-861 | H | H | CH₂CH₂-(5-CH₂CH₂CH₂CO₂Me-2-Tez) | 2-F | H | H |
| 2-862 | Ac | H | CH₂CH₂-(5-CH₂CH₂CH₂CO₂Me-2-Tez) | 2-F | H | H |
| 2-863 | H | H | CH₂CH₂-(5-CH₂CH₂CH₂CO₂Et-2-Tez) | 2-F | H | H |
| 2-864 | Ac | H | CH₂CH₂-(5-CH₂CH₂CH₂CO₂Et-2-Tez) | 2-F | H | H |
| 2-865 | H | H | CH₂-5-Tez | 2-F | H | H |
| 2-866 | Ac | H | CH₂-5-Tez | 2-F | H | H |
| 2-867 | H | H | CH₂-5-Tez | 2-F | 4-F | H |
| 2-868 | Ac | H | CH₂-5-Tez | 2-F | 4-F | H |
| 2-869 | H | H | CH₂-(1-CO₂Me-5-Tez) | 2-F | H | H |
| 2-870 | Ac | H | CH₂-(1-CO₂Me-5-Tez) | 2-F | H | H |
| 2-871 | H | H | CH₂-(1-CO₂Et-5-Tez) | 2-F | H | H |
| 2-872 | Ac | H | CH₂-(1-CO₂Et-5-Tez) | 2-F | H | H |
| 2-873 | H | H | CH₂-(2-CO₂Me-5-Tez) | 2-F | 4-F | H |
| 2-874 | Ac | H | CH₂-(2-CO₂Me-5-Tez) | 2-F | 4-F | H |
| 2-875 | H | H | CH₂-(2-CO₂Me-5-Tez) | 2-F | H | H |
| 2-876 | Ac | H | CH₂-(2-CO₂Me-5-Tez) | 2-F | H | H |
| 2-877 | H | H | CH₂-(2-CO₂Et-5-Tez) | 2-F | H | H |
| 2-878 | Ac | H | CH₂-(2-CO₂Et-5-Tez) | 2-F | H | H |
| 2-879 | H | H | CH₂-(1-CH₂CO₂H-5-Tez) | 2-F | H | H |
| 2-880 | Ac | H | CH₂-(1-CH₂CO₂H-5-Tez) | 2-F | H | H |
| 2-881 | H | H | CH₂-(1-CH₂CO₂Me-5-Tez) | 2-F | H | H |
| 2-882 | Ac | H | CH₂-(1-CH₂CO₂Me-5-Tez) | 2-F | H | H |
| 2-883 | H | H | CH₂-(1-CH₂CO₂Et-5-Tez) | 2-F | H | H |
| 2-884 | Ac | H | CH₂-(1-CH₂CO₂Et-5-Tez) | 2-F | H | H |
| 2-885 | H | H | CH₂-(2-CH₂CO₂H-5-Tez) | 2-F | H | H |
| 2-886 | Ac | H | CH₂-(2-CH₂CO₂H-5-Tez) | 2-F | H | H |
| 2-887 | H | H | CH₂-(2-CH₂CO₂Me-5-Tez) | 2-F | H | H |
| 2-888 | Ac | H | CH₂-(2-CH₂CO₂Me-5-Tez) | 2-F | H | H |
| 2-889 | H | H | CH₂-(2-CH₂CO₂Et-5-Tez) | 2-F | H | H |
| 2-890 | Ac | H | CH₂-(2-CH₂CO₂Et-5-Tez) | 2-F | H | H |
| 2-891 | H | H | CH₂-(1-CH₂CH₂CO₂H-5-Tez) | 2-F | H | H |
| 2-892 | Ac | H | CH₂-(1-CH₂CH₂CO₂H-5-Tez) | 2-F | H | H |
| 2-893 | H | H | CH₂-(1-CH₂CH₂CO₂Me-5-Tez) | 2-F | H | H |
| 2-894 | Ac | H | CH₂-(1-CH₂CH₂CO₂Me-5-Tez) | 2-F | H | H |
| 2-895 | H | H | CH₂-(1-CH₂CH₂CO₂Et-5-Tez) | 2-F | H | H |
| 2-896 | Ac | H | CH₂-(1-CH₂CH₂CO₂Et-5-Tez) | 2-F | H | H |
| 2-897 | H | H | CH₂-(2-CH₂CH₂CO₂H-5-Tez) | 2-F | H | H |
| 2-898 | Ac | H | CH₂-(2-CH₂CH₂CO₂H-5-Tez) | 2-F | H | H |
| 2-899 | H | H | CH₂-(2-CH₂CH₂CO₂Me-5-Tez) | 2-F | H | H |
| 2-900 | Ac | H | CH₂-(2-CH₂CH₂CO₂Me-5-Tez) | 2-F | H | H |
| 2-901 | H | H | CH₂-(2-CH₂CH₂CO₂Et-5-Tez) | 2-F | H | H |
| 2-902 | Ac | H | CH₂-(2-CH₂CH₂CO₂Et-5-Tez) | 2-F | H | H |
| 2-903 | H | H | CH₂-(1-CH₂CH₂CH₂CO₂H-5-Tez) | 2-F | H | H |
| 2-904 | Ac | H | CH₂-(1-CH₂CH₂CH₂CO₂H-5-Tez) | 2-F | H | H |
| 2-905 | H | H | CH₂-(1-CH₂CH₂CH₂CO₂Me-5-Tez) | 2-F | H | H |
| 2-906 | Ac | H | CH₂-(1-CH₂CH₂CH₂CO₂Me-5-Tez) | 2-F | H | H |
| 2-907 | H | H | CH₂-(1-CH₂CH₂CH₂CO₂Et-5-Tez) | 2-F | H | H |
| 2-908 | Ac | H | CH₂-(1-CH₂CH₂CH₂CO₂Et-5-Tez) | 2-F | H | H |
| 2-909 | H | H | CH₂-(2-CH₂CH₂CH₂CO₂H-5-Tez) | 2-F | H | H |
| 2-910 | Ac | H | CH₂-(2-CH₂CH₂CH₂CO₂H-5-Tez) | 2-F | H | H |
| 2-911 | H | H | CH₂-(2-CH₂CH₂CH₂CO₂Me-5-Tez) | 2-F | H | H |
| 2-912 | Ac | H | CH₂-(2-CH₂CH₂CH₂CO₂Me-5-Tez) | 2-F | H | H |
| 2-913 | H | H | CH₂-(2-CH₂CH₂CH₂CO₂Et-5-Tez) | 2-F | H | H |
| 2-914 | Ac | H | CH₂-(2-CH₂CH₂CH₂CO₂Et-5-Tez) | 2-F | H | H |
| 2-915 | H | H | CH₂CH₂-5-Tez | 2-F | H | H |
| 2-916 | Ac | H | CH₂CH₂-5-Tez | 2-F | H | H |
| 2-917 | H | H | CH₂CH₂-5-Tez | 2-F | 4-F | H |
| 2-918 | Ac | H | CH₂CH₂-5-Tez | 2-F | 4-F | H |
| 2-919 | H | H | CH₂CH₂-(1-CO₂Me-5-Tez) | 2-F | H | H |
| 2-920 | Ac | H | CH₂CH₂-(1-CO₂Me-5-Tez) | 2-F | H | H |
| 2-921 | H | H | CH₂CH₂-(1-CO₂Et-5-Tez) | 2-F | H | H |
| 2-922 | Ac | H | CH₂CH₂-(1-CO₂Et-5-Tez) | 2-F | H | H |
| 2-923 | H | H | CH₂CH₂-(2-CO₂Me-5-Tez) | 2-F | H | H |
| 2-924 | Ac | H | CH₂CH₂-(2-CO₂Me-5-Tez) | 2-F | H | H |
| 2-925 | H | H | CH₂CH₂-(2-CO₂Me-5-Tez) | 2-F | H | H |
| 2-926 | Ac | H | CH₂CH₂-(2-CO₂Me-5-Tez) | 2-F | H | H |
| 2-927 | H | H | CH₂CH₂-(2-CO₂Et-5-Tez) | 2-F | H | H |
| 2-928 | Ac | H | CH₂CH₂(2-CO₂Et-5-Tez) | 2-F | H | H |
| 2-929 | H | H | CH₂CH₂-(1-CH₂CO₂H-5-Tez) | 2-F | H | H |
| 2-930 | Ac | H | CH₂CH₂-(1-CH₂CO₂H-5-Tez) | 2-F | H | H |
| 2-931 | H | H | CH₂CH₂-(1-CH₂CO₂Me-5-Tez) | 2-F | H | H |
| 2-932 | Ac | H | CH₂CH₂-(-CH₂CO₂Me-5-Tez) | 2-F | H | H |
| 2-933 | H | H | CH₂CH₂-(1-CH₂CO₂Et-5-Tez) | 2-F | H | H |
| 2-934 | Ac | H | CH₂CH₂-(1-CH₂CO₂Et-5-Tez) | 2-F | H | H |
| 2-935 | H | H | CH₂CH₂-(2-CH₂CO₂H-5-Tez) | 2-F | H | H |
| 2-936 | Ac | H | CH₂CH₂-(2-CH₂CO₂H-5-Tez) | 2-F | H | H |
| 2-937 | H | H | CH₂CH₂-(2-CH₂CO₂Me-5-Tez) | 2-F | H | H |
| 2-938 | Ac | H | CH₂CH₂-(2-CH₂CO₂Me-5-Tez) | 2-F | H | H |
| 2-939 | H | H | CH₂CH₂-(2-CH₂CO₂Et-5-Tez) | 2-F | H | H |
| 2-940 | Ac | H | CH₂CH₂-(2-CH₂CO₂Et-5-Tez) | 2-F | H | H |
| 2-941 | H | H | CH₂CH₂-(1-CH₂CH₂CO₂H-5-Tez) | 2-F | H | H |
| 2-942 | Ac | H | CH₂CH₂-(1-CH₂CH₂CO₂H-5-Tez) | 2-F | H | H |
| 2-943 | H | H | CH₂CH₂-(1-CH₂CH₂CO₂Me-5-Tez) | 2-F | H | H |
| 2-944 | Ac | H | CH₂CH₂-(-CH₂CH₂CO₂Me-5-Tez) | 2-F | H | H |
| 2-945 | H | H | CH₂CH₂-(1-CH₂CH₂CO₂Et-5-Tez) | 2-F | H | H |
| 2-946 | Ac | H | CH₂CH₂-(1-CH₂CH₂CO₂Et-5-Tez) | 2-F | H | H |
| 2-947 | H | H | CH₂CH₂-(2-CH₂CH₂CO₂H-5-Tez) | 2-F | H | H |
| 2-948 | Ac | H | CH₂CH₂-(2-CH₂CH₂CO₂H-5-Tez) | 2-F | H | H |
| 2-949 | H | H | CH₂CH₂-(2-CH₂CH₂CO₂Me-5-Tez) | 2-F | H | H |
| 2-950 | Ac | H | CH₂CH₂-(2-CH₂CH₂CO₂Me-5-Tez) | 2-F | H | H |
| 2-951 | H | H | CH₂CH₂-(2-CH₂CH₂CO₂Et-5-Tez) | 2-F | H | H |
| 2-952 | Ac | H | CH₂CH₂-(2-CH₂CH₂CO₂Et-5-Tez) | 2-F | H | H |
| 2-953 | H | H | CH₂CH₂-(1-CH₂CH₂CH₂CO₂H-5-Tez) | 2-F | H | H |
| 2-954 | Ac | H | CH₂CHr(1-CH₂CH₂CH₂CO₂H-5-Tez) | 2-F | H | H |
| 2-955 | H | H | CH₂CH₂-(1-CH₂CH₂CH₂CO₂Me-5-Tez) | 2-F | H | H |
| 2-956 | Ac | H | CH₂CH₂-(1-CH₂CH₂CH₂CO₂Me-5-Tez) | 2-F | H | H |
| 2-957 | H | H | CH₂CH₂-(1-CH₂CH₂CH₂CO₂Et-5-Tez) | 2-F | H | H |
| 2-958 | Ac | H | CH₂CH₂-(1-CH₂CH₂CH₂CO₂Et-5-Tez) | 2-F | H | H |
| 2-959 | H | H | CH₂CH₂-(2-CH₂CH₂CH₂CO₂H-5-Tez) | 2-F | H | H |
| 2-960 | Ac | H | CH₂CH₂-(2-CH₂CH₂CH₂CO₂H-5-Tez) | 2-F | H | H |
| 2-961 | H | H | CH₂CH₂-(2-CH₂CH₂CH₂CO₂Me-5-Tez) | 2-F | H | H |
| 2-962 | Ac | H | CH₂CH₂-(2-CH₂CH₂CH₂CO₂Me-5-Tez) | 2-F | H | H |
| 2-963 | H | H | CH₂CH₂-(2-CH₂CH₂CH₂CO₂Et-5-Tez) | 2-F | H | H |
| 2-964 | Ac | H | CH₂CH₂-(2-CH₂CH₂CH₂CO₂Et-5-Tez) | 2-F | H | H |
| 2-965 | H | Me | CH₂-1-Pyza | 2-F | H | H |
| 2-966 | Ac | Me | CH₂-1-Pyza | 2-F | H | H |
| 2-967 | H | Me | CH₂-(3-CO₂H-1-Pyza) | 2-F | H | H |
| 2-968 | Ac | Me | CH₂-(3-CO₂H-1-Pyza) | 2-F | H | H |
| 2-969 | H | Me | CH₂-(3-CO₂Me-1-Pyza) | 2-F | H | H |
| 2-970 | Ac | Me | CH₂-(3-CO₂Me-1-Pyza) | 2-F | H | H |
| 2-971 | H | Me | CH₂-(3-CO₂Et-1-Pyza) | 2-F | H | H |
| 2-972 | Ac | Me | CH₂-(3-CO₂Et-1-Pyza) | 2-F | H | H |
| 2-973 | H | Me | CH₂-(4-CO₂H-1-Pyza) | 2-F | H | H |
| 2-974 | Ac | Me | CH₂-(4-CO₂H-1-Pyza) | 2-F | H | H |
| 2-975 | H | Me | CH₂-(4-CO₂Me-1-Pyza) | 2-F | H | H |
| 2-976 | Ac | Me | CH₂-(4-CO₂Me-1-Pyza) | 2-F | H | H |
| 2-977 | H | Me | CH₂-(4-CO₂Et-1-Pyza) | 2-F | H | H |
| 2-978 | Ac | Me | CH₂-(4-CO₂Et-1-Pyza) | 2-F | H | H |
| 2-979 | H | Me | CH₂-(3-CH₂CO₂H-1-Pyza) | 2-F | H | H |
| 2-980 | Ac | Me | CH₂-(3-CH₂CO₂H-1-Pyza) | 2-F | H | H |
| 2-981 | H | Me | CH₂-(3-CH₂CO₂Me-1-Pyza) | 2-F | H | H |
| 2-982 | Ac | Me | CH₂-(3-CH₂CO₂Me-1-Pyza) | 2-F | H | H |
| 2-983 | H | Me | CH₂-(3-CH₂CO₂Et-1-Pyza) | 2-F | H | H |
| 2-984 | Ac | Me | CH₂-(3-CH₂CO₂Et-1-Pyza) | 2-F | H | H |
| 2-985 | H | Me | CH₂-(4-CH₂CO₂H-1-Pyza) | 2-F | H | H |
| 2-986 | Ac | Me | CH₂-(4-CH₂CO₂H-1-Pyza) | 2-F | H | H |
| 2-987 | H | Me | CH₂-(4-CH₂CO₂Me-1-Pyza) | 2-F | H | H |
| 2-988 | Ac | Me | CH₂-(4-CH₂CO₂Me-1-Pyza) | 2-F | H | H |
| 2-989 | H | Me | CH₂-(4-CH₂CO₂Et-1-Pyza) | 2-F | H | H |
| 2-990 | Ac | Me | CH₂-(4-CH₂CO₂Et-1-Pyza) | 2-F | H | H |
| 2-991 | H | Me | CH₂-(3-CH₂CH₂CO₂H-1-Pyza) | 2-F | H | H |
| 2-992 | Ac | Me | CH₂-(3-CH₂CH₂CO₂H-1-Pyza) | 2-F | H | H |
| 2-993 | H | Me | CH₂-(3-CH₂CH₂CO₂Me-1-Pyza) | 2-F | H | H |
| 2-994 | Ac | Me | CH₂-(3-CH₂CH₂CO₂Me-1-Pyza) | 2-F | H | H |
| 2-995 | H | Me | CH₂-(3-CH₂CH₂CO₂Et-1-Pyza) | 2-F | H | H |
| 2-996 | Ac | Me | CH₂-(3-CH₂CH₂CO₂Et-1-Pyza) | 2-F | H | H |
| 2-997 | H | Me | CH₂-(4-CH₂CH₂CO₂H-1-Pyza) | 2-F | H | H |
| 2-998 | Ac | Me | CH₂-(4-CH₂CH₂CO₂H-1-Pyza) | 2-F | H | H |
| 2-999 | H | Me | CH₂-(4-CH₂CH₂CO₂Me-1-Pyza) | 2-F | H | H |
| 2-1000 | Ac | Me | CH₂-(4-CH₂CH₂CO₂Me-1-Pyza) | 2-F | H | H |
| 2-1001 | H | Me | CH₂-(4-CH₂CH₂CO₂Et-1-Pyza) | 2-F | H | H |
| 2-1002 | Ac | Me | CH₂-(4-CH₂CH₂CO₂Et-1-Pyza) | 2-F | H | H |
| 2-1003 | H | Me | CH₂CH₂-1-Pyza | 2-F | H | H |
| 2-1004 | Ac | Me | CH₂CH₂-1-Pyza | 2-F | H | H |
| 2-1005 | H | Me | CH₂CH₂-(3-CO₂H-1-Pyza) | 2-F | H | H |
| 2-1006 | Ac | Me | CH₂CH₂-(3-CO₂H-1-Pyza) | 2-F | H | H |
| 2-1007 | H | Me | CH₂CH₂-(3-CO₂Me-1-Pyza) | 2-F | H | H |
| 2-1008 | Ac | Me | CH₂CH₂-(3-CO₂Me-1-Pyza) | 2-F | H | H |
| 2-1009 | H | Me | CH₂CH₂-(3-CO₂Et-1-Pyza) | 2-F | H | H |
| 2-1010 | Ac | Me | CH₂CH₂-(3-CO₂Et-1-Pyza) | 2-F | H | H |
| 2-1011 | H | Me | CH₂CH₂-(4-CO₂H-1-Pyza) | 2-F | H | H |
| 2-1012 | Ac | Me | CH₂CH₂-(4-CO₂H-1-Pyza) | 2-F | H | H |
| 2-1013 | H | Me | CH₂CH₂-(4-CO₂Me-1-Pyza) | 2-F | H | H |
| 2-1014 | Ac | Me | CH₂CH₂-(4-CO₂Me-1-Pyza) | 2-F | H | H |
| 2-1015 | H | Me | CH₂CH₂-(4-CO₂Et-1-Pyza) | 2-F | H | H |
| 2-1016 | Ac | Me | CH₂CH₂-(4-CO₂Et-1-Pyza) | 2-F | H | H |
| 2-1017 | H | Me | CH₂CH₂-(3-CH₂CO₂H-1-Pyza) | 2-F | H | H |
| 2-1018 | Ac | Me | CH₂CH₂-(3-CH₂CO₂H-1-Pyza) | 2-F | H | H |
| 2-1019 | H | Me | CH₂CH₂-(3-CH₂CO₂Me-1-Pyza) | 2-F | H | H |
| 2-1020 | Ac | Me | CH₂CH₂-(3-CH₂CO₂Me-1-Pyza) | 2-F | H | H |
| 2-1021 | H | Me | CH₂CH₂-(3-CH₂CO₂Et-1-Pyza) | 2-F | H | H |
| 2-1022 | Ac | Me | CH₂CH₂-(3-CH₂CO₂Et-1-Pyza) | 2-F | H | H |
| 2-1023 | H | Me | CH₂CH₂-(4-CH₂CO₂H-1-Pyza) | 2-F | H | H |
| 2-1024 | Ac | Me | CH₂CH₂-(4-CH₂CO₂H-1-Pyza) | 2-F | H | H |
| 2-1025 | H | Me | CH₂CH₂-(4-CH₂CO₂Me-1-Pyza) | 2-F | H | H |
| 2-1026 | Ac | Me | CH₂CH₂-(4-CH₂CO₂Me-1-Pyza) | 2-F | H | H |
| 2-1027 | H | Me | CH₂CH₂-(4-CH₂CO₂Et-1-Pyza) | 2-F | H | H |
| 2-1028 | Ac | Me | CH₂CH₂-(4-CH₂CO₂Et-1-Pyza) | 2-F | H | H |
| 2-1029 | H | Me | CH₂CH₂-(3-CH₂CH₂CO₂H-1-Pyza) | 2-F | H | H |
| 2-1030 | Ac | Me | CH₂CH₂-(3-CH₂CH₂CO₂H-1-Pyza) | 2-F | H | H |
| 2-1031 | H | Me | CH₂CH₂-(3-CH₂CH₂CO₂Me-1-Pyza) | 2-F | H | H |
| 2-1032 | Ac | Me | CH₂CH₂-(3-CH₂CH₂CO₂Me-1-Pyza) | 2-F | H | H |
| 2-1033 | H | Me | CH₂CH₂-(3-CH₂CH₂CO₂Et-1-Pyza) | 2-F | H | H |
| 2-1034 | Ac | Me | CH₂CH₂-(3-CH₂CH₂CO₂Et-1-Pyza) | 2-F | H | H |
| 2-1035 | H | Me | CH₂CH₂-(4-CH₂CH₂CO₂H-1-Pyza) | 2-F | H | H |
| 2-1036 | Ac | Me | CH₂CH₂-(4-CH₂CH₂CO₂H-1-Pyza) | 2-F | H | H |
| 2-1037 | H | Me | CH₂CH₂-(4-CH₂CH₂CO₂Me-1-Pyza) | 2-F | H | H |
| 2-1038 | Ac | Me | CH₂CH₂-(4-CH₂CH₂CO₂Me-1-Pyza) | 2-F | H | H |
| 2-1039 | H | Me | CH₂CH₂-(4-CH₂CH₂CO₂Et-1-Pyza) | 2-F | H | H |
| 2-1040 | Ac | Me | CH₂CH₂-(4-CH₂CH₂CO₂Et-1-Pyza) | 2-F | H | H |
| 2-1041 | H | H | CH₂-(2-CO₂Me-4-Triz) | 2-F | H | H |
| 2-1042 | Ac | H | CH₂-(2-CO₂Me-4-Triz) | 2-F | H | H |
| 2-1043 | H | H | CH₂-(2-CO₂Et-4-Triz) | 2-F | H | H |
| 2-1044 | Ac | H | CH₂-(2-CO₂Et-4-Triz) | 2-F | H | H |
| 2-1045 | H | H | CH₂-(2-CH₂CO₂H-4-Triz) | 2-F | H | H |
| 2-1046 | Ac | H | CH₂-(2-CH₂CO₂H-4-Triz) | 2-F | H | H |
| 2-1047 | H | H | CH₂-(2-CH₂CO₂Me-4-Triz) | 2-F | H | H |
| 2-1048 | Ac | H | CH₂-(2-CH₂CO₂Me-4-Triz) | 2-F | H | H |
| 2-1049 | H | H | CH₂-(2-CH₂CO₂Et-4-Triz) | 2-F | H | H |
| 2-1050 | Ac | H | CH₂-(2-CH₂CO₂Et-4-Triz) | 2-F | H | H |
| 2-1051 | H | H | CH₂-(2-CH₂CH₂CO₂H-4-Triz) | 2-F | H | H |
| 2-1052 | Ac | H | CH₂-(2-CH₂CH₂CO₂H-4-Triz) | 2-F | H | H |
| 2-1053 | H | H | CH₂-(2-CH₂CH₂CO₂Me-4-Triz) | 2-F | H | H |
| 2-1054 | Ac | H | CH₂-(2-CH₂CH₂CO₂Me-4-Triz) | 2-F | H | H |
| 2-1055 | H | H | CH₂-(2-CH₂CH₂CO₂Et-4-Triz) | 2-F | H | H |
| 2-1056 | Ac | H | CH₂-(2-CH₂CH₂CO₂Et-4-Triz) | 2-F | H | H |
| 2-1057 | H | H | CH₂-(2-CH₂CH₂CH₂CO₂H-4-Triz) | 2-F | H | H |
| 2-1058 | Ac | H | CH₂-(2-CH₂CH₂CH₂CO₂H-4-Triz) | 2-F | H | H |
| 2-1059 | H | H | CH₂-(2-CH₂CH₂CH₂CO₂Me-4-Triz) | 2-F | H | H |
| 2-1060 | Ac | H | CH₂-(2-CH₂CH₂CH₂CO₂Me-4-Triz) | 2-F | H | H |
| 2-1061 | H | H | CH₂-(2-CH₂CH₂CH₂CO₂Et-4-Triz) | 2-F | H | H |
| 2-1062 | Ac | H | CH₂-(2-CH₂CH₂CH₂CO₂Et-4-Triz) | 2-F | H | H |
| 2-1063 | H | H | CH₂CH₂-(2-CO₂Me-4-Triz) | 2-F | H | H |
| 2-1064 | Ac | H | CH₂CH₂-(2-CO₂Me-4-Triz) | 2-F | H | H |
| 2-1065 | H | H | CH₂CH₂-(2-CO₂Et-4-Triz) | 2-F | H | H |
| 2-1066 | Ac | H | CH₂CH₂-(2-CO₂Et-4-Triz) | 2-F | H | H |
| 2-1067 | H | H | CH₂CH₂-(2-CH₂CO₂H-4-Triz) | 2-F | H | H |
| 2-1068 | Ac | H | CH₂CH₂-(2-CH₂CO₂H-4-Triz) | 2-F | H | H |
| 2-1069 | H | H | CH₂CH₂-(2-CH₂CO₂Me-4-Triz) | 2-F | H | H |
| 2-1070 | Ac | H | CH₂CH₂-(2-CH₂CO₂Me-4-Triz) | 2-F | H | H |
| 2-1071 | H | H | CH₂CH₂-(2-CH₂CO₂Et-4-Triz) | 2-F | H | H |
| 2-1072 | Ac | H | CH₂CH₂-(2-CH₂CO₂Et-4-Triz) | 2-F | H | H |
| 2-1073 | H | H | CH₂CH₂-(2-CH₂CH₂CO₂H-4-Triz) | 2-F | H | H |
| 2-1074 | Ac | H | CH₂CH₂(2-CH₂CH₂CO₂H-4-Triz) | 2-F | H | H |
| 2-1075 | H | H | CH₂CH₂-(2-CH₂CH₂CO₂Me-4-Triz) | 2-F | H | H |
| 2-1076 | Ac | H | CH₂CH₂-(2-CH₂CH₂CO₂Me-4-Triz) | 2-F | H | H |
| 2-1077 | H | H | CH₂CH₂-(2-CH₂CH₂CO₂Et-4-Triz) | 2-F | H | H |
| 2-1078 | Ac | H | CH₂CH₂-(2-CH₂CH₂CO₂Et-4-Triz) | 2-F | H | H |
| 2-1079 | H | H | CH₂CH₂-(2-CH₂CH₂CH₂CO₂H-4-Triz) | 2-F | H | H |
| 2-1080 | Ac | H | CH₂CH₂-(2-CH₂CH₂CH₂CO₂H-4-Triz) | 2-F | H | H |
| 2-1081 | H | H | CH₂CH₂-(2-CH₂CH₂CH₂CO₂Me-4-Triz) | 2-F | H | H |
| 2-1082 | Ac | H | CH₂CH₂-(2-CH₂CH₂CH₂CO₂Me-4-Triz) | 2-F | H | H |
| 2-1083 | H | H | CH₂CH₂-(2-CH₂CH₂CH₂CO₂Et-4-Triz) | 2-F | H | H |
| 2-1084 | Ac | H | CH₂CH₂-(2-CH₂CH₂CH₂CO₂Et-4-Triz) | 2-F | H | H |

**(Table 3)**

| | | | | | | |
|---|---|---|---|---|---|---|
| 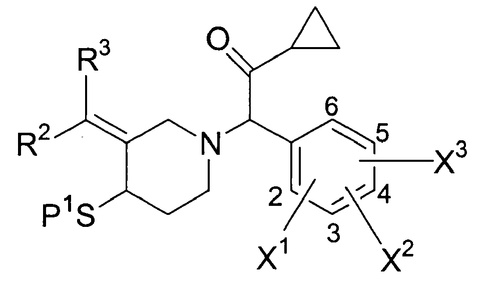 | | | | | | |

| Compound No. | P¹ | R² | R³ | X¹ | X² | X³ |
|---|---|---|---|---|---|---|
| 3-1 | H | H | CH₂OH | 2-F | H | H |
| 3-2 | Ac | H | CH₂OH | 2-F | H | H |
| 3-3 | H | H | CH₂CH₂OH | 2-F | H | H |
| 3-4 | Ac | H | CH₂CH₂OH | 2-F | H | H |
| 3-5 | H | H | CH₂OMe | 2-F | H | H |
| 3-6 | Ac | H | CH₂OMe | 2-F | H | H |
| 3-7 | H | H | CH₂CH₂OMe | 2-F | H | H |
| 3-8 | Ac | H | CH₂CH₂OMe | 2-F | H | H |
| 3-9 | H | H | CH₂OEt | 2-F | H | H |
| 3-10 | Ac | H | CH₂OEt | 2-F | H | H |
| 3-11 | H | H | CH₂CH₂OEt | 2-F | H | H |
| 3-12 | Ac | H | CH₂CH₂OEt | 2-F | H | H |
| 3-13 | H | H | CH₂CO₂H | 2-F | H | H |
| 3-14 | Ac | H | CH₂CO₂H | 2-F | H | H |
| 3-15 | H | H | CH₂CO₂Me | 2-F | H | H |
| 3-16 | Ac | H | CH₂CO₂Me | 2-F | H | H |
| 3-17 | H | H | CH₂CO₂Et | 2-F | H | H |
| 3-18 | Ac | H | CH₂CO₂Et | 2-F | H | H |
| 3-19 | H | H | 4-Pip | 2-F | H | H |
| 3-20 | Ac | H | 4-Pip | 2-F | H | H |
| 3-21 | H | H | 4-Pip | 2-F | 4-F | H |
| 3-22 | Ac | H | 4-Pip | 2-F | 4-F | H |
| 3-23 | H | H | 1-CO₂Me-4-Pip | 2-F | H | H |
| 3-24 | Ac | H | 1-CO₂Me-4-Pip | 2-F | H | H |
| 3-25 | H | H | 1-CO₂Et-4-Pip | 2-F | H | H |
| 3-26 | Ac | H | 1-CO₂Et-4-Pip | 2-F | H | H |
| 3-27 | H | H | 1-CH₂CO₂H-4-Pip | 2-F | H | H |
| 3-28 | Ac | H | 1-CH₂CO₂H-4-Pip | 2-F | H | H |
| 3-29 | H | H | 1-CH₂CO₂Me-4-Pip | 2-F | H | H |
| 3-30 | Ac | H | 1-CH₂CO₂Me-4-Pip | 2-F | H | H |
| 3-31 | H | H | 1-CH₂CO₂Et-4-Pip | 2-F | H | H |
| 3-32 | Ac | H | 1-CH₂CO₂Et-4-Pip | 2-F | H | H |
| 3-33 | H | H | 1-CH₂CH₂CO₂H-4-Pip | 2-F | H | H |
| 3-34 | Ac | H | 1-CH₂CH₂CO₂H-4-Pip | 2-F | H | H |
| 3-35 | H | H | 1-CH₂CH₂CO₂Me-4-Pip | 2-F | H | H |
| 3-36 | Ac | H | 1-CH₂CH₂CO₂Me-4-Pip | 2-F | H | H |
| 3-37 | H | H | 1-CH₂CH₂CO₂Et-4-Pip | 2-F | H | H |
| 3-38 | Ac | H | 1-CH₂CH₂CO₂Et-4-Pip | 2-F | H | H |
| 3-39 | Ac | H | 1-CH₂CH₂CO₂Et-4-Pip | 2-F | 4-F | H |
| 3-40 | H | H | 1-CH₂CH₂CH₂CO₂H-4-Pip | 2-F | H | H |
| 3-41 | Ac | H | 1-CH₂CH₂CH₂CO₂H-4-Pip | 2-F | H | H |
| 3-42 | H | H | 1-CH₂CH₂CH₂CO₂Me-4-Pip | 2-F | H | H |
| 3-43 | Ac | H | 1-CH₂CH₂CH₂CO₂Me-4-Pip | 2-F | H | H |
| 3-44 | H | H | 1-CH₂CH₂CH₂CO₂Et-4-Pip | 2-F | H | H |
| 3-45 | Ac | H | 1-CH₂CH₂CH₂CO₂Et-4-Pip | 2-F | H | H |
| 3-46 | H | H | CH₂CN | 2-F | H | H |
| 3-47 | Ac | H | CH₂CN | 2-F | H | H |
| 3-48 | H | H | CH₂CH₂CN | 2-F | H | H |
| 3-49 | Ac | H | CH₂CH₂CN | 2-F | H | H |
| 3-50 | H | H | CH₂N(Me)CH₂CO₂H | 2-F | H | H |
| 3-51 | Ac | H | CH₂N(Me)CH₂CO₂H | 2-F | H | H |
| 3-52 | H | H | CH₂N(Me)CH₂CO₂Me | 2-F | H | H |
| 3-53 | Ac | H | CH₂N(Me)CH₂CO₂Me | 2-F | H | H |
| 3-54 | H | H | CH₂N(Me)CH₂CO₂Et | 2-F | H | H |
| 3-55 | Ac | H | CH₂N(Me)CH₂CO₂Et | 2-F | H | H |
| 3-56 | H | H | CH₂N(Me)CH₂CH₂CO₂H | 2-F | H | H |
| 3-57 | Ac | H | CH₂N(Me)CH₂CH₂CO₂H | 2-F | H | H |
| 3-58 | H | H | CH₂N(Me)CH₂CH₂CO₂Me | 2-F | H | H |
| 3-59 | Ac | H | CH₂N(Me)CH₂CH₂CO₂Me | 2-F | H | H |
| 3-60 | H | H | CH₂N(Me)CH₂CH₂CO₂Et | 2-F | H | H |
| 3-61 | Ac | H | CH₂N(Me)CH₂CH₂CO₂Et | 2-F | H | H |
| 3-62 | H | H | CH₂N(Me)CH₂CH₂CH₂CO₂H | 2-F | H | H |
| 3-63 | Ac | H | CH₂N(Me)CH₂CH₂CH₂CO₂H | 2-F | H | H |
| 3-64 | H | H | CH₂N(Me)CH₂CH₂CH₂CO₂Me | 2-F | H | H |
| 3-65 | Ac | H | CH₂N(Me)CH₂CH₂CH₂CO₂Me | 2-F | H | H |
| 3-66 | H | H | CH₂N(Me)CH₂CH₂CH₂CO₂Et | 2-F | H | H |
| 3-67 | Ac | H | CH₂N(Me)CH₂CH₂CH₂CO₂Et | 2-F | H | H |
| 3-68 | H | H | CH₂N(Et)CH₂CO₂H | 2-F | H | H |
| 3-69 | Ac | H | CH₂N(Et)CH₂CO₂H | 2-F | H | H |
| 3-70 | H | H | CH₂N(Et)CH₂CO₂Me | 2-F | H | H |
| 3-71 | Ac | H | CH₂N(Et)CH₂CO₂Me | 2-F | H | H |
| 3-72 | H | H | CH₂N(Et)CH₂CO₂Et | 2-F | H | H |
| 3-73 | Ac | H | CH₂N(Et)CH₂CO₂Et | 2-F | H | H |
| 3-74 | H | H | CH₂N(iPr)CH₂CO₂H | 2-F | H | H |
| 3-75 | Ac | H | CH₂N(iPr)CH₂CO₂H | 2-F | H | H |
| 3-76 | H | H | CH₂N(iPr)CH₂CO₂Me | 2-F | H | H |
| 3-77 | Ac | H | CH₂N(iPr)CH₂CO₂Me | 2-F | H | H |
| 3-78 | H | H | CH₂N(iPr)CH₂CO₂Et | 2-F | H | H |
| 3-79 | Ac | H | CH₂N(iPr)CH₂CO₂Et | 2-F | H | H |

**(Table 4)**

| | | | | | | |
|---|---|---|---|---|---|---|
| 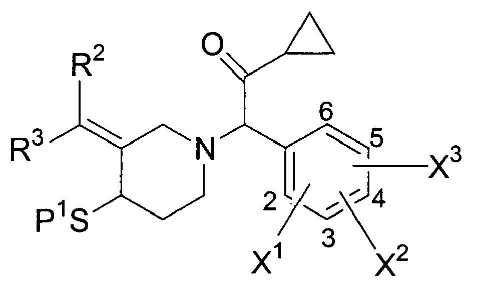 | | | | | | |

| Compound No. | P¹ | R² | R³ | X¹ | X² | X³ |
|---|---|---|---|---|---|---|
| 4-1 | H | H | CH₂OH | 2-F | H | H |
| 4-2 | Ac | H | CH₂OH | 2-F | H | H |
| 4-3 | H | H | CH₂CH₂OH | 2-F | H | H |
| 4-4 | Ac | H | CH₂CH₂OH | 2-F | H | H |
| 4-5 | H | H | CH₂OMe | 2-F | H | H |
| 4-6 | Ac | H | CH₂OMe | 2-F | H | H |
| 4-7 | H | H | CH₂CH₂OMe | 2-F | H | H |
| 4-8 | Ac | H | CH₂CH₂OMe | 2-F | H | H |
| 4-9 | H | H | CH₂OEt | 2-F | H | H |
| 4-10 | Ac | H | CH₂OEt | 2-F | H | H |
| 4-11 | H | H | CH₂CH₂OEt | 2-F | H | H |
| 4-12 | Ac | H | CH₂CH₂OEt | 2-F | H | H |
| 4-13 | H | H | CH₂CO₂H | 2-F | H | H |
| 4-14 | Ac | H | CH₂CO₂H | 2-F | H | H |
| 4-15 | H | H | CH₂CO₂Me | 2-F | H | H |
| 4-16 | Ac | H | CH₂CO₂Me | 2-F | H | H |
| 4-17 | H | H | CH₂CO₂Et | 2-F | H | H |
| 4-18 | Ac | H | CH₂CO₂Et | 2-F | H | H |
| 4-19 | H | H | 4-Pip | 2-F | H | H |
| 4-20 | Ac | H | 4-Pip | 2-F | H | H |
| 4-21 | H | H | 4-Pip | 2-F | 4-F | H |
| 4-22 | Ac | H | 4-Pip | 2-F | 4-F | H |
| 4-23 | H | H | 1-CO₂Me-4-Pip | 2-F | H | H |
| 4-24 | Ac | H | 1-CO₂Me-4-Pip | 2-F | H | H |
| 4-25 | H | H | 1-CO₂Et-4-Pip | 2-F | H | H |
| 4-26 | Ac | H | 1-CO₂Et-4-Pip | 2-F | H | H |
| 4-27 | H | H | 1-CH₂CO₂H-4-Pip | 2-F | H | H |
| 4-28 | Ac | H | 1-CH₂CO₂H-4-Pip | 2-F | H | H |
| 4-29 | H | H | 1-CH₂CO₂Me-4-Pip | 2-F | H | H |
| 4-30 | Ac | H | 1-CH₂CO₂Me-4-Pip | 2-F | H | H |
| 4-31 | H | H | 1-CH₂CO₂Et-4-Pip | 2-F | H | H |
| 4-32 | Ac | H | 1-CH₂CO₂Et-4-Pip | 2-F | H | H |
| 4-33 | H | H | 1-CH₂CH₂CO₂H-4-Pip | 2-F | H | H |
| 4-34 | Ac | H | 1-CH₂CH₂CO₂H-4-Pip | 2-F | H | H |
| 4-35 | H | H | 1-CH₂CH₂CO₂Me-4-Pip | 2-F | H | H |
| 4-36 | Ac | H | 1-CH₂CH₂CO₂Me-4-Pip | 2-F | H | H |
| 4-37 | H | H | 1-CH₂CH₂CO₂Et-4-Pip | 2-F | H | H |
| 4-38 | Ac | H | 1-CH₂CH₂CO₂Et-4-Pip | 2-F | H | H |
| 4-39 | Ac | H | 1-CH₂CH₂CO₂Et-4-Pip | 2-F | 4-F | H |
| 4-40 | H | H | 1-CH₂CH₂CH₂CO₂H-4-Pip | 2-F | H | H |
| 4-41 | Ac | H | 1-CH₂CH₂CH₂CO₂H-4-Pip | 2-F | H | H |
| 4-42 | H | H | 1-CH₂CH₂CH₂CO₂Me-4-Pip | 2-F | H | H |
| 4-43 | Ac | H | 1-CH₂CH₂CH₂CO₂Me-4-Pip | 2-F | H | H |
| 4-44 | H | H | 1-CH₂CH₂CH₂CO₂Et-4-Pip | 2-F | H | H |
| 4-45 | Ac | H | 1-CH₂CH₂CH₂CO₂Et-4-Pip | 2-F | H | H |
| 4-46 | H | H | CH₂CN | 2-F | H | H |
| 4-47 | Ac | H | CH₂CN | 2-F | H | H |
| 4-48 | H | H | CH₂CH₂CN | 2-F | H | H |
| 4-49 | Ac | H | CH₂CH₂CN | 2-F | H | H |
| 4-50 | H | H | CH₂N(Me)CH₂CO₂H | 2-F | H | H |
| 4-51 | Ac | H | CH₂N(Me)CH₂CO₂H | 2-F | H | H |
| 4-52 | H | H | CH₂N(Me)CH₂CO₂Me | 2-F | H | H |
| 4-53 | Ac | H | CH₂N(Me)CH₂CO₂Me | 2-F | H | H |
| 4-54 | H | H | CH₂N(Me)CH₂CO₂Et | 2-F | H | H |
| 4-55 | Ac | H | CH₂N(Me)CH₂CO₂Et | 2-F | H | H |
| 4-56 | H | H | CH₂N(Me)CH₂CH₂CO₂H | 2-F | H | H |
| 4-57 | Ac | H | CH₂N(Me)CH₂CH₂CO₂H | 2-F | H | H |
| 4-58 | H | H | CH₂N(Me)CH₂CH₂CO₂Me | 2-F | H | H |
| 4-59 | Ac | H | CH₂N(Me)CH₂CH₂CO₂Me | 2-F | H | H |
| 4-60 | H | H | CH₂N(Me)CH₂CH₂CO₂Et | 2-F | H | H |
| 4-61 | Ac | H | CH₂N(Me)CH₂CH₂CO₂Et | 2-F | H | H |
| 4-62 | H | H | CH₂N(Me)CH₂CH₂CH₂CO₂H | 2-F | H | H |
| 4-63 | Ac | H | CH₂N(Me)CH₂CH₂CH₂CO₂H | 2-F | H | H |
| 4-64 | H | H | CH₂N(Me)CH₂CH₂CH₂CO₂Me | 2-F | H | H |
| 4-65 | Ac | H | CH₂N(Me)CH₂CH₂CH₂CO₂Me | 2-F | H | H |
| 4-66 | H | H | CH₂N(Me)CH₂CH₂CH₂CO₂Et | 2-F | H | H |
| 4-67 | Ac | H | CH₂N(Me)CH₂CH₂CH₂CO₂Et | 2-F | H | H |
| 4-68 | H | H | CH₂N(Et)CH₂CO₂H | 2-F | H | H |
| 4-69 | Ac | H | CH₂N(Et)CH₂CO₂H | 2-F | H | H |
| 4-70 | H | H | CH₂N(Et)CH₂CO₂Me | 2-F | H | H |
| 4-71 | Ac | H | CH₂N(Et)CH₂CO₂Me | 2-F | H | H |
| 4-72 | H | H | CH₂N(Et)CH₂CO₂Et | 2-F | H | H |
| 4-73 | Ac | H | CH₂N(Et)CH₂CO₂Et | 2-F | H | H |
| 4-74 | H | H | CH₂N(iPr)CH₂CO₂H | 2-F | H | H |
| 4-75 | Ac | H | CH₂N(iPr)CH₂CO₂H | 2-F | H | H |
| 4-76 | H | H | CH₂N(iPr)CH₂CO₂Me | 2-F | H | H |
| 4-77 | Ac | H | CH₂N(iPr)CH₂CO₂Me | 2-F | H | H |
| 4-78 | H | H | CH₂N(iPr)CH₂CO₂Et | 2-F | H | H |
| 4-79 | Ac | H | CH₂N(iPr)CH₂CO₂Et | 2-F | H | H |

**(Table 5)**

| | | | | | | |
|---|---|---|---|---|---|---|
| 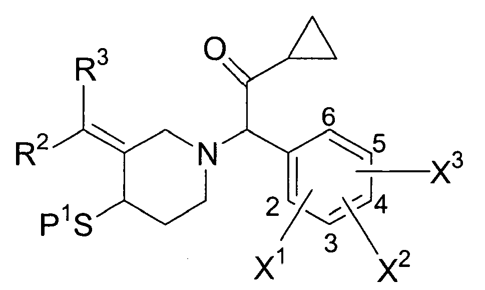 | | | | | | |

| Compound No. | P¹ | R² | R³ | X¹ | X² | X³ |
|---|---|---|---|---|---|---|
| 5-1 | H | H | CH₂-1-Pip | 2-F | H | H |
| 5-2 | Ac | H | CH₂-1-Pip | 2-F | H | H |
| 5-3 | H | H | CH₂-(4-CO₂H-1-Pip) | 2-F | H | H |
| 5-4 | Ac | H | CH₂-(4-CO₂H-1-Pip) | 2-F | H | H |
| 5-5 | H | H | CH₂-(4-CO₂Me-1-Pip) | 2-F | H | H |
| 5-6 | Ac | H | CH₂-(4-CO₂Me-1-Pip) | 2-F | H | H |
| 5-7 | H | H | CH₂-(4-CO₂Et-1-Pip) | 2-F | H | H |
| 5-8 | Ac | H | CH₂-(4-CO₂Et-1-Pip) | 2-F | H | H |
| 5-9 | H | H | CH₂-(4-CH₂CO₂H-1-Pip) | 2-F | H | H |
| 5-10 | Ac | H | CH₂-(4-CH₂CO₂H-1-Pip) | 2-F | H | H |
| 5-11 | H | H | CH₂-(4-CH₂CO₂Me-1-Pip) | 2-F | H | H |
| 5-12 | Ac | H | CH₂-(4-CH₂CO₂Me-1-Pip) | 2-F | H | H |
| 5-13 | H | H | CH₂-(4-CH₂CO₂Et-1-Pip) | 2-F | H | H |
| 5-14 | Ac | H | CH₂-(4-CH₂CO₂Et-1-Pip) | 2-F | H | H |
| 5-15 | H | H | CH₂-(4-CH₂CH₂CO₂H-1-Pip) | 2-F | H | H |
| 5-16 | Ac | H | CH₂-(4-CH₂CH₂CO₂H-1-Pip) | 2-F | H | H |
| 5-17 | H | H | CH₂-(4-CH₂CH₂CO₂Me-1-Pip) | 2-F | H | H |
| 5-18 | Ac | H | CH₂-(4-CH₂CH₂CO₂Me-1-Pip) | 2-F | H | H |
| 5-19 | H | H | CH₂-(4-CH₂CH₂CO₂Et-1-Pip) | 2-F | H | H |
| 5-20 | Ac | H | CH₂-(4-CH₂CH₂CO₂Et-1-Pip) | 2-F | H | H |
| 5-21 | H | H | CH₂-(4-CH₂CH₂CH₂CO₂H-1-Pip) | 2-F | H | H |
| 5-22 | Ac | H | CH₂-(4-CH₂CH₂CH₂CO₂H-1-Pip) | 2-F | H | H |
| 5-23 | H | H | CH₂-(4-CH₂CH₂CH₂CO₂Me-1-Pip) | 2-F | H | H |
| 5-24 | Ac | H | CH₂-(4-CH₂CH₂CH₂CO₂Me-1-Pip) | 2-F | H | H |
| 5-25 | H | H | CH₂-(4-CH₂CH₂CH₂CO₂Et-1-Pip) | 2-F | H | H |
| 5-26 | Ac | H | CH₂-(4-CH₂CH₂CH₂CO₂Et-1-Pip) | 2-F | H | H |
| 5-27 | H | H | CH₂-Mor | 2-F | H | H |
| 5-28 | Ac | H | CH₂-Mor | 2-F | H | H |
| 5-29 | H | H | CH₂-Thim | 2-F | H | H |
| 5-30 | Ac | H | CH₂-Thim | 2-F | H | H |
| 5-31 | H | H | CH₂-1-Piz | 2-F | H | H |
| 5-32 | Ac | H | CH₂-1-Piz | 2-F | H | H |
| 5-33 | H | H | CH₂-(4-Ac-1-Piz) | 2-F | H | H |
| 5-34 | Ac | H | CH₂-(4-Ac-1-Piz) | 2-F | H | H |
| 5-35 | H | H | CH₂-(4-Ac-1-Piz) | 2-F | 4-F | H |
| 5-36 | Ac | H | CH₂-(4-Ac-1-Piz) | 2-F | 4-F | H |
| 5-37 | H | H | CH₂-(4-CO₂Me-1-Piz) | 2-F | H | H |
| 5-38 | Ac | H | CH₂-(4-CO₂Me-1-Piz) | 2-F | H | H |
| 5-39 | H | H | CH₂-(4-CO₂Et-1-Piz) | 2-F | H | H |
| 5-40 | Ac | H | CH₂-(4-CO₂Et-1-Piz) | 2-F | H | H |
| 5-41 | H | H | CH₂-(4-CH₂CO₂H-1-Piz) | 2-F | H | H |
| 5-42 | Ac | H | CH₂-(4-CH₂CO₂H-1-Piz) | 2-F | H | H |
| 5-43 | H | H | CH₂-(4-CH₂CO₂Me-1-Piz) | 2-F | H | H |
| 5-44 | Ac | H | CH₂-(4-CH₂CO₂Me-1-Piz) | 2-F | H | H |
| 5-45 | H | H | CH₂-(4-CH₂CO₂Et-1-Piz) | 2-F | H | H |
| 5-46 | Ac | H | CH₂-(4-CH₂CO₂Et-1-Piz) | 2-F | H | H |
| 5-47 | H | H | CH₂-(4-CH₂CH₂CO₂H-1-Piz) | 2-F | H | H |
| 5-48 | Ac | H | CH₂-(4-CH₂CH₂CO₂H-1-Piz) | 2-F | H | H |
| 5-49 | H | H | CH₂-(4-CH₂CH₂CO₂Me-1-Piz) | 2-F | H | H |
| 5-50 | Ac | H | CH₂-(4-CH₂CH₂CO₂Me-1-Piz) | 2-F | H | H |
| 5-51 | H | H | CH₂-(4-CH₂CH₂CO₂Et-1-Piz) | 2-F | H | H |
| 5-52 | Ac | H | CH₂-(4-CH₂CH₂CO₂Et-1-Piz) | 2-F | H | H |
| 5-53 | H | H | CH₂-(4-CH₂CH₂CH₂CO₂H-1-Piz) | 2-F | H | H |
| 5-54 | Ac | H | CH₂-(4-CH₂CH₂CH₂CO₂H-1-Piz) | 2-F | H | H |
| 5-55 | H | H | CH₂-(4-CH₂CH₂CH₂CO₂Me-1-Piz) | 2-F | H | H |
| 5-56 | Ac | H | CH₂-(4-CH₂CH₂CH₂CO₂Me-1-Piz) | 2-F | H | H |
| 5-57 | H | H | CH₂-(4-CH₂CH₂CH₂CO₂Et-1-Piz) | 2-F | H | H |
| 5-58 | Ac | H | CH₂-(4-CH₂CH₂CH₂CO₂Et-1-Piz) | 2-F | H | H |
| 5-59 | H | H | CH₂-(2-oxo-1-Piz) | 2-F | H | H |
| 5-60 | Ac | H | CH₂-(2-oxo-1-Piz) | 2-F | H | H |
| 5-61 | H | H | CH₂-(4-Ac-2-oxo-1-Piz) | 2-F | H | H |
| 5-62 | Ac | H | CH₂-(4-Ac-2-oxo-1Piz) | 2-F | H | H |
| 5-63 | H | H | CH₂-(4-Ac-2-oxo-1-Piz) | 2-F | 4-F | H |
| 5-64 | Ac | H | CH₂-(4-Ac-2-oxo-1-Piz) | 2-F | 4-F | H |
| 5-65 | H | H | CH₂-(4-CO₂Me-2-oxo-1-Piz) | 2-F | H | H |
| 5-66 | Ac | H | CH₂-(4-CO₂Me-2-oxo-1-Piz) | 2-F | H | H |
| 5-67 | H | H | CH₂-(4-CO₂Et-2-oxo-1-Piz) | 2-F | H | H |
| 5-68 | Ac | H | CH₂-(4-CO₂Et-2-oxo-1-Piz) | 2-F | H | H |
| 5-69 | H | H | CH₂-(4-CH₂CO₂H-2-oxo-1-Piz) | 2-F | H | H |
| 5-70 | Ac | H | CH₂-(4-CH₂CO₂H-2-oxo-1-Piz) | 2-F | H | H |
| 5-71 | H | H | CH₂-(4-CH₂CO₂Me-2-oxo-1-Piz) | 2-F | H | H |
| 5-72 | Ac | H | CH₂-(4-CH₂CO₂Me-2-oxo-1-Piz) | 2-F | H | H |
| 5-73 | H | H | CH₂-(4-CH₂CO₂Et-2-oxo-1-Piz) | 2-F | H | H |
| 5-74 | Ac | H | CH₂-(4-CH₂CO₂Et-2-oxo-1-Piz) | 2-F | H | H |
| 5-75 | H | H | CH₂-(4-CH₂CH₂CO₂H-2-oxo-1-Piz) | 2-F | H | H |
| 5-76 | Ac | H | CH₂-(4-CH₂CH₂CO₂H-2-oxo-1-Piz) | 2-F | H | H |
| 5-77 | H | H | CH₂-(4-CH₂CH₂CO₂Me-2-oxo-1-Piz) | 2-F | H | H |
| 5-78 | Ac | H | CH₂-(4-CH₂CH₂CO₂Me-2-oxo-1-Piz) | 2-F | H | H |
| 5-79 | H | H | CH₂-(4-CH₂CH₂CO₂Et-2-oxo-1-Piz) | 2-F | H | H |
| 5-80 | Ac | H | CH₂-(4-CH₂CH₂CO₂Et-2-oxo-1-Piz) | 2-F | H | H |
| 5-81 | H | H | CH₂-(4-CH₂CH₂CH₂CO₂H-2-oxo-1-Piz) | 2-F | H | H |
| 5-82 | Ac | H | CH₂-(4-CH₂CH₂CH₂CO₂H-2-oxo-1-Piz) | 2-F | H | H |
| 5-83 | H | H | CH₂-(4-CH₂CH₂CH₂CO₂Me-2-oxo-1-Piz) | 2-F | H | H |
| 5-84 | Ac | H | CH₂-(4-CH₂CH₂CH₂CO₂Me-2-oxo-1-Piz) | 2-F | H | H |
| 5-85 | H | H | CH₂-(4-CH₂CH₂CH₂CO₂Et-2-oxo-1-Piz) | 2-F | H | H |
| 5-86 | Ac | H | CH₂-(4-CH₂CH₂CH₂CO₂Et-2-oxo-1-Piz) | 2-F | H | H |
| 5-87 | H | H | CH₂-(3-oxo-1-Piz) | 2-F | H | H |
| 5-88 | Ac | H | CH₂-(3-oxo-1-Piz) | 2-F | H | H |
| 5-89 | H | H | CH₂-(4-Ac-3-oxo-1-Piz) | 2-F | H | H |
| 5-90 | Ac | H | CH₂-(4-Ac-3-oxo-1-Piz) | 2-F | H | H |
| 5-91 | H | H | CH₂-(4-Ac-3-oxo-1-Piz) | 2-F | 4-F | H |
| 5-92 | Ac | H | CH₂-(4-Ac-3-oxo-1-Piz) | 2-F | 4-F | H |
| 5-93 | H | H | CH₂-(4-CO₂Me-3-oxo-1-Piz) | 2-F | H | H |
| 5-94 | Ac | H | CH₂-(4-CO₂Me-3-oxo-1-Piz) | 2-F | H | H |
| 5-95 | H | H | CH₂-(4-CO₂Et-3-oxo-1-Piz) | 2-F | H | H |
| 5-96 | Ac | H | CH₂-(4-CO₂Et-3-oxo-1-Piz) | 2-F | H | H |
| 5-97 | H | H | CH₂-(4-CH₂CO₂H-3-oxo-1-Piz) | 2-F | H | H |
| 5-98 | Ac | H | CH₂-(4-CH₂CO₂H-3-oxo-1-Piz) | 2-F | H | H |
| 5-99 | H | H | CH₂-(4-CH₂CO₂Me-3-oxo-1-Piz) | 2-F | H | H |
| 5-100 | Ac | H | CH₂-(4-CH₂CO₂Me-3-oxo-1-Piz) | 2-F | H | H |
| 5-101 | H | H | CH₂-(4-CH₂CO₂Et-3-oxo-1-Piz) | 2-F | H | H |
| 5-102 | Ac | H | CH₂-(4-CH₂CO₂Et-3-oxo-1-Piz) | 2-F | H | H |
| 5-103 | H | H | CH₂-(4-CH₂CH₂CO₂H-3-oxo-1-Piz) | 2-F | H | H |
| 5-104 | Ac | H | CH₂-(4-CH₂CH₂CO₂H-3-oxo-1-Piz) | 2-F | H | H |
| 5-105 | H | H | CH₂-(4-CH₂CH₂CO₂Me-3-oxo-1-Piz) | 2-F | H | H |
| 5-106 | Ac | H | CH₂-(4-CH₂CH₂CO₂Me-3-oxo-1-Piz) | 2-F | H | H |
| 5-107 | H | H | CH₂-(4-CH₂CH₂CO₂Et-3-oxo-1-Piz) | 2-F | H | H |
| 5-108 | Ac | H | CH₂-(4-CH₂CH₂CO₂Et-3-oxo-1-Piz) | 2-F | H | H |
| 5-109 | H | H | CH₂-(4-CH₂CH₂CH₂CO₂H-3-oxo-1-Piz) | 2-F | H | H |
| 5-110 | Ac | H | CH₂-(4-CH₂CH₂CH₂CO₂H-3-oxo-1-Piz) | 2-F | H | H |
| 5-111 | H | H | CH₂-(4-CH₂CH₂CH₂CO₂Me-3-oxo-1-Piz) | 2-F | H | H |
| 5-112 | Ac | H | CH₂-(4-CH₂CH₂CH₂CO₂Me-3-oxo-1-Piz) | 2-F | H | H |
| 5-113 | H | H | CH₂-(4-CH₂CH₂CH₂CO₂Et-3-oxo-1-Piz) | 2-F | H | H |
| 5-114 | Ac | H | CH₂-(4-CH₂CH₂CH₂CO₂Et-3-oxo-1-Piz) | 2-F | H | H |
| 5-115 | H | H | CH₂-(2,5-dioxo-1-Piz) | 2-F | H | H |
| 5-116 | Ac | H | CH₂-(2,5-dioxo-1-Piz) | 2-F | H | H |
| 5-117 | H | H | CH₂-(4-Ac-2,5-dioxo-1-Piz) | 2-F | H | H |
| 5-118 | Ac | H | CH₂-(4-Ac-2,5-dioxo-1-Piz) | 2-F | H | H |
| 5-119 | H | H | CH₂-(4-Ac-2,5-dioxo-1-Piz) | 2-F | 4-F | H |
| 5-120 | Ac | H | CH₂-(4-Ac-2,5-dioxo-1-Piz) | 2-F | 4-F | H |
| 5-121 | H | H | CH₂-(4-CO₂Me-2,5-dioxo-1-Piz) | 2-F | H | H |
| 5-122 | Ac | H | CH₂-(4-CO₂Me-2,5-dioxo-1-Piz) | 2-F | H | H |
| 5-123 | H | H | CH₂-(4-CO₂Et-2,5-dioxo-1-Piz) | 2-F | H | H |
| 5-124 | Ac | H | CH₂-(4-CO₂Et-2,5-dioxo-1-Piz) | 2-F | H | H |
| 5-125 | H | H | CH₂-(4-CH₂CO₂H-2,5-dioxo-1-Piz) | 2-F | H | H |
| 5-126 | Ac | H | CH₂-(4-CH₂CO₂H-2,5-dioxo-1-Piz) | 2-F | H | H |
| 5-127 | H | H | CH₂-(4-CH₂CO₂Me-2,5-dioxo-1-Piz) | 2-F | H | H |
| 5-128 | Ac | H | CH₂-(4-CH₂CO₂Me-2,5-dioxo-1-Piz) | 2-F | H | H |
| 5-129 | H | H | CH₂-(4-CH₂CO₂Et-2,5-dioxo-1-Piz) | 2-F | H | H |
| 5-130 | Ac | H | CH₂-(4-CH₂CO₂Et-2,5-dioxo-1-Piz) | 2-F | H | H |
| 5-131 | H | H | CH₂-(4-CH₂CH₂CO₂H-2,5-dioxo-1-Piz) | 2-F | H | H |
| 5-132 | Ac | H | CH₂-(4-CH₂CH₂CO₂H-2,5-dioxo-1-Piz) | 2-F | H | H |
| 5-133 | H | H | CH₂-(4-CH₂CH₂CO₂Me-2,5-dioxo-1-Piz) | 2-F | H | H |
| 5-134 | Ac | H | CH₂-(4-CH₂CH₂CO₂Me-2,5-dioxo-1-Piz) | 2-F | H | H |
| 5-135 | H | H | CH₂-(4-CH₂CH₂CO₂Et-2,5-dioxo-1-Piz) | 2-F | H | H |
| 5-136 | Ac | H | CH₂-(4-CH₂CH₂CO₂Et-2,5-dioxo-1-Piz) | 2-F | H | H |
| 5-137 | H | H | CH₂-(4-CH₂CH₂CH₂CO₂H-2,5-dioxo-1-Piz) | 2-F | H | H |
| 5-138 | Ac | H | CH₂-(4-CH₂CH₂CH₂CO₂H-2,5-dioxo-1-Piz) | 2-F | H | H |
| 5-139 | H | H | CH₂-(4-CH₂CH₂CH₂CO₂Me-2,5-dioxo-1-Piz) | 2-F | H | H |
| 5-140 | Ac | H | CH₂-(4-CH₂CH₂CH₂CO₂Me-2,5-dioxo-1-Piz) | 2-F | H | H |
| 5-141 | H | H | CH₂-(4-CH₂CH₂CH₂CO₂Et-2,5-dioxo-1-Piz) | 2-F | H | H |
| 5-142 | Ac | H | CH₂-(4-CH₂CH₂CH₂CO₂Et-2,5-dioxo-1-Piz) | 2-F | H | H |
| 5-143 | H | H | CH₂-1-Pyrd | 2-F | H | H |
| 5-144 | Ac | H | CH₂-1-Pyrd | 2-F | H | H |
| 5-145 | H | H | CH₂-(2-CO₂H-1-Pyrd) | 2-F | H | H |
| 5-146 | Ac | H | CH₂-(2-CO₂H-1-Pyrd) | 2-F | H | H |
| 5-147 | H | H | CH₂-(2-CO₂Me-1-Pyrd) | 2-F | H | H |
| 5-148 | Ac | H | CH₂-(2-CO₂Me-1-Pyrd) | 2-F | H | H |
| 5-149 | H | H | CH₂-(2-CO₂Et-1-Pyrd) | 2-F | H | H |
| 5-150 | Ac | H | CH₂-(2-CO₂Et-1-Pyrd) | 2-F | H | H |
| 5-151 | H | H | CH₂-(2-CH₂CO₂H-1-Pyrd) | 2-F | H | H |
| 5-152 | Ac | H | CH₂-(2-CH₂CO₂H-1-Pyrd) | 2-F | H | H |
| 5-153 | H | H | CH₂-(2-CH₂CO₂Me-1-Pyrd) | 2-F | H | H |
| 5-154 | Ac | H | CH₂-(2-CH₂CO₂Me-1-Pyrd) | 2-F | H | H |
| 5-155 | H | H | CH₂-(2-CH₂CO₂Et-1-Pyrd) | 2-F | H | H |
| 5-156 | Ac | H | CH₂-(2-CH₂CO₂Et-1-Pyrd) | 2-F | H | H |
| 5-157 | H | H | CH₂-(2-CH₂CH₂CO₂H-1-Pyrd) | 2-F | H | H |
| 5-158 | Ac | H | CH₂-(2-CH₂CH₂CO₂H-1-Pyrd) | 2-F | H | H |
| 5-159 | H | H | CH₂-(2-CH₂CH₂CO₂Me-1-Pyrd) | 2-F | H | H |
| 5-160 | Ac | H | CH₂-(2-CH₂CH₂CO₂Me-1-Pyrd) | 2-F | H | H |
| 5-161 | H | H | CH₂-(2-CH₂CH₂CO₂Et-1-Pyrd) | 2-F | H | H |
| 5-162 | Ac | H | CH₂-(2-CH₂CH₂CO₂Et-1-Pyrd) | 2-F | H | H |
| 5-163 | H | H | CH₂-(2-CH₂CH₂CH₂CO₂H-1-Pyrd) | 2-F | H | H |
| 5-164 | Ac | H | CH₂-(2-CH₂CH₂CH₂CO₂H-1-Pyrd) | 2-F | H | H |
| 5-165 | H | H | CH₂-(2-CH₂CH₂CH₂CO₂Me-1-Pyrd) | 2-F | H | H |
| 5-166 | Ac | H | CH₂-(2-CH₂CH₂CH₂CO₂Me-1-Pyrd) | 2-F | H | H |
| 5-167 | H | H | CH₂-(2-CH₂CH₂CH₂CO₂Et-1-Pyrd) | 2-F | H | H |
| 5-168 | Ac | H | CH₂-(2-CH₂CH₂CH₂CO₂Et-1-Pyrd) | 2-F | H | H |
| 5-169 | H | H | CH₂CH₂-Pip | 2-F | H | H |
| 5-170 | Ac | H | CH₂CH₂-1-Pip | 2-F | H | H |
| 5-171 | H | H | CH₂CH₂-(4-CO₂H-1-Pip) | 2-F | H | H |
| 5-172 | Ac | H | CH₂CH₂-(4-CO₂H-1-Pip) | 2-F | H | H |
| 5-173 | H | H | CH₂CH₂-(4-CO₂Me-1-Pip) | 2-F | H | H |
| 5-174 | Ac | H | CH₂CH₂-(4-CO₂Me-1-Pip) | 2-F | H | H |
| 5-175 | H | H | CH₂CH₂-(4-CO₂Et-1-Pip) | 2-F | H | H |
| 5-176 | Ac | H | CH₂CH₂-(4-CO₂Et-1-Pip) | 2-F | H | H |
| 5-177 | H | H | CH₂CH₂-(4-CH₂CO₂H-1-Pip) | 2-F | H | H |
| 5-178 | Ac | H | CH₂CH₂-(4-CH₂CO₂H-1-Pip) | 2-F | H | H |
| 5-179 | H | H | CH₂CH₂-(4-CH₂CO₂Me-1-Pip) | 2-F | H | H |
| 5-180 | Ac | H | CH₂CH₂-(4-CH₂CO₂Me-1-Pip) | 2-F | H | H |
| 5-181 | H | H | CH₂CH₂-(4-CH₂CO₂Et-1-Pip) | 2-F | H | H |
| 5-182 | Ac | H | CH₂CH₂-(4-CH₂CO₂Et-1-Pip) | 2-F | H | H |
| 5-183 | H | H | CH₂CH₂-(4-CH₂CH₂CO₂H-1-Pip) | 2-F | H | H |
| 5-184 | Ac | H | CH₂CH₂-(4-CH₂CH₂CO₂H-1-Pip) | 2-F | H | H |
| 5-185 | H | H | CH₂CH₂-(4-CH₂CH₂CO₂Me-1-Pip) | 2-F | H | H |
| 5-186 | Ac | H | CH₂CH₂-(4-CH₂CH₂CO₂Me-1-Pip) | 2-F | H | H |
| 5-187 | H | H | CH₂CH₂-(4-CH₂CH₂CO₂Et-1-Pip) | 2-F | H | H |
| 5-188 | Ac | H | CH₂CH₂-(4-CH₂CH₂CO₂Et-1-Pip) | 2-F | H | H |
| 5-189 | H | H | CH₂CH₂-(4-CH₂CH₂CH₂CO₂H-1-Pip) | 2-F | H | H |
| 5-190 | Ac | H | CH₂CH₂-(4-CH₂CH₂CH₂CO₂H-1-Pip) | 2-F | H | H |
| 5-191 | H | H | CH₂CH₂-(4-CH₂CH₂CH₂CO₂Me-1-Pip) | 2-F | H | H |
| 5-192 | Ac | H | CH₂CH₂-(4-CH₂CH₂CH₂CO₂Me-1-Pip) | 2-F | H | H |
| 5-193 | H | H | CH₂CH₂-(4-CH₂CH₂CH₂CO₂Et-1-Pip) | 2-F | H | H |
| 5-194 | Ac | H | CH₂CH₂-(4-CH₂CH₂CH₂CO₂Et-1-Pip) | 2-F | H | H |
| 5-195 | H | H | CH₂CH₂-Mor | 2-F | H | H |
| 5-196 | Ac | H | CH₂CH₂-Mor | 2-F | H | H |
| 5-197 | H | H | CH₂CH₂-Thim | 2-F | H | H |
| 5-198 | Ac | H | CH₂CH₂-Thim | 2-F | H | H |
| 5-199 | H | H | CH₂CH₂-1-Piz | 2-F | H | H |
| 5-200 | Ac | H | CH₂CH₂-1-Piz | 2-F | H | H |
| 5-201 | H | H | CH₂CH₂-(4-Ac-1-Piz) | 2-F | H | H |
| 5-202 | Ac | H | CH₂CH₂-(4-Ac-1-Piz) | 2-F | H | H |
| 5-203 | H | H | CH₂CH₂-(4-Ac-1-Piz) | 2-F | 4-F | H |
| 5-204 | Ac | H | CH₂CH₂-(4-Ac-1-Piz) | 2-F | 4-F | H |
| 5-205 | H | H | CH₂CH₂-(4-CO₂Me-1-Piz) | 2-F | H | H |
| 5-206 | Ac | H | CH₂CH₂-(4-CO₂Me-1-Piz) | 2-F | H | H |
| 5-207 | H | H | CH₂CH₂-(4-CO₂Et-1-Piz) | 2-F | H | H |
| 5-208 | Ac | H | CH₂CH₂-(4-CO₂Et-1-Piz) | 2-F | H | H |
| 5-209 | H | H | CH₂CH₂-(4-CH₂CO₂H-1-Piz) | 2-F | H | H |
| 5-210 | Ac | H | CH₂CH₂-(4-CH₂CO₂H-1-Piz) | 2-F | H | H |
| 5-211 | H | H | CH₂CH₂-(4-CH₂CO₂Me-1-Piz) | 2-F | H | H |
| 5-212 | Ac | H | CH₂CH₂-(4-CH₂CO₂Me-1-Piz) | 2-F | H | H |
| 5-213 | H | H | CH₂CH₂-(4-CH₂CO₂Et-1-Piz) | 2-F | H | H |
| 5-214 | Ac | H | CH₂CH₂-(4-CH₂CO₂Et-1-Piz) | 2-F | H | H |
| 5-215 | H | H | CH₂CH₂-(4-CH₂CH₂CO₂H-1-Piz) | 2-F | H | H |
| 5-216 | Ac | H | CH₂CH₂-(4-CH₂CH₂CO₂H-1-Piz) | 2-F | H | H |
| 5-217 | H | H | CH₂CH₂-(4-CH₂CH₂CO₂Me-1-Piz) | 2-F | H | H |
| 5-218 | Ac | H | CH₂CH₂-(4-CH₂CH₂CO₂Me-1-Piz) | 2-F | H | H |
| 5-219 | H | H | CH₂CH₂-(4-CH₂CH₂CO₂Et-1-Piz) | 2-F | H | H |
| 5-220 | Ac | H | CH₂CH₂-(4-CH₂CH₂CO₂Et-1-Piz) | 2-F | H | H |
| 5-221 | H | H | CH₂CH₂-(4-CH₂CH₂CH₂CO₂H-1-Piz) | 2-F | H | H |
| 5-222 | Ac | H | CH₂CH₂-(4-CH₂CH₂CH₂CO₂H-1-Piz) | 2-F | H | H |
| 5-223 | H | H | CH₂CH₂-(4-CH₂CH₂CH₂CO₂Me-1-Piz) | 2-F | H | H |
| 5-224 | Ac | H | CH₂CH₂-(4-CH₂CH₂CH₂CO₂Me-1-Piz) | 2-F | H | H |
| 5-225 | H | H | CH₂CH₂-(4-CH₂CH₂CH₂CO₂Et-1-Piz) | 2-F | H | H |
| 5-226 | Ac | H | CH₂CH₂-(4-CH₂CH₂CH₂CO₂Et-1-Piz) | 2-F | H | H |
| 5-227 | H | H | CH₂CH₂(2-oxo-1-Piz) | 2-F | H | H |
| 5-228 | Ac | H | CH₂CH₂-(2-oxo-1-Piz) | 2-F | H | H |
| 5-229 | H | H | CH₂CH₂-(4-Ac-2-oxo-1-Piz) | 2-F | H | H |
| 5-230 | Ac | H | CH₂CH₂-(4-Ac-2-oxo-1-Piz) | 2-F | H | H |
| 5-231 | H | H | CH₂CH₂(4-Ac-2-oxo-1-Piz) | 2-F | 4-F | H |
| 5-232 | Ac | H | CH₂CH₂-(4-Ac-2-oxo-1-Piz) | 2-F | 4-F | H |
| 5-233 | H | H | CH₂CH₂-(4-CO₂Me-2-oxo-1-Piz) | 2-F | H | H |
| 5-234 | Ac | H | CH₂CH₂-(4-CO₂Me-2-oxo-1-Piz) | 2-F | H | H |
| 5-235 | H | H | CH₂CH₂-(4-CO₂Et-2-oxo-1-Piz) | 2-F | H | H |
| 5-236 | Ac | H | CH₂CH₂-(4-CO₂Et-2-oxo-1-Piz) | 2-F | H | H |
| 5-237 | H | H | CH₂CH₂-(4-CH₂CO₂H-2-oxo-1-Piz) | 2-F | H | H |
| 5-238 | Ac | H | CH₂CH₂-(4-CH₂CO₂H-2-oxo-1-Piz) | 2-F | H | H |
| 5-239 | H | H | CH₂CH₂-(4-CH₂CO₂Me-2-oxo-1-Piz) | 2-F | H | H |
| 5-240 | Ac | H | CH₂CH₂-(4-CH₂CO₂Me-2-oxo-1-Piz) | 2-F | H | H |
| 5-241 | H | H | CH₂CH₂-(4-CH₂CO₂Et-2-oxo-1-Piz) | 2-F | H | H |
| 5-242 | Ac | H | CH₂CH₂-(4-CH₂CO₂Et-2-oxo-1-Piz) | 2-F | H | H |
| 5-243 | H | H | CH₂CH₂-(4-CH₂CH₂CO₂H-2-oxo-1-Piz) | 2-F | H | H |
| 5-244 | Ac | H | CH₂CH₂-(4-CH₂CH₂CO₂H-2-oxo-1-Piz) | 2-F | H | H |
| 5-245 | H | H | CH₂CH₂-(4-CH₂CH₂CO₂Me-2-oxo-1-Piz) | 2-F | H | H |
| 5-246 | Ac | H | CH₂CH₂-(4-CH₂CH₂CO₂Me-2-oxo-1-Piz) | 2-F | H | H |
| 5-247 | H | H | CH₂CH₂-(4-CH₂CH₂CO₂Et-2-oxo-1-Piz) | 2-F | H | H |
| 5-248 | Ac | H | CH₂CH₂-(4-CH₂CH₂CO₂Et-2-oxo-1-Piz) | 2-F | H | H |
| 5-249 | H | H | CH₂CH₂-(4-CH₂CH₂CH₂CO₂H-2-oxo-1-Piz) | 2-F | H | H |
| 5-250 | Ac | H | CH₂CH₂-(4-CH₂CH₂CH₂CO₂H-2-oxo-1-Piz) | 2-F | H | H |
| 5-251 | H | H | CH₂CH₂-(4-CH₂CH₂CH₂CO₂Me-2-oxo-1-Piz) | 2-F | H | H |
| 5-252 | Ac | H | CH₂CH₂-(4-CH₂CH₂CH₂CO₂Me-2-oxo-1-Piz) | 2-F | H | H |
| 5-253 | H | H | CH₂CH₂-(4-CH₂CH₂CH₂CO₂Et-2-oxo-1-Piz) | 2-F | H | H |
| 5-254 | Ac | H | CH₂CH₂-(4-CH₂CH₂CH₂CO₂Et-2-oxo-1-Piz) | 2-F | H | H |
| 5-255 | H | H | CH₂CH₂-(3-oxo-1-Piz) | 2-F | H | H |
| 5-256 | Ac | H | CH₂CH₂-(3-oxo-1-Piz) | 2-F | H | H |
| 5-257 | H | H | CH₂CH₂-(4-Ac-3-oxo-1-Piz) | 2-F | H | H |
| 5-258 | Ac | H | CH₂CH₂-(4-Ac-3-oxo-1-Piz) | 2-F | H | H |
| 5-259 | H | H | CH₂CH₂-(4-Ac-3-oxo-1-Piz) | 2-F | 4-F | H |
| 5-260 | Ac | H | CH₂CH₂-(4-Ac-3-oxo-1-Piz) | 2-F | 4-F | H |
| 5-261 | H | H | CH₂CH₂-(4-CO₂Me-3-oxo-1-Piz) | 2-F | H | H |
| 5-262 | Ac | H | CH₂CH₂-(4-CO₂Me-3-oxo-1-Piz) | 2-F | H | H |
| 5-263 | H | H | CH₂CH₂-(4-CO₂Et-3-oxo-1-Piz) | 2-F | H | H |
| 5-264 | Ac | H | CH₂CH₂-(4-CO₂Et-3-oxo-1-Piz) | 2-F | H | H |
| 5-265 | H | H | CH₂CH₂-(4-CH₂CO₂H-3-oxo-1-Piz) | 2-F | H | H |
| 5-266 | Ac | H | CH₂CH₂-(4-CH₂CO₂H-3-oxo-1-Piz) | 2-F | H | H |
| 5-267 | H | H | CH₂CH₂-(4-CH2CO₂Me-3-oxo-1-Piz) | 2-F | H | H |
| 5-268 | Ac | H | CH₂CH₂-(4-CH₂CO₂Me-3-oxo-1-Piz) | 2-F | H | H |
| 5-269 | H | H | CH₂CH₂-(4-CH₂CO₂Et-3-oxo-1-Piz) | 2-F | H | H |
| 5-270 | Ac | H | CH₂CH₂-(4-CH₂CO₂Et-3-oxo-1-Piz) | 2-F | H | H |
| 5-271 | H | H | CH₂CH₂-(4-CH₂CH₂CO₂H-3-oxo-1-Piz) | 2-F | H | H |
| 5-272 | Ac | H | CH₂CH₂-(4-CH₂CH₂CO₂H-3-oxo-1-Piz) | 2-F | H | H |
| 5-273 | H | H | CH₂CH₂-(4-CH₂CH₂CO₂Me-3-oxo-1-Piz) | 2-F | H | H |
| 5-274 | Ac | H | CH₂CH₂-(4-CH₂CH₂CO₂Me-3-oxo-1-Piz) | 2-F | H | H |
| 5-275 | H | H | CH₂CH₂-(4-CH₂CH₂CO₂Et-3-oxo-1-Piz) | 2-F | H | H |
| 5-276 | Ac | H | CH₂CH₂-(4-CH₂CH₂CO₂Et-3-oxo-1-Piz) | 2-F | H | H |
| 5-277 | H | H | CH₂CH₂-(4-CH₂CH₂CH₂CO₂H-3-oxo-1-Piz) | 2-F | H | H |
| 5-278 | Ac | H | CH₂CH₂-(4-CH₂CH₂CH₂CO₂H-3-oxo-1-Piz) | 2-F | H | H |
| 5-279 | H | H | CH₂CH₂-(4-CH₂CH₂CH₂CO₂Me-3-oxo-1-Piz) | 2-F | H | H |
| 5-280 | Ac | H | CH₂CH₂-(4-CH₂CH₂CH₂CO₂Me-3-oxo-1-Piz) | 2-F | H | H |
| 5-281 | H | H | CH₂CH₂-(4-CH₂CH₂CH₂CO₂Et-3-oxo-1-Piz) | 2-F | H | H |
| 5-282 | Ac | H | CH₂CH₂-(4-CH₂CH₂CH₂CO₂Et-3-oxo-1-Piz) | 2-F | H | H |
| 5-283 | H | H | CH₂CH₂-(2,5-dioxo-1-Piz) | 2-F | H | H |
| 5-284 | Ac | H | CH₂CH₂(2,5-dioxo-1-Piz) | 2-F | H | H |
| 5-285 | H | H | CH₂CH₂-(4-Ac-2,5-dioxo-1-Piz) | 2-F | H | H |
| 5-286 | Ac | H | CH₂CH₂-(4-Ac-2,5-dioxo-1-Piz) | 2-F | H | H |
| 5-287 | H | H | CH₂CH₂-(4-Ac-2,5-dioxo-1-Piz) | 2-F | 4-F | H |
| 5-288 | Ac | H | CH₂CH₂-(4-Ac-2,5-dioxo-1-Piz) | 2-F | 4-F | H |
| 5-289 | H | H | CH₂CH₂-(4-CO₂Me-2,5-dioxo-1-Piz) | 2-F | H | H |
| 5-290 | Ac | H | CH₂CH₂-(4-CO₂Me-2,5-dioxo-1-Piz) | 2-F | H | H |
| 5-291 | H | H | CH₂CH₂-(4-CO₂Et-2,5-dioxo-1-Piz) | 2-F | H | H |
| 5-292 | Ac | H | CH₂CH₂-(4-CO₂Et-2,5-dioxo-1-Piz) | 2-F | H | H |
| 5-293 | H | H | CH₂CH₂-(4-CH₂CO₂H-2,5-dioxo-1-Piz) | 2-F | H | H |
| 5-294 | Ac | H | CH₂CH₂-(4-CH₂CO₂H-2,5-dioxo-1-Piz) | 2-F | H | H |
| 5-295 | H | H | CH₂CH₂-(4-CH₂CO₂Me-2,5-dioxo-1-Piz) | 2-F | H | H |
| 5-296 | Ac | H | CH₂CH₂-(4-CH₂CO₂Me-2,5-dioxo-1-Piz) | 2-F | H | H |
| 5-297 | H | H | CH₂CH₂-(4-CH₂CO₂Et-2,5-dioxo-1-Piz) | 2-F | H | H |
| 5-298 | Ac | H | CH₂CH₂-(4-CH₂CO₂Et-2,5-dioxo-1-Piz) | 2-F | H | H |
| 5-299 | H | H | CH₂CH₂-(4-CH₂CH₂CO₂H-2,5-dioxo-1-Piz) | 2-F | H | H |
| 5-300 | Ac | H | CH₂CH₂-(4-CH₂CH₂CO₂H-2,5-dioxo-1-Piz) | 2-F | H | H |
| 5-301 | H | H | CH₂CH₂-(4-CH₂CH₂CO₂Me-2,5-dioxo-1-Piz) | 2-F | H | H |
| 5-302 | Ac | H | CH₂CH₂-(4-CH₂CH₂CO₂Me-2,5-dioxo-1-Piz) | 2-F | H | H |
| 5-303 | H | H | CH₂CH₂-(4-CH₂CH₂CO₂Et-2,5-dioxo-1-Piz) | 2-F | H | H |
| 5-304 | Ac | H | CH₂CH₂-(4-CH₂CH₂CO₂Et-2,5-dioxo-1-Piz) | 2-F | H | H |
| 5-305 | H | H | CH₂CH₂-(4-CH₂CH₂CH₂CO₂H-2,5-dioxo-1-Piz) | 2-F | H | H |
| 5-306 | Ac | H | CH₂CH₂-(4-CH₂CH₂CH₂CO₂H-2,5-dioxo-1-Piz) | 2-F | H | H |
| 5-307 | H | H | CH₂CH₂-(4-CH₂CH₂CH₂CO₂Me-2,5-dioxo-1-Piz) | 2-F | H | H |
| 5-308 | Ac | H | CH₂CH₂-(4-CH₂CH₂CH₂CO₂Me-2,5-dioxo-1-Piz) | 2-F | H | H |
| 5-309 | H | H | CH₂CH₂-(4-CH₂CH₂CH₂CO₂Et-2,5-dioxo-1-Piz) | 2-F | H | H |
| 5-310 | Ac | H | CH₂CH₂-(4-CH₂CH₂CH₂CO₂Et-2,5-dioxo-1-Piz) | 2-F | H | H |
| 5-311 | H | H | CH₂CH₂-1-Pyrd | 2-F | H | H |
| 5-312 | Ac | H | CH₂CH₂-1-Pyrd | 2-F | H | H |
| 5-313 | H | H | CH₂CH₂-(2-CO₂H-1-Pyrd) | 2-F | H | H |
| 5-314 | Ac | H | CH₂CH₂-(2-CO₂H-1-Pyrd) | 2-F | H | H |
| 5-315 | H | H | CH₂CH₂-(2-CO₂Me-1-Pyrd) | 2-F | H | H |
| 5-316 | Ac | H | CH₂CH₂-(2-CO₂Me-1-Pyrd) | 2-F | H | H |
| 5-317 | H | H | CH₂CH₂-(2-CO₂Et-1-Pyrd) | 2-F | H | H |
| 5-318 | Ac | H | CH₂CH₂-(2-CO₂Et-1-Pyrd) | 2-F | H | H |
| 5-319 | H | H | CH₂CH₂-(2-CH₂CO₂H-1-Pyrd) | 2-F | H | H |
| 5-320 | Ac | H | CH₂CH₂-(2-CH₂CO₂H-1-Pyrd) | 2-F | H | H |
| 5-321 | H | H | CH₂CH₂-(2-CH₂CO₂Me-1-Pyrd) | 2-F | H | H |
| 5-322 | Ac | H | CH₂CH₂-(2-CH₂CO₂Me-1-Pyrd) | 2-F | H | H |
| 5-323 | H | H | CH₂CH₂-(2-CH₂CO₂Et-1-Pyrd) | 2-F | H | H |
| 5-324 | Ac | H | CH₂CH₂-(2-CH₂CO₂Et-1-Pyrd) | 2-F | H | H |
| 5-325 | H | H | CH₂CH₂-(2-CH₂CH₂CO₂H-1-Pyrd) | 2-F | H | H |
| 5-326 | Ac | H | CH₂CH₂-(2-CH₂CH₂CO₂H-1-Pyrd) | 2-F | H | H |
| 5-327 | H | H | CH₂CH₂-(2-CH₂CH₂CO₂Me-1-Pyrd) | 2-F | H | H |
| 5-328 | Ac | H | CH₂CH₂-(2-CH₂CH₂CO₂Me-1-Pyrd) | 2-F | H | H |
| 5-329 | H | H | CH₂CH₂-(2-CH₂CH₂CO₂Et-1-Pyrd) | 2-F | H | H |
| 5-330 | Ac | H | CH₂CH₂-(2-CH₂CH₂CO₂Et-1-Pyrd) | 2-F | H | H |
| 5-331 | H | H | CH₂CH₂-(2-CH₂CH₂CH₂CO₂H-1-Pyrd) | 2-F | H | H |
| 5-332 | Ac | H | CH₂CH₂-(2-CH₂CH₂CH₂CO₂H-1-Pyrd) | 2-F | H | H |
| 5-333 | H | H | CH₂CH₂-(2-CH₂CH₂CH₂CO₂Me-1-Pyrd) | 2-F | H | H |
| 5-334 | Ac | H | CH₂CH₂-(2-CH₂CH₂CH₂CO₂Me-1-Pyrd) | 2-F | H | H |
| 5-335 | H | H | CH₂CH₂-(2-CH₂CH₂CH₂CO₂Et-1-Pyrd) | 2-F | H | H |
| 5-336 | Ac | H | CH₂CH₂(2-CH₂CH₂CH₂CO₂Et-1-Pyrd) | 2-F | H | H |
| 5-337 | H | H | CH₂-(3-CO₂H-1-Pip) | 2-F | H | H |
| 5-338 | Ac | H | CH₂-(3-CO₂H-1-Pip) | 2-F | H | H |
| 5-339 | H | H | CH₂-(3-CO₂Me-1-Pip) | 2-F | H | H |
| 5-340 | Ac | H | CH₂-(3-CO₂Me-1-Pip) | 2-F | H | H |
| 5-341 | H | H | CH₂-(3-CO₂Et-1-Pip) | 2-F | H | H |
| 5-342 | Ac | H | CH₂-(3-CO₂Et-1-Pip) | 2-F | H | H |
| 5-343 | H | H | CH₂-(3-CH₂CO₂H-1-Pip) | 2-F | H | H |
| 5-344 | Ac | H | CH₂-(3-CH₂CO₂H-1-Pip) | 2-F | H | H |
| 5-345 | H | H | CH₂-(3-CH₂CO₂Me-1-Pip) | 2-F | H | H |
| 5-346 | Ac | H | CH₂-(3-CH₂CO₂Me-1-Pip) | 2-F | H | H |
| 5-347 | H | H | CH₂-(3-CH₂CO₂Et-1-Pip) | 2-F | H | H |
| 5-348 | Ac | H | CH₂-(3-CH₂CO₂Et-1-Pip) | 2-F | H | H |
| 5-349 | H | H | CH₂-(3-CH₂CH₂CO₂H-1-Pip) | 2-F | H | H |
| 5-350 | Ac | H | CH₂-(3-CH₂CH₂CO₂H-1-Pip) | 2-F | H | H |
| 5-351 | H | H | CH₂-(3-CH₂CH₂CO₂Me-1-Pip) | 2-F | H | H |
| 5-352 | Ac | H | CH₂-(3-CH₂CH₂CO₂Me-1-Pip) | 2-F | H | H |
| 5-353 | H | H | CH₂-(3-CH₂CH₂CO₂Et-1-Pip) | 2-F | H | H |
| 5-354 | Ac | H | CH₂-(3-CH₂CH₂CO₂Et-1-Pip) | 2-F | H | H |
| 5-355 | H | H | CH₂-(3-CH₂CH₂CH₂CO₂H-1-Pip) | 2-F | H | H |
| 5-356 | Ac | H | CH₂-(3-CH₂CH₂CH₂CO₂H-1-Pip) | 2-F | H | H |
| 5-357 | H | H | CH₂-(3-CH₂CH₂CH₂CO₂Me-1-Pip) | 2-F | H | H |
| 5-358 | Ac | H | CH₂-(3-CH₂CH₂CH₂CO₂Me-1-Pip) | 2-F | H | H |
| 5-359 | H | H | CH₂-(3-CH₂CH₂CH₂CO₂Et-1-Pip) | 2-F | H | H |
| 5-360 | Ac | H | CH₂-(3-CH₂CH₂CH₂CO₂Et-1-Pip) | 2-F | H | H |
| 5-361 | H | H | CH₂CH₂-(3-CO₂H-1-Pip) | 2-F | H | H |
| 5-362 | Ac | H | CH₂CH₂-(3-CO₂H-1-Pip) | 2-F | H | H |
| 5-363 | H | H | CH₂CH₂-(3-CO₂Me-1-Pip) | 2-F | H | H |
| 5-364 | Ac | H | CH₂CH₂-(3-CO₂Me-1-Pip) | 2-F | H | H |
| 5-365 | H | H | CH₂CH₂-(3-CO₂Et-1-Pip) | 2-F | H | H |
| 5-366 | Ac | H | CH₂CH₂(3-CO₂Et-1-Pip) | 2-F | H | H |
| 5-367 | H | H | CH₂CH₂-(3-CH₂CO₂H-1-Pip) | 2-F | H | H |
| 5-368 | Ac | H | CH₂CH₂-(3-CH₂CO₂H-1-Pip) | 2-F | H | H |
| 5-369 | H | H | CH₂CH₂-(3-CH₂CO₂Me-1-Pip) | 2-F | H | H |
| 5-370 | Ac | H | CH₂CH₂-(3-CH₂CO₂Me-1-Pip) | 2-F | H | H |
| 5-371 | H | H | CH₂CH₂-(3-CH₂CO₂Et-1-Pip) | 2-F | H | H |
| 5-372 | Ac | H | CH₂CH₂-(3-CH₂CO₂Et-1-Pip) | 2-F | H | H |
| 5-373 | H | H | CH₂CH₂-(3-CH₂CH₂CO₂H-1-Pip) | 2-F | H | H |
| 5-374 | Ac | H | CH₂CH₂-(3-CH₂CH₂CO₂H-1-Pip) | 2-F | H | H |
| 5-375 | H | H | CH₂CH₂-(3-CH₂CH₂CO₂Me-1-Pip) | 2-F | H | H |
| 5-376 | Ac | H | CH₂CH₂-(3-CH₂CH₂CO₂Me-1-Pip) | 2-F | H | H |
| 5-377 | H | H | CH₂CH₂-(3-CH₂CH₂CO₂Et-1-Pip) | 2-F | H | H |
| 5-378 | Ac | H | CH₂CH₂-(3-CH₂CH₂CO₂Et-1-Pip) | 2-F | H | H |
| 5-379 | H | H | CH₂CH₂-(3-CH₂CH₂CH₂CO₂H-1-Pip) | 2-F | H | H |
| 5-380 | Ac | H | CH₂CH₂-(3-CH₂CH₂CH₂CO₂H-1-Pip) | 2-F | H | H |
| 5-381 | H | H | CH₂CH₂-(3-CH₂CH₂CH₂CO₂Me-1-Pip) | 2-F | H | H |
| 5-382 | Ac | H | CH₂CH₂-(3-CH₂CH₂CH₂CO₂Me-1-Pip) | 2-F | H | H |
| 5-383 | H | H | CH₂CH₂-(3-CH₂CH₂CH₂CO₂Et-1-Pip) | 2-F | H | H |
| 5-384 | Ac | H | CH₂CH₂-(3-CH₂CH₂CH₂CO₂Et-1-Pip) | 2-F | H | H |
| 5-385 | H | H | CH₂-(4-OH-2-CO₂H-1-Pyrd) | 2-F | H | H |
| 5-386 | Ac | H | CH₂-(4-OH-2-CO₂H-1-Pyrd) | 2-F | H | H |
| 5-387 | H | H | CH₂-(4-OH-2-CO₂Me-1-Pyrd) | 2-F | H | H |
| 5-388 | Ac | H | CH₂-(4-OH-2-CO₂Me-1-Pyrd) | 2-F | H | H |
| 5-389 | H | H | CH₂-(4-OH-2-CO₂Et-1-Pyrd) | 2-F | H | H |
| 5-390 | Ac | H | CH₂-(4-OH-2-CO₂Et-1-Pyrd) | 2-F | H | H |

**(Table 6)**

| | | | | | | |
|---|---|---|---|---|---|---|
| 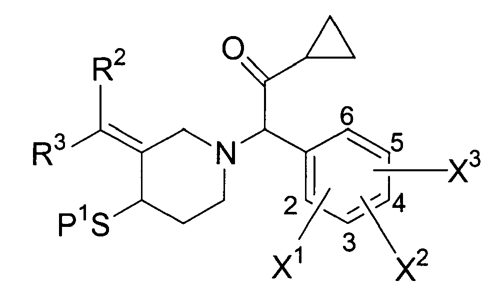 | | | | | | |

| Compound No. | P¹ | R² | R³ | X¹ | X² | X³ |
|---|---|---|---|---|---|---|
| 6-1 | H | H | CH₂-1-Pip | 2-F | H | H |
| 6-2 | Ac | H | CH₂-1-Pip | 2-F | H | H |
| 6-3 | H | H | CH₂-(4-CO₂H-1-Pip) | 2-F | H | H |
| 6-4 | Ac | H | CH₂-(4-CO₂H-1-Pip) | 2-F | H | H |
| 6-5 | H | H | CH₂-(4-CO₂Me-1-Pip) | 2-F | H | H |
| 6-6 | Ac | H | CH₂-(4-CO₂Me-1-Pip) | 2-F | H | H |
| 6-7 | H | H | CH₂-(4-CO₂Et-1-Pip) | 2-F | H | H |
| 6-8 | Ac | H | CH₂-(4-CO₂Et-1-Pip) | 2-F | H | H |
| 6-9 | H | H | CH₂-(4-CH₂CO₂H-1-Pip) | 2-F | H | H |
| 6-10 | Ac | H | CH₂-(4-CH₂CO₂H-1-Pip) | 2-F | H | H |
| 6-11 | H | H | CH₂-(4-CH₂CO₂Me-1-Pip) | 2-F | H | H |
| 6-12 | Ac | H | CH₂-(4-CH₂CO₂Me-1-Pip) | 2-F | H | H |
| 6-13 | H | H | CH₂-(4-CH₂CO₂Et-1-Pip) | 2-F | H | H |
| 6-14 | Ac | H | CH₂-(4-CH₂CO₂Et-1-Pip) | 2-F | H | H |
| 6-15 | H | H | CH₂-(4-CH₂CH₂CO₂H-1-Pip) | 2-F | H | H |
| 6-16 | Ac | H | CH₂-(4-CH₂CH₂CO₂H-1-Pip) | 2-F | H | H |
| 6-17 | H | H | CH₂-(4-CH₂CH₂CO₂Me-1-Pip) | 2-F | H | H |
| 6-18 | Ac | H | CH₂-(4-CH₂CH₂CO₂Me-1-Pip) | 2-F | H | H |
| 6-19 | H | H | CH₂-(4-CH₂CH₂CO₂Et-1-Pip) | 2-F | H | H |
| 6-20 | Ac | H | CH₂-(4-CH₂CH₂CO₂Et-1-Pip) | 2-F | H | H |
| 6-21 | H | H | CH₂-(4-CH₂CH₂CH₂CO₂H-1-Pip) | 2-F | H | H |
| 6-22 | Ac | H | CH₂-(4-CH₂CH₂CH₂CO₂H-1-Pip) | 2-F | H | H |
| 6-23 | H | H | CH₂-(4-CH₂CH₂CH₂CO₂Me-1-Pip) | 2-F | H | H |
| 6-24 | Ac | H | CH₂-(4-CH₂CH₂CH₂CO₂Me-1-Pip) | 2-F | H | H |
| 6-25 | H | H | CH₂-(4-CH₂CH₂CH₂CO₂Et-1-Pip) | 2-F | H | H |
| 6-26 | Ac | H | CH₂-(4-CH₂CH₂CH₂CO₂Et-1-Pip) | 2-F | H | H |
| 6-27 | H | H | CH₂-Mor | 2-F | H | H |
| 6-28 | Ac | H | CH₂-Mor | 2-F | H | H |
| 6-29 | H | H | CH₂-Thim | 2-F | H | H |
| 6-30 | Ac | H | CH₂-Thim | 2-F | H | H |
| 6-31 | H | H | CH₂-1-Piz | 2-F | H | H |
| 6-32 | Ac | H | CH₂-1-Piz | 2-F | H | H |
| 6-33 | H | H | CH₂-(4-Ac-1-Piz) | 2-F | H | H |
| 6-34 | Ac | H | CH₂-(4-Ac-1-Piz) | 2-F | H | H |
| 6-35 | H | H | CH₂-(4-Ac-l-Piz) | 2-F | 4-F | H |
| 6-36 | Ac | H | CH₂-(4-Ac-1-Piz) | 2-F | 4-F | H |
| 6-37 | H | H | CH₂-(4-CO₂Me-1-Piz) | 2-F | H | H |
| 6-38 | Ac | H | CH₂-(4-CO₂Me-1-Piz) | 2-F | H | H |
| 6-39 | H | H | CH₂-(4-CO₂Et-1-Piz) | 2-F | H | H |
| 6-40 | Ac | H | CH₂-(4-CO₂Et-1-Piz) | 2-F | H | H |
| 6-41 | H | H | CH₂-(4-CH₂CO₂H-1-Piz) | 2-F | H | H |
| 6-42 | Ac | H | CH₂-(4-CH₂CO₂H-1-Piz) | 2-F | H | H |
| 6-43 | H | H | CH₂-(4-CH₂CO₂Me-1-Piz) | 2-F | H | H |
| 6-44 | Ac | H | CH₂-(4-CH₂CO₂Me-1-Piz) | 2-F | H | H |
| 6-45 | H | H | CH₂-(4-CH₂CO₂Et-1-Piz) | 2-F | H | H |
| 6-46 | Ac | H | CH₂-(4-CH₂CO₂Et-1-Piz) | 2-F | H | H |
| 6-47 | H | H | CH₂-(4-CH₂CH₂CO₂H-1-Piz) | 2-F | H | H |
| 6-48 | Ac | H | CH₂-(4-CH₂CH₂CO₂H-1-Piz) | 2-F | H | H |
| 6-49 | H | H | CH₂-(4-CH₂CH₂CO₂Me-1-Piz) | 2-F | H | H |
| 6-50 | Ac | H | CH₂-(4-CH₂CH₂CO₂Me-1-Piz) | 2-F | H | H |
| 6-51 | H | H | CH₂-(4-CH₂CH₂CO₂Et-1-Piz) | 2-F | H | H |
| 6-52 | Ac | H | CH₂-(4-CH₂CH₂CO₂Et-1-Piz) | 2-F | H | H |
| 6-53 | H | H | CH₂-(4-CH₂CH₂CH₂CO₂H-1-Piz) | 2-F | H | H |
| 6-54 | Ac | H | CH₂-(4-CH₂CH₂CH₂CO₂H-1-Piz) | 2-F | H | H |
| 6-55 | H | H | CH₂-(4-CH₂CH₂CH₂CO₂Me-1-Piz) | 2-F | H | H |
| 6-56 | Ac | H | CH₂-(4-CH₂CH₂CH₂CO₂Me-1-Piz) | 2-F | H | H |
| 6-57 | H | H | CH₂-(4-CH₂CH₂CH₂CO₂Et-1-Piz) | 2-F | H | H |
| 6-58 | Ac | H | CH₂-(4-CH₂CH₂CH₂CO₂Et-1-Piz) | 2-F | H | H |
| 6-59 | H | H | CH₂-(2-oxo-1-Piz) | 2-F | H | H |
| 6-60 | Ac | H | CH₂-(2-oxo-1-Piz) | 2-F | H | H |
| 6-61 | H | H | CH₂-(4-Ac-2-oxo-1-Piz) | 2-F | H | H |
| 6-62 | Ac | H | CH₂-(4-Ac-2-oxo-1-Piz) | 2-F | H | H |
| 6-63 | H | H | CH₂-(4-Ac-2-oxo-1-Piz) | 2-F | 4-F | H |
| 6-64 | Ac | H | CH₂-(4-Ac-2-oxo-1-Piz) | 2-F | 4-F | H |
| 6-65 | H | H | CH₂-(4-CO₂Me-2-oxo-1-Piz) | 2-F | H | H |
| 6-66 | Ac | H | CH₂-(4-CO₂Me-2-oxo-1-Piz) | 2-F | H | H |
| 6-67 | H | H | CH₂-(4-CO₂Et-2-oxo-1-Piz) | 2-F | H | H |
| 6-68 | Ac | H | CH₂-(4-CO₂Et-2-oxo-1-Piz) | 2-F | H | H |
| 6-69 | H | H | CH₂-(4-CH₂CO₂H-2-oxo-1-Piz) | 2-F | H | H |
| 6-70 | Ac | H | CH₂-(4-CH₂CO₂H-2-oxo-1-Piz) | 2-F | H | H |
| 6-71 | H | H | CH₂-(4-CH₂CO₂Me-2-oxo-1-Piz) | 2-F | H | H |
| 6-72 | Ac | H | CH₂-(4-CH₂CO₂Me-2-oxo-1-Piz) | 2-F | H | H |
| 6-73 | H | H | CH₂-(4-CH₂CO₂Et-2-oxo-1-Piz) | 2-F | H | H |
| 6-74 | Ac | H | CH₂-(4-CH₂CO₂Et-2-oxo-1-Piz) | 2-F | H | H |
| 6-75 | H | H | CH₂-(4-CH₂CH₂CO₂H-2-oxo-1-Piz) | 2-F | H | H |
| 6-76 | Ac | H | CH₂-(4-CH₂CH₂CO₂H-2-oxo-1-Piz) | 2-F | H | H |
| 6-77 | H | H | CH₂-(4-CH₂CH₂CO₂Me-2-oxo-1-Piz) | 2-F | H | H |
| 6-78 | Ac | H | CH₂-(4-CH₂CH₂CO₂Me-2-oxo-1-Piz) | 2-F | H | H |
| 6-79 | H | H | CH₂-(4-CH₂CH₂CO₂Et-2-oxo-1-Piz) | 2-F | H | H |
| 6-80 | Ac | H | CH₂-(4-CH₂CH₂CO₂Et-2-oxo-1-Piz) | 2-F | H | H |
| 6-81 | H | H | CH₂-(4-CH₂CH₂CH₂CO₂H-2-oxo-1-Piz) | 2-F | H | H |
| 6-82 | Ac | H | CH₂-(4-CH₂CH₂CH₂CO₂H-2-oxo-1-Piz) | 2-F | H | H |
| 6-83 | H | H | CH₂-(4-CH₂CH₂CH₂CO₂Me-2-oxo-1-Piz) | 2-F | H | H |
| 6-84 | Ac | H | CH₂-(4-CH₂CH₂CH₂CO₂Me-2-oxo-1-Piz) | 2-F | H | H |
| 6-85 | H | H | CH₂-(4-CH₂CH₂CH₂CO₂Et-2-oxo-1-Piz) | 2-F | H | H |
| 6-86 | Ac | H | CH₂-(4-CH₂CH₂CH₂CO₂Et-2-oxo-1-Piz) | 2-F | H | H |
| 6-87 | H | H | CH₂-(3-oxo-1-Piz) | 2-F | H | H |
| 6-88 | Ac | H | CH₂-(3-oxo-1-Piz) | 2-F | H | H |
| 6-89 | H | H | CH₂-(4-Ac-3-oxo-1-Piz) | 2-F | H | H |
| 6-90 | Ac | H | CH₂-(4-Ac-3-oxo-1-Piz) | 2-F | H | H |
| 6-91 | H | H | CH₂-(4-Ac-3-oxo-1-Piz) | 2-F | 4-F | H |
| 6-92 | Ac | H | CH₂-(4-Ac-3-oxo-1-Piz) | 2-F | 4-F | H |
| 6-93 | H | H | CH₂-(4-CO₂Me-3-oxo-1-Piz) | 2-F | H | H |
| 6-94 | Ac | H | CH₂-(4-CO₂Me-3-oxo-1-Piz) | 2-F | H | H |
| 6-95 | H | H | CH₂-(4-CO₂Et-3-oxo-1-Piz) | 2-F | H | H |
| 6-96 | Ac | H | CH₂-(4-CO₂Et-3-oxo-1-Piz) | 2-F | H | H |
| 6-97 | H | H | CH₂-(4-CH₂CO₂H-3-oxo-1-Piz) | 2-F | H | H |
| 6-98 | Ac | H | CH₂-(4-CH₂CO₂H-3-oxo-1-Piz) | 2-F | H | H |
| 6-99 | H | H | CH₂-(4-CH₂CO₂Me-3-oxo-1-Piz) | 2-F | H | H |
| 6-100 | Ac | H | CH₂-(4-CH₂CO₂Me-3-oxo-1-Piz) | 2-F | H | H |
| 6-101 | H | H | CH₂-(4-CH₂CO₂Et-3-oxo-1-Piz) | 2-F | H | H |
| 6-102 | Ac | H | CH₂-(4-CH₂CO₂Et-3-oxo-1-Piz) | 2-F | H | H |
| 6-103 | H | H | CH₂-(4-CH₂CH₂CO₂H-3-oxo-1-Piz) | 2-F | H | H |
| 6-104 | Ac | H | CH₂-(4-CH₂CH₂CO₂H-3-oxo-1-Piz) | 2-F | H | H |
| 6-105 | H | H | CH₂-(4-CH₂CH₂CO₂Me-3-oxo-1-Piz) | 2-F | H | H |
| 6-106 | Ac | H | CH₂-(4-CH₂CH₂CO₂Me-3-oxo-1-Piz) | 2-F | H | H |
| 6-107 | H | H | CH₂-(4-CH₂CH₂CO₂Et-3-oxo-1-Piz) | 2-F | H | H |
| 6-108 | Ac | H | CH₂-(4-CH₂CH₂CO₂Et-3-oxo-1-Piz) | 2-F | H | H |
| 6-109 | H | H | CH₂-(4-CH₂CH₂CH₂CO₂H-3-oxo-1-Piz) | 2-F | H | H |
| 6-110 | Ac | H | CH₂-(4-CH₂CH₂CH₂CO₂H-3-oxo-1-Piz) | 2-F | H | H |
| 6-111 | H | H | CH₂-(4-CH₂CH₂CH₂CO₂Me-3-oxo-1-Piz) | 2-F | H | H |
| 6-112 | Ac | H | CH₂-(4-CH₂CH₂CH₂CO₂Me-3-oxo-1-Piz) | 2-F | H | H |
| 6-113 | H | H | CH₂-(4-CH₂CH₂CH₂CO₂Et-3-oxo-1-Piz) | 2-F | H | H |
| 6-114 | Ac | H | CH₂-(4-CH₂CH₂CH₂CO₂Et-3-oxo-1-Piz) | 2-F | H | H |
| 6-115 | H | H | CH₂-(2,5-dioxo-1-Piz) | 2-F | H | H |
| 6-116 | Ac | H | CH₂-(2,5-dioxo-1-Piz) | 2-F | H | H |
| 6-117 | H | H | CH₂-(4-Ac-2,5-dioxo-1-Piz) | 2-F | H | H |
| 6-118 | Ac | H | CH₂-(4-Ac-2,5-dioxo-1-Piz) | 2-F | H | H |
| 6-119 | H | H | CH₂-(4-Ac-2,5-dioxo-1-Piz) | 2-F | 4-F | H |
| 6-120 | Ac | H | CH₂-(4-Ac-2,5-dioxo-1-Piz) | 2-F | 4-F | H |
| 6-121 | H | H | CH₂-(4-CO₂Me-2,5-dioxo-1-Piz) | 2-F | H | H |
| 6-122 | Ac | H | CH₂-(4-CO₂Me-2,5-dioxo-1-Piz) | 2-F | H | H |
| 6-123 | H | H | CH₂-(4-CO₂Et-2,5-dioxo-1-Piz) | 2-F | H | H |
| 6-124 | Ac | H | CH₂-(4-CO₂Et-2,5-dioxo-1-Piz) | 2-F | H | H |
| 6-125 | H | H | CH₂-(4-CH₂CO₂H-2,5-dioxo-1-Piz) | 2-F | H | H |
| 6-126 | Ac | H | CH₂-(4-CH₂CO₂H-2,5-dioxo-1-Piz) | 2-F | H | H |
| 6-127 | H | H | CH₂-(4-CH₂CO₂Me-2,5-dioxo-1-Piz) | 2-F | H | H |
| 6-128 | Ac | H | CH₂-(4-CH₂CO₂Me-2,5-dioxo-1-Piz | 2-F | H | H |
| 6-130 | Ac | H | CH₂-(4-CH₂CO₂Et-2,5-dioxo-1-Piz) | 2-F | H | H |
| 6-131 | H | H | CH₂-(4-CH₂CH₂CO₂H-2,5-dioxo-1-Piz) | 2-F | H | H |
| 6-132 | Ac | H | CH₂-(4-CH₂CH₂CO₂H-2,5-dioxo-1-Piz) | 2-F | H | H |
| 6-133 | H | H | CH₂-(4-CH₂CH₂CO₂Me-2,5-dioxo-1-Piz) | 2-F | H | H |
| 6-134 | Ac | H | CH₂-(4-CH₂CH₂CO₂Me-2,5-dioxo-1-Piz) | 2-F | H | H |
| 6-135 | H | H | CH₂-(4-CH₂CH₂CO₂Et-2,5-dioxo-1-Piz) | 2-F | H | H |
| 6-136 | Ac | H | CH₂-(4-CH₂CH₂CO₂Et-2,5-dioxo-1-Piz) | 2-F | H | H |
| 6-137 | H | H | CH₂-(4-CH₂CH₂CH₂CO₂H-2,5-dioxo-1-Piz) | 2-F | H | H |
| 6-138 | Ac | H | CH₂-(4-CH₂CH₂CH₂CO₂H-2,5-dioxo-1-Piz) | 2-F | H | H |
| 6-139 | H | H | CH₂-(4-CH₂CH₂CH₂CO₂Me-2,5-dioxo-1-Piz) | 2-F | H | H |
| 5-140 | Ac | H | CH₂-(4-CH₂CH₂CH₂CO₂Me-2,5-dioxo-1-Piz) | 2-F | H | H |
| 6-141 | H | H | CH₂-(4-CH₂CH₂CH₂CO₂Et-2,5-dioxo-1-Piz) | 2-F | H | H |
| 6-142 | Ac | H | CH₂-(4-CH₂CH₂CH₂CO₂Et-2,5-dioxo-1-Piz) | 2-F | H | H |
| 6-143 | H | H | CH₂-1-Pyrd | 2-F | H | H |
| 6-144 | Ac | H | CH₂-1-Pyrd | 2-F | H | H |
| 6-145 | H | H | CH₂-(2-CO₂H-1-Pyrd) | 2-F | H | H |
| 6-146 | Ac | H | CH₂-(2-CO₂H-1-Pyrd) | 2-F | H | H |
| 6-147 | H | H | CH₂-(2-CO₂Me-1-Pyrd) | 2-F | H | H |
| 6-148 | Ac | H | CH₂-(2-CO₂Me-1-Pyrd) | 2-F | H | H |
| 6-149 | H | H | CH₂-(2-CO₂Et-1-Pyrd) | 2-F | H | H |
| 6-150 | Ac | H | CH₂-(2-CO₂Et-1-Pyrd) | 2-F | H | H |
| 6-151 | H | H | CH₂-(2-CH₂CO₂H-1-Pyrd) | 2-F | H | H |
| 6-152 | Ac | H | CH₂-(2-CH₂CO₂H-1-Pyrd) | 2-F | H | H |
| 6-153 | H | H | CH₂-(2-CH₂CO₂Me-1-Pyrd) | 2-F | H | H |
| 6-154 | Ac | H | CH₂-(2-CH₂CO₂Me-1-Pyrd) | 2-F | H | H |
| 6-155 | H | H | CH₂-(2-CH₂CO₂Et-1-Pyrd) | 2-F | H | H |
| 6-156 | Ac | H | CH₂-(2-CH₂CO₂Et-1-Pyrd) | 2-F | H | H |
| 6-157 | H | H | CH₂-(2-CH₂CH₂CO₂H-1-Pyrd) | 2-F | H | H |
| 6-158 | Ac | H | CH₂-(2-CH₂CH₂CO₂H-1-Pyrd) | 2-F | H | H |
| 6-159 | H | H | CH₂-(2-CH₂CH₂CO₂Me-1-Pyrd) | 2-F | H | H |
| 6-160 | Ac | H | CH₂-(2-CH₂CH₂CO₂Me-1-Pyrd) | 2-F | H | H |
| 6-161 | H | H | CH₂-(2-CH₂CH₂CO₂Et-1-Pyrd) | 2-F | H | H |
| 6-162 | Ac | H | CH₂-(2-CH₂CH₂CO₂Et-1-Pyrd) | 2-F | H | H |
| 6-163 | H | H | CH₂-(2-CH₂CH₂CH₂CO₂H-1-Pyrd) | 2-F | H | H |
| 6-164 | Ac | H | CH₂-(2-CH₂CH₂CH₂CO₂H-1-Pyrd) | 2-F | H | H |
| 6-165 | H | H | CH₂-(2-CH₂CH₂CH₂CO₂Me-1-Pyrd) | 2-F | H | H |
| 6-166 | Ac | H | CH₂-(2-CH₂CH₂CH₂CO₂Me-1-Pyrd) | 2-F | H | H |
| 6-167 | H | H | CH₂-(2-CH₂CH₂CH₂CO₂Et-1-Pyrd) | 2-F | H | H |
| 6-168 | Ac | H | CH₂-(2-CH₂CH₂CH₂CO₂Et-1-Pyrd) | 2-F | H | H |
| 6-169 | H | H | CH₂CH₂-1-Pip | 2-F | H | H |
| 6-170 | Ac | H | CH₂CH₂-1-Pip | 2-F | H | H |
| 6-171 | H | H | CH₂CH₂-(4-CO₂H-1-Pip) | 2-F | H | H |
| 6-172 | Ac | H | CH₂CH₂-(4-CO₂H-1-Pip) | 2-F | H | H |
| 6-173 | H | H | CH₂CH₂-(4-CO₂Me-1-Pip) | 2-F | H | H |
| 6-174 | Ac | H | CH₂CH₂-(4-CO₂Me-1-Pip) | 2-F | H | H |
| 6-175 | H | H | CH₂CH₂-(4-CO₂Et-1-Pip) | 2-F | H | H |
| 6-176 | Ac | H | CH₂CH₂-(4-CO₂Et-1-Pip) | 2-F | H | H |
| 6-177 | H | H | CH₂CH₂-(4-CH₂CO₂H-1-Pip) | 2-F | H | H |
| 6-178 | Ac | H | CH₂CH₂-(4-CH₂CO₂H-1-Pip) | 2-F | H | H |
| 6-179 | H | H | CH₂CH₂-(4-CH₂CO₂Me-1-Pip) | 2-F | H | H |
| 6-180 | Ac | H | CH₂CH₂-(4-CH₂CO₂Me-1-Pip) | 2-F | H | H |
| 6-181 | H | H | CH₂CH₂-(4-CH₂CO₂Et-1-Pip) | 2-F | H | H |
| 6-182 | Ac | H | CH₂CH₂-(4-CH₂CO₂Et-1-Pip) | 2-F | H | H |
| 6-183 | H | H | CH₂CH₂-(4-CH₂CH₂CO₂H-1-Pip) | 2-F | H | H |
| 6-184 | Ac | H | CH₂CH₂-(4-CH₂CH₂CO₂H-1-Pip) | 2-F | H | H |
| 6-185 | H | H | CH₂CH₂-(4-CH₂CH₂CO₂Me-1-Pip) | 2-F | H | H |
| 6-186 | Ac | H | CH₂CH₂-(4-CH₂CH₂CO₂Me-1-Pip) | 2-F | H | H |
| 6-187 | H | H | CH₂CH₂-(4-CH₂CH₂CO₂Et-1-Pip) | 2-F | H | H |
| 6-188 | Ac | H | CH₂CH₂-(4-CH₂CH₂CO₂Et-1-Pip) | 2-F | H | H |
| 6-189 | H | H | CH₂CH₂-(4-CH₂CH₂CH₂CO₂H-1-Pip) | 2-F | H | H |
| 6-190 | Ac | H | CH₂CH₂-(4-CH₂CH₂CH₂CO₂H-1-Pip) | 2-F | H | H |
| 6-191 | H | H | CH₂CH₂-(4-CH₂CH₂CH₂CO₂Me-1-Pip) | 2-F | H | H |
| 6-192 | Ac | H | CH₂CH₂-(4-CH₂CH₂CH₂CO₂Me-1-Pip) | 2-F | H | H |
| 6-193 | H | H | CH₂CH₂-(4-CH₂CH₂CH₂CO₂Et-1-Pip) | 2-F | H | H |
| 6-194 | Ac | H | CH₂CH₂-(4-CH₂CH₂CH₂CO₂Et-1-Pip) | 2-F | H | H |
| 6-195 | H | H | CH₂CH₂-Mor | 2-F | H | H |
| 6-196 | Ac | H | CH₂CH₂-Mor | 2-F | H | H |
| 6-197 | H | H | CH₂CH₂-Thim | 2-F | H | H |
| 6-198 | Ac | H | CH₂CH₂-Thim | 2-F | H | H |
| 6-199 | H | H | CH₂CH₂-1-Piz | 2-F | H | H |
| 6-200 | Ac | H | CH₂CH₂-1-Piz | 2-F | H | H |
| 6-201 | H | H | CH₂CH₂-(4-Ac-1-Piz) | 2-F | H | H |
| 6-202 | Ac | H | CH₂CH₂-(4-Ac-1-Piz) | 2-F | H | H |
| 6-203 | H | H | CH₂CH₂-(4-Ac-1-Piz) | 2-F | 4-F | H |
| 6-204 | Ac | H | CH₂CH₂-(4-Ac-1-Piz) | 2-F | 4-F | H |
| 6-205 | H | H | CH₂CH₂-(4-CO₂Me-1-Piz) | 2-F | H | H |
| 6-206 | Ac | H | CH₂CH₂-(4-CO₂Me-1-Piz) | 2-F | H | H |
| 6-207 | H | H | CH₂CH₂-(4-CO₂Et-1-Piz) | 2-F | H | H |
| 6-208 | Ac | H | CH₂CH₂-(4-CO₂Et-1-Piz) | 2-F | H | H |
| 6-209 | H | H | CH₂CH₂-(4-CH₂CO₂H-1-Piz) | 2-F | H | H |
| 6-210 | Ac | H | CH₂CH₂-(4-CH₂CO₂H-1-Piz) | 2-F | H | H |
| 6-211 | H | H | CH₂CH₂-(4-CH₂CO₂Me-1-Piz) | 2-F | H | H |
| 6-212 | Ac | H | CH₂CH₂-(4-CH₂CO₂Me-1-Piz) | 2-F | H | H |
| 6-213 | H | H | CH₂CH₂-(4-CH₂CO₂Et-1-Piz) | 2-F | H | H |
| 6-214 | Ac | H | CH₂CH₂-(4-CH₂CO₂Et-1-Piz) | 2-F | H | H |
| 6-215 | H | H | CH₂CH₂-(4-CH₂CH₂CO₂H-1-Piz) | 2-F | H | H |
| 6-216 | Ac | H | CH₂CH₂-(4-CH₂CH₂CO₂H-1-Piz) | 2-F | H | H |
| 6-217 | H | H | CH₂CH₂-(4-CH₂CH₂CO₂Me-1-Piz) | 2-F | H | H |
| 6-218 | Ac | H | CH₂CH₂-(4-CH₂CH₂CO₂Me-1-Piz) | 2-F | H | H |
| 6-219 | H | H | CH₂CH₂-(4-CH₂CH₂CO₂Et-1-Piz) | 2-F | H | H |
| 6-220 | Ac | H | CH₂CH₂-(4-CH₂CH₂CO₂Et-1-Piz) | 2-F | H | H |
| 6-221 | H | H | CH₂CH₂-(4-CH₂CH₂CH₂CO₂H-1-Piz) | 2-F | H | H |
| 6-222 | Ac | H | CH₂CH₂-(4-CH₂CH₂CH₂CO₂H-1-Piz) | 2-F | H | H |
| 6-223 | H | H | CH₂CH₂-(4-CH₂CH₂CH₂CO₂Me-1-Piz) | 2-F | H | H |
| 6-224 | Ac | H | CH₂CH₂-(4-CH₂CH₂CH₂CO₂Me-1-Piz) | 2-F | H | H |
| 6-225 | H | H | CH₂CH₂-(4-CH₂CH₂CH₂CO₂Et-1-Piz) | 2-F | H | H |
| 6-226 | Ac | H | CH₂CH₂-(4-CH₂CH₂CH₂CO₂Et-1-Piz) | 2-F | H | H |
| 6-227 | H | H | CH₂CH₂-(2-oxo-1-Piz) | 2-F | H | H |
| 6-228 | Ac | H | CH₂CH₂-(2-oxo-1-Piz) | 2-F | H | H |
| 6-229 | H | H | CH₂CH₂-(4-Ac-2-oxo-1-Piz) | 2-F | H | H |
| 6-230 | Ac | H | CH₂CH₂-(4-Ac-2-oxo-1-Piz) | 2-F | H | H |
| 6-231 | H | H | CH₂CH₂-(4-Ac-2-oxo-1-Piz) | 2-F | 4-F | H |
| 6-232 | Ac | H | CH₂CH₂-(4-Ac-2-oxo-1-Piz) | 2-F | 4-F | H |
| 6-233 | H | H | CH₂CH₂-(4-CO₂Me-2-oxo-1-Piz) | 2-F | H | H |
| 6-234 | Ac | H | CH₂CH₂-(4-CO₂Me-2-oxo-1-Piz) | 2-F | H | H |
| 6-235 | H | H | CH₂CH₂-(4-CO₂Et-2-oxo-1-Piz) | 2-F | H | H |
| 6-236 | Ac | H | CH₂CH₂-(4-CO₂Et-2-oxo-1-Piz) | 2-F | H | H |
| 6-237 | H | H | CH₂CH₂-(4-CH₂CO₂H-2-oxo-1-Piz) | 2-F | H | H |
| 6-238 | Ac | H | CH₂CH₂-(4-CH₂CO₂H-2-oxo-1-Piz) | 2-F | H | H |
| 6-239 | H | H | CH₂CH₂-(4-CH₂CO₂Me-2-oxo-1-Piz) | 2-F | H | H |
| 6-240 | Ac | H | CH₂CH₂-(4-CH₂CO₂Me-2-oxo-1-Piz) | 2-F | H | H |
| 6-241 | H | H | CH₂CH₂-(4-CH₂CO₂Et-2-oxo-1-Piz) | 2-F | H | H |
| 6-242 | Ac | H | CH₂CH₂-(4-CH₂CO₂Et-2-oxo-1-Piz) | 2-F | H | H |
| 6-243 | H | H | CH₂CH₂-(4-CH₂CH₂CO₂H-2-oxo-1-Piz) | 2-F | H | H |
| 6-244 | Ac | H | CH₂CH₂-(4-CH₂CH₂CO₂H-2-oxo-1-Piz) | 2-F | H | H |
| 6-245 | H | H | CH₂CH₂-(4-CH₂CH₂CO₂Me-2-oxo-1-Piz) | 2-F | H | H |
| 6-246 | Ac | H | CH₂CH₂-(4-CH₂CH₂CO₂Me-2-oxo-1-Piz) | 2-F | H | H |
| 6-247 | H | H | CH₂CH₂-(4-CH₂CH₂CO₂Et-2-oxo-1-Piz) | 2-F | H | H |
| 6-248 | Ac | H | CH₂CH₂-(4-CH₂CH₂CO₂Et-2-oxo-1-Piz) | 2-F | H | H |
| 6-249 | H | H | CH₂CH₂-(4-CH₂CH₂CH₂CO₂H-2-oxo-1-Piz) | 2-F | H | H |
| 6-250 | Ac | H | CH₂CH₂-(4-CH₂CH₂CH₂CO₂H-2-oxo-1-Piz) | 2-F | H | H |
| 6-251 | H | H | CH₂CH₂-(4-CH₂CH₂CH₂CO₂Me-2-oxo-1-Piz) | 2-F | H | H |
| 6-252 | Ac | H | CH₂CH₂-(4-CH₂CH₂CH₂CO₂Me-2-oxo-1-Piz) | 2-F | H | H |
| 6-253 | H | H | CH₂CH₂-(4-CH₂CH₂CH₂CO₂Et-2-oxo-1-Piz) | 2-F | H | H |
| 6-254 | Ac | H | CH₂CH₂-(4-CH₂CH₂CH₂CO₂Et-2-oxo-1-Piz) | 2-F | H | H |
| 6-255 | H | H | CH₂CH₂-(3-oxo-1-Piz) | 2-F | H | H |
| 6-256 | Ac | H | CH₂CH₂-(3-oxo-1-Piz) | 2-F | H | H |
| 6-257 | H | H | CH₂CH₂-(4-Ac-3-oxo-1-Piz) | 2-F | H | H |
| 6-258 | Ac | H | CH₂CH₂-(4-Ac-3-oxo-1-Piz) | 2-F | H | H |
| 6-259 | H | H | CH₂CH₂-(4-Ac-3-oxo-1-Piz) | 2-F | 4-F | H |
| 6-260 | Ac | H | CH₂CH₂-(4-Ac-3-oxo-1-Piz) | 2-F | 4-F | H |
| 6-261 | H | H | CH₂CH₂-(4-CO₂Me-3-oxo-1-Piz) | 2-F | H | H |
| 6-262 | Ac | H | CH₂CH₂-(4-CO₂Me-3-oxo-1-Piz) | 2-F | H | H |
| 6-263 | H | H | CH₂CH₂-(4-CO₂Et-3-oxo-1-Piz) | 2-F | H | H |
| 6-264 | Ac | H | CH₂CH₂-(4-CO₂Et-3-oxo-1-Piz) | 2-F | H | H |
| 6-265 | H | H | CH₂CH₂-(4-CH₂CO₂H-3-oxo-1-Piz) | 2-F | H | H |
| 6-266 | Ac | H | CH₂CH₂-(4-CH₂CO₂H-3-oxo-1-Piz) | 2-F | H | H |
| 6-267 | H | H | CH₂CH₂-(4-CH₂CO₂Me-3-oxo-1-Piz) | 2-F | H | H |
| 6-268 | Ac | H | CH₂CH₂-(4-CH₂CO₂Me-3-oxo-1-Piz) | 2-F | H | H |
| 6-269 | H | H | CH₂CH₂-(4-CH₂CO₂Et-3-oxo-1-Piz) | 2-F | H | H |
| 6-270 | Ac | H | CH₂CH₂-(4-CH₂CO₂Et-3-oxo-1-Piz) | 2-F | H | H |
| 6-271 | H | H | CH₂CH₂-(4-CH₂CH₂CO₂H-3-oxo-1-Piz) | 2-F | H | H |
| 6-272 | Ac | H | CH₂CH₂-(4-CH₂CH₂CO₂H-3-oxo-1-Piz) | 2-F | H | H |
| 6-273 | H | H | CH₂CH₂-(4-CH₂CH₂CO₂Me-3-oxo-1-Piz) | 2-F | H | H |
| 6-274 | Ac | H | CH₂CH₂-(4-CH₂CH₂CO₂Me-3-oxo-1-Piz) | 2-F | H | H |
| 6-275 | H | H | CH₂CH₂-(4-CH₂CH₂CO₂Et-3-oxo-1-Piz) | 2-F | H | H |
| 6-276 | Ac | H | CH₂CH₂-(4-CH₂CH₂CO₂Et-3-oxo-1-Piz) | 2-F | H | H |
| 6-277 | H | H | CH₂CH₂-(4-CH₂CH₂CH₂CO₂H-3-oxo-1-Piz) | 2-F | H | H |
| 6-278 | Ac | H | CH₂CH₂-(4-CH₂CH₂CH₂CO₂H-3-oxo-1-Piz) | 2-F | H | H |
| 6-279 | H | H | CH₂CH₂-(4-CH₂CH₂CH₂CO₂Me-3-oxo-1-Piz) | 2-F | H | H |
| 6-280 | Ac | H | CH₂CH₂-(4-CH₂CH₂CH₂CO₂Me-3-oxo-1-Piz) | 2-F | H | H |
| 6-281 | H | H | CH₂CH₂-(4-CH₂CH₂CH₂CO₂Et-3-oxo-1-Piz) | 2-F | H | H |
| 6-282 | Ac | H | CH₂CH₂-(4-CH₂CH₂CH₂CO₂Et-3-oxo-1-Piz) | 2-F | H | H |
| 6-283 | H | H | CH₂CH₂-(2,5-dioxo-1-Piz) | 2-F | H | H |
| 6-284 | Ac | H | CH₂CH₂-(2,5-dioxo-1-Piz) | 2-F | H | H |
| 6-285 | H | H | CH₂CH₂-(4-Ac-2,5-dioxo-1-Piz) | 2-F | H | H |
| 6-286 | Ac | H | CH₂CH₂-(4-Ac-2,5-dioxo-1-Piz) | 2-F | H | H |
| 6-287 | H | H | CH₂CH₂-(4-Ac-2,5-dioxo-1-Piz) | 2-F | 4-F | H |
| 6-288 | Ac | H | CH₂CH₂-(4-Ac-2,5-dioxo-1-Piz) | 2-F | 4-F | H |
| 6-289 | H | H | CH₂CH₂-(4-CO₂Me-2,5-dioxo-1-Piz) | 2-F | H | H |
| 6-290 | Ac | H | CH₂CH₂-(4-CO₂Me-2,5-dioxo-1-Piz) | 2-F | H | H |
| 6-291 | H | H | CH₂CH₂-(4-CO₂Et-2,5-dioxo-1-Piz) | 2-F | H | H |
| 6-292 | Ac | H | CH₂CH₂-(4-CO₂Et-2,5-dioxo-1-Piz) | 2-F | H | H |
| 6-293 | H | H | CH₂CH₂-(4-CH₂CO₂H-2,5-dioxo-1-Piz) | 2-F | H | H |
| 6-294 | Ac | H | CH₂CH₂-(4-CH₂CO₂H-2,5-dioxo-1-Piz) | 2-F | H | H |
| 6-295 | H | H | CH₂CH₂-(4-CH₂CO₂Me-2,5-dioxo-1-Piz) | 2-F | H | H |
| 6-296 | Ac | H | CH₂CH₂-(4-CH₂CO₂Me-2,5-dioxo-1-Piz) | 2-F | H | H |
| 6-297 | H | H | CH₂CH₂-(4-CH₂CO₂Et-2,5-dioxo-1-Piz) | 2-F | H | H |
| 6-298 | Ac | H | CH₂CH₂-(4-CH₂CO₂Et-2,5-dioxo-1-Piz) | 2-F | H | H |
| 6-299 | H | H | CH₂CH₂-(4-CH₂CH₂CO₂H-2,5-dioxo-1-Piz) | 2-F | H | H |
| 6-300 | Ac | H | CH₂CH₂-(4-CH₂CH₂CO₂H-2,5-dioxo-1-Piz) | 2-F | H | H |
| 6-301 | H | H | CH₂CH₂-(4-CH₂CH₂CO₂Me-2,5-dioxo-1-Piz) | 2-F | H | H |
| 6-302 | Ac | H | CH₂CH₂-(4-CH₂CH₂CO₂Me-2,5-dioxo-1-Piz) | 2-F | H | H |
| 6-303 | H | H | CH₂CH₂-(4-CH₂CH₂CO₂Et-2,5-dioxo-1-Piz) | 2-F | H | H |
| 6-304 | Ac | H | CH₂CH₂-(4-CH₂CH₂CO₂Et-2,5-dioxo-1-Piz) | 2-F | H | H |
| 6-305 | H | H | CH₂CH₂-(4-CH₂CH₂CH₂CO₂H-2,5-dioxo-1-Piz) | 2-F | H | H |
| 6-306 | Ac | H | CH₂CH₂-(4-CH₂CH₂CH₂CO₂H-2,5-dioxo-1-Piz) | 2-F | H | H |
| 6-307 | H | H | CH₂CH₂-(4-CH₂CH₂CH₂CO₂Me-2,5-dioxo-1-Piz) | 2-F | H | H |
| 6-308 | Ac | H | CH₂CH₂-(4-CH₂CH₂CH₂CO₂Me-2,5-dioxo-1-Piz) | 2-F | H | H |
| 6-309 | H | H | CH₂CH₂-(4-CH₂CH₂CH₂CO₂Et-2,5-dioxo-1-Piz) | 2-F | H | H |
| 6-310 | Ac | H | CH₂CH₂-(4-CH₂CH₂CH₂CO₂Et-2,5-dioxo-1-Piz) | 2-F | H | H |
| 6-311 | H | H | CH₂CH₂-1-Pyrd | 2-F | H | H |
| 6-312 | Ac | H | CH₂CH₂-1-Pyrd | 2-F | H | H |
| 6-313 | H | H | CH₂CH₂-(2-CO₂H-1-Pyrd) | 2-F | H | H |
| 6-314 | Ac | H | CH₂CH₂-(2-CO₂H-1-Pyrd) | 2-F | H | H |
| 6-315 | H | H | CH₂CH₂-(2-CO₂Me-1-Pyrd) | 2-F | H | H |
| 6-316 | Ac | H | CH₂CH₂-(2-CO₂Me-1-Pyrd) | 2-F | H | H |
| 6-317 | H | H | CH₂CH₂-(2-CO₂Et-1-Pyrd) | 2-F | H | H |
| 6-318 | Ac | H | CH₂CH₂-(2-CO₂Et-1-Pyrd) | 2-F | H | H |
| 6-319 | H | H | CH₂CH₂-(2-CH₂CO₂H-1-Pyrd) | 2-F | H | H |
| 6-320 | Ac | H | CH₂CH₂-(2-CH₂CO₂H-1-Pyrd) | 2-F | H | H |
| 6-321 | H | H | CH₂CH₂-(2-CH₂CO₂Me-1-Pyrd) | 2-F | H | H |
| 6-322 | Ac | H | CH₂CH₂-(2-CH₂CO₂Me-1-Pyrd) | 2-F | H | H |
| 6-323 | H | H | CH₂CH₂-(2-CH₂CO₂Et-1-Pyrd) | 2-F | H | H |
| 6-324 | Ac | H | CH₂CH₂-(2-CH₂CO₂Et-1-Pyrd) | 2-F | H | H |
| 6-325 | H | H | CH₂CH₂-(2-CH₂CH₂CO₂H-1-Pyrd) | 2-F | H | H |
| 6-326 | Ac | H | CH₂CH₂-(2-CH₂CH₂CO₂H-1-Pyrd) | 2-F | H | H |
| 6-327 | H | H | CH₂CH₂-(2-CH₂CH₂CO₂Me-1-Pyrd) | 2-F | H | H |
| 6-328 | Ac | H | CH₂CH₂-(2-CH₂CH₂CO₂Me-1-Pyrd) | 2-F | H | H |
| 6-329 | H | H | CH₂CH₂-(2-CH₂CH₂CO₂Et-1-Pyrd) | 2-F | H | H |
| 6-330 | Ac | H | CH₂CH₂-(2-CH₂CH₂CO₂Et-1-Pyrd) | 2-F | H | H |
| 6-331 | H | H | CH₂CH₂-(2-CH₂CH₂CH₂CO₂H-1-Pyrd) | 2-F | H | H |
| 6-332 | Ac | H | CH₂CH₂-(2-CH₂CH₂CH₂CO₂H-1-Pyrd) | 2-F | H | H |
| 6-333 | H | H | CH₂CH₂-(2-CH₂CH₂CH₂CO₂Me-1-Pyrd) | 2-F | H | H |
| 6-334 | Ac | H | CH₂CH₂-(2-CH₂CH₂CH₂CO₂Me-1-Pyrd) | 2-F | H | H |
| 6-335 | H | H | CH₂CH₂(2-CH₂CH₂CH₂CO₂Et-1-Pyrd) | 2-F | H | H |
| 6-336 | Ac | H | CH₂CH₂-(2-CH₂CH₂CH₂CO₂Et-1-Pyrd) | 2-F | H | H |
| 6-337 | H | H | CH₂-(3-CO₂H-1-Pip) | 2-F | H | H |
| 6-338 | Ac | H | CH₂-(3-CO₂H-1-Pip) | 2-F | H | H |
| 6-339 | H | H | CH₂-(3-CO₂Me-1-Pip) | 2-F | H | H |
| 6-340 | Ac | H | CH₂-(3-CO₂Me-1-Pip) | 2-F | H | H |
| 6-341 | H | H | CH₂-(3-CO₂Et-1-Pip) | 2-F | H | H |
| 6-342 | Ac | H | CH₂-(3-CO₂Et-1-Pip) | 2-F | H | H |
| 6-343 | H | H | CH₂-(3-CH₂CO₂H-1-Pip) | 2-F | H | H |
| 6-344 | Ac | H | CH₂-(3-CH₂CO₂H-1-Pip) | 2-F | H | H |
| 6-345 | H | H | CH₂-(3-CH₂CO₂Me-1-Pip) | 2-F | H | H |
| 6-346 | Ac | H | CH₂-(3-CH₂CO₂Me-1-Pip) | 2-F | H | H |
| 6-347 | H | H | CH₂-(3-CH₂CO₂Et-1-Pip) | 2-F | H | H |
| 6-348 | Ac | H | CH₂-(3-CH₂CO₂Et-1-Pip) | 2-F | H | H |
| 6-349 | H | H | CH₂-(3-CH₂CH₂CO₂H-1-Pip) | 2-F | H | H |
| 6-350 | Ac | H | CH₂-(3-CH₂CH₂CO₂H-1-Pip) | 2-F | H | H |
| 6-351 | H | H | CH₂-(3-CH₂CH₂CO₂Me-1-Pip) | 2-F | H | H |
| 6-352 | Ac | H | CH₂-(3-CH₂CH₂CO₂Me-1-Pip) | 2-F | H | H |
| 6-353 | H | H | CH₂-(3-CH₂CH₂CO₂Et-1-Pip) | 2-F | H | H |
| 6-354 | Ac | H | CH₂-(3-CH₂CH₂CO₂Et-1-Pip) | 2-F | H | H |
| 6-355 | H | H | CH₂-(3-CH₂CH₂CH₂CO₂H-1-Pip) | 2-F | H | H |
| 6-356 | Ac | H | CH₂-(3-CH₂CH₂CH₂CO₂H-1-Pip) | 2-F | H | H |
| 6-357 | H | H | CH₂-(3-CH₂CH₂CH₂CO₂Me-1-Pip) | 2-F | H | H |
| 6-358 | Ac | H | CH₂-(3-CH₂CH₂CH₂CO₂Me-1-Pip) | 2-F | H | H |
| 6-359 | H | H | CH₂-(3-CH₂CH₂CH₂CO₂Et-1-Pip) | 2-F | H | H |
| 6-360 | Ac | H | CH₂-(3-CH₂CH₂CH₂CO₂Et-1-Pip) | 2-F | H | H |
| 6-361 | H | H | CH₂CH₂-(3-CO₂H-1-Pip) | 2-F | H | H |
| 6-362 | Ac | H | CH₂CH₂-(3-CO₂H-1-Pip) | 2-F | H | H |
| 6-363 | H | H | CH₂CH₂-(3-CO₂Me-1-Pip) | 2-F | H | H |
| 6-364 | Ac | H | CH₂CH₂-(3-CO₂Me-1-Pip) | 2-F | H | H |
| 6-365 | H | H | CH₂CH₂-(3-CO₂Et-1-Pip) | 2-F | H | H |
| 6-366 | Ac | H | CH₂CH₂-(3-CO₂Et-1-Pip) | 2-F | H | H |
| 6-367 | H | H | CH₂CH₂-(3-CH₂CO₂H-1-Pip) | 2-F | H | H |
| 6-368 | Ac | H | CH₂CH₂-(3-CH₂CO₂H-1-Pip) | 2-F | H | H |
| 6-369 | H | H | CH₂CH₂-(3-CH₂CO₂Me-1-Pip) | 2-F | H | H |
| 6-370 | Ac | H | CH₂CH₂-(3-CH₂CO₂Me-1-Pip) | 2-F | H | H |
| 6-371 | H | H | CH₂CH₂-(3-CH₂CO₂Et-1-Pip) | 2-F | H | H |
| 6-372 | Ac | H | CH₂CH₂-(3-CH₂CO₂Et-1-Pip) | 2-F | H | H |
| 6-373 | H | H | CH₂CH₂-(3-CH₂CH₂CO₂H-1-Pip) | 2-F | H | H |
| 6-374 | Ac | H | CH₂CH₂-(3-CH₂CH₂CO₂H-1-Pip) | 2-F | H | H |
| 6-375 | H | H | CH₂CH₂-(3-CH₂CH₂CO₂Me-1-Pip) | 2-F | H | H |
| 6-376 | Ac | H | CH₂CH₂-(3-CH₂CH₂CO₂Me-1-Pip) | 2-F | H | H |
| 6-377 | H | H | CH₂CH₂-(3-CH₂CH₂CO₂Et-1-Pip) | 2-F | H | H |
| 6-378 | Ac | H | CH₂CH₂-(3-CH₂CH₂CO₂Et-1-Pip) | 2-F | H | H |
| 6-379 | H | H | CH₂CH₂-(3-CH₂CH₂CH₂CO₂H-1-Pip) | 2-F | H | H |
| 6-380 | Ac | H | CH₂CH₂-(3-CH₂CH₂CH₂CO₂H-1-Pip) | 2-F | H | H |
| 6-381 | H | H | CH₂CH₂-(3-CH₂CH₂CH₂CO₂Me-1-Pip) | 2-F | H | H |
| 6-382 | Ac | H | CH₂CH₂-(3-CH₂CH₂CH₂CO₂Me-1-Pip) | 2-F | H | H |
| 6-383 | H | H | CH₂CH₂-(3-CH₂CH₂CH₂CO₂Et-1-Pip) | 2-F | H | H |
| 6-384 | Ac | H | CH₂CH₂-(3-CH₂CH₂CH₂CO₂Et-1-Pip) | 2-F | H | H |
| 6-385 | H | H | CH₂-(4-OH-2-CO₂H-1-Pyrd) | 2-F | H | H |
| 6-386 | Ac | H | CH₂-(4-OH-2-CO₂H-1-Pyrd) | 2-F | H | H |
| 6-387 | H | H | CH₂-(4-OH -2-CO₂Me-1-Pyrd) | 2-F | H | H |
| 6-388 | Ac | H | CH₂-(4-OH-2-CO₂Me-1-Pyrd) | 2-F | H | H |
| 6-389 | H | H | CH₂-(4-OH-2-CO₂Et-1-Pyrd) | 2-F | H | H |
| 6-390 | Ac | H | CH₂-(4-OH-2-CO₂Et-1-Pyrd) | 2-F | H | H |

Exemplification Compound Nos. of Compounds obtained by substituting a cyclopropyl group of the compounds of Exemplification Compound Nos. 1-1 to 1-1084, 2-1 to 2-1084, 3-1 to 3-79, 4-1 to 4-79, 5-1 to 5-390 and 6-1 to 6-390 in Tables 1 to 6 with a methoxy group are respectively 7-1 to 7-1084, 8-1 to 8-1084, 9-1 to 9-79, 10-1 to 10-79, 11-1 to 11-390 and 12-1 to 12-390. For example, a compound obtained by substituting a cyclopropyl group of the compound of Exemplification Compound No. 5-103 in Table 7 with a methoxy group is a compound of Exemplification Compound No. 11-103.

Among the above compounds, preferred compounds are the compounds of Exemplification Compound Nos. 1-1, 1-2, 1-5, 1-6, 1-9, 1-10, 1-13, 1-14, 1-17, 1-18; 1-21, 1-22, 1-25, 1-26, 1-29, 1-30, 1-33, 1-34, 1-37, 1-38, 1-41, 1-42, 1-45, 1-46, 1-49, 1-50, 1-53, 1-54, 1-57, 1-58, 1-61, 1-62, 1-87, 1-88, 1-115, 1-116, 1-121, 1-122, 1-127, 1-128, 1-133, 1-134, 1-139, 1-140, 1-187, 1-188, 1-207, 1-208, 1-209, 1-210, 1-211, 1-212, 1-337, 1-338, 1-351, 1-352, 1-353, 1-354, 1-355, 1-356, 1-443, 1-444, 1-445,1-446, 1-447,1-448, 1-485,1-486,1-487, 1-488, 1-489, 1-490, 1-491,1-492, 1-493, 1-494, 1-495, 1-496, 1-497, 1-498,1-499, 1-500, 1-501, 1-502, 1-503, 1-504, 1-537, 1-538, 1-549, 1-550, 1-585,1-586,1-587, 1-588, 1-589, 1-590, 1-591, 1-592, 1-593, 1-594, 1-595, 1-596, 1-597, 1-598, 1-613, 1-614, 1-619, 1-620,1-637, 1-638, 1-651,1-652,1-653,1-654,1-655,1-656,1-663,1-664,1-665,1-666,1-667,1-668, 1-743, 1-744, 1-745, 1-746, 1-747, 1-748, 1-749, 1-750, 1-751, 1-752, 1-753, 1-754, 1-761,1-762,1-763,1-764,1-765,1-766,1-767, 1-768, 1-789,1-790,1-813,1-814, 1-815, 1-816, 1-817, 1-818, 1-819, 1-820, 1-841, 1-842, 1-865, 1-866, 1-879, 1-880, 1-881,1-882,1-883,1-884,1-885,1-886,1-887,1-888,1-889,1-890,1-965,1-966, 1-967, 1-968, 1-969,1-970,1-971, 1-972, 1-973, 1-974, 1-975, 1-976, 1-977, 1-978, 1-979, 1-980, 1-981, 1-982, 1-983, 1-984, 1-985, 1-986, 1-987, 1-988, 1-989, 1-990, 1-1005, 1-1006, 1-1011, 1-1012, 1-1017, 1-1018, 1-1023, 1-1024, 1-1045, 1-1046, 1-1047, 1-1048, 1-1049, 1-1050, 2-1, 2-2, 2-5, 2-6, 2-9, 2-10, 2-13, 2-14, 2-17, 2-18, 2-21, 2-22, 2-25, 2-26, 2-29, 2-30, 2-33, 2-34, 2-37, 2-38, 2-41, 2-42, 2-45, 2-46, 2-49, 2-50, 2-53, 2-54, 2-57, 2-58, 2-61, 2-62, 2-87, 2-88, 2-115, 2-116, 2-121, 2-122, 2-127, 2-128, 2-133, 2-134, 2-139, 2-140, 2-187, 2-188, 2-207, 2-208, 2-209, 2-210, 2-211, 2-212, 2-337, 2-338, 2-351, 2-352, 2-353, 2-354, 2-355, 2-356, 2-443, 2-444, 2-445, 2-446, 2-447, 2-448, 2-485, 2-486, 2-487, 2-488, 2-489, 2-490, 2-491, 2-492, 2-493, 2-494, 2-495, 2-496, 2-497, 2-498, 2-499, 2-500, 2-501, 2-502, 2-503, 2-504, 2-537, 2-538, 2-549, 2-550, 2-585, 2-586, 2-587, 2-588, 2-589, 2-590, 2-591, 2-592, 2-593, 2-594, 2-595, 2-596, 2-597, 2-598, 2-613, 2-614, 2-619, 2-620, 2-637, 2-638, 2-651, 2-652, 2-653, 2-654, 2-655, 2-656, 2-663, 2-664, 2-665, 2-666, 2-667, 2-668, 2-743, 2-744, 2-745, 2-746, 2-747, 2-748, 2-749, 2-750, 2-751, 2-752, 2-753, 2-754, 2-761, 2-762, 2-763, 2-764, 2-765, 2-766, 2-767, 2-768, 2-789, 2-790, 2-813, 2-814, 2-815, 2-816, 2-817, 2-818, 2-819, 2-820, 2-841, 2-842, 2-865, 2-866, 2-879, 2-880, 2-881, 2-882, 2-883, 2-884, 2-885, 2-886, 2-887, 2-888, 2-889, 2-890, 2-965, 2-966, 2-967, 2-968, 2-969, 2-970, 2-971, 2-972, 2-973, 2-974, 2-975, 2-976, 2-977, 2-978, 2-979, 2-980, 2-981, 2-982, 2-983, 2-984, 2-985, 2-986, 2-987, 2-988, 2-989, 2-990, 2-1005, 2-1006, 2-1011, 2-1012, 2-1017, 2-1018, 2-1023, 2-1024, 2-1045, 2-1046, 2-1047, 2-1048, 2-1049, 2-1050, 3-1, 3-2, 3-5, 3-6, 3-13, 3-14, 3-15, 3-16, 3-17, 3-18, 3-27, 3-28, 3-29, 3-30, 3-31, 3-32, 3-33, 3-34, 3-35, 3-36, 3-37, 3-38, 3-39, 3-46, 3-47, 3-50, 3-51, 3-52, 3-53, 3-54, 3-55, 3-56, 3-57, 3-58, 3-59, 3-60, 3-61, 3-62, 3-63, 3-64, 3-65, 3-66, 3-67, 3-68, 3-69, 3-70, 3-71, 3-72, 3-73, 3-74, 3-75, 3-76, 3-77, 3-78, 3-79, 4-1, 4-2, 4-5, 4-6, 4-13, 4-14, 4-15, 4-16, 4-17, 4-18, 4-27, 4-28, 4-29, 4-30, 4-31, 4-32, 4-33, 4-34, 4-35, 4-36, 4-37, 4-38, 4-39, 4-46, 4-47, 4-50, 4-51, 4-52, 4-53, 4-54, 4-55, 4-56, 4-57, 4-58, 4-59, 4-60, 4-61, 4-62, 4-63, 4-64, 4-65, 4-66, 4-67, 4-68, 4-69, 4-70, 4-71, 4-72, 4-73, 4-74, 4-75, 4-76, 4-77, 4-78, 4-79, 5-1, 5-2, 5-3, 5-4, 5-5, 5-6, 5-7, 5-8, 5-9, 5-10, 5-11, 5-12, 5-13, 5-14, 5-27, 5-28, 5-29, 5-30, 5-31, 5-32, 5-33, 5-34, 5-41, 5-42, 5-43, 5-44, 5-45, 5-46, 5-59, 5-60, 5-61, 5-62, 5-69, 5-70, 5-71, 5-72, 5-73, 5-74, 5-75, 5-76, 5-77, 5-78, 5-79, 5-80, 5-81, 5-82, 5-85, 5-86, 5-87, 5-88, 5-89, 5-90, 5-93, 5-94, 5-95, 5-96, 5-97, 5-98, 5-99, 5-100, 5-101, 5-102, 5-103, 5-104, 5-105, 5-106, 5-107, 5-108, 5-115, 5-116, 5-117, 5-118, 5-125, 5-126, 5-127, 5-128, 5-129, 5-130, 5-131, 5-132, 5-133, 5-134, 5-135, 5-136, 5-143, 5-144, 5-145, 5-146, 5-147, 5-148, 5-149, 5-150, 5-151, 5-152, 5-153, 5-154, 5-155, 5-156, 5-171, 5-172, 5-177, 5-178, 5-209, 5-210, 5-237, 5-238, 5-265, 5-266, 5-293, 5-294, 5-313, 5-314, 5-319, 5-320, 5-337, 5-338, 5-339, 5-340, 5-341, 5-342, 5-343, 5-344, 5-345, 5-346, 5-347, 5-348, 5-361, 5-362, 5-367, 5-368, 5-385, 5-386, 5-387, 5-388, 6-1, 6-2, 6-3, 6-4, 6-5, 6-6, 6-7, 6-8, 6-9, 6-10, 6-11, 6-12, 6-13, 6-14, 6-27, 6-28, 6-29, 6-30, 6-31, 6-32, 6-33, 6-34, 6-41, 6-42, 6-43, 6-44, 6-45, 6-46, 6-59, 6-60, 6-61, 6-62, 6-69, 6-70, 6-71, 6-72, 6-73, 6-74, 6-75, 6-76, 6-77, 6-78, 6-79, 6-80, 6-81, 6-82, 6-85, 6-86, 6-87, 6-88, 6-89, 6-90, 6-93, 6-94, 6-95, 6-96, 6-97, 6-98, 6-99, 6-100, 6-101, 6-102, 6-103, 6-104, 6-105, 6-106, 6-107, 6-108, 6-115, 6-116, 6-117, 6-118, 6-125, 6-126, 6-127, 6-128, 6-129, 6-130, 6-131, 6-132, 6-133, 6-134, 6-135, 6-136, 6-143, 6-144, 6-145, 6-146, 6-147, 6-148, 6-149, 6-150, 6-151, 6-152, 6-153, 6-154, 6-155, 6-156, 6-171, 6-172, 6-177, 6-178, 6-209, 6-210, 6-237, 6-238, 6-265, 6-266, 6-293, 6-294, 6-313, 6-314, 6-319, 6-320, 6-337, 6-338, 6-339, 6-340, 6-341, 6-342, 6-343, 6-344, 6-345, 6-346, 6-347, 6-348, 6-361, 6-362, 6-367, 6-368, 6-385, 6-386, 6-387 and 6-388,
more preferred compounds are the compounds of Exemplification Compound Nos. 1-1, 1-2, 1-5, 1-6, 1-17, 1-18, 1-21, 1-22, 1-25, 1-26, 1-53, 1-54, 1-207, 1-208, 1-351, 1-352, 1-443, 1-444, 1-485, 1-486, 1-487, 1-488, 1-489, 1-490, 1-491, 1-492, 1-493, 1-494, 1-587, 1-588, 1-589, 1-590, 1-591, 1-592, 1-651, 1-652, 1-743, 1-744, 1-763, 1-764, 1-815, 1-816, 1-967, 1-968, 1-973, 1-974, 1-975, 1-976, 1-977, 1-978, 2-1, 2-2, 2-5, 2-6, 2-17, 2-18, 2-21, 2-22, 2-25, 2-26, 2-53, 2-54, 2-207, 2-208, 2-351, 2-352, 2-443, 2-444, 2-485, 2-486, 2-487, 2-488, 2-489, 2-490, 2-491, 2-492, 2-493, 2-494, 2-587, 2-588, 2-589, 2-590, 2-591, 2-592, 2-651, 2-652, 2-743, 2-744, 2-763, 2-764, 2-815, 2-816, 2-967, 2-968, 2-973, 2-974, 2-975, 2-976, 2-977, 2-978, 3-1, 3-2, 3-13, 3-14, 3-15, 3-16, 3-17, 3-18, 3-27, 3-28, 3-29, 3-30, 3-31, 3-32, 3-50, 3-51, 3-54, 3-55, 3-62, 3-63, 3-66, 3-67, 4-1, 4-2, 4-13, 4-14, 4-15, 4-16, 4-17, 4-18, 4-27, 4-28, 4-29, 4-30, 4-31, 4-32, 4-50, 4-51, 4-54, 4-55, 4-62, 4-63, 4-66, 4-67, 5-3, 5-4, 5-5, 5-6, 5-7, 5-8, 5-9, 5-10, 5-11, 5-12, 5-13, 5-14, 5-27, 5-28, 5-29, 5-30, 5-33, 5-34, 5-69, 5-70, 5-71, 5-72, 5-73, 5-74, 5-75, 5-76, 5-77, 5-78, 5-79, 5-80, 5-81, 5-82, 5-85, 5-86, 5-87, 5-88, 5-89, 5-90, 5-93, 5-94, 5-95, 5-96, 5-97, 5-98, 5-99, 5-100, 5-101, 5-102, 5-103, 5-104, 5-105, 5-106, 5-107, 5-108, 5-145, 5-146, 5-147, 5-148, 5-149, 5-150, 5-151, 5-152, 5-153, 5-154, 5-155, 5-156, 5-337, 5-338, 5-339, 5-340, 5-341, 5-342, 5-343, 5-344, 5-345, 5-346, 5-347, 5-348, 5-387, 5-388, 6-3, 6-4, 6-5, 6-6, 6-7, 6-8, 6-9, 6-10, 6-11, 6-12, 6-13, 6-14, 6-27, 6-28, 6-29, 6-30, 6-33, 6-34, 6-69, 6-70, 6-71, 6-72, 6-73, 6-74, 6-75, 6-76, 6-77, 6-78, 6-79, 6-80, 6-81, 6-82, 6-85, 6-86, 6-87, 6-88, 6-89, 6-90, 6-93, 6-94, 6-95, 6-96, 6-97, 6-98, 6-99, 6-100, 6-101, 6-102, 6-103, 6-104, 6-105, 6-106, 6-107, 6-108, 6-145, 6-146, 6-147, 6-148, 6-149, 6-150, 6-151, 6-152, 6-153, 6-154, 6-155, 6-156, 6-337, 6-338, 6-339, 6-340, 6-341, 6-342, 6-343, 6-344, 6-345, 6-346, 6-347, 6-348, 6-387 and 6-388,
further more preferred compounds are the compounds of Exemplification Compound Nos. 1-17, 1-18, 1-53, 1-54, 1-487, 1-488, 1-489, 1-490, 1-491, 1-492, 1-587, 1-588, 1-589, 1-590, 1-591, 1-592, 1-973, 1-974, 2-17, 2-18, 2-53, 2-54, 2-487, 2-488, 2-489, 2-490, 2-491, 2-492, 2-587, 2-588, 2-589, 2-590, 2-591, 2-592, 2-973, 2-974, 3-1, 3-2, 3-13, 3-14, 3-31, 3-32, 3-50, 3-51, 3-54, 3-55, 3-62, 3-63, 3-66, 3-67, 4-1, 4-2, 4-13, 4-14, 4-31, 4-32, 4-50, 4-51, 4-54, 4-55, 4-62, 4-63, 4-66, 4-67, 5-3, 5-4, 5-5, 5-6, 5-7, 5-8, 5-75, 5-76, 5-77, 5-78, 5-79, 5-80, 5-81, 5-82, 5-89, 5-90, 5-93, 5-94, 5-95, 5-96, 5-97, 5-98, 5-99, 5-100, 5-101, 5-102, 5-103, 5-104, 5-105, 5-106, 5-107, 5-108, 5-145, 5-146, 5-147, 5-148, 5-149, 5-150, 5-151, 5-152, 5-153, 5-154, 5-155, 5-156, 5-337, 5-338, 5-339, 5-340, 5-341, 5-342, 5-387, 5-388, 6-3, 6-4, 6-5, 6-6, 6-7, 6-8, 6-75, 6-76, 6-77, 6-78, 6-79, 6-80, 6-81, 6-82, 6-89, 6-90, 6-93, 6-94, 6-95, 6-96, 6-97, 6-98, 6-99, 6-100, 6-101, 6-102, 6-103, 6-104, 6-105, 6-106, 6-107, 6-108, 6-145, 6-146, 6-147, 6-148, 6-149, 6-150, 6-151, 6-152, 6-153, 6-154, 6-155, 6-156, 6-337, 6-338, 6-339, 6-340, 6-341, 6-342, 6-387 and 6-388, and
particularly preferred compounds are
Exemplification Compound No. 1-17: (E)-3-[2-(4-carboxy-1H-pyrazol-1-yl)ethylidene]-1-[2-cyclopropyl-1-(2-fluorophenyl)-2-oxoethyl]-4-sulfanylpiperidine,
Exemplification Compound No. 1-53: (E)-3-{2-[4-(carboxymethyl)-1H-pyrazol-1-yl]ethylidene}-1-[2-cyclopropyl-1-(2-fluorophenyl)-2-oxoethyl]-4-sulfanylpiperidine,
Exemplification Compound No. 1-487: (E)-3-[2-(4-carboxy-1H-1,2,3-triazol-1-yl)ethylidene]-1-[2-cyclopropyl-1-(2-fluorophenyl)-2-oxoethyl]-4-sulfanylpiperidine,
Exemplification Compound No. 1-587: (E)-3-[2-(4-carboxy-2H-1,2,3-triazol-2-yl)ethylidene]-1-[2-cyclopropyl-1-(2-fluorophenyl)-2-oxoethyl]-4-sulfanylpiperidine
Exemplification Compound No. 1-973: (E)-3-{[2-(4-carboxy-1H-pyrazol-1-yl)-1-methyl]ethylidene}-1-[2-cyclopropyl-1-(2-fluorophenyl)-2-oxoethyl]-4-sulfanylpiperidine,
Exemplification Compound No. 3-1: (E)-1-[2-cyclopropyl-1-(2-fluorophenyl)-2-oxoethyl]-3-(2-hydroxyethylidene)-4-sulfanylpiperidine,
Exemplification Compound No. 3-13: (E)-3-(2-carboxyethylidene)-1-[2-cyclopropyl-1-(2-fluorophenyl)-2-oxoethyl]-4-sulfanylpiperidine,
Exemplification Compound No. 3-31: (E)-1-[2-cyclopropyl-1-(2-fluorophenyl)-2-oxoethyl]-3-{[1-(ethoxycarbonylmethyl)piperidin-4-yl]methylidene}-4-sulfanylpiperidine,
Exemplification Compound No. 3-50: (E)-3-{2-[N-(carboxymethyl)-N-methylainino]ethylidene}-1-[2-cyclopropyl-1-(2-fluorophenyl)-2-oxoethyl]-4-sulfanylpiperidine,
Exemplification Compound No. 3-54: (E)-1-[2-cyclopropyl-1-(2-fluorophenyl)-2-oxoethyl]-3-{2-[N-(ethoxycarbonylmethyl)-N-methylamino] ethylidene} -4-sulfanylpiperidine,
Exemplification Compound No. 3-62: (E)-3-(2- {N-[3-(carboxy)propyl]-N-methylamino}ethylidene)-1-[2-cyclopropyl-1-(2-fluorophenyl)-2-oxoethyl]-4-sulfanylpiperidine,
Exemplification Compound No. 3-66: (E)-1-[2-cyclopropyl-1-(2-fluorophenyl)-2-oxoethyl]-3-(2- {N-[3-(ethoxycarbonyl)propyl]-N-methylamino }ethylidene)-4-sulfanylpiperidine,
Exemplification Compound No. 5-3: (E)-3- {2-[4-(carboxypiperidin)-1-yl]ethylidene}-1-[2-cyclopropyl-1-(2-fluorophenyl)-2-oxoethyl]-4-sulfanylpiperidine,
Exemplification Compound No. 5-7: (E)-1-[2-cyclopropyl-1-(2-fluorophenyl)-2-oxoethyl]-3-{2-[4-(ethoxycarbonyl)piperidin-1-yl]ethylidene}-4-sulfanylpiperidine,
Exemplification Compound No. 5-75: (E)-3-(2-{4-[2-(carboxyethyl)]-2-oxopiperazin-1-yl}ethylidene)-1-[2-cyclopropyl-1-(2-fluorophenyl)-2-oxoethyl]-4-sulfanylpiperidine,
Exemplification Compound No. 5-79: (E)-1-[2-cyclopropyl-1-(2-fluorophenyl)-2-oxoethyl]-3-(2-{4-[2-(ethoxycarbonyl)ethyl]-2-oxopiperazin-1-yl}ethylidene)-4-sulfanylpiperidine,
Exemplification Compound No. 5-81: (E)-3-(2-{4-[3-(carboxypropyl)]-2-oxopiperazin-1-yl} ethylidene)-1-[2-cyclopropyl-1-(2-fluorophenyl)-2-oxoethyl]-4-sulfanylpiperidine,
Exemplification Compound No. 5-97: (E)-3-{2-[4-(carboxymethyl)-3-oxopiperazin-1-yl]ethylidene}-1-[2-cyclopropyl-1-(2-fluorophenyl)-2-oxoethyl]-4-sulfanylpiperidine,
Exemplification Compound No. 5-101: (E)-1-[2-cyclopropyl-1-(2-fluorophenyl)-2-oxoethyl]-3- {2-[4-(ethoxycarbonylmethyl)-3-oxopiperazin-1-yl] ethylidene} -4-sulfanylpiperidine,
Exemplification Compound No. 5-103: (E)-3-(2- {4-[2-(carboxyethyl)]-3-oxopiperazin-1-yl}ethylidene)-1-[2-cyclopropyl-1-(2-fluorophenyl)-2-oxoethyl]-4-sulfanylpiperidine,
Exemplification Compound No. 5-105: (E)-1-[2-cyclopropyl-1-(2-fluorophenyl)-2-oxoethyl]-3-(2- {4-[2-(methoxycarbonyl)ethyl]-3-oxopiperazin-1-yl}ethylidene)-4-sulfanylpiperidine,
Exemplification Compound No. 5-107: (E)-1-[2-cyclopropyl-1-(2-fluorophenyl)-2-oxoethyl]-3-(2-{4-[2-(ethoxycarbonyl)ethyl]-3-oxopiperazin-1-yl}ethylidene)-4-sulfanylpiperidine,
Exemplification Compound No. 5-147: (E)-1-[2-cyclopropyl-1-(2-fluorophenyl)-2-oxoethyl]-3-{2-[(2S)-(methoxycarbonyl)pyrrolidin-1-yl]ethylidene}-4-sulfanylpiperidine,
Exemplification Compound No. 5-151: (E)-3-{2-[(2S)-(carboxymethyl)pyrrolidin-1-yl]ethylidene}-1-[2-cyclopropyl-1-(2-fluorophenyl)-2-oxoethyl]-4-sulfanylpiperidine,
Exemplification Compound No. 5-153: (E)-1-[2-cyclopropyl-1-(2-fluorophenyl)-2-oxoethyl]-3-{2-[(2S)-(methoxycarbonylmethyl)pyrrolidin-1-yl]ethylidene}-4-sulfanylpiperidine,
Exemplification Compound No. 5-337: (E)-3- {2-[3-(carboxypiperidin)-1-yl] ethylidene} -1-[2-cyclopropyl-1-(2-fluorophenyl)-2-oxoethyl]-4-sulfanylpiperidine,
Exemplification Compound No. 5-387: (E)-1-[2-cyclopropyl-1-(2-fluorophenyl)-2-oxoethyl]-3-{2-[(2S,4R)-4-hydroxy-2-(methoxycarbonyl)pyrrolidin-1-yl]ethylidene}-4-sulfanylpiperidine, and
S-acetyl derivatives thereof (Compound Nos.: 1-18, 1-54, 1-488, 1-588, 1-974, 3-2, 3-14, 3-32, 3-51, 3-55, 3-63, 3-67, 5-4, 5-8, 5-76, 5-80, 5-82, 5-98, 5-102, 5-104, 5-106, 5-108, 5-148, 5-152, 5-154, 5-338 and 5-388) or geometrical isomers thereof ((Z)-isomers, Compound Nos.: 2-17, 2-18, 2-53, 2-54, 2-487, 2-488, 2-587, 2-588, 2-973, 2-974, 4-1, 4-2, 4-13, 4-14, 4-31, 4-32, 4-50, 4-51, 4-54, 4-55, 4-62, 4-63, 4-66, 4-67, 6-3, 6-4, 6-7, 6-8, 6-75, 6-76, 6-79, 6-80, 6-81, 6-82, 6-97, 6-98, 6-101, 6-102, 6-103, 6-104, 6-105, 6-106, 6-107, 6-108, 6-147, 6-148, 6-151, 6-152, 6-153, 6-154, 6-337, 6-338, 6-387 and 6-388).

### [Effect of the Invention]

The compounds of the present invention are chemically stable and exert excellent platelet anticoagulation activities and inhibiting action against thrombosis formation. Furthermore, the compounds of the present invention exert the said actions with short onset latencies and exhibit low toxicities. Thus, the compounds of the present invention may be useful in the prophylactic, prevention of recurrence, and therapeutic settings (particularly the latter) against diseases induced by platelet activation such as thrombosis formation and platelet coagulation and releasing responses of platelets, for example in percutaneous coronary intervention (PCI), angioplasty, endarterectomy, restenosis after stenting; acute coronary syndrome, stable or unstable angina, myocardial infarction, atrial fibrillation, cerebral ischemic attack, cerebral infarction, and atherosclerosis and diseases induced by thrombosis formation or embolus formation that are associated with diabetes mellitus, peripheral arterial disease, heparin-induced thrombocytopenia (HIT), thrombotic thrombocytopenic purpura (TTP), antiphospholipid antibody syndrome, venous thrombosis, and ichorrhemia.

### [Best Mode for Carrying out the Invention]

The compound (I) in the present invention can be obtained by Process A or Process B described below.

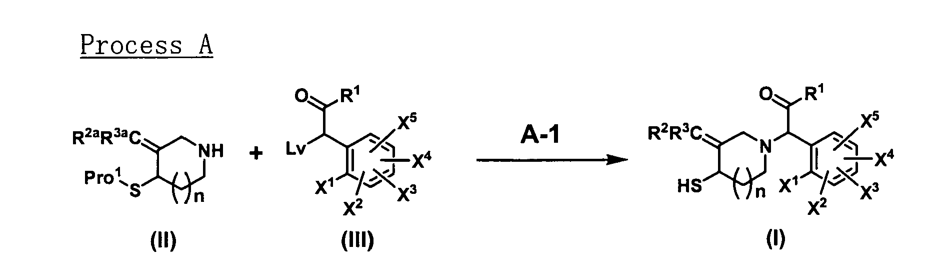

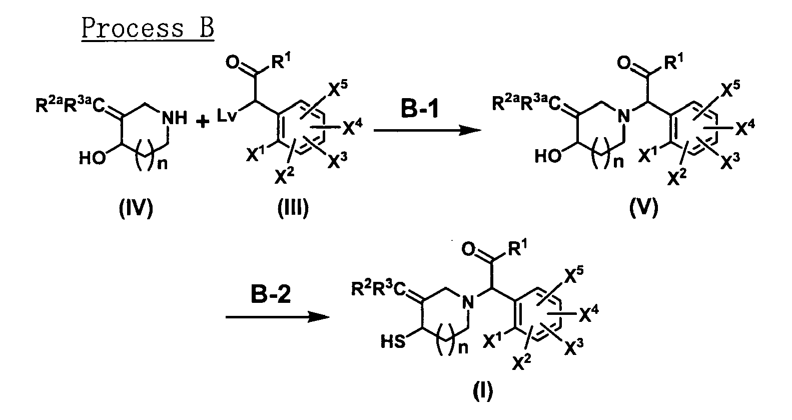

In the above, from R¹ to R³ and X¹ to X⁵ have the same meanings as defined above; R^{2a} and R^{3a} represent R² and R³ described above or a group by which an amino group or a hydroxyl group on R² and R³ may be protected, if necessary, by a protective group for an amino group or a hydroxyl group; Pro¹ represents a protective group for a sulfanyl group; and Lv represents a leaving group.

The protective group for a sulfanyl group as Pro¹ can be the same group as that described as the protective group for a sulfanyl group of the above "prodrug", and is preferably an acetyl group.

The protective group for an amino group of R^{2a} and R^{3a} is not particularly restricted provided that it can usually protect an amino group in chemical reactions, and specifically indicates a protective group that can be cleaved by a chemical process such as hydrogenolysis, hydrolysis, electrolysis and photolysis. Such protective group can be, for example, the above "aliphatic acyl group"; the above "aromatic acyl group"; the above "alkoxycarbonyl group"; the above "aralkyloxycarbonyl group"; the above "silyl group"; the above "aralkyl group"; a "substituted methylene group" that can form a Schiff base such as N,N-dimethylaminomethylene, benzylidene, 4-methoxybenzylidene, 4-nitrobenzylidene, salicylidene, 5-chlorosalicylidene, diphenylmethylene or (5-chloro-2-hydroxyphenyl)phenylmethylene group; an "aromatic sulfonyl group" consisting of: an arylsulfonyl group such as a benzenesulfonyl group, and an arylsulfonyl group substituted with lower alkyl or lower alkoxy group(s) such as a p-toluenesulfonyl, pentamethylbenzenesulfonyl, p-methoxybenzenesulfonyl, 2,4,6-trimethoxybenzenesulfonyl or 3-methoxy-4-t-butylbenzenesulfonyl group; and an "aliphatic sulfonyl group", including an alkylsulfonyl group such as a methanesulfonyl or t-butylsulfonyl group, and an alkylsulfonyl group substituted with halogen atom(s), silyl group(s), or aryl group(s) such as a trifluoromethylsulfonyl, trisilylethanesulfonyl or benzylsulfonyl group.

The protective group for a hydroxyl group of R^{2a} and R^{3a} is not particulary restricted provided that it can usually protect a hydroxyl group in chemical reactions, and specifically indicates a protective group that can be cleaved by a chemical process such as hydrogenolysis, hydrolysis, electrolysis and photolysis. Such protective group can be, for example, the above "aliphatic acyl group"; the above "aromatic acyl group"; the above "carbonyloxyalkyl group"; the above "residual group of a salt of a succinic acid half-ester"; the above "residual group of a salt of a phosphoric acid ester"; the above "residual group forming an amino acid ester"; a carbamoyl group; a carbamoyl group substituted with 1 or 2 lower alkyl groups; the above "carbonyloxyalkyloxycarbonyl group"; or the above "silyl group", and is preferably a group which forms a pharmacologically acceptable ester such as an "aliphatic acyl group", an "aromatic acyl group" or a "silyl group", more preferably a "C1-C6 alkanoyl group" such as an acetyl, propionyl, butyryl, isobutyryl, pentanoyl, or pivaloyl group, or a "silyl group" such as a t-butyldimethylsilyl or t-butyldiphenylsilyl group, and particularly preferably an acetyl group, a t-butyldimethylsilyl group or a t-butyldiphenylsilyl group.

The leaving group of Lv is not particularly restricted provided that this group is a functional group that can achieve displacement reaction by reacting with a nucleophilic substituent, and can be, for example, the above "halogen atom"; a "lower alkylsulfonyloxy group" such as a methanesulfonyloxy or ethanesulfonyloxy group; a "halogenated lower alkylsulfonyloxy group" such as a trifluoromethanesulfonyloxy group; or an "aromatic sulfonyloxy group" including an arylsulfonyloxy group such as a benzenesulfonyloxy group, a lower alkylated arylsulfonyloxy group such as a p-toluenesulfonyloxy group, a halogenated arylsulfonyloxy group such as a p-chlorobenzenesulfonyloxy group and a nitrated arylsulfonyloxy group such as a p-nitrobenzenesulfonyloxy group.

Each step of Process A and Process B is hereinafter described in detail.

### (Process A)

Process A is a step for the preparation of compound (I) by conducting a nucleophilic displacement reaction in the presence of a base using compound (II) obtainable by Process C and a compound (III) that is well known or easily prepared from known compounds (A-1). Furthermore, if necessary, several reactions such as deprotection of a protective group of a sulfanyl group (A-2), introduction of a substituent onto a sulfanyl group (A-3), hydrolysis of an ester group (A-4), conversion of a carboxy group into an amide group (A-5), conversion of a carboxy group into an ester group (A-6), deprotection of a protective group for an amino group (A-7), conversion of a carboxy group into a hydroxyamino group (A-8) and conversion of a R^{3a} group including a hydroxyl group into a R³ group (A-9) can also be carried out. The steps from A-2 to A-9 can be conducted either before or after the step A-1, and the order of these steps can easily be selected according to circumstances by a person having ordinary skill in the art.

### (A-1)

The solvent employed can be, for example, an aromatic hydrocarbon such as benzene, toluene or xylene; a halogenated hydrocarbon such as dichloromethane, chloroform, carbon tetrachloride, dichloroethane, chlorobenzene or dichlorobenzene; an ether such as diethyl ether, diisopropyl ether, tetrahydrofuran, dioxane, dimethoxyethane or di(ethylene glycol) dimethyl ether; a ketone such as acetone, methyl ethyl ketone, methyl isobutyl ketone, isophorone or cyclohexanone; a nitrile such as acetonitrile or isobutyronitrile; an amide such as formamide, N,N-dimethylformamide, N,N-dimethylacetamide, N-methyl-2-pyrrolidinone or hexamethylphosphoric triamide; or a sulfoxide such as dimethyl sulfoxide or sulfolane, and is preferably a halogenated hydrocarbon, a nitrile or an amide, and more preferably N,N-dimethylformamide, N,N-dimethylacetamide, acetonitrile or dichloromethane.

The base employed can be, for example, an inorganic base, including an alkali metal carbonate such as sodium carbonate, potassium carbonate or lithium carbonate; an alkali metal hydrogencarbonate such as sodium hydrogencarbonate, potassium hydrogencarbonate or lithium hydrogencarbonate; an alkali metal hydride such as lithium hydride, sodium hydride or potassium hydride; an alkali metal hydroxide such as sodium hydroxide, potassium hydroxide or lithium hydroxide; and an alkali metal fluoride such as sodium fluoride or potassium fluoride; an alkali metal alkoxide such as sodium methoxide, sodium ethoxide, potassium methoxide, potassium ethoxide, potassium t-butoxide or lithium methoxide; an alkali metal mercaptan such as sodium methylmercaptan or sodium ethylmercaptan; or an organic base such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) or 1,8-diazabicyclo[5.4.0]undec-7-ene (DBU), and is preferably an alkali metal carbonate or an organic base, and more preferably potassium carbonate or triethylamine.

The reaction temperature varies depending on the starting material and the reagent, and is between -50°C and 100°C, and preferably between 0°C and 50°C.

The reaction time varies depending on the reaction temperature, the starting material, the reagent and the kind of solvent employed, and is generally from 30 minutes to 48 hours, and preferably from 1 hour to 24 hours.

After completion of the reaction, the desired compound of this reaction can be obtained, for example, by concentration of the reaction mixture, addition to the mixture of an organic solvent immiscible with water such as ethyl acetate, washing the mixture with water, separation of the organic layer containing the desired compound, drying the organic layer over anhydrous magnesium sulfate or the like, and then evaporation of the organic solvent to give the desired product.

The obtained product, if necessary, is further purified by conventional treatments, for example, by recrystallization, reprecipitation, silica gel column chromatography or the like.

### (A-2)

This step is a step for the deprotection of a protective group for a sulfanyl group, and can easily be achieved according to procedures that are well known by a person having skill in the art (for example, the procedure described in Protective Groups in Organic Synthesis, Third Edition, T. W. Green, et al., John Wiley & Sons, Inc. (1999)), and is preferably carried out by a method for deprotection in the presence of an acid (A-2a) or a method for deprotection in the presence of a base (A-2b).

### (A-2a)

The solvent employed can be, for example, an aromatic hydrocarbon such as benzene, toluene or xylene; a halogenated hydrocarbon such as dichloromethane, chloroform, carbon tetrachloride, dichloroethane, chlorobenzene or dichlorobenzene; an ester such as ethyl formate, ethyl acetate, propyl acetate, butyl acetate or diethyl carbonate; an ether such as diethyl ether, diisopropyl ether, tetrahydrofuran, dioxane, dimethoxyethane or di(ethylene glycol) dimethyl ether; an alcohol such as methanol, ethanol, n-propanol, isopropanol, n-butanol, isobutanol, t-butanol, isoamyl alcohol, diethylene glycol, glycerol, octanol, cyclohexanol or 2-methoxyethanol; an amide such as formamide, N,N-dimethylformamide, N,N-dimethylacetamide, N-methyl-2-pyrrolidinone or hexamethylphosphoric triamide; a sulfoxide such as dimethyl sulfoxide or sulfolane; or a mixture of the above solvents, and is preferably an alcohol or a mixture solvent of a halogenated hydrocarbon and an alcohol, and more preferably methanol, ethanol or a mixture solvent of dichloromethane and methanol or ethanol.

The acid employed is not particularly restricted provided that it can be used in general reactions, and can be preferably an inorganic acid such as hydrochloric acid, hydrogen chloride, hydrobromic acid, sulfuric acid, perchloric acid or phosphoric acid; or an organic acid such as acetic acid, formic acid, oxalic acid, methanesulfonic acid, p-toluenesulfonic acid, camphorsulfonic acid, trifluoroacetic acid or trifluoromethanesulfonic acid, and is preferably an inorganic acid, and more preferably hydrogen chloride or hydrochloric acid.

The reaction temperature varies depending on the starting material and the reagent, and is between -50°C and 100°C, and preferably between 0°C and 50°C.

The reaction time varies depending on the reaction temperature, the starting material, the reagent and the kind of solvent employed, and is generally from 30 minutes to 48 hours, and preferably from 1 hour to 24 hours.

After completion of the reaction, the desired compound of this reaction can be obtained, for example, by concentration of the reaction mixture; addition to the mixture of an organic solvent immiscible with water such as ethyl acetate, washing the mixture with water, separation of the organic layer containing the desired compound, drying the organic layer over anhydrous magnesium sulfate or the like, and then evaporation of the organic solvent to give the desired product.

The obtained product, if necessary, is further purified by conventional treatments, for example, by recrystallization, reprecipitation, silica gel column chromatography or the like.

### (A-2b)

The solvent employed can be, for example, an aromatic hydrocarbon such as benzene, toluene or xylene; a halogenated hydrocarbon such as dichloromethane, chloroform, carbon tetrachloride, dichloroethane, chlorobenzene or dichlorobenzene; an ether such as diethyl ether, diisopropyl ether, tetrahydrofuran, dioxane, dimethoxyethane or di(ethylene glycol) dimethyl ether; an alcohol such as methanol, ethanol, n-propanol, isopropanol, n-butanol, isobutanol, t-butanol, isoamyl alcohol, diethylene glycol, glycerol, octanol, cyclohexanol or 2-methoxyethanol; an amide such as formamide, N,N-dimethylformamide, N,N-dimethylacetamide, N-methyl-2-pyrrolidinone or hexamethylphosphoric triamide; a sulfoxide such as dimethyl sulfoxide or sulfolane; water; or a mixture of the above solvents, and is preferably an alcohol or a mixed solvent of an alcohol and water, and more preferably methanol, ethanol, methanol containing water or ethanol containing water.

The base employed can be, for example, an inorganic base, including an alkali metal carbonate such as sodium carbonate, potassium carbonate or lithium carbonate; an alkali metal hydrogencarbonate such as sodium hydrogencarbonate, potassium hydrogencarbonate or lithium hydrogencarbonate; an alkali metal hydride such as lithium hydride, sodium hydride or potassium hydride; an alkali metal hydroxide such as sodium hydroxide, potassium hydroxide or lithium hydroxide; and an alkali metal fluoride such as sodium fluoride or potassium fluoride; an alkali metal alkoxide such as sodium methoxide, sodium ethoxide, potassium methoxide, potassium ethoxide, potassium t-butoxide or lithium methoxide; an alkali metal mercaptan such as sodium methylmercaptan or sodium ethylmercaptan; or an organic base such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) or 1,8-diazabicyclo[5.4.0]undec-7-ene (DBU), and is preferably an alkali metal carbonate or an alkali metal hydroxide, and more preferably potassium carbonate or sodium hydroxide.

The reaction temperature varies depending on the starting material and the reagent, and is between -50°C and 100°C, and preferably between -20°C and 50°C.

The reaction time varies depending on the reaction temperature, the starting material, the reagent and the kind of solvent employed, and is generally from 1 minute to 24 hours, and preferably from 5 minutes to 5 hours.

After completion of the reaction, the desired compound of this reaction can be obtained, for example, by concentration of the reaction mixture, addition to the mixture of an organic solvent immiscible with water such as ethyl acetate, washing the mixture with water, separation of the organic layer containing the desired compound, drying the organic layer over anhydrous magnesium sulfate or the like, and then evaporation of the organic solvent to give the desired product.

The obtained product, if necessary, is further purified by conventional treatments, for example, by recrystallization, reprecipitation, silica gel column chromatography or the like.

### (A-3)

This step is a step for introducing a substituent onto a sulfanyl group in the presence of a base or the like. When an acid chloride, an acid anhydride, a sulfanyl halide or an active ester is employed as a reagent, this step is carried out in the presence of a base (A-3a), when a carboxylic acid is employed, this step is carried out in the presence of a condensation agent (A-3b), and when a thiol is employed, this step is carried out in the presence of iodine or a base (A-3c).

### (A-3a)

The solvent employed can be, for example, an aromatic hydrocarbon such as benzene, toluene or xylene; a halogenated hydrocarbon such as dichloromethane, chloroform, carbon tetrachloride, dichloroethane, chlorobenzene or dichlorobenzene; an ester such as ethyl formate, ethyl acetate, propyl acetate, butyl acetate or diethyl carbonate; an ether such as diethyl ether, diisopropyl ether, tetrahydrofuran, dioxane, dimethoxyethane or di(ethylene glycol) dimethyl ether; a ketone such as acetone, methyl ethyl ketone, methyl isobutyl ketone, isophorone or cyclohexanone; an amide such as formamide, N,N-dimethylformamide, N,N-dimethylacetamide, N-methyl-2-pyrrolidinone or hexamethylphosphoric triamide; or a sulfoxide such as dimethyl sulfoxide or sulfolane, and is preferably a halogenated hydrocarbon, a ketone or an amide, and more preferably N,N-dimethylformamide, N,N-dimethylacetamide, dichloromethane or acetone.

The base employed can be, for example, an inorganic base, including an alkali metal carbonate such as sodium carbonate, potassium carbonate or lithium carbonate; an alkali metal hydrogencarbonate such as sodium hydrogencarbonate, potassium hydrogencarbonate or lithium hydrogencarbonate; an alkali metal hydride such as lithium hydride, sodium hydride or potassium hydride; an alkali metal hydroxide such as sodium hydroxide, potassium hydroxide or lithium hydroxide; and an alkali metal fluoride such as sodium fluoride or potassium fluoride; an alkali metal alkoxide such as sodium methoxide, sodium ethoxide, potassium methoxide, potassium ethoxide, potassium t-butoxide or lithium methoxide; an alkali metal mercaptan such as sodium methylmercaptan or sodium ethylmercaptan; or an organic base such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) or 1,8-diazabicyclo[5.4.0]undec-7-ene (DBU), and is preferably an alkali metal carbonate, an alkali metal hydride or an organic base, and more preferably sodium hydride, potassium carbonate or triethylamine.

The reaction temperature varies depending on the starting material and the reagent, and is between -30°C and 100°C, and preferably between -10°C and 50°C.

The reaction time varies depending on the reaction temperature, the starting material, the reagent and the kind of solvent employed, and is generally from 5 minutes to 24 hours, and preferably from 15 minutes to 10 hours.

After completion of the reaction, the desired compound of this reaction can be obtained, for example, by concentration of the reaction mixture, addition to the mixture of an organic solvent immiscible with water such as ethyl acetate, washing the mixture with water, separation of the organic layer containing the desired compound, drying the organic layer over anhydrous magnesium sulfate or the like, and then evaporation of the organic solvent to give the desired product.

The obtained product, if necessary, is further purified by conventional treatments, for example, by recrystallization, reprecipitation, silica gel column chromatography or the like.

### (A-3b)

The solvent employed can be, for example, an aromatic hydrocarbon such as benzene, toluene or xylene; a halogenated hydrocarbon such as dichloromethane, chloroform, carbon tetrachloride, dichloroethane, chlorobenzene or dichlorobenzene; an ester such as ethyl formate, ethyl acetate, propyl acetate, butyl acetate or diethyl carbonate; an ether such as diethyl ether, diisopropyl ether, tetrahydrofuran, dioxane, dimethoxyethane or di(ethylene glycol) dimethyl ether; a ketone such as acetone, methyl ethyl ketone, methyl isobutyl ketone, isophorone or cyclohexanone; an amide such as formamide, N,N-dimethylformamide, N,N-dimethylacetamide, N-methyl-2-pyrrolidinone or hexamethylphosphoric triamide; or a sulfoxide such as dimethyl sulfoxide or sulfolane, and is preferably a halogenated hydrocarbon or an amide, and more preferably N,N-dimethylformamide, N,N-dimethylacetamide or dichloromethane.

The "condensation agent" employed can be,
(i) a combination of a phosphate ester such as diethylphosphoryl cyanide, diphenylphosphoryl azide or diethyl cyanophosphonate and a base shown below;
(ii) a carbodiimide such as 1,3-dicyclohexylcarbodiimide (DCC), 1,3-diisopropylcarbodiimide or 1-(3-dimethylaminopropyl)-3-ethylcarbodiimide (WSC); a combination of said carbodiimide and a base shown below; a combination of said carbodiimide and an N-hydroxyimide such as N-hydroxysuccinimide, 1-hydroxybenzotriazole or N-hydroxy-5-norbomene-2,3-dicarboxyimide; and a combination of said carbodiimide, said N-hydroxyimide and a base shown below;
(iii) a combination of a disulfide such as 2,2'-dipyridyl disulfide or 2,2'-dibenzothiazolyl disulfide and a phosphine such as triphenylphosphine or tributylphosphine;
(iv) a carbonate such as N,N'-disuccinimidyl carbonate, diethyl pyrocarbonate (DEPC), di-2-pyridyl carbonate or S,S'-bis(1-phenyl-1H-tetrazol-5-yl)dithiocarbonate;
(v) a phosphinic chloride such as N,N'-bis(2-oxo-3-oxazolidinyl)phosphinic chloride;
(vi) an oxalate such as N,N'-disuccinimidyl oxalate, N,N'-diphthalimide oxalate, N,N'-bis(5-norbornene-2,3-dicarboxyimidyl) oxalate, 1,1'-bis(benzotriazolyl) oxalate, 1,1'-bis(6-chlorobenzotriazolyl) oxalate or 1,1'-bis(6-trifluoromethylbenzotriazolyl) oxalate;
(vii) a combination of said phosphine and azodicarboxylate or an azodicarboxyamide such as diethyl azodicarboxylate or 1,1'-(azodicarbonyl)dipiperidine;
(viii) an N-lower alkyl-5-arylisoxazolium-3'-sulfonate such as N-ethyl-5-phenylisoxazolium-3'-sulfonate;
(ix) a di-heteroaryl diselenide such as di-2-pyridyl diselenide;
(x) an arylsulfonyl triazolide such as p-nitrobenzenesulfonyl triazolide;
(xi) a 2-halo-1-lower alkylpyridinium halide such as 2-chloro-1-methylpyridinium iodide;
(xii) an imidazole such as 1,1'-oxalyldiimidazole or N,N'-carbonyldiimidazole (CDI);
(xiii) a 3-lower alkyl-2-halogen-benzothiazolium fluoroborate such as 3-ethyl-2-chloro-benzothiazolium fluoroborate;
(xiv) a 3-lower alkyl-benzothiazole-2-selone such as 3-methyl-benzothiazole-2-selone;
(xv) a phosphate such as phenyl dichlorophosphate or polyphosphate ester;
(xvi) a halogenosulfonyl isocyanate such as chlorosulfonyl isocyanate;
(xvii) a halogenosilane such as trimethylsilyl chloride or triethylsilyl chloride;
(xviii) a combination of a lower alkanesulfonyl halide such as methanesulfonyl chloride and a base shown below;
(xix) an N,N,N',N'-tetra-lower alkylhalogenoformamidium chloride such as N,N,N',N'-tetramethylchloroformamidium chloride; or
(xx) a combination of a pyridinium salt (Mukaiyama reagent) such as 1-methyl-2-chloropyridinium chloride or 1-ethyl-2-bromopyridinium chloride and a base shown below,
and is preferably a carbodiimide, and more preferably 1-(3-dimethylaminopropyl)-3-ethylcarbodiimide or 1,3-dicyclohexylcarbodiimide.

The base employed can be, for example, an organic base such as pyrrolidine, piperidine; morpholene, N-methylmorpholine, triethylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline or N,N-diethylaniline.

The reaction temperature varies depending on the starting material and the reagent, and is between -30°C and 100°C, and preferably between -10°C and 50°C.

The reaction time varies depending on the reaction temperature, the starting material, the reagent and the kind of solvent employed, and is generally from 5 minutes to 24 hours, and preferably from 30 minutes to 10 hours.

After completion of the reaction, the desired compound of this reaction can be obtained, for example, by concentration of the reaction mixture; addition to the mixture of an organic solvent immiscible with water such as ethyl acetate, washing the mixture with water, separation of the organic layer containing the desired compound, drying the organic layer over anhydrous magnesium sulfate or the like, and then evaporation of the organic solvent to give the desired product.

The obtained compound, if necessary, is further purified by conventional treatments, for example, by recrystallization, reprecipitation, silica gel column chromatography or the like.

### (A-3c)

The solvent employed can be, for example, an aromatic hydrocarbon such as benzene, toluene or xylene; a halogenated hydrocarbon such as dichloromethane, chloroform, carbon tetrachloride, dichloroethane, chlorobenzene or dichlorobenzene; an ester such as ethyl formate, ethyl acetate, propyl acetate, butyl acetate or diethyl carbonate; an ether such as diethyl ether, diisopropyl ether, tetrahydrofuran, dioxane, dimethoxyethane or di(ethylene glycol) dimethyl ether; a ketone such as acetone, methyl ethyl ketone, methyl isobutyl ketone, isophorone or cyclohexanone; an amide such as formamide, N,N-dimethylformamide, N,N-dimethylacetamide, N-methyl-2-pyrrolidinone or hexamethylphosphoric triamide; or a sulfoxide such as dimethyl sulfoxide or sulfolane, and is preferably an ether, and more preferably tetrahydrofuran.

The reaction temperature varies depending on the starting material and the reagent, and is between -30°C and 100°C, and preferably between -10°C and 50°C.

The reaction time varies depending on the reaction temperature, the starting material, the reagent and the kind of solvent employed, and is generally from 1 minute to 5 hours, and preferably from 5 minutes to 1 hour.

After completion of the reaction, the desired compound of this reaction can be obtained, for example, by concentration of the reaction mixture; addition to the mixture of an organic solvent immiscible with water such as ethyl acetate, washing the mixture with water, separation of the organic layer containing the desired compound, drying the organic layer over anhydrous magnesium sulfate or the like, and then evaporation of the organic solvent to give the desired product.

The obtained product, if necessary, is further purified by conventional treatments, for example, by recrystallization, reprecipitation, silica gel column chromatography or the like.

### (A-4)

This step is achieved by hydrolysis of an ester group (A-4).

This step is carried out in a similar manner to that indicated hereinbefore (A-2).

### (A-5)

This step is achieved by converting a carboxy group obtained in Step A-4 into an active ester, followed by reacting said active ester with a desired amino compound.

The solvent employed can be, for example, an aromatic hydrocarbon such as benzene, toluene or xylene; a halogenated hydrocarbon such as dichloromethane, chloroform, carbon tetrachloride, dichloroethane, chlorobenzene or dichlorobenzene; an ester such as ethyl formate, ethyl acetate, propyl acetate, butyl acetate or diethyl carbonate; an ether such as diethyl ether, diisopropyl ether, tetrahydrofuran, dioxane, dimethoxyethane or di(ethylene glycol) dimethyl ether; a ketone such as acetone, methyl ethyl ketone, methyl isobutyl ketone, isophorone or cyclohexanone; an amide such as formamide, N,N-dimethylformamide, N,N-dimethylacetamide, N-methyl-2-pyrrolidinone or hexamethylphosphoric triamide; or a sulfoxide such as dimethyl sulfoxide or sulfolane, and is preferably a halogenated hydrocarbon or an amide, and more preferably N,N-dimethylformamide, N,N-dimethylacetamide or dichloromethane.

The reagent employed is not particularly restricted provided that it is used for an active ester formation, and, for example, can be isobutyl chloroformate.

The base employed can be, for example, an inorganic base, including an alkali metal carbonate such as sodium carbonate, potassium carbonate or lithium carbonate; an alkali metal hydrogencarbonate such as sodium hydrogencarbonate, potassium hydrogencarbonate or lithium hydrogencarbonate; an alkali metal hydride such as lithium hydride, sodium hydride or potassium hydride; an alkali metal hydroxide such as sodium hydroxide, potassium hydroxide or lithium hydroxide; and an alkali metal fluoride such as sodium fluoride or potassium fluoride; an alkali metal alkoxide such as sodium methoxide, sodium ethoxide, potassium methoxide, potassium ethoxide, potassium t-butoxide or lithium methoxide; an alkali metal mercaptan such as sodium methylmercaptan or sodium ethylmercaptan; or an organic base such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) or 1,8-diazabicyclo[5.4.0]undec-7-ene (DBU), and is preferably an alkali metal carbonate or an organic base, and more preferably potassium carbonate or triethylamine.

The reaction temperature varies depending on the starting material and the reagent, and is between -30°C and 100°C, and preferably between -10°C and 50°C.

The reaction time varies depending on the reaction temperature, the starting material, the reagent and the kind of solvent employed, and is generally from 10 minutes to 24 hours, and preferably from 30 minutes to 10 hours.

After completion of the reaction, the desired compound of this reaction can be obtained, for example, by concentration of the reaction mixture, addition to the mixture of an organic solvent immiscible with water such as ethyl acetate, washing the mixture with water, separation of the organic layer containing the desired compound; drying the organic layer over anhydrous magnesium sulfate or the like; and then evaporation of the organic solvent to give the desired product.

The obtained product, if necessary, is further purified by conventional treatments, for example, by recrystallization, reprecipitation, silica gel column chromatography or the like.

### (A-6)

This step is carried out by conducting a dehydrating condensation reaction between a carboxylic acid obtained in Step A-4 and an alcohol (A-6a) or by conducting an ester interchange reaction using an ester in the presence of an acid or a base directly without performing the process of Step A-4 (A-6b).

### (A-6a)

The solvent employed can be, for example, an aromatic hydrocarbon such as benzene, toluene or xylene; a halogenated hydrocarbon such as dichloromethane, chloroform, carbon tetrachloride, dichloroethane, chlorobenzene or dichlorobenzene; an ether such as diethyl ether, diisopropyl ether, tetrahydrofuran, dioxane, dimethoxyethane or di(ethylene glycol) dimethyl ether; an alcohol such as methanol, ethanol, n-propanol, isopropanol, n-butanol, isobutanol, t-butanol, isoamyl alcohol, diethylene glycol, glycerol, octanol, cyclohexanol or 2-methoxyethanol; an amide such as formamide, N,N-dimethylformamide, N,N-dimethylacetamide, N-methyl-2-pyrrolidinone or hexamethylphosphorotriamide; a sulfoxide such as dimethyl sulfoxide or sulfolane; or a mixed solvent of an alcohol and the above solvent. However, when an alcohol is employed as a solvent in the above reaction, an addition of the alcohol as a reagent is not necessary.

The acid employed can be, for example, an inorganic acid such as hydrochloric acid, hydrogen chloride, hydrobromic acid, sulfuric acid, perchloric acid or phosphoric acid; or a sulfonic acid such as methanesulfonic acid, p-toluenesulfonic acid or camphorsulfonic acid, and is preferably an inorganic acid, and more preferably hydrogen chloride.

The reaction temperature varies depending on the starting material and the reagent, is between -30°C and 100°C, and preferably between -10°C and 50°C.

The reaction time varies depending on the reaction temperature, the starting material, the reagent and the kind of solvent employed, and is generally from 10 minutes to 24 hours, and preferably from 30 minutes to 10 hours.

After completion of the reaction, the desired compound of this reaction can be obtained, for example, by concentration of the reaction mixture, addition to the mixture of an organic solvent immiscible with water such as ethyl acetate, washing the mixture with water, separation of the organic layer containing the desired compound; drying the organic layer over anhydrous magnesium sulfate or the like; and then evaporation of the organic solvent to give the desired product.

The obtained product, if necessary, is further purified by conventional treatments, for example, by recrystallization, reprecipitation, silica gel column chromatography or the like.

This step is carried out in a similar manner to that indicated hereinbefore in (A-3b).

### (A-6b)

In this step, an alcohol corresponding to the desired alkoxy group is employed as a solvent. When an acid is employed, such acid employed can be, for example, an inorganic acid such as hydrochloric acid, hydrogen chloride, hydrobromic acid, sulfuric acid, perchloric acid or phosphoric acid; or a sulfonic acid such as methanesulfonic acid, p-toluenesulfonic acid or camphorsulfonic acid, and is preferably an inorganic acid, and more preferably hydrogen chloride.

When a base is employed, such base employed can be, for example, an inorganic base, including an alkali metal carbonate such as sodium carbonate, potassium carbonate or lithium carbonate; an alkali metal hydrogencarbonate such as sodium hydrogencarbonate, potassium hydrogencarbonate or lithium hydrogencarbonate; an alkali metal hydride such as lithium hydride, sodium hydride or potassium hydride; an alkali metal hydroxide such as sodium hydroxide, potassium hydroxide or lithium hydroxide; or an alkali metal fluoride such as sodium fluoride or potassium fluoride, and is preferably an alkali metal carbonate, and more preferably potassium carbonate.

The reaction temperature varies depending on the starting material and the reagent, and is between -30°C and 100°C, and preferably between -10°C and 50°C.

The reaction time varies depending on the reaction temperature, the starting material, the reagent and the kind of solvent employed, and is generally from 10 minutes to 24 hours, and preferably from 30 minutes to 10 hours.

After completion of the reaction, the desired compound of this reaction can be obtained, for example, by concentration of the reaction mixture, addition to the mixture of an organic solvent immiscible with water such as ethyl acetate, washing the mixture with water, separation of the organic layer containing the desired compound, drying the organic layer over anhydrous magnesium sulfate or the like, and then evaporation of the organic solvent to give the desired product.

The obtained compound, if necessary, is further purified by conventional treatments, for example, by recrystallization, reprecipitation, silica gel column chromatography or the like.

### (A-7)

This step is carried out in a similar manner to that indicated hereinbefore in Step A-2. When an acid is employed, trifluoroacetic acid or hydrogen chloride is employed as a particularly preferred reagent.

When the desired compound is obtained as its geometrical isomer, the desired compound can be obtained by conducting the photoisomerization reaction shown below.

The solvent employed can be, for example, an ester such as ethyl formate, ethyl acetate, propyl acetate, butyl acetate or diethyl carbonate; an ether such as diethyl ether, diisopropyl ether, tetrahydrofuran, dioxane, dimethoxyethane or di(ethylene glycol) dimethyl ether; an alcohol such as methanol, ethanol, n-propanol, isopropanol, n-butanol, isobutanol, t-butanol, isoamyl alcohol, diethylene glycol, glycerol, octanol, cyclohexanol or methyl cellosolve; a nitrile such as acetonitrile or isobutyronitrile; an amide such as formamide, N,N-dimethylformamide, N,N-dimethylacetamide, N-methyl-2-pyrroridone, N-methylpyrrolidinone or hexamethylphosphorotriamide; a sulfoxide such as dimethyl sulfoxide or sulfolane; water; or a mixture of these solvents, and is preferably water, an alcohol, a nitrile or a mixture of these solvents.

The light source employed is a low-pressure mercury lamp (having 20 W to 100 W, preferably 32 W).

The sensitizer employed can be, for example, benzophenone, fluorenone or anthraquinone.

This reaction can also be carried out by addition of an organic sulfur compound such as dimethyl disulfide, diethyl disulfide or diphenyl disulfide in order to accelerate the reaction.

The reaction temperature varies depending on the starting material and the reagent, and is between -20°C and 100°C, and preferably between 0°C and 50°C.

The reaction time varies depending on the reaction temperature, the starting material, the reagent and the kind of solvent employed, and is generally from 5 minutes to 8 hours, and preferably from 10 minutes to 3 hours.

After completion of the reaction, the desired compound of this reaction can be obtained, for example, by concentration of the reaction mixture, addition to the mixture of an organic solvent immiscible with water such as ethyl acetate, washing the mixture with water, separation of the organic layer containing the desired compound, drying the organic layer over anhydrous magnesium sulfate or the like, and then evaporation of the organic solvent to give the desired product.

The obtained product, if necessary, is further purified by conventional treatments, for example, by recrystallization, reprecipitation, silica gel column chromatography or the like.

### (A-8)

This step is a step for the conversion of a carboxy group into a hydroxylamino group, and can easily be carried out according to procedures that are well known by a person having ordinary skill in the art (for example, methods disclosed in A. Sekar Reddy, M. Suresh Kumar and G. Rabindra Reddy: Tetrahedron Letters, (2000), 41, 6285-6288).

### (A-9)

This step can be carried out according to a Mitsunobu reaction using an amine in an inert solvent or by conversion of a hydroxyl group into a leaving group followed by conducting a displacement reaction using an amine, and carried out in a similar manner to that indicated hereinunder (C-2e).

### (Process B)

Process B is steps comprising the preparation of compound (V) by conducting a nucleophilic displacement reaction in the presence of a base using a compound (III) that are well known or easily prepared from known compounds and the compounds (IV) obtained by the Process C described hereinafter (B-1) and the preparation of compound (I) by conversion of a hydroxyl group of the compound (V) (B-2). Furthermore, if necessary, in Process B, deprotection of the protective group for a sulfanyl group (B-3), introduction of a substituent onto a sulfanyl group (B-4), hydrolysis of an ester group (B-5), conversion of a carboxy group into an amide group (B-6), conversion of a carboxy group into an ester group (B-7), deprotection of a protective group for an amino group (B-8) and conversion of a carboxy group into a hydroxyamino group (B-9) can be also carried out. The steps from B-3 to B-9 can be conducted either before or after the steps B-1 and B-2, and the order of these steps can be selected according to circumstances by a person having ordinary skill in the art.

### (B-1)

The solvent employed can be, for example, an aromatic hydrocarbon such as benzene, toluene or xylene; a halogenated hydrocarbon such as dichloromethane, chloroform, carbon tetrachloride, dichloroethane, chlorobenzene or dichlorobenzene; an ester such as ethyl formate, ethyl acetate, propyl acetate, butyl acetate or diethyl carbonate; an ether such as diethyl ether, diisopropyl ether, tetrahydrofuran, dioxane, dimethoxyethane or di(ethylene glycol) dimethyl ether; a ketone such as acetone, methyl ethyl ketone, methyl isobutyl ketone, isophorone or cyclohexanone; an amide such as formamide, N,N-dimethylformamide, N,N-dimethylacetamide, N-methyl-2-pyrrolidinone or hexamethylphosphoric triamide; or a sulfoxide such as dimethyl sulfoxide or sulfolane, and is preferably a halogenated hydrocarbon or an amide, and more preferably N,N-dimethylformamide, N,N-dimethylacetamide or dichloromethane.

The base employed can be, for example, an inorganic base, including an alkali metal carbonate such as sodium carbonate, potassium carbonate or lithium carbonate; an alkali metal hydrogencarbonate such as sodium hydrogencarbonate, potassium hydrogencarbonate or lithium hydrogencarbonate; an alkali metal hydride such as lithium hydride, sodium hydride or potassium hydride; an alkali metal hydroxide such as sodium hydroxide, potassium hydroxide or lithium hydroxide; and an alkali metal fluoride such as sodium fluoride or potassium fluoride; an alkali metal alkoxide such as sodium methoxide, sodium ethoxide, potassium methoxide, potassium ethoxide, potassium t-butoxide or lithium methoxide; an alkali metal mercaptan such as sodium methylmercaptan or sodium ethylmercaptan; or an organic base such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di-(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) or 1,8-diazabicyclo[5.4.0]undec-7-ene (DBU), and is preferably an alkali metal carbonate or an organic base, and more preferably potassium carbonate or triethylamine.

The reaction temperature varies depending on the starting material and the reagent, and is between -30°C and 100°C, and preferably between -10°C and 50°C.

The reaction time varies depending on the reaction temperature, the starting material, the reagent and the kind of solvent employed, and is generally from 10 minutes to 48 hours, and preferably from 30 minutes to 24 hours.

After completion of the reaction, the desired compound of this reaction can be obtained, for example, by concentration of the reaction mixture, addition to the mixture of an organic solvent immiscible with water such as ethyl acetate, washing the mixture with water, separation of the organic layer containing the desired compound, drying of the organic layer over anhydrous magnesium sulfate or the like, and then evaporation of the organic solvent to give the desired product.

The obtained product, if necessary, is further purified by conventional treatments, for example, by recrystallization, reprecipitation, silica gel column chromatography or the like.

### (B-2)

This step is carried out by the Mitsunobu reaction in an inert solvent (B-2a), by a reaction in the presence of an amide-acetal reagent (B-2b), by converting a hydroxyl group into a leaving group and conducting a substitution reaction using Pro¹-SM (M represents an alkali metal, preferably potassium) (B-2c) or by converting a hydroxyl group into a leaving group and conducting a substitution reaction using Pro¹-SM in the presence of a palladium catalyst and a phosphine (B-2d).

### (B-2a)

The solvent employed can be, for example, an aromatic hydrocarbon such as benzene, toluene or xylene; a halogenated hydrocarbon such as dichloromethane, chloroform, carbon tetrachloride, dichloroethane, chlorobenzene or dichlorobenzene; or an ether such as diethyl ether, diisopropyl ether, tetrahydrofuran, dioxane, dimethoxyethane or di(ethylene glycol) dimethyl ether, and is preferably a halogenated hydrocarbon or an ether, and more preferably dichloromethane or tetrahydrofuran.

The reagent employed in the Mitsunobu reaction is not particularly restricted provided that it can generally be used for the Mitsunobu reaction, and, for example, is preferably a combination of an azo compound, including a di-lower-alkyl azodicarboxylate such as diethyl azodicarboxylate or diisopropyl azodicarboxylate or a heterocyclyl azodicarbonyl such as 1,1'-(azodicarbonyl)dipiperidine, and a phosphine, including a triarylphosphine such as triphenylphosphine or a tri-lower-alkylphosphine such as tri-n-butylphosphine, and more preferably a combination of a di-lower-alkyl azodicarboxylate and a triarylphosphine, and most preferably a combination of diethyl azodicarboxylate or diisopropyl azodicarboxylate and triphenylphosphine.

The reaction temperature varies depending on the starting material and the reagent, and is between -50°C and 100°C, and preferably between -10°C and 60°C.

The reaction time varies depending on the reaction temperature, the starting material, the reagent and the kind of solvent employed, and is generally from 10 minutes to 48 hours, and preferably from 30 minutes to 24 hours.

After completion of the reaction, the desired compound of this reaction can be obtained, for example, by concentration of the reaction mixture, addition to the mixture of an organic solvent immiscible with water such as ethyl acetate, washing the mixture with water, separation of the organic layer containing the desired compound, drying of the organic layer over anhydrous magnesium sulfate or the like, and then evaporation of the organic solvent to give the desired product.

The obtained product, if necessary, is further purified by conventional treatments, for example, by recrystallization, reprecipitation, silica gel column chromatography or the like.

### (B-2b)

The solvent employed can be, for example, an aromatic hydrocarbon such as benzene, toluene or xylene; a halogenated hydrocarbon such as dichloromethane, chloroform, carbon tetrachloride, dichloroethane, chlorobenzene or dichlorobenzene; an ether such as diethyl ether, diisopropyl ether, tetrahydrofuran, dioxane, dimethoxyethane or di(ethylene glycol) dimethyl ether; or an amide such as formamide, N,N-dimethylformamide, N,N-dimethylacetamide, N-methyl-2-pyrrolidinone or hexamethylphosphoric triamide, and is preferably an aromatic hydrocarbon, and more preferably toluene.

The amide acetal reagent employed can be, for example, a group of general formula (CH₃)₂NCH(OR')₂ (wherein R' represents a C₁-C₆ alkyl group or a C₇-C₁₅ aralkyl group), and is preferably N,N-dimethylformamide dineopentyl acetal.

The reaction temperature varies depending on the starting material and the reagent, and is between -50°C and 150°C, and preferably between -10°C and 120°C.

The reaction time varies depending on the reaction temperature, the starting material, the reagent and the kind of solvent employed, and is generally from 1 minute to 24 hours, and preferably from 5 minutes to 5 hours.

After completion of the reaction, the desired compound of this reaction can be obtained, for example, by concentration of the reaction mixture, addition to the mixture of an organic solvent immiscible with water such as ethyl acetate, washing the mixture with water, separation of the organic layer containing the desired compound, drying of the organic layer over anhydrous magnesium sulfate or the like, and then evaporation of the organic solvent to give the desired product.

The obtained product, if necessary, is further purified by conventional treatments, for example, by recrystallization, reprecipitation, silica gel column chromatography or the like.

### (B-2c)

The solvent employed for the conversion reaction of a hydroxyl group into a leaving group can be, for example, a halogenated hydrocarbon such as dichloromethane, chloroform, carbon tetrachloride, dichloroethane, chlorobenzene or dichlorobenzene; or an ether such as diethyl ether, diisopropyl ether, tetrahydrofuran, dioxane, dimethoxyethane or di(ethylene glycol) dimethyl ether, and is preferably a halogenated hydrocarbon, and more preferably dichloromethane.

The base employed can be, for example, an organic base such as pyrrolidine, piperidine, morpholine, N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) or 1,8-diazabicyclo[5.4.0]undec-7-ene (DBU), and is preferably 1,8-diazabicyclo[5.4.0]undec-7-ene (DBU).

The reagent employed is not particularly restricted provided that it forms a leaving group by reacting with a hydroxyl group, and the conversion into the leaving group is achieved by addition of a halogenation agent, sulfonylation agent or acylation agent. The halogenation agent employed can be a carbon tetrahalide such as carbon tetrabromide or carbon tetrachloride, and in these cases, a phosphine is used as a reagent. Such phosphine can be, for example, a tri-C₁-C₆ alkylphosphine such as trimethylphosphine, triethylphosphine, tripropylphosphine, tributylphosphine, tripentylphosphine or trihexylphosphine; a tri-C₆-C₁₀ arylphosphine such as triphenylphosphine, triindenylphosphine or trinaphthylphosphine; or a tri-C₆-C₁₀ arylphosphine which may have C₁-C₄ alkyl group(s) as substituent(s), such as tolyldiphenylphosphine, tritolylphosphine, trimesitylphosphine, tributylphenylphosphine or tri-6-ethyl-2-naphthylphosphine, and is preferably a tri-C₁-C₆ alkylphosphine (particularly trimethylphosphine, triethylphosphine, tripropylphosphine or tributylphosphine) or a tri-C₆-C₁₀ arylphosphine (particularly triphenylphosphine, triindenylphosphine or trinaphthylphosphine), and more preferably a tri-C₆-C₁₀ arylphosphine (particularly triphenylphosphine). The sulfonylation agent employed can be, for example, a sulfonyl halide such as methanesulfonyl chloride, ethanesulfonyl chloride or tosyl chloride, and is preferably methanesulfonyl chloride. The acylation agent employed can be, for example, acetyl chloride, acetic anhydride, trifluoroacetic anhydride or the like.

The reaction temperature varies depending on the starting material and the reagent, and is between -50°C and 100°C, and preferably between -20°C and 80°C.

The reaction time varies depending on the reaction temperature, the starting material, the reagent and the kind of solvent employed, and is generally from 30 minutes to 24 hours, and preferably from 1 hour to 10 hours.

The solvent employed in the substitution reaction of Pro¹-SM can be, for example, an aromatic hydrocarbon such as benzene, toluene or xylene; a halogenated hydrocarbon such as dichloromethane, chloroform, carbon tetrachloride, dichloroethane, chlorobenzene or dichlorobenzene; an ester such as ethyl formate, ethyl acetate, propyl acetate, butyl acetate or diethyl carbonate; an ether such as diethyl ether, diisopropyl ether, tetrahydrofuran, dioxane, dimethoxyethane or di(ethylene glycol) dimethyl ether; a ketone such as acetone, methyl ethyl ketone, methyl isobutyl ketone, isophorone or cyclohexanone; a nitro compound such as nitroethane or nitrobenzene; a nitrile such as acetonitrile or isobutyronitrile; an amide such as formamide, N,N-dimethylformamide, N,N-dimethylacetamide, N-methyl-2-pyrrolidinone or hexamethylphosphoric triamide; or a sulfoxide such as dimethyl sulfoxide or sulfolane, and is preferably a halogenated hydrocarbon, an amide or a sulfoxide, and more preferably N,N-dimethylformamide, N,N-dimethylacetamide, dichloromethane or dimethyl sulfoxide.

The base employed can be, for example, an inorganic base containing an alkali metal carbonate such as sodium carbonate, potassium carbonate or lithium carbonate; an alkali metal hydrogencarbonate such as sodium hydrogencarbonate, potassium hydrogencarbonate or lithium hydrogencarbonate; an alkali metal hydride such as lithium hydride, sodium hydride or potassium hydride; an alkali metal hydroxide such as sodium hydroxide, potassium hydroxide or lithium hydroxide; and an alkali metal fluoride such as sodium fluoride or potassium fluoride; an alkali metal alkoxide such as sodium methoxide, sodium ethoxide, potassium methoxide, potassium ethoxide, potassium t-butoxide or lithium methoxide; an alkali metal mercaptan such as sodium methylmercaptan or sodium ethylmercaptan; or an organic base such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di-(t-butyl)-4-methylpyridine, quinoline, N,N-dimethylaniline, N,N-diethylaniline, 1,5-diazabicyclo[4.3.0]non-5-ene (DBN), 1 ,4-diazabicyclo[2.2.2]octane (DABCO) or 1,8-diazabicyclo[5.4.0]undec-7-ene (DBU), and is preferably an alkali metal carbonate or an organic base, and more preferably potassium carbonate or triethylamine.

The reaction temperature varies depending on the starting material and the reagent, and is between -50°C and 100°C, and preferably between -20°C and 80°C.

The reaction time varies depending on the reaction temperature, the starting material, the reagent and the kind of solvent employed, and is generally from 30 minutes to 24 hours, and preferably from 1 hour to 10 hours.

After completion of the reaction, the desired compound of this reaction can be obtained, for example, by concentration of the reaction mixture, addition to the mixture of an organic solvent immiscible with water such as ethyl acetate, washing the mixture with water, separation of the organic layer containing the desired compound, drying of the organic layer over anhydrous magnesium sulfate or the like, and then evaporation of the organic solvent to give the desired product.

The obtained product, if necessary, is further purified by conventional treatments, for example, by recrystallization, reprecipitation, silica gel column chromatography or the like.

### (B-2d)

The reaction for converting a hydroxyl group into a leaving group can be carried out in a similar manner to (B-2c).

The solvent of the subsequent substitution reaction employed can be, for example, an aliphatic hydrocarbon such as hexane, cyclohexane, heptane, ligroin, or petroleum ether; an aromatic hydrocarbon such as benzene, toluene, or xylene; a halogenated hydrocarbon such as dichloromethane, chloroform, carbon tetrachloride, dichloroethane, chlorobenzene, or dichlorobenzene; an ether such as diethyl ether, diisopropyl ether, tetrahydrofuran, dioxane, dimethoxyethane, or di(ethylene glycol) dimethyl ether; a nitrile such as acetonitrile or isobutyronitrile; or a mixture of water and the above-mentioned solvents, and is preferably a mixture of water and dioxane.

The palladium catalyst employed can be, for example, palladium chloride, palladium acetate, tris(dibenzylideneacetone)dipalladium-chloroform adduct, tris(dibenzylideneacetone)dipalladium, bis(dibenzylideneacetone)palladium, π-allylpalladium chloride dimer, tetrakis(triphenylphosphine)palladium, 1,1'-bis(diphenylphosphino)ferrocene palladium dichloride, bis(triphenylphosphine)palladium chloride, bis(triphenylphosphine)palladium acetate, dichloro(1,5-cyclooctadiene)palladium, dichlorobis(acetonitrile)palladium, bis(tricyclohexylphosphine)palladium dichloride, bis(tricyclohexylphosphine)palladium, or bis(tri-O-tolylphosphine)palladium dichloride, and is preferably palladium chloride, palladium acetate, tris(dibenzylideneacetone)dipalladium-chloroform adduct or tris(dibenzylideneacetone)dipalladium.

The phosphine employed can be, for example, a tri-C₁-C₆ alkylphosphine such as trimethylphosphine, triethylphosphine, tripropylphosphine, tributylphosphine, tripentylphosphine or trihexylphosphine; a tri-C₆-C₁₀ arylphosphine such as triphenylphosphine, triindenylphosphine or trinaphthylphosphine; a tri-C₆-C₁₀ arylphosphine which may be substituted with a C₁-C₄ alkyl group such as tolyldiphenylphosphine, tritolylphosphine, trimesitylphosphine, tributylphenylphosphine or tri-6-ethyl-2-naphthylphosphine; a bis(diphenylphosphino)C₁-C₆ alkane such as bis(diphenylphosphino)methane, 1,2-bis(diphenylphosphino)ethane, 1,3-bis(diphenylphosphino)propane, 1,4-bis(diphenylphosphino)butane, 1,5-bis(diphenylphosphino)pentane or 1,6-bis(diphenylphosphino)hexane; or bis(diphenylphosphino)acetylene, 1,2-bis(diphenylphosphino)benzene, (R)-(+)-2,2'-bis(diphenylphosphino)-1,1'-binaphthyl, 2,2'-bis(diphenylphosphino)-1,1'-binaphthyl, (S)-(-)-2,2'-bis(diphenylphosphino)-1,1'-binaphthyl, 1,1'-bis(diphenylphosphino)ferrocene, 1,1-bis(diphenylphosphino)ethylene or 4,5-bis(diphenylphosphino)-9,9-dimethylxanthene, and is preferably 1,1'-bis(diphenylphosphino)ferrocene, (R)-(+)-2,2'-bis(diphenylphosphino)-1,1'-binaphthyl, 2,2'-bis(diphenylphosphino)-1,1'-binaphthyl or (S)-(-)-2,2'-bis(diphenylphosphino)-1,1'-binaphthyl.

The reaction temperature varies depending on the starting material and the reagent, and is between 0°C and 150°C, and preferably between 50°C and 120°C.

The reaction time varies depending on the reaction temperature, the starting material, the reagent and the kind of solvent employed, and is generally from 1 hour to 24 hours, and preferably from 6 hours to 20 hours.

After completion of the reaction, the desired compound of this reaction can be obtained, for example, by concentration of the reaction mixture, addition to the mixture of an organic solvent immiscible with water such as ethyl acetate, washing the mixture with water, separation of the organic layer containing the desired compound, drying of the organic layer over anhydrous magnesium sulfate or the like, and then evaporation of the organic solvent to give the desired product.

The obtained product, if necessary, is further purified by conventional treatments, for example, by recrystallization, reprecipitation, silica gel column chromatography or the like.

### From (B-3) to (B-9)

Each of these steps is carried out in a similar manner to that indicated in (A-2) to (A-8) hereinbefore, respectively.

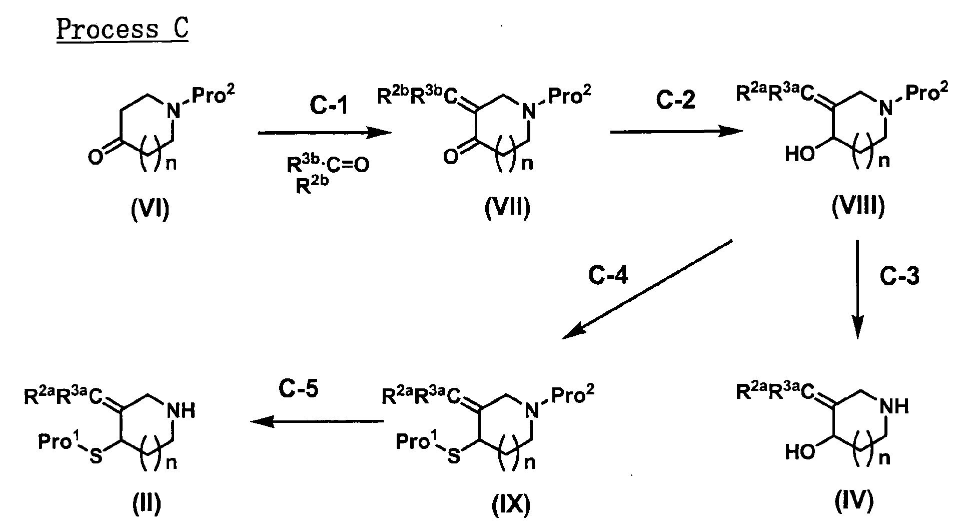

### (Process C)

In the above, Pro¹, R^{2a}, R^{3a} and, n have the same meanings as those indicated hereinbefore; R^{2b} and R^{3b} represent the same groups as those indicated for R^{2a} and R^{3a} described hereinbefore; and Pro² represents a protective group for an amino group.

The protective group for an amino group shown as Pro² is the same as that indicated for R^{2a} and R^{3a}.

Process C is hereinafter described in detail.

### (C-1)

This step is a step for the preparation of compound (VII) by reacting compound (VI) that is well known or easily prepared from known compounds with a compound having of formula R^{2b}-C(=O)-R^{3b} that is well known or can be easily prepared from known compounds, and is carried out by a route via the enamination (C-1a) or by the cross aldol reaction (C-1b).

### (C-1a)

The base employed can be, for example, an organic base such as pyrrolidine, piperidine, morpholine, N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di-(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) or 1,8-diazabicyclo[5.4.0]undec-7-ene (DBU), and is preferably pyrrolidine, piperidine or morpholine.

The solvent employed can be, for example, an aromatic hydrocarbon such as benzene, toluene or xylene; a halogenated hydrocarbon such as dichloromethane, chloroform, carbon tetrachloride, dichloroethane, chlorobenzene or dichlorobenzene; an alcohol such as methanol, ethanol, n-propanol, isopropanol, n-butanol, isobutanol, t-butanol, isoamyl alcohol, diethylene glycol, glycerol, octanol, cyclohexanol or 2-methoxyethanol; or an ether such as diethyl ether, diisopropyl ether, tetrahydrofuran, dioxane, dimethoxyethane or di(ethylene glycol) dimethyl ether; and is preferably benzene, toluene or ethanol.

The reaction temperature varies depending on the starting material and the reagent, and is between 0°C and 200°C, and preferably between 50°C and 150°C.

The reaction time varies depending on the reaction temperature, the starting material, the reagent and the kind of solvent employed, and is generally from 30 minutes to 24 hours, and preferably from 1 hour to 10 hours.

After completion of the reaction, the desired compound of this reaction can be obtained, for example, by concentration of the reaction mixture, addition to the mixture of an organic solvent immiscible with water such as ethyl acetate, washing the mixture with water, separation of the organic layer containing the desired compound, drying of the organic layer over anhydrous magnesium sulfate or the like, and then evaporation of the organic solvent to give the desired product.

The obtained product, if necessary, is further purified by conventional treatments, for example, by recrystallization, reprecipitation, silica gel column chromatography or the like.

When the compound having a hydroxyl group that is a reaction intermediate remains and the reaction is not completed, the desired product can be obtained by conducting furthermore a dehydration reaction in the presence of a base.

The solvent employed can be, for example, a halogenated hydrocarbon such as dichloromethane, chloroform, carbon tetrachloride, dichloroethane, chlorobenzene or dichlorobenzene; or an ether such as diethyl ether, diisopropyl ether, tetrahydrofuran, dioxane, dimethoxyethane or di(ethylene glycol) dimethyl ether, and is preferably a halogenated hydrocarbon, and more preferably dichloromethane.

The base employed can be an organic base such as pyrrolidine, piperidine, morpholine, N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di-(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) or 1,8-diazabicyclo[5.4.0]undec-7-ene (DBU), and is preferably 1,8-diazabicyclo[5.4.0]undec-7-ene (DBU).

The reagent employed is not particularly restricted provided that it forms a leaving group by reacting with a hydroxyl group, and the conversion into the leaving group is achieved by addition of a halogenation agent, sulfonylation agent or acylation agent. The halogenation agent employed can be, for example, a carbon tetrahalide such as carbon tetrabromide or carbon tetrachloride, and in these cases, a phosphine is used as a reagent. Such phosphine can be, for example, a tri-C₁-C₆ alkylphosphine such as trimethylphosphine, triethylphosphine, tripropylphosphine, tributylphosphine, tripentylphosphine or trihexylphosphine; a tri-C₆-C₁₀ arylphosphine such as triphenylphosphine, triindenylphosphine or trinaphthylphosphine; or tri-C₆-C₁₀ arylphosphine which may have C₁-C₄ alkyl group(s) as substituent(s), such as tolyldiphenylphosphine, tritolylphosphine, trimesitylphosphine, tributylphenylphosphine or tri-6-ethyl-2-naphthylphosphine, and is preferably a tri-C₁-C₆ alkylphosphine (particularly trimethylphosphine, triethylphosphine, tripropylphosphine or tributylphosphine) or a tri-C₆-C₁₀ arylphosphine (particularly triphenylphosphine, triindenylphosphine or trinaphthylphosphine), and more preferably a tri-C₆-C₁₀ arylphosphine (particularly triphenylphosphine). The sulfonylation agent employed can be, for example, a sulfonyl halide such as methanesulfonyl chloride, ethanesulfonyl chloride or tosyl chloride, and is preferably methanesulfonyl chloride. The acylation agent employed can be, for example, acetyl chloride, acetic anhydride, trifluoroacetic anhydride or the like.

The reaction temperature varies depending on the starting material and the reagent, and is between -50°C and 100°C, and preferably between -20°C and 80°C.

The reaction time varies depending on the reaction temperature, the starting material, the reagent and the kind of solvent employed, and is generally from 30 minutes to 24 hours, and preferably from 1 hour to 10 hours.

After completion of the reaction, the desired compound of this reaction can be obtained, for example, by concentration of the reaction mixture, addition to the mixture of an organic solvent immiscible with water such as ethyl acetate, washing the mixture with water, separation of the organic layer containing the desired compound, drying of the organic layer over anhydrous magnesium sulfate or the like, and then evaporation of the organic solvent to give the desired product.

The obtained product, if necessary, is further purified by conventional treatments, for example, by recrystallization, reprecipitation, silica gel column chromatography or the like.

### (C-1b)

The base employed can be, for example, an organic base such as pyrrolidine, piperidine, morpholine, N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di-(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) or 1,8-diazabicyclo[5.4.0]undec-7-ene (DBU); or an organometallic base such as butyllithium, lithium diisopropylamide, lithium bis(trimethylsilyl)amide, sodium bis(trimethylsilyl)amide or potassium bis(trimethylsilyl)amide, and is preferably lithium diisopropylamide, lithium bis(trimethylsilyl)amide, sodium bis(trimethylsilyl)amide or potassium bis(trimethylsilyl)amide.

The solvent employed can be, for example, an aromatic hydrocarbon such as benzene, toluene or xylene; a halogenated hydrocarbon such as dichloromethane, chloroform, carbon tetrachloride, dichloroethane, chlorobenzene or dichlorobenzene; an alcohol such as methanol, ethanol, n-propanol, isopropanol, n-butanol, isobutanol, t-butanol, isoamyl alcohol, diethylene glycol, glycerol, octanol, cyclohexanol or 2-methoxyethanol; or an ether such as diethyl ether, diisopropyl ether, tetrahydrofuran, dioxane, dimethoxyethane or di(ethylene glycol) dimethyl ether, and is preferably tetrahydrofuran.

The reaction temperature varies depending on the starting material and the reagent, and is between -100°C and 20°C, and preferably between -78°C and 0°C.

The reaction time varies depending on the reaction temperature, the starting material, the reagent and the kind of solvent employed, and is generally from 30 minutes to 24 hours, and preferably from 1 hour to 5 hours.

After completion of the reaction, the desired compound of this reaction can be obtained, for example, by concentration of the reaction mixture, addition to the mixture of an organic solvent immiscible with water such as ethyl acetate, washing the mixture with water, separation of the organic layer containing the desired compound, drying of the organic layer over anhydrous magnesium sulfate or the like, and then evaporation of the organic solvent to give the desired product.

The obtained product, if necessary, is further purified by conventional treatments, for example, by recrystallization, reprecipitation, silica gel column chromatography or the like.

When the compound having a hydroxyl group that is a reaction intermediate is remained, a dehydration reaction can be carried out in a similar manner to that indicated in (C-1a).

### (C-2)

This step is a step for the reduction reaction by conversion of a carbonyl group of compound (VII) obtained in C-1 into a hydroxyl group (C-2a). In addition, when R^{2b} is a hydrogen atom, R^{2a} is not a hydrogen atom and R^{3b} is an alkoxycarbonyl group, a reaction for introducing an R^{2a} group can be carried out (C-2b). Furthermore, when the R^{3b} group contains an ester group, if necessary, a reaction for introducing a suitable protective group to the hydroxyl group (C-2c), a reduction reaction for converting the ester group into a hydroxymethyl group (C-2d), a reaction for converting the R^{3b} group containing the hydroxyl group into an R^{3a} group (C-2e) and a reaction for removing the protective group for the hydroxyl group (C-2f) can be carried out. The steps from C-2c to C-2f can be conducted either before or after the steps C-2a and C-2b, and it is easy for a person having skill in the art to determine an appropriate order of these steps.

### (C-2a)

The solvent employed can be, for example, an aromatic hydrocarbon such as benzene, toluene or xylene; a halogenated hydrocarbon such as dichloromethane, chloroform, carbon tetrachloride, dichloroethane, chlorobenzene or dichlorobenzene; an ether such as diethyl ether, diisopropyl ether, tetrahydrofuran, dioxane, dimethoxyethane or di(ethylene glycol) dimethyl ether; an alcohol such as methanol, ethanol, n-propanol, isopropanol, n-butanol, isobutanol, t-butanol, isoamyl alcohol, diethylene glycol, glycerol, octanol, cyclohexanol or 2-methoxyethanol; or a mixture of an alcohol and a halogenated hydrocarbon, and is preferably an alcohol, a mixture of an alcohol and a halogenated hydrocarbon, or an ether, and more preferably methanol, ethanol, tetrahydrofuran or a mixture of said alcohol and dichloromethane.

The reagent employed is not particularly restricted provided that it can be used for a reduction reaction of a carbonyl group to a hydroxyl group, and can be, for example, an aluminium hydride reagent or boron compound such as sodium borohydride or diborane, and is preferably sodium borohydride.

The reaction temperature varies depending on the starting material and the reagent, and is between -78°C and 100°C, and preferably between 0°C and 50°C.

The reaction time varies depending on the reaction temperature, the starting material, the reagent and the kind of solvent employed, and is generally from 10 minutes to 12 hours, and preferably from 30 minutes to 5 hours.

After completion of the reaction, the desired compound of this reaction can be obtained, for example, by concentration of the reaction mixture, addition to the mixture of an organic solvent immiscible with water such as ethyl acetate, washing the mixture with water, separation of the organic layer containing the desired compound, drying of the organic layer over anhydrous magnesium sulfate or the like, and then evaporation of the organic solvent to give the desired product.

The obtained product, if necessary, is further purified by conventional treatments, for example, by recrystallization, reprecipitation, silica gel column chromatography or the like.

When R^{3b} is different from R^{3a}, it can be possible to introduce a substituent onto an amino group, if necessary. In this case, after removing a protective group for the amino group in a similar manner to that described in (A-7), the introduction of the substituent can be carried out as described below.

The solvent employed can be, for example, an aromatic hydrocarbon such as benzene, toluene or xylene; a halogenated hydrocarbon such as dichloromethane, chloroform, carbon tetrachloride, dichloroethane, chlorobenzene or dichlorobenzene; an ester such as ethyl formate, ethyl acetate, propyl acetate, butyl acetate or diethyl carbonate; an ether such as diethyl ether, diisopropyl ether, tetrahydrofuran, dioxane, dimethoxyethane or di(ethylene glycol) dimethyl ether; a ketone such as acetone, methyl ethyl ketone, methyl isobutyl ketone, isophorone or cyclohexanone; an amide such as formamide, N,N-dimethylformamide, N,N-dimethylacetamide, N-methyl-2-pyrrolidinone or hexamethylphosphoric triamide; or a sulfoxide such as dimethyl sulfoxide or sulfolane, and is preferably a halogenated hydrocarbon or an amide, and more preferably N,N-dimethylformamide, N,N-dimethylacetamide or dichloromethane.

The reagent employed is not particularly restricted provided that it can be used in the displacement reaction by an amino group, and can be, for example, a reagent in which a leaving group such as a halogen atom or a sulfonyl group is bonded to a desired functional group.

The base employed can be, for example, an inorganic base, including an alkali metal carbonate such as sodium carbonate, potassium carbonate or lithium carbonate; an alkali metal hydrogencarbonate such as sodium hydrogencarbonate, potassium hydrogencarbonate or lithium hydrogencarbonate; an alkali metal hydride such as lithium hydride, sodium hydride or potassium hydride; an alkali metal hydroxide such as sodium hydroxide, potassium hydroxide or lithium hydroxide; and an alkali metal fluoride such as sodium fluoride or potassium fluoride; an alkali metal alkoxide such as sodium methoxide, sodium ethoxide, potassium methoxide, potassium ethoxide, potassium t-butoxide or lithium methoxide; an alkali metal mercaptan such as sodium methylmercaptan or sodium ethylmercaptan; or an organic base such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di-(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) or 1,8-diazabicyclo[5.4.0]undec-7-ene (DBU), and is preferably potassium carbonate, sodium hydride or DBU.

The reaction temperature varies depending on the starting material and the reagent, and is between -30°C and 150°C, and preferably between 0°C and 100°C.

The reaction time varies depending on the reaction temperature, the starting material, the reagent and the kind of solvent employed, and is generally from 5 minutes to 48 hours, and preferably from 10 minutes to 15 hours.

After completion of the reaction, the desired compound of this reaction can be obtained, for example, by concentration of the reaction mixture, addition to the mixture of an organic solvent immiscible with water such as ethyl acetate, washing the mixture with water, separation of the organic layer containing the desired compound, drying of the organic layer over anhydrous magnesium sulfate or the like, and then evaporation of the organic solvent to give the desired product.

The obtained product, if necessary, is further purified by conventional treatments, for example, by recrystallization, reprecipitation, silica gel column chromatography or the like.

### (C-2b)

This step can be achieved by using an R^{2a}-Lv or an aldehyde or ketone which forms a R^{2a} group in an inert solvent in the presence of a base to give a compound of general formula (VII).

The solvent employed can be, for example, an aliphatic hydrocarbon such as hexane, cyclohexane, heptane, ligroin or petroleum ether; an aromatic hydrocarbon such as benzene, toluene or xylene; a halogenated hydrocarbon such as dichloromethane, chloroform, carbon tetrachloride, dichloroethane, chlorobenzene or dichlorobenzene; an ether such as diethyl ether, diisopropyl ether, tetrahydrofuran, dioxane, dimethoxyethane or di(ethylene glycol) dimethyl ether; or a nitrile such as acetonitrile or isobutyronitrile, and is preferably tetrahydrofuran.

The base employed can be, for example, lithium diisopropylamide, lithium benzyl(trimethylsilyl)amide, lithium dimethylamide, lithium diethylamide, lithium bis(trimethylsilyl)amide or lithium dicyclohexylamide, and is preferably lithium diisopropylamide.

The reagent employed can be, for example, a C₁-C₆ alkyl halide such as methyl iodide, ethyl iodide or propyl iodide; a C₁-C₆ alkyl mesylate; a C₁-C₆ alkyl tosylate; a C₇-C₁₅ aralkyl halide such as benzyl bromide, 1-naphthylmethyl bromide, 2-naphthylmethyl bromide or phenethyl bromide; a C₇-C₁₅ aralkyl mesylate; a C₇-C₁₅ aralkyl tosylate; a C₁-C₆ alkyl aldehyde such as formaldehyde or acetoaldehyde; or a C₁-C₆ alkyl ketone such as acetone.

The reaction temperature varies depending on the starting material and the reagent, and is between -100°C and 150°C, and preferably between -80°C and 50°C.

The reaction time varies depending on the reaction temperature, the starting material, the reagent and the kind of solvent employed, and is generally from 1 hour to 48 hours, and preferably from 1 hour to 10 hours.

After completion of the reaction, the desired compound of this reaction can be obtained, for example, by concentration of the reaction mixture, addition to the mixture of an organic solvent immiscible with water such as ethyl acetate, washing the mixture with water, separation of the organic layer containing the desired compound, drying the organic layer over anhydrous magnesium sulfate or the like, and then evaporation of the organic solvent to give the desired product.

The obtained product, if necessary, is further purified by conventional treatments, for example, by recrystallization, reprecipitation, silica gel column chromatography or the like.

### (C-2c)

This step is a step for the protection of a hydroxyl group, and can easily be achieved according to procedures that are well known by a person having skill in the art (for example, the procedure described in Protective Groups in Organic Synthesis, Third Edition, T. W. Green, et al., John Wiley & Sons, Inc. (1999)), and is preferably silyl protection (particularly t-butyldimethylsilyl group).

### (C-2d)

This step is achieved by reducing compound (VII) in an inert solvent in the presence of a reducing agent.

The solvent employed can be, for example, an aliphatic hydrocarbon such as hexane, cyclohexane, heptane, ligroin or petroleum ether; an aromatic hydrocarbon such as benzene, toluene or xylene; a halogenated hydrocarbon such as dichloromethane, chloroform, carbon tetrachloride, dichloroethane, chlorobenzene or dichlorobenzene; an ether such as diethyl ether, diisopropyl ether, tetrahydrofuran, dioxane, dimethoxyethane or di(ethylene glycol) dimethyl ether; or an alcohol such as methanol, ethanol, propanol or isobutanol. When the reducing agent is lithium aluminum hydride, diisobutyl aluminum hydride or diborane, the solvent employed can be an aliphatic hydrocarbon (particularly hexane or cyclohexane), an aromatic hydrocarbon (particularly benzene or toluene), a halogenated hydrocarbon (particularly dichloromethane) or an ether (particularly diethyl ether or tetrahydrofuran). When the reducing agent is sodium borohydride, the solvent employed can be an alcohol (particularly methanol or ethanol).

The reducing agent employed can be, for example, an aluminum hydride compound such as lithium aluminum hydride or diisobutyl aluminum hydride; sodium borohydride; or diborane, and is preferably diisobutyl aluminum hydride.

The reaction temperature varies depending on the starting material and the reagent, and is between -100°C and 100°C, and preferably between -80°C and 50°C.

The reaction time varies depending on the reaction temperature, the starting material, the reaction reagent and the kind of solvent employed, and is generally from 10 minutes to 24 hours, and preferably from 30 minutes to 5 hours.

After completion of the reaction, the desired compound of this reaction can be obtained, for example, by concentration of the reaction mixture, addition to the mixture of an organic solvent immiscible with water such as ethyl acetate, washing the mixture with water, separation of the organic layer containing the desired compound, drying the organic layer over anhydrous magnesium sulfate or the like, and then evaporation of the organic solvent to give the desired product.

The obtained product, if necessary, is further purified by conventional treatments, for example, by recrystallization, reprecipitation, silica gel column chromatography or the like.

### (C-2e)

This step is carried out by the Mitsunobu reaction in an inert solvent using an amine or the like (C-2e1) or by a substitution reaction using an amine or the like after converting a hydroxyl group into a leaving group (C-2e2). The step (C-2e1) can be carried out in a similar manner to that indicated in (B-2a) except that an amine or the like is employed instead of Pro¹-SM. The step (C-2e2) can be carried out in a similar manner to that indicated in (B-2c) except that an amine or the like is employed instead of Pro¹-SM.

### (C-2f)

This step is a step for the deprotection of a protective group for a hydroxyl group, and can easily be achieved according to procedures that are well known by a person having skill in the art (for example, the procedure discribed in Protective Groups in Organic Synthesis, Third Edition, T. W. Green, et al., John Wiley & Sons, Inc. (1999)).

### (C-3)

This step is carried out as in a similar manner to that indicated in A-7, and is carried out most preferably by using trifluoroacetic acid or acetic acid.

### (C-4)

This step is carried out in a similar manner to that indicated in B-2.

### (C-5)

This step is carried out in a similar manner to that indicated in C-3.

Compound (I) of the present invention, pharmacologically acceptable salts thereof or prodrugs thereof exert inhibiting activity in the inhibition of platelet aggregation. In addition, compound (I) of the present invention, pharmacologically acceptable salts thereof or prodrugs thereof exhibit excellent pharmacokinetics such as absorption, distribution, plasma half-life, and the like, and low toxicities in organs such as the kidney, liver and the like. Thus compound (I) of the present invention, pharmacologically acceptable salts thereof or prodrugs thereof are useful, for example as medicinal drugs, and particularly useful as therapeutic or prophylactic agents for various thrombotic diseases.

When a compound of the present invention is used as a prophylactic or therapeutic agent for diseases as described above, said compound having the general formula (I), or pharmacologically acceptable salts thereof or prodrugs thereof, can be administered alone or as a mixture with pharmaceutically acceptable excipients, diluents and the like, in various dosage forms such as tablets, capsules, granules, powders, syrups or the like for oral administration; and injections, suppositories, patches, external application or the like for parenteral administration.

Each of the above formulations can be prepared by well-known methods using additives for the formulation such as excipients (for example, organic excipients, including sugar derivatives such as lactose, sucrose, glucose, mannitol or sorbitol; starch derivatives such as corn starch, potato starch, α-starch or dextrin; cellulose derivatives such as crystalline cellulose; acacia; dextran; pullulan; and inorganic excipients including silicate derivatives such as light silicic acid anhydride, synthetic aluminium silicate, calcium silicate, or magnesium aluminate metasilicate; phosphate derivatives such as calcium hydrogenphosphate; carbonate derivatives such as calcium carbonate; or sulfate derivatives such as calcium sulfate), lubricants (for example, stearic acid; metal stearate derivatives such as calcium stearate or magnesium stearate; talc; colloidal silica; waxes such as beeswax or spermaceti; boric acid; adipic acid; sulfate derivatives such as sodium sulfate; glycol; fumaric acid; sodium benzoate; DL-leucine; lauryl sulfate derivatives such as sodium lauryl sulfate or magnesium lauryl sulfate; silicic acid derivatives such as silicic anhydride or silicic acid hydrate; or starch derivatives described above), binders (for example, hydroxypropylcellulose, hydroxypropylmethylcellulose, polyvinylpyrrolidone, macrogol or excipients as described above), disintegrants (for example, cellulose derivatives such as lower-substituted hydroxypropylcellulose, carboxymethylcellulose, calcium carboxymethylcellulose or internally cross-linked sodium carboxymethylcellulose; or chemically modified starch or cellulose derivatives such as carboxymethylstarch, sodium carboxymethylstarch, or cross-linked polyvinylpyrrolidone), emulsifiers (for example, colloidal clay such as bentonite or veegum; metal hydroxides such as magnesium hydroxide or aluminium hydroxide; anionic surfactants such as sodium lauryl sulfate or calcium stearate; cationic surfactants such as benzalkonium chloride; and non-ionic surfactants such as polyoxyethylene alkyl ethers, polyoxyethylene sorbitan fatty acid esters or sucrose esters of fatty acids), stabilizers (for example, para-hydroxybenzoic acid ester derivatives such as methylparaben or propylparaben; alcohol derivatives such as chlorobutanol, benzyl alcohol or phenylethyl alcohol; benzalkonium chloride; phenol derivatives such as phenol or cresol; thimerosal; dehydroacetic acid; or sorbic acid), corrigents (for example, sweeteners, souring agents, flavourings or the like which are conventionally used) and diluents.

The specific dose of a compound of the present invention will be varied according to the severity of the patient's symptoms, age and the like. For oral administration to a human adult the quantity of active ingredient in a unit dosage may be in the range of 0.016 mg/kg (preferably 0.5 mg/kg) to 33.3 mg/kg (preferably 25 mg/kg). A unit dose for intravenous administration may be in the range of 0.008 mg/kg (preferably 0.08 mg/kg) to 8.3 mg/kg (preferably 4.2 mg/kg) of a compound of the present invention. The unit dose may be administered to a human adult from 1 to 6 times per a day depending on the severity of the patient's symptoms.

### Examples

Hereinbelow, the present invention is described in more detail by way of Examples, Test Examples and Formulation Examples.

When CDCl₃ is used as a solvent for measurement of hydrochloride compounds in the Examples, NMR data for corresponding free bases are shown. The free bases can be obtained by treating a small amount of a hydrochloride compound with an aqueous sodium hydrogen carbonate solution and performing extraction with ethyl acetate or dichloromethane.

### (Example 1) (E)-4-(Acetylsulfanyl)-1-[2-cyclopropyl-1-(2-fluorophenyl)-2-oxoethyl]-3-[2-(morpholin-4-yl)ethylidene]piperidine dihydrochloride (Hydrochloride of the compound of Compound No. 5-28)

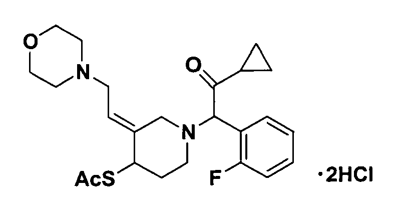

### (a) (E)-4-(t-Butyldimethylsilyloxy)-3-[(ethoxycarbonyl)methylidene]-1-(triphenylmethyl)piperidine

To a solution of (E)-3-[(ethoxycarbonyl)methylidene]-1-(triphenylmethyl)piperidin-4-ol (14.97 g) in N,N-dimethylformamide (130 ml) were added t-butyldimethylsilyl chloride (6.86 g), imidazole (3.34 g) and 4-dimethylaminopyridine (0.43 g) under ice cooling, and the resulting mixture was stirred at room temperature overnight. The reaction mixture was diluted with ethyl acetate and washed with a saturated aqueous sodium chloride solution, and the organic layer was dried over anhydrous sodium sulfate. The solvent was removed in vacuo, and the residue was purified by chromatography on a silica gel column (ethyl acetate / hexane = 1/19) to afford the title compound (16.38 g, yield 86%) as a colorless amorphous solid.

¹H NMR (400 MHz, CDCl₃) δ ppm : 0.01 (3H, s), 0.02 (3H, s), 0.90 (9H, s), 1.12 (3H, t, J=7.0), 1.59-1.74 (1H, m), 1.83-2.01 (3H, m), 3.00-3.12 (1H, m), 3.90 (1H, t, J=9.0), 3.96-4.15 (2H, m), 4.73 (1H, m), 6.06 (1H, s), 7.11-7.18 (3H, m), 7.20-7.28 (6H, m), 7.36-7.54 (6H, m).

### (b) (E)-4-(t-Butyldimethylsilyloxy)-3-(2-hydroxyethylidene)-1-(triphenylmethyl)piperidine

To a solution of (E)-4-(t-butyldimethylsilyloxy)-3-[(ethoxycarbonyl)methylidene]-1-(triphenylmethyl)piperidine (15.14 g) obtained in (a) above in dichloromethane (110 ml) was added a 1.01N solution of diisobutylaluminum hydride in toluene (66.40 ml) at -70°C, and the resulting mixture was stirred at the same temperature for 3 hours. The reaction mixture was charged with an aqueous ammonium chloride solution and then extracted with ethyl acetate. The extract was washed with a saturated aqueous sodium chloride solution, and the organic layer was dried over anhydrous sodium sulfate. The solvent was removed in vacuo, and the residue was purified by chromatography on a silica gel column (ethyl acetate / hexane = 1 / 3) to afford the title compound (13.22 g, yield 95%) as a colorless amorphous solid.

¹H NMR (400 MHz, CDCl₃) δ ppm : 0.01 (6H, s), 0.88 (9H, s), 1.62-2.00 (4H, m), 2.88-3.05 (1H, m), 3.51-3.71 (1H, m), 3.86 (1H, m), 4.07-4.24 (2H, m), 5.79 (1H, t, J=7.0), 7.11-7.33 (9H, m), 7.36-7.58 (6H, m).

### (c) (E)-4-(t-Butyldimethylsilyloxy)-3-[2-(tosyloxy)ethylidene]-1-(triphenyhnethyl)piperidine

To a solution of (E)-4-(t-butyldimethylsilyloxy)-3-(2-hydroxyethylidene)-1-(triphenylmethyl)piperidine (1.11 g) obtained in (b) above and p-toluenesulfonic anhydride (0.90 g) in dichloromethane (25 ml) was added triethylamine (0.37 ml) under ice cooling, and the resulting mixture was stirred at room temperature for 2.5 hours. The reaction mixture was charged with a saturated aqueous sodium chloride solution and then extracted with ethyl acetate. The extract was washed with a saturated aqueous sodium chloride solution, and the organic layer was dried over anhydrous sodium sulfate. The solvent was removed in vacuo to afford the title compound (1.64 g, quantitative yield) as a pale yellow oil.

¹H NMR (400 MHz, CDCl₃) δ ppm : -0.04 (3H, s), -0.01 (3H, s), 0.86 (9H, s), 1.60-1.92 (4H, m), 2.47 (3H, s), 2.96 (1H, bs), 3.47 (1H, m), 3.79 (1H, m), 4.61 (2H, m), 5.65 (1H, t, J=7.5), 7.13-7.48 (17H, m), 7.73 (2H, d, J=9.0).

### (d) (E)-4-(t-Butyldimethylsilyloxy)-3-[2-(morpholin-4-yl)ethylidene]-1-(triphenylmethyl)piperidine

To a solution of (E)-4-(t-butyldimethylsilyloxy)-3-[2-(tosyloxy)ethylidene]-1-(triphenylmethyl)piperidine (1.64 g) obtained in (c) above and morpholine (0.29 ml) in a mixed solvent of N,N-dimethylformamide (25 ml) and dichloromethane (5 ml) was added potassium carbonate (0.34 g) under ice cooling, and the resulting mixture was stirred at room temperature overnight. The reaction mixture was charged with a saturated aqueous sodium chloride solution and then extracted with ethyl acetate. The extract was washed with a saturated aqueous sodium chloride solution, and the organic layer was dried over anhydrous sodium sulfate. The solvent was removed in vacuo, and the residue was purified by chromatography on a silica gel column (ethyl acetate / hexane = 2 / 3) to afford the title compound (1.01 g, yield: 80%) as a colorless oil.

¹H NMR (400 MHz, CDCl₃) δ ppm : -0.03 (3H, s), -0.02 (3H, s), 0.84 (9H, s), 1.74-2.06 (4H, m), 2.42 (4H, bs), 2.77-2.93 (1H, m), 2.99 (1H, dd, J=13.5, 6.5), 3.07 (1H, dd, J=13.5, 6.5), 3.38-3.56 (1H, m), 3.71 (4H, t, J=4.5), 3.87 (1H, m), 5.62 (1H, t, J=6.5), 7.11-7.30 (9H, m), 7.38-7.55 (6H, m).

### (e) (E)-4-Hydroxy-3-[2-(morpholin-4-yl)ethylidene]-1-(triphenylmethyl)piperidine

To a solution of (E)-4-(t-butyldimethylsilyloxy)-3-[2-(morpholin-4-yl)ethylidene]-1-(triphenylmethyl)piperidine (1.00 g) obtained in (d) above in tetrahydrofuran (20 ml) was added tetrabutylammonium fluoride (0.99 g) under ice cooling, and the resulting mixture was stirred at room temperature for 14 hours.
The reaction mixture was charged with water and then extracted with ethyl acetate. The extract was washed with a saturated aqueous sodium chloride solution, and the organic layer was dried over anhydrous sodium sulfate. The solvent was removed in vacuo, and the residue was purified by chromatography on a silica gel column (methanol / dichloromethane =9/91) to afford the title compound (0.80 g, quantitative yield) as a colorless oil.

¹H NMR (400 MHz, CDCl₃) δ ppm : 1.74-1.86 (1H, m), 1.96-2.14 (2H, m), 2.45 (5H, bs), 2.59-2.69 (1H, m), 3.01 (2H, m), 3.10-3.34 (1H, m), 3.72 (4H, t, J=4.5), 3.98 (1H, m), 5.61 (1H, t, J=6.5), 7.11-7.32 (9H, m), 7.39-7.57 (6H, m).

### (f) (E)-4-Hydroxy-3-[2-(morpholin-4-yl)ethylidene]piperidine dihydrochloride

To a solution of (E)-4-hydroxy-3-[2-(morpholin-4-yl)ethylidene]-1-(triphenylmethyl)piperidine (1.08 g) obtained in (e) above in dioxane (9 ml) was added a 4N solution of hydrogen chloride in dioxane (3 ml) under ice cooling, and the resulting mixture was stirred at room temperature for 15 minutes. The reaction mixture was evaporated in vacuo, and the residue was partitioned with water and ethyl acetate. The aqueous layer was removed in vacuo to afford the title compound (0.69 g, quantitative yield) as a pale yellow amorphous solid.

¹H NMR (400 MHz, DMSO-d₆) δ ppm : 1.66-1.77 (1H, m), 1.95-2.07 (1H, m), 3.10 (4H, m), 3.21-3.65 (3H, m), 3.73-4.02 (6H, m), 4.06 (1H, d, J=13.5), 4.19 (1H, m), 5.90 (1H, t, J=8.0).

### (g) (E)-1-[2-Cyclopropyl-1-(2-fluorophenyl)-2-oxoethyl]-4-hydroxy-3-[2-(morpholin-4-yl)ethylidene]piperidine

To a solution of (E)-4-hydroxy-3-[2-(morpholin-4-yl)ethylidene]piperidine dihydrochloride (0.65 g) obtained in (f) above and 2-bromo-2-(2-fluorophenyl)-1-cyclopropylethanone (0.73 g) in N,N-dimethylformamide (12 ml) was added triethylamine (1.16 ml) under ice cooling, and the resulting mixture was stirred at room temperature overnight. The reaction mixture was diluted with ethyl acetate and washed with water and a saturated aqueous sodium chloride solution, and the organic layer was dried over anhydrous sodium sulfate. The solvent was removed in vacuo, and the residue was purified by chromatography on a silica gel column (methanol / dichloromethane = 3 / 17) to afford the title compound (0.65 g, yield 74%) as a pale yellow amorphous solid.

¹H NMR (500 MHz, CDCl₃) δ ppm : 0.78-0.90 (2H, m), 0.96-1.09 (2H, m), 1.66-1.78 (1H, m), 1.95-2.04 (1H, m), 2.15-2.25 (1H, m), 2.42 (4H, bs), 2.45-2.55 (1H, m), 2.78 and 3.00 (total 1H, each d, J=12.5), 2.79-2.88 (1H, m), 2.95 (2H, d, J=6.5), 3.31 and 3.45 (total 1H, each d, J=12.5), 3.70 (4H, t, J=4.5), 4.09 and 4.13 (total 1H, each m), 4.69 and 4.71 (total 1H, each s), 5.59 and 5.61 (total 1H, each t, J=6.5), 7.11 (1H, m), 7.17 (1H, m), 7.29-7.36 (1H, m), 7.38-7.45 (1H, m).

### (h) (E)-4-(Acetylsulfanyl)-1-[2-cyclopropyl-1-(2-fluorophenyl)-2-oxoethyl]-3-[2-(morpholin-4-yl)ethylidene]piperidine dihydrochloride

To a solution of (E)-1-[2-cyclopropyl-1-(2-fluorophenyl)-2-oxoethyl]-4-hydroxy-3-[2-(morpholin-4-yl)ethylidene]piperidine (890 mg) obtained in (g) above in toluene (15 ml) were added thioacetic acid (0.33 ml) and N,N-dimethylformamide dineopentyl acetal (1.92 ml) under ice cooling, and the resulting mixture was stirred at room temperature for 3 hours. The reaction mixture was diluted with ethyl acetate and washed with a saturated aqueous sodium chloride solution, and the organic layer was dried over anhydrous sodium sulfate. The solvent was removed in vacuo, and the residue was purified by chromatography on a silica gel column (methanol / dichloromethane = 1 / 19) to afford the free form (393 mg, yield 38%) of the title compound as a colorless amorphous solid. To a solution of a part of this compound (88 mg) in dioxane (4 ml) was added a 4N solution of hydrogen chloride in dioxane (0.25 ml) at room temperature, and the solvent was removed in vacuo to afford the title compound (111 mg, quantitative yield) as a colorless amorphous solid.

¹H NMR (400 MHz, CDCl₃) δ ppm : 0.76-0.90 (2H, m), 0.95-1.10 (2H, m), 1.74-1.85 (1H, m), 2.06-2.24 (2H, m), 2.28 and 2.29 (total 3H, each s), 2.38 (4H, bs), 2.47-2.79 (2H, m), 2.80 and 3.06 (total 1H, each d, J=12.5), 2.88 and 2.94 (total 2H, each d, J=6.5), 3.29 and 3.37 (total 1H, each d, J=12.5), 3.69 (4H, m), 4.30 and 4.33 (total 1H, each t, J=4.5), 4.68 and 4.70 (total 1H, each s), 5.64 (1H, t, J=6.5), 7.07-7.20 (2H, m), 7.29-7.43 (2H, m);

IR (KBr, cm⁻¹) : 1700, 1494.

### (Example 2) (E)-1-[2-Cyclopropyl-1-(2-fluorophenyl)-2-oxoethyl]-3-[2-(morpholin-4-yl)ethylidene]-4-sulfanylpiperidine dihydrochloride (Hydrochloride of the compound of Compound No. 5-27)

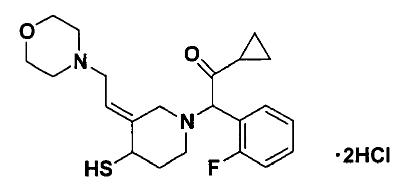

To a solution of (E)-4-(acetylsulfanyl)-1-[2-cyclopropyl-1-(2-fluorophenyl)-2-oxoethyl]-3-[2-(morpholin-4-yl)ethylidene]piperidine (180 mg) obtained in Example 1 (h) in methanol (4 ml) was added potassium carbonate (48 mg) under ice cooling, and the resulting mixture was stirred at room temperature for 15 minutes. The reaction mixture was diluted with ethyl acetate and washed with a saturated aqueous sodium chloride solution, and the organic layer was dried over anhydrous sodium sulfate. The solvent was removed in vacuo, and the residue was purified using a preparative HPLC (YMC-Pack ODS-A; YMC, eluent: acetonitrile / 0.024N hydrochloric acid = 15 / 85) to afford the title compound (83 mg, yield 50%) as a colorless amorphous solid.

¹H NMR (400 MHz, CDCl₃) δ ppm : 0.77-0.91 (2H, m), 0.95-1.10 (2H, m), 1.67-1.77 (1H, m), 2.12-2.26 (2H, m), 2.39 (4H, bs), 2.46-2.95 (3H, m), 2.88 and 3.20 (total 2H, each d, J=7.0), 3.13 and 3.37 (total 1H, each d, J=12.5), 3.69 (4H, m), 3.80 (1H, m), 4.71 and 4.73 (total 1H, each s), 5.59 and 5.62 (total 1H, each t, J=7.0), 7.07-7.21 (2H, m), 7.28-7.37 (1H, m), 7.39-7.45 (1H, m);

IR (KBr, cm⁻¹): 2556, 1712, 1494.

### (Example 3)

### (E)-4-(Acetylsulfanyl)-1-[2-cyclopropyl-1-(2-fluorophenyl)-2-oxoethyl]-3-[2-(piperidin-1-yl)ethylidene]piperidine (Compound of Compound No. 5-2)

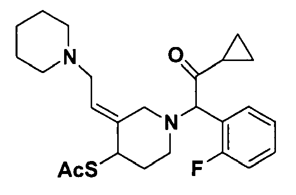

### (a) (E)-4-(t-Butyldimethylsilyloxy)-3-[2-(piperidin-1-yl)ethylidene]-1-(triphenylmethyl)piperidine

The title compound was synthesized in a yield of 67% as a colorless oil using piperidine instead of morpholine by conducting a reaction similar to that mentioned in Example 1 (d).

¹H NMR (400 MHz, CDCl₃) δ ppm : -0.01 (6H, s), 0.81 (9H, s), 1.37-1.61 (8H, m), 1.74-1.93 (2H, m), 2.31-2.39 (4H, m), 2.75-3.07 (4H, m), 3.86 (1H, m), 5.63 (1H, t, J=6.5), 7.11-7.18 (3H, m), 7.21-7.28 (6H, m), 7.41-7.51 (6H, m).

### (b) (E)-4-Hydroxy-3-[2-(piperidin-1-yl)ethylidene]-1-(triphenylmethyl)piperidine

The title compound was synthesized in a quantitative yield as a colorless oil using (E)-4-(t-butyldimethylsilyloxy)-3-[2-(piperidin-1-yl)ethylidene]-1-(triphenylmethyl)piperidine obtained in (a) above by conducting a reaction similar to that mentioned in Example 1 (e).

¹H NMR (500 MHz, CDCl₃) δ ppm : 1.48-2.11 (14H, m), 2.69-3.46 (4H, m), 4.01 (1H, m), 5.89 (1H, t, J=5.0), 7.15-7.21 (3H, m), 7.24-7.31 (6H, m), 7.40-7.51 (6H, m).

### (c) (E)-4-Hydroxy-3-[2-(piperidin-1-yl)ethylidene]piperidine bis(hydrogen trifluoroacetate)

To a solution of (E)-4-hydroxy-3-[2-(piperidin-1-yl)ethylidene]-1-(triphenylmethyl)piperidine (412 mg) obtained in (b) above in dichloromethane (10 ml) was added trifluoroacetic acid (0.21 ml) under ice cooling, and the resulting mixture was stirred at room temperature for 3 hours. To the rection mixture was added trifluoroacetic acid (0.21 ml), and the resulting mixture was stirred at room temperature for further 1 hour. To the reaction mixture was added methanol (5 ml), and the resulting mixture was evaporated in vacuo. The residue was partitioned with water and ethyl acetate, and the aqueous layer was removed in vacuo to afford the title compound (403 mg, quantitative yield) as a pale yellow amorphous solid.

¹H NMR (400 MHz, CDCl₃) δ ppm : 1.35-1.49 (2H, m), 1.82-2.21 (6H, m), 2.73-2.82 (2H, m), 3.45-3.88 (7H, m), 4.12 (1H, d, J=13.0), 4.31-4.36 (1H, m), 6.01 (1H, t, J=8.0).

### (d) (E)-1-[2-Cyclopropyl-1-(2-fluorophenyl)-2-oxoethyl]-4-hydroxy-3-[2-(piperidin-1-yl)ethylidene]piperidine

The title compound was synthesized in a yield of 60% as a pale yellow oil using (E)-4-hydroxy-3-[2-(piperidin-1-yl)ethylidene]piperidine bis(hydrogen trifluoroacetate) obtained in (c) above by conducting a reaction similar to that mentioned in Example 1 (g).

¹H NMR (400 MHz, CDCl₃) δ ppm : 0.79-1.10 (4H, m), 1.38-1.84 (8H, m), 1.93-2.03 (1H, m), 2.21-2.55 (6H, m), 2.78-3.01 (3H, m), 3.32 and 3.41 (total 1H, each d, J=12.5), 4.08-4.14 (1H, m), 4.67 and 4.69 (total 1H, each s), 5.63 and 5.64 (total 1H, each t, J=7.0), 7.07-7.19 (2H, m), 7.28-7.46 (2H, m).

### (e) (E)-4-(Acetylsulfanyl)-1-[2-cyclopropyl-1-(2-fluorophenyl)-2-oxoethyl]-3-[2-(piperidin-1-yl)ethylidene]piperidine

To a solution of triphenylphosphine (290 mg) in tetrahydrofuran (4.5 ml) under a nitrogen gas stream was added a 40% solution of diisopropyl azodicarboxylate in toluene (0.59 ml) at -10°C, and the resulting mixture was stirred at the same temperature for 1 hour. Subsequently, to a reaction mixture were added dropwise a solution of (E)-1-[2-cyclopropyl-1-(2-fluorophenyl)-2-oxoethyl]-4-hydroxy-3-[2-(piperidin-1-yl)ethylidene]piperidine (211 mg) in tetrahydrofuran (1.5 ml) obtained in (d) above and thioacetic acid (0.14 ml), and further the resulting mixture was stirred at room temperature for 22 hours. The reaction mixture was evaporated in vacuo, and the residue was purified by chromatography on a silica gel column (methanol / dichloromethane = 1 / 49) to afford the title compound (138 mg, yield 57%) as a pale yellow oil.

¹H NMR (400 MHz, CDCl₃) δ ppm : 0.78-0.90 (2H, m), 0.96-1.08 (2H, m), 1.41-1.50 (2H, m), 1.59-1.71 (4H, m), 1.75-1.84 (1H, m), 2.07-2.23 (2H, m), 2.28 and 2.29 (total 3H, each s), 2.40-3.10 (9H, m), 3.16-3.39 (1H, m), 4.29 and 4.33 (total 1H, each t, J=4.5), 4.68 and 4.72 (total 1H, each s), 5.69 (1H, t, J=6.5), 7.08-7.21 (2H, m), 7.29-7.41 (2H, m);

IR (liquid film, cm⁻¹) : 1696, 1488.

### (Example 4) (E)-1-[2-Cyclopropyl-1-(2-fluorophenyl)-2-oxoethyl]-3-[2-(piperidin-1-yl)ethylidene]-4-sulfanylpiperidine bis(hydrogen trifluoroacetate) (Trifluoroacetate of the compound of Compound No. 5-1)

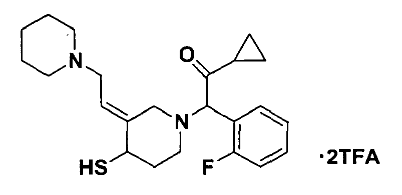

The title compound was synthesized in a yield of 11 % as a colorless oil using (E)-4-(acetylsulfanyl)-1-[2-cyclopropyl-1-(2-fluorophenyl)-2-oxoethyl]-3-[2-(piperidin-1-yl)ethylidene]piperidine obtained in Example 3 (e) by conducting a reaction similar to that mentioned in Example 2.

¹H NMR (400 MHz, CDCl₃) δ ppm : 0.76-0.93 (2H, m), 0.99-1.10 (2H, m), 1.31-1.47 (2H, m), 1.63-2.07 (7H, m), 2.22-2.32 (1H, m), 2.56-3.87 (10H, m), 4.93 and 4.98 (total 1H, each s), 5.74 (1H, t, J=6.5), 7.15-7.31 (3H, m), 7.36-7.46 (1H, m);

MS (FAB) m/z : 403 (M+H)⁺.

### (Example 5) (E)-4-(Acetylsulfanyl)-1-[2-cyclopropyl-1-(2-fluorophenyl)-2-oxoethyl]-3-[2-(thiomorpholin-4-yl)ethylidene]piperidine dihydrochloride (Hydrochloride of the compound of Compound No. 5-30)

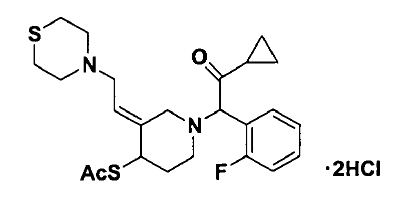

### (a) (E)-4-(t-Butyldimethylsilyloxy)-3-[2-(thiomorpholin-4-yl)ethylidene]-1-(triphenylmethyl)piperidine

The title compound was synthesized in a yield of 62% as a colorless oil using thiomorpholine instead of morpholine by conducting a reaction similar to that mentioned in Example 1 (d).

¹H NMR (500 MHz, CDCl₃) δ ppm : -0.03 (3H, s), -0.02 (3H, s), 0.84 (9H, s), 1.76-1.95 (4H, m), 2.61-2.72 (7H, m), 2.77-2.91 (1H, m), 2.98-3.10 (2H, m), 3.31-3.66 (2H, m), 3.87 (1H, m), 5.56-5.63 (1H, m), 7.10-7.19 (3H, m), 7.21-7.35 (6H ,m), 7.38-7.53 (6H, m).

### (b) (E)-4-Hydroxy-3-[2-(thiomorpholin-4-yl)ethylidene]-1-(triphenylmethyl)piperidine

The title compound was synthesized in a yield of 99% as a pale yellow amorphous solid using (E)-4-(t-butyldimethylsilyloxy)-3-[2-(thiomorpholin-4-yl)ethylidene]-1-(triphenylmethyl)piperidine obtained in (a) above by conducting a reaction similar to that mentioned in Example 1 (e).

¹H NMR (400 MHz, CDCl₃) δ ppm : 1.61-1.87 (2H, m), 1.98-2.14 (2H, m), 2.60-2.80 (8H, m), 2.98-3.06 (2H, m), 3.35-3.46 (2H, m), 3.93-4.02 (1H, m), 5.58 (1H, t, J=6.0), 7.12-7.20 (3H, m), 7.22-7.32 (6H, m), 7.38-7.56 (6H, m).

### (c) (E)-4-Hydroxy-3-[2-(thiomorpholin-4-yl)ethylidene]piperidine bis(hydrogen acetate)

To a solution of (E)-4-hydroxy-3-[2-(thiomorpholin-4-yl)ethylidene]-1-(triphenylmethyl)piperidine (4.91 g) obtained in (b) above in dichloromethane (100 ml) was added acetic acid (44 ml) at room temperature, and the resulting mixture was refluxed for 1 hour. The reaction mixture was evaporated in vacuo, and the residue was purified by chromatography on a silica gel column on a silica gel column (methanol / dichloromethane = 1 / 4) to afford the title compound (3.56 g, yield 98%) as a pale brown crystal.

¹H NMR (400 MHz, CDCl₃) δ ppm : 1.61-1.70 (1H, m), 1.97-2.02 (1H, m), 2.62-2.77 (8H, m), 2.82-2.90 (1H, m), 3.02-3.08 (2H, m), 3.17-3.23 (2H, m), 3.74-3.78 (1H, m), 4.15-4.20 (1H, m), 5.58 (1H, t, J=6.0).

### (d) (E)-1-[2-Cyclopropyl-1-(2-fluorophenyl)-2-oxoethyl]-4-hydroxy-3-[2-(thiomorpholin-4-yl)ethylidene]piperidine

The title compound was synthesized in a yield of 65% as a pale brown amorphous solid using (E)-4-hydroxy-3-[2-(thiomorpholin-4-yl)ethylidene]piperidine bis(hydrogen acetate) obtained in (c) above by conducting a reaction similar to that mentioned in Example 1 (g).

¹H NMR (500 MHz, CDCl₃) δ ppm : 0.76-1.12 (4H, m), 1.61-1.82 (1H, m), 1.94-2.04 (1H, m), 2.14-2.24 (1H, m), 2.44-2.54 (1H, m), 2.59-2.73 (8H, m), 2.76 and 2.98 (total 1H, each d, J=12.5), 2.77-2.89 (1H, m), 2.96 (2H, d, J=7.0), 3.30 and 3.45 (total 1H, each d, J=12.5), 4.05-4.16 (1H, m), 4.69 and 4.70 (total 1H, each s), 5.54-5.62 (1H, m), 7.06-7.22 (2H, m), 7.28-7.46 (2H, m).

### (e) (E)-4-(Acetylsulfanyl)-1-[2-cyclopropyl-1-(2-fluorophenyl)-2-oxoethyl]-3-[2-(thiomorpholin-4-yl)ethylidene]piperidine dihydrochloride

The title compound was synthesized in a yield of 26% as a pale yellow powder crystal using (E)-1-[2-cyclopropyl-1-(2-fluorophenyl)-2-oxoethyl]-4-hydroxy-3-[2-(thiomorpholin-4-yl)ethylidene]piperidine obtained in (d) above by conducting a reaction similar to that mentioned in Example 1 (h).

¹H NMR (500 MHz, CDCl₃) δ ppm : 0.74-1.12 (4H, in), 1.50-1.62 (1H, m), 1.74-1.85 (1H, m), 2.06-2.27 (2H, m), 2.29 and 2.30 (total 3H, each s), 2.44-2.72 (9H, m), 2.77 and 3.04 (total 1H, each d, J=12.5), 2.84-2.98 (2H, m), 3.27 and 3.35 (total 1H, each d, J=12.5), 4.26-4.36 (1H, m), 4.67 and 4.70 (total 1H, each s), 5.57-5.64 (1H, m), 7.07-7.22 (2H, m), 7.28-2.45 (2H, m);

MS (FAB) m/z : 463 (M+H)⁺.

### (Example 6) (E)-1-[2-Cyclopropyl-1-(2-fluorophenyl)-2-oxoethyl]-4-sulfanyl-3-[2-(thiomorpholin-4-yl)ethylidene]piperidine dihydrochloride (Hydrochloride of the compound of Compound No. 5-29)

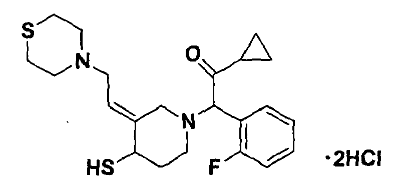

The title compound was synthesized in a yield of 94% as a pale yellow powder crystal using (E)-4-(acetylsulfanyl)-1-[2-cyclopropyl-1-(2-fluorophenyl)-2-oxoethyl]-3-[2-(thiomorpholin-4-yl)ethylidene]piperidine obtained in Example 5 (e) by conducting a reaction similar to that mentioned in Example 2.

¹H NMR (500 MHz, CDCl₃) δ ppm : 0.77-1.12 (4H, m), 1.68-1.77 (1H, m), 2.11-2.27 (2H, m), 2.55-2.71 (9H, m), 2.72-2.85 (1H, m), 2.86-2.97 (2H, m), 3.13-3.24 (1H, m), 3.11 and 3.36 (total 1H, each d, J=12.5), 3.70-3.82 (1H, m), 4.72 and 4.70 (total 1H, each s), 5.51-5.63 (1H, m), 7.07-7.21 (2H, m), 7.29-7.45 (2H, m);

MS (FAB) m/z : 418 (M+H)⁺.

### (Example 7) (E)-4-(Acetylsulfanyl)-1-[2-cyclopropyl-1-(2-fluorophenyl)-2-oxoethyl]-3-[2-(3-oxopiperazin-1-yl)ethylidene]piperidine (Compound of Compound No. 5-88)

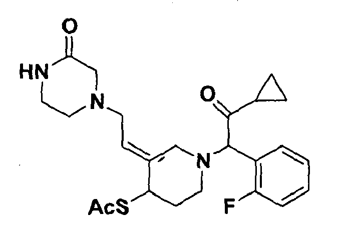

### (a) (E)-4-(t-Butyldimethylsilyloxy)-3-[2-(3-oxopiperazin-1-yl)ethylidene]-1-(triphenylmethyl)piperidine

The title compound was synthesized in a yield of 66% as a pale brown oil using 2-oxopiperazine instead of morpholine by conducting a reaction similar to that mentioned in Example 1 (d).

¹H NMR (500 MHz, CDCl₃) δ ppm : 0.02 (6H, s), 0.85 (9H, s), 1.72-1.95 (4H, m), 2.60-2.66 (2H, m), 2.80-3.19 (5H, m), 3.25-3.47 (3H, m), 3.82-3.90 (1H, m), 5.57-5.64 (1H, m), 7.13-7.21 (3H, m), 7.22-7.32 (6H, m), 7.39-7.57 (6H, m).

### (b) (E)-4-Hydroxy-3-[2-(3-oxopiperazin-1-yl)ethylidene]-1-(triphenylmethyl)piperidine

The title compound was synthesized in a yield of 96% as a pale yellow oil using (E)-4-(t-butyldimethylsilyloxy)-3-[2-(3-oxopiperazin-1-yl)ethylidene]-1-(triphenylmethyl)piperidine obtained in (a) above by conducting a reaction similar to that mentioned in Example 1 (e)

¹H NMR (400 MHz, CDCl₃) δ ppm : 1.75-2.12 (4H, m), 2.62-2.69 (2H, m), 3.02-3.16 (2H, m), 3.32-3.42 (6H, m), 3.92-4.01 (1H, m), 5.57-5.62 (1H, m), 7.12-7.20 (3H, m), 7.22-7.31 (6H, m), 7.38-7.56 (6H, m).

### (c) (E)-4-Hydroxy-3-[2-(3-oxopiperazin-1-yl)ethylidene]piperidine bis(hydrogen acetate)

The title compound was synthesized in a yield of 85% as a pale brown crystal using (E)-4-hydroxy-3-[2-(3-oxopiperazin-1-yl)ethylidene]-1-(triphenylmethyl)piperidine obtained in (b) above by conducting a reaction similar to that mentioned in Example 5 (c).

¹H NMR (400 MHz, CDCl₃) δ ppm : 1.59-1.73 (2H, m), 2.57-2.61 (1H, m), 2.64-2.70 (2H, m), 3.08-3.25 (5H, m), 3.29-3.39 (3H, m), 3.73-3.78 (1H, m), 4.16-4.22 (1H, m), 5.56-5.63 (1H, m).

### (d) (E)-1-[2-Cyclopropyl-1-(2-fluorophenyl)-2-oxoethyl]-4-hydroxy-3-[2-(3-oxopiperazin-1-yl)ethylidene]piperidine

The title compound was synthesized in a yield of 63% as a pale brown amorphous solid using (E)-4-hydroxy-3-[2-(3-oxopiperazin-1-yl)ethylidene]piperidine bis(hydrogen acetate) obtained in (c) above, by conducting a reaction similar to that mentioned in Example 1 (g).

¹H NMR (400 MHz, CDCl₃) δ ppm : 0.76-1.10 (4H, m), 1.62-1.80 (2H, m), 1.94-2.05 (1H, m), 2.12-2.23 (1H, m), 2.43-2.53 (1H, m), 2.57-2.68 (2H, m), 2.73 and 2.98 (total 1H, each d, J=12.5), 2.78-2.92 (1H, m), 2.94-3.08 (2H, m), 3.28-3.42 (3H, m), 3.45-3.52 (1H, m), 4.05-4.19 (1H, m), 4.67-4.74 (1H, m), 5.53-5.65 (1H, m), 7.06-7.23 (2H, m), 7.28-7.48 (2H, m).

### (e) (E)-4-(Acetylsulfanyl)-1-[2-cyclopropyl-1-(2-fluorophenyl)-2-oxoethyl]-3-[2-(3-oxopiperazin-1-yl)ethylidene]piperidine

The title compound was synthesized in a yield of 22% as a pale yellow amorphous solid using (E)-1-[2-cyclopropyl-1-(2-fluorophenyl)-2-oxoethyl]-4-hydroxy-3-[2-(3-oxopiperazin-1-yl)ethylidene]piperidine obtained in (d) above by conducting a reaction similar to that mentioned in Example 1 (h).

¹H NMR (500 MHz, CDCl₃) δ ppm : 0.69-1.10 (4H, m), 1.73-1.86 (1H, m), 2.06-2.26 (2H, m), 2.29 and 2.30 (total 3H, each s), 2.44-2.72 (4H, m), 2.74-2.85 (1H, m), 2.95 and 3.01 (total 2H, each d, J=7.0), 3.03-3.10 (2H, m), 3.28 and 3.38 (total 1H, each d, J=12.5), 3.29-3.38 (2H, m), 4.26-4.36 (1H, m), 4.69 and 4.71 (total 1H, each s), 5.62 (1H, t, J=7.0), 5.78 (1H, bs), 7.07-7.24 (2H, m), 7.28-7.48 (2H, m);

MS (FAB) m/z : 460 (M+H)⁺.

### (Example 8) (E)-1-[2-Cyclopropyl-1-(2-fluorophenyl)-2-oxoethyl]-3-[2-(3-oxopiperazin-1-yl)ethylidene]-4-sulfanylpiperidine dihydrochloride (Hydrochloride of the compound of Compound No. 5-87)

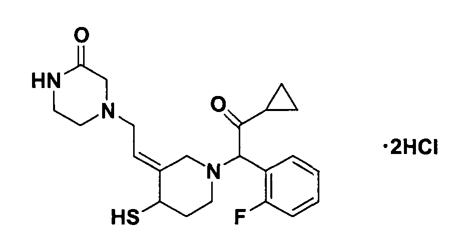

The title compound was synthesized in a yield of 91 % as a pale yellow powder crystal using (E)-4-(acetylsulfanyl)-1-[2-cyclopropyl-1-(2-fluorophenyl)-2-oxoethyl]-3-[2-(3-oxopiperazin-1-yl)ethylidene]piperidine obtained in Example 7 (e) by conducting a reaction similar to that mentioned in Example 2.

¹H NMR (500 MHz, CDCl₃) δ ppm : 0.75-1.10 (4H, m), 1.69-1.78 (1H, m), 2.11-2.28 (2H, m), 2.53-2.70 (3H, m), 2.73-2.86 (1H, m), 2.92-3.22 (4H, m), 3.30-3.45 (3H, m), 3.66-3.82 (1H, m), 4.72 and 4.73 (total 1H, each s), 5.52-5.64 (1H, m), 7.07-7.22 (2H, m), 7.29-7.44 (2H, m);

MS (FAB) m/z : 418 (M+H)⁺.

### (Example 9) (E)-4-(Acetylsulfanyl)-1-[2-cyclopropyl-1-(2-fluorophenyl)-2-oxoethyl]-3-[2-(piperazin-1-yl)ethylidene]piperidine trihydrochloride (Hydrochloride of the compound of Compound No. 5-32)

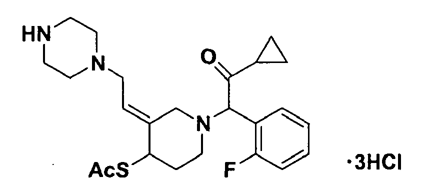

### (a) (E)-3-{2-[4-(t-Butoxycarbonyl)piperazin-1-yl]ethylidene}-4-(t-butyldimethylsilyloxy)-1-(triphenylmethyl)piperidine

The title compound was synthesized in a yield of 61 % as a colorless amorphous solid using 1-(t-butoxycarbonyl)piperazine instead of morpholine by conducting a reaction similar to that mentioned in Example 1 (d).

¹H NMR (500 MHz, CDCl₃) δ ppm : -0.03 (3H, s), -0.02 (3H, s), 0.84 (9H, s), 1.46 (9H, s), 1.73-1.96 (3H, m), 2.30-2.41 (4H, m), 2.74-3.12 (4H, m), 3.36-3.46 (4H, m), 3.47-3.52 (1H, m), 3.81-3.92 (1H, m), 5.66 (1H, t, J=6.0), 7.10-7.19 (3H, m), 7.20-7.32 (6H, m), 7.34-7.57 (6H, m).

### (b) (E)-3-{2-[4-(t-Butoxycarbonyl)piperazin-1-yl]ethylidene}-4-hydroxy-1-(triphenylmethyl)piperidine

The title compound was synthesized in a yield of 99% as a colorless amorphous solid using (E)-3-{2-[4-(t-butoxycarbonyl)piperazin-1-yl]ethylidene}-4-(t-butyldimethylsilyloxy)-1-(triphenylmethyl)piperidine obtained in (a) above by conducting a reaction similar to that mentioned in Example 1 (e).

¹H NMR (400 MHz, CDCl₃) δ ppm : 1.47 (9H, s), 1.57-1.84 (4H, m), 2.32-2.45 (4H, m), 2.92-3.08 (2H, m), 3.36-3.52 (6H, m), 3.93-4.03 (1H, m), 5.61 (1H, t, J=6.0), 7.11-7.20 (3H, m), 7.21-7.33 (6H, m), 7.40-7.55 (6H, m).

### (c) (E)-3-{2-[4-(t-Butoxycarbonyl)piperazin-1-yl]ethylidene}-4-hydroxypiperidine bis(hydrogen acetate)

The title compound was synthesized in a yield of 99% as a pale brown crystal using (E)-3-{2-[4-(t-butoxycarbonyl)piperazin-1-yl]ethylidene}-4-hydroxy-1-(triphenylmethyl)piperidine obtained in (b) above by conducting a reaction similar to that mentioned in Example 5 (c).

¹H NMR (400 MHz, CDCl₃) δ ppm : 1.45 (9H, s), 1.56-1.73 (1H, m), 1.86-2.04 (1H, m), 2.32-2.46 (4H, m), 2.80-2.91 (1H, m), 2.94-3.07 (2H, m), 3.17-3.29 (2H, m), 3.35-3.46 (4H, m), 3.51 and 3.74 (total 1H, each d, J=13.5), 4.03-4.11 (1H, m), 5.57-5.66 (1H, m).

### (d) (E)-3-{2-[4-(t-Butoxycarbonyl)piperazin-1-yl]ethylidene}-1-[2-cyclopropyl-1-(2-fluorophenyl)-2-oxoethyl]-4-hydroxypiperidine

The title compound was synthesized in a yield of 64% as a pale brown amorphous solid using (E)-3-{2-[4-(t-butoxycarbonyl)piperazin-1-yl]ethylidene}-4-hydroxypiperidine bis(hydrogen acetate) obtained in (c) above by conducting a reaction similar to that mentioned in Example 1 (g).

¹H NMR (400 MHz, CDCl₃) δ ppm : 0.76-1.11 (4H, m), 1.46 (9H, s), 1.64-1.81 (2H, m), 1.93-2.04 (1H, m), 2.13-2.25 (1H, m), 2.28-2.40 (4H, m), 2.44-2.55 (1H, m), 2.76 and 2.99 (total 1H, each d, J=13.0), 2.78-2.90 (1H, m), 2.90-2.98 (1H, m), 3.31 and 3.45 (total 1H, each d, J=13.0), 3.37-3.45 (4H, m), 4.04-4.18 (1H, m), 4.69 and 4.70 (total 1H, each s), 5.55-5.64 (1H, m), 7.06-7.21 (2H, m), 7.27-7.36 (1H, m), 7.37-7.45 (1H, m).

### (e) (E)-4-(Acetylsulfanyl)-3-{2-[4-(t-butoxycarbonyl)piperazin-1-yl]ethylidene}-1-[2-cyclopropyl-1-(2-fluorophenyl)-2-oxoethyl]piperidine

The title compound was synthesized in a yield of 36% as a brown oil using (E)-3- {2-[4-(t-butoxycarbonyl)piperazin-1-yl] ethylidene} -1-[2-cyclopropyl-1-(2-fluorophenyl)-2-oxoethyl]-4-hydroxypiperidine obtained in (d) above by conducting a reaction similar to that mentioned in Example 1 (h).

¹H NMR (400 MHz, CDCl₃) δ ppm : 0.76-1.10 (4H, m), 1.46 (9H, s), 1.75-1.84 (1H, m), 2.07-2.28 (2H, m), 2.22-2.37 (4H, m), 2.28 and 2.29 (total 3H, each s), 2.47-2.62 (1H, m), 2.65-2.80 (1H, m), 2.79 and 3.05 (total 1H, each d, J=13.0), 2.84-2.90 (1H, m), 2.91-2.96 (1H, m), 3.29 and 3.36 (total 1H, each d, J=13.0), 3.36-3.45 (4H, m), 4.27-4.36 (1H, m), 4.67 and 4.69 (total 1H, each s), 5.63 (1H, t, J=6.5), 7.08-7.20 (2H, m), 7.28-7.36 (1H, m), 7.40 (1H, m).

### (f) (E)-4-(Acetylsulfanyl)-1-[2-cyclopropyl-1-(2-fluorophenyl)-2-oxoethyl]-3-[2-(piperazin-1-yl)ethylidene]piperidine trihydrochloride

To a solution of (E)-4-(acetylsulfanyl)-3-{2-[4-(t-butoxycarbonyl)piperazin-1-yl]ethylidene}-1-[2-cyclopropyl-1-(2-fluorophenyl)-2-oxoethyl]piperidine (1.06 g) obtained in (e) above in dioxane (21 ml) was added a 4N solution of hydrogen chloride in dioxane (24 ml) under ice cooling, and the resulting mixture was stirred at room temperature for 3 hours. The reaction mixture was evaporated in vacuo to afford the title compound (1.25 g, quantitative yield) as a pale yellow crystal.

¹H NMR (500 MHz, CDCl₃) δ ppm : 0.76-1.10 (4H, m), 1.62-1.85 (2H, m), 2.09-2.25 (2H, m), 2.28 (3H, s), 2.31-2.45 (4H, m), 2.47-2.62 (1H, m), 2.64-2.81 (1H, m), 2.80 and 3.05 (total 1H, each d, J=12.5), 2.84-2.97 (4H, m), 3.29 and 3.35 (total 1H, each d, J=12.5), 3.40-3.67 (1H, m), 4.26-4.36 (1H, m), 4.67 and 4.69 (total 1H, each s), 5.64 (1H, t, J=7.0), 7.07-7.21 (2H, m), 7.28-7.45 (2H, m);

MS (FAB) m/z : 446 (M+H)⁺.

### (Example 10) (E)-1-[2-Cyclopropyl-1-(2-fluorophenyl)-2-oxoethyl]-3-[2-(piperazin-1-yl)ethylidene]-4-sulfanylpiperidine (Compound of Compound No. 5-31) and (E)-3-[2-(4-acetylpiperazin-1-yl)ethylidene]-1-[2-cyclopropyl-1-(2-fluorophenyl)-2-oxoethyl]-4-sulfanylpiperidine (Compound of Compound No. 5-33)

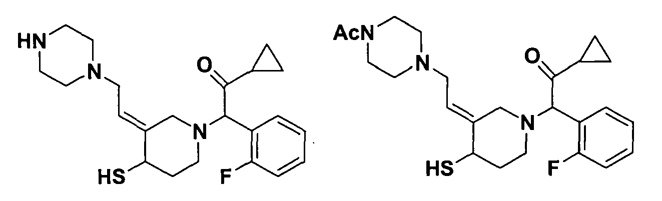

Both title compounds were synthesized using (E)-4-(acetylsulfanyl)-1-[2-cyclopropyl-1-(2-fluorophenyl)-2-oxoethyl]-3-[2-(piperazin-1-yl)ethylidene]piperidine obtained in Example 9 (f) by conducting a reaction similar to that mentioned in Example 2.
(E)-1-[2-Cyclopropyl-1-(2-fluorophenyl)-2-oxoethyl]-3-[2-(piperazin-1-yl)ethylidene]-4-sulfanylpiperidine having a high polarity: a pale yellow oil, yield 12%.

¹H NMR (500 MHz, CDCl₃) δ ppm : 0.77-1.09 (4H, m), 1.67-1.76 (2H, m), 2.12-2.26 (2H, m), 2.29-2.43 (4H, m), 2.55-2.67 (1H, m), 2.70-2.82 (1H, m), 2.83-2.89 (4H, m), 2.90-2.95 (1H, m), 3.14 and 3.35 (total 1H, each d, J=12.5), 3.43-3.68 (1H, m), 3.72-3.83 (1H, m), 4.70 and 4.71 (total 1H, each s), 5.56-5.66 (1H, m), 7.06-7.21 (2H, m), 7.27-7.46 (2H, m);

MS (FAB) m/z : 404 (M+H)⁺.
(E)-3-[2-(4-Acetylpiperazin-1-yl)ethylidene]-1-[2-cyclopropyl-1-(2-fluorophenyl)-2-oxoethyl]-4-sulfanylpiperidine having a low polarity: a colorless oil, yield 46%.

¹H NMR (500 MHz, CDCl₃) δ ppm : 0.75-1.09 (4H, m), 1.69-1.77 (1H, m), 2.08 (3H, s), 2.12-2.26 (2H, m), 2.27-2.43 (4H, m), 2.54-2.69 (1H, m), 2.73-2.85 (1H, m), 2.87-2.97 (2H, m), 3.08 and 3.37 (total 1H, each d, J=12.5), 3.13-3.24 (1H, m), 3.41-3.67 (4H, m), 3.69-3.82 (1H, m), 4.71 and 4.73 (total 1H, each s), 5.53-5.64 (1H, m), 7.08-7.20 (2H, m), 7.28-7.44 (2H, m);

MS (FAB) m/z : 446 (M+H)⁺.

### (Example 11) (E)-4-(Acetylsulfanyl)-1-[2-cyclopropyl-1-(2-fluorophenyl)-2-oxoethyl]-3-{2-[4-(ethoxycarbonylmethyl)piperazin-1-yl]ethylidene}piperidine (Compound of Compound No. 5-46)

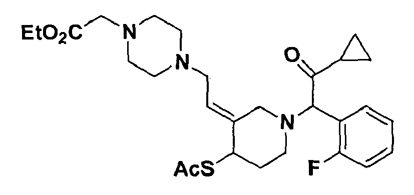

### (a) (E)-1-[2-Cyclopropyl-1-(2-fluorophenyl)-2-oxoethyl]-4-hydroxy-3-[2-(piperazin-1-yl)ethylidene]piperidine trihydrochloride

The title compound was synthesized in a yield of 99% as a pale yellow powder crystal using (E)-3-{2-[4-(t-butoxycarbonyl)piperazin-1-yl]ethylidene}-1-[2-cyclopropyl-1-(2-fluorophenyl)-2-oxoethyl]-4-hydroxypiperidine obtained in Example 9 (d) by conducting a reaction similar to that mentioned in Example 9 (f).

¹H NMR (400 MHz, DMSO-d₆) δ ppm : 0.89-1.15 (4H, m), 1.74-1.96 (2H, m), 2.00-2.15 (1H, m), 2.82-4.25 (15H, m), 5.94-6.19 (2H, m), 7.40-7.54 (2H, m), 7.64-7.84 (2H, m).

### (b) (E)-1-[2-Cyclopropyl-1-(2-fluorophenyl)-2-oxoethyl]-3- {2-[4-(ethoxycarbonylmethyl)piperazin-1-yl]ethylidene}-4-hydroxypiperidine

To a suspension of (E)-1-[2-cyclopropyl-1-(2-fluorophenyl)-2-oxoethyl]-4-hydroxy-3-[2-(piperazin-1-yl)ethylidene]piperidine trihydrochloride (1.14 g) obtained in (a) above in acetonitrile (35 ml) were added ethyl bromoacetate (0.31 ml) and triethylamine (1.92 ml) under ice cooling, and the resulting mixture was stirred at room temperature for 1 hour. After the reaction mixture was evaporated in vacuo, water was poured thereto and the mixture was extracted with a mixed solvent of dichloromethane and isopropanol. The organic layer was washed with a saturated aqueous sodium chloride solution and dried over anhydrous sodium sulfate. The solvent was removed in vacuo, and the residue was purified by chromatography on a silica gel column (methanol / dichloromethane = 1 / 9) to afford the title compound (0.71 g, yield 66%) as a pale yellow oil.

¹H NMR (400 MHz, CDCl₃) δ ppm : 0.77-0.90 (2H, m), 0.95-1.08 (2H, m), 1.28 (3H, t, J=7.0), 1.65-1.80 (2H, m), 1.94-2.03 (1H, m), 2.16-2.25 (1H, m), 2.41-2.70 (8H, m), 2.75-2.88 (2H, m), 2.96-3.02 (2H, m), 3.20 (2H, s), 3.30 and 3.44 (total 1H, each d, J=12.5), 4.06-4.15 (1H, m), 4.19 (2H, q, J=7.0), 4.68 and 4.70 (total 1H, each s), 5.58-5.66 (1H, m), 7.07-7.21 (2H, m), 7.28-7.45 (2H, m).

### (c) (E)-4-(Acetylsulfanyl)-1-[2-cyclopropyl-1-(2-fluorophenyl)-2-oxoethyl]-3-{2-[4-(ethoxycarbonylmethyl)piperazin-1-yl]ethylidene}piperidine

The title compound was synthesized in a yield of 23% as a pale brown oil using (E)-1-[2-cyclopropyl-1-(2-fluorophenyl)-2-oxoethyl]-3-{2-[4-(ethoxycarbonylmethyl)piperazin-1-yl]ethylidene}-4-hydroxypiperidine obtained in (b) above by conducting a reaction similar to that mentioned in Example 1 (h).

¹H NMR (500 MHz, CDCl₃) δ ppm : 0.77-1.09 (4H, m), 1.27 (3H, t, J=7.0), 1.75-1.83 (1H, m), 2.06-2.24 (2H, m), 2.28 (3H, s), 2.37-2.64 (8H, m), 2.65-2.81 (2H, m), 2.79 and 3.03 (total 1H, each d, J=12.5), 2.85-2.90 (1H, m), 2.92-2.98 (1H, m), 3.19 (2H, s), 3.28 and 3.34 (total 1H, each d, J=12.5), 4.19 (2H, q, J=7.0), 4.26-4.35 (1H, m), 4.66 and 4.68 (total 1H, each s), 5.61-5.67 (1H, m), 7.06-7.20 (2H, m), 7.29-7.43 (2H, m);

MS (FAB) m/z : 532 (M+H)⁺.

### (Example 12) (E)-1-[2-Cyclopropyl-1-(2-fluorophenyl)-2-oxoethyl]-3-{2-[4-(ethoxycarbonylmethyl)piperazin-1-yl]ethylidene}-4-sulfanylpiperidine (Compound of Compound No. 5-45)

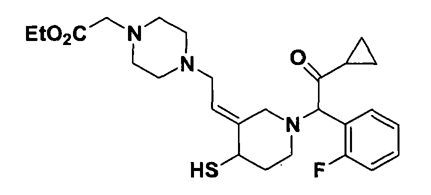

To a solution of (E)-4-(acetylsulfanyl)-1-[2-cyclopropyl-1-(2-fluorophenyl)-2-oxoethyl]-3-{2-[4-(ethoxycarbonylmethyl)piperazin-1-yl]ethylidene}piperidine (111 mg) obtained in Example 11 (c) in ethanol (3 ml) was added potassium carbonate (289 mg) under ice cooling, and the resulting mixture was stirred at room temperature for 2 hours. After the reaction mixture was poured into water and extracted with dichloromethane, the organic layer was dried over anhydrous sodium sulfate. The solvent was removed in vacuo to afford the title compound (77 mg, yield 75%) as a pale yellow oil.

¹H NMR (400 MHz, CDCl₃) δ ppm : 0.75-1.09 (4H, m), 1.27 (3H, t, J=7.0), 1.66-1.76 (1H, m), 2.09-2.26 (2H, m), 2.35-2.67 (8H, m), 2.69-2.84 (1H, m), 2.86-2.91 (1H, m), 2.91-2.98 (1H, m), 3.11 and 3.35 (total 1H, each d, J=12.5), 3.18 (2H, s), 3.48 (2H, s), 3.68-3.81 (1H, m), 4.17 (2H, q, J=7.0), 4.68 and 4.69 (total 1H, each s), 5.52-5.64 (1H, m), 7.02-7.19 (2H, m), 7.23-7.43 (2H, m);

MS (FAB) m/z : 490 (M+H)⁺.

### (Example 13) (E)-4-(Acetylsulfanyl)-1-[2-cyclopropyl-1-(2-fluorophenyl)-2-oxoethyl]-3-{2-[4-(ethoxycarbonylmethyl)-3-oxopiperazin-1-yl]ethylidene}piperidine (Compound of Compound No. 5-102)

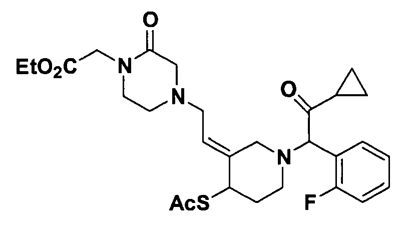

### (a) (E)-4-(t-Butyldimethylsilyloxy)-3-{2-[4-(ethoxycarbonylmethyl)-3-oxopiperazin-1-yl]ethylidene}-1-(triphenylmethyl)piperidine

The title compound was synthesized in a yield of 13% as a pale yellow amorphous solid using 1-(ethoxycarbonylmethyl)-2-oxo-piperazine hydrochloride instead of morpholine by conducting a reaction similar to that mentioned in Example 1 (d).

¹H NMR (500 MHz, CDCl₃) δ ppm : -0.01 (3H, s), -0.02 (3H, s), 0.85 (9H, s), 1.28 (3H, t, J=7.0), 1.69-2.08 (2H, m), 2.67-2.74 (2H, m), 3.00-3.20 (2H, m), 3.17 (2H, s), 3.34-3.42 (2H, m), 3.61-3.67 (2H, m), 3.73-3.81 (2H, m), 3.82-3.91 (1H, m), 4.11 (2H, s), 4.21 (2H, q, J=7.0), 5.56-5.65 (1H, m), 7.11-7.20 (3H, m), 7.21-7.32 (6H, m), 7.36-7.56 (6H, m).

### (b) (E)-3-{2-[4-(Ethoxycarbonylinethyl)-3-oxopiperazin-1-yl]ethylidene}-4-hydroxy-1-(triphenylmethyl)piperidine

The title compound was synthesized in a yield of 80% as a pale yellow amorphous solid using (E)-4-(t-butyldimethylsilyloxy)-3-{2-[4-(ethoxycarbonyhnethyl)-3-oxopiperazin-1-yl]ethylidene}-1-(triphenylmethyl)piperidine obtained in (a) above by conducting a reaction similar to that mentioned in Example 1 (e).

¹H NMR (400 MHz, CDCl₃) δ ppm : 1.28 (3H, t, J=7.0), 1.76-1.86 (1H, m), 2.02-2.12 (1H, m), 2.70-2.78 (2H, m), 3.02-3.14 (2H, m), 3.20 (2H, s), 3.37-3.44 (2H, m), 3.60-3.68 (2H, m), 3.73-3.81 (2H, m), 3.93-4.02 (1H, m), 4.12 (2H, s), 4.21 (2H, q, J=7.0), 5.60 (1H, t, J=7.0), 7.11-7.20 (3H, m), 7.21-7.31 (6H, m), 7.38-7.53 (6H, m).

### (c) (E)-3-{2-[4-(Ethoxycarbonylmethyl)-3-oxopiperazin-1-yl]ethylidene}-4-hydroxypiperidine bis(hydrogen acetate)

The title compound was synthesized in a yield of 99% as a pale brown crystal using (E)-3-{2-[4-(ethoxycarbonylmethyl)-3-oxopiperazin-1-yl]ethylidene}-4-hydroxy-1-(triphenylmethyl)piperidine obtained in (b) above by conducting a reaction similar to that mentioned in Example 5 (c).

¹H NMR (400 MHz, CDCl₃) δ ppm : 1.28 (3H, t, J=7.0), 1.56-1.69 (1H, m), 1.94-2.06 (2H, m), 2.70-2.88 (4H, m), 3.20 (2H, s), 3.37-3.49 (2H, m), 3.70-3.79 (2H, m), 4.10 (2H, s), 4.14-4.20 (2H, m), 4.20 (2H, q, J=7.0), 5.60 (1H, t, J=7.0).

### (d) (E)-1-[2-Cyclopropyl-1-(2-fluorophenyl)-2-oxoethyl]-3-{2-[4-(ethoxycarbonylmethyl)-3-oxopiperazin-1-yl]ethylidene}-4-hydroxypiperidine

The title compound was synthesized in a yield of 68% as a pale brown amorphous solid using (E)-3-{2-[4-(ethoxycarbonylmethyl)-3-oxopiperazin-1-yl]ethylidene}-4-hydroxypiperidine bis(hydrogen acetate) obtained in (c) above by conducting a reaction similar to that mentioned in Example 1 (g).

¹H NMR (400 MHz, CDCl₃) δ ppm : 0.76-1.09 (4H, m), 1.28 (3H, t, J=7.0), 1.60-1.77 (2H, m), 1.94-2.04 (1H, m), 2.13-2.23 (1H, m), 2.43-2.53 (1H, m), 2.65-2.77 (2H, m), 2.79-2.90 (1H, m), 2.93-3.07 (2H, m), 3.16 (2H, s), 3.33 and 3.47 (total 1H, each d, J=12.5), 3.35-3.42 (2H, m), 4.04-4.14 (1H, m), 4.11 (2H, s), 4.21 (2H, q, J=7.0), 4.70 and 4.71 (total 1H, each s), 5.54-5.63 (1H, m), 7.05-7.21 (2H, m), 7.28-7.36 (1H, m), 7.37-7.45 (1H, m).

### (e) (E)-4-(Acetylsulfanyl)-1-[2-cyclopropyl-1-(2-fluorophenyl)-2-oxoethyl]-3-{2-[4-(ethoxycarbonylmethyl)-3-oxopiperazin-1-yl]ethylidene}piperidine

The title compound was synthesized in a yield of 34% as a yellow oil using (E)-1-[2-cyclopropyl-1-(2-fluorophenyl)-2-oxoethyl]-3-{2-[4-(ethoxycarbonylmethyl)-3-oxopiperazin-1-yl] ethylidene}-4-hydroxypiperidine obtained in (d) above by conducting a reaction similar to that mentioned in Example 1 (h).

¹H NMR (500 MHz, CDCl₃) δ ppm : 0.76-1.09 (4H, m), 1.28 (3H, t, J=7.0), 1.75-1.84 (1H, m), 2.09-2.25 (2H, m), 2.29 and 2.30 (total 3H, each s), 2.48-2.59 (1H, m), 2.60-2.74 (2H, m), 2.75-2.83 (1H, m), 2.92-2.97 (1H, m), 2.99-3.03 (1H, m), 3.07 and 3.28 (total 1H, each d, J=12.5), 3.09-3.17 (2H, m), 3.34-3.41 (2H, m), 3.46-3.52 (1H, m), 4.11 (2H, s), 4.21 (2H, q, J=7.0), 4.26-4.34 (1H, m), 4.69 and 4.70 (total 1H, each s), 5.61 (1H, t, J=7.0), 7.07-7.21 (2H, m), 7.29-7.44 (2H, m);

MS (FAB) m/z : 546 (M+H)⁺.

### (Example 14) (E)-1-[2-Cyclopropyl-1-(2-fluorophenyl)-2-oxoethyl]-3-{2-[4-(methoxycarbonylmethyl)-3-oxopiperazin-1-yl]ethylidene}-4-sulfanylpiperidine (Compound of Compound No. 5-99)

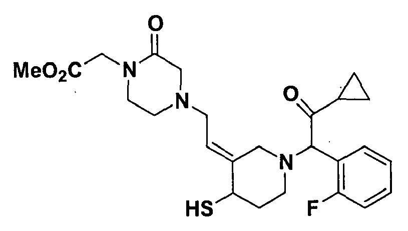

The title compound was synthesized in a yield of 93% as a yellow oil using (E)-4-(acetylsulfanyl)-1-[2-cyclopropyl-1-(2-fluorophenyl)-2-oxoethyl]-3-{2-[4-(ethoxycarbonylmethyl)-3-oxopiperazin-1-yl]ethylidene}piperidine obtained in Example 13 (e) by conducting a reaction similar to that mentioned in Example 2.

¹H NMR (400 MHz, CDCl₃) δ ppm : 0.76-1.09 (4H, m), 1.69-1.77 (1H, m), 2.12-2.26 (2H, m), 2.53-2.73 (3H, m), 2.74-2.85 (1H, m), 2.93-3.04 (2H, m), 3.07 and 3.38 (total 1H, each d, J=12.5), 3.12-3.22 (3H, m), 3.35-3.44 (2H, m), 3.68-3.81 (1H, m), 3.75 (3H, s), 4.13 (2H, s), 4.72 and 4.73 (total 1H, each s), 5.57 and 5.61 (total 1H, each t, J=7.0), 7.07-7.21 (2H, m), 7.29-7.44 (2H, m);

MS (FAB) m/z : 490 (M+H)⁺.

### (Example 15) (E)-3-{2-[4-(Carboxymethyl)-3-oxopiperazin-1-yl]ethylidene}-1-[2-cyclopropyl-1-(2-fluorophenyl)-2-oxoethyl]-4-sulfanylpiperidine dihydrochloride (Hydrochloride of the compound of Compound No. 5-97)

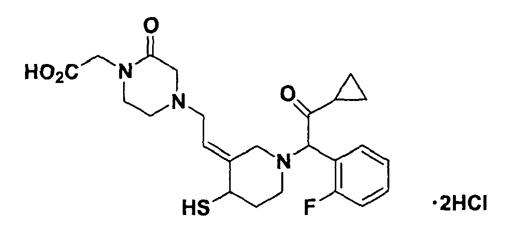

To a solution of (E)-1-[2-cyclopropyl-1-(2-fluorophenyl)-2-oxoethyl]-3-{2-[4-(methoxycarbonylmethyl)-3-oxopiperazin-1-yl]ethylidene}-4-sulfanylpiperidine (280 mg) obtained in Example 14 in water (1 ml) was added 3N hydrochloric acid (5 ml) at room temperature, and the resulting mixture was stirred at 50°C for 2.5 hours. The reaction mixture was evaporated in vacuo, and the residue was purified using a preparative HPLC (YMC-Pack ODS-A; YMC, eluent: acetonitrile / 0.024N hydrochloric acid = 15 / 85) to afford the title compound (203 mg, yield 65%) as a colorless powder crystal.

¹H NMR (400 MHz, pyridine-d₅) δ ppm : 0.78-0.88 (2H, m), 0.94-1.18 (2H, m), 1.74-1.86 (1H, m), 2.14-2.28 (1H, m), 2.40-2.60 (1H, m), 2.60-2.80 (3H, m), 2.86-3.02 (1H, m), 3.02-3.15 (2H, m), 3.30-3.46 (3H, m), 3.50-3.66 (3H, m), 3.88 (1H, bs), 4.50 (2H, s), 4.94 (1H, s), 5.80 (1H, t, J=7.0), 7.18-7.36 (3H, m), 7.70-7.78 (1H, m);

MS (FAB) m/z : 476 (M+H)⁺.

### (Example 16) (E)-4-(Acetylsulfanyl)-3-{2-[4-(carboxymethyl)-3-oxopiperazin-1-yl]ethylidene}-1-[2-cyclopropyl-1-(2-fluorophenyl)-2-oxoethyl]piperidine dihydrochloride (Hydrochloride of the compound of Compound No. 5-98)

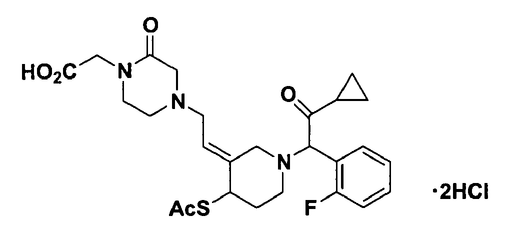

To a suspension of (E)-3-{2-[4-(carboxymethyl)-3-oxopiperazin-1-yl]ethylidene} -1-[2-cyclopropyl-1-(2-fluorophenyl)-2-oxoethyl]-4-sulfanylpiperidine dihydrochloride (100 mg) obtained in Example 15 in dichloromethane (5 ml) were added acetic anhydride (259 µl) and pyridine (514 µl) under ice cooling, and the resulting mixture was stirred at room temperature for 4 hours. The reaction mixture was evaporated in vacuo, and the residue was purified by chromatography on a silica gel column (methanol / dichloromethane = 3/17) to afford the free form (85 mg, yield 90%) of the title compound as a brown solid. To a solution of this compound in dichloromethane (3 ml) was added a 4N solution of hydrogen chloride in dioxane (82 µl) at room temperature, and the reaction mixture was evaporated in vacuo.
The residue was crystallized from ether to afford the title compound (44 mg, yield 45%) as a pale yellow amorphous solid.

¹H NMR (400 MHz, pyridine-d₅) δ ppm : 0.72-0.85 (2H, m), 0.98-1.05 (1H, m), 1.06-1.16 (1H, m), 1.79-1.90 (1H, m), 2.15-2.26 (1H, m), 2.23 and 2.24 (total 3H, each s), 2.35-2.49 (1H, m), 2.53-2.75 (3H, m), 2.75-2.91 (1H, m), 2.97-3.22 (3H, m), 3.30-3.38 (2H, m), 3.47-3.55 (2H, m), 3.56-3.65 (1H, m), 4.44-4.47 (2H, m), 4.51-4.55 (1H, m), 4.93 and 4.94 (total 1H, each s), 5.81 and 5.83 (total 1H, each t, J=7.0), 7.16-7.36 (3H, m), 7.64-7.72 (1H, m);

MS (FAB) m/z : 518 (M+H)⁺.

### (Example 17) (E)-4-(Acetylsulfanyl)-1-[2-cyclopropyl-1-(2-fluorophenyl)-2-oxoethyl]-3-(2- {4-[2-(ethoxycarbonyl)ethyl]-3-oxopiperazin-1-yl}ethylidene)piperidine (Compound of Compound No. 5-108)

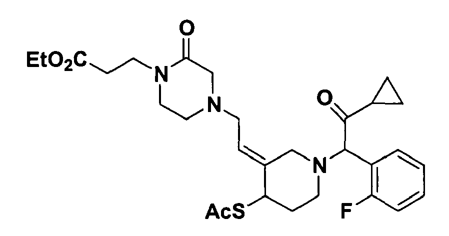

### (a) (E)-4-(t-Butyldimethylsilyloxy)-3-(2-{4-[2-(ethoxycarbonyl)ethyl]-3-oxopiperazin-1-yl}ethylidene)-1-(triphenyhmethyl)piperidine

The title compound was synthesized in a yield of 44% as a pale yellow oil using 1-[2-(ethoxycarbonyl)-ethyl]-2-oxopiperazine hydrochloride instead of morpholine by conducting a reaction similar to that mentioned in Example 1 (d).

¹H NMR (500 MHz, CDCl₃) δ ppm : -0.01 (3H, s), -0.02 (3H, s), 0.86 (9H, s), 1.27 (3H, t, J=7.0), 1.70-1.95 (4H, m), 2.59-2.67 (2H, m), 2.63 (2H, t, J=7.0), 2.84-2.97 (2H, m), 2.99-3.17 (2H, m), 3.11 (2H, s), 3.34-3.42 (2H, m), 3.64 (2H, t, J=7.0), 3.83-3.92 (1H, m), 4.14 (2H, q, J=7.0), 5.57-5.64 (1H, m), 7.10-7.21 (3H, m), 7.22-7.33 (6H, m), 7.37-7.57 (6H, m).

### (b) (E)-3-(2-{4-[2-(Ethoxycarbonyl)ethyl]-3-oxopiperazin-1-yl}ethylidene)-4-hydroxy-1-(triphenylmethyl)piperidine

The title compound was synthesized in a yield of 96% as a pale yellow amorphous solid using (E)-4-(t-butyldimethylsilyloxy)-3-(2-{4-[2-(ethoxycarbonyl)ethyl]-3-oxopiperazin-1-yl} ethylidene)-1-(triphenylmethyl)piperidine obtained in (a) above by conducting a reaction similar to that mentioned in Example 1 (e).

¹H NMR (500 MHz, CDCl₃) δ ppm : 1.26 (3H, t, J=7.0), 1.64-1.87 (4H, m), 2.62 (2H, t, J=7.0), 2.64-2.71 (2H, m), 2.98-3.17 (2H, m), 3.12 (2H, s), 3.35-3.43 (4H, m), 3.63 (2H, t, J=7.0), 3.91-4.01 (1H, m), 4.14 (2H, q, J=7.0), 5.58 (1H, t, J=7.0), 7.10-7.20 (3H, m), 7.21-7.33 (6H, m), 7.37-7.57 (6H, m).

### (c) (E)-3-(2-{4-[2-(Ethoxycarbonyl)ethyl]-3-oxopiperazin-1-yl}ethylidene)-4-hydroxypiperidine bis(hydrogen acetate)

The title compound was synthesized in a yield of 98% as a pale brown oil using (E)-3-(2-{4-[2-(ethoxycarbonyl)ethyl]-3-oxopiperazin-1-yl}ethylidene)-4-hydroxy-1-(triphenylmethyl)piperidine obtained in (b) above by conducting a reaction similar to that mentioned in Example 5 (c).

¹H NMR (500 MHz, CDCl₃) δ ppm : 1.26 (3H, t, J=7.0), 1.63-1.73 (2H, m), 2.61 (2H, t, J=7.0), 2.65-2.72 (2H, m), 3.07-3.15 (4H, m), 3.17-3.41 (6H, m), 3.62 (2H, t, J=7.0), 4.13 (2H, q, J=7.0), 4.17-4.22 (1H, m), 5.61 (1H, t, J=7.0).

### (d) (E)-1-[2-Cyclopropyl-1-(2-fluorophenyl)-2-oxoethyl]-3-(2-{4-[2-(ethoxycarbonyl)ethyl]-3-oxopiperazin-1-yl}ethylidene)-4-hydroxypiperidine

The title compound was synthesized in a yield of 77% as a pale brown oil using (E)-3-(2-{4-[2-(ethoxycarbonyl)ethyl]-3-oxopiperazin-1-yl}ethylidene)-4-hydroxypiperidine bis(hydrogen acetate) obtained in (c) above by conducting a reaction similar to that mentioned in Example 1 (g).

¹H NMR (500 MHz, CDCl₃) δ ppm : 0.78-0.90 (2H, m), 0.95-1.09 (2H, m), 1.26 (3H, t, J=7.0), 1.52-1.65 (2H, m), 1.67-1.78 (1H, m), 1.95-2.04 (1H, m), 2.13-2.22 (1H, m), 2.44-2.52 (1H, m), 2.56-2.68 (4H, m), 2.72 and 2.96 (total 1H, each d, J=13.0), 2.79-2.90 (1H, m), 2.93-3.02 (2H, m), 3.32 and 3.47 (total 1H, each d, J=13.0), 3.35-3.41 (2H, m), 3.63 (2H, t, J=7.0), 4.05-4.12 (1H, m), 4.14 (2H, q, J=7.0), 4.70 and 4.71 (total 1H, each s), 5.53-5.60 (1H, m), 7.08-7.21 (2H, m), 7.29-7.44 (2H, m).

### (e) (E)-4-(Acetylsulfanyl)-1-[2-cyclopropyl-1-(2-fluorophenyl)-2-oxoethyl]-3-(2-{4-[2-(ethoxycarbonyl)ethyl]-3-oxopiperazin-1-yl} ethylidene)piperidine

The title compound was synthesized in a yield of 22% as a pale brown oil using (E)-1-[2-cyclopropyl-1-(2-fluorophenyl)-2-oxoethyl]-3-(2-{4-[2-(ethoxycarbonyl)ethyl]-3-oxopiperazin-1-yl} ethylidene)-4-hydroxypiperidine obtained in (d) above by conducting a reaction similar to that mentioned in Example 1 (h).

¹H NMR (500 MHz, CDCl₃) δ ppm : 0.77-1.08 (4H, m), 1.26 (3H, t, J=7.0), 1.76-1.84 (1H, m), 2.08-2.24 (2H, m), 2.29 and 2.30 (total 3H, each s), 2.46-2.72 (6H, m), 2.74-2.82 (1H, m), 2.89-3.00 (2H, m), 3.01-3.09 (2H, m), 3.05 and 3.27 (total 1H, each d, J=13.0), 3.33-3.40 (2H, m), 3.63 (2H, t, J=7.0), 4.14 (2H, q, J=7.0), 4.26-4.35 (1H, m), 4.68 and 4.70 (total 1H, each s), 5.60 (1H, t, J=7.0), 7.08-7.20 (2H, m), 7.29-7.43 (2H, m);

MS (FAB) m/z : 560 (M+H)⁺.

### (Example 18) (E)-1-[2-Cyclopropyl-1-(2-fluorophenyl)-2-oxoethyl]-3-(2-{4-[2-(methoxycarbonyl)ethyl]-3-oxopiperazin-1-yl}ethylidene)-4-sulfanylpiperidine dihydrochloride (Hydrochloride of the compound of Compound No. 5-105)

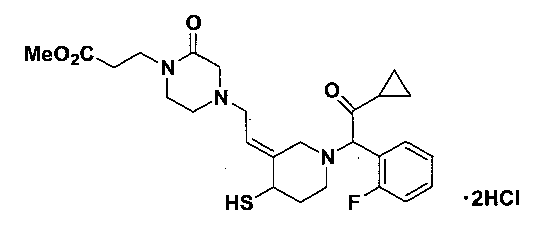

The title compound was synthesized in a yield of 71 % as a white powder crystal using (E)-4-(acetylsulfanyl)-1-[2-cyclopropyl-1-(2-fluorophenyl)-2-oxoethyl]-3-(2-{4-[2-(ethoxycarbonyl)ethyl]-3-oxopiperazin-1-yl}ethylidene)piperidine obtained in Example 17 (e) by conducting a reaction similar to that mentioned in Example 2.

¹H NMR (500 MHz, CDCl₃) δ ppm : 0.78-0.90 (2H, m), 0.96-1.08 (2H, m), 1.68-1.78 (1H, m), 2.12-2.26 (2H, m), 2.53-2.68 (5H, m), 2.74-2.85 (1H, m), 2.90-3.01 (2H, m), 3.03-3.10 (3H, m), 3.19 (1H, s), 3.34-3.43 (2H, m), 3.63 (2H, t, J=7.0), 3.67-3.81 (1H, m), 3.69 (3H, s), 4.71 and 4.73 (total 1H, each s), 5.55 and 5.59 (total 1H, each t, J=7.0), 7.08-7.21 (2H, m), 7.30-7.36 (1H, m), 7.38-7.43 (1H, m);

MS (FAB) m/z : 504 (M+H)⁺.

### (Example 19) (E)-3-(2-{4-[2-(Carboxyethyl)]-3-oxopiperazin-1-yl}ethylidene)-1-[2-cyclopropyl-1-(2-fluorophenyl)-2-oxoethyl]-4-sulfanylpiperidine dihydrochloride (Hydrochloride of the compound of Compound No. 5-103)

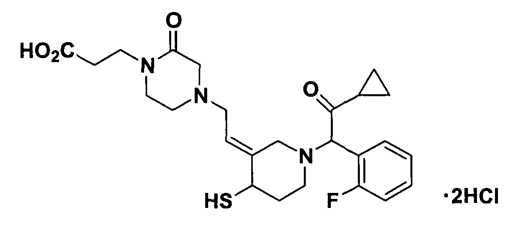

The title compound was synthesized in a yield of 48% as a white powder crystal using (E)-1-[2-cyclopropyl-1-(2-fluorophenyl)-2-oxoethyl]-3-(2-{4-[2-(methoxycarbonyl)ethyl]-3-oxopiperazin-1-yl}ethylidene)-4-sulfanylpiperidine obtained in Example 18 by conducting a reaction similar to that mentioned in Example 15.

¹H NMR (400 MHz, pyridine-d₅) δ ppm : 0.77-1.11 (4H, m), 1.76-1.87 (1H, m), 2.14-2.27 (1H, m), 2.40-2.48 (1H, m), 2.64-2.73 (3H, m), 2.87-2.99 (3H, m), 3.04-3.13 (2H, m), 3.28-3.34 (3H, m), 3.44-3.47 (2H, m), 3.50 and 3.59 (total 1H, each d, J=12.5), 3.85-3.93 (3H, m), 4.95 (1H, s), 5.77-5.82 (1H, m), 7.21-7.37 (3H, m), 7.69-7.75 (1H, m);

MS (FAB) m/z : 490 (M+H)⁺.

### (Example 20) (E)-4-(Acetylsulfanyl)-3-(2-{4-[2-(carboxyethyl)]-3-oxopiperazin-1-yl} ethylidene)-1-[2-cyclopropyl-1-(2-fluorophenyl)-2-oxoethyl]piperidine dihydrochloride (Hydrochloride of the compound of Compound No. 5-104)

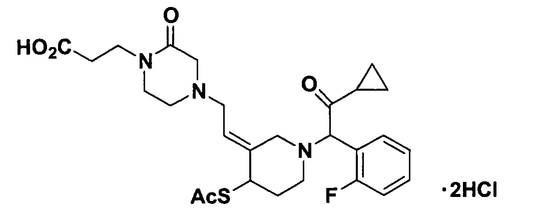

The title compound was synthesized in a yield of 35% as a pale yellow powder crystal using (E)-3-(2-{4-[2-(carboxyethyl)]-3-oxopiperazin-1-yl} ethylidene)-1-[2-cyclopropyl-1-(2-fluorophenyl)-2-oxoethyl]-4-sulfanylpiperidine dihydrochloride obtained in Example 19 by conducting a reaction similar to that mentioned in Example 16.

¹H NMR (500 MHz, CDCl₃) δ ppm : 0.78-1.17 (4H, m), 1.84-1.90 (1H, m), 2.21-2.29 (1H, m), 2.25 and 2.26 (total 3H, each s), 2.39-2.46 (1H, m), 2.57-2.88 (3H, m), 2.90-2.94 (3H, m), 2.98-3.01 (2H, m), 3.19-3.28 (2H, m), 3.33-3.38 (2H, m), 3.41-3.44 (2H, m), 3.88-3.93 (2H, m), 4.54-4.55 (1H, m), 4.95 and 4.96 (total 1H, each s), 5.82-5.86 (1H, m), 7.21-7.38 (3H, m), 7.66-7.73 (1H, m);

MS (FAB) m/z : 532 (M+H)⁺.

### (Example 21) (E)-4-(Acetylsulfanyl)-1-[2-cyclopropyl-1-(2-fluorophenyl)-2-oxoethyl]-3-(2-{4-[2-(ethoxycarbonyl)ethyl]-2-oxopiperazin-1-yl}ethylidene)piperidine dihydrochloride (Hydrochloride of the compound of Compound No. 5-80)

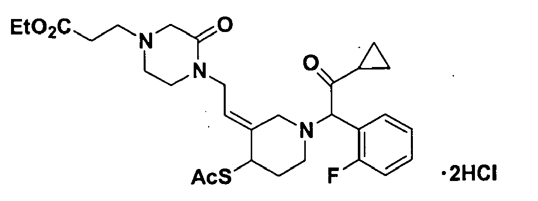

### (a) (E)-4-(t-Butyldimethylsilyloxy)-3-(2-{4-[2-(ethoxycarbonyl)ethyl]-2-oxopiperazin-1-yl} ethylidene)-1-(triphenylmethyl)piperidine

To a solution of sodium hydride (1.94 g) in N,N-dimethylformamide (430 ml) was added a solution of (E)-4-(t-butyldimethylsilyloxy)-3-[2-(tosyloxy)ethylidene]-1-(triphenylmethyl)piperidine (27.37 g) obtained in Example 1 (c) and 1-[2-(ethoxycarbonyl)ethyl]-3-oxopiperazine (9.70 g) in N,N-dimethylformamide (100 ml) under ice cooling, and the resulting mixture was stirred at room temperature for 3 hours. The reaction mixture was charged with a saturated aqueous sodium chloride solution and then extracted with ethyl acetate. The extract was washed with a saturated aqueous sodium chloride solution, and the organic layer was dried over anhydrous sodium sulfate. The solvent was removed in vacuo, and the residue was purified by chromatography on a silica gel column (ethyl acetate / hexane = 2 / 3 - 1 / 1) to afford the title compound (14.79 g, yield 54%) as a pale yellow amorphous solid.

¹H NMR (500 MHz, CDCl₃) δ ppm : -0.05 (3H, s), -0.02 (3H, s), 0.83 (9H, s), 1.26 (3H, t, J=7.5), 1.73-1.94 (2H, m), 2.48 (2H, t, J=7.5), 2.65-2.76 (4H, m), 2.84-2.93 (1H, m), 3.16 (2H, s), 3.20-3.27 (2H, m), 3.40-3.55 (1H, m), 3.81-4.18 (7H, m), 5.41-5.48 (1H, m), 7.11-7.29 (9H, m), 7.41-7.52 (6H, m).

### (b) (E)-3-(2-{4-[2-(Ethoxycarbonyl)ethyl]-2-oxopiperazin-1-yl}ethylidene)-4-hydroxy-1-(triphenylmethyl)piperidine

The title compound was synthesized in a yield of 99% as a pale yellow amorphous solid using (E)-4-(t-butyldimethylsilyloxy)-3-(2-{4-[2-(ethoxycarbonyl)ethyl]-2-oxopiperazin-1-yl} ethylidene)-1-(triphenylmethyl)piperidine obtained in (a) above by conducting a reaction similar to that mentioned in Example 1 (e).

¹H NMR (400 MHz, CDCl₃) δ ppm : 1.26 (3H, t, J=7.0), 1.60-1.86 (2H, m), 1.98-2.11 (1H, m), 2.49 (2H, t, J=7.0), 2.65-2.77 (4H, m), 3.16 (2H, s), 3.21-3.44 (3H, m), 3.88-4.20 (7H, m), 5.44 (1H, t, J=6.5), 7.10-7.31 (9H, m), 7.39-7.53 (6H, m).

### (c) (E)-3-(2-{4-[2-(Ethoxycarbonyl)ethyl]-2-oxopiperazin-1-yl}ethylidene)-4-hydroxypiperidine bis(hydrogen acetate)

The title compound was synthesized in a yield of 99% as a pale brown oil using (E)-3-(2-{4-[2-(ethoxycarbonyl)ethyl]-2-oxopiperazin-1-yl}ethylidene)-4-hydroxy-1-(triphenylmethyl)piperidine obtained in (b) above by conducting a reaction similar to that mentioned in Example 5 (c).

¹H NMR (400 MHz, CDCl₃) δ ppm : 1.26 (3H, t, J=7.0), 1.62-1.83 (2H, m), 2.01-2.12 (1H, m), 2.49 (2H, t, J=7.0), 2.66-2.77 (4H, m), 2.90-3.01 (1H, m), 3.15 (2H, s), 3.23-3.45 (3H, m), 3.83-4.27 (6H, m), 5.57 (1H, t, J=7.5).

### (d) (E)-1-[2-Cyclopropyl-1-(2-fluorophenyl)-2-oxoethyl]-3-(2-{4-[2-(ethoxycarbonyl)ethyl]-2-oxopiperazin-1-yl} ethylidene)-4-hydroxypiperidine

The title compound was synthesized in a yield of 93% as a pale yellow oil using (E)-3-(2-{4-[2-(ethoxycarbonyl)ethyl]-2-oxopiperazin-1-yl}ethylidene)-4-hydroxypiperidine bis(hydrogen acetate) obtained in (c) above by conducting a reaction similar to that mentioned in Example 1 (g).

¹H NMR (500 MHz, CDCl₃) δ ppm : 0.78-0.91 (2H, m), 0.97-1.10 (2H, m), 1.26 (3H, t, J=7.5), 1.62-1.77 (2H, m), 1.95-2.03 (1H, m), 2.09-2.16 (1H, m), 2.43-2.54 (2H, m), 2.65-2.77 (4H, m), 2.78-2.89 (1H, m), 3.00 and 3.33 (total 1H, each d, J=12.5), 3.15 (2H, s), 3.22-3.30 (2H, m), 3.47-3.55 (1H, m), 3.88-4.19 (5H, m), 4.73 and 4.75 (total 1H, each s), 5.42-5.49 (1H, m), 7.08-7.20 (2H, m), 7.29-7.41 (2H, m).

### (e) (E)-4-(Acetylsulfanyl)-1-[2-cyclopropyl-1-(2-fluorophenyl)-2-oxoethyl]-3-(2-{4-[2-(ethoxycarbonyl)ethyl]-2-oxopiperazin-1-yl}ethylidene)piperidine dihydrochloride

The title compound was synthesized in a yield of 4% as a pale yellow powder crystal using (E)-1-[2-cyclopropyl-1-(2-fluorophenyl)-2-oxoethyl]-3-(2-{4-[2-(ethoxycarbonyl)ethyl]-2-oxopiperazin-1-yl}ethylidene)-4-hydroxypiperidine obtained in (d) above by conducting a reaction similar to that mentioned in Example 1 (h).

¹H NMR (400 MHz, CDCl₃) δ ppm : 0.75-1.09 (4H, m), 1.27 (3H, t, J=7.0), 1.72-1.84 (1H, m), 2.05-2.32 (6H, m), 2.43-2.83 (7H, m), 3.04-3.42 (6H, m), 3.77-4.35 (5H, m), 4.71 and 4.74 (total 1H, each s), 5.43-5.52 (1H, m), 7.06-7.22 (2H, m), 7.29-7.41 (2H, m);

MS (FAB) m/z : 560 (M+H)⁺.

### (Example 22) (E)-1-[2-Cyclopropyl-1-(2-fluorophenyl)-2-oxoethyl]-3-(2-{4-[2-(ethoxycarbonyl)ethyl]-2-oxopiperazin-1-yl}ethylidene)-4-sulfanylpiperidine dihydrochloride (Hydrochloride of the compound of Compound No. 5-79)

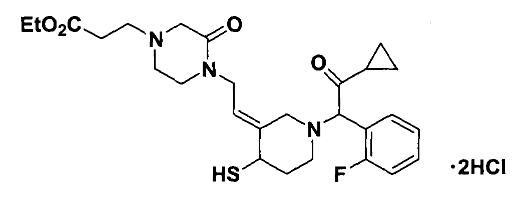

The title compound was synthesized in a yield of 80% as a white powder crystal using (E)-4-(acetylsulfanyl)-1-[2-cyclopropyl-1-(2-fluorophenyl)-2-oxoethyl]-3-(2- {4-[2-(ethoxycarbonyl)ethyl]-2-oxopiperazin-1-yl}ethylidene)piperidine obtained in Example 21 (e) by conducting a reaction similar to that mentioned in Example 12.

¹H NMR (400 MHz, pyridine-d₅) δ ppm : 0.76-0.89 (2H, m), 0.99-1.08 (2H, m), 1.13 (3H, t, J=7.5), 1.72-1.85 (1H, m), 2.12-2.26 (1H, m), 2.38-3.00 (9H, m), 3.21-3.42 (5H, m), 3.51-3.68 (1H, m), 3.81 (1H, bs), 4.06-4.21 (4H, m), 4.96 (1H, s), 5.62-5.71 (1H, m), 7.19-7.40 (3H, m), 7.66-7.76 (1H, m);

MS (FAB) m/z : 518 (M+H)⁺.

### (Example 23) (E)-3-(2-{4-[2-(Carboxyethyl)]-2-oxopiperazin-1-yl}ethylidene)-1-[2-cyclopropyl-1-(2-fluorophenyl)-2-oxoethyl]-4-sulfanylpiperidine dihydrochloride (Hydrochloride of the compound of Compound No. 5-75)

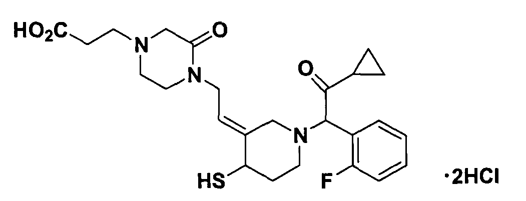

The title compound was synthesized in a yield of 88% as a white powder crystal using (E)-1-[2-cyclopropyl-1-(2-fluorophenyl)-2-oxoethyl]-3-(2-{4-[2-(ethoxycarbonyl)ethyl]-2-oxopiperazin-1-yl}ethylidene)-4-sulfanylpiperidine obtained in Example 22 by conducting a reaction similar to that mentioned in Example 15.

¹H NMR (400 MHz, pyridine-d₅) δ ppm : 0.77-0.89 (2H, m), 0.98-1.19 (2H, m), 1.71-1.84 (1H, m), 2.10-2.28 (1H, m), 2.38-3.01 (9H, m), 3.23-3.69 (6H, m), 3.81 (1H, bs), 4.05-4.29 (2H, m), 4.96 (1H, s), 5.63-5.72 (1H, m), 7.16-7.40 (3H, m), 7.67-7.78 (1H, m);

MS (FAB) m/z : 490 (M+H)⁺.

### (Example 24) (E)-4-(Acetylsulfanyl)-3-(2-{4-[2-(carboxyethyl)]-2-oxopiperazin-1-yl} ethylidene)-1-[2-cyclopropyl-1-(2-fluorophenyl)-2-oxoethyl]piperidine dihydrochloride (Hydrochloride of the compound of Compound No. 5-76)

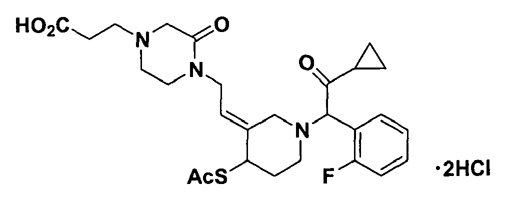

The title compound was synthesized in a yield of 44% as a pale yellow powder crystal using (E)-3-(2-{4-[2-(carboxyethyl)]-2-oxopiperazin-1-yl}ethylidene)-1-[2-cyclopropyl-1-(2-fluorophenyl)-2-oxoethyl]-4-sulfanylpiperidine dihydrochloride obtained in Example 23 by conducting a reaction similar to that mentioned in Example 16.

¹H NMR (400 MHz, pyridine-d₅) δ ppm : 0.76-0.91 (2H, m), 1.01-1.20 (2H, m), 1.81-1.90 (1H, m), 2.14-2.29 (4H, m), 2.34-2.48 (1H, m), 2.53-2.92 (8H, m), 3.17-3.72 (6H, m), 4.00-4.31 (2H, m), 4.46-4.56 (1H, m), 4.95 and 4.97 (total 1H, each s), 5.69-5.80 (1H, m), 7.18-7.40 (3H, m), 7.62-7.75 (1H, m);

MS (FAB) m/z : 532 (M+H)⁺.

### (Example 25) (E)-4-(Acetylsulfanyl)-1-[2-cyclopropyl-1-(2-fluorophenyl)-2-oxoethyl]-3- {2-[(2S)-(methoxycarbonyl)pyrrolidin-1-yl]ethylidene} piperidine dihydrochloride (Hydrochloride of the compound of Compound No. 5-148)

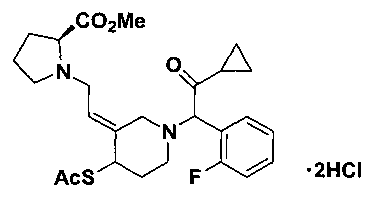

### (a) (E)-4-(t-Butyldimethylsilyloxy)-3-{2-[(2S)-(methoxycarbonyl)pyrrolidin-1-yl]ethylidene}-1-(triphenylmethyl)piperidine

To a solution of (E)-4-(t-butyldimethylsilyloxy)-3-[2-(tosyloxy)ethylidene]-1-(triphenylmethyl)piperidine (4.50 g) obtained in Example 1 (c) in N,N-dimethylformamide (60 ml) were added (2S)-(methoxycarbonyl)pyrrolidine hydrochloride (1.14 g) and triethylamine (2.11 ml) under ice cooling, and the resulting mixture was stirred at room temperature overnight. The reaction mixture was charged with a saturated aqueous sodium chloride solution and then extracted with ethyl acetate. The extract was washed with a saturated aqueous sodium chloride solution, and the organic layer was dried over anhydrous sodium sulfate. The solvent was removed in vacuo, and the residue was purified by chromatography on a silica gel column (ethyl acetate / hexane = 1 / 9) to afford the title compound (1.46 g, yield 35%) as a colorless amorphous solid.

¹H NMR (500 MHz, CDCl₃) δ ppm : -0.04 (3H, s), -0.02 (3H, s), 0.84 (9H, s), 1.69-1.98 (7H, m), 2.03-2.13 (1H, m), 2.31-2.54 (1H, m), 2.71-2.98 (1H, m), 3.03-3.29 (3H, m), 3.37-3.48 (2H, m), 3.59 and 3.62 (total 3H, each s), 3.80-3.93 (1H, m), 5.63-5.73 (1H, m), 7.08-7.19 (3H, m), 7.20-7.30 (6H, m), 7.36-7.56 (6H, m).

### (b) (E)-4-Hydroxy-3-{2-[(2S)-(methoxycarbonyl)pyrrolidin-1-yl]ethylidene}-1-(triphenylmethyl)piperidine

The title compound was synthesized in a yield of 99% as a pale yellow amorphous solid using (E)-4-(t-butyldimethylsilyloxy)-3-{2-[(2S)-(methoxycarbonyl)pyrrolidin-1-yl]ethylidene}1-(triphenylmethyl)piperidine obtained in (a) above by conducting a reaction similar to that mentioned in Example 1 (e).

¹H NMR (500 MHz, CDCl₃) δ ppm : 1.73-1.85 (2H, m), 1.87-1.98 (2H, m), 2.01-2.18 (3H, m), 2.33-2.43 (2H, m), 2.53-2.75 (1H, m), 3.05-3.23 (4H, m), 3.26-3.37 (1H, m), 3.64 and 3.66 (total 3H, each s), 3.92-4.04 (1H, m), 5.60-5.70 (1H, m), 7.10-7.20 (3H, m), 7.21-7.32 (6H, m), 7.37-7.57 (6H, m).

### (c) (E)-4-Hydroxy-3-{2-[(2S)-(methoxycarbonyl)pyrrolidin-1-yl]ethylidene}piperidine bis(hydrogen acetate)

The title compound was synthesized in a quantitative yield as a pale brown oil using (E)-4-hydroxy-3-{2-[(2S)-(methoxycarbonyl)pyrrolidin-1-yl]ethylidene}-1-(triphenylmethyl)piperidine obtained in (b) above by conducting a reaction similar to that mentioned in Example 5 (c).

¹H NMR (500 MHz, CDCl₃) δ ppm : 1.71-1.81 (2H, m), 1.87-2.05 (3H, m), 2.09-2.19 (2H, m), 2.34-2.43 (1H, m), 2.90-2.98 (1H, m), 3.09-3.33 (4H, m), 3.37-3.45 (1H, m), 3.73 and 3.74 (total 3H, each s), 3.78 (1H, d, J=14.0), 4.18-4.25 (1H, m), 5.70 (1H, t, J=7.5).

### (d) (E)-1-[2-Cyclopropyl-1-(2-fluorophenyl)-2-oxoethyl]-4-hydroxy-3-{2-[(2S)-(methoxycarbonyl)pyrrolidin-1-yl]ethylidene}piperidine

The title compound was synthesized in a yield of 54% as a pale brown oil using (E)-4-hydroxy-3-{2-[(2S)-(methoxycarbonyl)pyrrolidin-1-yl]ethylidene}piperidine bis(hydrogen acetate) obtained in (c) above by conducting a reaction similar to that mentioned in Example 1 (g).

¹H NMR (500 MHz, CDCl₃) δ ppm : 0.79-0.90 (2H, m), 0.96-1.09 (2H, m), 1.66-1.84 (2H, m), 1.87-2.02 (3H, m), 2.06-2.16 (1H, m), 2.20-2.29 (1H, m), 2.31-2.39 (1H, m), 2.42-2.56 (1H, m), 2.74-2.86 (1H, m), 2.85 and 2.95 (total 1H, each d, J=12.5), 2.98 and 3.06 (total 1H, each d, J=12.5), 3.08-3.19 (2H, m), 3.21-3.45 (2H, m), 3.70 and 3.71 (total 3H, each s), 4.06-4.16 (1H, m), 4.68 and 4.69 and 4.70 (total 1H, each s), 5.61-5.70 (1H, m), 7.06-7.21 (2H, m), 7.28-7.35 (1H, m), 7.39-7.47 (1H, m).

### (e) (E)-4-(Acetylsulfanyl)-1-[2-cyclopropyl-1-(2-fluorophenyl)-2-oxoethyl]-3-{2-[(2S)-(methoxycarbonyl)pyrrolidin-1-yl]ethylidene}piperidine dihydrochloride

The title compound was synthesized in a yield of 22% as a pale brown powder crystal using (E)-1-[2-cyclopropyl-1-(2-fluorophenyl)-2-oxoethyl]-4-hydroxy-3- {2-[(2S)-(methoxycarbonyl)pyrrolidin-1-yl]ethylidene}piperidine obtained in (d) above by conducting a reaction similar to that mentioned in Example 1 (h).

¹H NMR (500 MHz, CDCl₃) δ ppm : 0.78-0.90 (2H, m), 0.95-1.09 (2H, m), 1.72-1.82 (2H, m), 1.84-1.97 (2H, m), 2.04-2.40 (4H, m), 2.28 and 2.29 (total 3H, each s), 2.43-2.63 (1H, m), 2.65-2.78 (1H, m), 2.81-3.35 (6H, m), 3.69 and 3.70 and 3.71 and 3.72 (total 3H, each s), 4.26-4.32 (1H, m), 4.65 and 4.67 and 4.68 and 4.69 (total 1H, each s), 5.67-5.73 (1H, m), 7.07-7.19 (2H, m), 7.29-7.35 (1H, m), 7.39-7.45 (1H, m);

MS (FAB) m/z : 489 (M+H)⁺.

### (Example 26) (E)-1-[2-Cyclopropyl-1-(2-fluorophenyl)-2-oxoethyl]-3-{2-[(2S)-(methoxycarbonyl)pyrrolidin-1-yl]ethylidene}-4-sulfanylpiperidine dihydrochloride (Hydrochloride of the compound of Compound No. 5-147)

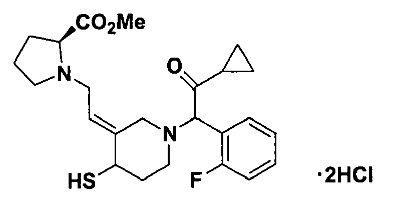

The title compound was synthesized in a yield of 46% as a pale pink powder crystal using (E)-4-(acetylsulfanyl)-1-[2-cyclopropyl-1-(2-fluorophenyl)-2-oxoethyl]-3-{2-[(2S)-(methoxycarbonyl)pyrrolidin-1-yl]ethylidene}piperidine obtained in Example 25 (e) by conducting a reaction similar to that mentioned in Example 2.

¹H NMR (500 MHz, CDCl₃) δ ppm : 0.78-0.90 (2H, m), 0.95-1.08 (2H, m), 1.66-1.99 (4H, m), 2.05-2.38 (4H, m), 2.53-2.66 (1H, m), 2.67-2.86 (1H, m), 3.01-3.39 (6H, m), 3.65-3.79 (1H, m), 3.71 and 3.72 (total 3H, each s), 4.69 and 4.71 (total 1H, each s), 5.63-5.71 (1H, m), 7.07-7.19 (2H, m), 7.28-7.35 (1H, m), 7.40-7.46 (1H, m);

MS (FAB) m/z : 447 (M+H)⁺.

### (Example 27) (E)-3-{2-[(2S)-Carboxypyrrolidin-1-yl]ethylidene}-1-[2-cyclopropyl-1-(2-fluorophenyl)-2-oxoethyl]-4-sulfanylpiperidine dihydrochloride (Hydrochloride of the compound of Compound No. 5-145)

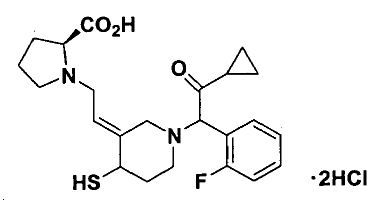

The title compound was synthesized in a yield of 76% as a pale yellow powder crystal using (E)-1-[2-cyclopropyl-1-(2-fluorophenyl)-2-oxoethyl]-3-{2-[(2S)-(methoxycarbonyl)pyrrolidin-1-yl]ethylidene}-4-sulfanylpiperidine obtained in Example 26 by conducting a reaction similar to that mentioned in Example 15.

¹H NMR (400 MHz, pyridine-d₅) δ ppm : 0.72-0.90 (2H, m), 0.95-1.20 (2H, m), 1.67-2.26 (4H, m), 2.28-2.53 (3H, m), 2.55-3.21 (3H, m), 3.37-4.38 (7H, m), 4.94-5.09 (1H, m), 6.10-6.31 (1H, m), 7.11-7.37 (3H, m), 7.62-7.83 (1H, m);

MS (FAB) m/z : 433 (M+H)⁺.

### (Example 28) (E)-4-(Acetylsulfanyl)-3-{2-[(2S)-carboxypyrrolidin-1-yl]ethylidene}-1-[2-cyclopropyl-1-(2-fluorophenyl)-2-oxoethyl]piperidine dihydrochloride (Hydrochloride of the compound of Compound No. 5-146)

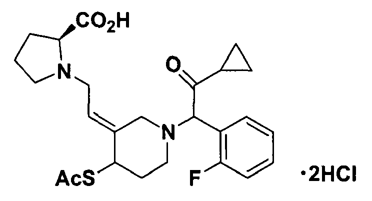

To a suspension of (E)-3-{2-[(2S)-carboxypyrrolidin-1-yl]ethylidene}-1-[2-cyclopropyl-1-(2-fluorophenyl)-2-oxoethyl]-4-sulfanylpiperidine dihydrochloride (100 mg) obtained in Example 27 in dichloromethane (5 ml) were added acetic anhydride (28 µl) and triethylamine (97 µl) under ice cooling, and the resulting mixture was stirred at room temperature for 2 hours. The solvent was removed in vacuo, and the residue was purified using a preparative HPLC (YMC-Pack ODS-A; YMC, eluent: acetonitrile / water containing triethylamine (0.2%) and acetic acid (0.2%) = 3/7) to afford the free form (57 mg, yield 61 %) of the title compound as a pale brown oil. To a solution of this compound in dichloromethane (1 ml) was added a 4N solution of hydrogen chloride in dioxane (60 µl) at room temperature, and the reaction mixture was evaporated in vacuo. The residue was crystallized from ether to afford the title compound (41 mg, yield 62%) as a pale brown powder crystal.

¹H NMR (400 MHz, pyridine-d₅) δ ppm : 0.72-0.92 (2H, m), 0.99-1.21 (2H, m), 1.71-2.00 (3H, m), 2.12-2.35 (2H, m), 2.25 and 2.26 and 2.27 (total 3H, each s), 2.38-2.89 (4H, m), 3.17-3.50 (3H, m), 3.55-4.06 (4H, m), 4.53 (1H, m), 4.94 and 4.97 and 4.99 (total 1H, each s), 6.10-6.21 (1H, m), 7.16-7.39 (3H, m), 7.63-7.77 (1H, m);

MS (FAB) m/z : 475 (M+H)⁺.

### (Example 29) (E)-4-(Acetylsulfanyl)-1-[2-cyclopropyl-1-(2-fluorophenyl)-2-oxoethyl]-3-{2-[4-(ethoxycarbonyl)piperidin-1-yl]ethylidene}piperidine dihydrochloride (Hydrochloride of the compound of Compound No. 5-8)

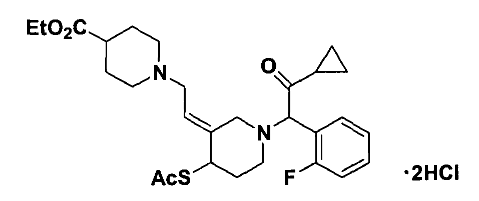

### (a) (E)-4-(t-Butyldimethylsilyloxy)-3-{2-[4-(ethoxycarbonyl)piperidin-1-yl]ethylidene}-1-(triphenylmethyl)piperidine

The title compound was synthesized in a yield of 37% as a colorless amorphous solid using 4-(ethoxycarbonyl)piperidine instead of morpholine by conducting a reaction similar to that mentioned in Example 1 (d).

¹H NMR (500 MHz, CDCl₃) δ ppm : -0.04 (3H, s), -0.02 (3H, s), 0.83 (9H, s), 1.25 (3H, t, J=7.0), 1.68-2.07 (10H, m), 2.20-2.30 (1H, m), 2.77-2.91 (3H, m), 2.93-3.10 (2H, m), 3.27-3.65 (1H, m), 3.82-3.92 (1H, m), 4.13 (2H, q, J=7.0), 5.57-5.66 (1H, m), 7.06-7.19 (3H, m), 7.20-7.30 (6H, m), 7.36-7.58 (6H, m).

### (b) (E)-3-{2-[4-(Ethoxycarbonyl)piperidin-1-yl]ethylidene}-4-hydroxy-1-(triphenylmethyl)piperidine

The title compound was synthesized in a yield of 99% as a colorless oil using (E)-4-(t-butyldimethylsilyloxy)-3-{2-[4-(ethoxycarbonyl)piperidin-1-yl]ethylidene}-1-(triphenylmethyl)piperidine obtained in (a) above by conducting a reaction similar to that mentioned in Example 1 (e).

¹H NMR (400 MHz, CDCl₃) δ ppm : 1.25 (3H, t, J=7.0), 1.64-2.11 (10H, m), 2.24-2.35 (1H, m), 2.83-2.94 (2H, m), 2.94-3.07 (2H, m), 3.35-3.44 (2H, m), 3.95-4.01 (1H, m), 4.14 (2H, q, J=7.0), 5.62 (1H, t, J=6.0), 7.13-7.21 (3H, m), 7.23-7.33 (6H, m), 7.41-7.55 (6H, m).

### (c) (E)-3-{2-[4-(Ethoxycarbonyl)piperidin-1-yl]ethylidene}-4-hydroxypiperidine bis(hydrogen acetate)

The title compound was synthesized in a yield of 99% as a brown crystal using (E)-3-{2-[4-(ethoxycarbonyl)piperidin-1-yl]ethylidene}-4-hydroxy-1-(triphenylmethyl)piperidine obtained in (b) above by conducting a reaction similar to that mentioned in Example 5 (c).

¹H NMR (500 MHz, CDCl₃) δ ppm : 1.25 (3H, t, J=7.0), 1.41-1.50 (1H, m), 1.60-2.12 (6H, m), 2.23-2.34 (1H, m), 2.83-3.09 (5H, m), 3.30 (2H, q, J=7.0), 3.39-3.51 (1H, m), 3.75-3.82 (1H, m), 4.13 (2H, q, J=7.0), 4.19-4.25 (1H, m), 5.58-5.75 (1H, m).

### (d) (E)-1-[2-Cyclopropyl-1-(2-fluorophenyl)-2-oxoethyl]-3-{2-[4-(ethoxycarbonyl)piperidin-1-yl]ethylidene}-4-hydroxypiperidine

The title compound was synthesized in a yield of 63% as a pale brown oil using (E)-3-{2-[4-(ethoxycarbonyl)piperidin-1-yl]ethylidene}-4-hydroxypiperidine bis(hydrogen acetate) obtained in (c) above by conducting a reaction similar to that mentioned in Example 1 (g).

¹H NMR (500 MHz, CDCl₃) δ ppm : 0.76-1.10 (4H, m), 1.25 (3H, t, J=7.0), 1.42-1.51 (1H, m), 1.65-2.04 (8H, m), 2.08-2.23 (2H, m), 2.27-2.37 (1H, m), 2.42-2.53 (1H, m), 2.76 and 2.96 (total 1H, each d, J=12.5), 2.77-2.95 (1H, m), 3.00-3.07 (2H, m), 3.32 and 3.45 (total 1H, each d, J=12.5), 4.06-4.11 (1H, m), 4.13 (2H, q, J=7.0), 4.69 and 4.72 (total 1H, each s), 5.61-5.70 (1H, m), 7.07-7.21 (2H, m), 7.28-7.45 (2H, m).

### (e) (E)-4-(Acetylsulfanyl)-1-[2-cyclopropyl-1-(2-fluorophenyl)-2-oxoethyl]-3-{2-[4-(ethoxycarbonyl)piperidin-1-yl]ethylidene} piperidine dihydrochloride

The title compound was synthesized in a yield of 17% as a yellow powder crystal using (E)-1-[2-cyclopropyl-1-(2-fluorophenyl)-2-oxoethyl]-3-{2-[4-(ethoxycarbonyl)piperidin-1-yl]ethylidene}-4-hydroxypiperidine obtained in (d) above by conducting a reaction similar to that mentioned in Example 1 (h).

¹H NMR (500 MHz, CDCl₃) δ ppm : 0.77-1.11 (4H, m), 1.25 (3H, t, J=7.0), 1.67-1.98 (7H, m), 2.07-2.29 (3H, m), 2.28 and 2.29 (total 3H, each s), 2.47-2.62 (1H, m), 2.65-2.96 (5H, m), 2.81 and 3.02 (total 1H, each d, J=12.5), 3.26-3.39 (1H, m), 4.13 (2H, q, J=7.0), 4.27-4.36 (1H, m), 4.66 and 4.69 (total 1H, each s), 5.64 (1H, t, J=7.0), 7.07-7.20 (2H, m), 7.28-7.45 (2H, m);

MS (FAB) m/z : 517 (M+H)⁺.

### (Example 30) (E)-1-[2-Cyclopropyl-1-(2-fluorophenyl)-2-oxoethyl]-3-{2-[4-(ethoxycarbonyl)piperidin-1-yl]ethylidene}-4-sulfanylpiperidine dihydrochloride (Hydrochloride of the compound of Compound No. 5-7)

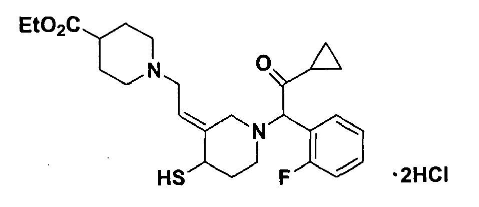

The title compound was synthesized in a yield of 97% as a pale yellow powder crystal using (E)-4-(acetylsulfanyl)-1-[2-cyclopropyl-1-(2-fluorophenyl)-2-oxoethyl]-3-{2-[4-(ethoxycarbonyl)piperidin-1-yl]ethylidene}piperidine obtained in Example 29 (e) by conducting a reaction similar to that mentioned in Example 12.

¹H NMR (500 MHz, CDCl₃) δ ppm : 0.75-1.10 (4H, m), 1.25 (3H, t, J=7.0), 1.67-1.80 (3H, m), 1.82-1.99 (4H, m), 2.10-2.31 (3H, m), 2.54-2.68 (1H, m), 2.69-2.97 (5H, m), 3.14 and 3.35 (total 1H, each d, J=12.5), 3.16-3.23 (1H, m), 3.72-3.83 (1H, m), 4.13 (2H, q, J=7.0), 4.69 and 4.71 (total 1H, each s), 5.61 (1H, m), 7.05-7.21 (2H, m), 7.23-7.46 (2H, m);

MS (FAB) m/z : 475 (M+H)⁺.

### (Example 31) (E)-3-{2-[4-(Carboxypiperidine)-1-yl]ethylidene}-1-[2-cyclopropyl-1-(2-fluorophenyl)-2-oxoethyl]-4-sulfanylpiperidine dihydrochloride (Hydrochloride of the compound of Compound No. 5-3)

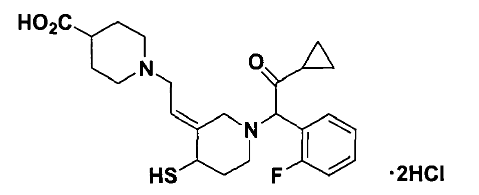

The title compound was synthesized in a yield of 90% as a pale yellow powder crystal using (E)-1-[2-cyclopropyl-1-(2-fluorophenyl)-2-oxoethyl]-3-{2-[4-(ethoxycarbonyl)piperidin-1-yl]ethylidene}-4-sulfanylpiperidine dihydrochloride obtained in Example 30 by conducting a reaction similar to that mentioned in Example 15.

¹H NMR (400 MHz, pyridine-d₅) δ ppm : 0.73-0.83 (2H, m), 1.03-1.13 (2H, m), 1.73-1.81 (1H, m), 2.13-2.31 (2H, m), 2.47 (2H, br), 2.59-2.98 (7H, m), 3.04-3.27 (1H, m), 3.39 and 3.45 (total 1H, each d, J=12.0), 3.51-3.68 (2H, m), 3.80-3.89 (3H, m), 5.00 and 5.01 (total 1H, each s), 6.41 (1H, t, J=7.5), 7.22-7.25 (2H, m), 7.27-7.35 (1H, m), 7.58-7.60 (1H, m);

MS (FAB) m/z : 447 (M+H)⁺.

### (Example 32) (E)-4-(Acetylsulfanyl)-1-[2-cyclopropyl-1-(2-fluorophenyl)-2-oxoethyl]-3-(2-hydroxyethylidene)piperidine (Compound of Compound No. 3-2)

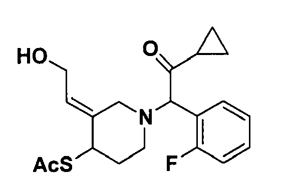

### (a) (E)-4-(Acetylsulfanyl)-3-[(ethoxycarbonyl)methylidene]-1-(triphenylmethyl)piperidine

To a solution of (E)-3-[(ethoxycarbonyl)methylidene]-1-(triphenylmethyl)piperidin-4-ol (23.06 g) in dichloromethane (200 ml) were added methanesulfonyl chloride (4.60 ml) and triethylamine (9.77 ml) under ice cooling, and the resulting mixture was stirred at room temperature for 1 hour. After a saturated aqueous sodium hydrogen carbonate solution was poured to the reaction mixture and the resulting mixture was extracted with dichloromethane, the organic layer was dried over anhydrous magnesium sulfate. The solvent was removed in vacuo to afford a crude product of (E)-3-[(ethoxycarbonyl)methylidene]-4-mesyloxy-1-(triphenylmethyl)piperidine as a pale yellow amorphous solid. To a solution of this compound in ethanol (250 ml) was added potassium thioacetate (12.32 g) at room temperature, and the resulting mixture was stirred at 70°C for 1 hour. The reaction mixture was evaporated in vacuo. The residue was diluted with ethyl acetate and washed with water and a saturated aqueous sodium chloride solution, and the organic layer was dried over anhydrous magnesium sulfate. The solvent was removed in vacuo, and the residue was purified by chromatography on a silica gel column (ethyl acetate / hexane = 1 / 5) to afford the title compound (18.35 g, yield 70%) as a pale yellow amorphous solid.

¹H NMR (400 MHz, CDCl₃) δ ppm : 1.11(3H, t, J=7.0), 1.89-1.99 (1H, m), 2.10-2.24 (1H, m), 2.27 (3H, s), 2.30-2.40 (2H, m), 2.51-2.65 (1H, m), 3.83 (1H, m), 3.98-4.08 (2H, m), 4.30-4.37 (1H, m), 5.98 (1H, s), 7.12-7.51 (15H, m).

### (b) (E)-3-(2-Hydroxyethylidene)-4-sulfanyl-1-(triphenylmethyl)piperidine

To a solution of (E)-4-(acetylsulfanyl)-3-[(ethoxycarbonyl)methylidene]-1-(triphenylmethyl)piperidine (18.35 g) obtained in (a) above in dichloromethane (300 ml) was added dropwise a 1.01N solution of diisobutylaluminum hydride in toluene (151 ml) at -78°C, and the resulting mixture was stirred at the same temperature for 1 hour. To the reaction mixture was added a saturated aqueous potassium sodium tartrate tetrahydrate solution, and the resulting mixture was stirred at room temperature for 3 hours. The reaction mixture was extracted with ethyl acetate and washed with water and a saturated aqueous sodium chloride solution, and the organic layer was dried over anhydrous sodium sulfate. The solvent was removed in vacuo, and the residue was purified by chromatography on a silica gel column (ethyl acetate / hexane / dichloromethane = 1 /2/1) to afford the title compound (10.00 g, yield 66%) as a pale yellow amorphous solid.

¹H NMR (400 MHz, CDCl₃) δ ppm : 1.75-1.92 (1H, m), 2.25-2.57 (3H, m), 2.74-3.13 (2H, m), 3.53-3.72 (1H, m), 4.03-4.22 (2H, m), 5.71-5.85 (1H, m), 7.10-7.60 (15H, m).

### (c) (E)-3-[2-(t-Butyldimethylsilyloxy)ethylidene]-4-sulfanyl-1-(triphenylmethyl)piperidine

To a solution of (E)-3-(2-hydroxyethylidene)-4-sulfanyl-1-(triphenylmethyl)piperidine (10.00 g) obtained in (b) above in dichloromethane (100 ml) were added t-butyldimethylsilyl chloride (5.63 g), triethylamine (5.23 ml) and 4-dimethylaminopyridine (0.15 g) under ice cooling, and the resulting mixture was stirred at room temperature for 1 hour. The reaction mixture was evaporated in vacuo. The residue was diluted with dichloromethane and washed with water and a saturated aqueous sodium chloride solution, and the organic layer was dried over anhydrous magnesium sulfate. The solvent was removed in vacuo, and the residue was purified by chromatography on a silica gel column (ethyl acetate / hexane = 1 / 10) to afford the title compound (10.66 g, yield 83%) as a colorless amorphous solid.

¹H NMR (400 MHz, CDCl₃) δ ppm : 0.01-0.05 (6H, m), 0.85-0.90 (9H, m), 1.73-1.85 (1H, m), 2.20-2.51 (3H, m), 2.86-3.20 (2H, m), 3.60-3.64 (1H, m), 4.11-4.19 (2H, m), 5.64-5.71 (1H, m), 7.12-7.53 (15H, m).

### (d) (E)-4-(Acetylsulfanyl)-3-[2-(t-butyldimethylsilyloxy)ethylidene]-1-(triphenylmethyl)piperidine

To a solution of (E)-3-[2-(t-butyldimethylsilyloxy)ethylidene]-4-sulfanyl-1-(triphenylmethyl)piperidine (10.66 g) obtained in (c) above in dichloromethane (100 ml) were added acetic anhydride (2.93 ml), pyridine (5.00 ml) and 4-dimethylaminopyridine (0.13 g) under ice cooling, and the resulting mixture was stirred at room temperature for 3 hours. The reaction mixture was evaporated in vacuo. The residue was diluted with ethyl acetate and washed with water and a saturated aqueous sodium chloride solution, and the organic layer was dried over anhydrous magnesium sulfate. The solvent was removed in vacuo, and the residue was purified by chromatography on a silica gel column (ethyl acetate / hexane = 1 / 10) to afford the title compound (10.95 g, yield 95%) as a colorless amorphous solid.

¹H NMR (400 MHz, CDCl₃) δ ppm : 0.04-0.07 (6H, m), 0.83-0.92 (9H, m), 1.75-1.92 (1H, m), 2.19-2.34 (5H, m), 2.41-2.70 (1H, m), 2.90-3.13 (2H, m), 4.06-4.21 (2H, m), 4.21-4.34 (1H, m), 5.66 (1H, t, J=6.0), 7.11-7.54 (15H, m).

### (e) (E)-4-(Acetylsulfanyl)-3-(2-hydroxyethylidene)piperidine hydrogen trifluoroacetate

To a solution of (E)-4-(acetylsulfanyl)-3-[2-(t-butyldimethylsilyloxy)ethylidene]-1-(triphenyhnethyl)piperidine (10.95 g) obtained in (d) above in dichloromethane (10 ml) was added trifluoroacetic acid (15 ml) under ice cooling, and the resulting mixture was stirred at room temperature for 1 hour. The reaction mixture was evaporated in vacuo, and the residue was purified by chromatography on a silica gel column (methanol / dichloromethane = 1/10 - 1/1) to afford the title compound (6.90 g, quantitative yield) as a brown oil.

¹H NMR (400 MHz, CDCl₃) δ ppm : 1.96-2.10 (1H, m), 2.26-2.43 (4H, m), 3.13-3.28 (1H, m), 3.29-3.41 (1H, m), 3.57-3.67 (1H, m), 4.02-4.11 (1H, m), 4.14-4.28 (2H, m), 4.40-4.45 (1H, m), 6.04-6.11 (1H, m).

### (f) (E)-4-(Acetylsulfanyl)-1-[2-cyclopropyl-1-(2-fluorophenyl)-2-oxoethyl]-3-(2-hydroxyethylidene)piperidine

The title compound was synthesized in a yield of 74% as a yellow oil using (E)-4-(acetylsulfanyl)-3-(2-hydroxyethylidene)piperidine hydrogen trifluoroacetate (6.90 g) obtained in (e) above by conducting a reaction similar to that mentioned in Example 1 (g).

¹H NMR (400 MHz, CDCl₃) δ ppm : 0.75-0.92 (2H, m), 0.96-1.11 (2H, m), 1.59-1.87 (2H, m), 2.07-2.19 (1H, m), 2.19-2.34 (3H, m), 2.44-2.63 (1H, m), 2.64-2.79 (1H, m), 2.82 and 3.09 (total 1H, each d, J=12.5), 3.26 and 3.41 (total 1H, each d, J=12.5), 3.97-4.18 (2H, m), 4.23-4.37 (1H, m), 4.73 and 4.78 (total 1H, each s), 5.75-5.87 (1H, m), 7.70-7.24 (2H, m), 7.29-7.41 (2H, m).

### (Example 33) (E)-4-(Acetylsulfanyl)-1-[2-cyclopropyl-1-(2-fluorophenyl)-2-oxoethyl]-3-{2-[4-(ethoxycarbonylmethyl)piperidin-1-yl]ethylidene}piperidine (Compound of Compound No. 5-14)

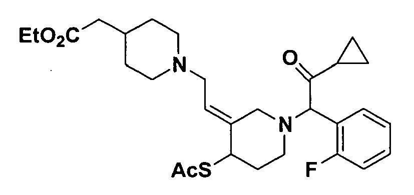

To a solution of (E)-4-(acetylsulfanyl)-1-[2-cyclopropyl-1-(2-fluorophenyl)-2-oxoethyl]-3-(2-hydroxyethylidene)piperidine (500 mg) obtained in Example 32 (f) and p-toluenesulfonic anhydride (580 mg) in acetonitrile (5 ml) was added triethylamine (0.22 ml) under ice cooling, the resulting mixture was stirred at room temperature for 5.5 hours. To the reaction mixture were added 4-(ethoxycarbonylmethyl)piperidine (450 mg) and triethylamine (0.37 ml), and the resulting mixture was stirred at room temperature for 3 hours and then allowed to stand still for 3 days. The reaction mixture was diluted with ethyl acetate and washed with water, and the organic layer was dried over anhydrous magnesium sulfate. The solvent was removed in vacuo, and the residue was roughly purified by chromatography on a silica gel column (methanol / dichloromethane =1/10) and then purified using a preparative HPLC (YMC-Pack ODS-A; YMC, eluent: acetonitrile / water containing triethylamine (0.2%) and acetic acid (0.2%)=13/7). The eluted fraction was extracted with ethyl acetate and washed with a saturated aqueous sodium chloride solution, and the organic layer was dried over anhydrous magnesium sulfate. The solvent was removed in vacuo to afford the title compound (204 mg, yield 29%) as a yellow oil.

¹H NMR (400 MHz, CDCl₃) δ ppm : 0.77-0.92 (2H, m), 0.94-1.10 (2H, m), 1.26 (3H, t, J=7.0), 1.55-1.89 (7H, m), 2.08-2.19 (2H, m), 2.21-2.26 (2H, m), 2.27-2.32 (3H, m), 2.48-2.61 (1H, m), 2.62-2.72 (1H, m), 2.72-3.40 (7H, m), 4.07-4.17 (2H, m), 4.24-4.36 (1H, m), 4.65-4.75 (1H, m), 5.69 (1H, bs), 7.08-7.22 (2H, m), 7.29-7.42 (2H, m).

### (Example 34) (E)-1-[2-Cyclopropyl-1-(2-fluorophenyl)-2-oxoethyl]-3- {2-[4-(ethoxycarbonylmethyl)piperidin-1-yl]ethylidene}-4-sulfanylpiperidine dihydrochloride (Hydrochloride of the compound of Compound No. 5-13)

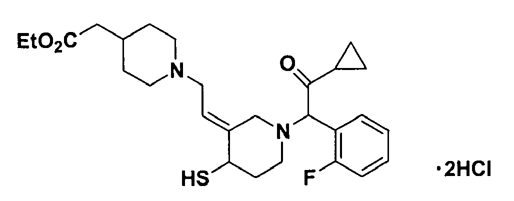

To a solution of (E)-4-(acetylsulfanyl)-1-[2-cyclopropyl-1-(2-fluorophenyl)-2-oxoethyl]-3-{2-[4-(ethoxycarbonylmethyl)piperidin-1-yl]ethylidene}piperidine (204 mg) obtained in Example 33 in ethanol (10 ml) was bubbled hydrogen chloride under ice cooling, and the resulting mixture was stirred at room temperature under tightly sealed conditions overnight. The reaction mixture was evaporated in vacuo to afford the title compound (201 mg, yield 93%) as a colorless amorphous solid.

¹H NMR (400 MHz, CDCl₃) δ ppm : 0.71-0.86 (2H, m), 1.03-1.12 (2H, m), 1.14 (3H, t, J=7.0), 1.71-1.82 (1H, m), 1.94-2.08 (2H, m), 2.09-2.49 (6H, m), 2.57-3.02 (4H, m), 3.33-3.48 (1H, m), 3.49-3.71 (4H, m), 3.76-3.83 (2H, m), 3.83-3.92 (1H, m), 4.11 (2H, q, J=7.0), 5.03 and 5.04 (total 1H, each s), 6.31-6.43 (1H, m), 7.13-7.31 (2H, m), 7.31-7.42 (1H, m), 7.54-7.65 (1H, m).

### (Example 35) (E)-3-[2-(4-Carboxymethylpiperidin-1-yl)ethylidene]-1-[2-cyclopropyl-1-(2-fluorophenyl)-2-oxoethyl]-4-sulfanylpiperidine dihydrochloride (Hydrochloride of the compound of Compound No. 5-9)

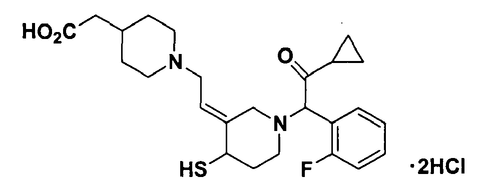

The title compound was synthesized in a yield of 57% as a colorless amorphous solid using (E)-1-[2-cyclopropyl-1-(2-fluorophenyl)-2-oxoethyl]-3-{2-[4-(ethoxycarbonylmethyl)piperidin-1-yl]ethylidene}-4-sulfanylpiperidine dihydrochloride obtained in Example 34 by conducting a reaction similar to that mentioned in Example 15.

¹H NMR (500 MHz, CDCl₃) δ ppm : 0.72-0.88 (2H, m), 1.04-1.17 (2H, m), 1.71-1.83 (1H, m), 1.94-2.11 (2H, m), 2.11-2.34 (5H, m), 2.46 (2H, bs), 2.65-2.76 (1H, m), 2.76-3.07 (4H, m), 3.46 (1H, d, J=12.5), 3.59 (1H, d, J=12.5), 3.62-3.75 (2H, m), 3.83-3.96 (2H, m), 5.08 (1H, s), 6.40 (1H, m), 7.22-7.31 (2H, m), 7.34-7.41 (1H, m), 7.61-7.67 (1H, m);

IR (KBr, cm⁻¹) :2556, 1714, 1493.

### (Example 36) (E)-4-(Acetylsulfanyl)-1-[2-cyclopropyl-1-(2-fluorophenyl)-2-oxoethyl]-3-{[1-(ethoxycarbonylmethyl)piperidin-4-yl]methylidene}piperidine dihydrochloride (Hydrochloride of the compound of Compound No. 3-32)

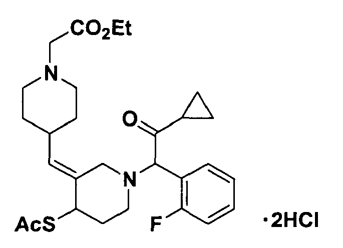

### (a) 3-{[1-(Ethoxycarbonylmethyl)piperidin-4-yl]hydroxymethyl}-1-(triphenylmethyl)piperidin-4-one

To a solution of 1-(triphenylmethyl)piperidin-4-one (22.99 g) in tetrahydrofuran (250 ml) was added dropwise a 1.0N solution of sodium bis(trimethylsilyl)amide in tetrahydrofuran (68 ml) at -78°C, and the resulting mixture was stirred at the same temperature for 20 minutes. To the reaction mixture was added dropwise a solution of 1-(ethoxycarbonylmethyl)piperidin-4-carbaldehyde (13.43 g) in tetrahydrofuran (35 ml) at -78°C, and the resulting mixture was stirred at the same temperature for 2 hours. The reaction mixture was charged with a saturated aqueous ammonium chloride solution and then extracted with a mixed solvent of ethyl acetate and diethyl ether. The extract was washed with water and a saturated aqueous sodium chloride solution, and the organic layer was dried over anhydrous sodium sulfate. The solvent was removed in vacuo, and the residue was purified by chromatography on a silica gel column (ethyl acetate / hexane = 1 / 4 - 1 / 0) to afford the title compound (12.32 g, yield 34%) as a pale yellow amorphous solid.

¹H NMR (400 MHz, CDCl₃) δ ppm : 1.27 (3H, t, J=7.0), 1.41-2.18 (8H, m), 2.30-2.51 (1H, m), 2.67-3.25 (8H, m), 3.45-3.71 (2H, m), 4.18 (2H, q, J=7.0); 7.11-7.60 (15H, m).

### (b) (E)-3-{[1-(Ethoxycarbonylmethyl)piperidin-4-yl]methylidene}-1-(triphenylmethyl)piperidin-4-one

To a solution of 3-{[1-(ethoxycarbonylmethyl)piperidin-4-yl]hydroxymethyl}-1-(triphenylmethyl)-piperidin-4-one (9.87 g) obtained in (a) above in tetrahydrofuran (150 ml) were added methanesulfonyl chloride (7.0 ml), 1,8-diazabicyclo[5.4.0]undec-7-ene (19 ml) and 4-dimethylaminopyridine (0.23 g) under ice cooling, and the resulting mixture was stirred at room temperature for 17 hours. The reaction mixture was diluted with a mixed solvent of ethyl acetate and diethyl ether and washed with water and a saturated aqueous sodium chloride solution, and the organic layer was dried over anhydrous sodium sulfate. The solvent was removed in vacuo, and the residue was purified by chromatography on a silica gel column (ethyl acetate / hexane = 1/4-4/1) to afford the title compound (3.92 g, yield 41 %) as a yellow amorphous solid.

¹H NMR (400 MHz, CDCl₃) δ ppm : 1.27 (3H, t, J=7.0), 1.50-1.66 (4H, m), 1.89-2.01 (1H, m), 2.10-2.21 (2H, m), 2.50-2.69 (4H, m), 2.83-2.93 (2H, m), 3.08-3.23 (4H, m), 4.18 (2H, q, J=7.0), 6.49 (1H, d, J=10.0), 7.14-7.33 (9H, m), 7.46-7.56 (6H, m).

### (c) (E)-3-{[1-(Ethoxycarbonylmethyl)piperidin-4-yl]methylidene}-1-(triphenylmethyl)piperidin-4-ol

To a solution of (E)-3-{[1-(ethoxycarbonylmethyl)piperidin-4-yl]methylidene}-1-(triphenylmethyl)piperidin-4-one (6.14 g) obtained in (b) above in a mixed solvent of ethanol (40 ml) and dichloromethane (40 ml) was added sodium borohydride (0.55 g) under ice cooling, and the resulting mixture was stirred at room temperature for 1 hour. The reaction mixture was diluted with ethyl acetate and washed with a saturated aqueous sodium hydrogen carbonate solution and a saturated aqueous sodium chloride solution, and the organic layer was dried over anhydrous sodium sulfate. The solvent was removed in vacuo, and the residue was purified by chromatography on a silica gel column (ethyl acetate / hexane = 2 / 3) to afford the title compound (4.17 g, yield 68%) as a colorless amorphous solid.

¹H NMR (400 MHz, CDCl₃) δ ppm : 1.29 (3H, t, J=7.5), 1.44-2.18 (7H, m), 2.77-3.22 (10H, m), 3.91-4.02 (1H, m), 4.20 (2H, q, J=7.5), 5.31 (1H, d, J=10.0), 7.09-7.30 (9H, m), 7.38-7.56 (6H, m).

### (d) (E)-4-(Acetylsulfanyl)-3-{[1-(ethoxycarbonylmethyl)piperidin-4-yl]methylidene}piperidine bis(hydrogen trifluoroacetate)

To a solution of (E)-3-{[1-(ethoxycarbonylmethyl)piperidin-4-yl]methylidene}-1-(triphenylmethyl)piperidin-4-ol (2.98 g) obtained in (c) above in toluene (50 ml) were added thioacetic acid (1.20 ml) and N,N-dimethylformamide dineopentyl acetal (4.80 ml) under ice cooling, and the resulting mixture was stirred at room temperature for 1.5 hours. The reaction mixture was diluted with ethyl acetate and washed with water and a saturated aqueous sodium chloride solution, and the organic layer was dried over anhydrous sodium sulfate. The solvent was removed in vacuo, and the residue was purified by chromatography on a silica gel column (ethyl acetate / hexane = 2 / 3) to afford (E)-4-(acetylsulfanyl)-3-{[1-(ethoxycarbonylmethyl)piperidin-4-yl]methylidene}-1-(triphenylmethyl)piperidine (1.97 g, containing impurities) as a pale yellow amorphous solid.

To a solution of the above mixture (1.97 g) in dichloromethane (30 ml) was added trifluoroacetic acid (0.60 ml) under ice cooling, and the resulting mixture was stirred at room temperature for 30 minutes. The solvent was removed in vacuo, and the residue was purified by chromatography on a silica gel column (ethanol / dichloromethane =1/4-2/3) to afford the title compound (1.53 g, yield 48%) as a yellow amorphous solid.

¹H NMR (400 MHz, CDCl₃) δ ppm : 1.29 (3H, t, J=7.0), 1.67-2.61 (10H, m), 3.00-4.03 (10H, m), 4.25 (2H, q, J=7.0), 4.39 (1H, t, J=4.5), 5.71 (1H, d, J=10.0).

### (e) (E)-4-(Acetylsulfanyl)-1-[2-cyclopropyl-1-(2-fluorophenyl)-2-oxoethyl]-3-{[1-(ethoxycarbonylmethyl)piperidin-4-yl]methylidene}piperidine dihydrochloride

The title compound was synthesized in a yield of 81 % as a colorless amorphous solid using (E)-4-(acetylsulfanyl)-3-{[1-(ethoxycarbonylmethyl)piperidin-4-yl]methylidene}piperidine bis(hydrogen trifluoroacetate) obtained in (d) above by conducting a reaction similar to that mentioned in Example 1 (g).

¹H NMR (400 MHz, CDCl₃) δ ppm : 0.76-0.89 (2H, m), 0.90-1.12 (2H, m), 1.28 (3H, t, J=7.0), 1.39-2.34 (11H, m), 2.41-2.98 (7H, m), 3.18 and 3.19 (total 2H, each s), 3.28-3.39 (1H, m), 4.13-4.33 (3H, m), 4.63 and 4.70 (total 1H, each s), 5.38 (1H, d, J=9.0), 7.05-7.21 (2H, m), 7.27-7.37 (1H, m), 7.39-7.51 (1H, m);

IR (KBr, cm⁻¹) : 2614, 1697.

### (Example 37) (E)-3-{[1-(Carboxymethyl)piperidin-4-yl]methylidene}-1-[2-cyclopropyl-1-(2-fluorophenyl)-2-oxoethyl]-4-sulfanylpiperidine dihydrochloride (Hydrochloride of the compound of Compound No. 3-27)

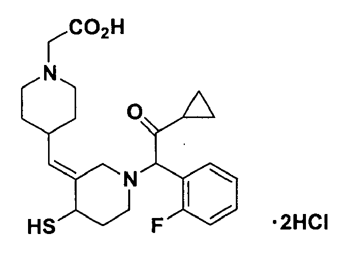

To a solution of (E)-4-(acetylsulfanyl)-1-[2-cyclopropyl-1-(2-fluorophenyl)-2-oxoethyl]-3-{[1-(ethoxycarbonylmethyl)piperidin-4-yl]methylidene}piperidine (1.13 g) obtained in Example 36 (e) in ethanol (20 ml) was bubbled hydrogen chloride under ice cooling, and the resulting mixture was stirred at room temperature under tightly sealed condition for 3 hours. After the reaction mixture was evaporated in vacuo, the obtained residue was dissolved in 3N hydrochloric acid (20 ml) and the resulting mixture was stirred at 50°C for 12.5 hours. The reaction mixture was removed in vacuo, and the residue was purified using a preparative HPLC (YMC-Pack ODS-A; YMC, eluent: acetonitrile / 0.024N hydrochloric acid = 10 / 90) to afford the title compound (0.38 g, yield 34%) as a colorless amorphous solid.

¹H NMR (400 MHz, pyridine-d₅) δ ppm : 0.75-0.87 (2H, m), 0.96-1.19 (2H, m), 1.62-2.54 (8H, m), 2.62-3.19 (4H, m), 3.31-3.87 (5H, m), 4.10 and 4.11 (total 2H, each s), 4.94 and 4.95 (total 1H, each s), 5.49 and 5.50 (total 1H, each d, J=9.0), 7.18-7.39 (3H, m), 7.70-7.78 (1H, m);

IR (KBr, cm⁻¹): 2659, 1713, 1494.

### (Example 38) (E)-1-[2-Cyclopropyl-1-(2-fluorophenyl)-2-oxoethyl]-3-{[1-(ethoxycarbonylmethyl)piperidin-4-yl]methylidene}-4-sulfanylpiperidine dihydrochloride (Hydrochloride of the compound of Compound No. 3-31)

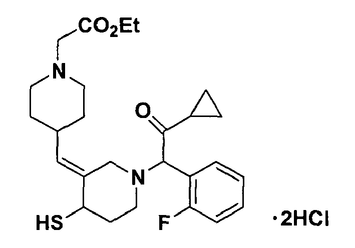

To a solution of (E)-3-{[1-(carboxymethyl)piperidin-4-yl]methylidene}-1-[2-cyclopropyl-1-(2-fluorophenyl)-2-oxoethyl]-4-sulfanylpiperidine dihydrochloride (0.17 g) obtained in Example 37 in ethanol (4 ml) was bubbled hydrogen chloride under ice cooling, and the resulting mixture was stirred at room temperature under tightly sealed conditions for 7 hours. The reaction mixture was allowed to stand still at -20°C for 3 days and then stirred at room temperature for further 7 hours.
The reaction mixture was evaporated in vacuo, and the residue was purified by chromatography on a silica gel column (ethanol / dichloromethane = 1 / 99 - 1 / 4) to afford the title compound (0.16 g, yield 89%) as a colorless amorphous solid.

¹H NMR (400 MHz, CDCl₃) δ ppm : 0.77-1.12 (4H, m), 1.28 (3H, t, J=7.0), 1.41-2.36 (8H, m), 2.55-3.36 (10H, m), 3.68-3.81 (1H, m), 4.19 (2H, q, J=7.0), 4.66 and 4.72 (total 1H, each s), 5.32 and 5.34 (total 1H, each d, J=9.0), 7.03-7.55 (4H, m);

IR (KBr, cm⁻¹): 2617, 1713, 1494.

### (Example 39) (E)-4-(Acetylsulfanyl)-3-{[(1-carboxymethyl)piperidin-4-yl]methylidene}-1-[2-cyclopropyl-1-(2-fluorophenyl)-2-oxoethyl]piperidine dihydrochloride (Hydrochloride of the compound of Compound No. 3-28)

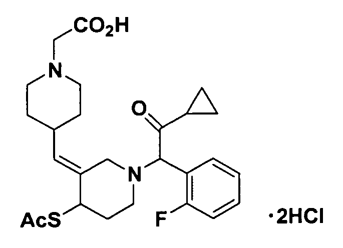

The title compound was synthesized in a yield of 52% as a colorless amorphous solid using (E)-3-{[1-(carboxymethyl)piperidin-4-yl]methylidene}-1-[2-cyclopropyl-1-(2-fluorophenyl)-2-oxoethyl]-4-sulfanylpiperidine dihydrochloride obtained in Example 37 by conducting a reaction similar to that mentioned in Example 28.

¹H NMR (400 MHz, CDCl₃) δ ppm : 0.73-0.89 (2H, m), 0.95-1.07 (2H, m), 1.67-2.34 (11H, m), 2.47-3.42 (10H, m), 4.21 and 4.27 (total 1H, each t, J=4.5), 4.73 and 4.75 (total 1H, each s), 5.37-5.45 (1H, m), 7.09-7.20 (2H, m), 7.31-7.39 (2H, m);

IR (KBr, cm⁻¹) : 2627, 1698.

### (Example 40) (E)-4-(Acetylsulfanyl)-1-[2-cyclopropyl-1-(2-fluorophenyl)-2-oxoethyl]-3-[2-(1H-pyrazol-1-yl)ethylidene]piperidine (Compound of Compound No. 1-2)

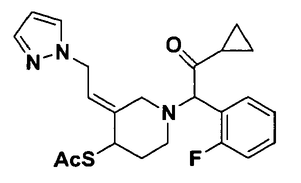

### (a) (E)-4-(t-Butyldimethylsilyloxy)-3-[2-(1H-pyrazol-1-yl)ethylidene]-1-(triphenylmethyl)piperidine

To a solution of (E)-4-(t-butyldimethylsilyloxy)-3-(2-hydroxyethylidene)-1-(triphenylmethyl)piperidine (14.7 g) obtained in Example 1 (b), 1H-pyrazole (2.0 g) and 1,1'-(azodicarbonyl)dipiperidine (8.9 g) in toluene (150 ml) under a stream of nitrogen gas was added tributylphosphine (8.8 ml) under ice cooling, and the resulting mixture was stirred at room temperature for 1 hour. The reaction mixture was evaporated in vacuo, and the residue was purified by chromatography on a silica gel column (ethyl acetate / hexane = 1 / 6) to afford the title compound (9.78 g, yield 57%) as a colorless amorphous solid.

¹H NMR (400 MHz, CDCl₃) δ ppm : 0.04 (3H, s), 0.06 (3H, s), 0.89 (9H, s), 1.82 (1H, bs), 1.90-2.03 (3H, m), 3.04 (1H, bs), 3.74 (1H, bs), 3.97 (1H, m), 4.78-4.90 (2H, m), 5.86 (1H, t, J=7.0), 6.33-6.34 (1H, m), 7.22-7.23 (5H, m), 7.28-7.32 (4H, m), 7.48-7.68 (8H, m).

### (b) (E)-4-Hydroxy-3-[2-(1H-pyrazol-1-yl)ethylidene]-1-(triphenylmethyl)piperidine

The title compound was synthesized in a yield of 77% as a colorless powder crystal using (E)-4-(t-butyldimethylsilyloxy)-3-[2-(1H-pyrazol-1-yl)ethylidene]-1-(triphenylmethyl)piperidine obtained in (a) above by conducting a reaction similar to that mentioned in Example 1 (e).

¹H NMR (400 MHz, CDCl₃) δ ppm : 1.44-1.50 (1H, m), 1.65-1.70 (1H, m), 1.82-1.89 (1H, m), 2.08-2.12 (1H, m), 2.85 (1H, bs), 3.38-3.41 (1H, m), 4.01 (1H, bs), 4.73-4.82 (2H, m), 5.79 (1H, t, J=6.5), 6.28 (1H, m), 7.15-7.17 (5H, m), 7.24-7.27 (4H, m), 7.42 (1H, m), 7.49 (6H, bs), 7.53 (1H, m).

### (c) (E)-4-(Acetylsulfanyl)-3-[2-(1H-pyrazol-1-yl)ethylidene]-1-(triphenylmethyl)piperidine

The title compound was synthesized in a yield of 56% as a yellow amorphous solid using (E)-4-hydroxy-3-[2-(1H-pyrazol-1-yl)ethylidene]-1-(triphenylmethyl)piperidine obtained in (b) above by conducting a reaction similar to that mentioned in Example 1 (h).

¹H NMR (400 MHz, CDCl₃) δ ppm : 1.85-1.92 (1H, m), 2.24 (3H, s), 2.26-2.34 (1H, m), 2.64-2.67 (1H, m), 2.79-2.83 (1H, m), 4.21-4.36 (3H, m), 4.65-4.78 (2H, m), 5.79 (1H, t, J=7.0), 6.26 (1H, m), 7.11-7.15 (5H, m), 7.22-7.27 (4H, m), 7.42 (1H, m), 7.45 (6H, bs), 7.62 (1H, m).

### (d) (E)-4-(Acetylsulfanyl)-3-[2-(1H-pyrazol-1-yl)ethylidene]piperidine hydrogen trifluoroacetate

The title compound was synthesized in a yield of 32% as a brown oil using (E)-4-(acetylsulfanyl)-3-[2-(1H-pyrazol-1-yl)ethylidene]-1-(triphenylmethyl)piperidine obtained in (c) above by conducting a reaction similar to that mentioned in Example 3 (c).

¹H NMR (400 MHz, CDCl₃) δ ppm : 2.03-2.07 (1H, m), 2.35 (3H, s), 2.38-2.45 (1H, m), 3.23-3.30 (1H, m), 3.38-3.43 (1H, m), 3.73 (1H, d, J=14.0), 4.12 (1H, d, J=14.0), 4.43-4.45 (1H, m), 4.68-4.84 (2H, m), 6.03 (1H, t, J=7.0), 6.23-6.24 (1H, m), 7.39-7.40 (1H, m), 7.46 (1H, bs).

### (e) (E)-4-(Acetylsulfanyl)-1-[2-cyclopropyl-1-(2-fluorophenyl)-2-oxoethyl]-3-[2-1H-pyrazol-1-yl)ethylidene]piperidine

The title compound was synthesized in a yield of 75% as a yellow oil using (E)-4-(acetylsulfanyl)-3-[2-(1H-pyrazol-1-yl)ethylidene]piperidine hydrogen trifluoroacetate obtained in (d) above by conducting a reaction similar to that mentioned in Example 1 (g).

¹H NMR (400 MHz, CDCl₃) δ ppm : 0.75-0.88 (2H, m), 0.97-1.06 (2H, m), 1.79-1.86 (1H, m), 2.10-2.21 (1H, m), 2.22-2.31 (1H, m), 2.28 and 2.29 (total 3H, each s), 2.49-2.62 (1H, m), 2.68-2.73 and 2.76-2.81 (total 1H, each m), 2.89 and 3.16 (total 1H, each d, J=12.5), 3.32 and 3.43 (total 1H, each d, J=12.5), 4.28-4.35 (1H, m), 4.67-4.75 (3H, m), 5.80 (1H, t, J=7.0), 6.22-6.24 (1H, m), 7.08-7.17 (2H, m), 7.28-7.38 (3H, m), 7.47-7.48 (1H, m).

### (Example 41) (E)-1-[2-Cyclopropyl-1-(2-fluorophenyl)-2-oxoethyl]-3-[2-(1H-pyrazol-1-yl)ethylidene]-4-sulfanylpiperidine hydrochloride (Hydrochloride of the compound of Compound No. 1-1)

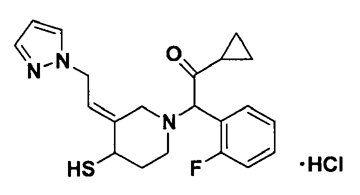

The title compound was synthesized in a yield of 27% as a colorless amorphous solid using (E)-4-(acetylsulfanyl)-1-[2-cyclopropyl-1-(2-fluorophenyl)-2-oxoethyl]-3-[2-(1H-pyrazol-1-yl)ethylidene]piperidine obtained in Example 40 (e) by conducting a reaction similar to that mentioned in Example 2.

¹H NMR (400 MHz, CDCl₃) δ ppm : 0.77-0.80 (2H, m), 0.98-1.03 (1H, m), 1.06-1.13 (1H, m), 1.74-1.81 (1H, m), 2.13-2.24 (1H, m), 2.39-2.47 (1H, m), 2.51-2.54 (1H, m), 2.83-2.88 and 2.91-2.97 (total 1H, each m), 3.33-3.38 (1H, m), 3.75-3.80 (1H, m), 4.90-4.96 (3H, m), 5.94-5.98 (1H, m), 6.33 (1H, t, J=2.0), 7.17-7.22 (1H, m), 7.26-7.32 (1H, m), 7.63-7.66 (3H, m), 7.70-7.71 (2H, m);

IR (KBr, cm⁻¹) : 1712, 1495.

### (Example 42) (E)-4-(Acetylsulfanyl)-1-[2-cyclopropyl-1-(2-fluorophenyl)-2-oxoethyl]-3-[2-(4-ethoxycarbonyl-1H-pyrazol-1-yl)ethylidene]piperidine hydrochloride (Hydrochloride of the compound of Compound No. 1-26)

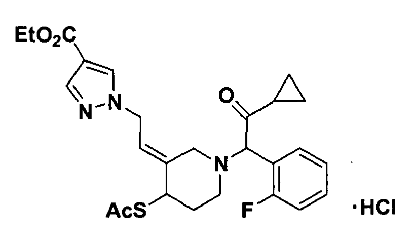

### (a) (E)-4-(t-Butyldimethylsilyloxy)-3-[2-(4-ethoxycarbonyl-1H-pyrazol-1-yl)ethylidene]-1-(triphenylmethyl)piperidine

The title compound was synthesized in a yield of 83% as a colorless amorphous solid using (E)-4-(t-butyldimethylsilyloxy)-3-(2-hydroxyethylidene)-1-(triphenylmethyl)piperidine obtained in Example 1 (b) and 4-ethoxycarbonyl-1H-pyrazole by conducting a reaction similar to that mentioned in Example 40 (a).

¹H NMR (400 MHz, CDCl₃) δ ppm : 0.04 (6H, s), 0.88 (9H, s), 1.36-1.49 (2H, m), 1.40 (3H, t, J=7.0), 1.71-1.82 (1H, m), 1.90-2.00 (2H, m), 2.40 (1H, bs), 3.93-3.97 (1H, m), 4.34 (2H, q, J=7.0), 4.73-4.86 (2H, m), 5.84 (1H, t, J=7.0), 7.19-7.23 (5H, m), 7.27-7.32 (5H, m), 7.52 (5H, bs), 7.95 (1H, s), 7.97 (1H, s).

### (b) (E)-3-[2-(4-Ethoxycarbonyl-1H-pyrazol-1-yl)ethylidene]-4-hydroxy-1-(triphenylmethyl)piperidine

The title compound was synthesized in a quantitative yield as a colorless amorphous solid using (E)-4-(t-butyldimethylsilyloxy)-3-[2-(4-ethoxycarbonyl-1H-pyrazol-1-yl)ethylidene]-1-(triphenylmethyl)piperidine obtained in (a) above by conducting a reaction similar to that mentioned in Example 1 (e).

¹H NMR (400 MHz, CDCl₃) δ ppm : 1.34 (3H, t, J=7.0), 1.81-1.90 (1H, m), 2.07-2.12 (1H, m), 2.87 (2H, bs), 3.46 (2H, bs), 3.98 (1H, bs), 4.28 (2H, q, J=7.0), 4.68-4.79 (2H, m), 5.76 (1H, t, J=7.0), 7.12-7.15 (3H, m), 7.21-7.25 (6H, m), 7.45 (6H, bs), 7.89 (2H, s).

### (c) (E)-4-(Acetylsulfanyl)-3-[2-(4-ethoxycarbonyl-1H-pyrazol-1-yl)ethylidene]-1-(triphenyhnethyl)piperidine

The title compound was synthesized in a yield of 88% as a yellow amorphous solid using (E)-3-[2-(4-ethoxycarbonyl-1H-pyrazol-1-yl)ethylidene]-4-hydroxy-1-(triphenylmethyl)piperidine obtained in (b) above by conducting a reaction similar to that mentioned in Example 1 (h).

¹H NMR (400 MHz, CDCl₃) δ ppm : 1.26 (3H, t, J=7.0), 1.86-1.94 (1H, m), 2.21-2.24 (1H, m), 2.24 (3H, s), 3.80-3.83 (1H, m), 4.11 (2H, q, J=7.0), 4.19-4.27 (6H, m), 5.77 (1H, t, J=7.0), 7.12-7.17 (5H, m), 7.22-7.26 (5H, m), 7.43-7.45 (5H, m), 7.85 (1H, s), 7.91 (1H, s).

### (d) (E)-4-(Acetylsulfanyl)-3-[2-(4-ethoxycarbonyl-1H-pyrazol-1-yl)ethylidene]piperidine hydrogen trifluoroacetate

The title compound was synthesized in a yield of 23% as a brown amorphous solid using (E)-4-(acetylsulfanyl)-3-[2-(4-ethoxycarbonyl-1H-pyrazol-1-yl)ethylidene]-1-(triphenylmethyl)piperidine obtained in (c) above by conducting a reaction similar to that mentioned in Example 3 (c).

¹H NMR (400 MHz, CDCl₃) δ ppm : 1.33 (3H, t, J=7.0), 2.07-2.10 (1H, m), 2.36 (3H, s), 2.38-2.46 (1H, m), 3.22-3.28 (1H, m), 3.36-3.42 (1H, m), 3.73 (1H, d, J=14.0), 4.11 (1H, d, J=14.0), 4.27 (2H, q, J=7.0), 4.44-4.46 (1H, m), 4.72-4.84 (2H, m), 6.04-6.07 (1H, m), 7.85 (1H, s), 7.88 (1H, s).

### (e) (E)-4-(Acetylsulfanyl)-1-[2-cyclopropyl-1-(2-fluorophenyl)-2-oxoethyl]-3-[2-(4-ethoxycarbonyl-1H-pyrazol-1-yl)ethylidene]piperidine hydrochloride

The title compound was synthesized in a yield of 74% as a yellow amorphous solid using (E)-4-(acetylsulfanyl)-3-[2-(4-ethoxycarbonyl-1H-pyrazol-1-yl)ethylidene]piperidine hydrogen trifluoroacetate obtained in (d) above by conducting a reaction similar to that mentioned in Example 1 (g).

¹H NMR (400 MHz, CDCl₃) δ ppm : 0.75-0.88 (2H, m), 1.00-1.07 (2H, m), 1.34 (3H, t, J=7.0), 1.80-1.87 (1H, m), 2.08-2.26 (2H, m), 2.28 and 2.30 (total 3H, each s), 2.50-2.60 (1H, m), 2.67-2.72 and 2.78-2.82 (total 1H, each m), 2.87 and 3.18 (total 1H, each d, J=12.5), 3.29 and 3.44 (total 1H, each d, J=12.5), 4.28 (2H, q, J=7.0), 4.33-4.35 (1H, m), 4.67-4.76 (3H, m), 5.79 (1H, t, J=7.0), 7.08-7.17 (2H, m), 7.29-7.37 (2H, m), 7.85-7.87 (2H, m).

### (Example 43) (E)-1-[2-Cyclopropyl-1-(2-fluorophenyl)-2-oxoethyl]-3-[2-(4-ethoxycarbonyl-1H-pyrazol-1-yl)ethylidene]-4-sulfanylpiperidine hydrochloride (Hydrochloride of the compound of Compound No. 1-25)

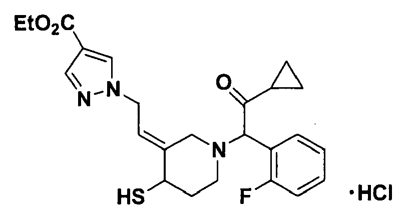

The title compound was synthesized in a yield of 46% as a yellow amorphous solid using (E)-4-(acetylsulfanyl)-1-[2-cyclopropyl-1-(2-fluorophenyl)-2-oxoethyl]-3-[2-(4-ethoxycarbonyl-1H-pyrazol-1-yl)ethylidene]piperidine obtained in Example 42 (e) by conducting a reaction similar to that mentioned in Example 34.

¹H NMR (400 MHz, CDCl₃) δ ppm : 0.76-0.88 (2H, m), 1.00-1.04 (2H, m), 1.35 (3H, t, J=7.0), 1.73-1.79 (1H, m), 2.09-2.12 (1H, m), 2.17-2.28 (1H, m), 2.54-2.60 and 2.61-2.67 (total 1H, each m), 2.78-2.85 (1H, m), 3.04 and 3.24 (total 1H, each d, J=12.5), 3.31 and 3.54 (total 1H, each d, J=12.5), 3.65-3.70 and 3.73-3.77 (total 1H, each m), 4.28 (2H, q, J=7.0), 4.69-4.77 (3H, m), 5.74-5.82 (1H, m), 7.09-7.17 (2H, m), 7.29-7.38 (2H, m), 7.87-7.90 (2H, m);

IR (KBr, cm⁻¹) : 2466, 1712, 1554.

### (Example 44) (E)-3-[2-(4-Carboxy-1H-pyrazol-1-yl)ethylidene]-1-[2-cyclopropyl-1-(2-fluorophenyl)-2-oxoethyl]-4-sulfanylpiperidine hydrochloride (Hydrochloride of the compound of Compound No. 1-17)

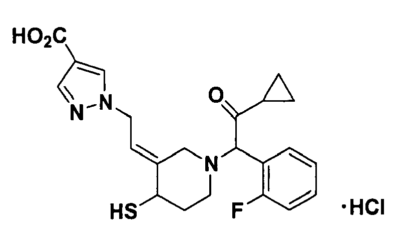

The title compound was synthesized in a yield of 14% as a colorless amorphous solid using (E)-1-[2-cyclopropyl-1-(2-fluorophenyl)-2-oxoethyl]-3-[2-(4-ethoxycarbonyl-1H-pyrazol-1-yl)ethylidene]-4-sulfanylpiperidine obtained in Example 43 by conducting a reaction similar to that mentioned in Example 15.

¹H NMR (400 MHz, pyridine-d₅) δ ppm : 0.79-0.82 (2H, m), 1.01-1.05 (1H, m), 1.09-1.13 (1H, m), 1.75-1.83 (1H, m), 2.16-2.25 (1H, m), 2.41-2.49 (1H, m), 2.62-2.68 (1H, m), 2.85-2.91 and 2.92-2.98 (total 1H, each m), 3.35 and 3.38 (total 1H, each d, J=13.0), 3.63 and 3.70 (total 1H, each d, J=13.0), 3.79-3.82 (1H, m), 4.91-5.05 (3H, m), 5.99-6.03 (1H, m), 7.19-7.27 (2H, m), 7.29-7.35 (1H, m), 7.68-7.72 (1H, m), 8.43 (1H, s), 8.53 (1H, d, J=1.5);

IR (KBr, cm⁻¹): 2546, 1709, 1555.

### (Example 45) (E)-4-(Acetylsulfanyl)-1-[2-cyclopropyl-1-(2-fluorophenyl)-2-oxoethyl]-3-(2-methoxyethylidene)piperidine hydrogen trifluoroacetate (Hydrogen trifluoroacetate of the compound of Compound No. 3-6)

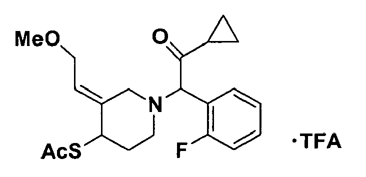

### (a) (E)-4-(t-Butyldimethylsilyloxy)-3-(2-methoxyethylidene)-1-(triphenylmethyl)piperidine

To a solution of (E)-4-(t-butyldimethylsilyloxy)-3-(2-hydroxyethylidene)-1-(triphenylmethyl)piperidine (1.85 g) obtained in Example 1 (b) in N,N-dimethylformamide (20 ml) was added sodium hydride (0.23 g) under ice cooling, and the resulting mixture was stirred at room temperature for 15 minutes. To the reaction mixture was added methyl iodide (0.34 ml) at the same temperature, and the resulting mixture was stirred at room temperature overnight. The reaction mixture was diluted with a mixed solvent of ethyl acetate and ether and washed with a saturated aqueous sodium hydrogen carbonate solution and a saturated aqueous sodium chloride solution, and the organic layer was dried over anhydrous sodium sulfate. The solvent was removed in vacuo, and the residue was purified by chromatography on a silica gel column (ethyl acetate / hexane = 1 / 24) to afford the title compound (1.41 g, yield 74%) as a colorless oil.

¹H NMR (400 MHz, CDCl₃) δ ppm : -0.02 (3H, s), -0.01 (3H, s), 0.86 (9H, s), 1.69-2.00 (4H, m), 2.87-2.96 (1H, m), 3.31 (3H, s), 3.49-3.61 (1H, m), 3.83-4.02 (3H, m), 5.72 (1H, t, J=8.0), 7.12-7.20 (3H, m), 7.22-7.28 (6H, m), 7.40-7.55 (6H, m).

### (b) (E)-4-Hydroxy-3-(2-methoxyethylidene)-1-(triphenylmethyl)piperidine

The title compound was synthesized in a quantitative yield as a colorless amorphous solid using (E)-4-(t-butyldimethylsilyloxy)-3-(2-methoxyethylidene)-1-(triphenylmethyl)piperidine obtained in (a) above by conducting a reaction similar to that mentioned in Example 1 (e).

¹H NMR (400 MHz, CDCl₃) δ ppm : 1.75-2.13 (4H, m), 2.66-2.81 (2H, m), 3.35 (3H, m), 3.88-4.02 (3H, m), 5.70 (1H, t, J=7.0), 7.13-7.20 (3H, m), 7.23-7.30 (6H, m), 7.43-7.53 (6H, m).

### (c) (E)-4-Hydroxy-3-(2-methoxyethylidene)piperidine bis(hydrogen trifluoroacetate)

The title compound was synthesized in a yield of 95% as a colorless oil using (E)-4-hydroxy-3-(2-methoxyethylidene)-1-(triphenylmethyl)piperidine obtained in (b) above by conducting a reaction similar to that mentioned in Example 3 (c).

¹H NMR (400 MHz, CDCl₃+DMSO-d₆) δ ppm : 1.88-2.13 (2H, m), 3.11-3.19 (1H, m), 3.32 (3H, s), 3.39-3.48 (1H, m), 3.75 (1H, d, J=13.5), 3.92 (1H, d, J=13.5), 4.01 (2H, d, J=6.0), 4.22-4.27 (1H, m), 5.87 (1H, t, J=6.0).

### (d) (E)-1-[2-Cyclopropyl-1-(2-fluorophenyl)-2-oxoethyl]-4-hydroxy-3-(2-methoxyethylidene)piperidine

The title compound was synthesized in a yield of 78% as a brown oil using (E)-4-hydroxy-3-(2-methoxyethylidene)piperidine bis(hydrogen trifluoroacetate) obtained in (c) above by conducting a reaction similar to that mentioned in Example 1 (g).

¹H NMR (400 MHz, CDCl₃) δ ppm : 0.78-1.09 (4H, m), 1.68-1.79 (1H, m), 1.96-2.05 (1H, m), 2.19-2.28 (1H, m), 2.42-2.54 (1H, m), 2.76-3.00 (2H, m), 3.30 (3H, s), 3.36 and 3.47 (total 1H, each d, J=12.0), 3.92 (2H, d, J=6.0), 4.07-4.15 (1H, m), 4.69 and 4.71 (total 1H, each s), 5.68 and 5.69 (total 1H, each t, J=6.0), 7.07-7.20 (2H, m), 7.28-7.46 (2H, m).

### (e) (E)-4-(Acetylsulfanyl)-1-[2-cyclopropyl-1-(2-fluorophenyl)-2-oxoethyl]-3-(2-methoxyethylidene)piperidine hydrogen trifluoroacetate

The title compound was synthesized in a yield of 30% as a pale brown oil using (E)-1-[2-cyclopropyl-1-(2-fluorophenyl)-2-oxoethyl]-4-hydroxy-3-(2-methoxyethylidene)piperidine obtained in (d) above by conducting a reaction similar to that mentioned in Example 3 (e).

¹H NMR (400 MHz, CDCl₃) δ ppm : 0.79-0.89 (2H, m), 0.95-1.08 (2H, m), 1.77-1.85 (1H, m), 2.11-2.28 (2H, m), 2.29 and 2.30 (total 3H, each s), 2.46-3.08 (3H, m), 3.26 and 3.28 (total 3H, each s), 3.30-3.56 (1H, m), 3.81-3.92 (2H, m), 4.28-4.36 (1H, m), 4.68 and 4.71 (total 1H, each s), 5.72 (1H, t, J=6.5), 7.07-7.20 (2H, m), 7.29-7.35 (1H, m), 7.39-7.45 (1H, m);

IR (liquid film, cm⁻¹) : 1711, 1495.

### (Example 46) (E)-1-[2-Cyclopropyl-1-(2-fluorophenyl)-2-oxoethyl]-3-(2-methoxyethylidene)-4-sulfanylpiperidine hydrogen trifluoroacetate (Hydrogen trifluoroacetate of the compound of Compound No. 3-5)

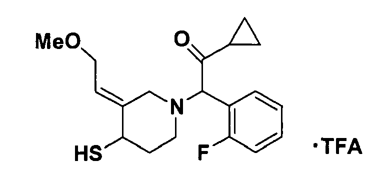

The title compound was synthesized in a yield of 60% as a colorless oil using (E)-4-(acetylsulfanyl)-1-[2-cyclopropyl-1-(2-fluorophenyl)-2-oxoethyl]-3-(2-methoxyethylidene)piperidine hydrogen trifluoroacetate obtained in Example 45 (e) by conducting a reaction similar to that mentioned in Example 2.

¹H NMR (400 MHz, CDCl₃) δ ppm : 0.79-0.90 (2H, m), 0.95-1.09 (2H, m), 1.69-1.78 (1H, m), 2.13-2.32 (2H, m), 2.51-2.87 (2H, m), 3.03-3.57 (2H, m), 3.27 and 3.29 (total 3H, each s), 3.70-3.92 (3H, m), 4.70 and 4.73 (total 1H, each s), 5.68 and 5.71 (total 1H, each t, J=6.5), 7.07-7.20 (2H, m), 7.28-7.46 (2H, m);

IR (liquid film, cm⁻¹) : 2564, 1714, 1495.

### (Example 47) (E)-4-(Acetylsulfanyl)-1-[2-cyclopropyl-1-(2-fluorophenyl)-2-oxoethyl]-3-[2-(4-ethoxycarbonyl-2H-1,2,3-triazol-2-yl)ethylidene]piperidine (Compound of Compound No. 1-592), (E)-4-(acetylsulfanyl)-1-[2-cyclopropyl-1-(2-fluorophenyl)-2-oxoethyl]-3-[2-(4-ethoxycarbonyl-1H-1,2,3-triazol-1-yl)ethylidene]piperidine (Compound of Compound No. 1-492) and (E)-4-(acetylsulfanyl)-1-[2-cyclopropyl-1-(2-fluorophenyl)-2-oxoethyl]-3-[2-(5-ethoxycarbonyl-1H-1,2,3-triazol-1-yl)ethylidene]piperidine (Compound of Compound No. 1-498)

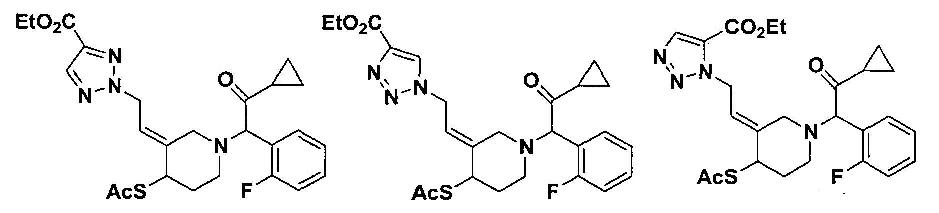

To a solution of (E)-4-(acetylsulfanyl)-1-[2-cyclopropyl-1-(2-fluorophenyl)-2-oxoethyl]-3-(2-hydroxyethylidene)piperidine (1000 mg) obtained in Example 32 (f) and 4-ethoxycarbonyl-1H-1,2,3-triazole (449 mg) in toluene (10 ml) under a stream of nitrogen gas were added 1,1'-(azodicarbonyl)dipiperidine (802 mg) and tributylphosphine (0.79 ml) under ice cooling, and the resulting mixture was stirred at room temperature for 18 hours. To the reaction mixture were added 4-ethoxycarbonyl-1H-1,2,3-triazole (224 mg), 1,1'-(azodicarbonyl)dipiperidine (802 mg) and tributylphosphine (0.79 ml), and the resulting mixture was stirred at room temperature for 4.5 hours. To the reaction mixture were added 4-ethoxycarbonyl-1H-1,2,3-triazole (112 mg), 1,1'-(azodicarbonyl)dipiperidine (200 mg) and tributylphosphine (0.20 ml), and the resulting mixture was further stirred at room temperature for 2 hours. The reaction mixture was evaporated in vacuo, and the residue was roughly purified by chromatography on a silica gel column (ethyl acetate / hexane = 1 /2 - 1 / 1) and then purified using a preparative HPLC (YMC-Pack ODS-A; YMC, eluent: acetonitrile / 0.024N hydrochloric acid = 15 / 85) to afford the hydrochlorides of the three title compounds. Each of the obtained hydrochlorides was neutralized with a saturated aqueous sodium hydrogen carbonate solution and extracted with ethyl acetate. The extract was washed with a saturated aqueous sodium chloride solution, and the organic layer was dried over anhydrous magnesium sulfate. The solvent was removed in vacuo to afford the three title compounds.
(E)-4-(Acetylsulfanyl)-1-[2-cyclopropyl-1-(2-fluorophenyl)-2-oxoethyl]-3-[2-(4-ethoxycarbonyl-1H-1,2,3-triazol-1-yl)ethylidene]piperidine having the highest polarity: a colorless oil, yielded amount 120 mg, yield 9%

¹H NMR (400 MHz, CDCl₃) δ ppm : 0.76-0.90 (2H, m), 1.02-1.05 (2H, m), 1.42 (3H, t, J=7.0), 1.80-1.86 (1H, m), 1.98-2.04 (1H, m), 2.09-2.19 and 2.20-2.28 (total 1H, each m), 2.29 and 2.31 (total 3H, each s), 2.53-2.59 (1H, m), 2.66-2.71 and 2.78-2.84 (total 1H, each m), 2.90 and 3:51 (total 1H, each d, J=12.5), 3.28 (1H, bs), 4.25-4.27 and 4.32-4.34 (total 1H, each m), 4.44 (2H, q, J=7.0), 4.80 and 4.81 (total 1H, each s), 4.98-5.04 (2H, m), 5.80 (1H, t, J=7.5), 7.12-7.21 (2H, m), 7.31-7.39 (2H, m), 8.19 and 8.22 (total 1H, each s).
(E)-4-(Acetylsulfanyl)-1-[2-cyclopropyl-1-(2-fluorophenyl)-2-oxoethyl]-3-[2-(5-ethoxycarbonyl-1H-1,2,3-triazol-1-yl)ethylidene]piperidine having the second highest polarity: a yellow oil, yielded amount 219 mg, yield 17%

¹H NMR (400 MHz, CDCl₃) δ ppm : 0.81-0.91 (2H, m), 1.00-1.11 (2H, m), 1.39 and 1.40 (total 3H, each t, J=7.0), 1.77-1.84 (1H, m), 2.14-2.24 (2H, m), 2.25 (3H, s), 2.44-2.51 and 2.57-2.63 (total 1H, each m), 2.68-2.77 (1H, m), 3.01 and 3.19 (total 1H, each d, J=12.5), 3.48 and 3.53 (total 1H, each d, J=12.5), 4.24-4.29 (1H, m), 4.37 and 4.38 (total 2H, each q, J=7.0), 4.76 and 4.78 (total 1H, each s), 5.27-5.35 (2H, m), 5.72-5.77 (1H, m), 7.09-7.20 (2H, m), 7.28-7.44 (2H, m), 8.07 (1H, s).
(E)-4-(Acetylsulfanyl)-1-[2-cyclopropyl-1-(2-fluorophenyl)-2-oxoethyl]-3-[2-(4-ethoxycarbonyl-2H-1,2,3-triazol-2-yl)ethylidene]piperidine having the lowest polarity: a colorless oil, yielded amount 236 mg, yield 18%

¹H NMR (400 MHz, CDCl₃) δ ppm : 0.82-0.89 (2H, m), 1.00-1.08 (2H, m), 1.41 (3H, t, J=7.0), 1.79-1.86 (1H, m), 2.12-2.30 (2H, m), 2.27 and 2.28 (total 3H, each s), 2.50-2.63 (1H, m), 2.68-2.80 (1H, m), 2.95 and 3.22 (total 1H, each d, J=13.0), 3.34 and 3.68 (total 1H, each d, J=13.0), 4.28-4.35 (1H, m), 4.43 (2H, q, J=7.0), 4.75 and 4.78 (total 1H, each s), 5.05-5.13 (2H, m), 5.86 (1H, t, J=7.0), 7.11-7.22 (2H, m), 7.32-7.43 (2H, m), 8.03 (1H, s).

### (Example 48) (E)-1-[2-Cyclopropyl-1-(2-fluorophenyl)-2-oxoethyl]-3-[2-(4-ethoxycarbonyl-2H-1,2,3-triazol-2-yl)ethylidene]-4-sulfanylpiperidine hydrochloride (Hydrochloride of the compound of Compound No. 1-591)

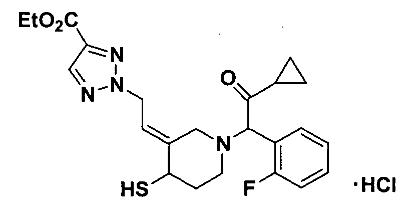

The title compound was synthesized in a quantitative yield as a pale yellow oil using (E)-4-(acetylsulfanyl)-1-[2-cyclopropyl-1-(2-fluorophenyl)-2-oxoethyl]-3-[2-(4-ethoxycarbonyl-2H-1,2,3-triazol-2-yl)ethylidene]piperidine obtained in Example 47 by conducting a reaction similar to that mentioned in Example 34.

¹H NMR (400 MHz, pyridine-d₅) δ ppm : 0.84-0.91 (2H, m), 1.01-1.07 (1H, m), 1.14-1.18 (1H, m), 1.23 (3H, t, J=7.0), 1.74-1.84 (1H, m), 2.14-2.24 (1H, m), 2.48-2.64 (2H, m), 2.84-2.98 (1H, m), 3.35-3.41 (1H, m), 3.69 and 3.75 (total 1H, each d, J=12.5), 3.79-3.82 (1H, m), 4.35 (2H, q, J=7.0), 4.95 and 4.96 (total 1H, each s), 5.30-5.35 (2H, m), 6.10-6.14 (1H, m), 7.23-7.35 (3H, m), 7.69-7.75 (1H, m), 8.37 (1H, s);

IR (KBr, cm⁻¹) : 2914, 1717.

### (Example 49) (E)-3-[2-(4-Carboxy-2H-1,2,3-triazol-2-yl)ethylidene]-1-[2-cyclopropyl-1-(2-fluorophenyl)-2-oxoethyl]-4-sulfanylpiperidine hydrochloride (Hydrochloride of the compound of Compound No. 1-587)

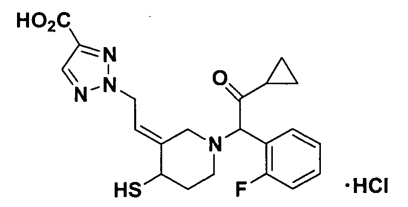

The title compound was synthesized in a yield of 74% as a colorless amorphous solid using (E)-1-[2-cyclopropyl-1-(2-fluorophenyl)-2-oxoethyl]-3-[2-(4-ethoxycarbonyl-2H-1,2,3-triazol-2-yl)ethylidene]-4-sulfanylpiperidine hydrochloride obtained in Example 48 by conducting a reaction similar to that mentioned in Example 15.

¹H NMR (400 MHz, pyridine-d₅) δ ppm : 0.80-0.94 (2H, m), 1.01-1.07 (1H, m), 1.14-1.19 (1H, m), 1.74-1.84 (1H, m), 2.14-2.25 (1H, m), 2.52-2.66 (2H, m), 2.84-2.98 (1H, m), 3.38-3.42 (1H, m), 3.70 and 3.76 (total 1H, each d, J=13.0), 3.79-3.84 (1H, m), 4.95-4.97 (1H, m), 5.31-5.38 (2H, m), 6.12-6.17 (1H, m), 7.24-7.36 (3H, m), 7.71-7.78 (1H, m), 8.51 (1H, s);

IR (KBr, cm⁻¹): 2927, 1712.

### (Example 50) (E)-1-[2-Cyclopropyl-1-(2-fluorophenyl)-2-oxoethyl]-3-[2-(4-ethoxycarbonyl-1H-1,2,3-triazol-1-yl)ethylidene]-4-sulfanylpiperidine hydrochloride (Hydrochloride of the compound of Compound No. 1-491)

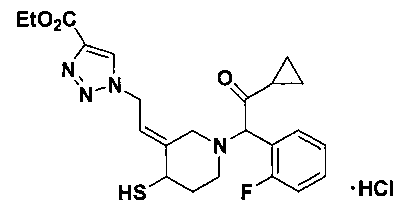

The title compound was synthesized in a quantitative yield as a colorless oil using (E)-4-(acetylsulfanyl)-1-[2-cyclopropyl-1-(2-fluorophenyl)-2-oxoethyl]-3-[2-(4-ethoxycarbonyl-1H-1,2,3-triazol-1-yl)ethylidene]piperidine obtained in Example 47 by conducting a reaction similar to that mentioned in Example 34.

¹H NMR (400 MHz, CDCl₃) δ ppm : 0.77-0.90 (2H, m), 1.02-1.05 (2H, m), 1.42 (3H, t, J=7.0), 1.54-1.58 (1H, m), 1.73-1.79 (1H, m), 1.99-2.03 (1H, m), 2.15-2.29 (1H, m), 2.53-2.58 and 2.62-2.67 (total 1H, each m), 2.78-2.85 (1H, m), 3.27-3.35 (1H, m), 3.63-3.66 and 3.72-3.76 (total 1H, each m), 4.44 (2H, q, J=7.0), 4.83 (1H, s), 5.01-5.06 (2H, m), 5.76-5.85 (1H, m), 7.12-7.26 (3H, m), 7.33-7.36 (1H, m), 8.23 and 8.24 (total 1H, each s);

MS (FAB) m/z : 459 (M+H)⁺

### (Example 51) (E)-3-[2-(4-Carboxy-1H-1,2,3-triazol-1-yl)ethylidene]-1-[2-cyclopropyl-1-(2-fluorophenyl)-2-oxoethyl]-4-sulfanylpiperidine hydrochloride (Hydrochloride of the compound of Compound No. 1-487)

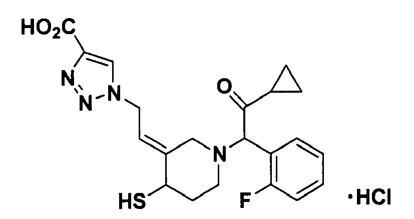

The title compound was synthesized in a yield of 71% as a colorless amorphous solid using (E)-1-[2-cyclopropyl-1-(2-fluorophenyl)-2-oxoethyl]-3-[2-(4-ethoxycarbonyl-1H-1,2,3-triazol-1-yl)ethylidene]-4-sulfanylpiperidine hydrochloride obtained in Example 50 by conducting a reaction similar to that mentioned in Example 15.

¹H NMR (400 MHz, pyridine-d₅) δ ppm : 0.77-0.81 (2H, m), 0.99-1.11 (2H, m), 1.73-1.81 (1H, m), 2.12-2.23 (1H, m), 2.29-2.36 (1H, m), 2.60-2.63 (1H, m), 2.82-2.96 (1H, m), 3.34 and 3.40 (total 1H, each d, J=12.5), 3.65 and 3.73 (total 1H, each d, J=12.5), 3.76-3.83 (1H, m), 4.96 and 4.97 (total 1H, each s), 5.21-5.27 (2H, m), 5.96-6.01 (1H, m), 7.20-7.32 (3H, m), 7.64-7.68 (1H, m), 8.88 (1H, s);

IR (KBr, cm⁻¹) : 2927, 1711.

### (Example 52) (E)-1-[2-Cyclopropyl-1-(2-fluorophenyl)-2-oxoethyl]-3-[2-(5-ethoxycarbonyl-1H-1,2,3-triazol-1-yl)ethylidene]-4-sulfanylpiperidine hydrochloride (Hydrochloride of the compound of Compound No. 1-497)

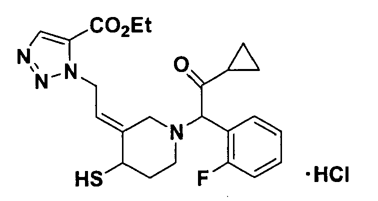

The title compound was synthesized in a yield of 90% as a colorless amorphous solid using (E)-4-(acetylsulfanyl)-1-[2-cyclopropyl-1-(2-fluorophenyl)-2-oxoethyl]-3-[2-(5-ethoxycarbonyl-1 H-1,2,3-triazol-1-yl)ethylidene]piperidine obtained in Example 47 by conducting a reaction similar to that mentioned in Example 34.

¹H NMR (400 MHz, pyridine-d₅) δ ppm : 0.84-0.91 (2H, m), 1.03-1.09 (1H, m), 1.15-1.22 (1H, m), 1.21 (3H, t, J=7.0), 1.75-1.83 (1H, m), 2.15-2.25 (1H, m), 2.50-2.57 (1H, m), 2.60-2.68 (1H, m), 2.84-2.89 and 2.93-2.99 (total 1H, each m), 3.46-3.54 (1H, m), 3.77-3.81 (2H, m), 4.28 (2H, q, J=7.0), 4.98 (1H, s), 5.52-5.56 (2H, m), 6.04-6.09 (1H, m), 7.21-7.35 (3H, m), 7.69-7.76 (1H, m), 8.39 (1H, s);

MS (FAB) m/z : 459 (M+H)⁺.

### (Example 53) (E)-3-[2-(5-Carboxy-1H-1,2,3-triazol-1-yl)ethylidene]-1-[2-cyclopropyl-1-(2-fluorophenyl)-2-oxoethyl]-4-sulfanylpiperidine hydrochloride (Hydrochloride of the compound of Compound No. 1-493)

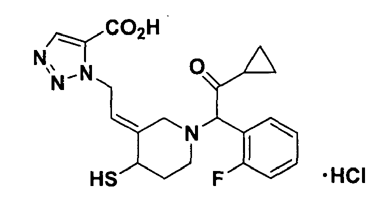

The title compound was synthesized in a yield of 68% as a colorless amorphous solid using (E)-1-[2-cyclopropyl-1-(2-fluorophenyl)-2-oxoethyl]-3-[2-(5-ethoxycarbonyl-1H-1,2,3-triazol-1-yl)ethylidene]-4-sulfanylpiperidine hydrochloride obtained in Example 52 by conducting a reaction similar to that mentioned in Example 15.

¹H NMR (400 MHz, pyridine-d₅) δ ppm : 0.77-0.91 (2H, m), 0.99-1.04 (1H, m), 1.13-1.19 (1H, m), 1.71-1.81 (1H, m), 2.10-2.21 (1H, m), 2.49-2.66 (2H, m), 2.78-2.96 (1H, m), 3.48-3.52 and 3.57-3.60 (total 1H, each m), 3.77-3.83 (2H, m), 4.95 (1H, s), 5.68-5.72 (2H, m), 6.14-6.22 (1H, m), 7.21-7.33 (3H, m), 7.70-7.78 (1H, m), 8.55 (1H, s);

MS (FAB) m/z : 431 (M+H)⁺.

### (Example 54) (E)-4-(Acetylsulfanyl)-1-[2-cyclopropyl-1-(2-fluorophenyl)-2-oxoethyl]-3-{2-[(2S)-(methoxycarbonylmethyl)pyrrolidin-1-yl]ethylidene}piperidine dihydrochloride (Hydrochloride of the compound of Compound No. 5-154)

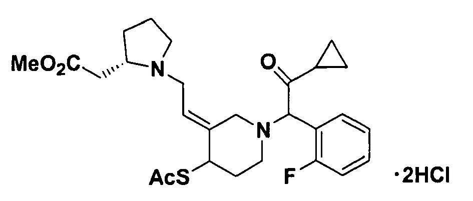

The title compound was synthesized in a yield of 17% as a yellow oil using (E)-4-(acetylsulfanyl)-1-[2-cyclopropyl-1-(2-fluorophenyl)-2-oxoethyl]-3-(2-hydroxyethylidene)piperidine obtained in Example 32 (f) and (2S)-(methoxycarbonylmethyl)pyrrolidine hydrochloride by conducting a reaction similar to that mentioned in Example 33.

¹H NMR (500 MHz, pyridine-d₅) δ ppm : 0.71-0.87 (2H, m), 1.02-1.15 (2H, m), 1.74-1.89 (2H, m), 1.89-2.04 (2H, m), 2.08-2.34 (5H, m), 2.54-2.81 (2H, m), 3.02-3.50 (5H, m), 3.53-3.69 (4H, m), 3.70-3.79 (1H, m), 3.79-3.99 (2H, m), 4.03-4.19 (1H, m), 4.42-4.51 (1H, m), 4.99-5.06 (1H, m), 6.29-6.41 (1H, m), 7.20-7.31 (2H, m), 7.32-7.41 (1H, m), 7.54-7.64 (1H, m);

IR (KBr, cm⁻¹) : 2547, 1700, 1494.

### (Example 55) (E)-1-[2-Cyclopropyl-1-(2-fluorophenyl)-2-oxoethyl]-3-{2-[(2S)-(methoxycarbonylmethyl)pyrrolidin-1-yl]ethylidene}-4-sulfanylpiperidine dihydrochloride (Hydrochloride of the compound of Compound No. 5-153)

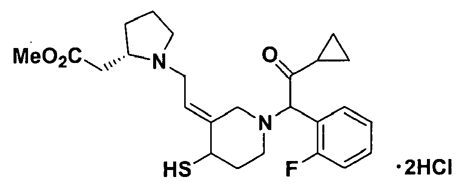

To a solution of (E)-4-(acetylsulfanyl)-1-[2-cyclopropyl-1-(2-fluorophenyl-2-oxoethyl]-3-{2-[(2S)-(methoxycarbonylmethyl)pyrrolidin-1-yl]ethylidene}piperidine dihydrochloride (407 mg) obtained in Example 54 in methanol (10 ml) was bubbled hydrogen chloride under ice cooling, and the resulting mixture was stirred at room temperature under tightly sealed conditions overnight. The reaction mixture was removed in vacuo, and the residue was purified using a preparative HPLC (YMC-Pack ODS-A; YMC, eluent: acetonitrile / water containing triethylamine (0.2%) and acetic acid (0.2%) = 1/1). The eluted fraction was extracted with ethyl acetate and washed with a saturated aqueous sodium hydrogen carbonate solution and a saturated aqueous sodium chloride solution, and the organic layer was dried over anhydrous magnesium sulfate. The solvent was removed in vacuo, and to a solution of the residue in dichloromethane (10 ml) was added a 4N solution of hydrogen chloride in dioxane (0.26 ml) at room temperature. The reaction mixture was evaporated in vacuo to afford the title compound (192 mg, yield 48%) as a pale yellow amorphous solid.

¹H NMR (500 MHz, pyridine-d₅) δ ppm : 0.73-0.89 (2H, m), 1.02-1.14 (2H, m), 1.69-1.82 (1H, m), 1.82-2.11 (3H, m), 2.12-2.28 (2H, m), 2.29-2.43 (1H, m), 2.59-2.73 (1H, m), 2.74-2.98 (1H, m), 3.11-3.42 (3H ,m), 3.44-3.71 (5H, m), 3.76-4.02 (4H, m), 4.13-4.28 (1H, m), 5.00-5.07 (1H, m), 6.25-6.36 (1H, m), 7.22-7.32 (2H, m), 7.34-7.44 (1H, m), 7.58-7.67 (1H, m);

IR (KBr, cm⁻¹): 2558, 1714, 1493.

### (Example 56) (E)-3-{2-[(2S)-(Carboxymethyl)pyrrolidin-1-yl]ethylidene}-1-[2-cyclopropyl-1-(2-fluorophenyl)-2-oxoethyl]-4-sulfanylpiperidine dihydrochloride (Hydrochloride of the compound of Compound No. 5-151)

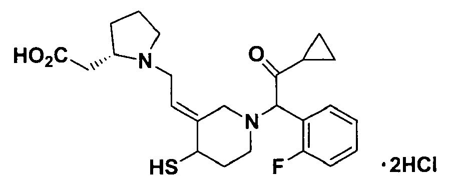

The title compound was synthesized in a yield of 46% as a colorless amorphous solid using (E)-1-[2-cyclopropyl-1-(2-fluorophenyl)-2-oxoethyl]-3-{2-[(2S)-(methoxycarbonylmethyl)pyrrolidin-1-yl]ethylidene}-4-sulfanylpiperidine dihydrochloride obtained in Example 55 by conducting a reaction similar to that mentioned in Example 15.

¹H NMR (500 MHz, pyridine-d₅) δ ppm : 0.71-0.86 (2H, m), 0.98-1.15 (2H, m), 1.71-1.90 (2H, m), 1.93-2.09 (2H, m), 2.11-2.24 (1H, m), 2.25-2.45 (2H, m), 2.56-2.75 (2H, m), 2.77-2.99 (1H, m), 3.26-4.05 (8H, m), 4.06-4.23 (1H, m), 4.95-5.02 (1H, m), 6.25-6.39 (1H, m), 7.19-7.29 (2H, m), 7.30-7.38 (1H ,m), 7.57-7.67 (1H, m);

IR (KBr, cm⁻¹) : 2562, 1713, 1493.

### (Example 57) (E)-4-(Acetylsulfanyl)-1-[2-cyclopropyl-1-(2-fluorophenyl)-2-oxoethyl]-3-{2-[3-(ethoxycarbonyl)piperidin-1-yl]ethylidene}piperidine dihydrochloride (Hydrochloride of the compound of Compound No. 5-342)

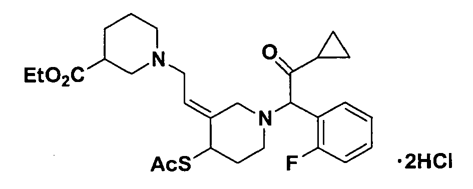

The title compound was synthesized in a yield of 61 % as a brown amorphous solid using (E)-4-(acetylsulfanyl)-1-[2-cyclopropyl-1-(2-fluorophenyl)-2-oxoethyl]-3-(2-hydroxyethylidene)piperidine obtained in Example 32 (f) and 3-(ethoxycarbonyl)piperidine by conducting a reaction similar to that mentioned in Example 33.

¹H NMR (400 MHz, pyridine-d₅) δ ppm : 0.73-0.89 (2H, m), 1.02-1.14 (5H, m), 1.43-1.56 (1H, m), 1.65-1.76 (1H, m), 1.79-1.89 (1H, m), 1.98-2.09 (1H, m), 2.09-2.36 (6H, m), 2.54-2.84 (3H, m), 2.88-3.02 (1H, m), 3.17-3.66 (4H, m), 3.67-3.82 (3H, m), 4.02-4.11 (2H, m), 4.46-4.54 (1H, m), 4.99-5.05 (1H, m), 6.28-6.37 (1H, m), 7.18-7.42 (3H, m), 7.58-7.70 (1H, m);

IR (KBr, cm⁻¹): 2526, 1710, 1493.

### (Example 58) (E)-1-[2-Cyclopropyl-1-(2-fluorophenyl)-2-oxoethyl]-3-{2-[3-(ethoxycarbonyl)piperidin}-1-yl]ethylidene}-4-sulfanylpiperidine dihydrochloride (Hydrochloride of the compound of Compound No. 5-341)

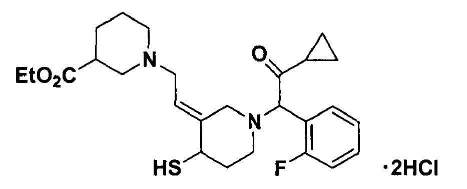

The title compound was synthesized in a yield of 49% as a pale yellow amorphous solid using (E)-4-(acetylsulfanyl)-1-[2-cyclopropyl-1-(2-fluorophenyl)-2-oxoethyl]-3-{2-[3-(ethoxycarbonyl)piperidin-1-yl]ethylidene}piperidine dihydrochloride obtained in Example 57 by conducting a reaction similar to that mentioned in Example 34.

¹H NMR (400 MHz, pyridine-d₅) δ ppm : 0.74-0.86 (2H, m), 1.01-1.15 (5H, m), 1.45-1.56 (1H, m), 1.64-1.82 (2H, m), 1.98-2.38 (4H, m), 2.50-3.08 (5H, m), 3.27-3.92 (7H, m), 4.00-4.12 (2H, m), 4.97-5.03 (1H, m), 6.22-6.36 (1H, m), 7.20-7.38 (3H, m), 7.60-7.72 (1H, m);

IR (KBr, cm⁻¹) : 2533, 1726, 1493.

### (Example 59) (E)-3-{2-[3-(Carboxypiperidin)-1-yl]ethylidene}-1-[2-cyclopropyl-1-(2-fluorophenyl)-2-oxoethyl]-4-sulfanylpiperidine dihydrochloride (Hydrochloride of the compound of Compound No. 5-337)

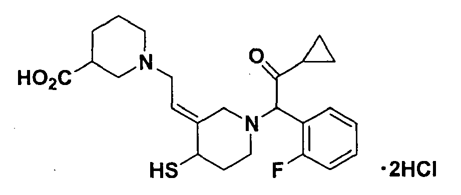

The title compound was synthesized in a yield of 72% as a colorless amorphous solid using (E)-1-[2-cyclopropyl-1-(2-fluorophenyl)-2-oxoethyl]-3-{2-[3-(ethoxycarbonyl)piperidin}-1-yl]ethylidene}-4-sulfanylpiperidine dihydrochloride obtained in Example 58 by conducting a reaction similar to that mentioned in Example 15.

¹H NMR (500 MHz, pyridine-d₅) δ ppm : 0.75-0.92 (2H, m), 1.02-1.17 (2H, m), 1.62-1.89 (3H, m), 2.11-2.38 (4H, m), 2.61-2.74 (1H, m), 2.74-3.00 (2H, m), 3.13-3.32 (1H, m), 3.37-3.54 (1H, m), 3.54-3.69 (3H, m), 3.85-4.10 (4H, m), 5.01-5.08 (1H, m), 6.27-6.37 (1H, m), 7.22-7.46 (3H, m), 7.65-7.79 (1H, m);

IR (KBr, cm⁻¹) : 2542, 1713, 1493.

### (Example 60) (E)-4-(Acetylsulfanyl)-1-[2-cyclopropyl-1-(2-fluorophenyl)-2-oxoethyl]-3-{[2-(4-ethoxycarbonyl-1H-pyrazol-1-yl)-1-methyl]ethylidene}piperidine (Compound of Compound No. 1-978)

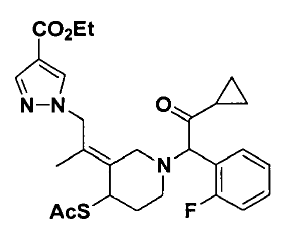

### (a) (E)-3-[1-(Ethoxycarbonyl)ethylidene]-4-hydroxy-1-(triphenylmethyl)piperidine

To a solution of (E)-4-(t-butyldimethylsilyloxy)-3-[(ethoxycarbonyl)methylidene]-1-(triphenylmethyl)piperidine (1.67 g) obtained in Example 1 (a) in tetrahydrofuran (20 ml) was added dropwise a 2.0N solution of lithium diisopropylamide in heptane / tetrahydrofuran / ethyl benzene solution (2.30 ml) at -78°C, and the resulting mixture was stirred at the same temperature for 1 hour. To the reaction mixture was added dropwise methyl iodide (0.31 ml) at -78°C, and then the resulting mixture was stirred for 2 hours while gradually warmed to room temperature. The reaction mixture was charged with a saturated aqueous ammonium chloride solution and then extracted with ethyl acetate. The extract was washed with water and a saturated aqueous sodium chloride solution, and the organic layer was dried over anhydrous sodium sulfate. The solvent was removed in vacuo, and the residue was purified by chromatography on a silica gel column (ethyl acetate / hexane = 3/97) to afford a 1:2 mixture (1.18 g, yield 69%) of (E)-4-(t-butyldimethylsilyloxy)-3-[(1-ethoxycarbonyl)ethylidene]-1-(triphenylmethyl)piperidine and the (Z)-isomer thereof as a pale yellow amorphous solid. To a solution of this mixture (0.97 g) in tetrahydrofuran (15 ml) was added a 75% solution of tetrabutylammonium fluoride (1.40 g) in water, and the resulting mixture was stirred at 60°C for 2 hours. The reaction mixture was diluted with ethyl acetate and washed with water and a saturated aqueous sodium chloride solution, and the organic layer was dried over anhydrous sodium sulfate. The solvent was removed in vacuo, and the residue was purified by chromatography on a silica gel column (ethyl acetate / hexane =1/19-1/1) to afford the title compound (292 mg, yield 38%) as a pale yellow oil.

¹H NMR (400 MHz, CDCl₃) δ ppm : 1.03 (3H, t, J=7.0), 1.78-2.50 (6H, m), 2.74-2.83 (1H, m), 3.20-3.28 (1H, m), 3.84-4.03 (3H, m), 4.79 (1H, bs), 7.08-7.32 (9H, m), 7.37-7.50 (6H, m).

### (b) (E)-4-(t-Butyldimethylsilyloxy)-3-[1-(ethoxycarbonyl)ethylidene]-1-(triphenylmethyl)piperidine

To a solution of (E)-3-[1-(ethoxycarbonyl)ethylidene]-4-hydroxy-1-(triphenylmethyl)piperidine (1.05 g) obtained in (a) above in dichloromethane (20 ml) were added dropwise t-butyldimethylsilyl trifluoromethanesulfonate (0.60 ml) and 2,6-lutidine (0.83 ml) under ice cooling, and the resulting mixture was stirred at the same temperature for 10 minutes. The reaction mixture was charged with a saturated aqueous sodium hydrogen carbonate solution and then extracted with ethyl acetate. The extract was washed with a saturated aqueous sodium chloride solution, and the organic layer was dried over anhydrous sodium. The solvent was removed in vacuo, and the residue was purified by chromatography on a silica gel column (toluene / hexane =1/9-1/4, then ethyl acetate / hexane =1/9-1/4) to afford the title compound (1.05 g, yield 79%) as a pale yellow oil.

¹H NMR (400 MHz, CDCl₃) δ ppm : -0.16 (3H, s), -0.06 (3H, s), 0.63 (9H, s), 0.99 (3H, t, J=7.5), 1.64-2.32 (7H, m), 2.75-2.83 (1H, m), 3.83-4.02 (3H, m), 4.68 (1H, bs), 7.07-7.34 (9H, m), 7.38-7.52 (6H, m).

### (c) (E)-4-(t-Butyldimethylsilyloxy)-3-[(2-hydroxy-1-methyl)ethylidene]-1-(triphenylmethyl)piperidine

To a suspension of (E)-4-(t-butyldimethylsilyloxy)-3-[1-(ethoxycarbonyl)ethylidene]-1-(triphenylmethyl)piperidine (1.05 g) obtained in (b) above in dichloromethane (15 ml) was added dropwise a 1.01N solution of diisobutylaluminum hydride in toluene (5.7 ml) at -78°C, and the resulting mixture was stirred at the same temperature for 30 minutes. To the reaction mixture was added an aqueous potassium sodium tartrate solution, and then the resulting mixture was stirred at room temperature for 2 hours. The reaction mixture was diluted with ethyl acetate and washed with a saturated aqueous sodium chloride solution, and the organic layer was dried over anhydrous sodium sulfate. The solvent was removed in vacuo, and the residue was purified by chromatography on a silica gel column (ethyl acetate / hexane =1/19-1/4) to afford the title compound (0.72 g, yield 74%) as a colorless amorphous solid.

¹H NMR (400 MHz, CDCl₃) δ ppm : -0.20 (3H, s), -0.07 (3H, s), 0.61 (9H, s), 1.62-2.20 (7H, m), 2.76-2.84 (1H, m), 3.57 (1H, d, J=12.0), 4.03-4.08 (2H, m), 4.70 (1H, bs), 7.08-7.28 (9H, m), 7.40-7.54 (6H, m).

### (d) (E)-4-(t-Butyldimethylsilyloxy)-3-{[2-(4-ethoxycarbonyl-1H-pyrazo1-1-yl)-1-methyl]ethylidene}-1-(triphenylmethyl)piperidine

The title compound was synthesized in a yield of 94% as a pale yellow amorphous solid using (E)-4-(t-butyldimethylsilyloxy)-3-[(2-hydroxy-1-methyl)ethylidene]-1-(triphenylmethyl)piperidine obtained in (c) above by conducting a reaction similar to that mentioned in Example 40 (a).

¹H NMR (400 MHz, CDCl₃) δ ppm : -0.20 (3H, s), -0.08 (3H, s), 0.62 (9H, s), 1.34 (3H, t, J=7.5), 1.62-2.08 (6H, m), 2.30-2.36 (1H, m), 2.83-2.90 (1H, m), 3.60-3.67 (1H, m), 4.22-4.35 (2H, m), 4.67-4.75 (3H, m), 7.07-7.33 (9H, m), 7.43-7.57 (6H, m), 7.77 (1H, s), 7.92 (1H, s).

### (e) (E)-3-{[2-(4-Ethoxycarbonyl-1H-pyrazol-1-yl)-1-methyl]ethylidene}-4-hydroxy-1-(triphenylmethyl)piperidine

The title compound was synthesized in a yield of 69% as a yellow amorphous solid using (E)-4-(t-butyldimethylsilyloxy)-3-{[2-(4-ethoxycarbonyl-1H-pyrazol-1-yl)-1-methyl]ethylidene}-1-(triphenylmethyl)piperidine obtained in (d) above by conducting a reaction similar to that mentioned in Example 1 (e).

¹H NMR (400 MHz, CDCl₃) δ ppm : 1.36 (3H, t, J=7.5), 1.69 (3H, s), 1.86-2.35 (4H, m), 2.88-2.97 (1H, m), 3.75 (1H, d, J=13.5), 4.31 (2H, q, J=7.5), 4.62-4.73 (2H, m), 4.83 (1H, bs), 7.10-7.30 (9H, m), 7.40-7.56 (6H, m), 7.80 (1H, s), 7.92 (1H, s).

### (f) (E)-4-(Acetylsulfanyl)-3-{[2-(4-ethoxycarbonyl-1H-pyrazol-1-yl)-1-methyl]ethylidene}piperidine hydrogen trifluoroacetate

The title compound was synthesized in a yield of 67% as a colorless oil using (E)-3-{[2-(4-ethoxycarbonyl-1H-pyrazol-1-yl)-1-methyl]ethylidene}-4-hydroxy-1-(triphenylmethyl)piperidine obtained in (e) above by conducting a reaction similar to that mentioned in Example 36 (d).

¹H NMR (400 MHz, CDCl₃) δ ppm : 1.33 (3H, t, J=7.0), 1.80 (3H, s), 2.01-2.12 (1H, m), 2.37 (3H, s), 3.16-3.56 (4H, m), 4.28 (2H, q, J=7.0), 4.47 (1H, d, J=13.5), 4.72 (2H, s), 4.89 (1H, bs), 7.86 (1H, s), 7.88 (1H, s).

### (g) (E)-4-(Acetylsulfanyl)-1-[2-cyclopropyl-1-(2-fluorophenyl)-2-oxoethyl]-3-{[2-(4-ethoxycarbonyl-1 H-pyrazol-1-yl)-1-methyl]ethylidene}piperidine

The title compound was synthesized in a yield of 78% as a colorless amorphous solid using (E)-4-(acetylsulfanyl)-3-{[2-(4-ethoxycarbonyl-1H-pyrazol-1-yl)-1-methyl]ethylidene}piperidine hydrogen trifluoroacetate obtained in (f) above by conducting a reaction similar to that mentioned in Example 1 (g).

¹H NMR (400 MHz, CDCl₃) δ ppm : 0.65-1.08 (4H, m), 1.35 (3H, t, J=7.0), 1.70 and 1.73 (total 3H, each s), 1.76-1.91 (1H, m), 2.02-2.99 (7H, m), 3.80-3.93 (1H, m), 4.24-4.34 (2H, m), 4.56-4.88 (5H, m), 7.08-7.23 (2H, m), 7.27-7.47 (2H, m), 7.77-7.91 (2H, m).

### (Example 61) (E)-1-[2-Cyclopropyl-1-(2-fluorophenyl)-2-oxoethyl]-3-{[2-(4-ethoxycarbonyl-1H-pyrazol-l-yl)-1-methyl]ethylidene}-4-sulfanylpiperidine hydrochloride (Hydrochloride of the compound of Compound No. 1-977)

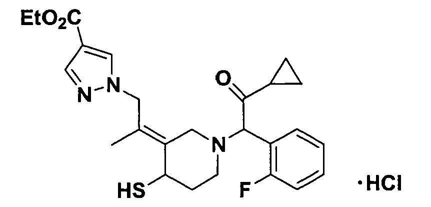

The title compound was synthesized in a yield of 64% as a colorless amorphous solid using (E)-4-(acetylsulfanyl)-1-[2-cyclopropyl-1-(2-fluorophenyl)-2-oxoethyl]-3-{[2-(4-ethoxycarbonyl-1H-pyrazol-1-yl)-1-methyl]ethylidene}piperidine obtained in Example 60 (g) above by conducting a reaction similar to that mentioned in Example 34.

¹H NMR (400 MHz, pyridine-d₅) δ ppm : 0.69-0.87 (2H, m), 0.96-1.16 (2H, m), 1.22 (3H, t, J=7.0), 1.70-2.80 (7H, m), 2.89-3.00 (1H, m), 3.19 and 3.36 (total 1H, each d, J=13.5), 4.01-4.48 (4H, m), 4.75-5.04 (3H, m), 7.14-7.37 (3H, m), 7.71-7.81 (1H, m), 8.25 (1H, s), 8.38 and 8.48 (total 1H, each s);

IR (KBr, cm⁻¹) : 2611, 1713, 1494.

### (Example 62) (E)-3-{[2-(4-Carboxy-1H-pyrazol-1-yl)-1-methyl]ethylidene}-1-[2-cyclopropyl-1-(2-fluorophenyl)-2-oxoethyl]-4-sulfanylpiperidine hydrochloride (Hydrochloride of the compound of Compound No. 1-973)

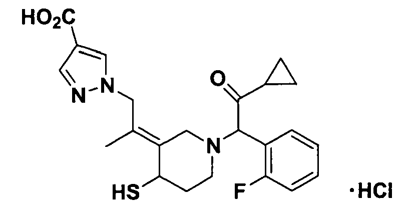

The title compound was synthesized in a yield of 75% as a colorless amorphous solid using (E)-1-[2-cyclopropyl-1-(2-fluorophenyl)-2-oxoethyl]-3-{[2-(4-ethoxycarbonyl-1H-pyrazol-1-yl)-1-methyl]ethylidene}-4-sulfanylpiperidine hydrochloride obtained in Example 61 by conducting a reaction similar to that mentioned in Example 15.

¹H NMR (400 MHz, pyridine-d₅) δ ppm : 0.72-0.88 (2H, m), 0.97-1.17 (2H, m), 1.57-3.03 (8H, m), 3.20 and 3.37 (total 1H, each d, J=12.5), 4.06 and 4.18 (total 1H, each d, J=12.5), 4.37-4.47 (1H, m), 4.77-5.04 (3H, m), 7.16-7.35 (3H, m), 7.74-7.84 (1H, m), 8.41 (1H, s), 8.47 and 8.57 (total 1H, each s);

IR (KBr, cm⁻¹) : 2630, 1711, 1494.

### (Example 63) (E)-4-(Acetylsulfanyl)-3-(2-cyanoethylidene)-1-[2-cyclopropyl-1-(2-fluorophenyl)-2-oxoethyl]piperidine hydrochloride (Hydrochloride of the compound of Compound No. 3-47)

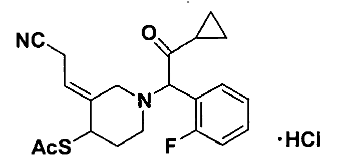

### (a) (E)-4-(t-Butyldimethylsilyloxy)-3-(2-cyanoethylidene)-1-(triphenylmethyl)piperidine

To a solution of sodium cyanide (13.7 g) in a mixed solvent of N,N-dimethylformamide (250 ml) and water (25 ml) was added (E)-4-(t-butyldimethylsilyloxy)-3-[2-(tosyloxy)ethylidene]-1-(triphenylmethyl)piperidine (34.4 g) obtained in Example 1 (c) at room temperature, and the resulting mixture was stirred for 30 minutes. The reaction mixture was charged with water and then extracted with ethyl acetate. The extract was washed with water and a saturated aqueous sodium chloride solution, and the organic layer was dried over anhydrous magnesium sulfate. The solvent was removed in vacuo to afford the title compound (26.8 g, quantitative yield) as a brown oil.

¹H NMR (500 MHz, CDCl₃) δ ppm : 0.01 (3H, s), 0.02 (3H, s), 0.88 (9H, s), 1.64-1.98 (4H, m), 2.94-3.16 (3H, m), 3.45-3.61 (1H, m), 3.82-3.89 (1H, m), 5.57 (1H, t, J=7.5), 7.12-7.36 (9H, m), 7.39-7.56 (6H, m).

### (b) (E)-3-(2-Cyanoethylidene)-1-(triphenylmethyl)piperidin-4-ol

The title compound was synthesized in a yield of 65% as a pale yellow amorphous solid using (E)-4-(t-butyldimethylsilyloxy)-3-(2-cyanoethylidene)-1-(triphenylmethyl)piperidine obtained in (a) above by conducting a reaction similar to that mentioned in Example 1 (e).

¹H NMR (400 MHz, CDCl₃) δ ppm : 1.75-2.28 (4H, m), 2.75-2.95 (1H, m), 3.07 (2H, m), 3.19-3.41 (1H, m), 3.91-4.02 (1H, m), 5.58 (1H, t, J=7.5), 7.14-7.35 (9H, m), 7.39-7.55 (6H, m).

### (c) (E)-4-(Acetylsulfanyl)-3-(2-cyanoethylidene)piperidine hydrogen trifluoroacetate

The title compound was synthesized in a yield of 12% as a colorless solid using (E)-3-(2-cyanoethylidene)-1-(triphenylmethyl)piperidin-4-ol obtained in (b) above by conducting a reaction similar to that mentioned in Example 36 (d).

¹H NMR (400 MHz, DMSO-d₆) δ ppm : 1.82-1.93 (1H, m), 2.11-2.23 (1H, m), 2.37 (3H, s), 3.05-3.17 (1H, m), 3.25-3.43 (1H, m), 3.47 (2H, d, J=7.0), 3.60-3.68 (1H, m), 3.90-3.97 (1H, m), 4.40 (1H, m), 5.77 (1H, t, J=7.0).

### (d) (E)-4-(Acetylsulfanyl)-3-(2-cyanoethylidene)-1-[2-cyclopropyl-1-(2-fluorophenyl)-2-oxoethyl]piperidine hydrochloride

The title compound was synthesized in a yield of 58% as a yellow oil using (E)-4-(acetylsulfanyl)-3-(2-cyanoethylidene)piperidine hydrogen trifluoroacetate obtained in (c) above by conducting a reaction similar to that mentioned in Example 1 (g).

¹H NMR (500 MHz, CDCl₃) δ ppm : 0.79-0.92 (2H, m), 0.99-1.09 (2H, m), 1.77-1.85 (1H, m), 2.05-2.27 (2H, m), 2.29 and 2.31 (total 3H, each s), 2.46-2.58 (1H, m), 2.64-2.71 and 2.75-2.81 (total 1H, each m), 2.76 and 3.08 (total 1H, each d, J=12.5), 3.02-3.11 (2H, m), 3.15 and 3.33 (total 1H, each d, J=12.5), 4.27 and 4.32 (total 1H, each m), 4.74 and 4.77 (total 1H, each s), 5.57 (1H, t, J=7.0), 7.11-7.23 (2H, m), 7.32-7.39 (2H, m).

### (Example 64) (E)-3-(2-Carboxyethylidene)-1-[2-cyclopropyl-1-(2-fluorophenyl)-2-oxoethyl]-4-sulfanylpiperidine hydrochloride (Hydrochloride of the compound of Compound No. 3-13)

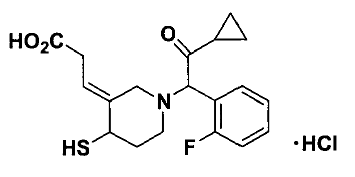

The title compound was synthesized in a yield of 39% as a colorless amorphous solid using (E)-4-(acetylsulfanyl)-3-(2-cyanoethylidene)-1-[2-cyclopropyl-1-(2-fluorophenyl)-2-oxoethyl]piperidine obtained in Example 63 (d) by conducting a reaction similar to that mentioned in Example 37.

¹H NMR (500 MHz, pyridine-d₅) δ ppm : 0.77-0.94 (2H, m), 0.97-1.04 (1H, m), 1.10-1.20 (1H, m), 1.75-1.85 (1H, m), 2.17-2.29 (1H, m), 2.51-2.60 (1H, m), 2.62-2.69 (1H, m), 2.87 and 2.97 (total 1H, each m), 3.32-3.44 (3H, m), 3.59 (1H, m), 3.88-3.95 (1H, m), 4.91 and 4.93 (total 1H, each s), 6.18-6.24 (1H, m), 7.17-7.26 (2H, m), 7.27-7.34 (1H, m), 7.70-7.75 (1H, m).

MS (FAB) m/z : 364 (M+H)⁺.

### (Example 65) (E)-1-[2-Cyclopropyl-1-(2-fluorophenyl)-2-oxoethyl]-3-[2-(ethoxycarbonyl)ethylidene]-4-sulfanylpiperidine hydrochloride (Hydrochloride of the compound of Compound No. 3-17)

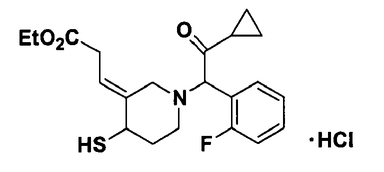

The title compound was synthesized in a yield of 81 % as a colorless amorphous solid using (E)-3-(2-carboxyethylidene)-1-[2-cyclopropyl-1-(2-fluorophenyl)-2-oxoethyl]-4-sulfanylpiperidine hydrochloride obtained in Example 64 by conducting a reaction similar to that mentioned in Example 38.

¹H NMR (400 MHz, pyridine-d₅) δ ppm : 0.76-0.88 (2H, m), 0.97-1.04 (1H, m), 1.11-1.17 (1H, m), 1.11 (3H, t, J=7.0), 1.73-1.84 (1H, m), 2.12-2.26 (1H, m), 2.44-2.53 (1H, m), 2.60-2.69 (1H, m), 2.80-2.99 (1H, m), 3.20-3.35(3H, m), 3.53 (1H, m), 3.87 (1H, m), 4.10 (2H, q, J=7.0), 4.91 and 4.92 (total 1H, each s), 5.98-6.05 (1H, m), 7.17-7.26 (2H, m), 7.28-7.36 (1H, m), 7.66-7.72 (1H, m).

MS (FAB) m/z : 392 (M+H)⁺.

### (Example 66) (Z)-4-(Acetylsulfanyl)-1-[2-cyclopropyl-1-(2-fluorophenyl)-2-oxoethyl]-3-(2-hydroxyethylidene)piperidine hydrochloride (Hydrochloride of the compound of Compound No. 4-2)

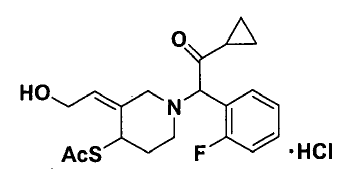

### (a) (E)-3-(2-Hydroxyethylidene)-1-(triphenylmethyl)piperidin-4-ol

The title compound was synthesized in a yield of 86% as a colorless amorphous solid using (E)-4-(t-butyldimethylsilyloxy)-3-(2-hydroxyethylidene)-1-(triphenylmethyl)piperidine obtained in Example 1 (b) by conducting a reaction similar to that mentioned in Example 1 (e).

¹H NMR (400 MHz, CDCl₃) δ ppm : 1.75-1.87 (2H, m), 2.02-2.04 (2H, m), 2.78 (1H, bs), 3.33 (1H, bs), 3.97 (1H, bs), 4.10-4.22 (2H, m), 5.74 (1H, t, J=7.0), 7.13-7.31 (15H, m).

### (b) (E)-3-[2-(t-Butyldiphenylsilyloxy)ethylidene]-1-(triphenylmethyl)piperidin-4-ol

To a solution of (E)-3-(2-hydroxyethylidene)-1-(triphenylmethyl)piperidin-4-ol (5.50 g) obtained in (a) above in dichloromethane (120 ml) were added t-butyldiphenylsilyl chloride (6.30 g), triethylamine (4.00 ml) and 4-dimethylaminopyridine (0.35 g) under ice cooling, and the resulting mixture was stirred at room temperature for 24 hours. The reaction mixture was evaporated in vacuo and then diluted with dichloromethane. The resulting mixture was washed with water and a saturated aqueous sodium chloride solution, and the organic layer was dried over anhydrous magnesium sulfate. The solvent was removed in vacuo, and the residue was purified by chromatography on a silica gel column (ethyl acetate /hexane = 1 / 4) to afford the title compound (7.54 g, yield 85%) as a colorless amorphous solid.

¹H NMR (400 MHz, CDCl₃) δ ppm : 1.03 (9H, s), 1.68-1.81 (2H, m), 1.97-2.09 (2H, m), 2.64 (1H, bs), 3.03 (1H, bs), 3.89 (1H, bs), 4.22-4.35 (2H, m), 5.66 (1H, t, J=7.0), 7.04-7.50 (20H, m), 7.60-7.75 (5H, m).

### (c) (E)-4-Acetoxy-3-[2-(t-butyldiphenylsilyloxy)ethylidene]-1-(triphenylmethyl)piperidine

To a solution of (E)-3-[2-(t-butyldiphenylsilyloxy)ethylidene]-1-(triphenylmethyl)piperidin-4-ol (512 mg) obtained in (b) above in dichloromethane (8 ml) were added acetic anhydride (0.10 ml), triethylamine (0.17 ml) and 4-dimethylaminopyridine (10 mg) under ice cooling, and the resulting mixture was stirred at room temperature overnight. The reaction mixture was diluted with ethyl acetate and washed with a saturated aqueous sodium chloride solution, and the organic layer was dried over anhydrous sodium sulfate. The solvent was removed in vacuo, and the residue was purified by chromatography on a silica gel column (ethyl acetate / hexane = 1 / 19 - 3 / 17) to afford the title compound (527 mg, yield 96%) as a colorless amorphous solid.

¹H NMR (500 MHz, CDCl₃) δ ppm : 1.03 (9H, s), 1.76-1.89 (1H, m), 1.94-2.10 (4H, m), 2.40-2.76 (2H, m), 2.80-3.18 (1H, m), 3.39-3.64 (1H, m), 4.26 (2H, t, J=6.0), 4.99-5.11 (1H, m), 5.61 (1H, t, J=6.0), 7.03-7.48 (21H, m), 7.59-7.70 (4H, m).

### (d) (Z)-4-(Acetylsulfanyl)-3-(2-hydroxyethylidene)-1-(triphenylmethyl)piperidine and (E)-4-(acetylsulfanyl)-3-(2-hydroxyethylidene)-1-(triphenylmethyl)piperidine

Palladium chloride (7 mg) and 1,1'-bis(diphenylphosphino)ferrocene (44 mg) were dissolved in 1,4-dioxane (4 ml) under a nitrogen gas stream, and the resulting mixture was stirred at room temperature for 30 minutes. To the resulting mixture were added a solution of (E)-4-acetoxy-3-[2-(t-butyldiphenylsilyloxy)ethylidene]-1-(triphenylmethyl)piperidine (527 mg) obtained in (c) above in 1,4-dioxane (2 ml) and a solution of potassium thioacetate (283 mg) in water (1.5 ml), and the resulting mixture was stirred at 100°C for 6 hours. The reaction mixture was diluted with ethyl acetate and washed with a saturated aqueous sodium chloride solution, and the organic layer was dried over anhydrous sodium sulfate. The solvent was removed in vacuo, and the residue was purified by chromatography on a silica gel column (ethyl acetate / hexane = 1 / 19 - 3 / 17) to afford a 1:7 mixture (479 mg, yield 89%) of (Z)-4-(acetylsulfanyl)-3-[2-(t-butyldiphenylsilyloxy)ethylidene]-1-(triphenylmethyl)piperidine and the (E)-isomer thereof as a colorless oil.

To a solution of this 1:7 mixture (374 mg) in tetrahydrofuran (5 ml) were added acetic acid (0.12 ml) and a 75% solution of tetrabutylammonium fluoride (750 mg) in water at room temperature, and the resulting mixture was stirred at the same temperature for 3 hours and at 50°C for further 2 hours. The reaction mixture was diluted with ethyl acetate and washed with a saturated aqueous sodium chloride solution, and the organic layer was dried over anhydrous sodium sulfate. The solvent was removed in vacuo, and the residue was purified by chromatography on a silica gel column (ethyl acetate / hexane =1/19-1/3) to afford the (Z) form of the title compound (25 mg, yield 11 %) as a colorless amorphous solid and the (E) form (178 mg, yield 74%) as a white crystal.

### (Z)-4-(Acetylsulfanyl)-3-(2-hydroxyethylidene)-1-(triphenylmethyl)piperidine:

¹H NMR (400 MHz, CDCl₃) δ ppm : 1.60 (1H, dd, J=12.0, 2.0), 1.77-1.83 (1H, m), 2.13 (1H, d, J=12.0), 2.20 (3H, s), 2.31-2.42 (1H, m), 3.03-3.10 (1H, m), 3.37 (1H, dd, J=12.0, 2.0), 4.19-4.28 (1H, m), 4.40-4.48 (1H, m), 4.86 (1H, d, J=4.0), 5.51 (1H, t, J=7.0), 7.11-7.54 (15H, m).

### (E)-4-(Acetylsulfanyl)-3-(2-hydroxyethylidene)-1-(triphenylmethyl)piperidine:

¹H NMR (400 MHz, CDCl₃) δ ppm : 1.83-1.96 (1H, m), 2.21-2.42 (1H, m), 2.37 (3H, s), 2.95-3.16 (2H, m), 3.36-3.48 (1H, m), 3.60-3.79 (3H, m), 4.40 (1H, m), 5.98 (1H, t, J=6.0), 7.19-7.31 (9H, m), 7.36-7.40 (6H, m).

### (e) (Z)-4-(Acetylsulfanyl)-1-[2-cyclopropyl-1-(2-fluorophenyl)-2-oxoethyl]-3-(2-hydroxyethylidene)piperidine hydrochloride

To a solution of (Z)-4-(acetylsulfanyl)-3-(2-hydroxyethylidene)-1-(triphenylmethyl)piperidine (130 mg) obtained in (d) above in dichloromethane (5 ml) was added trifluoroacetic acid (0.30 ml) under ice cooling, and the resulting mixture was stirred at room temperature for 1 hour. The solvent was removed in vacuo, and the residue was purified by chromatography on a silica gel column (methanol / dichloromethane = 1 / 5) to afford (Z)-4-(acetylsulfanyl)-3-(2-hydroxyethylidene)piperidine hydrogen trifluoroacetate as a colorless amorphous solid.

To a solution of the above amorphous solid and 2-bromo-2-(2-fluorophenyl)-1-cyclopropylethanone (150 mg) in acetonitrile (2 ml) was added triethylamine (0.10 ml) under ice cooling, and the resulting mixture was stirred at room temperature for 15 minutes. The reaction mixture was diluted with ethyl acetate and washed with water and a saturated aqueous sodium chloride solution, and the organic layer was dried over anhydrous sodium sulfate. The solvent was removed in vacuo, and the residue was purified by chromatography on a silica gel column (ethyl acetate / hexane = 1 / 1) to afford the free form (54 mg, yield 49%) of the title compound as a colorless amorphous solid. To a solution of this compound in dioxane (3 ml) was added a 4N solution of hydrogen chloride in dioxane (0.11 ml) at room temperature, and the solvent was removed in vacuo to afford the title compound (61 mg, quantitative yield) as a colorless amorphous solid.

¹H NMR (500 MHz, pyridine-d₅) δ ppm : 0.70-0.84 (2H, m), 0.94-1.13 (2H, m), 1.75-1.89 (1H, m), 2.13-2.61 (3H, m), 2.19 (3H, s), 2.87-3.11 (1H, m), 2.91 and 3.07 (total 1H, each d, J=12.0), 3.37 and 3.54 (total 1H, each d, J=12.0), 4.54-4.64 (1H, m), 4.71-4.81 (1H, m), 4.83 and 4.88 (total 1H, each s), 5.12 (1H, bs), 5.81 and 5.91 (total 1H, each t, J=6.5), 7.17-7.24 (2H, m), 7.27-7.33 (1H, m), 7.61-7.68 (1H, m).

MS (FAB) m/z : 378 (M+H)⁺.

### (Example 67) (Z)-4-(Acetylsulfanyl)-1-[2-cyclopropyl-1-(2-fluorophenyl)-2-oxoethyl]-3-(2-{4-[2-(ethoxycarbonyl)ethyl]-3-oxopiperazin-1-yl}ethylidene)piperidine (Compound of Compound No. 6-108)

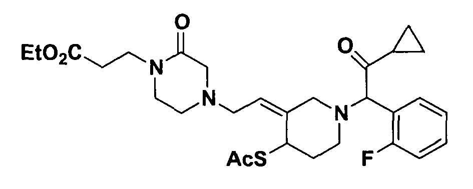

The title compound was synthesized in a yield of 50% as a colorless amorphous solid using (Z)-4-(acetylsulfanyl)-1-[2-cyclopropyl-1-(2-fluorophenyl)-2-oxoethyl]-3-(2-hydroxyethylidene)piperidine obtained in Example 66 (e) and 1-[2-(ethoxycarbonyl)ethyl]-2-oxopiperazine hydrochloride by conducting a reaction similar to that mentioned in Example 33.

¹H NMR (400 MHz, CDCl₃) δ ppm : 0.79-0.90 (2H, m), 0.94-1.07 (2H, m), 1.26 and 1.27 (total 3H, each t, J=7.0), 1.71-1.84 (1H, m), 2.06-2.50 (3H, m), 2.30 (3H, s), 2.57-2.72 (6H, m), 2.77-3.33 (5H, m), 3.34-3.42 (2H, m), 3.58-3.66 (2H, m), 4.13 and 4.15 (total 2H, each q, J=7.0), 4.63 and 4.67 (total 1H, each s), 4.78 (1H, bs), 5.27 and 5.36 (total 1H, each t, J=7.0), 7.07-7.20 (2H, m), 7.28-7.44 (2H, m).

MS (FAB) m/z : 560 (M+H)⁺.

### (Example 68) (Z)-1-[2-Cyclopropyl-1-(2-fluorophenyl)-2-oxoethyl]-3-(2-{4-[2-(ethoxycarbonyl)ethyl]-3-oxopiperazin-1-yl}ethylidene)-4-sulfanylpiperidine dihydrochloride (Hydrochloride of the compound of Compound No. 6-107)

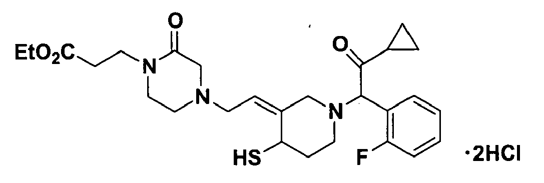

The title compound was synthesized in a yield of 66% as a colorless amorphous solid using (Z)-4-(acetylsulfanyl)-1-[2-cyclopropyl-1-(2-fluorophenyl)-2-oxoethyl]-3-(2- {4-[2-(ethoxycarbonyl)ethyl]-3-oxopiperazin-1-yl}ethylidene)piperidine obtained in Example 67 by conducting a reaction similar to that mentioned in Example 34.

¹H NMR (400 MHz, pyridine-d₅) δ ppm : 0.71-0.83 (2H, m), 0.95-1.02 (1H, m), 1.05-1.11 (4H, m), 1.69-1.80 (1H, m), 2.15-2.30 (1H, m), 2.40-2.48 (1H, m), 2.64-3.00 (6H, m), 3.10-3.18 (2H, m), 3.19-3.51 (6H, m), 3.72-3.78 (2H, m), 4.07 and 4.08 (total 2H, each q, J=7.0), 4.43 (1H, bs), 4.90 and 4.92 (total 1H, each s), 5.35 and 5.43 (total 1H, each t, J=7.0), 7.16-7.23 (2H, m), 7.27-7.33 (2H, m).

MS (FAB) m/z : 518 (M+H)⁺.

### (Example 69) (Z)-3-(2-{4-[2-(Carboxyethyl)]-3-oxopiperazin-1-yl}ethylidene)-1-[2-cyclopropyl-1-(2-fluorophenyl)-2-oxoethyl]-4-sulfanylpiperidine dihydrochloride (Hydrochloride of the compound of Compound No. 6-103)

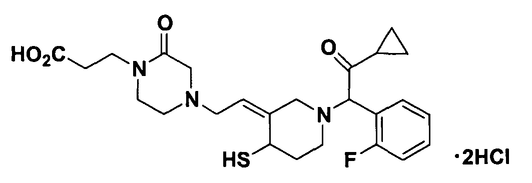

The title compound was synthesized in a yield of 95% as a colorless amorphous solid using (Z)-1-[2-cyclopropyl-1-(2-fluorophenyl)-2-oxoethyl]-3-(2-{4-[2-(ethoxycarbonyl)ethyl]-3-oxopiperazin-1-yl}ethylidene)-4-sulfanylpiperidine dihydrochloride obtained in Example 68 by conducting a reaction similar to that mentioned in Example 15.

¹H NMR (400 MHz, pyridine-d₅) δ ppm : 0.69-0.85 (2H, m), 0.94-1.13 (2H, m), 1.66-1.82 (1H, m), 2.12-2.33 (1H, m), 2.39-2.50 (1H, m), 2.60-3.04 (6H, m), 3.08-3.43 (6H, m), 3.43-3.54 (2H, m), 3.83-3.94 (2H, m), 4.44 (1H, bs), 4.90 and 4.92 (total 1H, each s), 5.33 and 5.44 (total 1H, each t, J=7.0), 7.16-7.33 (3H, m), 7.64-7.72 (1H, m).

MS (FAB) m/z : 490 (M+H)⁺.

### (Example 70) (E)-4-(Acetylsulfanyl)-1-[2-cyclopropyl-1-(2-fluorophenyl)-2-oxoethyl]-3-(2-{4-[3-(ethoxycarbonyl)propyl]-2-oxopiperazin-1-yl}ethylidene)piperidine dihydrochloride (Hydrochloride of the compound of Compound No. 5-86)

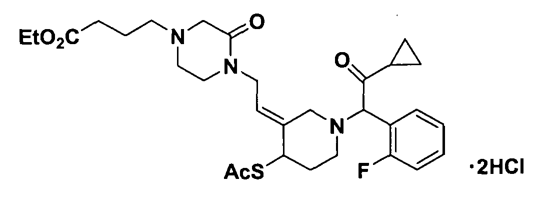

### (a) (E)-4-(t-Butyldimethylsilyloxy)-3-(2-{4-[3-(ethoxycarbonyl)propyl]-2-oxopiperazin-1-yl}ethylidene)-1-(triphenylmethyl)piperidine

The title compound was synthesized in a yield of 62% as a yellow oil using (E)-4-(t-butyldimethylsilyloxy)-3-[2-(tosyloxy)ethylidene]-1-(triphenylmethyl)piperidine obtained in Example 1 (c) and 1-[3-(ethoxycarbonyl)propyl]-3-oxopiperazine by conducting a reaction similar to that mentioned in Example 21 (a).

¹H NMR (400 MHz, CDCl₃) δ ppm : -0.05 (3H, s), -0.02 (3H, s), 0.83 (9H, s), 1.25 (3H, t, J=7.0), 1.75-1.94 (3H, m), 1.81 (2H, t, J=7.0), 2.35 (2H, t, J=7.0), 2.41 (2H, t, J=7.0), 2.63 (2H, t, J=5.5), 2.80-2.95 (1H, m), 3.12 (2H, s), 3.18-3.27 (2H, m), 3.50 (1H, m), 3.81-3.89 (1H, m), 3.94-4.10 (3H, m), 4.12 (2H, q, J=7.0), 5.44 (1H, t, J=6.5), 7.10-7.19 (3H, m), 7.21-7.31 (6H, m), 7.37-7.56 (6H, m).

### (b) (E)-3-(2-{4-[3-(Ethoxycarbonyl)propyl]-2-oxopiperazin-1-yl}ethylidene)-4-hydroxy-1-(triphenylmethyl)piperidine

The title compound was synthesized in a yield of 98% as a yellow amorphous solid using (E)-4-(t-butyldimethylsilyloxy)-3-(2-{4-[3-(ethoxycarbonyl)propyl]-2-oxopiperazin-1-yl}ethylidene)-1-(triphenylmethyl)piperidine obtained in (a) above by conducting a reaction similar to that mentioned in Example 1 (e).

¹H NMR (400 MHz, CDCl₃) δ ppm : 1.24 (3H, t, J=7.0), 1.74-1.84 (1H, m), 1.80 (2H, t, J=7.0), 1.91-2.11 (3H, m), 2.34 (2H, t, J=7.0), 2.40 (2H, t, J=7.0), 2.65 (2H, t, J=6.0), 2.74 (1H, m), 3.12 (2H, s), 3.19-3.40 (1H, m), 3.25 (2H, t, J=5.5), 3.90-4.01 (2H, m), 4.02-4.09 (1H, m), 4.11 (2H, q, J=7.0), 5.43 (1H, t, J=6.5), 7.06-7.32 (9H, m), 7.35-7.58 (6H, m).

### (c) (E)-4-(Acetylsulfanyl)-3-(2-{4-[3-(ethoxycarbonyl)propyl]-2-oxopiperazin-1-yl} ethyhdene)-1-(triphenylmethyl)piperidine

The title compound was synthesized in a yield of 10% as a yellow amorphous solid using (E)-3-(2-{4-[3-(ethoxycarbonyl)propyl]-2-oxopiperazin-l-yl}ethylidene)-4-hydroxy-1-(triphenylmethyl)piperidine obtained in (b) above by conducting a reaction similar to that mentioned in Example 1 (h).

¹H NMR (500 MHz, CDCl₃) δ ppm : 1.26 (3H, t, J=7.0), 1.74-1.88 (1H, m), 1.82 (2H, t, J=7.0), 2.15-2.39 (2H, m), 2.24 (3H, s), 2.36 (2H, t, J=7.5), 2.41 (2H, t, J=7.0), 2.49-2.70 (4H, m), 2.98-3.33 (1H, m), 3.13 (2H, s), 3.20 (2H, t, J=5.5), 3.92-4.07 (2H, m), 4.11 (2H, q, J=7.0), 4.25 (1H, bs), 5.44 (1H, t, J=6.5), 7.12-7.21 (3H, m), 7.23-7.32 (6H, m), 7.38-7.56 (6H, m).

### (d) (E)-4-(Acetylsulfanyl)-3-(2-{4-[3-(ethoxycarbonyl)propyl]-2-oxopiperazin-1-yl}ethylidene)piperidine bis(hydrogen trifluoroacetate)

The title compound was synthesized in a quantitative yield as a pale orange amorphous solid using (E)-4-(acetylsulfanyl)-3-(2-{4-[3-(ethoxycarbonyl)propyl]-2-oxopiperazin-1-yl}ethylidene)-1-(triphenylmethyl)piperidine obtained in (c) above by conducting a reaction similar to that mentioned in Example 3 (c).

¹H NMR (400 MHz, CDCl₃) δ ppm : 1.26 (3H, t, J=7.0), 1.93-2.05 (3H, m), 2.32-2.44 (1H, m), 2.35 (3H, s), 2.41 (2H, t, J=7.0), 2.99 (2H, t, J=7.5), 3.16-3.42 (4H, m), 3.50-3.82 (4H, m), 3.79 (2H, t, J=7.5), 4.13 (2H, q, J=7.0), 4.26-4.36 (2H, m), 4.42 (1H, m), 5.92 (1H, t, J=7.5).

### (e) (E)-4-(Acetylsulfanyl)-1-[2-cyclopropyl-1-(2-fluorophenyl)-2-oxoethyl]-3-(2-{4-[3-(ethoxycarbonyl)propyl]-2-oxopiperazin-1-yl} ethylidene)piperidine dihydrochloride

The title compound was synthesized in a yield of 70% as a pale brown amorphous solid using (E)-4-(acetylsulfanyl)-3-(2-{4-[3-(ethoxycarbonyl)propyl]-2-oxopiperazin-1-yl}ethylidene)piperidine bis(hydrogen trifluoroacetate) obtained in (d) above by conducting a reaction similar to that mentioned in Example 1 (g).

### ¹H NMR (500 MHz, pyridine-d₅) δ ppm : 0.76-0.88 (2H, m), 1.04-1.09 (1H, m), 1.10-1.18 (1H, m), 1.15 (3H, t, J=7.0), 1.77-1.91 (1H, m), 1.81 (2H, t, J=7.0), 2.15-2.35 (3H, m), 2.24 and 2.26 (total 3H, each s), 2.38 (2H, t, J=7.0), 2.40-2.47 (1H, m), 2.48-2.75 (3H, m), 2.77-2.83 and 2.85-2.91 (total 1H, each m), 3.06-3.32 (3H, m), 3.24 (2H, s), 3.64-3.70 (1H, m), 4.06-4.22 (1H, m), 4.13 (2H, q, J=7.0), 4.24-4.31 (1H, m), 4.52 (1H, m), 4.96 and 4.98 (total 1H, each s), 5.73-5.81 (1H, m), 7.20-7.39 (3H, m), 7.65-7.73 (1H, m).

IR (KBr, cm⁻¹) : 1727, 1662, 1495.

### (Example 71) (E)-1-[2-Cyclopropyl-1-(2-fluorophenyl)-2-oxoethyl]-3-(2-{4-[3-(ethoxycarbonyl)propyl]-2-oxopiperazin-1-yl}ethylidene)-4-sulfanylpiperidine dihydrochloride (Hydrochloride of the compound of Compound No. 5-85)

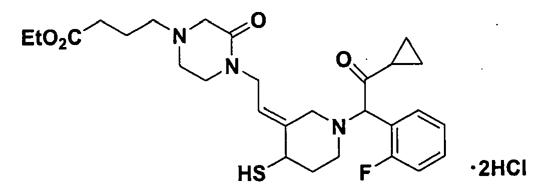

The title compound was synthesized in a yield of 35% as a pale yellow amorphous solid using (E)-4-(acetylsulfanyl)-1-[2-cyclopropyl-1-(2-fluorophenyl)-2-oxoethyl]-3-(2-{4-[3-(ethoxycarbonyl)propyl]-2-oxopiperazin-1-yl}ethylidene)piperidine dihydrochloride obtained in Example 70 (e) by conducting a reaction similar to that mentioned in Example 34.

¹H NMR (400 MHz, pyridine-d₅) δ ppm : 0.79-0.88 (2H, m), 1.00-1.08 (1H, m), 1.10-1.19 (1H, m), 1.15 (3H, t, J=7.0), 1.74-1.88 (1H, m), 1.83 (2H, t, J=7.0), 2.14-2.27 (1H, m), 2.29-2.57 (3H, m), 2.33 (2H, t, J=7.0), 2.39 (2H, t, J=7.0), 2.61-2.74 (1H, m), 2.85-3.04 (1H, m), 3.24-3.32 (2H, m), 3.27 (2H, s), 3.39 (1H, dd, J=12.0, 3.5), 3.53-3.68 (1H, m), 3.80-3.86 (1H, m), 4.13 (2H, q, J=7.0), 4.17-4.29 (2H, m), 4.97 (1H, s), 5.68-5.75 (1H, m), 7.21-7.42 (3H, m), 7.68-7.75 (1H, m).

IR (KBr, cm⁻¹) : 2536, 1727, 1661, 1495.

### (Example 72) (E)-3-(2-{4-[3-(Carboxypropyl)]-2-oxopiperazin-1-yl}ethylidene)-1-[2-cyclopropyl-1-(2-fluorophenyl)-2-oxoethyl]-4-sulfanylpiperidine dihydrochloride (Hydrochloride of the compound of Compound No. 5-81)

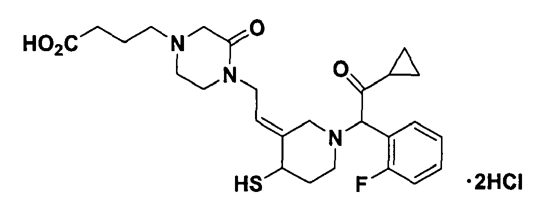

The title compound was synthesized in a yield of 57% as a colorless amorphous solid using (E)-1-[2-cyclopropyl-1-(2-fluorophenyl)-2-oxoethyl]-3-(2-{4-[3-(ethoxycarbonyl)propyl]-2-oxopiperazin-1-yl}ethylidene)-4-sulfanylpiperidine dihydrochloride obtained in Example 71 by conducting a reaction similar to that mentioned in Example 15.

¹H NMR (500 MHz, pyridine-d₅) δ ppm : 0.76-0.88 (2H, m), 1.01-1.08 (1H, m), 1.10-1.18 (1H, m), 1.74-1.84 (1H, m), 1.96 (2H, t, J=7.0), 2.13-2.28 (1H, m), 2.38-2.60 (5H, m), 2.42 (2H, t, J=7.0), 2.61-2.74 (1H, m), 2.84-3.00 (1H, m), 3.27 (2H, t, J=5.5), 3.30 (2H, s), 3.34-3.42 (1H, m), 3.55 and 3.64 (total 1H, each d, J=12.5), 3.79-3.86 (1H, m), 4.10-4.28 (2H, m), 4.96 (1H, s), 5.67-5.74 (1H, m), 7.19-7.37 (3H, m), 7.68-7.76 (1H, m).

IR (KBr, cm⁻¹) : 2576, 1713, 1659, 1495.

### (Example 73) (E)-4-(Acetylsulfanyl)-1-[2-cyclopropyl-1-(2-fluorophenyl)-2-oxoethyl]-3-{2-[4-(ethoxycarbonylmethyl)-1H-pyrazol-1-yl]ethylidene}piperidine (Compound of Compound No. 1-62)

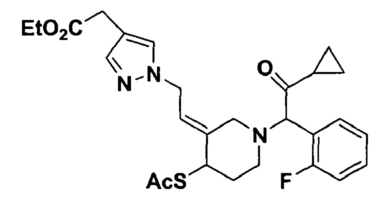

### (a) (E)-4-(t-Butyldimethylsilyloxy)-3-{2-[4-(ethoxycarbonylmethyl)-1H-pyrazol-1-yl] ethylidene} -1-(triphenylmethyl)piperidine

The title compound was synthesized in a yield of 45% as a colorless amorphous solid using (E)-4-(t-butyldimethylsilyloxy)-3-[2-(tosyloxy)ethylidene]-1-(triphenylmethyl)piperidine obtained in Example 1 (c) and 4-(ethoxycarbonylmethyl)-1H-pyrazole by conducting a reaction similar to that mentioned in Example 21 (a).

¹H NMR (400 MHz, CDCl₃) δ ppm : 0.06 (6H, s), 0.88 (9H, s), 1.29 (3H, t, J=7.0), 1.89-2.04 (4H, m), 3.03 (1H, bs), 3.55 (2H, s), 3.69 (1H, bs), 3.93-3.98 (1H, m), 4.19 (2H, q, J=7.0), 4.73-4.84 (2H, m), 5.84 (1H, t, J=7.5), 7.17-7.37 (15H, m), 7.44 (1H, s), 7.49 (1H, s).

### (b) (E)-3-{2-[4-(Ethoxycarbonylmethyl)-1H-pyrazol-1-yl]ethylidene}-4-hydroxy-1-(triphenylmethyl)piperidine

The title compound was synthesized in a yield of 63% as a colorless amorphous solid using (E)-4-(t-butyldimethylsilyloxy)-3-{2-[4-(ethoxycarbonylmethyl)-1H-pyrazol-1-yl]ethylidene}-1-(triphenylmethyl)piperidine obtained in (a) above by conducting a reaction similar to that mentioned in Example 1 (e).

¹H NMR (400 MHz, CDCl₃) δ ppm : 1.26 (3H, t, J=7.0), 1.58-1.61 (1H, m), 1.81-1.89 (1H, m), 2.00 (1H, bs), 2.08-2.14 (1H, m), 2.84 (1H, bs), 3.45 (1H, bs), 3.50 (2H, s), 4.00 (1H, bs), 4.16 (2H, q, J=7.0), 4.68-4.79 (2H, m), 5.77 (1H, t, J=7.0), 7.14-7.18 (3H, m), 7.24-7.28 (12H, m), 7.41 (1H, s), 7.44 (1H, s).

### (c) (E)-4-(Acetylsulfanyl)-3-{2-[4-(ethoxycarbonylmethyl)-1H-pyrazol-1-yl]ethylidene}-1-(triphenylmethyl)piperidine

The title compound was synthesized in a yield of 60% as a yellow oil using (E)-3- {2-[4-(ethoxycarbonylmethyl)-1H-pyrazol-1-yl]ethylidene}-4-hydroxy-1-(triphenylmethyl)piperidine obtained in (b) above by conducting a reaction similar to that mentioned in Example 1 (h).

### ¹H NMR (400 MHz, CDCl₃) δ ppm : 1.25 (3H, t, J=7.0), 1.79-1.92 (1H, m), 2.24 (3H, s), 2.29-2.33 (2H, m), 3.10-3.14 (1H, m), 3.41 (2H, s), 3.98 (1H, bs), 4.13 (2H, q, J=7.0), 4.25-4.36 (2H, m), 4.61-4.73 (2H, m), 5.78 (1H, t, J=7.0), 7.12-7.20 (3H, m), 7.21-7.31 (6H, m), 7.40-7.56 (8H, m).

### (d) (E)-4-(Acetylsulfanyl)-3-{2-[4-(ethoxycarbonylmethyl)-1H-pyrazol-1-yl]ethylidene}piperidine hydrogen trifluoroacetate

The title compound was synthesized in a yield of 20% as a colorless oil using (E)-4-(acetylsulfanyl)-3-{2-[4-(ethoxycarbonylmethyl)-1H-pyrazol-1-yl]ethylidene}-1-(triphenylmethyl)piperidine obtained in (c) above by conducting a reaction similar to that mentioned in Example 3 (c).

¹H NMR (400 MHz, CDCl₃) δ ppm : 1.28 (3H, t, J=7.0), 2.03-2.08 (1H, m), 2.36 (3H, s), 2.37-2.41 (1H, m), 3.24-3.31 (1H, m), 3.39-3.44 (1H, m), 3.47 (2H, s), 3.73 (1H, d, J=14.0), 4.10 (1H, d, J=14.0), 4.16 (2H, q, J=7.0), 4.44 (1H, t, J=4.0), 4.63-4.68 (1H, m), 4.73-4.80 (1H, m), 6.05 (1H, t, J=7.0), 7.41 (2H, s).

### (e) (E)-4-(Acetylsulfanyl)-1-[2-cyclopropyl-1-(2-fluorophenyl)-2-oxoethyl]-3-{2-[4-(ethoxycarbonylmethyl)-1H-pyrazol-1-yl]ethylidene}piperidine

The title compound was synthesized in a yield of 57% as a pale yellow oil using (E)-4-(acetylsulfanyl)-3-{2-[4-(ethoxycarbonylmethyl)-1H-pyrazol-1-yl]ethylidene}piperidine hydrogen trifluoroacetate obtained in (d) above by conducting a reaction similar to that mentioned in Example 1 (g).

¹H NMR (400 MHz, CDCl₃) δ ppm : 0.75-0.88 (2H, m), 0.97-1.07 (2H, m), 1.27 (3H, t, J=7.0), 1.78-1.86 (1H, m), 2.12-2.22 (2H, m), 2.28 and 2.29 (total 3H, each s), 2.49-2.62 (1H, m), 2.68-2.73 and 2.76-2.81 (total 1H, each m), 2.88 and 3.15 (total 1H, each d, J=12.5), 3.30 and 3.41 (total 1H, each d, J=12.5), 3.46 (2H, s), 4.15 (2H, q, J=7.0), 4.28-4.34 (1H, m), 4.62-4.64 and 4.67-4.70 (total 2H, each m), 4.71 and 4.74 (total 1H, each s), 5.78 (1H, t, J=7.0), 7.07-7.17 (2H, m), 7.19-7.27 (1H, m), 7.28-7.37 (1H, m), 7.33 and 7.34 (total 1H, each s), 7.39 (1H, s).

### (Example 74) (E)-3-{2-[4-(Carboxymethyl)-1H-pyrazol-1-yl]ethylidene}-1-[2-cyclopropyl-1-(2-fluorophenyl)-2-oxoethyl]-4-sulfanylpiperidine hydrochloride (Hydrochloride of the compound of Compound No. 1-53)

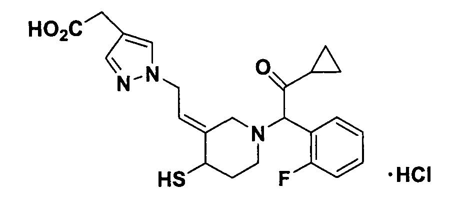

The title compound was synthesized in a yield of 28% as a colorless amorphous solid using (E)-4-(acetylsulfanyl)-1-[2-cyclopropyl-1-(2-fluorophenyl)-2-oxoethyl]-3-{2-[4-(ethoxycarbonylmethyl)-1H-pyrazol-1-yl]ethylidene}piperidine obtained in Example 73 (e) by conducting a reaction similar to that mentioned in Example 15.

¹H NMR (400 MHz, pyridine-d₅) δ ppm : 0.78-0.83 (2H, m), 0.98-1.04 (1H, m), 1.09-1.15 (1H, m), 1.74-1.83 (1H, m), 2.14-2.24 (1H, m), 2.46-2.55 (1H, m), 2.61-2.67 (1H, m), 2.82-2.98 (1H, m), 3.36 and 3.67 (total 2H, each d, J=12.5), 3.79 (3H, bs), 4.85-4.93 (3H, m), 5.98 (1H, t, J=7.0), 7.23-7.26 (2H, m), 7.29-7.35 (1H, m), 7.69-7.74 (1H, m), 7.81 (1H, s), 7.84 (1H, s).

MS (FAB) m/z : 444 (M+H)⁺.

### (Example 75) (E)-4-(Acetylsulfanyl)-1-[2-cyclopropyl-1-(2-fluorophenyl)-2-oxoethyl]-3-[2-(3-ethoxycarbonyl-1H-pyrazol-1-yl)ethylidene]piperidine (Compound of Compound No. 1-14)

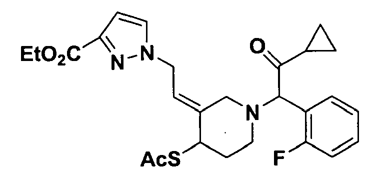

The title compound was synthesized in a yield of 12% as a yellow oil using (E)-4-(acetylsulfanyl)-1-[2-cyclopropyl-1-(2-fluorophenyl)-2-oxoethyl]-3-(2-hydroxyethylidene)piperidine obtained in Example 32 (f) and 3-ethoxycarbonyl-1H-pyrazole instead of 4-ethoxycarbonyl-1H-1,2,3-triazole by conducting a reaction similar to that mentioned in Example 47.

¹H NMR (400 MHz, CDCl₃) δ ppm : 0.80-0.91 (2H, m), 0.99-1.11 (2H, m), 1.37 (3H, t, J=7.0), 1.76-1.84 (1H, m), 2.12-2.35 (2H, m), 2.26 (3H, s), 2.47 and 2.62 (total 1H, each m), 2.73 (1H, m), 3.01 and 3.11 (total 1H, each d, J=12.5), 3.48 and 3.51 (total 1H, each d, J=12.5), 4.28-4.36 (3H, m), 4.75 and 4.77 (total 1H, each s), 5.13-5.21 (2H, m), 5.70-5.74 (1H, m), 6.81-6.82 (1H, m), 7.09-7.21 (2H, m), 7.33 (1H, m), 7.43-7.49 (2H, m).

### (Example 76) (E)-1-[2-Cyclopropyl-1-(2-fluorophenyl)-2-oxoethyl]-3-[2-(3-ethoxycarbonyl-1H-pyrazol-1-yl)ethylidene]-4-sulfanylpiperidine hydrochloride (Hydrochloride of the compound of Compound No. 1-13)

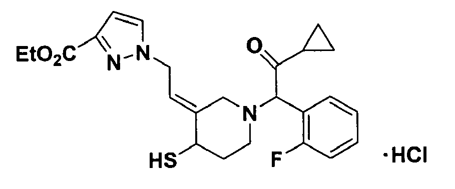

The title compound was synthesized in a yield of 39% as a colorless amorphous solid using (E)-4-(acetylsulfanyl)-1-[2-cyclopropyl-1-(2-fluorophenyl)-2-oxoethyl]-3-[2-(3-ethoxycarbonyl-1H-pyrazol-1-yl)ethylidene]piperidine obtained in Example 75 by conducting a reaction similar to that mentioned in Example 34.

¹H NMR (400 MHz, pyridine-d₅) δ ppm : 0.84-0.92 (2H, m), 1.03-1.09 (1H, m), 1.16-1.24 (1H, m), 1.20 and 1.21 (total 3H, each t, J=7.0), 1.74-1.82 (1H, m), 2.14-2.24 (1H, m), 2.55-2.68 (2H, m), 2.83-2.90 and 2.93-2.99 (total 1H, each m), 3.46 and 3.54 (total 1H, each t, d=12.0), 3.69-3.82 (2H, m), 4.26 (2H, q, J=7.0), 4.97 (1H, s), 5.43-5.46 (2H, m), 6.08 (1H, m), 6.99-7.00 (1H, m), 7.23-7.36 (3H, m), 7.68-7.69 (1H, m), 7.74-7.80 (1H, m).

MS (FAB) m/z : 458 (M+H)⁺.

### (Example 77) (E)-3-[2-(3-Carboxy-1H-pyrazol-1-yl)ethylidene]-1-[2-cyclopropyl-1-(2-fluorophenyl)-2-oxoethyl]-4-sulfanylpiperidine hydrochloride (Hydrochloride of the compound of Compound No. 1-5)

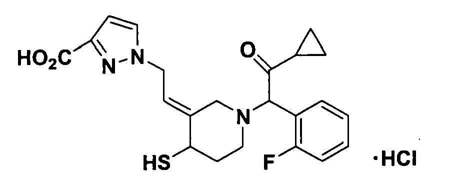

The title compound was synthesized in a yield of 34% as a colorless amorphous solid using (E)-1-[2-cyclopropyl-1-(2-fluorophenyl)-2-oxoethyl]-3-[2-(3-ethoxycarbonyl-1H-pyrazol-1-yl)ethylidene]-4-sulfanylpiperidine hydrochloride obtained in Example 76 by conducting a reaction similar to that mentioned in Example 15.

¹H NMR (400 MHz, pyridine-d₅) δ ppm : 0.81-0.95 (2H, m), 1.02-1.06 (1H, m), 1.16-1.21 (1H, m), 1.74-1.81 (1H, m), 2.14-2.22 (1H, m), 2.59-2.67 (2H, m), 2.85 and 2.95 (total 1H, each m), 3.50 and 3.61 (total 1H, each d, J=12.5), 3.77-3.84 (2H, m), 4.96 (1H, s), 5.59-5.63 (2H, m), 6.17-6.21 (1H, m), 7.19-7.34 (4H, m), 7.73 (1H, s), 7.75-7.81 (1H, m).

MS (FAB) m/z : 430 (M+H)⁺.

### (Example 78) (E)-4-(Acetylsulfanyl)-1-[2-cyclopropyl-1-(2-fluorophenyl)-2-oxoethyl]-3-{2-[(2S,4R)-4-hydroxy-2-(methoxycarbonyl)pyrrolidin-1-yl]ethylidene}piperidine dihydrochloride (Hydrochloride of the compound of Compound No. 5-388)

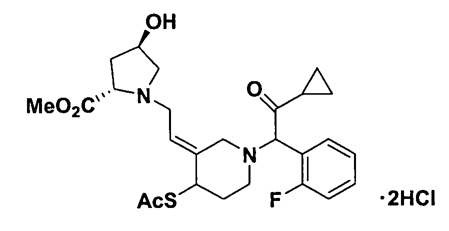

The title compound was synthesized in a yield of 57% as a yellow amorphous solid using (E)-4-(acetylsulfanyl)-1-[2-cyclopropyl-1-(2-fluorophenyl)-2-oxoethyl]-3-(2-hydroxyethylidene)piperidine obtained in Example 32 (f) and (2S,4R)-4-hydroxy-2-(methoxycarbonyl)pyrrolidine instead of 4-(ethoxycarbonylmethyl)piperidine by conducting a reaction similar to that mentioned in Example 33.

¹H NMR (400 MHz, pyridine-d₅) δ ppm : 0.79-0.88 (2H, m), 1.00-1.07 (1H, m), 1.10-1.21 (1H, m), 1.82-1.93 (1H, m), 2.17-2.27 (1H, m), 2.25 and 2.26 (total 3H, each s), 2.31-2.60 (4H, m), 2.67-2.92 (3H, m), 3.14-3.25 (1H, m), 3.36-3.53 (2H, m), 3.58-3.75 (1H, m), 3.69 and 3.71 (total 3H, each s), 3.80-3.90 (1H, m), 4.53-4.58 (1H, m), 4.71-4.77 (1H, m), 4.92 and 4.95 (total 1H, each s), 6.01-6.08 (1H, m), 7.20-7.34 (3H, m), 7.67-7.78 (1H, m).

IR (KBr, cm⁻¹) : 1747, 1702, 1494.

### (Example 79) (E)-1-[2-Cyclopropyl-1-(2-fluorophenyl)-2-oxoethyl]-3-{2-[(2S,4R)-4-hydroxy-2-(methoxycarbonyl)pyrrolidin-1-yl]ethylidene}-4-sulfanylpiperidine dihydrochloride (Hydrochloride of the compound of Compound No. 5-387)

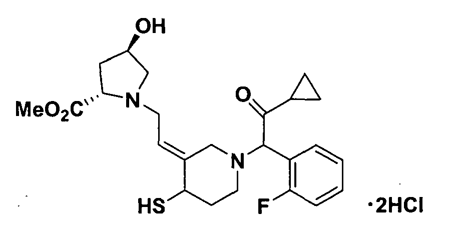

The title compound was synthesized in a yield of 58% as a pale yellow amorphous solid using (E)-4-(acetylsulfanyl)-1-[2-cyclopropyl-1-(2-fluorophenyl)-2-oxoethyl]-3-{2-[(2S,4R)-4-hydroxy-2-(methoxycarbonyl)pyrrolidin-1-yl]ethylidene}piperidine dihydrochloride obtained in Example 78 by conducting a reaction similar to that mentioned in Example 55.

¹H NMR (400 MHz, pyridine-d₅) δ ppm : 0.77-0.97 (2H, m), 0.98-1.08 (1H, m), 1.09-1.21 (1H, m), 1.74-1.85 (1H, m), 2.13-2.29 (1H, m), 2.35-2.60 (4H, m), 2.61-2.71 (1H, m), 2.81-3.04 (2H, m), 3.34-3.61 (3H, m), 3.65-3.80 (1H, m), 3.72 and 3.73 and 3.74 (total 3H, each s), 3.82-4.06 (2H, m), 4.72-4.80 (1H, m), 4.94 and 4.95 and 4.96 (total 1H, each s), 5.95-6.06 (1H, m), 7.19-7.37 (3H, m), 7.71-7.81 (1H, m).

IR (KBr, cm⁻¹) : 2545, 1746, 1712, 1494.

### (Example 80) (E)-3-{2-[(2S,4R)-2-Carboxy-4-hydroxypyrrolidin-1-yl]ethylidene}-1-[2-cyclopropyl-1-(2-fluorophenyl)-2-oxoethyl]-4-sulfanylpiperidine dihydrochloride (Hydrochloride of the compound of Compound No. 5-385)

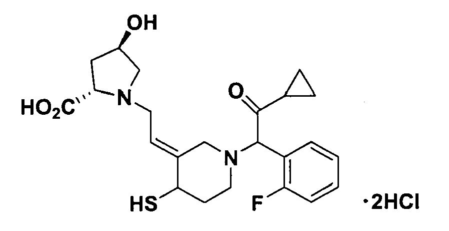

The title compound was synthesized in a yield of 75% as a colorless amorphous solid using (E)-1-[2-cyclopropyl-1-(2-fluorophenyl)-2-oxoethyl]-3-{2-[(2S,4R)-4-hydroxy-2-(methoxycarbonyl)pyrrolidin-1-yl]ethylidene}-4-sulfanylpiperidine dihydrochloride obtained in Example 79 by conducting a reaction similar to that mentioned in Example 15.

¹H NMR (500 MHz, pyridine-d₅) δ ppm : 0.76-0.95 (2H, m), 0.99-1.06 (1H, m), 1.11-1.20 (1H, m), 1.73-1.84 (1H, m), 2.15-2.26 (1H, m), 2.45-2.55 (1H, m), 2.56-2.86 (3H, m), 2.93-3.03 (1H, m), 3.34-4.03 (4H, m), 4.05-4.35 (3H, m), 4.53-4.66 (1H, m), 4.84-4.90 (1H, m), 4.97 and 4.99 and 5.00 and 5.02 (total 1H, each s), 6.18-6.29 (1H, m), 7.16-7.34 (3H, m), 7.70-7.80 (1H, m).

IR (KBr, cm⁻¹) : 2559, 1738, 1711, 1494.

### (Example 81) (E)-4-(Acetylsulfanyl)-1-[2-cyclopropyl-1-(2-fluorophenyl)-2-oxoethyl]-3-{2-[N-(ethoxycarbonylmethyl)-N-methylamino]ethylidene}piperidine dihydrochloride (Hydrochloride of the compound of Compound No. 3-55)

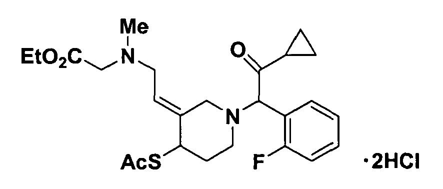

The title compound was synthesized in a yield of 56% as a pale yellow amorphous solid using (E)-4-(acetylsulfanyl)-1-[2-cyclopropyl-1-(2-fluorophenyl)-2-oxoethyl]-3-(2-hydroxyethylidene)piperidine obtained in Example 32 (f) and N-(ethoxycarbonylmethyl)-N-methylamine hydrochloride instead of 4-(ethoxycarbonylmethyl)piperidine by conducting a reaction similar to that mentioned in Example 33.

¹H NMR (500 MHz, pyridine-d₅) δ ppm : 0.76-0.92 (2H, m), 0.99-1.06 (1H, m), 1.11-1.22 (1H, m), 1.13 and 1.14 (total 3H, each t, J=7.0), 1.81-1.93 (1H, m), 2.16-2.30 (1H, m), 2.25 and 2.26 (total 3H, each s), 2.39-2.51 (1H, m), 2.45 and 2.47 (total 3H, each s), 2.52-2.60 and 2.67-2.75 (total 1H, each m), 2.77-2.90 (1H, m), 3.13 and 3.22 (total 1H, each d, J=13.0), 3.27-3.51 (4H, m), 3.56-3.72 (1H, m), 4.13 (2H, q, J=7.0), 4.52-4.58 (1H, m), 4.94 and 4.96 (total 1H, each s), 5.90-6.00 (1H, m), 7.20-7.30 (2H, m), 7.31-7.39 (1H, m), 7.66-7.74 (1H, m).

IR (KBr, cm⁻¹) : 1745, 1700, 1494.

### (Example 82) (E)-1-[2-Cyclopropyl-1-(2-fluorophenyl)-2-oxoethyl]-3-{2-[N-(ethoxycarbonylmethyl)-N-methylamino]ethylidene}-4-sulfanylpiperidine dihydrochloride (Hydrochloride of the compound of Compound No. 3-54)

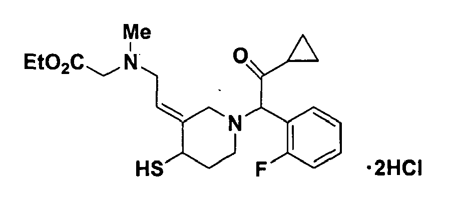

The title compound was synthesized in a yield of 63% as a pale yellow amorphous solid using (E)-4-(acetylsulfanyl)-1-[2-cyclopropyl-1-(2-fluorophenyl)-2-oxoethyl]-3-{2-[N-(ethoxycarbonylmethyl)-N-methylamino]ethylidene}piperidine dihydrochloride obtained in Example 81 by conducting a reaction similar to that mentioned in Example 34.

¹H NMR (500 MHz, pyridine-d₅) δ ppm : 0.79-0.88 (2H, m), 1.01-1.07 (1H, m), 1.11-1.20 (1H, m), 1.14 and 1.15 (total 3H, each t, J=7.0), 1.74-1.83 (1H, m), 2.16-2.27 (1H, m), 2.43-2.54 (1H, m), 2.47 and 2.49 (total 3H, each s), 2.63-2.72 (1H, m), 2.81-2.90 and 2.93-3.00 (total 1H, each m), 3.30-3.61 (6H, m), 3.84-3.89 (1H, m), 4.14 and 4.15 (total 2H, each q, J=7.0), 4.94 and 4.95 (total 1H, each s), 5.86-5.91 (1H, m), 7.21-7.28 (2H, m), 7.31-7.37 (1H, m), 7.70-7.75 (1H, m).

IR (KBr, cm⁻¹) 2536, 1745, 1713, 1494.

### (Example 83) (E)-3-{2-[N-(Carboxymethyl)-N-methylamino]ethylidene}-1-[2-cyclopropyl-1-(2-fluorophenyl)-2-oxoethyl]-4-sulfanylpiperidine dihydrochloride (Hydrochloride of the compound of Compound No. 3-50)

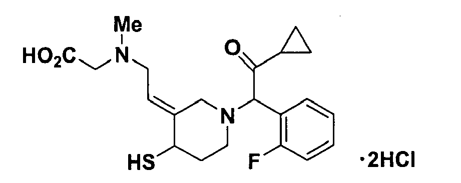

The title compound was synthesized in a yield of 17% as a colorless amorphous solid using (E)-1-[2-cyclopropyl-1-(2-fluorophenyl)-2-oxoethyl]-3-{2-[N-(ethoxycarbonylmethyl)-N-methylamino]ethylidene}-4-sulfanylpiperidine dihydrochloride obtained in Example 82 by conducting a reaction similar to that mentioned in Example 15.

¹H NMR (500 MHz, pyridine-d₅) δ ppm : 0.79-0.92 (2H, m), 1.00-1.08 (1H, m), 1.12-1.21 (1H, m), 1.74-1.86 (1H, m), 2.15-2.28 (1H, m), 2.44-2.60 (1H, m), 2.63-2.75 (1H, m), 2.71 and 2.73 (total 3H, each s), 2.80-2.89 and 2.93-3.02 (total 1H, each m), 3.38-3.69 (4H, m), 3.72-3.93 (3H, m), 4.94 and 4.96 (total 1H, each s), 5.99-6.06 (1H, m), 7.17-7.35 (3H, m), 7.69-7.76 (1H, m).

IR (KBr, cm⁻¹) : 2630, 1741, 1712, 1494.

### (Example 84) (Z)-4-(Acetylsulfanyl)-1-[2-cyclopropyl-1-(2-fluorophenyl)-2-oxoethyl]-3-{2-[N-(ethoxycarbonylmethyl)-N-methylamino]ethylidene}piperidine dihydrochloride (Hydrochloride of the compound of Compound No. 4-55)

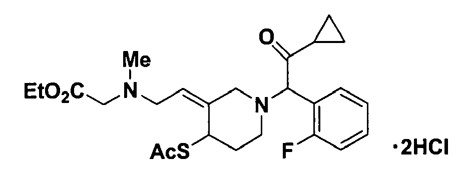

### (a) (Z)-4-(Acetylsulfanyl)-3-{2-[N-(ethoxycarbonylmethyl)-N-methylamino] ethylidene}-1-(triphenylmethyl)piperidine

The title compound was synthesized in a yield of 38% as a colorless amorphous solid using (Z)-4-(acetylsulfanyl)-3-(2-hydroxyethylidene)-1-(triphenylmethyl)piperidine obtained in Example 66 (d) instead of (E)-4-(acetylsulfanyl)-1-[2-cyclopropyl-1-(2-fluorophenyl)-2-oxoethyl]-3-(2-hydroxyethylidene)piperidine and N-(ethoxycarbonylmethyl)-N-methylamine hydrochloride instead of 4-(ethoxycarbonylmethyl)piperidine by conducting a reaction similar to that mentioned in Example 33.

¹H NMR (400 MHz, CDCl₃) δ ppm : 1.29 (3H, t, J=7.0), 1.73-1.85 (1H, m), 1.94-2.05 (1H, m), 2.15-2.52 (4H, m), 2.20 (3H, s), 2.41 (3H, s), 3.00-3.47 (4H, m), 4.21 (2H, q, J=7.0), 4.82 (1H, bs), 5.39 (1H, t, J=6.5), 7.03-7.75 (15H, m).

### (b) (Z)-4-(Acetylsulfanyl)-3-{2-[N-(ethoxycarbonylmethyl)-N-methylamino]ethylidene}piperidine bis(hydrogen trifluoroacetate)

The title compound was synthesized in a yield of 90% as a colorless amorphous solid using (Z)-4-(acetylsulfanyl)-3-{2-[N-(ethoxycarbonylmethyl)-N-methylamino]ethylidene}-1-(triphenylmethyl)piperidine obtained in (a) above by conducting a reaction similar to that mentioned in Example 3 (c).

¹H NMR (400 MHz, CDCl₃) δ ppm : 1.30 (3H, t, J=7.0), 1.99-2.10 (1H, m), 2.35 (3H, s), 2.35-2.50 (1H, m), 2.88 (3H, s), 3.07-3.20 (1H, m), 3.36-3.49 (1H, m), 3.65-3.98 (5H, m), 4.25 (2H, q, J=7.0), 4.28-4.38 (1H, m), 4.78 (1H, bs), 5.78-5.86 (1H, m).

### (c) (Z)-4-(Acetylsulfanyl)-1-[2-cyclopropyl-1-(2-fluorophenyl)-2-oxoethyl]-3-{2-[N-(ethoxycarbonylmethyl)-N-methylamino]ethylidene}piperidine dihydrochloride

The title compound was synthesized in a yield of 50% as a colorless amorphous solid using (Z)-4-(acetylsulfanyl)-3-{2-[N-(ethoxycarbonylmethyl)-N-methylamino]ethylidene}piperidine bis(hydrogen trifluoroacetate) obtained in (b) above by conducting a reaction similar to that mentioned in Example 1 (g).

¹H NMR (400 MHz, CDCl₃) δ ppm : 0.75-0.92 (2H, m), 0.93-1.09 (2H, m), 1.23-1.35 (3H, m), 1.70-1.87 (1H, m), 2.06-2.56 (2H, m), 2.29 (3H, s), 2.35 and 2.36 (total 3H, each s), 2.65-3.00 (2H, m), 3.08-3.36 (6H, m), 4.14-4.25 (2H, m), 4.62 and 4.67 (total 1H, each s), 4.79 (1H, bs), 5.35 and 5.45 (total 1H. each t, J=6.5), 7.04-7.22 (2H, m), 7.26-7.48 (2H, m).

IR (KBr, cm⁻¹) : 1745, 1699, 1494.

### (Example 85) (Z)-1-[2-Cyclopropyl-1-(2-fluorophenyl)-2-oxoethyl]-3-{2-[N-(ethoxycarbonylmethyl)-N-methylamino]ethylidene}-4-sulfanylpiperidine dihydrochloride (Hydrochloride of the compound of Compound No. 4-54)

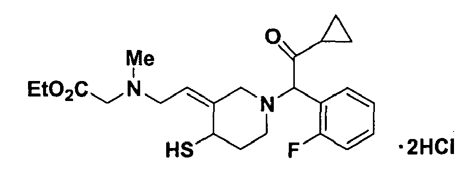

The title compound was synthesized in a yield of 90% as a colorless amorphous solid using (Z)-4-(acetylsulfanyl)-1-[2-cyclopropyl-1-(2-fluorophenyl)-2-oxoethyl]-3- {2-[N-(ethoxycarbonylmethyl)-N-methylamino]ethylidene}piperidine dihydrochloride obtained in Example 84 by conducting a reaction similar to that mentioned in Example 34.

¹H NMR (400 MHz, CDCl₃) δ ppm : 0.75-0.91 (2H, m), 0.94-1.09 (2H, m), 1.22-1.33 (3H, m), 1.65-1.79 (1H, m), 2.07-2.28 (2H, m), 2.37 and 2.39 (total 3H, each s), 2.44-3.35 (8H, m), 4.12-4.24 (2H, m), 4.30 (1H, bs), 4.68 and 4.73 (total 1H, each s), 5.23 and 5.33 (total 1H, each t, J=7.0), 7.05-7.21 (2H, m), 7.26-7.48 (2H, m).

IR (KBr, cm⁻¹) : 2626, 2560, 1745, 1712, 1494.

### (Example 86) (Z)-3-{2-[N-(Carboxymethyl)-N-methylamino]ethylidene}-1-[2-cyclopropyl-1-(2-fluorophenyl)-2-oxoethyl]-4-sulfanylpiperidine dihydrochloride (Hydrochloride of the compound of Compound No. 4-50)

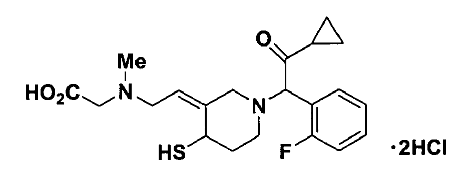

The title compound was synthesized in a yield of 50% as a colorless amorphous solid using (Z)-1-[2-cyclopropyl-1-(2-fluorophenyl)-2-oxoethyl]-3-{2-[N-(ethoxycarbonylmethyl)-N-methylamino]ethylidene}-4-sulfanylpiperidine dihydrochloride obtained in Example 85 by conducting a reaction similar to that mentioned in Example 15.

¹H NMR (400 MHz, pyridine-d₅) δ ppm : 0.71-0.94 (2H, m), 0.96-1.17 (2H, m), 1.71-1.87 (1H, m), 2.21-2.42 (1H, m), 2.43-2.56 (1H, m), 2.60-3.07 (2H, m), 2.98 and 3.02 (total 3H, each s), 3.25-3.62 (2H, m), 4.00-4.26 (4H, m), 4.71 (1H, bs), 4.90 and 4.96 (total 1H, each s), 5.76 and 5.87 (total 1H, each t, J=7.0), 7.10-7.36 (3H, m), 7.62-7.72 (1H, m).

IR (KBr, cm⁻¹) : 2558, 1740, 1712, 1494.

### (Example 87) (4S)-(Z)-4-(Acetylsulfanyl)-1-[2-cyclopropyl-1-(2-fluorophenyl)-2-oxoethyl]-3-(2-hydroxyethylidene)piperidine hydrochloride (Hydrochloride of the (4S) form of the compound of Compound No. 4-2)

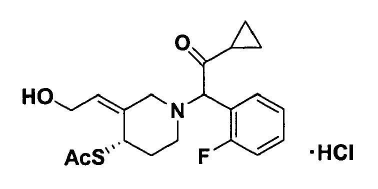

### (a) (4S)-(Z)-4-(Acetylsulfanyl)-3-(2-hydroxyethylidene)-1-(triphenylmethyl)piperidine and (4R)-(Z)-4-(acetylsulfanyl)-3-(2-hydroxyethylidene)-1-(triphenylmethyl)piperidine

(Z)-4-(Acetylsulfanyl)-3-(2-hydroxyethylidene)-1-(triphenylmethyl)piperidine (102 mg) obtained in Example 66 (d) was separated using a preparative HPLC (Daicel, Chiralcel OD-H, eluent: hexane / ethanol / diethylamine = 90 /10 / 0.1) to afford (4S)-(Z)-4-(acetylsulfanyl)-3-(2-hydroxyethylidene)-1-(triphenylmethyl)piperidine (41 mg) having a shorter retention time and (4R)-(Z)-4-(acetylsulfanyl)-3-(2-hydroxyethylidene)-1-(triphenylmethyl)piperidine (43 mg) having a longer retention time as colorless amorphous solids, respectively.

The ¹H NMR was the same as that of (Z)-4-(acetylsulfanyl)-3-(2-hydroxyethylidene)-1-(triphenylmethyl)piperidine of Example 66 (d).

[α]D of (4S) form: -15.2° (c=1.00, MeOH)

[α]D of (4R) form: +11.5° (c=0.50, MeOH)

### (b) (4S)-(Z)-4-(Acetylsulfanyl)-1-[2-cyclopropyl-1-(2-fluorophenyl)-2-oxoethyl]-3-(2-hydroxyethylidene)piperidine hydrochloride

The title compound was synthesized in a yield of 67% as a colorless amorphous solid using (4S)-(Z)-4-(acetylsulfanyl)-3-(2-hydroxyethylidene)-1-(triphenyhnethyl)piperidine obtained in (a) above by conducting a reaction similar to that mentioned in Example 66 (e).

The ¹H NMR was the same as in Example 66 (e).

IR(KBr, cm⁻¹): 3345, 1696, 1494.

### (Example 88) (4R)-(Z)-4-(Acetylsulfanyl)-1-[2-cyclopropyl-1-(2-fluorophenyl)-2-oxoethyl]-3-(2-hydroxyethylidene)piperidine hydrochloride (Hydrochloride of the (4R) form of the compound of Compound No. 4-2)

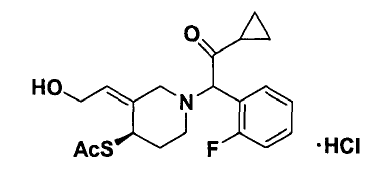

The title compound was synthesized in a yield of 70% as a colorless amorphous solid using (4R)-(Z)-4-(acetylsulfanyl)-3-(2-hydroxyethylidene)-1-(triphenylmethyl)piperidine obtained in Example 87 (a) by conducting a reaction similar to that mentioned in Example 66 (e).

The ¹H NMR was the same as in Example 66 (e).

IR(KBr, cm⁻¹): 3350, 1696, 1494.

### (Example 89) (Z)-4-(Acetylsulfanyl)-1-[2-cyclopropyl-1-(2-fluorophenyl)-2-oxoethyl]-3-(2- {N-[3-(ethoxycarbonyl)propyl]-N-methylamino}ethylidene)piperidine dihydrochloride (Hydrochloride of the compound of Compound No. 4-67)

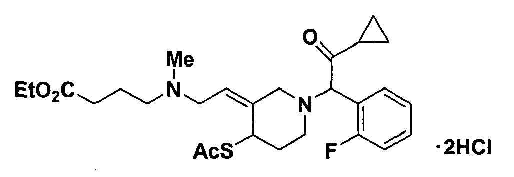

The title compound was synthesized in a yield of 43% as a pale orange amorphous solid using (Z)-4-(acetylsulfanyl)-1-[2-cyclopropyl-1-(2-fluorophenyl)-2-oxoethyl]-3-(2-hydroxyethylidene)piperidine obtained in Example 66 (e) instead of (E)-4-(acetylsulfanyl)-1-[2-cyclopropyl-1-(2-fluorophenyl)-2-oxoethyl]-3-(2-hydroxyethylidene)piperidine and N-[3-(ethoxycarbonyl)propyl]-N-methylamine hydrochloride instead of 4-(ethoxycarbonylmethyl)piperidine by conducting a reaction similar to that mentioned in Example 33.

¹H NMR (500 MHz, pyridine-d₅) δ ppm : 0.76-0.85 (1H, m), 0.99-1.08 (2H, m), 1.09-1.18 (1H, m), 1.11 and 1.12 (total 3H, each t, J=7.0), 1.81-1.89 (1H, m), 2.23-2.42 (3H, m), 2.25 and 2.26 (total 3H, each s), 2.47-2.61 (1H, m), 2.51 (2H, t, J=7.0), 2.77 and 2.78 (total 3H, each s), 2.96-3.13 (2H, m), 3.15-3.26 (2H, m), 3.35 and 3.55 (total 1H, each d, J=12.0), 3.60-3.67 (1H, m), 3.98-4.12 (1H, m), 4.08 and 4.09 (total 2H, each q, J=7.0), 4.13-4.23 (1H, m), 4.86 and 4.96 (total 1H, each s), 4.89-4.94 (1H, m), 6.10 and 6.21 (total 1H, each t, J=7.0), 7.19-7.38 (3H, m), 7.57-7.66 (1H, m).

IR (KBr, cm⁻¹) : 1725, 1712, 1495.

### (Example 90) (Z)-1-[2-Cyclopropyl-1-(2-fluorophenyl)-2-oxoethyl]-3-(2-{N-[3-(ethoxycarbonyl)propyl)]-N-methylamino}ethylidene)-4-sulfanylpiperidine dihydrochloride (Hydrochloride of the compound of Compound No. 4-66)

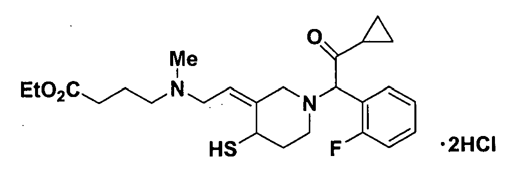

The title compound was synthesized in a yield of 76% as a yellow amorphous solid using (Z)-4-(acetylsulfanyl)-1-[2-cyclopropyl-1-(2-fluorophenyl)-2-oxoethyl]-3-(2- {N-[3-(ethoxycarbonyl)propyl]-N-methylamino}ethylidene)piperidine dihydrochloride obtained in Example 89 by conducting a reaction similar to that mentioned in Example 34.

¹H NMR (500 MHz, pyridine-d₅) δ ppm : 0.74-0.85 (1H, m), 0.87-0.96 (1H, m), 0.99-1.06 (1H, m), 1.07-1.15 (1H, m), 1.10 and 1.11 (total 3H, each t, J=7.0), 1.74-1.85 (1H, m), 2.25-2.40 (3H, m), 2.43-2.54 (3H, m), 2.65-2.72 (1H, m), 2.81 and 2.83 (total 3H, each s), 2.93-2.98 (1H, m), 3.19-3.64 (4H, m), 3.89-4.00 (2H, m), 4.07 and 4.09 (total 2H, each q, J=7.0), 4.54-4.59 (1H, m), 4.89 and 4.95 (total 1H, each s), 5.85 and 5.96 (total 1H, each t, J=7.0), 7.17-7.25 (2H, m), 7.26-7.34 (1H, m), 7.57-7.68 (1H, m).

IR (KBr, cm⁻¹) : 2550, 1728, 1495.

### (Example 91) (Z)-3-(2-{N-[3-(Carboxy)propyl]-N-methylamino}ethylidene)-1-[2-cyclopropyl-1-(2-fluorophenyl)-2-oxoethyl]-4-sulfanylpiperidine dihydrochloride (Hydrochloride of the compound of Compound No. 4-62)

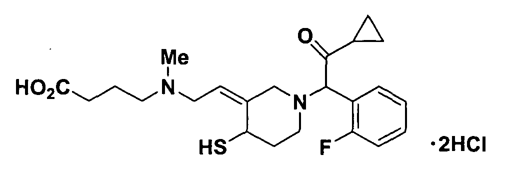

The title compound was synthesized in a yield of 50% as a colorless amorphous solid using (Z)-1-[2-cyclopropyl-1-(2-fluorophenyl)-2-oxoethyl]-3-(2-{N-[3-(ethoxycarbonyl)propyl]-N-methylamino}ethylidene)-4-sulfanylpiperidine dihydrochloride obtained in Example 90 by conducting a reaction similar to that mentioned in Example 15.

¹H NMR (500 MHz, pyridine-d₅) δ ppm : 0.73-0.83 (1H, m), 0.86-0.97 (1H, m), 0.98-1.06 (1H, m), 1.07-1.17 (1H, m), 1.71-1.85 (1H, m), 2.25-2.54 (4H, m), 2.58-2.89 (3H, m), 2.81 and 2.83 (total 3H, each s), 2.90-3.01 (1H, m), 3.21-3.37 (2H, m), 3.39-3.48 (1H, m), 3.50-3.56 (1H, m), 3.86-4.00 (2H, m), 4.53-4.60 (1H, m), 4.89 and 4.97 (total 1H, each s), 5.86 and 5.97 (total 1H, each t, J=7.0), 7.16-7.24 (2H, m), 7.25-7.34 (1H, m), 7.62-7.68 (1H, m).

IR (KBr, cm⁻¹) : 2553, 1713, 1494.

### (Example 92) (Z)-4-(Acetylsulfanyl)-1-[2-cyclopropyl-1-(2-fluorophenyl)-2-oxoethyl]-3-{2-[N-(ethoxycarbonylmethyl)-N-isopropylamino]ethylidene}piperidine dihydrochloride (Hydrochloride of the compound of Compound No. 4-79)

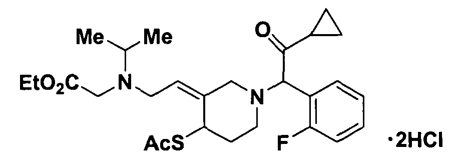

The title compound was synthesized in a yield of 33% as a brown amorphous solid using (Z)-4-(acetylsulfanyl)-1-[2-cyclopropyl-1-(2-fluorophenyl)-2-oxoethyl]-3-(2-hydroxyethylidene)piperidine obtained in Example 66 (e) instead of (E)-4-(acetylsulfanyl)-1-[2-cyclopropyl-1-(2-fluorophenyl)-2-oxoethyl]-3-(2-hydroxyethylidene)piperidine and N-(ethoxycarbonylmethyl)-N-isopropylamine instead of 4-(ethoxycarbonylmethyl)piperidine by conducting a reaction similar to that mentioned in Example 33.

¹H NMR (400 MHz, pyridine-d₅) δ ppm : 0.74-0.86 (2H, m), 0.98-1.13 (2H, m), 1.04 and 1.06 (total 3H, each d, J=6.5), 1.07 and 1.09 (total 3H, each d ,J=6.5), 1.17 and 1.19 (total 3H, each t, J=7.0), 1.85-1.96 (1H, m), 2.28 (3H, s), 2.30-2.41 (1H, m), 2.42-2.52 (1H, m), 2.59-2.68 (1H, m), 2.90-3.01 (1H, m), 3.03-3.12 (1H, m), 3.13-3.22 (1H, m), 3.36-3.70 (5H, m), 4.18 and 4.20 (total 2H, each q, J=7.0), 4.87 and 4.92 (total 1H, each s), 5.14 (1H, bs), 5.63 and 5.72 (total 1H, each t, J=7.0), 7.19-7.27 (2H, m), 7.29-7.36 (1H, m), 7.64-7.70 (1H, m).

IR (KBr, cm⁻¹) : 1745, 1696, 1495.

### (Example 93) (Z)-1-[2-Cyclopropyl-1-(2-fluorophenyl)-2-oxoethyl]-3-{2-[N-(ethoxycarbonylmethyl)-N-isopropylamino]ethylidene}-4-sulfanylpiperidine dihydrochloride (Hydrochloride of the compound of Compound No. 4-78)

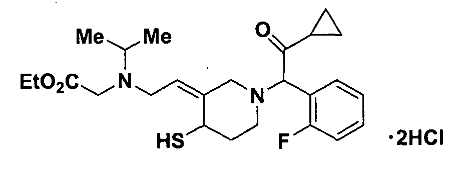

The title compound was synthesized in a yield of 41 % as a colorless amorphous solid using (Z)-4-(acetylsulfanyl)-1-[2-cyclopropyl-1-(2-fluorophenyl)-2-oxoethyl]-3- {2-[N-(ethoxycarbonylmethyl)-N-isopropylamino] ethylidene} piperidine dihydrochloride obtained in Example 92 by conducting a reaction similar to that mentioned in Example 34.

¹H NMR (400 MHz, pyridine-d₅) δ ppm : 0.75-0.83 (2H, m), 0.97-1.13 (2H, m), 1.03 (3H, d, J=6.5), 1.06 (3H, d, J=6.5), 1.16 and 1.18 (total 3H, each t, J=7.0), 1.73-1.85 (1H, m), 2.22-2.37 (1H, m), 2.45-2.54 (1H, m), 2.66-2.75 (1H, m), 2.81-3.27 (3H, m), 3.36-3.55 (5H, m), 4.17 and 4.18 (total 2H, each q, J=7.0), 4.61 (1H, bs), 4.89 and 4.93 (total 1H, each s), 5.44 and 5.55 (total 1H, each t, J=7.0), 7.18-7.24 (2H, m), 7.27-7.34 (1H, m), 7.67-7.74 (1H, m).

IR (KBr, cm⁻¹) : 2545, 1745, 1712, 1494.

### (Example 94) (Z)-3-{2-[N-(Carboxymethyl)-N-isopropylamino]ethylidene}-1-[2-cyclopropyl-1-(2-fluorophenyl)-2-oxoethyl]-4-sulfanylpiperidine dihydrochloride (Hydrochloride of the compound of Compound No. 4-74)

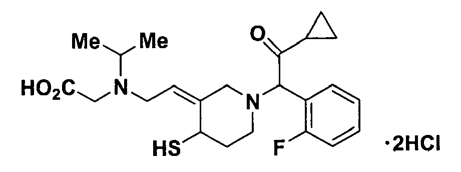

The title compound was synthesized in a yield of 40% as a colorless amorphous solid using (Z)-1-[2-cyclopropyl-1-(2-fluorophenyl)-2-oxoethyl]-3-{2-[N-(ethoxycarbonylmethyl)-N-isopropylamino] ethylidene} -4-sulfanylpiperidine dihydrochloride obtained in Example 93 by conducting a reaction similar to that mentioned in Example 15.

¹H NMR (400 MHz, pyridine-d₅) δ ppm : 0.70-0.87 (2H, m), 0.94-1.04 (1H, m), 1.05-1.19 (1H, m), 1.13 and 1.14 (total 3H, each d, J=6.5), 1.15 and 1.16 (total 3H, each d, J=6.5), 1.71-1.84 (1H, m), 2.22-2.41 (1H, m), 2.48-2.57 (1H, m), 2.64-3.02 (2H, m), 3.21-3.44 (2H, m), 3.49-3.69 (5H, m), 4.67 (1H, bs), 4.87 and 4.92 (total 1H, each s), 5.55 and 5.67 (total 1H, each t, J=7.0), 7.16-7.23 (2H, m), 7.25-7.32 (1H, m), 7.66-7.73 (1H, m).

IR (KBr, cm⁻¹): 2554, 1740, 1712, 1494.

### (Example 95) (Z)-4-(Acetylsulfanyl)-1-[2-cyclopropyl-1-(2-fluorophenyl)-2-oxoethyl]-3- {2-[N-(ethoxycarbonylmethyl)-N-ethylamino] ethylidene}piperidine dihydrochloride (Hydrochloride of the compound of Compound No. 4-73)

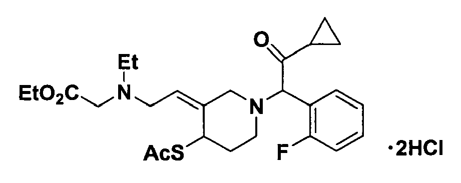

The title compound was synthesized in a yield of 38% as a brown amorphous solid using (Z)-4-(acetylsulfanyl)-1-[2-cyclopropyl-1-(2-fluorophenyl)-2-oxoethyl]-3-(2-hydroxyethylidene)piperidine obtained in Example 66 (e) instead of (E)-4-(acetylsulfanyl)-1-[2-cyclopropyl-1-(2-fluorophenyl)-2-oxoethyl]-3-(2-hydroxyethylidene)piperidine and N-(ethoxycarbonylmethyl)-N-ethylamine instead of 4-(ethoxycarbonylmethyl)piperidine by conducting a reaction similar to that mentioned in Example 33.

¹H NMR (500 MHz, pyridine-d₅) δ ppm : 0.74-0.88 (2H, m), 0.99-1.05 (1H, m), 1.07-1.15 (1H, m), 1.10 and 1.12 (total 3H, each t, J=7.5), 1.17 and 1.18 (total 3H, each t, J=7.0), 1.84-1.94 (1H, m), 2.27 (3H, s), 2.30-2.68 (3H, m), 2.83 and 2.86 (total 2H, each d, J=7.5), 2.92-3.01 (1H, m), 3.05-3.12 (1H, m), 3.40 and 3.55 (total 1H, each d, J =13.0), 3.52-3.67 (3H, m), 3.72-3.81 (1H, m), 4.15-4.22 (2H, m), 4.89 and 4.94 (total 1H, each s), 5.12 (1H, bs), 5.63 and 5.73 (total 1H, each t, J=7.0), 7.20-7.26 (2H, m), 7.30-7.36 (1H, m), 7.64-7.69 (1H, m).

IR (KBr, cm⁻¹) : 1745, 1697, 1494.

### (Example 96) (Z)-1-[2-Cyclopropyl-1-(2-fluorophenyl)-2-oxoethyl]-3-{2-[N-(ethoxycarbonylmethyl)-N-ethylamino]ethylidene}-4-sulfanylpiperidine dihydrochloride (Hydrochloride of the compound of Compound No. 4-72)

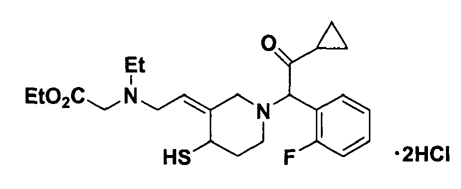

The title compound was synthesized in a yield of 64% as a colorless amorphous solid using (Z)-4-(acetylsulfanyl)-1-[2-cyclopropyl-1-(2-fluorophenyl)-2-oxoethyl]-3-{2-[N-(ethoxycarbonylmethyl)-N-ethylamino]ethylidene}piperidine dihydrochloride obtained in Example 95 by conducting a reaction similar to that mentioned in Example 34.

¹H NMR (400 MHz, pyridine-d₅) δ ppm : 0.71-0.86 (2H, m), 0.96-1.04 (1H, m), 1.06-1.20 (1H, m), 1.09 (3H, t, J=7.0), 1.15 and 1.16 (total 3H, each t, J=7.0), 1.70-1.85 (1H, m), 2.17-2.36 (1H, m), 2.44-2.54 (1H, m), 2.64-3.04 (4H, m), 3.25 and 3.40 (total 1H, each d, J=12.5), 3.37-3.62 (5H, m), 4.16 and 4.17 (total 2H, each q, J=7.0), 4.57 (1H, bs), 4.90 and 4.94 (total 1H, each s), 5.46 and 5.57 (total 1H, each t, J=7.0), 7.16-7.26 (2H, m), 7.27-7.36 (1H, m), 7.64-7.76 (1H, m).

IR (KBr, cm⁻¹) : 2558, 1744, 1712, 1494.

### (Example 97) (Z)-3-{2-[N-(Carboxymethyl)-N-ethylamino]ethylidene}-1-[2-cyclopropyl-1-(2-fluorophenyl)-2-oxoethyl]-4-sulfanylpiperidine dihydrochloride (Hydrochloride of the compound of Compound No. 4-68)

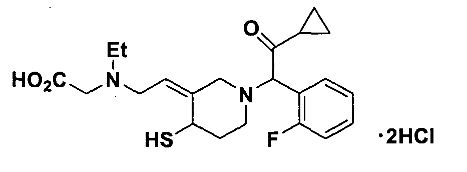

The title compound was synthesized in a yield of 53% as a colorless amorphous solid using (Z)-1-[2-cyclopropyl-1-(2-fluorophenyl)-2-oxoethyl]-3-{2-[N-(ethoxycarbonylmethyl)-N-ethylamino]ethylidene}-4-sulfanylpiperidine dihydrochloride obtained in Example 96 by conducting a reaction similar to that mentioned in Example 15.

¹H NMR (400 MHz, pyridine-d₅) δ ppm : 0.72-0.92 (2H, m), 0.97-1.05 (1H, m), 1.06-1.15 (1H, m), 1.33 and 1.35 (total 3H, each t, J=7.0), 1.72-1.84 (1H, m), 2.23-2.41 (1H, m), 2.46-2.56 (1H, m), 2.62-2.98 (2H, m), 3.22-3.58 (4H, m), 3.71-4.12 (4H, m), 4.68 (1H, bs), 4.89 and 4.95 (total 1H, each s), 5.17 and 5.89 (total 1H, each t, J=7.0), 7.16-7.24 (2H, m), 7.25-7.34 (1H, m), 7.64-7.72 (1H, m).

IR (KBr, cm⁻¹): 2556, 1740, 1713, 1494.

### (Example 98) (E)-4-(Acetylsulfanyl)-3-(2-{4-[2-(ethoxycarbonyl)ethyl]-3-oxopiperazin-1-yl}ethylidene)-1-[1-(2-fluorophenyl)-2-methoxy-2-oxoethyl]piperidine dihydrochloride (Hydrochloride of the compound of Compound No. 11-108)

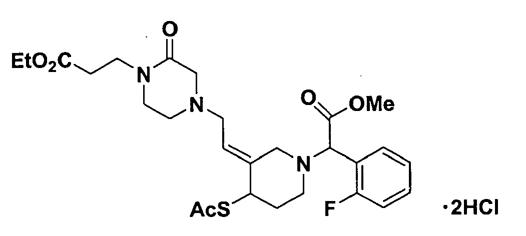

### (a) (E)-4-(Acetylsulfanyl)-3-(2-{4-[2-(ethoxycarbonyl)ethyl]-3-oxopiperazin-1-yl}ethylidene)-1-(triphenylmethyl)piperidine

The title compound was synthesized in a yield of 40% as a yellow powder crystal using (E)-3-(2-{4-[2-(ethoxycarbonyl)ethyl]-3-oxopiperazin-1-yl}ethylidene)-4-hydroxy-1-(triphenylmethyl)piperidine obtained in Example 17 (b) by conducting a reaction similar to that mentioned in Example 1 (h).

¹H NMR (400 MHz, CDCl₃) δ ppm : 1.27 (3H, t, J=7.0), 1.79-1.91 (1H, m), 2.14-2.32 (2H, m), 2.24 (3H, s), 2.51-2.66 (6H, m), 2.93-3.06 (3H, m), 3.09 (2H, s), 3.38 (2H, t, J=5.0), 3.63 (2H, t, J=7.0), 4.15 (2H, q, J=7.0), 4.25 (1H, m), 5.59 (1H, t, J=7.0), 7.13-7.22 (3H, m), 7.23-7.34 (6H, m), 7.40-7.54 (6H, m).

### (b) (E)-4-(Acetylsulfanyl)-3-(2-{4-[2-(ethoxycarbonyl)ethyl]-3-oxopiperazin-1-yl}ethylidene)piperidine bis(hydrogen trifluoroacetate)

The title compound was synthesized in a yield of 81 % as a brown oil using (E)-4-(acetylsulfanyl)-3-(2-{4-[2-(ethoxycarbonyl)ethyl]-3-oxopiperazin-1-yl}ethylidene)-1-(triphenylmethyl)piperidine obtained in (a) above by conducting a reaction similar to that mentioned in Example 3 (c).

¹H NMR (400 MHz, CDCl₃) δ ppm : 1.26 (3H, t, J=7.0), 2.00-2.09 (1H, m), 2.36 (3H, s), 2.38-2.45 (1H, m), 2.62 (2H, t, J=6.5), 2.86-2.95 (1H, m), 2.97-3.06 (1H, m), 3.17-3.26 (1H, m), 3.28-3.45 (5H, m), 3.50-3.54 (2H, m), 3.63 (2H, t, J=7.0), 3.67 (1H, d, J=13.0), 4.13 (2H, q, J=7.0), 4.16 (1H, d, J=13.0), 4.43-4.47 (1H, m), 5.59 (1H, t, J=7.0).

### (c) (E)-4-(Acetylsulfanyl)-3-(2-{4-[2-(ethoxycarbonyl)ethyl]-3-oxopiperazin-1-yl}ethylidene)-1-[1-(2-fluorophenyl)-2-methoxy-2-oxoethyl]piperidine dihydrochloride

To a solution of (E)-4-(acetylsulfanyl)-3-(2-{4-[2-(ethoxycarbonyl)ethyl]-3-oxopiperazin-1-yl}ethylidene)piperidine bis(hydrogen trifluoroacetate) (0.76 g) obtained in (b) above and methyl bromo(2-fluorophenyl)acetate (0.45 g) in acetonitrile (10 ml) was added triethylamine (0.51 ml) under ice cooling, and the resulting mixture was stirred at room temperature for 30 minutes. The reaction mixture was diluted with ethyl acetate and washed with water and a saturated aqueous sodium chloride solution, and the organic layer was dried over anhydrous magnesium sulfate. The solvent was removed in vacuo, and the residue was purified by chromatography on a silica gel column (methanol / dichloromethane = 1 / 20) to afford the free form (0.50 g, yield 74%) of the title compound as a pale yellow oil. To a solution of this compound in dichloromethane (8 ml) was added a 4N solution of hydrogen chloride in dioxane (0.69 ml) at room temperature, and the reaction mixture was evaporated in vacuo to afford the title compound (0.66 g, quantitative yield) as a pale brown amorphous solid.

¹H NMR (400 MHz, pyridine-d₅) δ ppm : 1.12 (3H, t, J=7.0), 1.80-1.89 (1H, m), 2.14-2.24 (1H, m), 2.25 and 2.27 (total 3H, each s), 2.45-2.65 (2H, m), 2.73 (2H, t, J=7.0), 2.77-3.02 (5H, m), 3.19 and 3.37 (total 1H, each d, J=12.5), 3.23 and 3.25 (total 2H, each s), 3.29-3.36 (2H, m), 3.68 (3H, s), 3.76 (2H, t, J=7.0), 4.10 (2H, q, J=7.0), 4.50-4.57 (1H, m), 4.95 and 4.97 (total 1H, each s), 5.81 (1H, t, J=7.0), 7.20-7.28 (2H, m), 7.31-7.38 (1H, m), 7.73-7.80 (1H, m).

IR (KBr, cm⁻¹) : 1752, 1730, 1697, 1664, 1497.

### (Example 99) (E)-3-(2- {4-[2-(Ethoxycarbonyl)ethyl]-3-oxopiperazin-1-yl}ethylidene)-1-[1-(2-fluorophenyl)-2-methoxy-2-oxoethyl]-4-sulfanylpiperidine dihydrochloride (Hydrochloride of the compound of Compound No. 11-107)

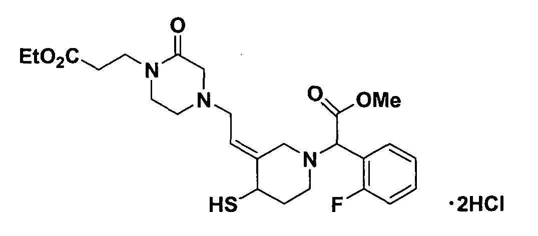

The title compound was synthesized in a yield of 65% as a colorless amorphous solid using (E)-4-(acetylsulfanyl)-3-(2-{4-[2-(ethoxycarbonyl)ethyl]-3-oxopiperazin-1-yl}ethylidene)-1-[1-(2-fluorophenyl)-2-methoxy-2-oxoethyl]piperidine dihydrochloride obtained in Example 98 by conducting a reaction similar to that mentioned in Example 34.

¹H NMR (400 MHz, pyridine-d₅) δ ppm : 1.12 (3H, t, J=7.0), 1.71-1.83 (1H, m), 2.11-2.24 (1H, m), 2.54-2.65 (2H, m), 2.67-2.82 (1H, m), 2.74 (2H, t, J=7.0), 2.96-3.09 (1H, m), 3.01 (2H, t, J=6.5), 3.26 (2H, s), 3.34 (2H, t, J=5.0), 3.37 and 3.48 (total 1H, each d, J=12.5), 3.60 and 3.73 (total 1H, each d, J=12.5), 3.67 (3H, s), 3.77 (2H, t, J=7.0), 3.80-3.89 (1H, m), 4.10 (2H, q, J=7.0), 4.94 and 4.95 (total 1H, each s), 5.69-5.78 (1H, m), 7.18-7.28 (2H, m), 7.30-7.38 (1H, m), 7.75-7.83 (1H, m).

IR (KBr, cm⁻¹) : 2522, 1752, 1730, 1661, 1496.

### (Example 100) (E)-3-(2- {4-[2-(Carboxyethyl)]-3-oxopiperazin-1-yl}ethylidene)-1-[1-(2-fluorophenyl)-2-methoxy-2-oxoethyl]-4-sulfanylpiperidine dihydrochloride (Hydrochloride of the compound of Compound No. 11-103)

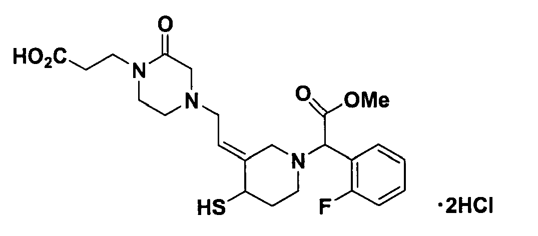

The title compound was synthesized in a quantitative yield as a colorless amorphous solid using (E)-3-(2-{4-[2-(ethoxycarbonyl)ethyl]-3-oxopiperazin-1-yl}ethylidene)-1-[1-(2-fluorophenyl)-2-methoxy-2-oxoethyl]-4-sulfanylpiperidine dihydrochloride obtained in Example 99 by conducting a reaction similar to that mentioned in Example 15.

¹H NMR (400 MHz, pyridine-d₅) δ ppm : 1.71-1.83 (1H, m), 2.12-2.24 (1H, m), 2.53-2.64 (2H, m), 2.67-2.84 (1H, m), 2.92 (2H, t, J=7.0), 2.95-3.09 (1H, m), 2.99 (2H, t, J=6.5), 3.26 and 3.27 (total 2H, each s), 3.34-3.46 (2H, m), 3.37 and 3.47 (total 1H, each d, J=12.5), 3.60 and 3.72 (total 1H, each d, J=12.5), 3.67 (3H, s), 3.80-3.88 (1H, m), 3.91 (2H, t, J=7.0), 4.94 and 4.95 (total 1H, each s), 5.69-5.78 (1H, m), 7.18-7.26 (2H, m), 7.29-7.36 (1H, m), 7.75-7.82 (1H, m).

IR (KBr, cm⁻¹) : 2570, 1750, 1659, 1496.

### (Example 101) (E)-4-(Acetylsulfanyl)-1-[2-cyclopropyl-1-(2-fluorophenyl)-2-oxoethyl]-3-{3-[1H-pyrazol-3(5)-yl]propylidene}piperidine dihydrochloride (Hydrochloride of the compound of Compound No. 1-264)

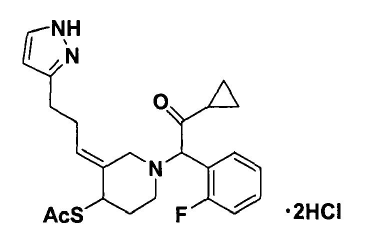

### (a) (E)-3-{3-[1-(t-Butoxycarbonyl)-1H-pyrazole-3-yl]-1-hydroxypropyl}-1-(triphenyhnethyl)piperidin-4-one

The title compound was synthesized in a yield of 45% as a colorless amorphous solid using 3-[1-(t-butoxycarbonyl)-1H-pyrazol-3-yl]propanal instead of 1-(ethoxycarbonylmethyl)piperidin-4-carbaldehyde by conducting a reaction similar to that mentioned in Example 36 (a).

¹H NMR (400 MHz, CDCl₃) δ ppm : 1.63 (9H, s), 1.66-1.80 (2H, m), 1.84-2.01 (1H, m), 2.37-2.45 (1H, m), 2.68-2.95 (4H, m), 3.11-3.32 (2H, m), 3.73-3.84 (2H, m), 6.16 (1H, d, J=2.5), 7.14-7.21 (3H, m), 7.23-7.34 (6H, m), 7.41-7.59 (6H, m), 7.94 (1H, d, J=2.5).

### (b) (E)-3-{3-[1-(t-Butoxycarbonyl)-1H-pyrazole-3-yl]propylidene}-1-(triphenylmethyl)piperidin-4-one

To a solution of (E)-3-{3-[1-(t-butoxycarbonyl)-1H-pyrazole-3-yl]-1-hydroxypropyl}-1-(triphenylmethyl)piperidin-4-one (1.92 g) obtained in (a) above in dichloromethane (45 ml) were added methanesulfonyl chloride (0.29 ml) and triethylamine (0.61 ml) under ice cooling, and the resulting mixture was stirred at the same temperature for 2.5 hours. The reaction mixture was diluted with ethyl acetate and washed with a saturated aqueous sodium chloride solution, and the organic layer was dried over anhydrous sodium sulfate. The solvent was removed in vacuo to afford a mesyl compound (2.14 g) as a colorless amorphous solid. To a solution of this compound in tetrahydrofuran (50 ml) was added 1,8-diazabicyclo[5.4.0]undeca-7-ene (0.98 ml), the resulting mixture was refluxed for 2 hours. The reaction mixture was diluted with ethyl acetate and washed with a saturated aqueous ammonia solution and a saturated aqueous sodium chloride solution, and the organic layer was dried over anhydrous sodium sulfate. The solvent was removed in vacuo, and the residue was purified by chromatography on a silica gel column (ethyl acetate / hexane = 7 / 13) to afford the title compound (1.20 g, yield 65%) as a colorless amorphous solid.

¹H NMR (400 MHz, CDCl₃) δ ppm : 1.64 (9H, s), 2.28-2.35 (2H, m), 2.58 (2H, bs), 2.66 (2H, t, J=6.0), 2.79 (2H, t, J=7.5), 3.12 (2H, bs), 6.12 (1H, d, J=2.5), 6.68 (1H, t, J=7.5), 7.12-7.21 (3H, m), 7.22-7.32 (6H, m), 7.41-7.56 (6H, m), 7.94 (1H, d, J=2.5).

### (c) (E)-3-{3-[1-(t-Butoxycarbonyl)-1H-pyrazole-3-yl]propylidene}-4-hydroxy-1-(triphenylmethyl)piperidine

The title compound was synthesized in a yield of 68% as a colorless solid using (E)-3-{3-[1-(t-butoxycarbonyl)-1H-pyrazole-3-yl]propylidene}-1-(triphenylmethyl)piperidin-4-one obtained in (b) above by conducting a reaction similar to that mentioned in Example 36 (c).

¹H NMR (400 MHz, CDCl₃) δ ppm : 1.63 (9H, s), 1.72-1.83 (1H, m), 2.01-2.25 (2H, m), 2.31-2.42 (2H, m), 2.59 (2H, bs), 2.78 (2H, t, J=7.5), 3.06 (1H, bs), 3.98 (1H, bs), 5.52 (1H, t, J=7.5), 6.15 (1H, d, J=2.5), 7.09-7.19 (3H, m), 7.20-7.31 (6H, m), 7.36-7.56 (6H, m), 7.97 (1H, d, J=2.5).

### (d) (E)-3-{3-[1-(t-Butoxycarbonyl)-1H-pyrazole-3-yl]propylidene}-4-hydroxypiperidine hydrogen trifluoroacetate

The title compound was synthesized in a yield of 65% as a colorless oil using (E)-3-{3-[1-(t-butoxycarbonyl)-1H-pyrazole-3-yl]propylidene}-4-hydroxy-1-(triphenylmethyl)piperidine obtained in (c) above by conducting a reaction similar to that mentioned in Example 3 (c).

¹H NMR (400 MHz, CDCl₃) δ ppm : 1.60 (9H, s), 1.91-2.05 (2H, m), 2.39-2.50 (1H, m), 2.53-2.65 (1H, m), 2.75-2.92 (2H, m), 3.27-3.38 (1H, m), 3.58-3.70 (1H, m), 3.88-3.96 (1H, m), 3.97-4.06 (1H, m), 4.37 (1H, m), 5.73 (1H, t, J=8.0), 6.18 (1H, d, J=2.5), 7.85 (1H, d, J=2.5).

### (e) (E)-3-(3-[1-(t-Butoxycarbonyl)-1H-pyrazole-3-yl]propylidene)-1-[2-cyclopropyl-1-(2-fluorophenyl)-2-oxoethyl]-4-hydroxypiperidine

The title compound was synthesized in a yield of 93% as a colorless amorphous solid using (E)-3-{3-[1-(t-butoxycarbonyl)-1H-pyrazole-3-yl]propylidene}-4-hydroxypiperidine hydrogen trifluoroacetate obtained in (d) above by conducting a reaction similar to that mentioned in Example 1 (g).

¹H NMR (400 MHz, CDCl₃ + D₂O) δ ppm : 0.77-0.89 (2H, m), 0.92-1.08 (2H, m), 1.64 (9H, s), 1.66-1.77 (1H, m), 1.89-2.10 (1H, m), 2.17-2.27 (1H, m), 2.29-2.39 (2H, m), 2.41-2.55 (1H, m), 2.69-2.82 (3H, m), 2.84 and 2.93 (total 1H, each d, J=12.5), 3.16 and 3.26 (total 1H, each d, J=12.5), 4.08 (1H, m), 4.67 and 4.68 (total 1H, each s), 5.52 (1H, m), 6.18 and 6.19 (total 1H, each d, J=2.5), 7.06-7.22 (2H, m), 7.25-7.34 (1H, m), 7.38-7.46 (1H, m), 7.95 and 7.96 (total 1H, each d, J=2.5).

### (f) (E)-4-(Acetylsulfanyl)-3-{3-[1-(t-butoxycarbonyl)-1H-pyrazole-3-yl]propylidene}-1-[2-cyclopropyl-1-(2-fluorophenyl)-2-oxoethyl]piperidine

The title compound was synthesized in a yield of 17% as a colorless oil using (E)-3-{3-[1-(t-butoxycarbonyl)-1H-pyrazole-3-yl]propylidene}-1-[2-cyclopropyl-1-(2-fluorophenyl)-2-oxoethyl]-4-hydroxypiperidine obtained in (e) above by conducting a reaction similar to that mentioned in Example 1 (h).

¹H NMR (400 MHz, CDCl₃) δ ppm : 0.77-0.91 (2H, m), 0.92-1.09 (2H, m), 1.65 (9H, s), 1.71-1.84 (1H, m), 2.06-2.62 (5H, m), 2.28 and 2.29 (total 3H, each s), 2.65-2.92 (4H, m), 3.28-3.39 (1H, m), 4.32 (1H, m), 4.63 and 4.69 (total 1H, each s), 5.59 (1H, m), 6.15 and 6.19 (total 1H, each d, J=2.5), 7.06-7.22 (2H, m), 7.25-7.47 (2H, m), 7.95 and 7.96 (total 1H, each d, J=2.5).

### (g) (E)-4-(Acetylsulfanyl)-1-[2-cyclopropyl-1-(2-fluorophenyl)-2-oxoethyl]-3-{3-[1H-pyrazol-3(5)-yl]propylidene}piperidine dihydrochloride

The title compound was synthesized in a quantitative yield as a colorless amorphous solid using (E)-4-(acetylsulfanyl)-3-{3-[1-(t-butoxycarbonyl)-1H-pyrazole-3-yl]propylidene}-1-[2-cyclopropyl-1-(2-fluorophenyl)-2-oxoethyl]piperidine obtained in (f) above by conducting a reaction similar to that mentioned in Example 9 (f).

¹H NMR (400 MHz, pyridine-d₅) δ ppm : 0.75-0.90 (2H, m), 0.95-1.05 (1H, m), 1.08-1.17 (1H, m), 1.81-1.92 (1H, m), 2.17-2.32 (1H, m), 2.25 and 2.27 (total 3H, each s), 2.42-2.74 (4H, m), 2.76-2.95 (3H, m), 3.00 and 3.07 (total 1H, each d, J=12.5), 3.58 and 3.67 (total 1H, each d, J=12.5), 4.56 (1H, m), 4.87 and 4.92 (total 1H, each s), 5.85 (1H, m), 6.30 and 6.31 (total 1H, each d, J=2.0), 7.18-7.36 (3H, m), 7.64-7.74 (1H, m), 7.81 and 7.82 (total 1H, each d, J=2.0).

MS (FAB) m/z : 442 (M+H)⁺.

### (Example 102) (E)-1-[2-Cyclopropyl-1-(2-fluorophenyl)-2-oxoethyl]-3-{3-[1H-pyrazol-3(5)-yl]propylidene}-4-sulfanylpiperidine dihydrochloride (Hydrochloride of the compound of Compound No. 1-263)

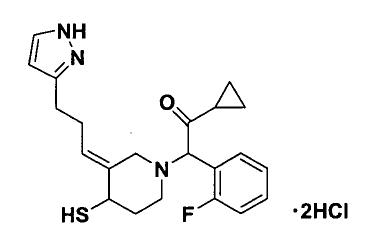

The title compound was synthesized in a yield of 82% as a colorless amorphous solid using (E)-4-(acetylsulfanyl)-1-[2-cyclopropyl-1-(2-fluorophenyl)-2-oxoethyl]-3-{3-[1H-pyrazol-3(5)-yl]propylidene}piperidine dihydrochloride obtained in Example 101 (g) by conducting a reaction similar to that mentioned in Example 34.

¹H NMR (400 MHz, pyridine-d₅) δ ppm : 0.73-0.89 (2H, m), 0.94-1.04 (1H, m), 1.06-1.17 (1H, m), 1.71-1.82 (1H, m), 2.13-2.28 (1H, m), 2.44-2.61 (3H, m), 2.62-2.72 (1H, m), 2.79-3.01 (1H, m), 2.91 and 2.92 (total 2H, each t, J=7.5), 3.32 and 3.41 (total 1H, each d, J=12.5), 3.48 and 3.49 (total 1H, each d, J=12.5), 3.85 (1H, m), 4.90 and 4.92 (total 1H, each s), 5.75 and 5.76 (total 1H, each t, J=7.5), 6.31 and 6.32 (total 1H, each d, J=2.0), 7.16-7.35 (3H, m), 7.69-7.76 (1H, m), 7.82 and 7.83 (total 1H, each d, J=2.0).

MS (FAB) m/z : 400 (M+H)⁺.

### (Example 103) (E)-4-(Acetylsulfanyl)-1-[2-cyclopropyl-1-(2-fluorophenyl)-2-oxoethyl]-3-(2-{N-[2-(ethoxycarbonyl)ethyl]-N-methylamino}ethylidene)piperidine dihydrochloride (Hydrochloride of the compound of Compound No. 3-61)

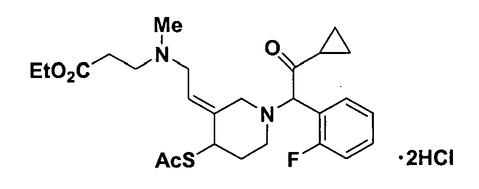

The title compound was synthesized in a yield of 35% as a colorless amorphous solid using (E)-4-(acetylsulfanyl)-1-[2-cyclopropyl-1-(2-fluorophenyl)-2-oxoethyl]-3-(2-hydroxyethylidene)piperidine obtained in Example 32 (f) and N-[2-(ethoxycarbonyl)ethyl]-N-methylamine instead of 4-(ethoxycarbonylmethyl)piperidine by conducting a reaction similar to that mentioned in Example 33.

¹H NMR (500 MHz, pyridine-d₅ + D₂O) δ ppm : 0.79-0.96 (2H, m), 1.06-1.16 (2H, m), 1.16 (3H, t, J=7.0), 1.81-1.90 (1H, m), 2.17-2.37 (2H, m), 2.31 and 2.34 (total 3H, each s), 2.50-2.57 and 2.67-2.74 (total 1H, each m), 2.73 and 2.75 (total 3H, each s), 2.79-2.89 (1H, m), 2.94-3.02 (2H, m), 3.11 and 3.24 (total 1H, each d, J=12.5), 3.26-3.41 (2H, m), 3.62-3.68 (2H, m), 3.70-3.75 (1H, m), 4.14 (2H, q, J=7.0), 4.46-4.51 (1H, m), 5.03 and 5.04 (total 1H, each s), 6.07 and 6.09 (total 1H, each t, J=7.5), 7.28-7.40 (2H, m), 7.45-7.52 (1H, m), 7.67-7.73 (1H, m).

IR (KBr, cm⁻¹) : 1730, 1709, 1495.

### (Example 104) (E)-1-[2-Cyclopropyl-1-(2-fluorophenyl)-2-oxoethyl]-3-(2-{N-[2-(ethoxycarbonyl)ethyl]-N-methylamino } ethylidene)-4-sulfanylpiperidine dihydrochloride (Hydrochloride of the compound of Compound No. 3-60)

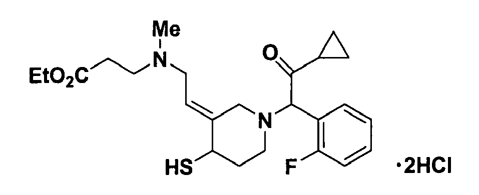

The title compound was synthesized in a yield of 31 % as a colorless amorphous solid using (E)-4-(acetylsulfanyl)-1-[2-cyclopropyl-1-(2-fluorophenyl)-2-oxoethyl]-3-(2-{N-[2-(ethoxycarbonyl)ethyl)-N-methylamino}ethylidene)piperidine dihydrochloride obtained in Example 103 by conducting a reaction similar to that mentioned in Example 34.

¹H NMR (400 MHz, pyridine-d₅ + D₂O) δ ppm : 0.79-0.98 (2H, m), 1.05-1.17 (2H, m), 1.17 (3H, t, J=7.0), 1.75-1.86 (1H, m), 2.19-2.34 (2H, m), 2.63-2.73 (1H, m), 2.80 and 2.83 (total 3H, each s), 2.76-2.84 and 2.89-2.97 (total 1H, each m), 3.01 and 3.03 (total 2H, each t, J=7.0), 3.35-3.47 (3H, m), 3.52 and 3.60 (total 1H, each d, J=12.5), 3.66-3.80 (2H, m), 3.89-3.95 (1H, m), 4.15 (2H, q, J=7.0), 5.01 and 5.03 (total 1H, each s), 6.05-6.12 (1H, m), 7.29-7.39 (2H, m), 7.45-7.53 (1H, m), 7.67-7.73 (1H, m).

IR (KBr, cm⁻¹) : 2463, 1730, 1495.

### (Example 105) (E)-3-(2-{N-[2-(Carboxy)ethyl]-N-methylamino}ethylidene)-1-[2-cyclopropyl-1-(2-fluorophenyl)-2-oxoethyl]-4-sulfanylpiperidine dihydrochloride (Hydrochloride of the compound of Compound No. 3-56)

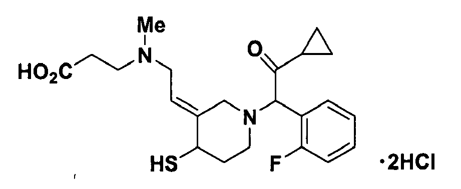

(E)-4-(Acetylsulfanyl)-1-[2-cyclopropyl-1-(2-fluorophenyl)-2-oxoethyl]-3-(2-{N-[2-(ethoxycarbonyl)ethyl)-N-methylamino}ethylidene)piperidine (365 mg) obtained in Example 103 was treated with 3N hydrochloric acid (10 ml) and then the resulting mixture was stirred at 60°C for 3 hours. The reaction mixture was evaporated in vacuo, and the residue was purified using a preparative HPLC (YMC-Pack ODS-A; YMC, eluent: acetonitrile / 0.012N hydrochloric acid = 15 / 85) to afford the title compound (271 mg, yield 74%) as a colorless amorphous solid.

¹H NMR (500 MHz, pyridine-d₅ + D₂O) δ ppm : 0.77-0.86 (1H, m), 0.87-0.94 (1H, m), 1.07-1.15 (2H, m), 1.75-1.84 (1H, m), 2.20-2.31 (2H, m), 2.64-2.71 (1H, m), 2.76-2.83 and 2.89-2.96 (total 1H, each m), 2.98 and 2.99 (total 3H, each s), 3.21 and 3.22 (total 2H, each t, J=7.0), 3.44 and 3.45 (total 1H, each d, J=12.0), 3.55 and 3.62 (total 1H, each d, J=12.0), 3.59-3.68 (2H, m), 3.88-3.94 (2H, m), 3.93-4.02 (1H, m), 5.03 and 5.05 (total 1H, each s), 6.15 (1H, t, J=7.5), 7.27-7.38 (2H, m), 7.43-7.50 (1H, m), 7.66-7.72 (1H, m).

IR (KBr, cm⁻¹) : 2553, 1713, 1494.

### (Example 106) (Z)-4-(Acetylsulfanyl)-1-[2-cyclopropyl-1-(2-fluorophenyl)-2-oxoethyl]-3-(2- {N-[2-(ethoxycarbonyl)ethyl]-N-methylamino }ethylidene)piperidine dihydrochloride (Hydrochloride of the compound of Compound No. 4-61)

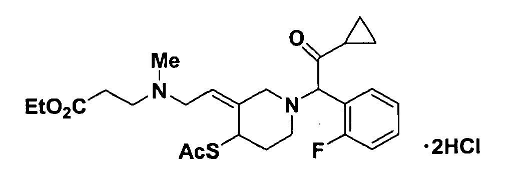

The title compound was synthesized in a yield of 36% as a colorless amorphous solid using (Z)-4-(acetylsulfanyl)-1-[2-cyclopropyl-1-(2-fluorophenyl)-2-oxoethyl]-3-(2-hydroxyethylidene)piperidine obtained in Example 66 (e) instead of (E)-4-(acetylsulfanyl)-1-[2-cyclopropyl-1-(2-fluorophenyl)-2-oxoethyl]-3-(2-hydroxyethylidene)piperidine and N-[2-(ethoxycarbonyl)ethyl)-N-methylamine instead of 4-(ethoxycarbonylmethyl)piperidine by conducting a reaction similar to that mentioned in Example 33.

¹H NMR (400 MHz, pyridine-d₅) δ ppm : 0.76-0.84 (1H, m), 0.96-1.18 (3H, m), 1.07 and 1.08 (total 3H, each t, J=7.0), 1.80-1.91 (1H, m), 2.23-2.42 (1H, m), 2.26 and 2.27 (total 3H, each s), 2.46-2.65 (2H, m), 2.77 and 2.79 (total 3H, each s), 2.95-3.13 (2H, m), 3.22-3.32 (2H, m), 3.40-3.57 (3H, m), 3.93-4.20 (2H, m), 4.07 and 4.08 (total 2H, each q, J=7.0), 4.87 and 4.95 (total 1H, each s), 4.93-4.99 (1H, m), 6.02 and 6.13 (total 1H, each t, J=7.0), 7.20-7.39 (3H, m), 7.58-7.67 (1H, m).

IR (KBr, cm⁻¹) : 1731, 1711, 1494.

### (Example 107) (Z)-1-[2-Cyclopropyl-1-(2-fluorophenyl)-2-oxoethyl]-3-(2- {N-[2-(ethoxycarbonyl)ethyl]-N-methylamino) ethylidene)-4-sulfanylpiperidine dihydrochloride (Hydrochloride of the compound of Compound No. 4-60)

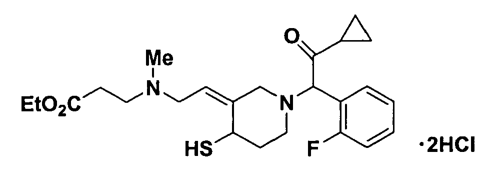

The title compound was synthesized in a yield of 60% as a colorless amorphous solid using (Z)-4-(acetylsulfanyl)-1-[2-cyclopropyl-1-(2-fluorophenyl)-2-oxoethyl]-3-(2- {N-[2-(ethoxycarbonyl)ethyl] -N-methylamino}ethylidene)piperidine dihydrochloride obtained in Example 106 by conducting a reaction similar to that mentioned in Example 34.

¹H NMR (400 MHz, pyridine-d₅ + D₂O) δ ppm : 0.77-0.89 (1H, m), 0.90-0.98 (1H, m), 0.99-1.06 (1H, m), 1.07-1.15 (1H, m), 1.12 and 1.13 (total 3H, each t, J=7.0), 1.75-1.88 (1H, m), 2.25-2.50 (2H, m), 2.58-3.01 (2H, m), 2.86 and 2.90 (total 3H, each s), 3.08-3.18 (2H, m), 3.31 and 3.37 (total 1H, each d, J=12.5), 3.42-3.57 (3H, m), 3.86-4.02 (2H, m), 4.10 and 4.12 (total 2H, each q, J=7.0), 4.61-4.66 (1H, m), 4.87 and 4.94 (total 1H, each s), 5.71 and 5.84 (total 1H, each t, J=7.5), 7.22-7.30 (2H, m), 7.34-7.43 (1H, m), 7.63-7.70 (1H, m).

IR (KBr, cm⁻¹) : 2550, 1730, 1714, 1494.

### (Example 108) (Z)-3-(2-{N-[2-(Carboxy)ethyl)-N-methylamino}ethylidene)-1-[2-cyclopropyl-1-(2-fluorophenyl)-2-oxoethyl]-4-sulfanylpiperidine dihydrochloride (Hydrochloride of the compound of Compound No. 4-56)

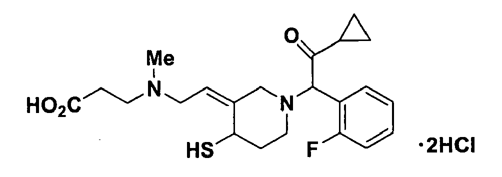

The title compound was synthesized in a yield of 42% as a colorless amorphous solid using (Z)-1-[2-cyclopropyl-1-(2-fluorophenyl)-2-oxoethyl]-3-(2-{N-[2-(ethoxycarbonyl)ethyl]-N-methylamino }ethylidene)-4-sulfanylpiperidine dihydrochloride obtained in Example 107 by conducting a reaction similar to that mentioned in Example 15.

¹H NMR (500 MHz, pyridine-d₅ + D₂O) δ ppm : 0.79-0.96 (2H, m), 0.99-1.05 (1H, m), 1.06-1.13 (1H, m), 1.76-1.87 (1H, m), 2.28-2.48 (2H, m), 2.60-2.99 (2H, m), 2.83 and 2.87 (total 3H, each s), 3.13 and 3.16 (total 2H, each t, J=7.0), 3.30 and 3.36 (total 1H, each d, J=12.0), 3.42-3.56 (3H, m), 3.80-3.97 (2H, m), 4.60-4.64 (1H, m), 4.87 and 4.93 (total 1H, each s), 5.65 and 5.78 (total 1H, each t, J=7.0), 7.23-7.30 (2H, m), 7.36-7.44 (1H, m), 7.64-7.70 (1H, m).

MS (FAB) m/z : 421 (M+H)⁺.

### (Example 109) (Z)-4-(Acetylsulfanyl)-1-[2-cyclopropyl-1-(2-fluorophenyl)-2-oxoethyl]-3-[2-(4-ethoxycarbonyl-1H-pyrazol-1-yl)ethylidene]piperidine hydrochloride (Hydrochloride of the compound of Compound No. 2-26)

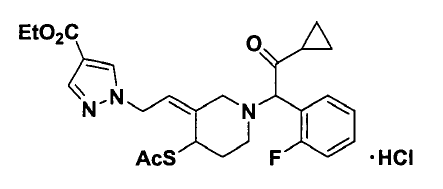

The title compound was synthesized in a yield of 11% as a pale yellow amorphous solid using (Z)-4-(acetylsulfanyl)-1-[2-cyclopropyl-1-(2-fluorophenyl)-2-oxoethyl]-3-(2-hydroxyethylidene)piperidine obtained in Example 66 (e) and 4-ethoxycarbonyl-1H-pyrazole by conducting a reaction similar to that mentioned in Example 40 (a).

¹H NMR (400 MHz, CDCl₃) δ ppm : 0.77-0.89 (2H, m), 0.96-1.06 (2H, m), 1.33 and 1.34 (total 3H, each t, J=7.0), 1.81-1.89 (1H, m), 2.10-2.21 (2H, m), 2.30-2.38 (1H, m), 2.32 (3H, s), 2.79 and 2.97 (total 1H, each d, J=12.0), 2.83-2.85 and 2.98-3.00 (total 1H, each m), 3.15 and 3.30 (total 1H, each d, J=12.0), 4.28 and 4.29 (total 2H, each q, J=7.0), 4.67 and 4.71 (total 1H, each s), 4.83 (1H, bs), 4.90-5.05 (2H, m), 5.47 and 5.56 (total 1H, each t, J=7.0), 7.09-7.18 (2H, m), 7.30-7.39 (2H, m), 7.86 and 7.87 (total 1H, each s), 7.88 and 7.90 (total 1H, each s).

IR (KBr, cm⁻¹) : 1713, 1495.

### (Example 110) (Z)-1-[2-Cyclopropyl-1-(2-fluorophenyl)-2-oxoethyl]-3-[2-(4-ethoxycarbonyl-1H-pyrazol-1-yl)ethylidene]-4-sulfanylpiperidine hydrochloride (Hydrochloride of the compound of Compound No. 2-25)

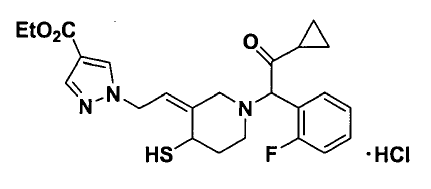

The title compound was synthesized in a yield of 88% as a colorless amorphous solid using (Z)-4-(acetylsulfanyl)-1-[2-cyclopropyl-1-(2-fluorophenyl)-2-oxoethyl]-3-[2-(4-ethoxycarbonyl-1H-pyrazol-1-yl)ethylidene]piperidine hydrochloride obtained in Example 109 by conducting a reaction similar to that mentioned in Example 34.

¹H NMR (400 MHz, pyridine-d₅) δ ppm : 0.73-0.83 (2H, m), 0.97-1.04 (1H, m), 1.06-1.13 (1H, m), 1.18-1.25 (3H, m), 1.75-1.84 (1H, m), 2.20-2.36 (1H, m), 2.65-2.71 and 2.97-3.01 (total 1H, each m), 2.82-2.93 (2H, m), 3.23 and 3.40 (total 1H, each d, J=12.0), 3.37 and 3.53 (total 1H, each d, J=12.0), 4.25-4.34 (2H, m), 4.68 (1H, bs), 4.91 and 4.93 (total 1H, each s), 5.02 and 5.05 (total 2H, each d, J=7.0), 5.52 and 5.63 (total 1H, each t, J=7.0), 7.17-7.25 (2H, m), 7.29-7.34 (1H, m), 7.64-7.68 (1H, m), 8.28 and 8.29 (total 1H, each s), 8.41 and 8.44 (total 1H, each s).

IR (KBr, cm⁻¹) : 2541, 1713, 1494.

### (Example 111) (Z)-3-[2-(4-Carboxy-1H-pyrazol-1-yl)ethylidene]-1-[2-cyclopropyl-1-(2-fluorophenyl)-2-oxoethyl]-4-sulfanylpiperidine hydrochloride (Hydrochloride of the compound of Compound No. 2-17)

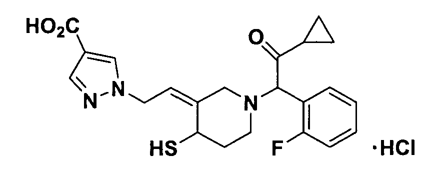

The title compound was synthesized in a yield of 49% as a colorless amorphous solid using (Z)-1-[2-cyclopropyl-1-(2-fluorophenyl)-2-oxoethyl]-3-[2-(4-ethoxycarbonyl-1H-pyrazol-1-yl)ethylidene]-4-sulfanylpiperidine hydrochloride obtained in Example 110 by conducting a reaction similar to that mentioned in Example 15.

¹H NMR (400 MHz, pyridine-d₅) δ ppm : 0.71-0.84 (2H, m), 0.96-1.04 (1H, m), 1.06-1.15 (1H, m), 1.72-1.89 (1H, m), 2.17-2.36 (1H, m), 2.62-3.02 (3H, m), 3.22 and 3.39 (total 1H, each d, J=12.0), 3.37 and 3.53 (total 1H, each d, J=12.5), 4.68 (1H, bs), 4.90 and 4.92 (total 1H, each s), 4.98-5.09 (2H, m), 5.52 and 5.63 (total 1H, each t, J=7.0), 7.16-7.35 (3H, m), 7.66 (1H, m), 8.43 and 8.45 (total 1H, each s), 8.52 and 8.55 (total 1H, each s).

IR (KBr, cm⁻¹) : 3399, 2547, 1710, 1494.

### (Example 112) (Z)-4-(Acetylsulfanyl)-1-[2-cyclopropyl-1-(2-fluorophenyl)-2-oxoethyl]-3- {2-[4-(ethoxycarbonylmethyl) -1H-pyrazol-1-yl]ethylidene}piperidine hydrochloride (Hydrochloride of the compound of Compound No. 2-62)

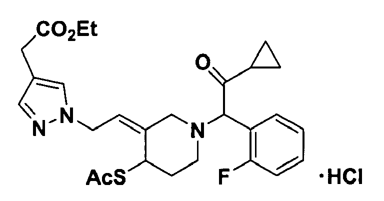

### (a) (Z)-4-Acetoxy-3-[2-(t-butyldiphenylsilyloxy)ethylidene]-1-(triphenylmethyl)piperidine

To a solution of (E)-4-acetoxy-3-[2-(t-butyldiphenylsilyloxy)ethylidene]-1-(triphenylmethyl)piperidine (200 mg) obtained in Example 66 (c) and 1,1'-bis(diphenylphosphino)ferrocene (33 mg) in toluene (2 ml) were added a solution of potassium acetate (147 mg) in water (1 ml) at room temperature and then palladium acetate (7 mg) at 50°C, and the resulting mixture was stirred at the same temperature for 1.5 hours. The reaction mixture was diluted with ethyl acetate and washed with water and a saturated aqueous sodium chloride solution, and the organic layer was dried over anhydrous sodium sulfate. The solvent was removed in vacuo, and the residue was purified by chromatography on a silica gel column (ethyl acetate / hexane =1/19-1/9) to afford a 1:11 mixture (143 mg) of the title compound and the (E)-isomer thereof as a colorless amorphous solid. This mixture was further purified using a preparative HPLC (YMC-Pack ODS-A; YMC, eluent: acetonitrile / water = 19 / 1) to afford the title compound (3 mg, yield 2%) and the (E) form (73 mg, yield 37%) thereof as a colorless amorphous solid, respectively.

¹H NMR (400 MHz, CDCl₃) δ ppm : 1.10 (9H, s), 1.67-1.80 (1H, m), 1.77 (3H, s), 1.84-1.96 (2H, m), 2.25-2.37 (1H, m), 2.69-2.81 (1H, m), 3.07-3.16 (1H, m), 4.38 (1H, dd, J=14.0, 4.5), 4.54 (1H, dd, J=14.0, 7.0), 5.42 (1H, bs), 5.55 (1H, dd, J=7.0, 4.5), 7.14-7.20 (3H, m), 7.23-7.30 (6H, m), 7.37-7.50 (12H, m), 7.71-7.77 (4H, m).

### (b) (Z)-3-[2-(t-Butyldiphenylsilyloxy)ethylidene]-1-(triphenyhnethyl)piperidin-4-ol

To a solution of (Z)-4-acetoxy-3-[2-(t-butyldiphenylsilyloxy)ethylidene]-1-(triphenylmethyl)piperidine (3.00 g) obtained in (a) above in methanol (60 ml) was added potassium carbonate (2.00 g) under ice cooling, and the resulting mixture was stirred at room temperature for 4 hours. Insolubles in the reaction mixture were removed by filtration, and the solvent was evaporated in vacuo. The obtained residue was diluted with ethyl acetate and washed with a saturated aqueous sodium chloride solution, and the organic layer was dried over anhydrous sodium sulfate. The solvent was removed in vacuo, and the residue was purified by chromatography on a silica gel column (ethyl acetate / hexane = 1 / 10) to afford the title compound (2.02 g, yield 72%) as a colorless amorphous solid.

¹H NMR (400 MHz, CDCl₃) δ ppm : 1.06 (9H, s), 1.72-1.83 (1H, m), 1.89-2.01 (1H, m), 2.06-2.27 (1H, m), 2.40-2.65 (2H, m), 2.77-2.95 (1H, m), 4.30-4.41 (3H, m), 5.50 (1H, t, J=6.5), 7.12-7.20 (3H, m), 7.22-7.31 (6H, m), 7.34-7.53 (12H, m), 7.64-7.77 (4H, m).

### (c) (Z)-3-(2-Hydroxyethylidene)-1-(triphenylmethyl)piperidin-4-ol

The title compound was synthesized in a yield of 96% as a colorless amorphous solid using (Z)-3-[2-(t-butyldiphenylsilyloxy)ethylidene]-1-(triphenylmethyl)piperidin-4-ol obtained in (b) above by conducting a reaction similar to that mentioned in Example 1 (e).

¹H NMR (400 MHz, CDCl₃) δ ppm : 1.78-1.89 (1H, m), 1.97-2.09 (1H, m), 2.11-2.67 (3H, m), 2.79-2.95 (1H, m), 4.21 (1H, dd, J=12.5, 6.5), 4.31 (1H, dd, J=12.5, 7.5), 4.55 (1H, bs), 5.52 (1H, dd, J=7.5, 6.5), 7.11-7.18 (3H, m), 7.21-7.30 (6H, m), 7.41-7.55 (6H, m).

### (d) (Z)-3-(2-Acetoxyethylidene)-1-(triphenylmethyl)piperidin-4-ol

To a suspension of (Z)-3-(2-hydroxyethylidene)-1-(triphenylmethyl)piperidin-4-ol (33.0 g) obtained in (c) above in dichloromethane (500 ml) were added a solution of acetic anhydride (8.0 ml) in dichloromethane (60 ml) and triethylamine (24.0 ml) under ice cooling, and the resulting mixture was stirred at room temperature for 20 hours. The reaction mixture was diluted with dichloromethane and washed with water and a saturated aqueous sodium chloride solution, and the organic layer was dried over anhydrous sodium sulfate. The solvent was removed in vacuo, and the residue was purified by chromatography on a silica gel column (ethyl acetate / hexane = 1 / 2) to afford the title compound (30.9 g, yield 84%) as a colorless amorphous solid.

¹H NMR (400 MHz, CDCl₃) δ ppm : 1.85-2.17 (5H, m), 2.36-2.45 (1H, m), 2.50-2.59 (1H, m), 2.62-2.74 (1H, m), 2.97-3.09 (1H, m), 4.53 (1H, dd, J=12.0, 6.0), 4.74 (1H, bs), 5.05 (1H, dd, J=12.0, 9.0), 5.35 (1H, dd, J=9.0, 6.0), 7.11-7.19 (3H, m), 7.21-7.29 (6H, m), 7.40-7.52 (6H, m).

### (e) (Z)-4-(t-Butyldimethylsilyloxy)-3-(2-acetoxyethylidene)-1-(triphenylmethyl)piperidine

The title compound was synthesized in a yield of 68% as a colorless amorphous solid using (Z)-3-(2-acetoxyethylidene)-1-(triphenylmethyl)piperidin-4-ol obtained in (d) above by conducting a reaction similar to that mentioned in Example 1 (a).

¹H NMR (500 MHz, CDCl₃) δ ppm : -0.13 (3H, s), -0.05 (3H, s), 0.67 (9H, s), 1.69-1.78 (1H, m), 1.84-1.93 (1H, m), 2.08 (3H, s), 2.12-2.30 (1H, m), 2.43-2.68 (2H, m), 2.79-2.98 (1H, m), 4.40 (1H, bs), 4.66-4.85 (2H, m), 5.35 (1H, t, J=7.5), 7.08-7.18 (3H, m), 7.20-7.29 (6H, m), 7.39-7.54 (6H, m).

### (f) (Z)-4-(t-Butyldimethylsilyloxy)-3-(2-hydroxyethylidene)-1-(triphenylmethyl)piperidine

The title compound was synthesized in a yield of 94% as a colorless amorphous solid using (Z)-4-(t-butyldimethylsilyloxy)-3-(2-acetoxyethylidene)-1-(triphenylmethyl)piperidine obtained in (e) above by conducting a reaction similar to that mentioned in Example 112 (b).

¹H NMR (500 MHz, CDCl₃) δ ppm : -0.09 (3H, s), -0.03 (3H, s), 0.72 (9H, s), 1.71-1.94 (2H, m), 2.21-2.85 (4H, m), 4.16-4.36 (2H, m), 4.46 (1H, bs), 5.50 (1H, t, J=7.0), 7.09-7.18 (3H, m), 7.20-7.29 (6H, m), 7.39-7.56 (6H, m).

### (g) (Z)-4-(t-Butyldimethylsilyloxy)-3-{2-[4-(ethoxycarbonylmethyl)-1H-pyrazol-1-yl]ethylidene}-1-(triphenylmethyl)piperidine

To a solution of (Z)-4-(t-butyldimethylsilyloxy)-3-(2-hydroxyethylidene)-1-(triphenylmethyl)piperidine (5.80 g) obtained in (f) above and p-toluenesulfonic anhydride (3.80 g) in acetonitrile (60 ml) was added triethylamine (1.94 ml) at room temperature, and the resulting mixture was stirred at the same temperature for 2.5 hours. The reaction mixture was diluted with ethyl acetate and washed with water and a saturated aqueous sodium chloride solution, and the organic layer was dried over anhydrous sodium sulfate. The solvent was removed in vacuo to afford (Z)-4-(t-butyldimethylsilyloxy)-3-[2-(tosyloxy)ethylidene]-1-(triphenylmethyl)piperidine (9.08 g, quantitative yield) as a brown oil.

To a solution of 4-(ethoxycarbonylmethyl)-1H-pyrazole (1.80 g) in N,N-dimethylformamide (40 ml) was added sodium hydride (0.56 g) under ice cooling, and the resulting mixture was stirred at the same temperature for 20 minutes. To the reaction mixture was added a solution of (Z)-4-(t-butyldimethylsilyloxy)-3-[2-(tosyloxy)ethylidene]-1-(triphenylmethyl)piperidine obtained above in N,N-dimethylformamide (10 ml), and then the resulting mixture was stirred at room temperature for 1.5 hours and at 60°C for further 30 minutes. The reaction mixture was diluted with ethyl acetate and washed with water and a saturated aqueous sodium chloride solution, and the organic layer was dried over anhydrous sodium sulfate. The solvent was removed in vacuo, and the residue was purified by chromatography on a silica gel column (ethyl acetate / hexane = 1 / 5 - 1 / 4) to afford the title compound (3.83 g, yield 52%) as a yellow oil.

¹H NMR (400 MHz, CDCl₃) δ ppm : -0.11 (3H, s), -0.04 (3H, s), 0.70 (9H, s), 1.22-1.31 (3H, m), 1.72-1.99 (4H, m), 2.15-2.40 (1H, m), 2.53-2.79 (1H, m), 3.51 (2H, s), 4.16 (2H, q, J=7.5), 4.51-4.57 (1H, m), 4.88-4.98 (2H, m), 5.46 (1H, t, J=7.0), 7.09-7.28 (15H, m), 7.42 (1H, s), 7.45 (1H, s).

### (h) (Z)-3-{2-[4-(Ethoxycarbonylmethyl)-1H-pyrazol-1-yl]ethylidene}-4-hydroxy-1-(triphenylmethyl)piperidine

The title compound was synthesized in a yield of 67% as a colorless amorphous solid using (Z)-4-(t-butyldimethylsilyloxy)-3-{2-[4-(ethoxycarbonylmethyl)-1H-pyrazol-1-yl]ethylidene}-1-(triphenylmethyl)piperidine obtained in (g) above by conducting a reaction similar to that mentioned in Example 1 (e).

¹H NMR (400 MHz, CDCl₃) δ ppm : 1.26 (3H, t, J=7.0), 1.92-2.00 (1H, m), - 2.08-2.23 (2H, m), 2.57-2.69 (2H, m), 2.91-3.03 (1H, m), 3.44 (2H, s), 4.14 (2H, q, J=7.0), 4.59-4.68 (1H, m), 4.75 (1H, m), 4.97-5.06 (1H, m), 5.48 (1H, t, J=8.0), 7.10-7.29 (15H, m), 7.36 (1H, s), 7.37 (1H, s).

### (i) (Z)-4-(Acetylsulfanyl)-3- {2-[4-(ethoxycarbonylmethyl)-1H-pyrazol-1-yl] ethylidene}-1-(triphenylmethyl)piperidine

The title compound was synthesized in a yield of 88% as a brown amorphous solid using (Z)-3-{2-[4-(ethoxycarbonylmethyl)-1H-pyrazol-1-yl]ethylidene}-4-hydroxy-1-(triphenylmethyl)piperidine obtained in (h) above by conducting a reaction similar to that mentioned in Example 1 (h).

¹H NMR (400 MHz, CDCl₃) δ ppm : 1.26 (3H, t, J=7.0), 1.62-1.69 (1H, m), 1.80-1.87 (1H, m), 2.06-2.12 (1H, m), 2.23 (3H, s), 2.36-2.49 (1H, m), 3.06-3.14 (1H, m), 3.38-3.44 (1H, m), 3.47 (2H, s), 4.15 (2H, q, J=7.0), 4.85-4.89 (1H, m), 4.95 (2H, d, J=6.5), 5.50 (1H, t, J=6.5), 7.12-7.30 (15H, m), 7.37 (1H, s), 7.42 (1H, s).

### (j) (Z)-4-(Acetylsulfanyl)-3-{2-[4-(ethoxycarbonylmethyl)-1H-pyrazol-1-yl]ethylidene}piperidine hydrogen trifluoroacetate

The title compound was synthesized in a yield of 87% as a brown oil using (Z)-4-(acetylsulfanyl)-3-{2-[4-(ethoxycarbonylmethyl)-1H-pyrazol-1-yl]ethylidene}-1-(triphenylmethyl)piperidine obtained in (i) above by conducting a reaction similar to that mentioned in Example 3 (c).

¹H NMR (400 MHz, CDCl₃) δ ppm : 1.26 (3H, t, J=7.0), 2.06-2.10 (1H, m), 2.39 (3H, s), 2.39-2.51 (1H, m), 3.10-3.21 (1H, m), 3.38-3.46 (1H, m), 3.47 (2H, s), 3.62-3.77 (2H, m), 4.12 (2H, q, J=7.0), 4.86 (1H, m), 4.89-5.04 (2H, m), 5.80 (1H, t, J=6.5), 7.42 (1H, s), 7.43 (1H, s).

### (k) (Z)-4-(Acetylsulfanyl)-1-[2-cyclopropyl-1-(2-fluorophenyl)-2-oxoethyl]-3-{2-[4-(ethoxycarbonylmethyl)-1H-pyrazol-1-yl]ethylidene}piperidine hydrochloride

The title compound was synthesized in a yield of 80% as a pale yellow amorphous solid using (Z)-4-(acetylsulfanyl)-3-{2-[4-(ethoxycarbonylmethyl)-1H-pyrazol-1-yl]ethylidene}piperidine hydrogen trifluoroacetate obtained in (j) above by conducting a reaction similar to that mentioned in Example 1 (g).

¹H NMR (400 MHz, pyridine-d₅) δ ppm : 0.70-0.83 (2H, m), 0.96-1.12 (2H, m), 1.11 and 1.12 (total 3H, each t, J=7.0), 1.81-1.93 (1H, m), 2.04-2.15 (1H, m), 2.26-2.44 (2H, m), 2.26 (3H, s), 2.48-2.59 (1H, m), 2.90-2.98 and 3.04-3.12 (total 1H, each m), 3.31 and 3.46 (total 1H, each d, J=12.0), 3.61 (2H, s), 4.11 and 4.12 (total 2H, each q, J=7.0), 4.87 and 4.90 (total 1H, each s), 5.05-5.23 (3H, m), 5.65 and 5.74 (total 1H, each t, J=6.5), 7.16-7.27 (2H, m), 7.28-7.36 (1H, m), 7.57-7.65 (1H, m), 7.69 and 7.72 (total 1H, each s), 7.73 and 7.75 (total 1H, each s).

MS (FAB) m/z : 514 (M+H)⁺.

### (Example 113) (Z)-1-[2-Cyclopropyl-1-(2-fluorophenyl)-2-oxoethyl]-3-{2-[4-(ethoxycarbonylmethyl)-1H-pyrazol-1-yl]ethylidene}-4-sulfanylpiperidine hydrochloride (Hydrochloride of the compound of Compound No. 2-61)

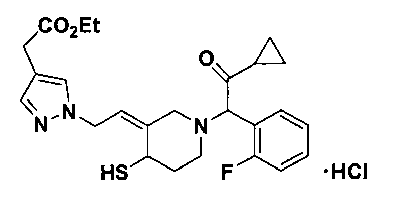

The title compound was synthesized in a yield of 45% as a colorless amorphous solid using (Z)-4-(acetylsulfanyl)-1-[2-cyclopropyl-1-(2-fluorophenyl)-2-oxoethyl]-3- {2-[4-(ethoxycarbonylmethyl)-1H-pyrazol-1-yl]ethylidene}piperidine hydrochloride obtained in Example 112 (k) by conducting a reaction similar to that mentioned in Example 34.

¹H NMR (400 MHz, pyridine-d₅) δ ppm : 0.71-0.81 (2H, m), 0.95-1.13 (2H, m), 1.11 and 1.12 (total 3H, each t, J=7.0), 1.71-1.83 (1H, m), 2.16-2.36 (1H, m), 2.38-2.47 (1H, m), 2.61-3.02 (3H, m), 3.18 and 3.36 (total 1H, each d, J=12.5), 3.51 and 3.61 (total 1H, each d, J=12.5), 3.64 (2H, s), 4.11 and 4.12 (total 2H, each q, J=7.0), 4.63 (1H, m), 4.87-4.97 (2H, m), 5.46 and 5.58 (total 1H, each t, J=6.5), 7.15-7.35 (3H, m), 7.56-7.78 (3H, m).

MS (FAB) m/z : 472 (M+H)⁺.

### (Example 114) (Z)-3-{2-[4-(Carboxymethyl)-1H-pyrazol-1-yl]ethylidene}-1-[2-cyclopropyl-1-(2-fluorophenyl)-2-oxoethyl]-4-sulfanylpiperidine hydrochloride (Hydrochloride of the compound of Compound No. 2-53)

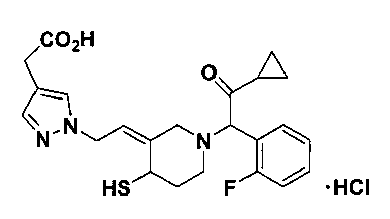

The title compound was synthesized in a yield of 23% as a colorless amorphous solid using (Z)-4-(acetylsulfanyl)-1-[2-cyclopropyl-1-(2-fluorophenyl)-2-oxoethyl]-3-{2-[4-(ethoxycarbonylmethyl)-1H-pyrazol-1-yl]ethylidene}piperidine hydrochloride obtained in Example 112 (k) by conducting a reaction similar to that mentioned in Example 105.

¹H NMR (400 MHz, pyridine-d₅) δ ppm : 0.71-0.83 (2H, m), 0.96-1.12 (2H, m), 1.73-1.82 (1H, m), 2.19-2.35 (1H, m), 2.40-2.48 (1H, m), 2.80-3.00 (2H, m), 3.18 and 3.35 (total 1H, each d, J=11.5), 3.35 and 3.51 (total 1H, each d, J=11.5), 3.79 and 3.80 (total 2H, each s), 4.64 (1H, bs), 4.86-5.01 (2H, m), 4.88 and 4.91 (total 1H, each s), 5.47 and 5.59 (total 1H, each t, J=7.0), 7.16-7.33 (3H, m), 7.65 (1H, m), 7.78 and 7.81 (total 1H, each s), 7.83 and 7.85 (total 1H, each s).

MS (FAB) m/z : 444 (M+H)⁺.

### (Example 115) (E)-4-(Acetylsulfanyl)-1-[2-cyclopropyl-1-(2-fluorophenyl)-2-oxoethyl]-3-(2-{4-[2-(ethoxycarbonyl)ethyl)-1H-pyrazol-1-yl}ethylidene)piperidine (Compound of Compound No. 1-88)

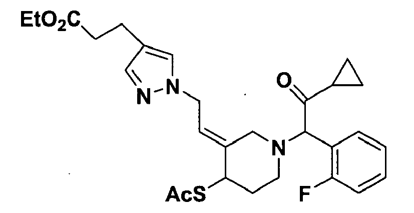

### (a) (E)-4-(t-Butyldimethylsilyloxy)-3-(2-{4-[2-(ethoxycarbonyl)ethyl]-1H-pyrazol-1-yl}ethylidene)-1-(triphenylmethyl)piperidine

The title compound was synthesized in a yield of 64% as a colorless oil using (E)-4-(t-butyldimethylsilyloxy)-3-[2-(tosyloxy)ethylidene]-1-(triphenylmethyl)piperidine obtained in Example 1 (c) and 4-[2-(ethoxycarbonyl)ethyl]-1H-pyrazole by conducting a reaction similar to that mentioned in Example 21 (a).

¹H NMR (400 MHz, CDCl₃) δ ppm : 0.06 (6H, s), 0.88 (9H, s), 1.27 (3H, t, J=7.0), 1.82 (1H, m), 1.90-2.03 (3H, m), 2.60 (2H, t, J=7.0), 2.86 (2H, t, J=7.0), 3.03 (1H, bs), 3.70 (1H, bs), 3.91-3.98 (1H, m), 4.16 (2H, q, J=7.0), 4.71-4.81 (2H, m), 5.81-5.84 (1H, m), 7.21 (3H, m), 7.28-7.33 (9H, m), 7.40 (1H, s), 7.53 (4H, m).

### (b) (E)-3-(2-{4-[2-(Ethoxycarbonyl)ethyl]-1H-pyrazol-1-yl}ethylidene)-4-hydroxy-1-(triphenylmethyl)piperidine

The title compound was synthesized in a yield of 58% a colorless amorphous solid using (E)-4-(t-butyldimethylsilyloxy)-3-(2- {4-[2-(ethoxycarbonyl)ethyl]-1H-pyrazol-1-yl}ethylidene)-1-(triphenylmethyl)piperidine obtained in (a) above by conducting a reaction similar to that mentioned in Example 1 (e).

¹H NMR (400 MHz, CDCl₃) δ ppm : 1.23 (3H, t, J=7.0), 1.57-1.63 (1H, m), 1.81-1.90 (1H, m), 2.10-2.14 (1H, m), 2.34 (1H, bs), 2.56 (2H, t, J=7.0), 2.80 (2H, t, J=7.0), 2.84 (1H, bs), 3.42 (1H, bs), 4.01 (1H, m), 4.12 (2H, q, J=7.0), 4.65-4.76 (2H, m), 5.75 (1H, t, J=7.0), 7.14-7.27 (10H, m), 7.35 (1H, s), 7.48 (6H, m).

### (c) (E)-4-(Acetylsulfanyl)-3-(2-{4-[2-(ethoxycarbonyl)ethyl]-1H-pyrazol-1-yl} ethylidene)-1-(triphenylmethyl)piperidine

The title compound was synthesized in a yield of 65% as a colorless oil using (E)-3-(2- {4-[2-(ethoxycarbonyl)ethyl]-1H-pyrazol-1-yl}ethylidene)-4-hydroxy-1-(triphenylmethyl)piperidine obtained in (b) above by conducting a reaction similar to that mentioned in Example 1 (h).

¹H NMR (400 MHz, CDCl₃) δ ppm : 1.24 (3H, t, J=7.0), 1.86-1.94 (1H, m), 2.25 (3H, s), 2.31 (2H, m), 2.56 (2H, t, J=7.0), 2.81 (2H, t, J=7.0), 2.86 (1H, m), 4.12 (2H, q, J=7.0), 4.16-4.34 (3H, m), 4.58-4.71 (2H, m), 5.77 (1H, t, J=7.0), 7.15-7.29 (10H, m), 7.36 (1H, s), 7.60 (6H, m).

### (d) (E)-4-(Acetylsulfanyl)-3-(2-{4-[2-(ethoxycarbonyl)ethyl]-1H-pyrazol-1-yl}ethylidene)piperidine hydrogen trifluoroacetate

The title compound was synthesized in a yield of 23% as a colorless oil using (E)-4-(acetylsulfanyl)-3-(2-{4-[2-(ethoxycarbonyl)ethyl]-1H-pyrazol-1-yl}ethylidene)-1-(triphenylmethyl)piperidine obtained in (c) above by conducting a reaction similar to that mentioned in Example 3 (c).

¹H NMR (400 MHz, CDCl₃) δ ppm : 1.25 (3H, t, J=7.0), 2.03-2.09 (1H, m), 2.36 (3H, s), 2.39-2.47 (1H, m), 2.55 (2H, t, J=7.0), 2.79 (2H, t, J=7.0), 3.24-3.31 (1H, m), 3.41-3.49 (1H, m), 3.75 (1H, d, J=14.0), 4.11-4.17 (3H, m), 4.44 (1H, m), 4.63 (1H, dd, J=15.5, 5.5), 4.83 (1H, dd, J=15.5, 9.0), 5.93-5.96 (1H, m), 7.31 (1H, s), 7.39 (1H, s).

### (e) (E)-4-(Acetylsulfanyl)-1-[2-cyclopropyl-1-(2-fluorophenyl)-2-oxoethyl]-3-(2-{4-[2-(ethoxycarbonyl)ethyl]-1H-pyrazol-1-yl} ethylidene)piperidine

The title compound was synthesized in a yield of 67% as a colorless oil using (E)-4-(acetylsulfanyl)-3-(2-{4-[2-(ethoxycarbonyl)ethyl]-1H-pyrazol-1-yl}ethylidene)piperidine hydrogen trifluoroacetate obtained in (d) above by conducting a reaction similar to that mentioned in Example 1 (g).

¹H NMR (400 MHz, CDCl₃) δ ppm : 0.77-0.89 (2H, m), 0.99-1.07 (2H, m), 1.25 (3H, t, J=7.0), 1.83 (1H, m), 2.15 (2H, m), 2.29 and 2.30 (total 3H, each s), 2.54 (2H, t, J=7.0), 2.55-2.64 (1H, m), 2.68-2.76 (1H, m), 2.78 (2H, t, J=7.0), 2.84-2.92 and 3.12-3.18 (total 1H, each m), 3.26-3.35 and 3.39-3.46 (total 1H, each m), 4.14 (2H, q, J=7.0), 4.28-4.35 (1H, m), 4.60-4.76 (3H, m), 5.77 (1H, m), 7.09-7.22 (2H, m), 7.27-7.42 (4H, m).

### (Example 116) (E)-1-[2-Cyclopropyl-1-(2-fluorophenyl)-2-oxoethyl]-3-(2-{4-[2-(ethoxycarbonyl)ethyl]-1H-pyrazol-1-yl} ethylidene)-4-sulfanylpiperidine hydrochloride (Hydrochloride of the compound of Compound No. 1-87)

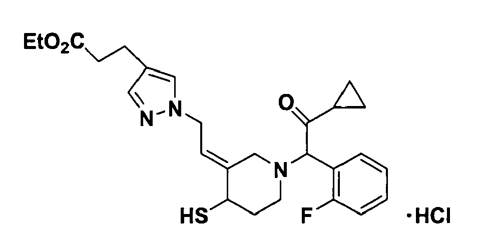

The title compound was synthesized in a yield of 74% as a colorless amorphous solid using (E)-4-(acetylsulfanyl)-1-[2-cyclopropyl-1-(2-fluorophenyl)-2-oxoethyl]-3-(2-{4-[2-(ethoxycarbonyl)ethyl]-1H-pyrazol-1-yl}ethylidene)piperidine obtained in Example 115 (e) by conducting a reaction similar to that mentioned in Example 34.

¹H NMR (400 MHz, pyridine-d₅) δ ppm : 0.77-0.83 (2H, m), 0.99-1.06 (1H, m), 1.09-1.16 (1H, m), 1.12 (3H, t, J=7.0), 1.74-1.83 (1H, m), 2.14-2.24 (1H, m), 2.47-2.52 (1H, m), 2.62 (2H, t, J=7.0), 2.65 (1H, m), 2.86 (2H, t, J=7.0), 2.83-2.99 (1H, m), 3.34 and 3.36 (total 1H, each d, J=12.5), 3.77-3.81 (1H, m), 4.12 (2H, q, J=7.0), 4.84-4.92 (3H, m), 4.93 and 4.95 (total 1H, each s), 5.95-5.99 (1H, m), 7.19-7.27 (2H, m), 7.28-7.36 (1H, m), 7.58 (1H, s), 7.62 (1H, s), 7.66-7.73 (1H, m).

MS (FAB) m/z : 486 (M+H)⁺.

### (Example 117) (Z)-4-(Acetylsulfanyl)-3-(2-cyanoethylidene)-1-[2-cyclopropyl-1-(2-fluorophenyl)-2-oxoethyl]piperidine hydrochloride (Hydrochloride of the compound of Compound No. 4-47)

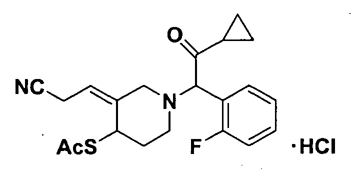

### (a) (Z)-4-(t-Butyldimethylsilyloxy)-3-(2-cyanoethylidene)-1-(triphenylmethyl)piperidine

To a solution of (Z)-4-(t-butyldimethylsilyloxy)-3-(2-hydroxyethylidene)-1-(triphenylmethyl)piperidine (10.00 g) obtained in Example 112 (f) in benzene (500 ml) were added tributylphosphine (6.10 g), acetone cyanohydrin (3.60 g) and 1,1'-azobis(N,N-dimethylformamide) (5.20 g), and the resulting mixture was stirred at room temperature for 2 hours. The reaction mixture was diluted with ethyl acetate and washed with a saturated aqueous sodium chloride solution, and the organic layer was dried over anhydrous magnesium sulfate. The solvent was removed in vacuo, and the residue was purified by chromatography on a silica gel column (ethyl acetate / hexane = 1 / 6) to afford the title compound (9.27 g, yield 91 %) as a colorless amorphous solid.

¹H NMR (400 MHz, CDCl₃) δ ppm : 0.00-0.10 (6H, m), 0.81 (9H, s), 1.82-2.01 (2H, m), 2.15-3.02 (4H, m), 3.35-3.60 (2H, m), 4.40 (1H, bs), 5.29 (1H, t, J=7.5), 7.18-7.25 (3H, m), 7.28-7.36 (5H, m), 7.46-7.59 (7H, m).

### (b) (Z)-3-(2-Cyanoethylidene)-1-(triphenylmethyl)piperidin-4-ol

The title compound was synthesized in a quantitative yield as a colorless amorphous solid using (Z)-4-(t-butyldimethylsilyloxy)-3-(2-cyanoethylidene)-1-(triphenylmethyl)piperidine obtained in (a) above by conducting a reaction similar to that mentioned in Example 1 (e).

¹H NMR (400 MHz, CDCl₃) δ ppm : 1.86-1.97 (1H, m), 1.99-2.10 (1H, m), 2.23-2.54 (2H, m), 2.64-2.88 (2H, m), 3.37 (2H, d, J=7.5), 4.50 (1H, bs), 5.30 (1H, t, J=7.5), 7.13-7.20 (1H, m), 7.23-7.31 (7H, m), 7.42-7.52 (7H, m).

### (c) (Z)-4-(Acetylsulfanyl)-3-(2-cyanoethylidene)-1-(triphenylmethyl)piperidine

The title compound was synthesized in a yield of 83% as a colorless amorphous solid using (Z)-3-(2-cyanoethylidene)-1-(triphenylmethyl)piperidin-4-ol obtained in (b) above by conducting a reaction similar to that mentioned in Example 1 (h).

¹H NMR (400 MHz, CDCl₃) δ ppm : 1.54-1.64 (1H, m), 1.78-1.85 (1H, m), 2.08 (1H, d, J=13.0), 2.22 (3H, s), 2.34-2.45 (1H, m), 3.06-3.13 (1H, m), 3.28 (1H, dd, J=18.0, 7.0), 3.41 (1H, d, J=13.0), 3.47 (1H, dd, J=18.0, 7.0), 4.68 (1H, m), 5.24 (1H, t, J=7.0), 7.13-7.32 (7H, m), 7.39-7.53 (8H, m).

### (d) (Z)-4-(Acetylsulfanyl)-3-(2-cyanoethylidene)piperidine hydrogen trifluoroacetate

The title compound was synthesized in a yield of 54% as a colorless amorphous solid using (Z)-4-(acetylsulfanyl)-3-(2-cyanoethylidene)-1-(triphenylmethyl)piperidine obtained in (c) above by conducting a reaction similar to that mentioned in Example 3 (c).

¹H NMR (400 MHz, CDCl₃) δ ppm : 2.02-2.12 (1H, m), 2.36-2.47 (1H, m), 2.39 (3H, s), 3.04-3.17 (1H, m), 3.33-3.77 (5H, m), 4.70 (1H, bs), 5.59 (1H, t, J=7.0).

### (e) (Z)-4-(Acetylsulfanyl)-3-(2-cyanoethylidene)-1-[2-cyclopropyl-1-(2-fluorophenyl)-2-oxoethyl]piperidine hydrochloride

The title compound was synthesized in a yield of 80% as a colorless amorphous solid using (Z)-4-(acetylsulfanyl)-3-(2-cyanoethylidene)piperidine hydrogen trifluoroacetate obtained in (d) above by conducting a reaction similar to that mentioned in Example 1 (g).

¹H NMR (400 MHz, CDCl₃) δ ppm : 0.75-0.88 (2H, m), 0.97-1.07 (2H, m), 1.27 (3H, t, J=7.0), 1.78-1.86 (1H, m), 2.12-2.22 (2H, m), 2.28 and 2.29 (total 3H, each s), 2.49-2.62 (1H, m), 2.68-2.73 and 2.76-2.81 (total 1H, each m), 2.88 and 3.15 (total 1H, each d, J=12.5), 3.30 and 3.41 (total 1H, each d, J=12.5), 3.46 (2H, s), 4.15 (2H, q, J=7.0), 4.28-4.34 (1H, m), 4.62-4.64 and 4.67-4.70 (total 2H, each m), 4.71 and 4.74 (total 1H, each s), 5.78 (1H, t, J=7.0), 7.07-7.17 (2H, m), 7.19-7.27 (1H, m), 7.28-7.37 (1H, m), 7.33 and 7.34 (total 1H, each s), 7.39 (1H, s).

MS (FAB) m/z : 387 (M+H)⁺.

### (Example 118) (Z)-3-(2-Carboxyethylidene)-1-[2-cyclopropyl-1-(2-fluorophenyl)-2-oxoethyl]-4-sulfanylpiperidine (Compound of Compound No. 4-13)

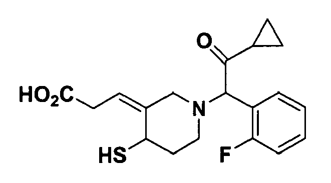

(Z)-4-(Acetylsulfanyl)-3-(2-cyanoethylidene)-1-[2-cyclopropyl-1-(2-fluorophenyl)-2-oxoethyl]piperidine hydrochloride (48 mg) obtained in Example 117 (e) was treated with 6N hydrochloric acid (6 ml) at room temperature and then the resulting mixture was stirred at 60°C for 3 hours. The reaction mixture was evaporated in vacuo, and the residue was purified using a preparative HPLC (YMC-Pack ODS-A; YMC, eluent: acetonitrile / water / acetic acid / triethylamine = 1 / 1 / 0.05 / 0.05) to afford the title compound (22 mg, yield 56%) as a colorless oil.

¹H NMR (400 MHz, pyridine-d₅) δ ppm : 0.72-0.86 (2H, m), 0.96-1.04 (1H, m), 1.07-1.15 (1H, m), 1.70-1.84 (1H, m), 2.19-2.38 (1H, m), 2.46-2.54 (1H, m), 2.61-3.09 (2H, m), 3.30 and 3.39 (total 1H, each d, J=14.0), 3.41-3.58 (3H, m), 4.55 (1H, bs), 4.89 and 4.93 (total 1H, each s), 5.72 and 5.83 (total 1H, each t, J=7.5), 7.15-7.25 (2H, m), 7.26-7.34 (1H, m), 7.67-7.73 (1H, m).

MS (FAB) m/z : 364 (M+H)⁺.

### (Test Example 1) Test for confirming in vitro platelet aggregation inhibitory effect

Male Sprague-Dawley rats (8-week old, Japan SLC, Inc.), 3 to 4 animals per group, were used for the test. The platelet aggregation was measured with an automatic platelet aggregometer (MCM Hema Tracer 313M, MC Medical, Inc.) by a partially modified method of Born et al., The Journal of Physiology, Vol. 168, Section 178 (1963). 6.3 ml of blood was sampled from the ventral aorta of a rat anesthetized with pentobarbital (40 mg/kg) using 3.8% (w/v) sodium citrate solution (0.7 ml) as an anticoagulant. The obtained blood containing citrate was centrifuged (230 g, 15 minutes, room temperature) to separate a platelet-rich plasma (hereinbelow abbreviated as PRP). The blood after the separation of PRP was further centrifuged (2,000 g, 10 minutes, room temperature) to separate a platelet-poor plasma (hereinbelow abbreviated as PPP). After the number of blood platelet in PRP was measured with a multi-item automatic blood corpuscle counter (KX-21N, Sysmex Corporation), PPP was added thereto to adjust the blood platelet count to 5×10⁸/ml. After PRP (239 µl) was pipetted into cuvettes, test compounds (1 µl) dissolved in dimethylsulfoxide (DMSO) were added to PRP and the cuvettes were set in the automatic platelet aggregometer. DMSO (1 µl) was added in place of the test compounds for the control group. After preliminary warming (37°C) for 1.5 minutes, 10 µl of adenosine 5'-diphosphate (ADP) solution (final concentration at 10 µM) was added to cause platelet aggregation. Platelet aggregation was measured for 5 minutes and the maximum platelet aggregation ratio was determined. The results are shown in Table 7.

**(Table 7)**

| Test Compound | Test Example 1 (% inhibition) 10 µg/ml |
|---|---|
| Compound of Example 15 | 90 |
| Compound of Example 18 | 73 |
| Compound of Example 19 | 77 |
| Compound of Example 22 | 67 |
| Compound of Example 23 | 77 |
| Compound of Example 44 | 81 |
| Compound of Example 50 | 79 |
| Compound of Example 51 | 81 |
| Compound of Example 74 | 80 |
| Compound of Example 83 | 68 |
| Compound of Example 86 | 82 |
| Compound of Example 91 | 74 |
| Compound of Example 111 | 82 |

The compounds of the present invention showed a remarkable platelet aggregation inhibitory effect. The compounds of the present invention are useful as an antithrombotic drug.

### (Test Example 2) Test for confirming ex vivo platelet aggregation inhibitory effect

Male Sprague-Dawley rats (8-week old, Japan SLC, Inc.), 3 to 4 animals per group, were used for the test. The platelet aggregation was measured with an automatic platelet aggregometer (MCM Hema Tracer 313M, MC Medical, Inc.) by a partially modified method of Born et al., The Journal of Physiology, Vol. 168, Section 178 (1963). Test compounds suspended in 5% Arabian gum solution or 10% dimethylacetamide/64% polyethylene glycol 400/16% Tween 80 solution were orally administered in a dose ratio of 1 mL/kg 4 hours before blood sampling. Vehicle was orally administered 4 hours in a dose ratio of 1 mL/kg before the sampling of the blood for the control group. 6.3 ml of blood was sampled from the ventral aorta of a rat anesthetized with pentobarbital (40 mg/kg) using 3.8% (w/v) sodium citrate solution (0.7 ml) as an anticoagulant. The obtained blood containing citrate was centrifuged (230 g, 15 minutes, room temperature) to separate PRP. The blood after the separation of PRP was further centrifuged (2,000 g, 10 minutes, room temperature) to separate PPP. After the number of blood platelet in PRP was measured with a multi-item automatic blood corpuscle counter (KX-21N, Sysmex Corporation), PPP was added thereto to adjust the blood platelet to 5×10⁸/ml. After PRP (240 µl) was pipetted into cuvettes, the cuvettes were set in the automatic platelet aggregometer. After preliminary warming (37°C) for 1.5 minutes, 10 µl of ADP solution (final concentration 3 µM) was added to cause platelet aggregation. Platelet aggregation was measured for 5 minutes and the maximum platelet aggregation ratio was determined. The results are shown in Table 8.

**(Table 8)**

| Test Compound | Test Example 1 (% inhibition) | |
|---|---|---|
| | 3 mg/kg | 10 mg/kg |
| Compound of Example 13 | - | 63 |
| Compound of Example 17 | - | 92 |
| Compound of Example 18 | - | 74 |
| Compound of Example 19 | - | 60 |
| Compound of Example 21 | - | 90 |
| Compound of Example 22 | - | 79 |
| Compound of Example 23 | - | 72 |
| Compound of Example 24 | - | 85 |
| Compound of Example 25 | - | 92 |
| Compound of Example 26 | - | 92 |
| Compound of Example 29 | - | 89 |
| Compound of Example 30 | - | 93 |
| Compound of Example 31 | - | 82 |
| Compound of Example 32 | - | 86 |
| Compound of Example 36 | - | 80 |
| Compound of Example 38 | - | 66 |
| Compound of Example 44 | 61 | - |
| Compound of Example 62 | - | 94 |
| Compound of Example 64 | - | 94 |
| Compound of Example 66 | - | 87 |
| Compound of Example 72 | - | 76 |
| Compound of Example 74 | - | 100 |
| Compound of Example 78 | - | 75 |
| Compound of Example 81 | - | 83 |
| Compound of Example 82 | - | 81 |
| Compound of Example 84 | - | 82 |
| Compound of Example 85 | - | 82 |
| Compound of Example 89 | - | 83 |
| Compound of Example 111 | - | 82 |

The compounds of the present invention showed a remarkable platelet aggregation inhibitory effect. The compounds of the present invention are useful as an antithrombotic drug.

### (Preparation Example 1) Hard capsules

Each standard two-part hard gelatine capsule is filled with 100 mg of powdered compound of Example 1, 150 mg of lactose, 50 mg of cellulose and 6 mg of magnesium stearate to prepare single unit capsules, which are washed and then dried.

### (Preparation Example 2) Soft capsules

A mixture of the compound of Example 2 in digestible oil such as soybean oil, cottonseed oil and olive oil is prepared and injected into gelatine with a direct exchange pump to obtain soft capsules containing 100 mg of an active substance, which are washed and then dried.

### (Preparation Example 3) Tablets

Tablets are prepared according to a common method using 100 mg of the compound of Example 3, 0.2 mg of colloidal silicon dioxide, 5 mg of magnesium stearate, 275 mg of microcrystalline cellulose, 11 mg of starch and 98.8 mg of lactose.

A tablet coating is applied, if desired.

### (Preparation Example 4) Suspension

A suspension is prepared such that 5 ml of the suspension contains 100 mg of finely powdered compound of Example 4, 100 mg of sodium carboxymethyl cellulose, 5 mg of sodium benzoate, 1.0 g of sorbitol solution (Japan Pharmacopoeia) and 0.025 ml of vanillin.

### (Preparation Example 5) Cream

A cream is prepared by mixing 100 mg of finely powdered compound of Example 5 into 5 g of a cream containing 40% white petrolatum, 3% microcrystalline wax, 10% lanolin, 5% Span 20, 0.3% Tween 20 and 41.7% water.

### (Preparation Example 6) Injection agent

1.5% by weight of the compound of Example 6 was the resultant mixture was stirred in 10% by weight of propylene glycol and after the volume was adjusted to a predetermined volume with water for injection, the mixture was sterilized to prepare an injectable preparation.

### Industrial Applicability

The compounds according to the present invention are chemically stable and have excellent platelet activation-inhibiting activity and thrombosis-inhibiting activity, and also the responses thereof are quick and the toxicities thereof are low. Therefore, the compounds are useful as drugs for prophylaxis, recurrence prevention, or therapy (in particular, for therapy) of thromboembolism or diseases induced by activation of platelets such as platelet aggregation or platelet release reaction, for example, restenosis after percutaneous coronary intervention (PCI), angioplasty, endarterectomy, or stenting; acute coronary syndromes; stable or unstable angina; myocardial infarction; atrial fibrillation; cerebral ischemic attack; cerebral infarction; atherosclerosis; cardiovascular and cerebrovascular diseases associated with diabetes mellitus, such as thromboembolism; peripheral arterial diseases; heparin-induced thrombocytopenia (HIT); thrombotic thrombocytopenic purpura (TTP); antiphospholipid antibody syndromes; venous thrombosis; and septicemia.

## Claims

1. A compound having the general formula (I) shown below, 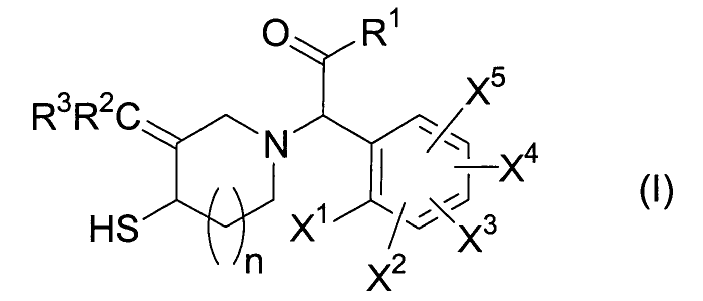
[where R¹ represents a hydrogen atom, a C1-C6 alkyl group which may be substituted (said substituent group represents a halogen atom or a C1-C6 alkoxy group), a C3-C6 cycloalkyl group which may be substituted (said substituent group represents a halogen atom or a C1-C6 alkoxy group), a C1-C6 alkoxy group which may be substituted (said substituent group represents a halogen atom or a C1-C6 alkoxy group), or a C6-C10 aryl group which may be substituted (said substituent group represents a halogen atom, a C1-C6 alkyl group, a C1-C6 alkoxy group, a cyano group, or a nitro group);
R² represents a hydrogen atom, a halogen atom, a carboxy group, a C2-C7 alkoxycarbonyl group, a carbamoyl group, a cyano group, a C1-C6 alkyl group, a halogeno C1-C6 alkyl group, a C1-C6 alkyl group substituted by a heteroaryl group, a C1-C6 alkoxy group, a halogeno C1-C6 alkoxy group, a hydroxy C1-C6 alkyl group, a C2-C12 alkoxyalkyl group, a formyl group, a C2-C7 alkanoyl group, a C4-C7 cycloalkylcarbonyl group, a C2-C7 alkylcarbamoyl group, a di(C1-C6 alkyl)carbamoyl group, a group of formula R⁴-CO-CR⁵R⁶-(CH₂)ₘ {wherein R⁴ represents a hydroxyl group, an amino group, a C1-C6 alkyl group, a C1-C6 alkylamino group, a di(C1-C6 alkyl)amino group, a hydroxyamino group, a C1-C6 alkoxyamino group or a C1-C6 alkoxy group; R⁵ and R⁶ are the same or different and each represents a hydrogen atom or a C1-C6 alkyl group; and m represents an integer of from 0 to 5}, a group of formula R⁷-CO-(CH₂)₁-N(R⁸) {wherein R⁷ represents a hydroxyl group, an amino group, a C1-C6 alkyl group, a C1-C6 alkylamino group, a di(C1-C6 alkyl)amino group, a hydroxyamino group, a C1-C6 alkoxyamino group or a C1-C6 alkoxy group; R⁸ represents a hydrogen atom or a C1-C6 alkyl group; and 1 represents an integer of from 0 to 5} or a sulfamoyl C1-C6 alkyl group;
R³ represents a substituted C1-C6 alkyl group {said substituent group represents a C6-C10 aryl group or a C6-C10 aryl group substituted with from 1 to 5 substituents selected from <Substituent group α>; a heterocyclyl group or a heterocyclyl group substituted with from 1 to 5 substituents selected from <Substituent group α> (said heterocyclyl groups may be substituted by from 1 to 4 oxo groups); a heteroaryl group or a heteroaryl group substituted with from 1 to 5 substituents selected from <Substituent group α>; or a substituent selected from <Substituent group β>}, or a heterocyclyl group or a heterocyclyl group substituted with from 1 to 5 substituents selected from <Substituent group α> (said heterocyclyl groups may be substituted by from 1 to 4 oxo groups);
X¹, X², X³, X⁴ and X⁵ each independently represents a hydrogen atom, a halogen atom, an amino group, a carboxy group, a carbamoyl group, a cyano group, a nitro group, a C1-C6 alkyl group, a halogeno C1-C6 alkyl group, a C1-C6 alkoxy group or a halogeno C1-C6 alkoxy group;
n represents an integer of from 0 to 2,
<Substituent group α> is defined by a halogen atom, an amino group, a carboxy group, a C2-C7 alkoxycarbonyl group, a carbamoyl group, a cyano group, a hydroxyl group, a nitro group, a C1-C6 alkyl group, a halogeno C1-C6 alkyl group, a C1-C6 alkyl group substituted with heteroaryl group(s), a C1-C6 alkoxy group, a halogeno C1-C6 alkoxy group, a hydroxy C1-C6 alkyl group, a C2-C12 alkoxyalkyl group, a formyl group, a C2-C7 alkanoyl group, a C4-C7 cycloalkylcarbonyl group, a C1-C6 alkylamino group, a di(C1-C6 alkyl)amino group, a C2-C7 alkylcarbamoyl group, a di(C1-C6 alkyl)carbamoyl group, a group of formula R⁴-CO-CR⁵R⁶-(CH₂)ₘ- {wherein R⁴ represents a hydroxyl group, an amino group, a C1-C6 alkyl group, a C1-C6 alkylamino group, a di(C1-C6 alkyl)amino group, a hydroxyamino group, a C1-C6 alkoxyamino group or a C1-C6 alkoxy group; R⁵ and R⁶ are the same or different and each represents a hydrogen atom or a C1-C6 alkyl group; and m represents an integer of from 0 to 5} and a sulfamoyl C1-C6 alkyl group; and
<Substituent group β> is defined by a halogen atom, an amino group, a carboxy group, a C2-C7 alkoxycarbonyl group, a carbamoyl group, a cyano group, a hydroxyl group, a nitro group, a C1-C6 alkoxy group, a halogeno C1-C6 alkoxy group, a formyl group, a C2-C7 alkanoyl group, a C4-C7 cycloalkylcarbonyl group, a C1-C6 alkylamino group, a di(C1-C6 alkyl)amino group, a C2-C7 alkylcarbamoyl group, a di(C1-C6 alkyl)carbamoyl group, a hydroxyaminocarbonyl group, a (C1-C6 alkoxy)aminocarbonyl group, a group of formula R⁹-CO-(CH₂)ₖ-N(R¹⁰)- {wherein R⁹ represents a hydroxyl group, an amino group, a C1-C6 alkyl group, a C1-C6 alkylamino group, a di(C1-C6 alkyl)amino group, a hydroxyamino group, a C1-C6 alkoxyamino group or a C1-C6 alkoxy group; R¹⁰ represents a hydrogen atom or a C1-C6 alkyl group; and k represents an integer of from 0 to 5} and a sulfamoyl C1-C6 alkyl group], pharmacologically acceptable salts thereof or prodrugs thereof..

2. A compound according to claim 1 wherein R¹ represents a C1-C6 alkyl group, a halogeno C1-C6 alkyl group, a C3-C6 cycloalkyl group, a halogeno C3-C6 cycloalkyl group or a C1-C6 alkoxy group, pharmacologically acceptable salts thereof or prodrugs thereof.

3. A compound according to claim 1 wherein R¹ represents a C3-C6 cycloalkyl group, a halogeno C3-C6 cycloalkyl group or a C1-C6 alkoxy group, pharmacologically acceptable salts thereof or prodrugs thereof.

4. A compound according to claim 1 wherein R¹ represents a C3-C6 cycloalkyl group or a C1-C6 alkoxy group, pharmacologically acceptable salts thereof or prodrugs thereof.

5. A compound according to claim 1 wherein R¹ represents a cyclopropyl group or a methoxy group, pharmacologically acceptable salts thereof or prodrugs thereof.

6. A compound according to claim 1 wherein R¹ is a cyclopropyl group, pharmacologically acceptable salts thereof or prodrugs thereof.

7. A compound according to any one of claims 1 to 6 wherein R² represents a hydrogen atom or a C1-C6 alkyl group, pharmacologically acceptable salts thereof or prodrugs thereof.

8. A compound according to any one of claims 1 to 6, wherein R² represents a hydrogen atom or a methyl group, pharmacologically acceptable salts thereof or prodrugs thereof.

9. A compound according to any one of claims 1 to 6, wherein R² represents a hydrogen atom, pharmacologically acceptable salts thereof or prodrugs thereof.

10. A compound according to any one of claims 1 to 9, wherein R³ represents a substituted C1-C6 alkyl group {said substituent group represents a heterocyclyl group or a heterocyclyl group substituted with 1 or 2 substituents selected from <Substituent group α> (said heterocyclyl groups may be substituted by 1 or 2 oxo groups), a heteroaryl group or a heteroaryl group substituted with 1 or 2 substituents selected from <Substituent group α> or a substituent selected from <Substituent group β>}, or a heterocyclyl group or a heterocyclyl group substituted with 1 or 2 substituents selected from <Substituent group α> (said heterocyclyl groups may be substituted by 1 or 2 oxo groups), pharmacologically acceptable salts thereof or prodrugs thereof.

11. A compound according to any one of claims 1 to 9, wherein R³ represents a substituted C1-C6 alkyl group {said substituent group represents a 4- to 7-membered heterocyclyl group containing at least one nitrogen atom which may be substituted with 1 or 2 substituents selected from <Substituent group α1> (said heterocyclyl group may be substituted by one oxo group), a heteroaryl group containing at least one nitrogen atom which may be substituted with 1 or 2 substituents selected from <Substituent group α1>, a carboxy group, a C2-C7 alkoxycarbonyl group, a cyano group, a hydroxyl group, a C1-C6 alkoxy group or a group of formula R⁹-CO-(CH₂)ₖ-N(R¹⁰)- (wherein R⁹ represents a hydroxyl group or a C1-C6 alkoxy group; R¹⁰ represents a C1-C6 alkyl group; and k represents an integer of from 1 to 5)}, or a 4- to 7-membered heterocyclyl group containing at least one nitrogen atom which may be substituted with one substituent selected from <Substituent group α1> (said heterocyclyl group may be substituted by one oxo group), and
<Substituent group α1> is a group consisting of a carboxy group, a C2-C7 alkoxycarbonyl group and a group of formula R⁴-CO-CR⁵R⁶-(CH₂)ₘ- (wherein R⁴ represents a hydroxyl group, an amino group, a C1-C6 alkylamino group, a di(C1-C6 alkyl)amino group, a hydroxyamino group, a C1-C6 alkoxyamino group or a C1-C6 alkoxy group; R⁵ and R⁶ each represents a hydrogen atom; and m represents an integer of from 0 to 5), pharmacologically acceptable salts thereof or prodrugs thereof.

12. A compound according to any one of claims 1 to 9, wherein R³ represents a substituted C1-C3 alkyl group {said substituent group represents a pyrrolidinyl, piperidinyl, morpholinyl, thiomorpholinyl, piperazinyl, pyrazolyl, triazolyl or tetrazolyl group which may be substituted with 1 or 2 substituents selected from <Substituent group α2> (said pyrrolidinyl, piperidinyl, morpholinyl, thiomorpholinyl or piperazinyl group may be substituted by one oxo group), a carboxy group, a C2-C4 alkoxycarbonyl group, a hydroxyl group or a group of formula R⁹-CO-(CH₂)ₖ-N(R¹⁰)- (wherein R⁹ represents a hydroxyl group or a C1-C3 alkoxy group; R¹⁰ represents a C1-C3 alkyl group; and k represents an integer of from 1 to 3)}, or a pyrrolidinyl, piperidinyl, morpholinyl, thiomorpholinyl or piperazinyl group which may be substituted one substituent selected from <Substituent group α2> (said pyrrolidinyl, piperidinyl, morpholinyl, thiomorpholinyl or piperazinyl group may be substituted by one oxo group), and
<Substituent group α2> is a group consisting of a carboxy group, a C2-C4 alkoxycarbonyl group and a group of formula R⁴-CO-CR⁵R⁶-(CH₂)ₘ- (wherein R⁴ represents a hydroxyl group or a C1-C3 alkoxy group, R⁵ and R⁶ each represents a hydrogen atom, and m represents an integer of from 0 to 2), pharmacologically acceptable salts thereof or prodrugs thereof.

13. A compound according to any one of claims 1 to 9, wherein R³ represents a substituted methyl or ethyl group {said substituent group represents a pyrrolidinyl, piperidinyl, piperazinyl or pyrazolyl group which may be substituted with 1 or 2 substituents selected from <Substituent group α3> (said pyrrolidinyl, piperidinyl or piperazinyl group may be substituted by one oxo group), a carboxy group or a group of formula R⁹-CO-(CH₂)ₖ-N(R¹⁰)- (wherein R⁹ represents a hydroxyl group, a methoxy group or an ethoxy group; R¹⁰ represents a methyl group, an ethyl group or an isopropyl group; and k represents an integer of from 1 to 3), and
<Substituent group α3> is a group consisting of a carboxy group, a methoxycarbonyl group, an ethoxycarbonyl group, a carboxymethyl group, a methoxycarbonylmethyl group, an ethoxycarbonylmethyl group, a 2-(carboxy)ethyl group, a 2-(methoxycarbonyl)ethyl group and a 2-(ethoxycarbonyl)ethyl group, pharmacologically acceptable salts thereof or prodrugs thereof.

14. A compound according to any one of claims 1 to 13 wherein X¹, X², X³, X⁴ and X⁵ represent independently a hydrogen atom or a halogen atom, pharmacologically acceptable salts thereof or prodrugs thereof.

15. A compound according to any one of claims 1 to 13 wherein X¹ and X² represent independently a hydrogen atom or a halogen atom; and X³, X⁴ and X⁵ represent a hydrogen atom, pharmacologically acceptable salts thereof or prodrugs thereof.

16. A compound according to any one of claims 1 to 13 wherein X¹ represents a halogen atom; and X², X³, X⁴ and X⁵ represent a hydrogen atom, pharmacologically acceptable salts thereof or prodrugs thereof.

17. A compound according to any one of claims 1 to 13 wherein X¹ represents a fluorine atom; and X², X³, X⁴ and X⁵ represent a hydrogen atom, pharmacologically acceptable salts thereof or prodrugs thereof.

18. A compound according to any one of claims 1 to 17 wherein n represents 0 or 1, pharmacologically acceptable salts thereof or prodrugs thereof.

19. A compound according to any one of claims 1 to 17 wherein n represents 1, pharmacologically acceptable salts thereof or prodrugs thereof.

20. A compound according to any one of claims 1 to 19 wherein a prodrug which is a pharmacologically acceptable ester is formed on the sulfanyl moiety, pharmacologically acceptable salts thereof, or prodrugs thereof.

21. The compound according to claim 20 wherein a functional group forming the pharmacologically acceptable ester with a sulfanyl group is a C1-C6 alkanoyl group or an arylcarbonyl group, pharmacologically acceptable salts thereof or prodrugs thereof.

22. The compound according to claim 20 wherein a functional group forming the pharmacologically acceptable ester with a sulfanyl group is a C1-C3 alkanoyl group or a benzoyl group, pharmacologically acceptable salts thereof or prodrugs thereof.

23. The compound according to claim 20 wherein a functional group forming the pharmacologically acceptable ester with a sulfanyl group is an ester formed with an acetyl group, pharmacologically acceptable salts thereof or prodrugs thereof.

24. A pharmaceutical composition containing a compound according to any one of claims 1 to 23, pharmacologically acceptable salts thereof or prodrugs thereof as an active ingredient.

25. A pharmaceutical composition according to claim 24 wherein the pharmaceutical composition is a composition for prophylactic or therapeutic agents for diseases related to thrombus or embolus formation.

26. Use of a compound according to any one of claims 1 to 23, pharmacologically acceptable salts thereof or prodrugs thereof to manufacture a pharmaceutical composition.

27. Use according to claim 26 wherein the pharmaceutical composition is a composition for prophylactic or therapeutic agents for diseases related to thrombus or embolus formation.

28. Use of a compound according to any one of claims 1 to 23, pharmacologically acceptable salts thereof or prodrugs thereof for prophylactic or therapeutic agents for diseases related to thrombus or embolus formation.

29. A prophylactic or therapeutic method for diseases by administration of a compound according to any one of claims 1 to 23, pharmacologically acceptable salts thereof or prodrugs thereof to warm-blooded animals at pharmacologically effective doses.

30. A method according to claim 29 wherein the diseases are diseases related to thrombus or embolus formation.

31. A method according to claim 29 or 30 wherein the warm-blooded animals are humans.
